# EUROPEAN PATENT APPLICATION

(11) **EP 2 251 438 A1**
(43) Date of publication of application: **17.11.2010**
(21) Application number: 09305442.7
(22) Date of filing: 15.05.2009
(51) Int. Cl.: C12Q 1/68

(54) **Genetic markers associated with sporadic pancreatic cancer**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Jacobson, Claude

(57) **Abstract**

The present invention relates to genetic markers associated with sporadic pancreatic cancer and to their use for detecting a predisposition to sporadic pancreatic cancer and/or for diagnosing sporadic pancreatic cancer.

SEQUENCE LISTING

<110>  INSERM

<120>  Genetic markers associated with sporadic pancreatic cancer

<130>  BET09P0228

<160>  62

<170>  PatentIn version 3.5

<210>  1
<211>  20158
<212>  DNA
<213>  Homo sapiens

<220>
<221>  misc_feature
<222>  (1)..(20158)
<223>  chromosome 1 nucleotides 48784697 to 48804854

<400>  1
ggacaaagac cggtcttaaa gcgggtggaa gagaaagaca agtatcagca gagaaacgga      60

gtgcggtcag gcacatgacc tgaaataaca cagaaaaggg tccgaaggcc catgtagcta     120

accgcgtaga gtggaaggtg acgctgaaat attaggctag agacaagtga gtgaaggctt     180

tgaatgccaa gttaagaaac ttaaacttct attctgtagg caatgaaagc cagtgaacat     240

atatgggttg tatctgtaaa tatgaattta ctcagtgtat atttaatatc atctactatg     300

tttattatgt tcaggctttt tggaaggctc agggtatact atggttaaaa acaaaagaag     360

acaaaaccaa gacaatggtt cttaagtcta gaagcagaga caaatagcga ggtagtaaaa     420

caaacaaaca cacacacaca cacacacaca cacacacaca catacataat agcaattgat     480

atatactatg aagaaaatgc acagagtctg tacctgcaga taatgcaggc gtgggaaggc     540

ctctctgagg aggggatatg aactgagagt gagcagtgac tgcagcggta cctaagaaga     600

gggaagggtg tctgggaagg gtcctcgggt gagagacgga aactggaaga gggctggcag     660

agggtgatag gcaagggcta gatcaccaag aactctcggc caccttaagg agtctggact     720

ttcttggtga atgggaagct actgaagagt ttgaagcagg aaaatgacac ggtctcattt     780

ttatttccaa agtcactgct gcactaaaag agaaggcaag agtggaagca ggcagagtag     840

ctagggacga ctgcaatggt acaggggaag aaaagggagg tcccagctgt acagaggcag     900

tagagatggg aagatgtatt tgtaggaaga gcaaacagga cctgcttcca agttggatgt     960

ggggcccaaa ggaggggaag gagtgaagga tgactccttc atctttggct ttagcagctg    1020

ggtcgatggg ggtgccctga tctggcatat gaaagaatgt ggcaggaata gcttcagggg    1080

agagattagg aggtcagttc ggagcatgtt gggtttgagc tatgagacat taagtatcca    1140

ggttctgaat cagaaatggg aaaaaatcag aaaatgggaa ttgtttctga attttcagct    1200

ctttgggatt attcatgcca caaattcttg ctgactgtgc agtgtgttcc attctgctca    1260

ggtctccacc tcttcctccc tgctcttagg gtgtcccaag ggctttaggg gaatggcagt    1320

aactgaagga tcctctgttc tcctgtctca gggcctcaga tctggggcct tgagagtgca    1380

cacaagtggg aatcaagaga ccaatgtggt tttcatcatt aactagccat gtgaccttgg 1440
gccatttccc ttcactgggc ctaacttctc tgtgggtaaa tgtagtctct tgagtccttt 1500
ttcagttaac atggtacatt gaagcaaagc cctcaataca ataagcttat gagaaatccc 1560
atctgcattc tttcactcac aaatatttag ggagaactga ctatgtgcca agttatttta 1620
gacacttggg atatacagtg aacaaaactg gcaaagacct ctgtctttat ggagtttaaa 1680
ttctagctat gggaaacaag ctgtagacct aataatacat gtgtaaatta tatagaatgc 1740
tggaacgtga taagtgctat ggagtaaaga taaagagtag ggtaagggga gtgctggggc 1800
caagaagaag atcgcagcat taagtagagt gctcagagag gcttccttga gaaggtgggt 1860
gggaatgggc ttgggggagg tgaaggtgct ggccagcacc aaagcggagt taatcctcct 1920
catctgaaac acagccctga ccagtcattt cacaattagg ctcttgctaa atccagccca 1980
atcccaaact ggggataata atcaacctca gggacgagtc ctccctcatg gggtcaggaa 2040
cacctttttc gtggtaatgg ctgttttctg tctccaggtc caggtcctct tcacctccca 2100
gagcagcttc ggcagagaca agcctcacac acatcctccc gcctggcctg cggcctccct 2160
tccctgtcct gctcctcccc ttgtggactc atctctgaag ctttctccat acttctcact 2220
ccctgcagct cttctagaac cctcagcaag ctcagctggc ctgactgcat tgaagggtgc 2280
tgagctctgc cccacttctc agacacaata acccacttct cctttccccc tagttgtggg 2340
ccatggcagc tctgattacc atctccattt tgcagatgaa aaatcagaag tcacttgtct 2400
gatgagtcac atcaagtatt ggcgcaagga ggattgaaac ccattgattt attctctcat 2460
ttgttcattt atatattcat attaagtggc ttctatgtgc cagacactgt tctaggagct 2520
aggatacaac aaagagaaaa cagacacatc cctgtcctca taaaggtctt ttcttgccat 2580
tatcctgctt ccaaataaga aaattgaatt cctacctgct ccctgtttgg aaactcccac 2640
ctagaattca ttttctgctc atactcctgc ccctcccagc aacagatgat caccattaaa 2700
gggggaagat taattgtgca gagggatagg ccaaggaaaa gtgatttctc tttggtaagg 2760
ctgacagcac tgctagaata ttaagaggca cctgcctagg agcctctagg taaagctaga 2820
agaaaaatgt gacaatgaaa tatcttttgt ctctgcctgc ctcaccaatc ctcccacagt 2880
gtcagacaag tgcatgacta aacaggtgaa tccttctctg aagtgcagtg tggaggctgc 2940
agatgcaatt gcagagtcct ttcccattgc aggcaagtcc agaggcaccc cctaggtaag 3000
aagacctgtg aaagagagga gagggagaag gagagggaga gggagaggga gaaagagaga 3060
aagagataga gagagatgga ttagagggtc agggagagac agcaggagag aggaagagac 3120
aaagaggccg cagaggcaga gaccaaaagc gaaagagatg caaatggaca ggaaaataca 3180
gagaaactgg tcaggatgag gcaagaagat aaggagagaa ggataatcag agaggctgag 3240
agagactcag gcttgaaaac tgaggagaaa tggggtgagg agcagagaga gaaaaacagg 3300
gagcaaatga cagaaaagga aagaatcaaa ctgaaagagg tgtgagagag atataaatgt 3360
aggcaacagg agaagggagg gagagagggg agaaaggcaa aagataagac agaaagagac 3420

aaaagaaaaa aatgcagaga agcacagccg cagaggacag actgtaaatg agatgcataa 3480
gactgaaata gagacaggaa cagtgagaga gactgagaag gactgagaga agaaggtgga 3540
gagacacaga tagggacggg aagggaggcc tcttttttga tgggtgaaat gcaattccac 3600
agagtaggta tttcctgaaa ggctcacttc acggaggatg ctggagatga atgcttatac 3660
ggaaattgtg agaagagtgt ccaatccatc acagaatgaa ttaaagtttg gggtaactat 3720
aaggctcgaa attagttttc cattgccact gtaacaaatc accacacact tagtggctta 3780
aaacaacaga aatgtattct ctcagttctg gaggccagaa gtcagaaatc agtttcactg 3840
gggccaaaat ctaggtgtca caggcctgca ttccctccac aggccccggg gagaaaacag 3900
agaaatagtt ggtttctggt ggctgttggc attccttgct ttgtgatcat atcattccca 3960
tctctgcccc catggtcaca ttgcctcctc cctcttcagt gtatgtcaga tctctctctg 4020
cctgtctctt ctagggatac atgtgattgt atttaggacc tatccagata atccaggata 4080
gtctcaacct cccatctgaa gagccttaac cataacattt acaaagacct tgtttccata 4140
taaggtatta ttgataggtt ctagggatca ggaaccgaat gtctttgggg gtcattattc 4200
agcctattcc acactgcctg agatttcatc tgcttagcta agagctggca gatgaaacat 4260
cctggcatac ccaggatgaa ggtgctgggt atcaggggtg gagttctggc tctgtgtaag 4320
acaccttgct gggtgctcca ccaactccac cccacctgac ctacacatgc agcaagtgga 4380
tttaccagga ttcagaccca gatatgtcta atttcagagt gctttttgct ggcattattt 4440
gctgcctctc aagggcaatg ttgcctaccg tacttcttaa acatagtaaa cctgctcctc 4500
ctcttcctcc actgctattg cattgttcag atccttgtct cttatctgaa ctattgcttc 4560
agcatcctaa ttggtcttct ggcttccagt ttctccccat tattgctgag aatgatgttc 4620
tcacaattgc tgaccttgtc acttcctgac ctacaaccct acactgattc actgtcacct 4680
ccaaggtaag acctaaattc tcagatctgg ctttcatggc cttccataag ctagtataag 4740
ccttatctcc tgacactgta ttccgttgtg catgtattta gaaggccagg tttaaagtcc 4800
aaaaagcatg ggttttaatc ctggctccta catgtttagc tgtgtgaacc tgggcaggct 4860
atttactgtc tctgagcctt agtgtcctca tctctaaaat acataatgct tcctacctca 4920
ggattattgc attgattaga aattatatat ccaagatacc cagtacaatg ctggtatgca 4980
gtagcttttt tattatcatt actacatatg aaactgtcat ctccaacaaa gtatgagctc 5040
ttcaaggaca agagtgtgtc acattcacat ttgtttccag agttatttag catgttaggc 5100
ctatgaacaa aggtagggag aaaaagcaag acaaaaacaa atcaagatga gagagagaga 5160
aagaaaagaa cagagagaca gagacgggga gagatgaaga aactatttgt aaaattctgt 5220
gttttctgga cgtttggtat aacatcttgt aggtttcttc catgctgtgt gccttggttt 5280
cctctcctgt aaaattggcg ggcagttgaa ctagatgatc tctagttcta attctcactc 5340
tccgctctac cctgggaaaa acgtttccct ggagcagggg atatcctttc tgaccaagca 5400
gatgtgcagc agctttatca ggtgtgacag tgctgcccat caccctaatg gggtcttttg 5460
aagagaagcc aagttagagg ctggtgggct agatcagatg gagatctgac ctcattttta 5520

atgtgcctga ttctggatga gataaccttg gtgggtattt ggacttcaga ccacattagg 5580
caggttgctg ttattcattc acttgttcat tcattattta tttttcattc attcatttat 5640
ttttgagatg gagtcttgct ctgtcgctca ggctggagtg gagtggtgcg atctcagctc 5700
actgcaacct ccgcctcctg ggttcaagtg attctcctgc ctcagcctcc caagtagctg 5760
ggactacagg tgtgtgccac catgcccggc taatttttgt gtttttaaca gagacgaggt 5820
ttcaccatgc tgatcaggct tgtctcgaac tcctgacctt atgatccgcc tgcctcagct 5880
tcccaaagtg ctaggattac aggcatgagc cactgcaccc agcctcattc attatttatt 5940
actcattgat tcattcaaca gataagttat caatcacctc ctctgtgctg ggccatgtaa 6000
taggtataca gaacattcac actggctatg tagagtaaac aaaacaggaa tggctgtgct 6060
ctcaatctgt ttatagtcca ggtactattg acatgtttgg ctaaatgatt ctgtgttgtg 6120
ggggcagccc tgtgcattgt agggtgttta gcagcatccg tggtttctcc cactagatgt 6180
cagcagcacc cacctcattg tgacaaccaa gtgtgtcttc aaacattgcc aaacactccc 6240
tggggcaaac acacctgggg gtgagaacca ctggtctagt ggttgtacag tatctggtgg 6300
agatacagca agtgacatga gagaggtggg caggatcttg actgggaagg gtcttggctt 6360
ccctacttag aaaaaagtaa tccctgaatg gatccttgga gacagggaag gggagaggga 6420
taccactcac aggctgcatt aatctagtaa gacggttatc taatcaccct cctcattact 6480
ttggcaggga agagtgtcct ccctccattt ctagcacacc tcaatacccg agatgcctct 6540
cccttctcta cttgcccaag actgctggta tttatagctt gtctgtctaa ctcagaccca 6600
cttctcactc aagagggaaa ttaatctctc tctccaaatc ccacactatc acttgcctca 6660
tggcttcctc attcccctgc tcctccaatc caggcaccag caatttccca gcagtgcctc 6720
cctttcccca ctgacagttg accgtggctc tgaaaattgg ctcttggaat cagctgtgcc 6780
tgcccagctg ggccccagct cagcagtgcc tgggactttg atgcagttca acctgagagg 6840
gggctctgct tttctgagag atattcagct cctgcttgtc tcacatatat ttttagaagc 6900
ttcaactgga tgcacagtta aaaatgagac tgaagaccaa agtaaatatt tgcatagcag 6960
gatcttggag tggcggagaa gagagaagga agagaggagt tctgatgggc ctggcttcag 7020
gcctcagctc tgcttctgag tggctgcact tgcttggagc aagtctcctt taggtatcag 7080
ttttccaatc tgtaaatttg gaggagggac tggcttgccg atgttttgaa acatttcagc 7140
tccgatgttc tgtgagcctc tatatcagaa gggccattaa ggggttcctg aaccctctga 7200
tactactgac aaactgtgta aattttctgg gaagagtatt cataattttc tacaattttc 7260
aaaaaaaaaa tcagcatctt ctaaaatgtt aagaatctct gctctaggtc tgtggataga 7320
gataattgag tttatcacca acttcctggt aatgttcctt cttctcattt tctaacctgt 7380
tcccttgtta tagtctgact cttctgcgat tagtgagaat ctggcagagt gcaggggagt 7440
aaaaccaagc tagacttgaa atggaatgaa caatatgaag aatctatcct gagtgcatct 7500
gcattaactt aactagaaaa cctcaaagag tcctgtggcc caagtctgct gcagcggtca 7560

aagaacagtg cagcaaagaa gatccctagg aggtaactgc aaaactcagc tgaagtctgg 7620
gccattctgt gggtgagaat tggggttcgg tcttttcctg ggaagcatca aatcctgggc 7680
tcagaactta aaaggtccac taaagggttt gctggaggga caggacatgg ggtcctctac 7740
ctttaaaaga agcccactcc cctgctcttg tgtcttcacc cctgattagg cctgaatctg 7800
aagcacttac atggattaat ggaatgagca atgtcctgtg aatcaagaga cctatatttg 7860
agcaacagct ttcaccaaat gctgtatgcc ttgggcaggt cactgaccca ctcctgggct 7920
tcagtttctc catttagaca ataaagagga attggcttgc ttcacagttt caccaaagaa 7980
tgactattat tatagtgaaa ctccattata atatctcatt attggtcatt tccttgcatt 8040
tattcctcat tttgagacat ccttgttata tttcaagcaa aacagcaatg aatcccatgc 8100
ctaggaagtg tgtgattata aacttaaatc ccaatcttta ggaccctcat ggttctaaca 8160
ttttatgatt ttttttttct gctgtgatgt tactttcttt ttaactgttg ttttgaactt 8220
aaaacttagg gaatgaaatt aatgttgatg accacggact attttccagg tactgtgcta 8280
gtttcattta ttacaagcaa ggtagatttt ggtatccttg tgttataggt tagaaaactg 8340
aggttcagag atgtcacctt tctaatacca ctcagttagc aagcggacgg ttaggactta 8400
aactcgggtc tgtcctacta caaggcctgt ggcttgaaaa attattttta tgctataatt 8460
ctttattatt ttaactttct taaaattaat tcatttattc aatatttatt gagtatctgc 8520
tgtgtaaggt actgtgccag acgctatggg tttggagatg agaaatcagt ccctgttctt 8580
gagcagcctt tagagaagac atgtaaataa aagttgcaag ttagggcaat gacactatgg 8640
caggggaaat aagggaagtc agggtgcaaa aagtgggatt taatattgga gacaccctgg 8700
aagcttctca gaataggaaa cacctgagct gaattgaggg ggtaagattg tctttttttt 8760
ttttcctgag ataactcttt attaaacaag ttagtcaaca attaattgag aatctatgat 8820
gtaccagata cttttctagt ttcaggaata caattgcata caacgacaaa ctcttataga 8880
gctcacaagg gaagaaaggc agtaaaccaa ccaacaaata cagtataaga ttgaaatcca 8940
ggtgactgga aaagtaagac ttggccaggt aaagagagtt gaatggcatg aaacagcatc 9000
ctgaataaag gcagaggcat gtgcaagcat gctgaggcat gatgatccct ttgaagatgt 9060
ttgtcacggc tggaatatct tctaaaagat aaagaaggaa gcaagagaaa aatgcaagca 9120
cagatcatga gtgccttgtg tgatgttatg agtatgaact ttctcctgca gataatggga 9180
agccagggga gggttttaag agacagaggc acaatgctca aatttggttc cagggtgatt 9240
tctcctgtgg ccctggtgaa gtgggatttg aaggggaccc agactgcaga caggaagaat 9300
ggtcctggtc cagggagaat ggtgagttag ggcctgagct caggcactct caggggagtt 9360
gtgaggagga caaagaatta gaaatatttg tctagtaaga aagatgatac ttgggtgctg 9420
gcagaggtga ctggactagg aaaagcatca agaatagcac accaggttcc tgccttggag 9480
aactgcatgg tggtgttccc atcaaccttg ggaaggaagt ctaaaggtgg ctcctagaag 9540
gcagggagct ctgtgctccc tagagtactt tccatcctgc ccttcttagt agtaatgttg 9600
cctaatttta ttttgctata ccgatgtgcc aagtagctat tatgattcct atagtgaatc 9660

tccattaaaa ggaccagtta gtggaatttt cttgtatcta atgctcttct ttggacatat 9720
caagcaaagc tgacaatgat cccactgaca ggatgggtat gaggctggcc ttaaactccc 9780
catgaggcca acatatttat ccaatcttta ggacagaata tgttttctct ggaacttttc 9840
ctttctcttc cattctcact cccccacacc tctgacgggc cagctggcct gtctgtctga 9900
ctgactgcct ccctctcttt aatatatgct aatcaggatc cactcactgt caggatataa 9960
aacatgcctg ttccatgtct cactccatcc tgctccattt tagtaactat aagaacaagc 10020
atattatatc ccacgcgttc attcacccca tgggcaacag gatttcttaa accagttcta 10080
tgccacttgt catcttccct ctgtggcagg gcctctgccc aaaaggctgt tccaaacccc 10140
ctgcctcgtt gcctctaaat aactcctact aattcttcag acgtcagctc cactgccact 10200
ttggcaagac agccatccct gacctcaggg ctgagtcagt ctgccatcct atactctccc 10260
agagcctcct ggcactcctt cacagttctc tcatgatggt aaatttacat tgtatgtgta 10320
attgtttata aatacctatt tcttttttgt tttgttttgt tttgtttttg agatggagtg 10380
tcactctgta gcccaagctg gagtgtagtg gcacgatctc ggctcactgc aacctcccac 10440
ctcctgggct caagcgattc tgatgcctca gcctcccaag tagccaggac tacaggggca 10500
tgccatcgag cctggctaat tttttgtatt ttagtagaga cgggatttca ctatgttgcc 10560
cagggtggtc acaaactcct gagctcagga gatcctccta cctcggcctt tcaaactgct 10620
gggattacag gtgttagcca ctgtgcctgg ccaaatatct gtttttttca ccaggttacc 10680
aagttctgtg attgcaacaa tgatgtctgc tttgttcacc ataatttccc ctgtgtttaa 10740
cagtgtctgg cacatcaggt gttcagttaa atattttcta tgtgaaacaa taaatattgc 10800
taatggcagg tatttacatt agcttccatt ccttttcttc ttcctaggcc aggattatct 10860
caggatttca tgaagaggag aaatggaggt catttcctag agattaatgg ataatttgat 10920
tgtgagaaga aaattgttta aggagcaggg gtgggtatgg aggcaaatgt cacacactcc 10980
agaaggtaaa gtgacctggc tcttcccctc tcactccttg gaaagaagga aagctcatca 11040
gagccaggac cctggaaaag tgatgtgatg aagagaagga ggccctgaat accaccatct 11100
gaaggcattt ccaaggaagc cagggatgtg actctgtctc ggttgcagag atcaaaaggt 11160
tttgtgaagg aagcaatggc tctctagccc ccttctgggt ggattctggg gtagaaggat 11220
ccggttcctt tgataactgg ttagttatag ctatagggtg aagccaattg agtaacattg 11280
gtctgggctt ccagagacac aaggtcttct tgaaccataa gctctcagaa caatgcccca 11340
cctaggcaga gcagagaacc ctgaaccttt tgcctggaga gaacaaagaa gcgcacagct 11400
caggagccac cagacctggg actacccatt ggacccctaa tctccactgt tcttggcctg 11460
tctctctttc ccattagatt gtgagctctt tgagaacaga gactacctct tctccatcct 11520
tgtttcccta gcctagtaca ccatcaaata atgctgtgga aagaaaaagt aaaggaataa 11580
ataaataacc cttagataag tgggtcatcc accaatcctc aattcctgat tagtaaaatg 11640
agaataacaa tagctactaa caagagtatt gtgtagactg tatgacttaa tgaatgtgaa 11700

agcacccgta tggagtttaa cataaaatag ataataaaaa atacattatc ctctttgttt 11760
cggctttaat cacgcatcct cgctttaagt actccctgaa ctttagtgac cttcatattt 11820
tctagcctta acttgacagt gcatcagtgg ttaaagaatc ataaaatatc agagcaagag 11880
ggcaccaaaa gtggacattt gtcttgcttt gtcttgtttt attttcctgc tcttaatgta 11940
ttcccctctc acacacatca ttttcctgta ttgagccccc agtctcaatt ccatgactca 12000
ggaggagctg actccactca gactgtgggt ggggatggag tcagcccaat ggtggccaat 12060
cagagcactg atatacctct ttccacagtg attagtgacg tatggcatat ggcccatacc 12120
tgactcagtt ttggaacttg tgaaacaggg aattaccctt atgctgagag ggtgggatat 12180
ccaccaggag cttctggcag ccaatttgct tctagcaggg gagggctggt tgaataatga 12240
agccaatgcc caagaaacag tgctgagcgg ttgaggacag agagcttcag accaagcctt 12300
gatgatgctg agtccttaga tccagtaatg cctaaaacaa gtatcccttt ggacttttga 12360
gttttatgag ctaattaatt tcccttttta tttaagtcag tttgagttgg gttttctgtc 12420
ccttgcaacc aaattttggc taatacaaga tgttggagtt tgtttcatca cacctcttaa 12480
ttttacagat gaggaagaga tttgcaaatc atatgagcag tttttacaca caaccaattt 12540
tccaaataag tatattgctt caattccttt tatccttact gctgaccaga acaggggcag 12600
attgttctta taccgggata gggtcataga atattgattt tggtcacaga atgcctaatc 12660
tttctgtaac agagcaaatt ttaaaattca agtccctggg aatcagtttt attttgcatt 12720
acaatgtttt tatcccccaa gcagaactgc tgatggctgt ccttttcctt ccacacagta 12780
taatcagcag caattaatta taatcacaag gagacagcac aaatgaacag agcattaatt 12840
ggacagaagt aatcaaaatc tcccttagcc cacagttagt acctgtatgc aggaagatgt 12900
gaggaggcac atgcatgtgt acacacacac aaacatgcac acaccccata gtcaaccaga 12960
caataagaag aggatacagg atgagcatgc tctctcatca gcatggtgta gtagaaaaaa 13020
aaatcaagga ctttggaatc acaaagatcc caccactaac cagttgctta aacttagctt 13080
tgtcatttct aaaacaagga tacacagacc tacttcagag tggatgagaa tattaaataa 13140
gagtactgta tgtaaagcac tttttactgg tatgtagcag gaactctata ccttctagtt 13200
ctttttccct ccactgtggc tgctagagaa tgaagatcta ataagatcgg aaattttaga 13260
ccctcgaatg ttcttaatcc agcctaacca atttgattgt ctcccccaga ccctccactt 13320
aatcctatac cattcccctt tcccagtctc tgccccctcc cctacattcc tgcttcctct 13380
tgcctctttg cctctttccc ctcttggtta taaattctgg gagggcagct tggtatccct 13440
gttgcccctc acaggacctg acacacagta aacaatgagt ggacatttgt ttaataacta 13500
ttgatggagc ctgtgtctgt gcttatgttc tcctttcacc tgcagcatcc agcccttcta 13560
tcttggtctc agtcttagtc atccttcaag gcttagccta gaagctacca cctcctccat 13620
gaaatctttg aaacttgtcc ctaggacccc tatcccctcc tctgaactcg gaacctcttt 13680
catgacactt gcagcttttt atcttgtgtt ataagattta cgcaggtgtt gctttttcac 13740
tatgacctca agtttcttca ctcaatcatt caacctcttt gagttagtgt atgctggtaa 13800

ctatagatac tgaggcatca gaaagggtat gtgtccttga gatgctcagg ttcaaactag 13860
tacttgagca cagcaactgt gtctgactca tctttcaata ccccatgcct tgggccactg 13920
tgggtgatcc tcaaatattt gataatggca ataggttgcc taggagaagg catctgagct 13980
agaatactgg tgagtcttgt gaggttatag aaggccagtg agctccctca ggttggacac 14040
aaatgcccct gggctttctg aaggaaaata ttttacatta tggagagaaa tctggctcta 14100
gatcatgcca tatactatgt acttgaatca aaaaatccct gaataagctg tgacttggta 14160
cccagggata tacaccttca ataatttact tgaagaagct ctggcctaca gtcagtccca 14220
gctttgccac tggtttcaca gtgatgttga acaagtcatt tatcttctct gggtatcagt 14280
ttagctacct caactttatg caaccaacaa ttatggacac ctactgtgtt tctggcactg 14340
tgcctagtcc tggaaataca gaaataactc atatatgatt agcaattttg aaggcactaa 14400
tttagtaggc actataaaaa gggccatcaa agagaggtgt tgtaacaact ctacattgtg 14460
tagctctcat tcttcatttc tttctggata aattcccaag ctcctttacc tgacatcaag 14520
cttcttgtga tttgcccctg tctagtttct cctccatagt gaccctgcac cctggccata 14580
ctgcagtgct agtcattccc taatgctcca ttatcatcca tatctttgcc catgcagtat 14640
cactccaaaa ttctctacct gctaaattta tgatgatcct tcaacaaagt gaaagtacca 14700
taattgaggt gtacacagaa agagcatcac atttgtaaag gaagaagaat tgtaatatta 14760
tagctttttt taaagcttct tttattcttt tctttttcct tctttctctt tccttccttc 14820
cttccctcct tcccttccct tctcttcctt cctcttctct ctctcctttc tttcttttgt 14880
aaaaggaata gattggatta gattaactct accaacgctg taccactgga tgatcaatga 14940
tgtagtaatt atgtaatctt tgtactgggt ctttggaact cgtgatcaca ctgtctgacc 15000
tgtgcttccc tcatggtgtt tgaaaatgtc tcaagagcca attaacccat gtgatggttc 15060
attttatgtg tcaacttgac tgagatgccc agacatttgg tcaaacatta ttctggatat 15120
gtctgtgagg atatttctgg gtgagattaa aatttaagtg agtagactta gtcaaggaga 15180
ttgccctccc tgatatgggt gggcctcatc caatcagttg aagactgaat aaacaaagag 15240
gctgagaggg aatgcctcct gactacttga gctgggacat gggtgttttt tggcttttgg 15300
acaagaactg aaatacagtt cttcttgggt ctcaagtctg ttgactttca gattggaact 15360
tgcgccatca gctctcctgt ttctcaggcc ttcagcctca gacatatcag ctctcctggg 15420
acttcagctc attgactgca gatttgggac ttctcagcct ccataattgc ataagccaat 15480
tctttataat aaatgtcttt atgcatataa tctctttaca tatatatatg aaagcaggac 15540
caataggtga tatatagatg tagatatgga tatagacata gacttagata cagatattga 15600
tattgacata gatattctat tggttctgtt tctctggaga accctgacta atgcaaccat 15660
attgcagacc ttgggcttgg cttcctgaag aatgttcctc tcaatcgtga gtaagatgtc 15720
aagtctcctc aaccacaccc aacttttcat ttcctatatt cttggctctg ctgttgatta 15780
ttgttttctc cctagaagaa agatccccta agatatggga aagaacgatt ttgtagaatc 15840

ctgctacatg gagctaacag gacccttaga gataattagg attcttattt cagagcagtg 15900
aggtgcctgt ccatggtttt gttctttgta gaaacttgtg gaatgccttc ctgttcctcc 15960
aactctctct tctcctctta ttgaaggctt aacctccctt ttagacccag ctcaaaatct 16020
gctccattta gaaggtcttc ctagattccc caagtttgaa ttcattgctc tgttttctgt 16080
atccccacag agttctgttc atatctttta cttagcttta atttgtttta taattggaga 16140
tagatcaaga ggtaagttag gcagatgggg tacaaggtat tatttgtagt gggataataa 16200
agcatcagta aaaacagaac aagttggaga aaatgatatt tatcagaatt cttcaccaat 16260
atgtaactgg aagaaaattt gagactcaga gagataagga acttctgctc aaatcattca 16320
gctagaaagt agctgagctt ggattcgaat ccaagtctga ctgcaagagc catgctcttg 16380
ttggttattc atgcaataat ttgtgtaaaa tgttaactgt gtgccaggta ccatgctaga 16440
cactgggatg caaaagttca agcctggttg ttcagggtta tggcaggtag tggcagaata 16500
caaccaaatt ccttgtgtta gtctttggtc tctaggtctg agactcttct gtcccactgg 16560
gtgatttcac cactaggata ctccacagcc agttccacag gcttctttct ttccaaaagc 16620
agacaagaca ctttcttaga ataacccctt tgcctgcccc aatatctttg gctgtgccca 16680
ctgtggaaag agagccaggc tgctgccaag ttcattaata atttcggctt tctggaggaa 16740
ggagttggat tgctttgctc ttcaaatatc aaaacatcca attaacacag ctaattagca 16800
tcactagcag cagtcaaaag aatggcaatt tagatgcaga atttcaaagg tttcctagtt 16860
gtaggccaag gcaacccatc tcttgctaat gacaagagtt actcacttag gcaattctat 16920
agcagccatc aattaaccac tgggcagaga ctggcctcat ccctgagtca tttgtctaat 16980
cagaactgtg aatagagaaa aagaggcaga atgaaaatgg agagaggaag agagggagag 17040
agggagatga cagaaatgga gtgagggggt ctgagagagg agagagagag aattaaaaga 17100
gataaacaga gaggaggaga tacagagagt aacaacacac aaagacaaat tctcagagaa 17160
agtgacagag agagagaatt agaaggaaaa acaaacagaa agggtgactt acagatagat 17220
agacagacaa tcaggtaggt acatagaaaa atacatttct taaaacatac agaaggacag 17280
gacaaaaagc ttctagccac cagtagcttt ggaaaggtcg gtgatcactg ggaagcctca 17340
gcattctgat ctgtgaagtc aggcctagct gtaaaagttt aaatcaaaag cttggctgct 17400
gtagtggaat tcaaaagagg aagagaccag gagactcaaa ccagaacttt tctcttccct 17460
tctcacactg acaactggat ttcctatgag ccttgatttg tctgtcgaaa gtcaaatatg 17520
cagccagtgc tcccacattt gtgtaccttc tcctgtaaac gcagtaagct caatgatcgg 17580
gcctatgatc agccaggggg gtattgtttt ctccctcaag gaaggaaaca ccaatggatc 17640
tcctactgat tccacctcag acatgacttt cccatctctc cttctctcca ttgcacagcc 17700
tctgcccctg ttcaggcctc atggtcactt ctgtaggtta ctgcagcagc ctctggtctc 17760
ttcttgttgc tatccaatcc gttcttatca ctgctgccaa agccatatgc ctaagatgca 17820
gagctgactg tgtccctttc tgacttaaac cttttcaaca gttctgtact ttccctggat 17880
caagtccaaa cccattaatc atggtagtca gggcctctgt ctaggctcct gctgccttct 17940

cctgacccat ctctcccttg tccccattcc tcactgacgg ccccaccaca ctagatgaca 18000
tgggggctcc tggatgctcc acactgtctt tacctctggg ctcctcctca tactacttgg 18060
cccctgcaat acctcaaatc cctgttcatc tctgtgcctt tctatgtcta tgcttttgtt 18120
tccatacttc ccctgcctat caacttgttc tttgaatgac ttcatctctt aaccagcatg 18180
tataccagta tcacaaattc aaatgtcttc agaggatgga ttcataatgt aaatatgtgc 18240
aatgataagt atgaaacaat agagggagca gtgacaagtg agacagtaaa tgccacgctc 18300
aaaaacctca aactcaaaac tcaaccaacc aaccaaccaa ctcaccagac aacaaaacat 18360
tttccttgcc atacccacca tctacaggac ggagtcagcc atgggccacc agttgatgac 18420
tgttgatggc cctccatctg taacctgact ctaagcttgg cttggcccac agggtcttct 18480
gcgctcagac tctactttca actcttgcta tctccatgca tctggctccc tgttcagggg 18540
aattttcaca gtctgtgctg tctcctcccc tttggacctc acagggatgt tcactgtgtg 18600
taaatagctg aaaagaacac taacatttac agtgagtaag ccaatcccat catacacctg 18660
gcccaggcaa gacgttatta atcagtatct taatgaagcc taattacagc agacgatgcc 18720
tcctaccagg taaggcaatg gttacagatg ggagcaaacc aggaaatcac tgccctctcc 18780
caagagcttg atccactgca gcactggagg gttaactctg ggggtctgaa tggctggact 18840
cccagagtcc agccgagggc cctattagca gtaagaaatg acctctctcc ctaccgccaa 18900
cataaacgca gatgggacag gaagtggagc tgggcttcta gttggtcagt gaccaccttc 18960
tcttccagct gacattctgt gtatttccag acatgactgg gtctccttgt ttctagtatc 19020
tacccctgag tcaggccttg gtaaggcatt atagtcttca aaacatgatc tttggttcct 19080
caacttcttg tttttttctt tattcctcca tttacttctc ttttctctct acagcactca 19140
agcatccagg gtccctccca atcccacctt ctattgcagt tcagtgggtt aatagtgcag 19200
gctgtggaat taatcagctc taaggtgaat tctgaccctc cacatctacc agctgtatga 19260
ccttgggaaa gttattttac tctctggact tcagtgtctc ataagttaaa tgagaacaat 19320
aatctctgtc ttgtcttctt cacatagttg ctttgaggtt gaattgggga agccgtctag 19380
ggcagtacct gccacataac tgactgccct cccttaatcc tgcaacttca ctgagaagtc 19440
attagggagg agaagttctt ggcccttgct aattgctcct ttcttgactg gggaatgaag 19500
tatgggcttc acaattctct cgaaatcata ctaaggatat cctagaccac tggtctagga 19560
taactctagc tccttctgca tataccatcc actcctgtta atacaataat ttcctcatgt 19620
tactctcctg ctcaagaacc atctctggct ccctgttgcc tacgatctaa aactcgaaat 19680
tagtactatg gcatttaagg ctttccatga tctgacccca tataactttt tcaactttag 19740
ttcctaagcc tcccttgtat gaactcagca ttcaggccaa tttcttcaca tgctcagttc 19800
tgtcaccgag cctttattca tcatgaccca ctcttcacaa tgttttccct gttttgtaag 19860
ttccttcact agactgtgag ggtagatgtt attttttaaa atttcaatat caatagagct 19920
gataaaatga aactctcaat aaatagttgt taaactgggc taaatgccat cacttcaaat 19980

acttcttctc ttgtaagttc cagaacagat ccttttggat ggtctaatgt agtcaagttc 20040

agactctgaa accaaaagac tttgggtcaa acccaagctc taccactgtg atggttaact 20100

gtacgtgtca atttggctgg gccatgatgc tcaaataagt tgtcaaacat tattctgg 20158

<210> 2
<211> 8435
<212> DNA
<213> Homo sapiens

<220>
<221> misc_feature
<222> (1)..(8435)
<223> chromosome 2 nucleotides 132999435 to 133007869

<400> 2
agggatttta taagtaatcc taggtaaggg atcatttatg tttgctaatg ctccctgact 60

ttcgtcatca tttgcggtga tgcatgcacg tatctcaact gcctgaggac agagtgaatc 120

ccggcctgat ttacctttta tgctacgcag aattcagttt gatgctttcc cacaatcatg 180

actcaataag gttgactgag gagtgggtag ggttacgaac actgtgatcg tttctgcctg 240

tcttactaca aggtggagga atgggacttc cctttggccg tcagacatac agcaagtgct 300

cactgtgtgc caggcacagg actagctggc agtggaggca caaatatgtt ccagacatga 360

tctccaaggc cagttatagg caaaggtcac gtgtgtgaca aggaagacat gtcccttcat 420

gttgtttatt cattgcctga gcagcagcta gatggggaga gctgctgggg cccaaaaaag 480

ggggtggggg agcattccct gagtgcctga gataaatgct ccatgaaagt ggacacggaa 540

catctcacaa gtgtcttgga agcaacctcc ttcgtgacat catgacaaat ggcttttcta 600

ggaaaacaga ccaaatgtga gcaaaaaggt ctgtgatccc agaccccaaa agttctgaaa 660

atgggatatt tactataact tacttggtgg caaaacctgg accaacctga tatgaggcta 720

tttatggtat tcatcctcct gagtgtgaaa atccatgcct ttgctgcaga aatatgtgtg 780

tgtaatgaca ggacacttcc ccagacctta ttggagatgt ttttagcatg cggtagatgc 840

actgtatcac cttactaaaa tctgggaaat tttgaattcc tacagatatt tgaccccaaa 900

agttttagcc ataaggattg cagacctgta gaagctgcag cagtgaggga ctgccttcca 960

gtcaatcctg cctcccacta agtttttctt cctgtaggct cccaggcaaa tggttttctt 1020

ctacctgggg aaggccagtc ccagtatctt caaagtgcag gcagggctca gctcaacctg 1080

cagccaaacc ctagtgggat cttttactta attcagaagt atgtttttct taatgtgaga 1140

aacacagctc agcatgagct tacaggaaag gtgggcttag tgttgcctgc aggcatcttc 1200

tgggccttat tggatggttg caccatcctg ctgggcttaa taccccacac tgcccatccc 1260

ccggagcacc tgcatactct ggactttctc tgctgccttt ccatgtccgt ggtgtgtagg 1320

gaaaatgtcc cattttctca gggccctgat agctcctaag ctcctatatt aactgtgctc 1380

cagtattaat aaatgtgtag gtactaggtt ttatatttct agataccatt tgcactttag 1440

caaatgtact ttatgcttta acccataagg tgactaacag agggtgttca aataatgtgc 1500

tagacccttt ttttaaaatt aattctacct actttgtttt ttaaattttt attttatttt 1560
actttaagtt gtgggataca tgtgcagaat gtgcaggttt attacctaag tatacatgtg 1620
ccatggtggt ttgctgcatc tatcaacccg tcatctaggt tttaagcccc gcatggatta 1680
ggtatttgtc ctaatgctct ccttcccctc gctccccacc caccgtcagg ccctggtgag 1740
tctctgtgtc catgtgttct cattgttcac ctcccactta ggagttagaa cgtgctatgt 1800
ttggttttct attcctgtgt tagtttgctg aggatgatgg cttccagctt catccatgtc 1860
cctgcaaagg acatgttctc attcttttct atggctacat agtattccat ggtgtatatg 1920
taccacattt tctttatcca gtctatcact gatgagtatt tgggttggtt ctttgtcttt 1980
gctattgtaa atagtgctgc agtaaacata tatgtgcatg tatctttata gtagaatgat 2040
ttacgttcgt ttgggtatat acccagtaat ggcatggctg ggtcaaatgg tgtttgtagt 2100
tctagatctt tgaagaattg ccacactgtc ttccacaatg gttgaactaa tttacattcc 2160
taccaacagt ataaaagcat tccaatttct ccacagcatt gccagcatct attgtttctt 2220
gactttttaa taatcaccat tctgactgct gtgagatggt atctcattgt ggttttgatt 2280
tgcatttctc taatgatcag tgacgttgag ctttttttgt atgtttgttg gctacataaa 2340
tgtcttcttt tgagaagtgt ctgttcatat cctactccca ctttttatgg ggttgttttt 2400
tttcttgtaa attggtttaa gttccctgta gattctggat attaaacctt tgtcagatga 2460
gtagattgca aaacttttct tcactctgat gatagttttt tttgttttgt tttgttttgc 2520
tgtgcagaag ctctttagtt taattagatc ccatttgtct gttttgcttt tgttaccatt 2580
gcttttggtg ttttagtcat gaagtctttg cccatgcctg tgtcctgaat ggcattgcct 2640
aggttttctt ctagggtttt tatggttttg ggttttacat ttaagtcttt aatccatctt 2700
gagttaattt ttgtataaga tttaaggaag ggattcagta tcagttttct tcacatggct 2760
agtgagtttt cccagtacca cttattaaat ggggaatcct ttctccattg cttgtttttg 2820
tcaggtttgt tgaagatcag atggttgtgg atatgtggtg ttatttctga gttctctgct 2880
ctgttccatt ggtctatatg tcttttttgg tacaagtacc atgctgttct ggttgctata 2940
gccttgtgat atagtttgaa cccaggtagc atgatgcctc cagctttgtt ctttttgctt 3000
aggattgtct tggctataca gggtctttta tggttccata tgaaatttaa aatagttttt 3060
tctaattctg tgaagaatgt caatggtagt ttgatgggaa gagcatggaa tctctaaatt 3120
actttgggca atatggccat tttcacacta ttgattcttc ctatctatga ggatggaatg 3180
ttttccattg tttttccatt tccatgcact gattttcttg aacagtggtt tgcagttctc 3240
cttgaagagg tccttcatat tccttatagg ctgtattcct aggtattctg ttctctttgt 3300
agtaattgtg aatcagaggt cattcatgat ttggttctct acttgtctat tgttggtata 3360
taggaatgct tgtgattttt gcacattgat tttatattct gaaactttgc tgaagttttt 3420
tatcagctta aggagttttg ggctgagaca atagggtttt ctaaacatag aatcatgtca 3480
aatcaatcct gggggtggag ccaagatgac tgaataggaa cagctccagt ctacagctcc 3540
cagcatgagc gatgcagaag acaggtgatt tctgcatttc caactgaggt accaggttca 3600

tctcactggg gagtgccaga cagtgggtgc aggacagtgg gtgcagcgca ctgtgcgtga 3660
gctgaagcag gcctcaccca ggaagcacaa ggggtcaggg aattcccttt cctagtcaaa 3720
gaaaggggtg atagacagca cctggaaaat cgagtcactc ccaccctaat actgcgcttt 3780
tccaacaggc ttaacaaaca gcacaccaga ttatatccca cacctggctc ggagggtcct 3840
atgcacatgt agcctcgctc attgctagca cagcagtctg agatcaaact gcaaggcggc 3900
agcgaggctg gggagggggc gcccgccatt gccgaggctt gagtaggtaa acaaagtggc 3960
caggaagctc gaactgggtg gagcccacca cagctcaagg aggcctgcct gcctctgtag 4020
gttccacctc tgggggcagg gcacagacaa acaaaaggca gcagtaacct ctgcagactt 4080
aaatgtcgct gtctgacagc tttgaaaaga gtagtggttc tcccagcacg cagcttgaga 4140
tctgagaatg ggcggactgc ctcctcaagt gggtccctga ccactgagta gcctaagtgg 4200
gaggcatccc ccagtagggg cggactgaca cctcacacgg ccgggtactc ctctgagaca 4260
aaacttccag aggaacaatc aggcagcagc atttgcggtt caccaatatc ggctgttctg 4320
cagccaccgc tgctgatacc caggcaaata gggtctggag tggacctcca gcaacctcca 4380
acagacctgc agctgagggt cctgactgtt agaaggaaag ctagcaaaca gaaaggacat 4440
ccacaccaaa accccatctg tatgtcacca tcatcaaaga ccaaaggtag ataaaaccac 4500
aaagatgggg aaaaaacaga gcagaaaaac cggaaactct aaaaatcaga gcgcctctcc 4560
tcctccaaag gaacacagct cctcaccagc aatggaacaa agctggacgg agaatgactt 4620
tgacgagttg agagaagaag ccttcagaag atcaacctac tctgagctaa aggaggaagt 4680
tcgaaccaat ggcaaagaag ttaaaaacct taaaaaaaaa aattagacga atggctaact 4740
agaataacca atgcagagaa gtccttaaag gacctgatgg agctgaaaac catggcacaa 4800
gaacgatgtg acaaatgcac aagcctcagt agctgatgag atcaactgga agaaagggta 4860
tcagtgatgg aagatgaaat gaatgaaatg aagagagaag agaagtttag agaaaaaaga 4920
ataaaaagaa atgaacaaag cctccaagaa atatgggact acgtgaaaag actaaatcta 4980
catcagattg gtgtacctga aagtgatggg gagaatggaa ccaagttgga aaacactctg 5040
caggttatta tccaggagaa cttccccaat ctagcaaggc aggccaacat tcaaattcag 5100
gaaatacaga gaatgccaca aagatactcc ttgagaagac caactccaag acacataatt 5160
ttcagtcacc aaagttgaaa tgaaggaaaa aatgttaagg gcagccagag agaaaggtcg 5220
ggttacccac aaagggaagc ccatcagact aacatctgat ctcttggcag aaactctaca 5280
agccagaaga gagtgggggc tcatattcaa cattcttaaa gaaaagaatt tccaacccag 5340
aatttcatat ccagccaaac taagcttcat aagtgaagga gaaataaaat cctttacaga 5400
caagcaaatg ctgagagatt ttgtcaccac caggcctgcc ctaaaagagc tcctgaagga 5460
agcactaaac atggaaagga acaacttgta ccagccactg caaaaacatg ccaaattgta 5520
aagaccatca aggctaggaa gaaactgcat caactaacga gcaaaataac cagctaacat 5580
cataatgaca ggatcagatt cacacataac aatattaacc ttaaatgtaa atgggttaaa 5640

tgctccaatt aaaagacaca gactggcaaa ttggataaag agtcaagacc catcagtgtg 5700
ctgtgttcag gaaacccatc tcacctgcag agacacatat aggctcaaaa taaagggatg 5760
gaggaatatc taccaagcaa atggaaaaca aaaaaaggca ggggttgcaa tcctagtctc 5820
agataaaaca gactttaaac caacaaagat caaaagagac aaagaaggcc attacataat 5880
ggtaaaggga tcaattcaac aagaagagct aactatccta aatatatatg cacccgatac 5940
aggagcatcc agattcatag agcaagtcct tagtgacctg caaagagact tagactccca 6000
cacaataata atgggagact ttaacatccc actgtcaaca ttagacagat caacgagaca 6060
gaaagttaac aaggatatcc aggaattgaa ctcagctctg caccaagtgg acctaataga 6120
caactacaga actctccacc ccaaatcaac agaatctaca ttctttttag caccacacct 6180
attccaaaat tgaccacgta gttggaagta aagctctcct cagcaaatgt aaaagaacag 6240
aaattataac aaactatctc tcagaccaca gtgcaatcaa actagaactc aggattaaga 6300
aactcactca aaactgctca actacatgga aactgaacaa cctgctcctg aatgactact 6360
ggggacataa caaaatgaag gcagaaataa agatgttctt tgaaaccaat gagaacaaag 6420
acacaatata ccagaatctc tgggacacat tcaaagcagt gtgtagaggg aaatttatag 6480
cactaaatgc ccacaagaga aagcaggaaa gatataaaat tgacaccata acatcacaat 6540
taaaagaact agagaagcaa gagcaaacac attcaaaaac tagcagaagg caagaagtaa 6600
ctaagatcag agcagaactg aaggagatag agacacaaaa aacccttcaa aaaatcaatg 6660
aatccaggag ctggtttttt gaaagatcaa caaaattgat agaccactag caagactaag 6720
aagaaaagag agaagaatca aatagacaca ataaaaaatg acaaagggga tatcaccacc 6780
aatcccacag aaatacaaac taccatcaga gaatactata aacagctcta tgcaaataaa 6840
ctagaaaatc tagaagaaat ggataatttc tcgacacata caccctccca agactaaacc 6900
aggaagaagt tgaatctctg gatagaccaa caacaggctc tgaaattgtg gcaataatta 6960
atagcttacc aacccaaaaa aagtccagga ccagatggat tcacagccga attctaccag 7020
aggtacaagg aggaagtggt accattcctt ctgaaactat tccaatcaat agaaaaagag 7080
ggaatcctcc ctaactcatt ttatgaggcc agcatcatcc tgataccaaa gcctggcaga 7140
gacacaacca aaaaagagaa ttttagacca atatccttga tgaacattga tgcaaacatc 7200
ctcaataaaa tactggcaaa ccaaatccag cagcacgtca aaaagcttat ccaccatgat 7260
caagtgggct tcatccctgg gatgcaaggc tgtttcaaca tacgaaaatc aataaacata 7320
atccggcata taaacagaac caaagacaaa aaacacttga ttatctcaat agatgcagaa 7380
aaggcctttg acaaaattca acaacccttc atgctaaaaa ccctcaataa attaggtatt 7440
gatgggacac atctcaaaat aataagagct atctatgaca aacccacagc caatgtcata 7500
ttgaatggac aagaactgga agcattccct ttgaaaactg gcacaagaca gggatgccct 7560
ctctcaccac tcctattcaa cgtagtgttg gaagttctgg ccagggcaat caggcaggag 7620
aaggaaataa agggcattca attaggaaaa gaggaagtca aattgtccct gtttgcagat 7680
gacatgattg tatatctaga aaaccccatc gtctcagccc aaaatctcct taagctgata 7740

aacaactcag cgaagtccca ggacgcaaaa tcaatgtgca aaaatcagaa gcattcttat 7800

acaccaacaa cagacaaaca gagagccaaa tcatgagtga actcccattc acaattgctt 7860

caaagagaat aaaattccta ggaatccaac ttacaaggga tgtgaaggat cccttcaagg 7920

agagctacaa accactgctc aatgaaataa aagaggatac aaacaaatgg aagaacattc 7980

catgctcatg ggtaggaaga atcaatatcg tgaaaatggc catactgccc caggtaattt 8040

atagattcaa tgtcatcccc atcaagctac caatgacttt cttcacagaa ttggaaaaaa 8100

ctactttaaa gttcatatgg aaccaaaaaa gagcccgcat tgccaagtca atcctaagcg 8160

aaaagaacaa agctggaggc atcccactac ctgacttcaa actatattac aaggctacag 8220

taaccaaaac agcatggtac tggtaccaca acagagatat agaccaatgg aacagaacag 8280

agctgttaga aataatgctg catatctaca actatctgat ctttgacaaa cctgagaaaa 8340

gcaatgggaa aaggattccc tatttaataa atggtgctgg gaaaactggc tagccatatg 8400

tagaaagctg aaactggatc ccttccttac acctt 8435

<210> 3
<211> 1742
<212> DNA
<213> Homo sapiens

<220>
<221> misc_feature
<222> (1)..(1742)
<223> chromosome 2 nucleotides 159101071 to 159102812

<400> 3
aataagtttt atgaacatgc taatttattg caaccccaaa cataatgtca aggcacttgg 60

ttaaggaatt gcaaagtagt atcttataaa attaacctga attttgagag gaaaagtctg 120

ggttcttcag tagagtatat ctaagccagt aaaatacctc acacatactc cccttgggca 180

attcatggta agtggtagga aaggaaatgg caagcaacca atagcagtct tgttaacaaa 240

ccgttagtct attaaatatc tgaaattgga catagagtgt cataaaacag tgaaatcttt 300

cttcactggg ctatgctttc cctagaatcc tccagtgcca gaagctagct gttccttgtt 360

ctttggcata atgctagaat ttacaatgtc ttttatgaag tgataacctc tcacagtggt 420

atcagtcatg tggatgcgta cctgcgaagg tacagaaaaa tccttgctcc ttgacagaaa 480

gaatctttgc tacttgtgtt caaacgctgc gtttttgatg aaacataccc agcctgaagt 540

caaatctctc tcctgatctc ttttagcact ttatactact gccatgactc ttaatagtat 600

gcttctttgg atttatataa tcagttacct gaggatagag ccgtgtttta ggccttgttt 660

tatacctgtg ttacaggttg ctcctctccc agcaccttgg atatattaga cctgcaataa 720

atggttgttc gagcagggaa tgaatagata atacttactg tcgattactt tgtcaccaac 780

ttgcggtttt attaagataa cagaacctgt agccacgttc acagaaacat tgtgagcaag 840

agagtctcac agcacctaga aaacttgtca ctcagccaaa ccttcttaag acaatttgaa 900

ttgcactata tccatattga taaaggattt aaattcaatc tttgcatagt acaaaataaa 960

attttatttc atatctatgg tcaaataatg acataggtgt cataactttc atctcccgtc 1020

tgtccaattt agtcgaaagt taagtttagg gaataaaagg atgtggttat cttggtgatg 1080

tcacaaaata tccaccagtg ttatgtgtat caggtgatac gggtaactac taaagaaatg 1140

ttaagaatta tgctttaaga gaaataaaaa gtcaatccat gggaataaca aagtaatgtg 1200

attctgaaat tacaggaagt atcagaactt cagccaccac ctggctgtaa ttaggatcat 1260

cctttagcag attctactgt tcagctcaac tcctaataag aatccaggag tccatgatga 1320

atacagatgt aggaaaggag gaatctccta atagttttca aaaatgtctg acaaacttga 1380

tactggttag tgcctagctt actctcttag ttgtaataac tagataaata cagcgctggg 1440

catggtggtt catgcctgta atcctagcac tttgggaggc tgaggtgggc agatggcttg 1500

agctcaggag ttcaaggcca gcctgggcaa catgacaaaa accctgtctc tacaaaaaat 1560

acaaaaaatt ggccaggtgt gatggtgtgc gcctgtggtc ccagctactc aggagaatca 1620

cttgagtctg ggaggtcaag gctgcagtaa gccgtgatca tgccactgca ctccagcctg 1680

ggtgacagag caagaacctg tctccaaaaa aagaaagaaa aatgtggtgc cctcaaagga 1740

tc 1742

<210> 4
<211> 8013
<212> DNA
<213> Homo sapiens

<220>
<221> misc_feature
<222> (1)..(8013)
<223> chromosome 3 nucleotides 54983458 to 54991470

<400> 4
actttgtttg aggtcacacc gacagcagat gtcatagtct gtgattctca aactggtgtc 60

ttagcctttg ggctagtacc ccttccagtg tacacaaggt tagtactgcc atttgtcact 120

cccaggcacc ctgcccttcc aatctgtgac tgcccaattc ccatggcctg ggtttgggct 180

ttaacttcag gtatctacct gatccacctc tgtgactaag aatcagactt ctctgagtaa 240

tacggagcca tccgaatagt tgcaggaaga tgcctttttt aaaacatcaa cttccctctg 300

tgctctccac cctgcccttt attgctgcct ttttaaaaac aacctcatcc ccagatgttt 360

tgccaaaggt agttgtggag taggtgctta caagtgtctg tttgaactcc taatttcttc 420

aagctcttca acttgttagt gcacccttgg ggcagggtga cagatagaca tcttctaaga 480

tgttgggctt aatttgggag gaaagggtag cacaagcgag ggaaagctga gaaaatagtt 540

cccatttcct tttattggac aggccatatg gagaagggaa ttgaatatta tttattgtaa 600

agtgatatgg aaaaaacagt tgatcagact ggactctagc taattctgtc ctaatttatc 660

cataaaccca gcactctgac cccctaactc cagtgggctc actccactgc actctacttc 720

tcaataataa tttaatccaa gtggtcattg acagggaggt ctgtttagaa agtcatcttg 780

tcactcaatt tgccttttaa tgtttacagt tagacaaagt aacagcagtg tctataaaga 840

aaatgtggct gggcacgatg gctcacacct gtaatcccag cactttggga ggccaaggca 900
ggcagatcac ttgaggtcag gagtttgaga gcagcctggc caacatggtg aaatcccgtc 960
tttactaaaa atacaaaaat tagctaggca ttggggtgtg cacctgtagt ctcagctact 1020
caggaggctg aagcaggaga atcacctgaa cctgggaggc agaggttgca gtgagctaac 1080
atctcatgcc actgcactcc agcctgggca acagagtgag acttggtctc caaaaaaaaa 1140
aaaaaaaaaa aaaaaaacaa cgtggccccc tttggtggaa atggaagctt aggcatgttg 1200
tccttcagga tccctggcca gttctccgcc ttgttctctt cccatcattg tttgctagga 1260
tgtagagcaa tcttaccaaa atgagtggtt cattaagtat atttttagac tctagcctat 1320
aatggacata tttttttctt ccatagatta ttctgtagca agttgtggag atggatagaa 1380
aaaatccccc taaactatgt cttggttcag atattagctc aagaattgct atgcccaggg 1440
agtctaggaa cctataattg gggttgatac cacttgacca aactgaccca gacgtcagtc 1500
acttttattg tttttacttg ctttcagcca caataataac accccagaca cttggtacat 1560
tgatttttct gccatttgtt gtggtttcct ttggtagcag gtgtcagaca agatctgtct 1620
taggtatcca tagagattag atataaatat aggtatagat atagatacac acacacacac 1680
acacacacac acacacacac acattatata aagggattgc ttttttcaaa caagcaaagc 1740
cttccatgag caggcaaaat ccctagaccc tactgtgtcc tgctgtcttt tagaaagtaa 1800
gggtgggggt cagccagtag aaagagtgtg ggctttggag tcccatagca atggactcaa 1860
gtctcacttg tgtgtgtgtg accttggaga agacaacatc tcacaacagt ccccagagtg 1920
tgatgttccc cttcctgttg tggggtgggg ggagggggga gagatagcat tgggagatat 1980
acctaatgct agatgacgag ttagtgggtt gcagtgcacc agcatgtcac atgtatacat 2040
atgtaactaa cctgcacatt gtgcacatgt accctaaaac ttaaagtata ataataataa 2100
ttaaaaaaag acaacatctc agttttctct tctttccaag agaaaaattc cctgcttgat 2160
gctgtgaaaa gtgaggtttt catgagtcga tgtcctcctg tagctgccat cctccttatt 2220
cttcttctta cattcttatt gttactatta ttagtgacag tcatgtttta tgaaaaactt 2280
ctttgtgtga tagttttcat ctggtattct cccaaggagg ccactcccct atttttgcag 2340
cttggttttc caagttctga gttaaagcag tgtttgcccc tgctccagag ctctcagctc 2400
tgttccttcc acaccagaag tgggagtggg agggagcagg gcagacgcct gagaagtgag 2460
tcttctgatc tcccttctac tggtgctggc tgttataagg agcattataa ggagggcctc 2520
gttctaagaa gaaaggtcag aaaaagaacc aaatgtttac agcaaataat aaaaagttat 2580
tttgaatatt tttctctaaa taaagtggaa agtatgaaac tgtgtttatt ttttaaataa 2640
atttaggtta tatttgcaat tgtactctaa ttttccaaag aaacaaatgg ggtaaggggc 2700
caggagttta gtgtttctga taagatgata aaatagcaca atatgaaatc cttcacttta 2760
tattttgatt tttctcagaa tgaccacaaa aagggtaaaa tatagtaaag agtttgatca 2820
attttcctag ggtacttttc ttagtccttt gctgttgaaa ttattctctt tggaagtaca 2880

gctcatgttt tatttgtagc aggtattttt tcatctttct gcctctgaca gattctaatg 2940
tgccaatggc ttcctgtgcg attggagctg tttaacgtta ctttcatgga cttcatgaaa 3000
tcttgcctct ttgtcaagat tttcaattga ctttataatc tatttgcctt gtacctgtat 3060
tgcattgctc aatatttaca gtattaggca aacagaaaga tattgaccaa agatgtaggt 3120
ctaatagcaa gagtactaat tagttagaga tgttggagta gggactaagt tttaactagg 3180
agtagaggtt atgatgattt gtggccttat atgcagctgt ggggatttga atattgacag 3240
tatctgaata tggaggccac ttctgtactc aacatgtatt tactgactca acagaggtta 3300
gccgcccctt gcttcattgt ctgtatctgc agaatgagca tcatgatgtt acctgaaagg 3360
gatcccaatc cagaccccaa gagagggttc ttggacctca tacaagaaag aattcatgga 3420
aagtccacag agtaaactga aaacaagttt attaagaaca taaagcaata aaagaatggg 3480
tgttccatag gcagagcagc ctcgagggct gctggttacc catttttatc gttatttctt 3540
gatgatatgc taaacaatgg gtggattatt catgcctctc ctttttagac cgtatagggt 3600
atcttcctga cgttgccatg gcatttgtaa actgtcatgg cactggtggg agtgtagcag 3660
tgaggatgat cagagtttac tctcatcacc atcttggttt tggtggtttt agccagcttc 3720
tttactgcat cctgtttatc agcaagatct ttatgacctg tatcttgtgc tgacctccta 3780
actcatcctg tgacacagaa tgccttaacc gtctgggaat gcagcccaat aggtctcagc 3840
ctcattttac ccagccccta ttcaagatgg agttgctctg gttcaaacac ctctaacatt 3900
tcccccgtcc cttttataag aaaaccctta atcctaaggg ttgcagaggg ataaagatgc 3960
atcttctgta acttcttcaa gctgataggg gcaatgccat tcctgcctaa ctattgggtc 4020
tcttgcattc agggtagata gagaggagct cagtcagaaa gcattggtat ggcaagggcc 4080
gttcatgact ccgagttctg acaaaagatg atatctagaa gattaaaaag tgctcagtgt 4140
aagaaaacat ttagtaagct tgtccagcat ttctacacaa agagtacaat agcaacgtat 4200
tccaaaacag taaaacaaaa taagtaaaat tatcccaagt aaactaaata aggcgttcca 4260
tgaactgggc aactattgga accaagctga tatgaggttg ctagctgatt ccaatacatg 4320
cccagaatta gaatattgat ccagattttt acattaccca tccctgttgt ttcttctgag 4380
cagcagtcaa gagatcactg gttggttcac aggaataaac agggttggcc taaattacag 4440
aaaaaaacct taaaatcaac tgatgagact agaacttaat aacaggttat accacagttc 4500
ttgaaacata attgttctct agtttcccat ttttgctgga gacaaatcat agtaagacta 4560
atttgcttta ttatacttgg cctgattatt tgtatacagt gcagcaagaa taattatttt 4620
tcacataggc ttttaaattt gtctttgatg aaattttgtt ccatagaggg aatctcagat 4680
aagacttttt tttaaagcca agcctagcca tgggtttgta ccccagatat ctatgagttg 4740
agtaaattct tctcctcttg aggtcccaaa ataacttggg gctcctgggc ctttcagaag 4800
gtgacattct ttacttacca caggtcagga accctgtaca gggactgtgt agacaaggta 4860
tgaggctagt ttttttcaag gggctcttct tggctctcta agtcaagttt gattccttaa 4920
aggtaaacac accattccag tcaaagcctt ggtaaaataa ccagtttctc cagttgtatc 4980

ctattacaat agaaaagaga ttcttactgc atttatgcaa ataactatat tgccataagt 5040
taagatactc tcaaatagtt tccaaattct ggaggaacca ggtagagaga aacaaatatt 5100
ctctaaattt tttcgcagaa gtatagttta ctcaattgtt aaaagctgta aatagctcaa 5160
aagaaaaatt ttcttggccc tggaaaacaa aaaggatcag caatgtttta agcaaaaaag 5220
taaaagaaaa atattacttc agttttttat tagttcagtt catgcagttg actcctattc 5280
tgcttgaaca gaactcctgt tctgtcatga acatttcagc tctctgtgag agtcctgaaa 5340
ctttcttcct ctattctaat gtcacaatct ccaacattat cagaaatctg cattcaatag 5400
cactgtcaga gttctatagt tgattataaa ccatctcttg aagaagatta aaacaagaca 5460
acagttgtct gtggatgata aaaagtctta ggacagccac tattaaagcc acaattgata 5520
aagaaatttg gttacttctg tggcatccaa atcttacata acaattataa ctattaataa 5580
catacagtaa gccatgttag aattatagga cttcttataa ttttggaaca cataccaata 5640
gcacaactat ataaatatat cccaaagaaa gccaaacact gtttcacatt tgataatgct 5700
tcctgttatg atttttatac caaataagcc aagttttacc tttgcattag tgtactatta 5760
atgttaaatc caattcttaa taaaacctta tagacaaatg tattcagtct taatcagttt 5820
gaccacgagg taagattttc ataaaccttg tataaccctt tacatttttt tgtgaaagag 5880
cagatcagtg ttctaagaaa aacctgttgt gcttttattc cagtgtttaa tttacagaaa 5940
aactgaatac tacctcttta actttagtca atgtccacac acagaatttc ttttaccatt 6000
aattttttta caaaccttcc acaagtcatt caaactttta gctttatcct aacttaaaac 6060
aatcctttaa ccctttaatc taggcagaaa aaatccacct tccaaaatat attctacttt 6120
tcttacacac cttgagtgta gaactgtttc cttggtggtc tcaaatacat gttacagtgt 6180
taactcttag caacttttac ttttggtaaa aaccttggta agtaagggat tttaattatg 6240
tactaggtgc cgagcctagg acaccataag gtctgactgt ttccagcata actagagggc 6300
atgactctcc atatgtcccc aggccttatc tagagtctaa tgctgcaaaa taggtaagct 6360
gaacaatttt caaaaatcaa agaagcagtt tatgacctta aaatatttag caaagctaat 6420
atctgacttg cataatttag accaaatgtt tacatttttg aaggtatttt tattttatca 6480
ataatcttta aaactatctt tatttcccaa agattactaa agtcacctga actaaaagac 6540
attacacttt ttacttttct gacaatattt gatttaagca cctattattt ttaagccaat 6600
gaatcaaagc tcttttatat tacacataca acacatataa atacacagac aggcagaaga 6660
taaaagactc attccctaaa ccaggaattg aaccctgaac ccatgctgcc atggtgaaaa 6720
gagaaagcac agtcacatgg ttacaaggtc aagtttccaa ggacatacaa gacaataggg 6780
aaatgtcatc cagttctttt ttcttttttc agggatgtgc agcaaagttt ttaactgacc 6840
agtttgttgc gccttcttga acagtggttt tatgggagtc ctaggctcgc actgtatctt 6900
agagtacccc tcttttatga cagaacaata cagaaagaca cacacagcac accagattca 6960
ctacagttta agactagcct cacaaatcct ttttcccatt tatcaaaact ttacagagga 7020

ttttttacct aacagaggat aaatccttta cagagaattt ttacctaaac agtgattttt 7080

accattcatt caacccattg ggagcgaggg ggagagaaag aaagagagga aaaaggagag 7140

agagagagaa aggggggctg gggagagaga gaaaaagagg ctagaagtct gactggtaag 7200

aaatgtttac ccttttattg gcatcccagg cttctgggtt ccctttccct gagcagccgt 7260

agtgaccggg ctggctggac cacagccctg ggagccaagc cgcaacacaa aggaaaatga 7320

ttttccattc tggccagagc aaaatacatg tgacaaaaca cagacattag cccctttgct 7380

tagcgcccag tatcaaactg gcaaggctca tacttgcccc cccattgggt cccatcattg 7440

ttaatccaac ctctgaccag gagtttcaac ttgttgcctc tgggcaagat ggtggccctg 7500

ggtaacagaa aagataagaa aggtaaagga gagagagcaa agcactgcct gtggcagggt 7560

ggggaaagtg aagagctcag ggaggccaga aaaagactca cctattgcag tgaaactaaa 7620

aagttcaggt ggccacttgt cggtagtgaa gggatgtttt ctggcagtcc catcagctct 7680

caagtttccc tttttcgggg gaggaaaaag ctccccatgt cctgtggtcc tgtacatgcc 7740

ttatcctgtc acccatagcc cttagcaaca agtgcaagac agattaatcc aaagagaata 7800

gtagttaaca ttccatagtg ccaaacccgt tcttagctga gagggacttt actgagagga 7860

gcctctaacc ccctaaatcc taggaatgac tctaaccttc ctgagttgag ccttgaaccc 7920

aagtttgatc aagcatcttt gcctttcatt aataggagcc tttagcccac tctgccttag 7980

gagagactct taactcccct aagttgggcc tct 8013

<210> 5
<211> 1082
<212> DNA
<213> Homo sapiens

<220>
<221> misc_feature
<222> (1)..(1082)
<223> chromosome 4 nucleotides 10223944 to 10225025

<400> 5
agagcaggtg ggggaatcag agtgctgggt ggggtgggca ggtgggtatg aatagtcctt 60

ggggtggtca aggtgagcct tgctggaaag atgggaacag ggaaaaggca ctgggcacat 120

cagctgtggg ataactgaag gaaaagttca aaggcaggaa tgtggctaag tagttgaatc 180

aaggtaatga ggtccacgtg gctgaactgg aatgcctgca gggatggatg gggaagatgg 240

tccaagagcc ttttgggaga atcaaggcag atgtgacaat accctgtctg tagtcattga 300

gaggcactag acttaggcca tggctatgaa gccatgtcag ggtctcagac acaggaacac 360

aaagataaca attatgactc aataggatct ctctgtcttc tggctagaga ataggtatgt 420

cattgaggga taaagtggaa gcagagagac cctttgggaa gctattgcca caaccccagc 480

aggagatgga acagggtggg agcaggggag gtgttactct ctttgtagta gctgagactt 540

cacccacaga attaatctct cctgaatgtc tgctcttttg gaaaccatta aaaaagcaag 600

cccactttgt atgttttccc ttgttccatc ctgaatagtt catctgactc tgtggctttg 660

tctttgacaa cctgcaggat aggttctgtc tcacctggag aatgtgctta agattccaga 720

ggaagcaggg agccaggtgc tgaccaagga ccctgaatgc ccaggccacc ctcagtggca 780

accaccatac caactggaga ttgagagagc aacatcggac tcatagtgtg cctgccctct 840

ccacattgaa atttctccag ctgtaatata ggaatttaga ttatttgggc tctgagagtc 900

ttctggctca aactccagta aatgtagtga ttaaattctc ctcccctggc agatgtctgg 960

gtgagatgtc tgcatggagc ctgctggtag gaaatttctg tgggatgtta gacatcttgt 1020

gtgtatggta actagcacgt tgatgagtgg ctcaaataat ggtaaacact gtcattacag 1080

ag 1082

<210> 6
<211> 8325
<212> DNA
<213> Homo sapiens

<220>
<221> misc_feature
<222> (1)..(8325)
<223> chromosome 5 nucleotides 150498493 to 150506817

<400> 6
atcaaccgtt caaacttgcc cgtcaattca tactttaaat cagcaatgag gtcctggcag 60

gtggggaaga agaggagata gaagaggaaa gaggaagagt agttacaacg gtaagaatta 120

cacttaatga gcacttactc tggcaggtgc tgggctaagt aatctcatta aatcatcacc 180

cttggaggta tgtactgtta ccaaccctaa gagacaggct cagaaacaac agaggtctta 240

ctccatgtca tgctgctagc aaacggctat gcagggtcag gtccacagcc ctgtgccact 300

tccccaccac tgtctgtttc ctcgtttact gtgtatctct ctcactatct ggcagtccac 360

agagttgaca cctcacccat cttattcact agtgcagccc taacacctag cacgagccca 420

gcatacagca ggtgctgtgg gtgtattaaa ggctgaaaga cgcccagctg atgagaagaa 480

aaagagcagg gtaaggtgtc agcccacagg ctaacctgga gtgccatatc caggaaataa 540

cctgaacgag cacacaggtg aatgaaatca ttcttccacc cgcaccccat gggccctccg 600

gttgctgtgg ttaccttgcc gtagagggac ttgtagctct ggcagacctc ctgcctctgc 660

ctgttgctcc gtgaggtgat tatgtccagt atggcctcct tgtcactgcc tggaataggg 720

gagagcaatc atcatcctac ccaccctgcc tccaggaagc ccaccagggc cacttccttc 780

cctggacaca ggcctagtag ctccctgcag gcctcagcat gagccttcca ctcatcaggt 840

gctggtgtca gggcagaggg tcatggggat ttattaagtc acgactgaaa gtggacacag 900

cacacacaat atctgtgctc agaaggcccc tgatccagct cctcactgca caaatcagga 960

aattaaggcc cagagagggg caagaacttg tccaaggtct cacaatgaac ctggcttaga 1020

aaacccaggt atcctaacac ccagtacagg gttctttcca ccacaagaca aagaagaaca 1080

taagcaccac cttcggattg tggctgcctc tgggcaaagg cagcgggcgg cattatcagg 1140

agggggatct accaaggaaa cttcaactat gctaatgaag cactagttct taaatttggt 1200

ggtaaatcta tgggtgaaat ttctattatt ctttatactt tttttctgaa acattttatt 1260
ataacatcat gaaaattaat aaaaagagga gaccaaaaaa atgaataagt gcacttcacc 1320
atttcacaga ccctaccacc accaccagaa gggctctggg ctgtgtctac ttttggggga 1380
tacccctcaa gctcccatga agcctggcac ccaccaaagc ccttcatggc agtgtacaga 1440
gcctcggcat cctggttggg gtcaaagcct gggaagtcat ggatggagcc ccggtacttg 1500
gcaccctgtg gagaaaaaag tagagggtga gctgccaacc ccggactgag accaagctcc 1560
cagccccaac ctcaggtcag atgcctaccc tggtccaggc tgagctgctt tccctgcaaa 1620
atccagtgct ggccacatac accctgggga gcggtgtgga tgactttccc ataccacctg 1680
ctgcaacccg cccaaaggac agtgccattt ccccaaggga ggggggcttg gccaaggcct 1740
gcagccctgc accaccttcc aaagcatcca ggagagggta aaaaagcctg gctgagtata 1800
ggagatgaaa atgatccctg caagaatagc aagagtcaaa tatatatata tatatatata 1860
tatatatttt aagagatggg gtctcactct attgccctgg ctagttttga atgcctggcc 1920
tcaagcagtc ctcccacatt ggcctcccaa agtgctggga ttacaggcat gaaccagtgc 1980
acccagccag cccctcatat ttttatggca ttttatgttt acactttcac ctctgtgaat 2040
gtcattagtg tagccatttg tagacgctct taagattatt tctatcacag aagccaagcc 2100
caaagaagag ccacttgtgg gaggtcacac agaactcaga ccttttccct ctgctctcca 2160
gccccgctct gctctccagc tccaggaacc ttgcaggtgc cagctactga gggtggggtc 2220
ctgggcacag ggggcccaga gacactctca tttcaatgcc aagacaaggc actgcccctc 2280
tgggcccagt aaactgcgct gtgaactggc atctgaggac aagctggctt ggtctttgag 2340
gggaaggaga gaagcggcag agagccaggg gatcttagac atcgaacctg aacctgaatg 2400
cacaatggga ctggttcaca gagaagtcag atgtgggtca gttacatggc actgaaaagt 2460
ggggggtctg ggagagcagc tgagagctgg cagagtggag gatgccagag cctgtcaccg 2520
agcacacagc acagtgacaa ggcacttcat ttgtgctagg ccctacacta atggctgagg 2580
aattggggag aaaggggacc caattcctgt ctttcacgag ctcctggccc agtggcagag 2640
acagacaagc acatcaatta ctataaagct tgtagtaaat acattaagac aaaaataagg 2700
taatgtggga aaacagcaga ggctgatcca gctgcgggaa atcaggaaag aatccaaaaa 2760
agagggaatg gagggtgggc cacacagatc ttgccttcaa ggagtcagag gtggagcaga 2820
gtgagtcctg gaccgcctcc ccctctcccc gtctccgtgc ctcccaacgt ctgcactgca 2880
ggaccccggc caggcctctg gatgctctct ggtctctgtc tgcagaggct ggctcctctg 2940
aggtatgcat gcgtcggcgt ggagttacta tttaaagttc tgaccagaaa ggaacacagc 3000
tctcttcctg ccacctggga atcccctgca acctgatcac gcccctgggc agagacttaa 3060
agagtaacac atgccgaata cccagagcag ggcgccttgc aaggagctca gtgagcgcag 3120
gatgggggct ttactgccag cgctcccctt ggccttactt cagacctcac ggccggagct 3180
tgagaaattc ctgctgcctt ctccaccccg gtgggcagtt tgatccagaa acctctgaga 3240
gcagatacca gatccatctg cccactgcac agagattgac caaatactca agtatttgcc 3300

acatgaataa aatgaacaag aacacgaaac aactggggtc ctccaggaat ttcacttcta 3360
aatccttttc tctttctttc tggacacctc cagtccagcg atatcaaaaa agcacagcag 3420
gcaatattag ctactaggat ggatggagaa acaagaggtg cctgggcaca gtggctcatg 3480
cctgtaatcc cagcactttg ggaggctaga cgggagacca cttgaggtag ggagtcagag 3540
accagcctgg ccaatgtaat gaaaccctgt ttcagctaaa aatacaaaaa ttagccaggc 3600
atggtggcag gcacctgtaa tctcagctac tcaggaggct gggtcaggag aatcacttga 3660
acctgggagg cagaggttgt agtgagctca gatcgcatca ctgcattcca gcctgggcga 3720
cagaatgaga ctgactctgt ctcaaaaaaa aaaaaaggaa acaagaaggt tcagaggctc 3780
aaaattattt ctttgcaacg atccttaaag accttttagt tcagcttccc actcagttat 3840
tgagtccatg gagaatggag actgtcgtga ataagacaaa tcattctgcc taagaagaac 3900
cgtggagccc cagctctagc acaggcttgg catacaggct ggaagtaact gtgactgggc 3960
taaatgaatg gatggagaca cccatgacgg cccaagctct gagatggagg acgccgaagg 4020
ggaagacagt gaagggagac ctccctggca ctggatccct agcaatgctg tctccaatgt 4080
caggacctcc tgggaccagt gtggaaatat taagctttga aagatgccac caggggcagg 4140
tcctccaggg tctgtccggg ccctctgcag gctagcctgt tgccatgatt catggctgtt 4200
tttccttcca cctgtctgac caacctgtcc taatccacag cagcaccagt ctccaatcca 4260
cttcagtcat aggatgtccc tcccacggct cacttggaat aagagaggga aaggactttg 4320
agaaccacaa gctgagaact aaatgaggaa ggtaggtcta ttaccacttg tgtcccagaa 4380
gacccagaac agagagggca ggtgacatgt caagaccaca cagcaagtca gaggcccagc 4440
tgggaaggga atccaaggct ctttctaaca gaccatctct cccttcccct cagattccat 4500
ctgggtggga gctggagata agaagggaca accaccacat agcccactgc ttcctcctgg 4560
tcaaccatgc cagcctgccc ctctccttga atactgaagc atttgagtta ttgccctgtg 4620
ctgtttgcct ctatgccatt tcgaccctat attcaaggct ataaaaaggc agcaatgctg 4680
tccaatccac agcatcctaa gtgcttctca cattcagata ctcacaattc cagggcagct 4740
agggctaccc taccattcat tcattcaatc aagcaagcaa gcaacattta ctgagagtct 4800
actaagtaga tgcttaggac cagggaccac taagaactca gaatctggta gagatacatg 4860
acagcttcaa ctacaagaca gtgtggcaga tacgatgcta agggcagcat gggacccagg 4920
aaatatttcc tttcgaagtt ccgggagggc agggactgtg tctctcttgc tcactgacgc 4980
atttctagca ccaagtacag ggcctggagc aatcaagcac tcactgaata tgtttcataa 5040
atggatggat gaatgactgc gaatatattc caagcagatc agagaaggct ttctggaggt 5100
gacgtctaag ctgagtcctg agggatgagc aacaggctgg ccccaaggag tagagtaggg 5160
aggggaaggc gaagaggaag aaggcatcac acagaggaga cacatgacaa agacctggag 5220
ggaagcaagg ggcattcggg aaagccagcc cagagtctgc agggctcaga gcagatgctg 5280
gctgtcacta agatcatcca ctagaagcgg ggaaggcaaa gagatgaggc tggagaagat 5340

gatggggata aacaattcag actcttgtaa gggcagctct agccctgctg ggctggcctg 5400
ggcacaacac ctaagtcagg acctgggtaa gggcaagact ctaacatcct agtgtggttt 5460
tccttaccca ttaaactcag ctgaccactc acaaccctgt ccctagtatc tggcattctt 5520
tactacgttg agccatgtaa cttgccttgt atgtagatta aaaacagtca aatagcagca 5580
gatttcatat ggttcatgtg acaggtattt catacaaata cctgtctggt tgatatgtgg 5640
gatctagaaa ctattttaac ttgtctgctg gggcaggggt gtgtgggcgg tgacaagtac 5700
ttaagataga gtctgattct tcagagctca cagttagatg cccaaagtcg ctaaagtcat 5760
gggattgtaa gctgccggga agggatgcgg agaagaggga agcatgtccc caggatgaag 5820
ggcggaggat gaggcagcac gcagacccca atttcctatt gctgctcctc cccctccact 5880
gttctccccc tcatacaatg tgggtggtca cagcagtgtc tggagaaggg gctgaagtgc 5940
catgctgggg gaagggtgct taatgaccta cacaagcctt gatccaaacg ctcacttaga 6000
gacaagcttc ttgagcagca agctcgggct cctgcacagt tctttgcacg ggactaataa 6060
gaccttccaa gtgatgacct actagattag ttgtcctggg accccacagg atcatagcca 6120
ctagaagcaa aggtgggggg cttagcacta gtcacatttg gagatacaga gacagcccct 6180
caaatcatcc aggcccaggc ttcccaccgt gtcattctca caccaacttc atgaattctg 6240
catctctaag cgccacctgt gcaaccacac aggcttctcc tcacccaccc caccctgact 6300
gcctgtcaca cctccggcag acaggttccc ggctgcggga acccaggatc aaggcagggg 6360
acagcagaga agggactttc caccctgaaa tattcagcag gtcctggtgc cagctggtga 6420
gcagccgggc cctggggctc tgtggggctg ggccccaccc aggctgctgc tggagcagga 6480
gtcctagtgg cctcttggga gcagagatga tgtccccaga gagacaggta catggccagg 6540
actccagctg agagctgcct tggccggtgc tgacctaggt ctcacctcca cagctcctag 6600
aagtcccagg cacaagcacc tgcctatcct gccctgtcct caaggtgttt ttatgttcat 6660
tattgcattc aaacccagaa cgtccctgtg aaatggaggc cacagcgcca tgcctcctgg 6720
gtcttccatg gcaccatcct acttcttcat gtctttgggc ctcaagttct cctcttccaa 6780
aaggagagat agggagaaca gcacaataac cacagtggaa ggaactgggg ggtgtctagg 6840
ccagagaaga cggagcagtt tgctgtgtgc atgcacacgt gtgtatacat gcatgcatgt 6900
gtgcgtgtgt tggagttttg gaggacacca gattcaagct aataattgaa agccgagcta 6960
ataactggaa ggaacaactg cagctcaacg gcagggaggc ttttttcaca tttctctgtt 7020
attgtcttct gcaaccctca cctctccccc aaaccactgc accccaagca taccaaatta 7080
cttttactcc cctgagcacg ctgcatttgc tctccttcta gcctctgtcc cagttgtccc 7140
tgcctagacc actctgagcc cccttgttcc tcccctgagt agctctccct aggcagcctc 7200
ccctctctcc tcaacctagg tggggtgccc tcctgcatgc cccagagctc ctttactgcc 7260
ccaccagaac actgtgcatg gagggtgcca actcatccca taggaggagg gttccctaag 7320
gccctgccac cctgggactc acttgcagga ggtctactga accctagtcc atggcaacaa 7380
gaaaccacag atcagccagg cagaaggcat ggcccaggag cactggacca aggtccaaag 7440

atctgcaccc taggtcctgg cctctctgat cttccagggc ctggcctgtc cacttggatc 7500

atctagaatc ctgcagttca aatgcctttc aggaagtagc tctagtatga ctacacatag 7560

gaaatgggga acagtgaaag ttccaccttg gatttttgcc cataatcagt aacattttct 7620

cctcctggtc agagaggcca cttgtacaca cagtatactc ctcagtggct gtgaggaagg 7680

ccacagctag gacttctctg agggtactga tcctataaca tcctgtgctc ttcagaaact 7740

gtgaatatcc tataagccca ggttcactgg gggcagtcat gatgtcaagg atggatggag 7800

ttttattatt actgaattca accattccgt aaggggaaca tgaggcccaa agacctcaca 7860

gacagcttct gtctgtgcat tctgtggcca gtagttctgg gttggagtaa gggacaacgg 7920

ggcccagtcc catgattttc ttcaagatcc ccaagtttct tctccctgcc aatcccatcg 7980

tcacactgac tgcatggctt tctccaacgc cagccaacag acagcaccct caggcaaacc 8040

ccaacagagt agagtggggt tttttgtttt ttgagacttg ctctgtagcc caggctggag 8100

tgcagtggca tgatctcagc tcatcacagc ctcaatctcc tgggatcagg tgagtttccc 8160

accttagcct cctaagcagc tgggactaca agtacatgcc acctttgtat tttttctaga 8220

gacagggttc tgccatattg tccaggctgg tgtcgaaatc ctgcgctcaa gtgatctgct 8280

cacttcagcc tcccaaagtg ctgggattat aggtgagagt caccg 8325

<210> 7
<211> 2624
<212> DNA
<213> Homo sapiens

<220>
<221> misc_feature
<222> (1)..(2624)
<223> chromosome 6 nucleotides 30023851 to 30026474

<400> 7
gaaggtgaag ataaaactta cagagcttca agagtggcca ctgcagtgaa gtgttatggg 60

tccagtggtt aggggcatgc agagctcccc gccgccacac acacacacaa agtaaaagac 120

acacttgcat cttgcatcct caccagaagg aaggatgcac actaccttgt gagcctctct 180

gggttctagc aacaccacat tccacatcta agtttattgc tttggctgac actctgggtg 240

atataggagg aggccagctt tgagtggggc ctggactgga aaggacactg cagcagaccc 300

aggctgtggt gcagtcagtc accatccctc agacccctgg tgctggaggt ggcggtctgg 360

ggaaagaagc aggatggagc tgaaccaagc atcagtggga aagtcagaat gcagggcctg 420

ggatcaggag taaggccatg gagtccacag cagagaaaca tgctccatgt tagaagcaac 480

ttttagcatg ttactggccc tgataagata gaatgcttga gcataggaca ccaagcaacc 540

atgtgattcc aagtgcccgt gtgtattggc ttctatgtga cccatagagt cattcactgg 600

acaggcccag cggcatctat catgagacga aaacggtcca tgtcggttga gcctcaattc 660

catgttaaca cccacagaaa acacccagtc ctgatgtggc cctgaataac caaacaaatt 720

gaagacaaat tgaagttagc cagtctgcat catggatcag cccaggcctg ataggaagga 780

cccgtgagtg gagcaaccac agtggcaggg atgaagctac aaatgagtcc agcagcactg 840
tctctccact accaaggccc acccagctac tgcttcctct gaatactctg ctcgtgagca 900
ttgcagacca atgataggca ccaatagggc actatttctt aaagtaactg actagcccct 960
aagtgacaag ttgaatagct tgagcaccat ccatcctgga aggggcagaa gtttatcctc 1020
acagggatag gctcacaggg atgcgatgtg gtgtggtttt cctctctgct ctcagaccct 1080
cagtcaacaa cactattggc attcctgatc cactggctca gaatttcagt acactatctg 1140
cctgggggac acacctgttg gggaagggga tgaagtgtgg gccctgacca tgggatcccc 1200
tggtcgtatc accacctgcg cctctcaagt gctgccaggc acacagagtc atggacagga 1260
ctctacaggc acaattcagt accagcttgg atgaaaccct ctgaggaatg ggtgccatct 1320
ttcaggatgt gatgcatgta ttgaatcaaa gacgtctcta aggcactgtt ttcagaagga 1380
agaatacgcg ggtccaaaaa ccaagaagtc aaagcaggtg tgtctcattc cttatattca 1440
cccccagggt gatttactta taagtaaata aatacataac atgaatactt aaataaattt 1500
atttatgcat gtatgtatgt atgtatttat tttattcatt atattcaccc ccagggtgat 1560
tttgctcttc ttacttccaa aatctggact ctgcagggta ggaggtcctg gtttcccaaa 1620
gagggcaccc tggcaaggag acaaatgaga gtccatggaa ctacacattg tggttgcacc 1680
cagggatatt tgaatagtat gtgcccagag acaagcaggt gagaagagga ggaggcagcg 1740
ctgctatcac acaatgaggg caggagaagt gtgtgtggaa accaggaatc cacttgggga 1800
cgtcctggtt tcccttgtcc gttgtgtgag cggaatcatc cagcaaccca gcctgagagg 1860
gtttgatatt caagagccca gaaccctcag gaaggaagga ttgagtgata ctcctaggta 1920
atgtcccacg tctctgcttc tgtgctctga catcctcagc aggattagtg cagaagccct 1980
gcttccatga gttgttccca gccagtgacc ggtcacagca agcacactaa ggcaggccat 2040
tactgggaga catgggactc ctctgatggc caaatgtggc tccaggactc ctccatgccc 2100
ttcctcaact ctccttagac tgcctctgct ctaggatgcg tcgaacagac cttgtctcct 2160
tctgtccagc acttggggtc acacttgcat cattgtctgc cgccttttcc agggatttct 2220
ggctcgcgtc tcatattccc ttacaggtgt gtcccctcat aagatgccgt agactttaag 2280
ctcatcttgg catctgctcc ttggaggact tggactaaaa agcattgcca tgtgcacacc 2340
aataactctt acttattcca acctgtaaaa tccatctctt taaccaactt ctgccacccc 2400
cataaaatct attttgcgcg cgttcgtagt atctctttga attaacagat atttgttgta 2460
ttaagccact aaattttgag gtagtttgtg acacagcagt taataactat taaggctttc 2520
ttaagtttct gttattccat ggatgttatc tacatctttt aatttcctgc attttaataa 2580
tattagccac acttgctgtt tctaatcctt tcctcctatt cttt 2624

<210> 8
<211> 13590
<212> DNA
<213> Homo sapiens

<220>
<221>  misc_feature
<222>  (1)..(13590)
<223>  chromosome 9 nucleotides 129132654 to 129146243

<400>  8
tatgatttct cctgcacagg actcaggttc tagtggtaga aacagatacg caagttcaat      60

aaaagattta cagagtgtaa atagtgcttc gaggagaata aacaaggttc tccgatggag     120

aataacagca ggaaggaggg tggctttata tggggagatc aggaaaggcc actctggaaa     180

ttgacacttg agctgaaacc tacgtgatgg gagagagcca gtcatccccg gaactgtgga     240

agagttccag gtggaggggt cagtttggga gtgtcctctc tcttcctgta tagaaaacca     300

ctgttagtga tgagtgtgca tctcaggtgt gttggggaca gaaaaatgtt cggatgcaca     360

gccttagaac ttggattttc ggatttttaa tactctcaga tccagctatc ctgaatcctg     420

gcaatcgcct tgacttcctg cctctgaccc attggccagt gtgctttaaa tatctttgtc     480

ccttgtctac ccttaattga ccaccaagaa tttcttcatc tagtggtggg tcaaccaacc     540

ttgtagaggt cgattctctt ccaacatttg attttgtgtt tattatgaaa tacttcaggt     600

atacaaaaaa aagtataggg aataacataa tgaagaccta caaaaccact actggccggg     660

catggtggct cacacctgta atcccagcac tttgggaggc tgaggcaggt ggattgcctg     720

aggtcaggag ttcaagacca gtctggccaa catggtgaaa ccccatctct actaaaaata     780

caaaaaaaaa ttagccaggc atggtggcat atgcttgtaa tcccagctac tcaggaggct     840

gaggcagggg aattgcttga accagggagg tggaggttgc agtgagctga gatcacgcca     900

ctgcactcca gcctgggtga cagagtaaga ctctgtctca aaaaaagaaa gaaaaaaaaa     960

aaccactacc caatttaagg aagaaaagca ttaaaaatat gattgagtcc ctgtttacgc    1020

ctccctgccc ctgttcctct gtcttcctcc cttccctgtg gaagctgcta tctatcattt    1080

ccaccagatc agaattaatt cggtttaaag ttggagatgg tagattgcac atttgtacca    1140

gctactgctt gcttggcttg ggcttgcttt tttttttttt ttcacatctg ttttgtgact    1200

tcttcagatt gaatatcttt aggaattggg gaaaatgagc ttttagtata aattgcctat    1260

aattttcatt tctgtgtcta aaagagtcca caatatatca gcctgagatt atgttaagct    1320

ttgaattaca acgaactctt cttcgctgac tgggatttgg acggttgctc atgggttgat    1380

tggaagatgg tgccaggcct gtgagttcct gtggccattt gttggaaccc tgttttgcca    1440

cagaacagaa ctggtatcct agagatgcca ttagtaatga aattacaaat gaacgaattc    1500

ataaaagcat acagtctcat ctgtaagcta taaataccta tcaaaatgaa ggaatgattg    1560

ttgttaacat tggtcaataa cccagatgga tgggaggaag ggatcccctg actgtccatc    1620

ctggtgactg ccattgtgct aagtcacgaa cagcccatct caaaactata tacgctgtga    1680

tgataaaatt aactgtaaca ccttttgtta ttatcctaag ggctctgtga atttcaagtt    1740

tggggttctt tttgccaaag atgggcagct cactgatgat gagatgttca gcaatggtga    1800

gtgatctcct cccgctctct gctgccagag accctgtcct gccttgttgc attcttgcca    1860

gttggagctt aacttgtctg ttgagttgga gtctttaata cgaggagata gttttacaca 1920
atttcagcgt gaattttgta gggcagttgt tgcccagttt cattcatttg gcaaatattt 1980
attgatcacc tcgtttgtac ccagtcactg ttccgggcac tggagagaca gaagagcaca 2040
tgggatttgg agaatttggt tctacacagt cccaagagca gtggaagatt tttctgtgat 2100
ttttatgaga ggaagaatta taagatggcg tcatcttcct aatatttagc acttttcgtg 2160
ttaaatacct gcttattctg aacgcaagca gtaataacat ttttgttttg cgatagtaaa 2220
cacctgtggt cccaggctgg cagtagtaag ctgggttcat ctgattctgc agttttcctt 2280
tccttggagt cctgggaatt gtggggcttc ctcatggtgc tcaagaagaa ggaaatgtga 2340
cttacccatg ctgtttacca tgcatgaatc ggcagagccc agttaatttt ccagcgattc 2400
cggtccatag ttagaaaatg aggacttgtt gatgcccata ctttcctcat actctacttc 2460
ttcctgcaga aattggaagc gagccttttc aaaaattttt aaatcttctg ggtgacacaa 2520
tcactctaaa gggctggacg ggctaccgtg gcggtctgga taccaaaagt aagcctgcct 2580
cttgatctat tctgttgtaa tttgtataac ttttgtaagt cagcctgacc cgtgtctgaa 2640
cttataccag agaaggggga agggatggat gagagaagac tggagggagg tgagctccac 2700
accctgttca acatagagtc atggacctgt ttggcatttc cattgagctt cgtaaaacta 2760
aatcagccct cacagccctg gacagtgtca gaagtcctgg tctgcccatg aatattactg 2820
ctcagcacca tctgtgtgcc aggactctgg ggatatagaa gtggataaac caacatgggg 2880
tcctcctttc taggagcctg cagtccaggg gacatccagc agatgttatg cagataatca 2940
tcaaatggtt tagcactaag tacagaaggc aactgagaag aaaaagtaca gagtgctatg 3000
gggcattttg cctaatctgg gggattaagg aaggcttctc tgagcttttt tgaggccaga 3060
gggaggggcg agtcggaata attggagggt ctcgagggga gaagagaatt ccaagcagag 3120
agaacatcca tgaagacctt gtggcagaaa agaacatgtt gtgttctaga aactaagaga 3180
atttgtttat ggctggaggg aacgaggcac tgagaatgat gcaatgtggc ctggagtagg 3240
agagggggca tgaccctgaa gggagttttg aggttatatt aataagtctg cattttattc 3300
tctgtcatta aggggtttca agtaggggag tggtgagatt agatctgtac ctttgagcag 3360
acaggggagg cagaggagaa gcctgctctc agaggctgtg gcaacagtcc aaacaagaga 3420
cagggttgcc ttgggagagg gccataggtc agaagggaga caggggaact aattcaagag 3480
ccattttcat ggtagaattg gcaagtggag gttgaaagag gagacagtac ttgaagacac 3540
ttcagggttt ctgccctggg tagctgggtg gggttggcac cagcggtgag attggaaaca 3600
cctgaaagga agcattttga ggatgggggc ctgagtgggg aaggtaggta agattacaag 3660
cttagggttg gaagttcttt caccatctca tttttgtgta gcattgctag cttttccctt 3720
tgtttctgtt tttttgcttt atttgatctt cacagtcagt ccttgaggta agtcaggcag 3780
cgggtgtgag gcccttttgg cctaatagtg ataggcttcg agtgcaggtc tccttaccct 3840
ctgggagcct catcttccca ccagcctgcc atggcatcac ggattctgaa ttcgggagag 3900
ctaagtgtgc cctaactgac agatgcaggg ataggggatt gggccccagt tgtcaggttc 3960

cagcttcact cgaacacaca tagagtgcac aaggtgaatt cttcaatgcc tgcctctcat 4020
acccccgctg ccaaccctca gcgtctccaa tatggtcata acaaccatcc actgagccct 4080
tgatacagcc aggcattctg ctaagcactt tgcaaatata atctcattga gtctccccaa 4140
tatccctagg agttagatac gacttttatc ctcattttca aattaaggaa ctgaggctca 4200
gagagaagtg aggtaacttg tcaaaagcca catagccaat gacaaattga atccaagtcg 4260
gcctcattgt aaagcctttg cttttaatca caaacaattc tgtcacaaat accacttcct 4320
tttggcttat ggacataatt gtttgtgtca tcttgtgtta atttcatccc gtacctccca 4380
cagcaactgc aaggcaagga agctcaatac tctcctcttt agcctggact tcttatgacc 4440
ttctccattc tgaggcaagg catgaattgg acagcaagtg agcttaggga gagaggaaga 4500
ggcctttcca tgtggggggt tgcatcaagt ggagggttgt tgcttccatg caggtggata 4560
atccaaatat cttggagaat gtcctgtata cagtatttcc ctagtctgca aatgaaaatg 4620
ctttcctttt ggctcgaatt ctctgtgctc actgaatgca tatttatatt ttgcagatga 4680
taccacaggg atacattcag tttatactgt gtaccaaggg catgagatca tgtttcatgt 4740
ttccaccatg ttgccatatt ccaaagagaa caaacagcag gtacatgtga acatacaaac 4800
catcaaatag tgatgtgaat gtggagaggg caccattatg attccataga attaggggct 4860
atgccggtta acatatgagc tgagctgtgg ccaccaattt agggatgctt ttggcggtct 4920
gtattggaac tttctacaaa tcttaggaag agctattctc actggtgctg tcaccttaga 4980
gaaataaggg gtcagagata ataatgtgca tgcgtgtgta tgtgtaaaca tgtataggta 5040
tttgtatgtg tgtgagtgtg gtttcataga ctcttttttg tctctctctt tttttttaat 5100
atatttgatt caaacattct tctatccaac atggactctt gagagactat tttgtatagg 5160
tgttaccttt tctctgtgcc ttctctcagt gtgccttgtg tcttgtcaaa atgagactct 5220
ccaggacata tcataagtgg tcctgtgtga tggttgacaa gtattgttat caggtcactg 5280
caggaagtta acactgagta tctacatggg atgtaccagg ctgggaaaaa acctctatct 5340
aagatactcc tcacccaccc cagagcttgg ccactgagac aggagcagag gagatgggtt 5400
ggtgagcaag ggcctaggct ggcaaaacat gggaagcctg aagtcaaaga tgtctgaagt 5460
tcagggctct gtcatagtgg ctgtggtttg ttatattggg ttatgttttt ttaactctgt 5520
gaatgtgcca tgtttgagag tctttctcag tgatgttatt tatgctcact acaggtggaa 5580
aggaaacgcc acattggaaa cgatatcgtc accattgtgt tccaagaagg agaggaatct 5640
tctcctgcct ttaagccttc catgatccgc tcccacttta cacgtatcct gggcctttgt 5700
aaatgctcca gtgtggcaag ctgtggttca gcctcactgg acttccatga cccttaaacc 5760
aaatcttgtc atggacttgt tttgtaatgc tagctttaga ctcacttttg gggaattttt 5820
cctaagcatt gtgtatagtt cccccttcct ctctccttcc atttcagtag tagatggatt 5880
aattctcaga gaattcttga ctcctgaaat atggtctctt ttctcaggta gtctacaaaa 5940
tgaggaaaac ttcctgctat ccttcagtca taaatgattt agaagaatta taggtgctga 6000

attctctgag aaatttgctt taggcataaa aacttgcaag ttaagataaa aggccaaaat 6060
aatttttctt gttgatttaa gccctggtaa tcgctgttgt attaccatct cttgaaattt 6120
gagaaatgat agaattaata gtatttttat ctgattcccc taatccctgt tcagctcata 6180
ttgctggcaa agtccagcaa tggggctgaa gcttctgcag tgtctgagct cagctctgcc 6240
agacaggcat ggggcatgag cttgacctgc ccagcagaca ctcagttgtg gccctgttgc 6300
tctgggtccc agaccatgac agcaaaaccc aaagttggtg gctctactgg ttatacataa 6360
taagcaaaag gcaacaaatc agcattttca gaataaattc tagtcgagtt actcatttcc 6420
ctggaggctc caagtaaaca tgtttggaga caggcccacg gtgactcatg gcatctggag 6480
acttggactt gttccttgtg acataaaaat agaggatgga agtaagtgtt taaaagttaa 6540
cagtttccaa actttcttcc atgagggtag ggaagcaaac aatatgcact tgcaccttca 6600
ttagaatttt cttccctttt catccctgct ttctgagtaa tatctgcccc tgtcccctgg 6660
gatttaggac taatatttcc cctttctaga ggtggttctt cttgtataac agaaaacttc 6720
tccaaattca cagtagaagc agtggtgagg tgactgttca ggtgattgag aggtagtcat 6780
tattttttag aatttccctg agttatacgt tttagatttg gggcttggca catttgttcc 6840
tgaaaagggg caagcagatc attaaggcag attcattcca gggagcctag aaatggagac 6900
actaaggaat tgatggctcc atagccctag ccaaggaaag caggggttgg gagatggtgg 6960
gtggaggtgg aaggggagga agaagagtgg gagaaaggaa gaagctggat ggattctttc 7020
ctactatttt agactctgca aaccccagca caagctaagt agattctgct cagaatctgc 7080
caccagctac aacaggcttg ttttgttttg tctaccaaac ccaaaaggag agaaagggaa 7140
ggtatacagc ggaaacattt aaattgcttt ccctttccaa atgtgtgaga tgaaatgtat 7200
tactaatttc ctattgggat cttgtttgta cttccaggca agggaactgc tctttttgct 7260
ttgctgactg cttagttgtt taatagctat atttctattg cttttctgac agctttggag 7320
atttaaacct tttccccaaa ggatgcacat acaaacattg aacctactca tggtgccctc 7380
acataagtct ccctgggctg tctcttggaa agaagtatgg gttctgtccc aacctaagag 7440
tgataggtgc cctaagcaca gaagcgctgg tcgagaatga ctctgcacaa gggattttca 7500
gagcgcaagc tgtcagtcgt gagcatcagt gttgtgatta gttcccttaa ccatcaccac 7560
agatattttt gccttagtga gatacaatca acaaaatgac aattacaggt aggtaatgac 7620
tttgtctcga ttgcttgtcc taattctcat gcttgttaat ttagcattga tttttacaag 7680
actcttgata tacatatttc ttgattgatt taatatgagt gcctgcacct tacaaaactt 7740
tcttcaaggc caccatctct tcaggaaaca tttactccat ccagtgttgt tccatgaagg 7800
agacctaggg gtcagggtta gtttgatatt ttattgtact ttaccatggg aacaggtttg 7860
atatatttga tcttcatcct ttcctagtct acattcttgc ctgcttatct ttctgttctc 7920
aaatattaat ctgttaaaag gtattatgtt gtaaaaagaa cctgaacttt gagccaaata 7980
gatttgagtt cgaatcccag ttctatcatt tatttgaggc tttgaaaaaa atgttttatc 8040
ttcttgaagc ctcaggcctt ctgtgtagag tgaaggtaat gacactaccc cggcaggtgt 8100

agtgacaatc agaggcaata ctgtatctta aaataccaag tatgctgcct ggtatgcagc 8160
aggtgctcag tgatggccat tgctcttctt cctgggtacc tggcttgatg ggttggacca 8220
tgaaggattc aggaagacct ggtctctatc ctcaaagcac attgactaag ctggagggga 8280
aagcgaggta gtggcctcgg ccctggcaga ccagagaagg cactcctggc atggagcaaa 8340
tattcctgac acatcccata tgcagctgga taaatggaag gacagaatag tggtgtatta 8400
cttcattttc atactgctaa taaagacata cccaagactg ggaagaaaaa gaggtttaat 8460
tgggcctggc gtggtggctc atgcctgtaa tcccagcact ttggaaggct gaggcgggcg 8520
gatcacgagg tcaggagatc gagaccatcc tggctaacac gatgaaaccc cgtctgtact 8580
aaaaatacaa aaaattagcc gggcgtggtg gcaggtgcct gtagtcccag ctacttggga 8640
ggctgaggca ggagaatggt gtgaacccgg gaggcggagc ttgcagtgag cccagatcgc 8700
accactgcac tctagcccgg gtgacagagc gagactccgt ctcaaaaaaa aaaaaaaaga 8760
ggtttaattg tacttacggt tccacatggc tggggaggcc tcagaattat ggtgggaggc 8820
aaaagtcatg tcttacatgg tggcggcaag agaaaatgag gaggaaacaa aagcagaaac 8880
ccctgatagg cctatcagat ctcatgagac ttattcacta tcacgagaat agcacgggaa 8940
agactggccc ccatgattca gttacctctc cctgggtccc acccatggca tgtgggaatt 9000
ctgggagata caattcaagt ggcgatttgg gtggggacac agccaaacca tatcaagtgc 9060
taatattaat tctgcaatat tctctactta ggctgaaaat attttcagaa gagagcgtac 9120
cactctttgg ccctcccttg ccaactccac cagtgtttac agaccaccag gaattcaggg 9180
actttttgct agtgaaatgt aagtttctgt tttgaaacaa ttttgcaact tgttctatgt 9240
cagaatacat ttcttcagat tctcccagaa tgctgtttaa ggaaatgtca ttgtttaagc 9300
attccatgtt ccgttaattc tttgcacaac atgtgtttag tattctggtc atgttgtgca 9360
cgttttctcc tatgttttca tccccacctt ccatcctgtc acctgccttg aagcttctct 9420
acactcaccc actaaggacc ctcgggcctc aaatttgcct ttctgccctg tgttctgata 9480
gagccattgc tgaatctctg cttgtatatg tatctgtatc tgtcagagag tgggatgtag 9540
gtgggggaaa gagtggttgt gggtatatgc ttagggatag ggagagttgt tggggaatgt 9600
gtgtgcacac ttgcgtgagt gtatatgtgc acccatgcac atgtgtggga aagagaaaga 9660
cagaatgaga acataatgtg tgtagaccat gactgccagg catcacttgc tggtgagcac 9720
atattttcac tctactggta atacccaaat acccgactct gaggccacat ctcttaaagc 9780
acaagagctc tgcgcatccc tcctcagggc acccctggga attatttctg gtagtcccat 9840
ggtgggactg tgtcattgat cagaaacaag ttactcgggt tcatctgctg cagcttgcta 9900
gccgcacgcc ctggcagggc tgcttggtct atgccctcca tgaagatact gctttccagc 9960
ctcctgccag gtatgctggg cattgcagct gctttgctgg ctgcctcctg tccccacctg 10020
caactcctcc ccactacagt ctttgcctaa gtctgtcact gttgccgtgc agctctgaga 10080
catctagaat gcccctctct tcactctgtc cgtcctggtc acctggcctc aaggaagcat 10140

tgactgagcc caaagcctca ctgagtccta cgtggacaag tgttacccaa cctccaatct 10200
taatcctact ccagtactgg cccaagccag tccctcaggc cagggcaagg gacaggcaca 10260
tgttgtttct cctctgtctt cctgtctccc cagtcactcc ttcacagtcc caaacatgac 10320
ttctcctgca cagaaacagt gccaagcatg agtgtcagtt ctagtcacca gaaacatcca 10380
agtcctggcc aggctgtttg gtagcctctc ttccatagga aacccctccc ttccctttca 10440
gagaccttgg catgattcca caactttgtc ctggagaaca gagacatgaa agacaccttt 10500
gctgaggctc cctctgcttt ccagctgggc tgcctctctt cccagctgga ccaccagcgt 10560
tgttacctcc catactccaa cctataccag ggtccatggg tgatgtctcc ctcgacacat 10620
tagaggcagc agacactgca aaccatgtgc aacagatgat gattttgtct ctccctttcc 10680
agtatttata ctcattattt caaatgctac aacaaggtcc caaactcaga tgcctggaga 10740
gccaggcagg gagcatgagc aagtgaggcg ggctgagtgg aggacagtca ggagtgctgg 10800
gagctgtgac acacttgaga gcatgtgctc actccatcca gctgaggcct ttcagagcat 10860
gcaccatggc cagggttgcc agatactctg agttttcaaa tcaagccaga aagctgaaat 10920
tgtatgtgaa attgtccgag tctcatgtgt tcaagtccag aggatcagac agagagcaag 10980
aaacagatct gaaacagagt cagcaacaga gtgggtgtag ctactggaag ctgaaagggt 11040
gttggcagca tggggctcag gaagacagca ttcactgtag ggggcctgtg gcaaggaagg 11100
ggccgctaaa gacctgtaac caccaattgg agatcactca gcagcactag ggacccggct 11160
tataagtgga gccagtcaac agatggactt tcatcagcgt tgtcaaagac ttgaggtgaa 11220
atgaaacgaa gggctgggtt gatttgacat tgaagatttg aaagtagaaa cagcagaaga 11280
atgcaggctc aaggtgatgg agagggtgtg ttgaggagat ggagtgtggg gccaggaagg 11340
accaggaggg gttggtgcgc tgagaggaca ggtagcagcc ggcggatcat agagtctcgg 11400
aattccaaag tggattgagc agcgaggttg aaaactctga gattacagga gatggcattt 11460
caacaaaaat cttgaccttt tccttttcag ccgaagcgag gctttaaaat ggattgttcc 11520
aaggtactaa gcagaaaaaa aacctcaaac aaaaaaattc ctagattcac agaactaaag 11580
aaaagaacag acaagcatag tttcaattct tgtgtgtgtg tcaagagata tcttgtaatt 11640
ccctttttaa cttgatttat tttagtaatt aatggtgaaa aagccacttt ggaaacccca 11700
acatttgccc agaaacgtcg gcgtaccctg gatatgttga ttagatcttt acaccaggat 11760
ttgatgccag atttgcataa ggtaattctg ggtccatggt cttctttcct tcttatgggg 11820
tctgagttga gaacacaagt cctcaactca gcgatgaggc cctggtgaga aaagcgtagg 11880
aggcgccttg atccttgctg tcaggactgt gctgtggcca ccatgggctg acagtctctc 11940
attgtgacac aagaaggaag gacattgggt ttccatacac aggaatgcga tttccagaaa 12000
gccagggcct gtggaaatca aggtgcaaac cagggacagg gaggttgggt atcaatcagc 12060
aatctctgga gcattgaaga gtgctttttt tggggtggct gttcttggta atactactca 12120
gcattgccag tcacatgagc tgatgggcag gcaaaggtgg gatttttaga aagacatagg 12180
gtgctttttt ttggagattt attggccagc cccatattca ggcactgaaa caagcattcc 12240

catttactga agacccagac acttcgtagg cctgagacat agccacactg atgagaccca 12300

acacctttct atcacttcag gtgcacatgt gctcttttct tttttttttt tttttttttt 12360

ggagacagag cttctctctt gttgcccagg ctggagtgcc atggcatgat ctcggctcac 12420

tgcaacctcc acctcctggg ttcaagcaat tctcctacct cagcctccca agtagctggg 12480

gttataggca tacaccactg cacctggcta attttttgta tttttagtag ggacggggtt 12540

tctccatggt agtcaggctg gtctcaaact cccaacttca ggtgatccac ctgccttggc 12600

ctcccaaagt gctgggatta ctgtcatgag tcactgcgcc cggccgacca gcaccatttt 12660

ataggcactc cttgactctt gtctggtcgg tgtaccccaa ccactcgcag ggattgggcc 12720

aggctaaccc caggacccta aagagcaaac ctcaattggt aatctctcaa ttccgtaagg 12780

tcagggaaga cacagagagg gtaccaagat gagcaatgat gtccttcaag ataagtaatc 12840

atatcccagc tgaagcatca ctgcctgagc caggcttccc tagcccccca gttgaagcca 12900

ggtcccctgt taaatgtttt catgtacttg ccacacttcc ccttcatagc agtgatcaca 12960

tgtgctaaac atgtcgttgc ttaatctgtc ttcccagtag actctgtgct ccacaaggat 13020

agagactatg tctatcacat tcccaaaatc tggcagaaga ctaggtgctc agtcggtact 13080

tgctgattga atgagcatac cccattcagt gactacagtt ttaaagagaa ctaacattac 13140

taagtatgca ctaggcactg tgcgaagtac cttacatgca ttgcctcatt taaacctcat 13200

aacacaccta ggagacaggt attaaccaca caaagacact gagattcgga aattaactaa 13260

tttgctcaag gcgacccaca aggctgcaga tttgcacagc tctaggggta ctgttctcat 13320

agaattcagt ttgttgtgtt ccagggggtg ctgctgtgta agattggcat cccagagctg 13380

tgcaatgtgg tggcccaggt cacactgtta gtaagttagt aagtgataca gctgcaggtc 13440

tgctgatttc aaagccgtgt cctcagcccc catgcaatgc tggctctcca cctagtgact 13500

ctgaatctag tatctaccat ggacctggta cacagcaggt gctccatcaa tgcttattga 13560

gtgaatgaaa cagaaggtag aaataggacc 13590

<210> 9
<211> 25396
<212> DNA
<213> Homo sapiens

<220>
<221> misc_feature
<222> (1)..(25396)
<223> chromosome 10 nucleotides 25827676 to 25853071

<400> 9
caaaccattt ttgaatgtag ggctgtgtgg gaggtgaata gggttgctcg cattagaaca 60

gcctttgcct agaagatgtt ggaatgccat cagtaggtgc tggatggcac agctgtcgga 120

tttgtgtcaa aaggcaatat atcccctgca ttgagactgg agcgcaatga ctcatttgct 180

tgaggacttt cggaacccta caagcctgca gggcctcttc tcgtggccta aggatgcctt 240

caggtgtgac taagatattt cagacttgga agaatacaga attgaattct caggaatctg 300

gttaagtggg aagggcagaa acatctcagt tctgtgaaat tatttatatt ttagccagaa 360
gattgtcata gcagcaagct gataaaagtc gatatatttc atttattttc tttatatttt 420
tctaaaagtt ttctgatttc agtaaaactg taacagaatt ataattcaca aaaaaccaat 480
aacatcgcaa tttccagagt ctggaagaat ttcattatga ttcagtgaga ggacaattta 540
cctttcctga aatacagtgc gctcacaagc tccataccca gggctccaaa cccttttgca 600
aagtctcctg atgagtggtt aacccaccat tcagaagagc gggtgctggg atgccttggt 660
accaaggtgg tatgtggaat ctcagccaag agcctaggag gcttcttcct gctctgtgaa 720
gatcacatgt ttgttccttg cctttgtgta tttcctttga actaatcacc tgtttgcttt 780
tggaggatat agcagttaac aagagacaag actcttgctc tcatgaagct gatattctag 840
cagagaagac agaaaatgac attaactaat aaacatttca gataatgata gatgctataa 900
agaatacaaa atgggagagg ggtgttgggt tgaggcatta gaaggccact ctgcagaagt 960
gacatgtgac aaaagaaata aatgaagaca agggaccact cttgtgcaca cacccacaac 1020
aacagcactc caggcaacat gcatagcaag tggaaagcct cgaggcagga taggctttga 1080
catggaggat ggaaaagggg cccagtgtgc ctgcgtcact gtgggaaggg agaagaacag 1140
taggaggtga gctcagagag gcagacgggc taacttacct agggccttgg tgaccatagg 1200
gagctggatt ttattccaag ttcaatgaga agcctttgag cccaacaggt tgattgcctt 1260
cacttgatgt gattttagca tatgattaac cagattttta aaagagacta aatccatttg 1320
tctcgtaaga tcagacattt gtgaattttc ttttcactta catatgaact tttcacttac 1380
atgtgaactt ttcacttaca tgtgaacttt gatggtaaca gaggaagata cgacagatgt 1440
gtgtttgaat tcctatcctt taacctggta gtaatttgag cttgtagttt ctggcagtca 1500
ttactttaat tgaatttggt ttgttgggag ctggagactt ctttaaaaac ctggatttta 1560
tacaaaagaa ttcatgtact gcagataaat atggaaaaag gtgcctgtgt gaggccttca 1620
ttggatttta gaatgatact tgcttttaaa ttgacggtct acttggattt tttcaaaatt 1680
cctggggatt actacgtgcc ttttggcaga acaaaaagga agaaatgaaa gcacagattg 1740
aattgctatc acttctaaat cctgaaagaa gtcagacagg acccttatta agttactcta 1800
gaatgtgaca tatataacct ttacacagta atttatttga gatgatcaat cttcactaaa 1860
tgatcaagtg tttgttcgct tggaattaaa ccataaatgc tgttagattt ttctctgttt 1920
tctttgctga cctgtggaac tggctgggct taacatttag gcacatgcat gcatttaaat 1980
cttggagagg tatagacagc gtgaagtaac tgataaatga acagcattta cagtcacaaa 2040
gttattgcac atggcagctt attggcatat aaattaaact gctgcaaggt agaagaatgg 2100
aagcaaaaat aggttgctgt gttagatcaa attaatttga gaatcatttc aacattttat 2160
ttctctgaaa agtttgcttg gaggtctcca gctatgtttg gcatcaagtc acattcatac 2220
tgaaggaaaa tgtgctttaa gagtcaaagt tatacaacat gcattggtat cagcctatcg 2280
tacgcgtaaa tggaggttgc tttatggaga taatttctgg taggagatag aagccaggaa 2340

gaggcacagg gaagatcagg gactccgacc tgttgagcaa gctgggtagg agaaatcccc 2400
cgggtcatgg cacagaatag aaaaagtcaa aggcggcttt gagaagagag ttgccgggag 2460
tgggataggt tagcaccaga cagcgtgggg acccctcaca gagatgctgc actgcgcagt 2520
ctcactcggg gcatggcgtc cttctactcg ctcatccttc aagtctcagc ttaaatgcca 2580
ctttcttcag aggaaccttt cctgaccgtc tttatcaaag gtctctcctc ccatgatttt 2640
ctatgattga atctactttt ccttcatgat aaattgaaat tatatcttta tatgtttact 2700
catgtattgt ctttctctta ctaacttaca atttgcacta aggcaggctc atgttttctt 2760
tgtgtctctt cagcatttag ctctgcctgg cagatagtag gcacgtatga aatacttgct 2820
gaatgaacat agaacctgca ggaaaacagt ctgaaaactg ctgtggactg gggctgcacc 2880
ccacatgaaa cagacttggg aatgcggctc tggccagtgt gcgctcagag agacgtgtgt 2940
atattcccga ctcaagtagc cggagcagac ttgtacctgc aaactgggtt gatgagggtg 3000
ctggacaata tctcataaat tatcctaata acctgtgaaa tagactgagg gaaatcatag 3060
tttccattct agtgtttaga agacagaaca aacaccagaa aagaaactac ccaacttacg 3120
tggctaattt gaacttcaac accagtccct cgtctccctt tctcatctcc tcacactgct 3180
gtccgtatgc ttggtacccc cagcacttgc aaactgtttg tctctcccta catacaccat 3240
accagggaac tgtttctgag ctctataacg cttctgcctc caaagcaaat ctgctactct 3300
cctcctgggt gctgctagaa aacacgccca gtgccctgga agattacagg ctctaattcc 3360
tgcttcttga gatgaaacaa ttcagcccag ggctagttcc aaagcaggaa tgcctgagtc 3420
tgagactatt ttaagcttcc cctgagacag agatgggctt atataacctc agaccagtca 3480
tgatttccac agctcactgc agagctgatt aaagtagata tagccagggt atttccacca 3540
tcaaccaccc tccctaggac ccgtgacagg ggagaaaact ttcatgtact gagcatctcc 3600
catgtgtcaa gcattttaca tttatcatcc tgtacacggt gaactgaggt aactgtgggg 3660
tacaaccaaa accctgcgtg aggctgtcat ttagaaccac attcacagag ctgccctgga 3720
aacagcaatg tgagtaaggg gcaaggtggg accactttgc agcccagtag tcctccaaac 3780
actgaaaata atctggctta gtttccatgt tcctgctggg taatgaagca ccaaacagtc 3840
ggtaggtttt tcagtatggg ggactaactt gcaaaaagta caatgagcaa actaagaggc 3900
agtgagttgc taattcccaa ggacaccatc tataggcagg accatgtctg ctgtcaaaca 3960
gactgtgaat aaatttcaag caaggaaagg ctctgaggta catttgcagc ttctcttccc 4020
tgcatgtgta cctttcacat ataaaggtgc tgggaattta tcacattcct ttctataggc 4080
actggaatgg caaaaatgat aaaggtgggt agtaaattgg ctggttcctt tacaaaaaaa 4140
gggcaccaaa cacaggatta ctcagcaatt ccatagaaag ttcccaagcc acctttttaa 4200
aattgttata tagtgctatc aaaaaggcaa agcttctctt ttggtaacat gttttaggga 4260
agatcatttt aaaatccttg ctctgaaatc atgagtgtaa ctttatagtt gtaggaaaat 4320
ctaggaatat atatgaaatt aaaatttaat tgaggtccac gtgcagtggc tcatgcctgt 4380
aatcccagca ctatgggagg atctaagggg accaggaatt caggagctcg tgaccagaag 4440

gggcaacata gcaagacctc attgcaaaaa aaaaaaaaaa attataatca accggacatg 4500
gttgcacatg cctctagtcc tagctactta ggaggctgag ctgggaagat tgcttgagtc 4560
caggaatttg aggtcgtagt gagctatgat catgccactg cactcctatc tgggacaaaa 4620
aaataattta attcagttcc agtttagttt aaaaaaaaat cccaaccttt gtcatggata 4680
tctaagtatc tgtggacata ttgccctttg ccttgtaaca gattctgcat tattaagagt 4740
ggagaaaaat agaaatggga ggaaagtgca gtcagcatag gcaaggaatt tagctctctt 4800
actaaagcag aattcaaaat ctctactgtg tcagtattac agggagaatc tctgggtata 4860
ttattcctta ccagttccct ggaatgtatt aaactctgca caaataggca tgctgtgatc 4920
tcaggttgca gtttttgttt actgcatgtc acatgattga aaaccaagtt cttgtcctgg 4980
ctatgatgtt gcttcctcag aaagccttct ggtctaccca cagctcccac tctcactgtt 5040
gtgttctctc tttgcatgct atttctttct ttagtcacat aatatttcat aatatcaatt 5100
tgtttactta tttatagtgt ccccccttca tttggtttca actctatttt cacttttttt 5160
ttttttttga gacagagcct cgctctgtca ccccagctgg agtgcgatgg ggtgaccttg 5220
gctcactgca atctctacct cccaggttca agcgattctc gtgcctcagc ctcccaagta 5280
gctgggatta caggtgcctg ccaccatgcc cagctaattt ttgtattatt aatagacata 5340
gggtttcgcc atatcagcca ggctagtctc aaactcctga tctcaagtga tccacttgcc 5400
tcagcctccc aaagtactgg gattacaggc atgagccacc acacctggcc ctattttact 5460
acatttttaa tttactgatt tattctcctt tgcctagcat agtgcctgtt atatgaattc 5520
aaatatttgt tgaataaatg agtgagtgaa tggcagaata agtgaattag tggggctgcc 5580
caagtacccc tgcctttttc taaatatatt gagcccccca ttccttaact tgcttttatc 5640
tttatttgct ccaaggggtt gactgcctga gccctgtctc acaagaattg actctaatct 5700
cttagccttg tttggttctt tacttcctag cctttggcta gcttccaaca tggtggatag 5760
catcttcaca tccatcatct gaaggtatgt taaacagtca ccttcacagt caggaccaac 5820
aaaggcacat ctcaactctg ctgcccgctg gagccctccc actgggtcta ctttcccgga 5880
gagaagtttt cttccccgag tgcctgactt cattgagaat cttgttcttt aggtgacatc 5940
tcacctccta cctgcaggag tgtggctttt tcctactgac ctaaccgttt tatcacagct 6000
gaatactctt aagtttatat aaattttttt tatcctttca tgggaaagtt ttcattctat 6060
catgttatca tgtgcactgg attaggaact tgagtattcg ttctgggtca gctattacct 6120
gtgtggctta gatgcagtaa gtattgaatt gagagcagtt gcagttcagt gcggagttga 6180
gagcatgagc tctaaagtaa tactgcctgg aattcaactg cagcacctac tagctgtgca 6240
gtcttaggaa aattatgtaa tctttatatt cctcactttt ctcatctgta aaatgggggc 6300
agtaatagtg cctgcctcat agaattgtta tgaggattaa attagatgat acgtgtaaaa 6360
cttagaactg tgcctaacac ataatagaca ttgtataaac attagctctt attttgataa 6420
tgatgttgac aatgaaagcc tttttgtgta ttttctgttc tctgttgatt ttgaaagtaa 6480

aggagatgtg tgaggtatgt tctcatgtcc cttctggatt caaataaatc caagagtcta 6540
tgctgctctg tttctgagca cagaattcta agactgtaga ataactcctt aacataaagg 6600
cagacaaatt cagaacaacc tcattctggt aaacaaatcc tttttactct tgcctcatgc 6660
tttaagatga ttcctctggg tccacctcgg tgtctgcatc ttctagatct ccaagtccct 6720
atggtttctc tcatctctgg atttcttttg caccttttac ttcagttcca tctatcttcc 6780
acataattga caggataaat tttcaaaaac atgagtttga ctaggttact ctactgctta 6840
caacctctca gtggcatctt atcaaccgta tgatgaaatc tcagctcctt ggtatggtat 6900
agtatgcaag gcactcagag acaatgctgt cctcagatcc agcctccctg ttagcaccct 6960
ctactccagg aaatctgata gtgcttgtgc ttcttcacaa tcacaaaact accccacgcc 7020
ttcatgctct tgtatgtgcc tcatgcaaac actccttcgt tttttttcaa ctgggtagtt 7080
cctacttgtc ctgtaaggct caactcaggc tttttgtcat caagaaagtt ttctgaccca 7140
agctttcctc cctgtgcatg tccttctacc agtacacgtt tgtcattgag ttgaaatgat 7200
gtaggcacag ctttgtctcc atctgtagac tctgaattcc tccatagcaa atgcaggtct 7260
tagctttctt tgtggtccaa cacggtatct gacacatttt aagcaatcaa tcaatgttga 7320
gctattttgg atctcttgaa caactcgaca ctaggttctg gctgtagcac acaagtgtga 7380
tttgccttta agtgctataa tcccaccata gccagttatg gatcattttt gttacatgac 7440
ttctatgctt ctaaaagtaa ccatcaagga ggaatttcag gaaatgttta tggtgatagt 7500
agcattaaga taataagata tgctgtccat acatattact caaaggatga cattctgtta 7560
agtctcataa agttatagtc atgaagagat tattgaaaaa ctatcctcca gaaagtggac 7620
cccaacttag gcagatctct caggcttaaa gggataggta tccattggta tagtcctaat 7680
ctaacccaac agcaggatag ctaaataagc atatgaaaat ataagtaaag aagaaagtca 7740
tctttgaagc attctattat attttgttga aacaataggc actgttaacc acaaacccct 7800
tttatcacag gcgttcttca taccaaagtt caaagactca cataaagaga caactctgct 7860
gtgtgttcac gttcctctga caagtgcagt aatgagtttc ttagggatac atcttgaact 7920
aatcatcagt gagggctgat agcatgatac aaaaatacaa ttcaggaaaa tccatacagg 7980
cccagccaat gtagcatgag tatgcagtac tctgatgata gtatgcctta attcagataa 8040
tgaagagtag ctgggagagt atatttttct tatttcttgc aacttagctt ttgctacata 8100
ttgcttaaca tagaattatg tatctgatat gcaaagtaca gacattatgt gttattattt 8160
gagggtcaac ctcacaaagg agttttgact gtaatgtcct attcatcaaa gtctatgaca 8220
cataccacta ggctatgctg tgataatgaa caatctcaaa ctcttaatag tttagaacaa 8280
gaaggtttgc ttcgggttca tgacataaat ctatcatgca tcagccaggg cctctggtca 8340
gcatgctggt tgtcctcatt ccaggatcca gacagctaca tggagcattg ctagccatag 8400
gacagatgga aaggagaatg tggcaaagta cacactggct catgcagctc ccaagaaaaa 8460
tcatatggct gttcctaatt ttaagaggaa gaagtggaat ggcagtctaa tcatttattg 8520
cctgcatgga aagagaagaa tatttttgaa cagcactaaa gatgactaca cagagctgag 8580

gagcctcgtt aaaaagtcac cacccttcag aaagaagata gaaccaggaa gcacacacat 8640
tcagagatgt ttctgtctgt aaacagtttg ggggtagata gaaaacacta tctataaaac 8700
ataggtgatt gggagcccaa atgtatatgc aacaaagcag taattttcat gttgtgtttg 8760
tacaattcga gaaatctatg ccatgctgca cagattctac cagcaaaagg aaacataaat 8820
ctgactatag aaatatgttt atagcattct taaaagccaa gctatatatc acaaaggaag 8880
aaaagataat actgtatcag ggaaaatctt ttaaaaaatt taatgacatt gcaacaatga 8940
tctgacatga caatcactct ggtatgttat tttaaaataa aattatcaga gtcatcagtt 9000
aatttctctt tatggcaaag gggaaaagga aaattcaaag ctcctttgac acacaaaaga 9060
agtcaaagat ttatacttcc ggaaatctag gattccagct tgactgttac ccaaggcaat 9120
caatttgaat gcttactaag gaagaaccta caatactaaa gttatattcc ataaaatgta 9180
gccataattt ctgatgtgca ggtttttttt aagttagtca ttttcatgac agaaatttta 9240
taaggacggt tttttaaggt aacaaaaaag gcgggatgat tacgaatgga aatttccacc 9300
tgaattccca tatcagtcaa gggacggtag ttggtgtttt tgtgtgtgtt tggtttccaa 9360
cttcatcata atttttagaa ttagatgcta gaatttaatc tttttattta tttatttatt 9420
ttttttgtaa ctgctgttaa caagtgacca tgagatctgt ggaggaagga aaaggagaac 9480
acttttatct tcagagaggc aatctgcaaa tcagggaaag tcacctccag gaaaactgaa 9540
accacatgct cagaagaggg agagggagag ggaaagctgg cattcctgtc tttcaggtct 9600
gccttccatg catttttagc aggtttgggg aaaattacgg gtacttataa gggaagtcaa 9660
gcacatatgc agtaggcaaa cctatatgtc acatatattc tatgttcatt ttagggtgga 9720
gttttaacat taaaatgaag cggaatttgg ctttttacaa caaaaggtga actagagggt 9780
ataaaggcat tttgtgcaca gtctctgtat gctggcggac gctagcttga ggcctgtagc 9840
tgtttatcag gaaagaatgt ttgcaaggtc agtctcctgt ccaatcagag ttgctgatgg 9900
gaccacaagt cgggggtagg ggaagtcagg tcagctggca tctggcaggt caccagggtc 9960
aattgggaaa ctcctggttc aagctgtttt gataatgttt tttaagtact agtttctgct 10020
tacttacagg aaagaagacc ttattgcaat tagtaatata agggtatttg accagcccct 10080
ctccctgcta tggctgcttg gttctgtttc tggtatgctt tattttagcc acagggagtc 10140
catttagtct gtcagctgca ggtatgtttt aacacgcctt agcatctttt tttctcccac 10200
ttacctgtta agaatttcct tcacaagtag ctactagttg gtaaagtcta tgtgctcaaa 10260
gctattggcc tcgttcaagg attcttagct ttggcactat tgccattttg gcctagataa 10320
ttctttgttg gtggaggggg taggctgtcc tgtgcatggt aggatgttta gcagcatctc 10380
tggcaaccac ccactaaaca ccagaagcac cgcctccctc actgtgatga tcaaaaatgt 10440
ctctggacat tttcacatgt cacctgggag cacaatcgct tctgattgat tgagaactac 10500
atgcttaatt taatcagaat ccacctctgg ggcaatatat agagtaagtg tcccctgaat 10560
atcatggact tcgtgcgcag ggttgtttca gttactatgg ctgcataaca aattacctca 10620

aagcttaggc ataaaacagc tacttattat gcccgtggaa tctgtgggtc aataattctg 10680
acaagctact gcagaaatgg cttctctctg ctcccggatg tctgagcctc ttctggaaga 10740
cttaaaggct taggatgaca tttttctgaa ggtttctcac taagacattt actgttggtg 10800
cttgttcttg gctgggatga ctataatagc tacagctgta actgaaacat atacacctat 10860
gtgtattttc tgtgtggatg tttgacttcc ttacaaaaca tgcccatgtt caaagggcag 10920
gcaatccaag gagaacacaa ccaggtgtaa gctgtattgc cttttatttt atgattttta 10980
ttttttattt tttcgacgca gaatctcgct ctgtcaccca ggctggagtg cagtggtgcg 11040
atctccgctc accacaacct ctacctgggt tcaaaaaatt ctcctgcctc agcctcctga 11100
gtagctggga ctataagtgt gtgccactgc acctggctaa tttttgtatt tttagttgag 11160
acggggtttc accatgttgg ccaggctggt cttgaactcc tgacctcagg tgatccacct 11220
gccctggcct cccaaagtgc tgggattaca ggcatgagcc accgtgccca gcctatattg 11280
ccttttatgg cttaacttcc aaagtcccaa taacatcact tccagtatac tctactggac 11340
gaggcagtta caaggtgcaa ggaggtaagt aagcatcaca tagtaagaag agcatgtggg 11400
atgagacata gatcagtgtg ctcatctttg gaaaatagag tcttccacag agttggacgc 11460
ttgaataaca tctgaattct gttggtaatg aagaagaata attttgttca gatagacaac 11520
caagagtgtt tccgaatgta cctttatttg ataaaatagt tcaaagaaaa ataaaaaagt 11580
atatactata tagctaataa attaattcca aagatgatat tgtataggag cttcacattt 11640
taatagaata gccatgttgt aaacccctta gttttactta ttttagcatg atacgtaaat 11700
gagtacaaag gtgatagtaa tattatttgg cagcttagcc cagtacaatc taattgttta 11760
gatgaattgt ggagaattca tcaaaggact ttgttttatt tatttattta tttattatgt 11820
tttatttcaa tagttttttg gggaacaggt ggtgtttgtt tacatgaata agttttttag 11880
tggtgatttc tgagattttg gtgcacccat cacctgagca gtgtacactg tacccaatgt 11940
gtagtctttt atccctcaac cctgctgcca ccctttctgg gaatctccca ttatatcatt 12000
cttacgcctt tgcatcctcc tagctgagct cccacttatg tgtaagaaca tacaatggtt 12060
ggtttcccat tactgagtta cttcacttag aataatggtc tccaattcca tccgggttgc 12120
tgtgaatgct attattttgt tccttgttat ggctcagtag tattcacagt atgtataaac 12180
cacaatttct ttatccgctc attgcttgat gggcatatgg gcaggttcca tatttttgca 12240
attgcaaatt atgctgctat aaccatgcat gtgcaagtat ctttctcata gaatgacttc 12300
tttttttctg ggtagatacc tggtagtggg attgctagat caaatggtag ttctactttt 12360
gttctttaag gactttccac gctgtttaca atagtggttg tactagttta tattcccacc 12420
agcagtgtag aagtgttccc tttttgccac atccctgcca acatctatta ttttttgaat 12480
ttttggttat ggccattctt gcagaagtaa agtggtatcg cattgtggtt ttgattttta 12540
tttccctgat cattagtgat gttgaacatt tttcatatgt ttgttggcca tttgtatatc 12600
ttcttctgag aattgtctac tcatgtcctt agcccacatt ttgatgggat tatttgtttt 12660
actcttgctg atttgagttt gttgtagatt ccggatatta gtcctttgtc agatgtatag 12720

attgcaaaaa ttttcttcca ctctctgggt tgtctgttta ctctgctgat tgtttctttt 12780
gctatgcaga agcactttag tttaagtccc ctctatttat ctttgttttt gttgcatttg 12840
ctttcgggtt cttggtcatt aagtctttgt ctgagccaat gtctagaagg gcttttccag 12900
tgttatattc tagaattttt atggtttcag gtcttagact taagtctttg atccatcttg 12960
agttttgtat aaggtgagag atgaggatcc agtttcattt ttctacacgt ggcttgccaa 13020
ttatcccagc accctttgtt gaaaagggtg tcctttcccc actttatgtt tttgtttgat 13080
ttgtcgaaga tcagttggct gtaagtattt ggctttattt ctgggttctc tattctgttt 13140
cattggtcta tgtgcctatt tttatgccag taccatgttg ttttggggat tatggcctta 13200
cagtataggt tgaagtcggg taatgtgatg cctccatatt tgttctcttt gcttagtctt 13260
gctttggtta tgcgggctct ttttttggtt ccatatgaat tttaggattg tttttcctag 13320
ttctgtgaag aatgatggtg gtattttgat gagaattgta ttgaatttgt agatcacttt 13380
tggcaggatg gtcattttca caatattgat tccaccaatc catgagcatg aatgtgtttc 13440
catttgtttg tgttgagtat aatttttttc atcagtgttt tgtggttttc cttgtagagg 13500
tcttttacct tcttggttag gtatattcct aggtatttta cttttttgca gctatcataa 13560
aggcagtcga attcttgatt taattatctg cttggtcact gttggaatat agcagagcca 13620
ctgctttgtg tacattaatt ttataacctg aaactttgct gaattaattt aacagttcga 13680
ggagcttttt ggatgagtct taagggtttt ctttgtatac tgtcatatca tcagcaaaca 13740
gcaacaattt gacttcctct ttaccaattt tgatgccctt tatttctttc tcttctctgc 13800
tctggctagg acttccagta ctatgttgaa ttgaagtggt aaaagtggtc agccttgtct 13860
tgtgttccag ttctcagggg gaatgctctc aacttttccc tgttcggtat aatgttggct 13920
gtggatttgt catagatagc ttttattacc ttaaggtatg tccgtcctgt actgactttg 13980
ctgagagttt taatcataaa agaatgctgg attttgtcaa atgctttttc tatgtctatt 14040
gacatgatca tgtgattttt gcttttaata acgtttatgt gctatatcac atttattgac 14100
ttgtggatat taaaccattt ctgcatccct ggtatgaaat ccacttgatc atggtggatt 14160
gtctttttga tatactgttg gattcagtta gctagtattt tattaagaat ttttgcatct 14220
gtgttcatca gggatatttg tctgtagttt tctttttttg ttatgctatt tcctggtttt 14280
ggaattaggt tgatattggc ttcatagaat gattcaggga ggattccctc tttctctatc 14340
ttttgggata gtgtcaatag gatcaattgg taccaattct tctttgaatg tctgatagaa 14400
ttcagctgtg aattcatctg gtcctggatt tttttttttt gttggcaatt ttttaattat 14460
aatttcagtc tcgctgtttg ccattggtct gttcagagtt tctatttctt cctggattaa 14520
tgtaggagaa ttatatattt ccaggaattt gtccatctcc tctaggtttt ctagtttttg 14580
tgcattcata gtagccttga atgatctttt gtatttctgt gttatcagtt gtaatatctt 14640
tcatttctaa ttgagcttat ttggatcttc tctcttgttt tcttggttaa tctcactaac 14700
agtctgtcaa ttttattatc ttttcaaaga actagttttt tgtttcattt atcttttttt 14760

ttttggttca attttattta gcactgctct gatattcgtt gtttcttttc ttctgctgag 14820
tttgggtttg gattgttcct ttttcttcag ttctgtgagg tgtgacctaa aattgcctat 14880
ttgtgctcct tcagactttt gatgtaggca tttaatgcta tgaacttttc tcttaacatt 14940
gcttttgctg tatcccagag gatttgatat gttgtgtcac tattatcctt cagttcaaag 15000
aattttaaaa ttttcatttt gatttcattt ttgacccaat gatcattcag tagcaggtta 15060
tttaatttct atgtatttgc atggctttgg gggttcattt tggagttgat ttccaatatt 15120
attccactgt ggtcagagag agtacttgat ataatatcaa ttgttttaaa tttattggga 15180
cttgtcttgt gtcctatcat atggtctgtc ttggagaatg ttccatgtgc tgatgaatag 15240
aatgtacatt ctgcagttgt caggtacaat gttctaaaca tatctgttaa gtccatttgt 15300
tctaaggtac gtttaaatcc attgtttctt tgttgactat ctcgatgatc taagagctgt 15360
cagtggagta ttcaagtctc ccactattgt gttgcagtct gtctcatttc ttaggtctag 15420
tattgtttta taaatttggg agctccagca ttaggtgcat acatatttaa gattgtgata 15480
ttttcccgtt ggacactttt taaatcatta tataatgtcc ctctgtcttt ttttaactgc 15540
tgttgtttta aagtttgttt tgtcagaaat aagagtagtt actcctgctc acttttggtg 15600
tccctttgca tggagtatct tttatccttg ccctttacct taagtttatg tgagtcctta 15660
tatgttaggt gagtctcgtg agtctcttga agacagcagt tacttggttg gtgaattctt 15720
ttccattctg ccattcttct tagtggagca tttatgccat ttacattcaa tgttagtatt 15780
gagatattga gatgtgaagt actattctat tcctcatgct gtttgttgcc cgaatacctt 15840
ctttttcatt gtgttattgt tatataggcc ctgtgagatt tatgctttaa ggaggtttta 15900
ttttggtgta tttcaaggat ttatttcaag atttagagct ccttttagca gttcttgtag 15960
tgctggcttg gttgtgaatt ctcttagcat ttgtttgtct gtaaaacact gtttctttcc 16020
ttcatttatg aagctttgtt ttgctggata caaaattctt ggctgataac tgtttttgtt 16080
taaggaggct aaaaatggga ccccaatctt ttctagcttg tagggtttct gctgataatt 16140
ctgctgttaa cttgatagat tttcctttat aggttacctg gtgcttttgc ttcatagctc 16200
ttaagatttt ttccttcatc ttgactttaa ataacctaat gactatgtgc ctaggtgatg 16260
atctttttgt gataaatttc ccaggtgttc tttgagcttc ttatatttgg atgtctagat 16320
ctctaacaag gctggggaag ttttcctcaa ttattccctc aaatgttttc caaacttttc 16380
ttccttagga acaccaatta ttcttagctt tggtcattta acataatccc aaacttcttg 16440
gaggctttgt tcatttttta aaaaattttt tgtctttgtt gattaataca aaagccttgt 16500
ctttgagctc tcaagttctt tcttctactt gtttgattct atagctgaga ctttccagtg 16560
cattttgcat ttcgctaagt atgtccttca tttttagaaa ttgtgattgt ttttaattta 16620
tgttatctat ttcttttttt ttttatactt taagttttag ggtacatgtg cacattgtgc 16680
aggttagtta catatgtata catgtgccat gctggtgcgc tgcacccact aactcgtcat 16740
ctagcattag gtatatctcc cagtgctatc cctcccccct ccccccaccc caccacagtc 16800
cccagagtgt gatattcccc ttcctgtgtc catgtgatct cattgttcaa ttcccaccta 16860

tgagtgagaa tatgcggtgt ttggtttttt gttcttgtga tagtttactg agaatgatga 16920
tttccaattt catccatgtc cctacaaagg acatgaactc atcctttttt atggctgcat 16980
agtattccat ggtgtatatg tgccacattt tcttaatcca gtctatcatt gttggacatt 17040
tgggttggtt ccaagtcttt gctattgtga ataatgccac aataaacata cgtgtgcatg 17100
tgtctttata gcagcatgat ttatagtcat ttgggtatat acccagtaat gggatggctg 17160
ggttaaatgg tatttctagt tctagatccc tgaggaatca ccacactgac ttccacaatg 17220
gttgaactag tttacagtcc caccaacagt gtaaaagtgt tcctatttct ccacatcctc 17280
tccagcacct gttgtttcct gactttttaa tgatcaccat tctaactggt gtgagatgat 17340
atctcatagt ggttttgatt tgcatttctc tgatggccag tgatgatgag cattttttca 17400
tgtgtttttt ggctgcataa atgtcttctt ttgagaagtg tctgctcatg tcctttgccc 17460
actttttgat ggggttgttt gtttttttct tgtaaatttg tttgagttca ttgtagattc 17520
tggatattag ccctttgtca gatgagtagg ttgcgaaaat ttttctccat gttgtaggtt 17580
gcctgttcac tctgatggta gtttctttgg ctgtgcagaa gctctttagt ttaattagat 17640
cccatttgtc aattttggct tttgctgcca ttgcttttgg tgttttggac gtgaagtcct 17700
tgcccatgcc tatgtcctga atggtaatgc ctaggttttc ttctagggtt tttatggttt 17760
taggtctaac gtttaaatct ttaatccatc ttgaattgat ttttgtataa ggtgtaagga 17820
agggatccag tttcagcttt ctccatatgg ctagccagtt ttcccagcac catttattaa 17880
atagggaatc ctttccccat tgcttgtttt tctcaggttt gtcaaagatc agatagttgt 17940
aggtatgcgg cgttatttct gagggctctg ttctgttcca ttgatctata tctctgtttt 18000
ggtaccagta ccatgctgtt ttggttactg tagccttgta gtatagtttg aagtcaggta 18060
gtgtgatgcc tccagctttg ttcttttggc ttaggattga cttggcgatg tgggctcttt 18120
tttggttcca tatgaacttt aaagtagttt tttccaattc tgtgaagaaa gtcattggta 18180
gcttgatggg gatggcattg aatctgcaaa ttaccttggg cagtatggcc attttcacga 18240
tattgattct tcctacccat gagcatggaa tgctcttcca tttgtttgta tcctcttttg 18300
tttcattgag cagtggcttg tagttctcct tgaagaggtc cttcacatcc cttgtaagtt 18360
ggattcctag gtattttatt ctctttgaag caattgtgaa tgggagttca ctcatgattt 18420
ggctctctgt ttgtctgttg ttggtgtata agaatgcttg tgatttttgt acattgattt 18480
tgtatcctga gactttgctg aagttgctta tcagcttaag gagattttgg gctgagacaa 18540
tggggttttc tagataaaca atcatgtcgt ctgcaaacag ggacaatttg acttcctctt 18600
ttcctaactg aatacccttt atttccttct tctgcctgat tgccctggcc agaacttcca 18660
acactatgtt gaataggagt ggtgagagag ggcatccctg tcttgtgcca gttttcaaag 18720
ggaatgcttc cagtttttgc ccattcagta tgatataggc tgtgggtttg tcatagacag 18780
ctcttattat tttgaaatac gtcccatcaa tacctaattt attgagagtt tttagcatga 18840
agggttgttg aattttgtca aaggcctttt ctgcatctat tgagataatc atgtggtttt 18900

tgtctttagt tctgtttata tgctggatta catttattga tttgcgtata ttgaaccagc 18960
cttgcatccc agggatgaag cccacttgat catggtggat aagctttttg atgtgctgct 19020
ggattcagtt tgccagtatt ttgttgagga tttttgcatc aatgttcatc aaggatattg 19080
gtctaaaatt ctcttttttg gttgtgtctc tgcctggctt tggtatcaga atgatgctgg 19140
cctcataaaa tgagttaggg aggattccct ctttttctat tgattggaat agtttcagaa 19200
ggaatggtac cagttcctcc ttgtacctct ggtagaattc ggctgtgaat ccatctggtc 19260
ctggactctt tttggttggt aaactattga ttattgccac aatttcagct cctgttattg 19320
gtctattcag agattcaact tcttcctggt ttagtcttgg gagagtgtat gtgtcgagga 19380
atgtatccat ttcttctaga tattctagtt tatttgtgta gaggtgtttg tagtattctc 19440
tgatggtagt ttgtatttct gtgggattgg tggtgatatc ccctttatca ttttttattg 19500
tgtctatttg attcttctct ctttttttct ttattagtct tgctagcggc ctatcaattt 19560
tgttgatcct ttcaaaaaac cagctcctgg attcattgat tttttgaagg gttttttgtg 19620
tctctatttc cttcagttct gctctgattt tagttatttc ttgccttctg ctagcttttg 19680
aatgtgtttg ctcttgcttt tctagttctt ttaattgtgt tgttcgggtg tcaattttag 19740
atctttcctg ctttctcttg tgggcattta gtgctataaa tttccctcta cacactgctt 19800
tgaatgcgtc ccagagattc tggtatgtgg tgtctttgtt ctcattggtt tcaaagaaca 19860
tctttatttc tgccttcatt tcgttatgta cccagtagtc attcaggagc aggttgttca 19920
gtttccatgt agttgagtgg ctttgagtga gattcttaat cctgagttct agtttgattg 19980
cactgtggtc tgagagatag tttgttataa tttctgttct tttacatttg ctgaggagag 20040
ctttacttcc aagtatgtgg tcaattttgg aataggtgtg gtgtggtgct gaaaaaaatg 20100
tatattctgt tgatttgggg tggagagttc tgtagatgtc tattaggtcc gcttggtgca 20160
gagctgagtt caattcctgg gtatccttgt tgactttctg tctcgttgat ctgtctaatg 20220
ttgacagtgg ggtgttaaag tctcccatta ttaatgtgtg ggagtctaag tctctttgta 20280
ggtcactcag gacttgcttt atgaatctgg gtgctcctgt attgggtgca taaatattta 20340
ggatagttag ctcctcttgt tgaattgatc cctttaccat tatgtaatgg ccttctttgt 20400
ctcttttgat ctttgttggt ttaaagtctg ttttatcaga gagtaggatt gcaacccctg 20460
cctttttttg ttttccattt gcttggtaga tcttcctcca tccttttatt ttgagcctat 20520
gtgtgtctct gcacgtgaga tgggtttcct gaatacagca cactggtggg tcttgactct 20580
ttatccaatt tgccagtctg tgtcttttaa ttggagaatt tagtccattt acatttaaag 20640
ttaatattgt tatgtgtgaa tttgatcctg tcattatgat gttagctggt gattttgctc 20700
gttagttgat gcagtttctt cctagtctcg atggtcttta cattttggca tgattttgca 20760
gcagctggta ccggttgttc ctttccatgt ttagcgcttc cttcaggagc tcttttaggg 20820
caggcctggt ggtgacaaaa tctctcagca tttgcttgtc tgtaaagtat tttatttctc 20880
cttcacttac gaagcttagc ttggctggat atgaaattct gggttgaaaa ttcttttctt 20940
taagaatgtt gaatattggc ccccactctc ttctggcttg tagggtttct gctgagagat 21000

ccgctgttag tctgatgggc ttccctttga gggtaacccg acctttctct ctggctgccc 21060
ttaacatttt ttccttcatt tcaactttgg tgaatctgac aattatgtgt cttggagttg 21120
ctcctctcga ggagtatctt tgtggcgttc tctgtatttc ctgaatctga acgttggcct 21180
gccttgctag attggggaag ttctcctgga taatatcctg cagagtgttt tccaacttgg 21240
ttccattctc cgcatcactt tcaggtacac caatcagacg tagatttggt cttttcacat 21300
agtcccatat ttcttggagg ctttgctcat ttctttttat tctttttctt ctaaacttcc 21360
cttctcgctt catttcattc atttcatctt ccattgctga taccctttct tccagttgat 21420
cgcatcggct cctgaggctt ctgcattctt cacgtagttc tcgagccttg gttttcagct 21480
ccatcagctc ctttaagcac ttctctgtat tggttattct agttatacat tcttctaaat 21540
ttttttcaaa gttttcaact tctttgcctt tggtttgaat gtcctcccgt agctcagagt 21600
aatttgatca tctgaagcct tcttctctca gctcgtcaaa gtcattctcc atccagcttt 21660
gttctgttgc tggtgaggaa ctgcgttcct ttggaggagg agaggcgttc tgcgttttag 21720
agtttccagt tttctgttct tgtttttttcc ccatctttgt ggttttatct acttttggtc 21780
tttgatgatg gtgatgtaca gatgggtttt cagtgtggat gtcctttctg tttgttagtt 21840
ttccttctaa cagacaggac cctcagctgc aggtctgttg gaataccctg ccgtgtgagg 21900
tgtcagtgtg cccctgctgg ggggtgcctc ccagttaggc tgctcggggg tcaggggtca 21960
gggacccgct tgaggaggca gtctgcccgt tctcagatct gcagctgcgt gctgggagaa 22020
ccactgctct cttcaaagct gtcagacagg gacagcagag gttactgctg tctttttgtt 22080
tgtctgtgcc ctgcccccag aggtggagcc tacagaggca ggcaggcctc cttgagctgt 22140
ggtgggctcc gcccagttgg agcttcccgg cttctttgtt tacctaagca agcctgggca 22200
atggcgggcg cccctccccc agcctcgctg gcgccttgca gtttgatctc agactgctgt 22260
gctagcaatc agcgagactc tgtgggcgta ggaccctccg agccaggtgt gggatatagt 22320
ctcgtggtgc gccatttcct aagcccgtcg gaaaagcgca gtattcgggc gggagtgacc 22380
cgattttcca ggtgcatccg tcaccccttt ctttgactcg gaaaggaaac tccctgaccc 22440
cttgcgcttc ccaggtgagg caatgcctcg ccctgcttcg gctcgcgcac ggtgcacgca 22500
cccactggcc tgcgcccact gtctggcact ccctagtgag atgaacccgg tacctcagat 22560
ggaaatgcag aaatcacccg tcttctgcgt cgctcacgct gggagctgta ggccggagct 22620
gttcctattc ggccatcttg gctcctccct cctctattgt tatctatttc actgaagatt 22680
tttttccttc ctaacttgca tcatattatt gatttaagtt tgacttcacc tttgtctggt 22740
gcctccttga ttagcttaat agttgacctc ctgaattctt tttctggcaa cttagagatt 22800
tcttcttggt ttggatccat tgctggtgag ccagtgtgat cttttggggg cattaaagaa 22860
ccttgtattg tcatattacc agaattgttt ttctagtttc ttctcatttg gtagactatg 22920
tcagagggaa gatctggggt gaaagagctg ctggtcagta aaacatggaa cgcttcatga 22980
atttgtgtgt catccttgca catgggccat gctaatgcag cacacatcaa atgactttag 23040

tagactgtta tcttgctgat ggtatgaaag caatggcaga aaaaaatcct acagatatta 23100
gtttatatga aaactgctgc caaaaggtat tgaaggagta gtcattgaat cctacataga 23160
caataagaag atgataattt ttcagagttg aggcactgtg ggtgaaagga gataagggcc 23220
cagaaactca cttgcttgat cttctctttt gactgtcata aataacctct gagatgtctc 23280
cacagaggcc agtttaataa ccagtaatta gggtaaaaag cagagaacaa gggatcagaa 23340
ctgagttata attctggtgt agccagttgt gtgacctagc acaagtcact tagtcttttg 23400
gggtctcagc ttcttactat gtaagttaag agtattgaca taggtattct caacagcact 23460
tctcacttct gtcatttctt ggcagtttta gagaaattcc ccaaaatatt tagatccacg 23520
ttttcagcta gttcctgtta accccgttac cctcccagtg cttggcatgg tcagggaatg 23580
taataaatcg cctgctttta aaaagctctg gacatccatc tgagtcctgt tttcaaggaa 23640
attgtaggct tactttttta taaagagaga tattccagca gagtcatggc ctccttttcc 23700
cttgtaaccc tgttgatggc aatgccaact tccatcaaat tctctttggc tttggtaatg 23760
agatatagat gttgggggga aatgactaat gagaaaaggg atctggattt gactttgcct 23820
tggaggaaat tgcatttatg taattgaatt cactataaag acagacctgt gagttgcaga 23880
aggctgaaac acaattgact acttacatac aagtttgaaa actcacaaaa tacgcttata 23940
tattatttat ggatacatat aaatggaaca aacatagaaa acatgtactg gatggcttta 24000
tgtcagactt tttagtgctt gcctctagag aaagtagaag acatgggact gtgaagggga 24060
attaggaagc ttctccttaa tatgggtaaa gttttcagtt ttttaaagac ccatagtgat 24120
tgtaacaaaa tttatacacg tattaatttg tgtggtgtgc agatgtcaag gttatattaa 24180
tctcaatttc tatttttttt aaacttaaaa aaagtaaaat tgtatttaaa agtactttca 24240
agagctgagc tgaactggag aagctgagct gctagatgtc agagtaactg gttaaggcaa 24300
caagccgaaa tggaagaaac agtcaagccc ttcatcactt agtataagca agaggctgaa 24360
ctgaagaaag tgctgtcttc cctgctgttc catttttccc catgggatgg caccatgcac 24420
aagggtcaga tgggtcaaca caggcatggg gaatgtctca ctgcagtggg agccctgaac 24480
aaaaggctcc tgaagtcctg tggatccagg ggttgggaga agggctagaa atggaagagt 24540
actgagtcag agtgaagaaa acatctttaa ggtcctctgt gtggaagaac tcttggatgg 24600
cttggatgga gacagctctg ctaggaatgc gacatgagta agagcatggc tagccaggga 24660
gcctgagtcc ttgactgcag ccccctctag caactgcagt acatcaagac tgtgcaccag 24720
gtggggagga cagagttccc gtaaaaggtt tgccaggtaa agtttttata attgcctgtg 24780
gttgggcctg aaaaatcaga cataggtttt ggtcagtagc tggactcctc gaattctaat 24840
gtttttctgc tacagaaagc tgtgtagctg agaaatagac aatttaatca aaatatttgg 24900
agacttcggt accctacttt ttgtaatgaa tagaacacct aaacagaaga tcaacaagga 24960
aacagtataa accaactaga cctagcagac atctgtagaa tactcctcta agatcaggaa 25020
tcacacaatg atttatactg ttacaacttc tattcaacat tgtgctggag attctagcca 25080
cagcaattag gcaagaaaat gaagaagaag aaaaaaaagg cttttacttt agaaaggaag 25140

aagtaaaact gtctctattt gcagatgaaa cgatcttata gagagaaaac tctaaggaat 25200

gcacaaaaaa aagatcttta gaactaataa gttcagcaaa gttgcagaat acgagatcag 25260

aatataaaag caatttaatt ttcttatacc agcaatgaac aatcctaaaa tgaaattaat 25320

gaaacaattc cattagaaaa agaataaaat atttacaaat ctaataaaag aagtgcaaga 25380

ctatacaatg aaaact 25396

<210> 10
<211> 12904
<212> DNA
<213> Homo sapiens

<220>
<221> misc_feature
<222> (1)..(12904)
<223> chromosome 10 nucleotides 49651428 to 49664331

<400> 10
agcaacaaaa gagaatgtgg acagtcctaa gagtttttgt aaaggaacat cccacccaat 60

ccaaaccctt tatccagcct gggaaggata attccaaaga tttatctttt acaatgaaag 120

aatgattgta aaagcaatgt gtggtattag gctcaaatat ttactgagcc cagtctatgc 180

actcttttct atgtgcttca aaggacataa gcagagggaa gtagctggaa acctagttca 240

gggttaagat aagagcacag ctattcatgt ggcagcatgt gcttaggact gagggacagg 300

cacaggatga tgggagggtg ggcggaggga gatggcaccc aggggatgag agtggcttca 360

gcaaacgctt ctccccagag actttagttg gcctgcagac tttgagtgtg gtttgagatg 420

tctgtggtgg ggcctgtatc tgtcaaggca gagtgtgtat gtttggtaag tggtacttct 480

tttagtcttg tactcccagg cagattccac cttttactcc gggggtgcga actccaacac 540

aggaactgtt aggggagtaa agccaagctg ggggggatgt tggacccctg gagggcacag 600

ctcccaccca gcaccagcca ccttcccccc acagaggtgc aagccagcat ggccagatcg 660

tcccatcttt tgagagaggc cacaagtctg gatttttatg tgacatcttc agacttttca 720

atgctagcag ttaattcaga aactgctgaa atattgtgtg ggtcaaacac agcacgtcta 780

agaggcagac gcagcctcct gcctgctggc atgtggcctc catctttagc cattgacctc 840

tccccacact gacctgggca gcttgtgtcc tgccagaggc tgcctcccac agacttgccc 900

cccgccaggt gaggagggca ctggaagaac cctgagacag gcgtgggtgt agtggggatg 960

gggtatggtg ggcgcagtct ccttgttcca caactcaccc caggccaagt tggagcctat 1020

aaaagctgtc cacaaggtgt tctgccctgg agggctcaca gcttgctttg gtcactctta 1080

gctttccgag gagatccagt gctccctggc cagtcatatc cagtccctgg tgaagtcgga 1140

gaagaaccgc caggtcatgt gcgaagcagg cttgcttggg accctcatgg cctcctgcca 1200

cagggccctg gtcaccagtg gcagccccct ccactcacgc ctcatcagga tctttgagaa 1260

gctcgcttcc caggccattg aaccggatgt gctaaggtac cacatgctgc atattcagtg 1320

ccgccaagct gggcagccat gtcctttgca gggcaggggt tgcacaatgg gcagtggaga 1380

gactgcaggg acagatggag ggcacggctc tgtcctggca ttcaaggctg gagtccatgg 1440
ctgagccttg ggtctgtgcc aggcagtgtg caggtgccca caaagcagac atagccccct 1500
ctaatggatc acgtgggggc tggagggggc tccaaggaag tggagctgca ttccgaggcc 1560
tgtagagttc actagacaaa gagaggaggg aactgggcct ggaaggctct gccgggaggg 1620
gtggggctgg gaggggctga ctgtgtcctg tagggagttg ggcagtaagc atgtagggac 1680
tgcagacatg gaggaggatg gggcagaacc cacctggatc ctccaggggg ctttcctgct 1740
gtcagcctct acatgaagcc acattcctgg agagccatgg cagggagaga aagtgcaaag 1800
gtgcacaggg tgggaagcag ggagcagacg tggaagacaa gagagcagag ggaagggaag 1860
ctactgggga gggcaggagt ctcctatgcc acctggagtg ctgagggcca tgcacagagc 1920
ggggggccct gctgcttata cacaggagca agtgtagagg gcgcacagac agcctttcca 1980
gccagcagac atgggaccca ggggcgggca cgacagctgc cagaggaggc tgctggttat 2040
agtgctgcat gggaaattcc attagaaact tataaacaat tactacaaat ttactttgtg 2100
acattatttt ttgttatgac atccatcttg aggctcctaa acttaccagc tctctggtct 2160
ggtctggctg gcagggcaga gacaccctgt ctccacattg ctttcagaaa gggtgttcca 2220
gtggggctgg gaggtcaggg agctccaggc atctaggaca gttgtcagga taaagttgga 2280
gaacactgtt gctagtaaaa tgtcttcatt ttttcctctt gtatgttatg ttcagacagt 2340
ttctaggtct tggaattccc tcatctctgt cggccacaac aaaaatcctt gattcatctc 2400
acacacacag aggcaaccct gggtgctcag gtgaggacag tggcaagaac ggggcaggtt 2460
aatgggaagt aaaagatgat aggcattcct gctgagggat cctttacaac aggtgggaaa 2520
gcaaacaggt ttaaacatgg ttttgtctgc tgctcaatgc agcaactgag gaaaaagcaa 2580
agaaattcct ggtctctgga gaaagaccat atccagatgg agaaagatga acccccaccc 2640
agacatataa gacagatgat ccctttgggt tccagatcat ggaagcagag acagagtttc 2700
cttcatgaca cagcagtacg tggcgctgca ctagacagag gtgtgcaggg aaggcttctg 2760
tacagctcac agctcaaggg catgtcagag ccttggaggc ccaagatgca ggtggtaccc 2820
ctgtgtcagg cctagtacct ggggaatgtt ggccagggaa ccagcttaga gaaaagaaac 2880
tcgtggacat cttgaatgta agatcacata agacctcaag gaaacaggca ggcccagtgg 2940
agaagcaagg gtgcgggatc aggcttgggg ctgacagctg cggtgggagc ccagagctta 3000
caaggagagg ctgctgtgaa ggaaagtgtg ggctttgtga gcagacacaa agctccagtg 3060
agtaactcca atggggcaag ccagagacag aggtggcagc agtagaatgt gctatgctgg 3120
aaaagggatc tgtgctggaa tgaaagaaaa gtttctagaa ggagcaggtt agcaacacca 3180
ttccttgtta tcagactcag cagggggccc gggctggggg ccagggagcc accctcgctg 3240
gtgcttagga agtacctggt gactgtgcga aactctcttt ctgggctgcg gcagatactt 3300
tggggttatt gtcagaagcg agacagaaat gctttgagca gagacacatc tcttctcttg 3360
cttgctgccc tagggtcaca gactgcacag ggcttggctg aggggccctg gccagctgcc 3420

ccagatgctg ggctgcaccc tggagtcaca caggccccgc agcccttggg ggaatcccag 3480
gattcaacta ctgctcttca gacggcgctg agcctcatct ccatgacctc cccacgcaac 3540
ctgcagcctc agagggcagc cctggcccca tcgtttgtgg aatttgacat gtccgtggaa 3600
ggttatgggt atgacaggct ttcacatatt taaaatgcac tttattaatt ttgcagtgcc 3660
tctgatgaat tataatattg attgcaaact tgtagttccc attgaatctc accactcagc 3720
agaggaaaat gtctgggtca tggacacccg cctactcagc ggcctcccag gggctgtgtc 3780
cccagctatg gagggccaag accctctttt ccctggtgtg gctgcattct ggaacaccct 3840
cccatcctgc ctgcagtgca attccttccc ttaggcatcc taccagcacc agctgcagtc 3900
catccttggg accttatggt ctaaggaggg ttacagtgcc ggcaggaggg tagagtggga 3960
agatgtcatg gcccagacta gctgtgtcag tgcaagagat tggcttgcag tccaatgatc 4020
tgagacaatt ttctatttcc agctgtttgt ttattccaac cctgtccaca gttatgggaa 4080
ccagcactga gtactctgtc tctggaggaa ttgggacagg taggtgtttt tcccagatgg 4140
tttagctctc catcccttcc aaagtatgag tcttcagata gccttgattg cagattttta 4200
cttggtgttt caataaagtg tgagctgtgc tgggcatggt ggctcacacc tgtaatactg 4260
caatttggga ggccaagaca ggaggattgt ttgaggccag gagttccagg ctgcagtgaa 4320
ctatgattga accactgcac tccaacctgg gctacagtgt aagacccagt ttcaaaaaaa 4380
gaaaagcaaa catgagtctt gccttggtcc tgagtatctg cagatgagtt gttcttcaac 4440
tttgctcctc ttttagagca agatgttctg ttcattgtgc atcccaaggg tccagcctat 4500
agctagggac tcaattgaca ttgaaaaatt ggctcatttc gctggtgact gatgtaggct 4560
gagatggaca cattacctgc cttagatctc tctctagaga gaaatatgag ttgggctgat 4620
ggccgttgag aatagaccaa tctttggcaa gtggcttcta tgcacagcac tgcacttagc 4680
tgaaaacacg ctgggtggga cagtgcaagt gcctgatgct gcctgagcca cacgctctgt 4740
taggagaatt gggtttgggg tttctatggg atcatgggtg ctggggagcc attgccttca 4800
tgacgttttt ccatgtgagt tggcaccaag tctgaggaca cagcagctga tcctgcctgt 4860
tctgtgagga caatggacct gtctatatcg ccaaccctgt tatgtgatgg gagggcacat 4920
ggcattccta gattggtgcc ctttcctatc caaggttcag ctgagctagg ccaaggtcct 4980
ctgccccaca gagcacactg cctggctata gatcagctga ccaggagccc acgtgtgacg 5040
gacctcaggg agatcgagat cttctctgct cccttggcaa ggcagcatgc cataatgggg 5100
aggagacaac tggtgattag gcaagacacc cagtaggtgc tgcacctggg ctaaatcagc 5160
accatagtga atctgaacat gcatgctcag cagggcagaa caaagcctga gggcatgcct 5220
gtgttcccac ccaggtgcca ccagaccgtt ccctcctcct ggaggtctga ccttctcctg 5280
ctggttcctg atcagccggc atggagctgc cactgagggc cacccgctgc gcttcctgac 5340
gttggtgcgc cacctggcca ggactgagca accctttgtt tgcttctccg tcagcctctg 5400
cccagacgac ctctccttgg ttgtttctac agaagagaag gagtttcagc ctctgggtaa 5460
ggtgctggga tccaaagcca gtttcatcca catgtggggg aaatggggct aagtcagaag 5520

ctctcaacac aggtgtggtt agaaacctgg tgaccttctg tttctcctca tcctttgaaa 5580
ttgctccctt gggttccata cctggtgaaa ggtctacaat atttgttgtg gttcactgtg 5640
gcctgggctc taggcttgta agtgtcaggc agatgaggcc ccagcccttg gcacagcttc 5700
agagatgatg ctcagtggct tttcagaaac ttcgccctgg cagaacagtc acaatgggac 5760
ccagggcttt gccttgctgg gacacaggct gcatcctctc ctgagtgagg tttgtgaaga 5820
acacacacaa tttagctgga gccgaagcag ctacagacat ataggcgctc ccacatatgc 5880
tcaaccccaa acccagaagg ctggcccatg cttggcatgg cgcccagggc cacccctcct 5940
ttggcagtgc tagaggccag ccctggctct gccctttgga gcccagcgtt gttctccagg 6000
ctcatttgcc tgagatggtc aagtccagtg gattctgtcc acttgttctt ctcctgtgtc 6060
ctgagagtgt cactgtttca tagggaaaaa cattgggagg ccctatgaaa cagtggaagc 6120
ggctgacacc cttgtacaca gcacaactgc ctatccaact ctctgggact cggcctacaa 6180
tggagtctct gtggaggtag tctaccttgg gatagacctg aagcctggct tctgggtttg 6240
aatcctggct gtatgacctt gggcaagtca cccaacctct ctgtgcctca ggatggtaaa 6300
ttgggcatgt atgagcgcct acctcacagg gctgtgaaca tgacatgagt tgatatttgt 6360
aacatgctta aaactggcac aaaggaagag ctcaaagaat gactgctgct attgctgctg 6420
ctgttgtcat ttttcatgag aggaggatct agggctccat ggtacaaggc ccagtggttg 6480
acgtccgcct atgccctccc cattctcctg ggttcctgca gtgtagcacc acacgtgaga 6540
ctcagtgttc atgctgtctt ccagatgtca tggaacctga ggatgactcc gagccttctg 6600
caggatgcca gcttcaggtc aggtgtggcc agctcctggc ttgtggtcag tggcatcact 6660
tggctgtggt tgtcactaag gaaatgaaaa ggcattgtac agtttccacc tgtctggatg 6720
gacaggtcat tggctctgcc aaggtgagat ggctcctcca agctgcactt gccccacaac 6780
catacctcct gctactaccc tgaagtggcc tcggtttcat tctatgtttt tgttgttgtt 6840
tgtttgtttg tttattactg gtagggttgg catctaatga aatctgcctt actgttcagg 6900
aaacttggct gagttcagac atgaaaagga catctttaga aaatgcattg aaggtcacgc 6960
taccagggca gaaggcaaga catgcatcca ggtctctaag aatagtcatg ttcatgaaaa 7020
tagaaaactc cagaatgaac aaagcaaagc ttctcatttt gaggggcaaa ctcctcaggg 7080
aggtctgggc agggcctaag gtatgacttt tccaagaagc tcccaggtga ggccaacgca 7140
gctcatcttt gcaccacacg gaggagcagg acactagagt gggcagacac caacacgtgt 7200
cgtcagattt agagcaagaa ccatgaggaa ggtaatagaa tgcaaatgta tagctggagg 7260
aagcatctca tggctgtcat ttagagacac cccaggtata agagaaagct gggaaaaggt 7320
ggagctggca ggcgccagca gccattgtca gagaagagag ggcccggagg accatagaag 7380
gcaggtaggg ctggattttc acacaagttc cagggagagc atcccaagct ggctgcattt 7440
taaaatcacc tgcaaaggct ttttaaagtt ctcttgcaca ggctgcaccc agaataatta 7500
catcagaatc tctctgggtc aggtaatttg acaacttact aatgtttgta aaattttcta 7560

ggtgattcta atatgcagcc agaatggaga accttacata cacagggttt ctggattccc 7620
cctaaatgcc atagtgttcc tcccctgacc ttgcccagtt gtgaccacca gtaacatctc 7680
cagacatttc caaatatacc ctgggggtca aagttgttcc aggttaagag tcatcagatg 7740
agaacaaatt cttagtccca aattctgtgg gacacatata ggtgaatttg ataccatctg 7800
ctggtaaagt tctgttatca aaaaaaggca gtgatgacca aatatatata tatgtgtgtg 7860
tgtgtgtgta tatatatatg tgtgtgtgtg tatatatata tatgtgtgtg tgtgtgtata 7920
tatatatatg tttttttgag ataggatttc gctcttgtcg cccaggctgg agtgcaatgg 7980
tgcgatctca gctctccgca accactgcct ccaggattca agcgattctc ctttctcaac 8040
ctcctgacta ggtgggatta caagcgccca ccaccatgcc cactaatttt tgtattttta 8100
gtagagacgg ggtttcacca ggctggccag gctggtctga aactcctgac ctcaggcgat 8160
ccacctgcct tgccctccca aagtgctggg ataacaggtg tgagccactg cgcctggctg 8220
accagaaaga ttttgacagg ggcattgaaa gaacaggtcc ttcaaaacat agttaacctt 8280
ttctgcaatt attaacaata tcagtcctcc ctccttccct ttctctcttt cttcttttcc 8340
tcattaaata tttattgagc acctgttatg tgttagctaa tattttaggt gctaagaatt 8400
gtttatgcct ataagctaaa gacctccagg aacatacata tagtataagg tgctaagaat 8460
tgttttaggt tctaagaatt atttatgctt ataagccaaa gacctccagg aacatacata 8520
tagtataaaa taaaaattag atttaaactg actatagcaa gagatttcct atcccatggg 8580
gtagtaagga gttaggagtg gcttgttaca gaaactagat tcattttagg tgatttttaa 8640
aagggtttaa agaagtggag gtctgtctgt tctgaattgg gtgctgctgg aaagaggtgc 8700
aatttggtga ctgtgaatgt taataacttt tatccagaag gtagagacac ggaacagggc 8760
tggacttatc attagagagg aagcagaagt ccctcacatt agttgggatg ggatgtgtgg 8820
tgtcttttgt ggttgcaata tctttgtttt tatctgttta ggctcaatta ctgagttcac 8880
ttgttcttgt cttctttcac agaatatgtt gatattttgt gaaatacttc aaataccttg 8940
aggcatgtct tattgatgtt tgtaccttcc ttgaacttgg cgcaggggct gacacacagt 9000
aagcatggaa taaacacttg atgaactgaa ttgaatgtgc agccacagga ggaacatgac 9060
tggctgctag tgtggttggg tgaagtggta caggcattaa catcctccct gcgggacctg 9120
ctaagcctag ggccatcaac agggaggtca ggagtggtgt gttgctatat ggagtggcca 9180
aagccaagac tgacagagtg cgctttccaa aataatctca acaagttggg tcaggaccag 9240
caagtgaaac tcaaccaaga taaacataaa ggcctgtgtt ttgatgcaaa catctgttgg 9300
acaagagtaa gatgtgagaa cttcagctta atttgactag tttaacagcc cttctcaaaa 9360
tgctgagcct ttgtttttat tgccaaagga aacttacata aatttgtaaa gatatggagc 9420
attttaatag tttgaattgg gcaaaatgat ttgggaacga tatgagacat tagacattta 9480
ttaggcaacc tgaatggggc agtgtgcata cactgggaat ttggaagaca ggagcaaggt 9540
gggttggagg ggtgagtggt gcttcagtgt gggagagaga ggaggactct gtgcctgcga 9600
gaacagagag ataagagagc acagaaaatc atactcctgg caagccttga tggctgaaca 9660

attggtttgt ttttatttga aaatctcact taattggtag agtgaaaaag aatatttctg 9720
aaattctgta aactctcatt tagaaaaata cagtcatccc tcagtttcct caggggattg 9780
gctccaggac ccttcctgga taccaaactg catggatact caagtccctt aaataaaatg 9840
gtgtagtatt tgcaaataat agatgcatac cctcctgcat gcttaactaa gctctagatg 9900
atttataata cctaatacaa tgtgaatgct acataaatag ttgtcatact gtattttttt 9960
atttgtattt tttattgtta tagcactatt ttttattgtt ctttttcctg aatatttttg 10020
agccaaagtt gattgaatcc aaggatatgg aacccccaga tatgcagggc caattgtaca 10080
tatttatata tgtacaattg tatatatctt atgtatatat cttatgtata aaatcaaaga 10140
tgtatatatc aaatgtatat atcttatata tagtgatttt atagtaatta taagaataat 10200
ataaatgcat tttgaaatag ctcaaaaata gagaaaagat tagctaccct cctactactt 10260
tatcatattt tgtaatttca gttttatccc tttaaaaaat agtgtggcct cgaagtatag 10320
aaaatcttgt atactgctta gttcatttaa cctctcattt aacctaacac ttaggctata 10380
tggtatagct tattgctcct aggctataaa cctagacagc atgttgctat actgaatact 10440
gtaggcaact gtaacacagt ggtgaatatt tgtgtatcta aacactctaa acatggaaaa 10500
agtacagtaa aaatatgata ttattatctg atggaccacc attgcatatg tgctctgaca 10560
ctgacttgga acattgtaat gtggcacaca gcaatatatg gaagtatact tggaaattca 10620
tccaaacttt tgcctgtagc cacagttcat tcttttttat ttctgagtag tatttcaagg 10680
tatggatgtg ccatagtttg tttagtcatt tatccattga aagacatctg ggatgtttca 10740
ggttttggct attacaaata aagctttgtg agcatttgtg tacaggtttt tgtgtgaatt 10800
taagtcttca tctatctggg atgaattcca agagttcaat tgctggtttc tatggtaatt 10860
gaatgtttag ttttataaga aactgccaaa ctatttcgaa aatgattgta tcatttgaca 10920
tttccgctaa caatggatga gtgaggcagt ttttctgcac ttagcattgt cactattttt 10980
atattagcca ttttgatagt tgtgtagtaa tatctcactg tgtttttaat ttgtatttcc 11040
ttagtggcta atgaggttga acatcacctt ttcatgtgct tatttgccat ctgtatattc 11100
tcctcagtga tgtccatcca tattttttgc ccattttcta attgcattgt ttgctttttt 11160
tttttttttt tttttttttt ttttgagatg gagtctcact ctgttggcag gctggagtga 11220
agtggtgcaa tctcggctca ctgaaacctc tgcctcctgg gttcaagtga ttcttctgct 11280
tcagaatcca gagttgctgg gactacaggt gcccaccacc atgcctagct aatttttgta 11340
tttttagtag agatgggttt tcactatgtt ggccaggatg gtctcaatct cctgacctcg 11400
tgatctgccc acctcagcct cccaaagtgc tgggattaca ggcatgagcc accacaccca 11460
gttgcatcgt ttactttttt tttttttttt tttttgagac ggagtagtct cgctctgtca 11520
ccccaggctg gagtgcagtg gtgcgatctc ggctcactat aagctccgcc tcctgggttc 11580
gcgccattct cctgcctcag cctccccagt agctgggact acaggcaccc accaccatgc 11640
ctggctaatg tttgttgtgt tttttagtag agacagggtt tcaccatgtt agccaggatg 11700

gtctcgatct cctgacctcg tgatccgcct gcctcggcct cccaaagtgc tgggattaca 11760

gacgtgagcc attgcgcctg gccttaaact gttgagtttt aagggtttta gggttttaaa 11820

atgtatacat atatgtgtat gtgtgtctat atatatacat atatattcta gacactagtc 11880

ctttgttaga tatgtgattt gcgataattc ctcctagtct gtagcatgtc ttttaatcct 11940

ctttcacatg ggtaaaattt ctggttttgc tgaaatataa ttcattagtt tttccttata 12000

tgactcatgc ttttggtgtc gtttgagaat attttgccta gtcctagatc ctgaagatat 12060

tctcctattt ttttccctaa aaattttcta gttttacgtt ttacactaga gtttgcaatc 12120

aattatgaat taatttttgt ataaggtgta aggtttaagt tcatttcttt gcctgtggat 12180

gttcaattgc tccagcacca tttgttgaag agtctatcat ttctcaattg aattgctttt 12240

gcatctttgt caaaaatcag gtgagcattc ttatataagc ttatttctgg gttctctatt 12300

ctgttccatc gatctatgtg gttatcttta taccaatgtc acactatatt gattttatag 12360

ctatataata agtcattatt agagtagttc ctcccccaaa ttaattcttc tttttcagag 12420

ttgtttaaac tattctagga cctgtgcctt cccatataca ttttagaatt agcttgtttg 12480

tgtctacaaa agaccttgct ggaattctga tagtctacaa aaaacctttc tggaattgca 12540

ttatacttac tgatcaactc gaggggaaat aatatcttta ctatagtgaa cctttctatc 12600

catgaacctg gtatgtctct ccatttatat aggtcgtctt ggactttttc catcatcact 12660

taggaatttt cagtatacag atcctctact tgtcttacta agtatttcat tttcttgtgc 12720

ataattgtaa gtggtattgt gtttaatttt agtttctgtt tgtttattgt tagcctacac 12780

aaataaaatt gatttttgtg tattgatcat gtatcctgtg accttgctga attcacttat 12840

tctaggatat taaaaaaaat tccttggaac tttctacatg gacatgatag aaacagtttt 12900

attt 12904

<210> 11
<211> 22388
<212> DNA
<213> Homo sapiens

<220>
<221> misc_feature
<222> (1)..(22388)
<223> chromosome 10 nucleotides 102301095 to 102323482

<400> 11
tcattaatct gaatctgtta ggagacagag aggggaagga tccttatcag tgggccagcc 60

cagtatgggg agacactccc tccctctgcc cccagagact cctgggctac atcctctttc 120

agtattgcca caagtgggca gagcttgtat ttcttaacaa agattaggga cccagttgcc 180

agcctgagat ggatatagga acagacatct ttgggcatga gccaacaaag atagaaatag 240

atgagtgtca ggtacaagct ataaggcaaa gaataacagt ggaaaagttt tgacatgtgt 300

ttgccatttg tggaaaatgt ttttgtaata gtcaacaccc ctcatagccc acctgtgaca 360

agtacttgtt acattccagc actgtagggc gtgaggaaaa tctggtcctt atcaaatccc 420

aggagcttct gcttagttgg ggaagaaatt acatgaagca accagaggtt ataaggccac 480
acttgtatat cgtgcaccct gtgtggacaa gattagggac tgttgagaga ggaggaaacc 540
agtagagagc aaagctctac ccaggctcct tgtaagcctc tgggctcccc cgagagggcc 600
tcgctactct acgcttccta gcaacgttga tgtccccaca accccacatc agtgcagctg 660
tggctgtgtg gaggggctct gaggcctctg aggccagatg tgtaaacagt gctgaggttc 720
agtaatagga tgagtcttca ggtgtggagc agcccacctt ggctcttccc atgtctctgt 780
gttacttctc atattctgct gtcctttcaa acttcaagga cagtattaat ttatactagt 840
atttcttcct cagttttgtg acttgaatgc agtgagtgcc ttagaggatc caaggatgaa 900
ggaatgcggg ttggtggttc tctctttcag aatgggaact tcccaaaaat ggggctgcgt 960
ctcgcctctc agtaggttcc ctacctctgg gtcttccacc cttcaaaatc tggtacagaa 1020
tttagcaggg gctgcaggga atgaccctca gggaccagtt tcacccagat ggggtagatg 1080
atgaggtata cctagaagta gctcagggct ccagagtctg gcctttcagc tggtgtctta 1140
ctcccctttg agaccttcag ctgcttgtct tccactgcct gcatacttct ctgatgtctt 1200
ctcaaactgt tggctctaaa gggatgttcc agcctccctc tccagagctg cctgcagttt 1260
cccttattca gctgtctcct gcctagtctc tccagcggcc tccttaccaa gacccttttt 1320
cctaaacagt tgtccttccc tcagaaactc catggctgca agtgtgaata gacttgtctt 1380
taggatccct ctctctggtc atccctgatc atcttagggc cgtctccctt ttctgatttt 1440
tgacagctct gtttattttg cccatggcta gtcctcttgt tttgtgtctg ctgtgtgttc 1500
ttgcccttcc attgtcagtt ctgtacatcc tggttcagct tgtctcactg tggtagcccc 1560
ttctgcttgt cttcagccat cttggtttaa tcctcatagg tcatggatga atggagcctt 1620
gctgagcact ttgagggcat ttctgttggg cacaacctca gaaacgggaa gtcttgaggc 1680
ctgggggcgt gtggtcagtg aggcctccag aggtgggtta ggggtgggca cctacaaggg 1740
ccagttgagg ggagaggtgc agggattgct gaggagactt tgtgagaatg atgtcaggcc 1800
cgggaagctg gtgtggaaga actgagctgt cagaaggaga taccaatatc ccctttgcag 1860
ggtggtgatg ggcatcaaac atgacgtgga acatgtcccc ttcatggagg gtttccatct 1920
aaatgcatct gtgtgcctta gggatcagta gttcaggggt ggtggacttt tttgagtgtc 1980
tcctaatttc tctgaggtgt ctttggctct gtgtcaccaa atgtgcctga cctctgcatc 2040
ccagtggttg ctgaaaggga aacagtatat tgcgtagtca gatgtcagga gtagagatct 2100
taatgagaaa ggccctggca gctggagaga tgagaggacc tagtggtcag cttataccca 2160
gactatctct ccactcctcc aagagtttga gtcccatagc tgatgaaaga atttacatat 2220
gggctccccc ctgctttccc actgaaaatc tattctcata ttttgggaag gagcatatgt 2280
cctttaactt tcaatggtaa acaatctagg atagtgttta aaggcatgga ttttggagtc 2340
acatatagtt ggtttgaatt tctagcttca ctacttacca gctgggtttg accttggttg 2400
atttacttaa cctctctgag cctcagtttc ctcaactata ataacatggg tggagggagg 2460
catgttgaaa atatctacct cagggttgtt ggaataactg aaacaatgtt tgtaaagctt 2520

tagcacagtg cctggcaagc acttaataaa tggctgtggt ggtggtggtt atatttatta 2580
tgtgatttat tcagaaactt ggtttcagtg atctctaaac agatttccag tggttctttc 2640
tctctcaaga aaagcatagg gaaaaacgag aaacacctta gataaaccat attgagttga 2700
gggtagacag aagagtctag tacctaaact caccctagag gaagagaaac aggaagtgtc 2760
ttatgcctct tctgcctggg cagccagact ttactgctgt actaccagcc cagggccttg 2820
ggtgagaagg tttcaggtct gagctcccct tgcactgtgt gagaggccgt ggagccgaat 2880
gagtccttat tatacctgga gaagctgttg tctcctcaca ccatgaaatg taatagcagt 2940
caaatcaggc ccctcttgat tcaattttag tccatgcctt tccatgtatt taaattatat 3000
aagtgttggc caggtgtggt ggctcacacc tgtaatccca gcacttcggg aggctgaggc 3060
aggaggatca cttgagccca ggagttcgaa ccaaccttgg gcagcatagt gagacccctt 3120
ctcttaataa tttttttgaa aaataatttt ttaaaaatta caaatatgga ccagacacct 3180
gggaggatta cctcacgcct gtaatcccag cactttggga ggctgaggtg ggtggatcac 3240
ctgaggtcag gagtttgaga ctatcctggc caacatggtg aaaccctgtc tctactaaaa 3300
atacaaaaat tagccagatg tggtggtggg tacctgtaat cccagctact cgggaggctg 3360
agacaggaga atcacttgaa cctgggaggc ggaggttgca gtgagctgag atcacgtcat 3420
tgcactccag cctgggtgac tacagtgaga ctccctctca aaaaaaaaaa aaaaaaatta 3480
tgagtatgtt aagattgttc taggggagaa gggcaatcaa aattcagaaa aatatgagtg 3540
ggatgttaat agctaccatt tgttgaacac ttctgttcca ggcactgtat tagttcgtcc 3600
ccatgtggtg ctctcagatc agtaggggag acaggcaggc agttagaatt atggtgtata 3660
gaggagaagc attgcactat gtccagtttt ttatggtagg cttcctgcag aaagtgataa 3720
gtccaagctg atatgtggaa gatgaataat taaccagagg ttgggggatt tcccccttag 3780
cttctctttc ttccagtcca gcagtccaaa acaaacactg cttcccaatg cctaagggtt 3840
ttgtcgtacc ttactccagg gaatgggtga gacagtagat cccaagaaga aaaccctttg 3900
gttagtcaca gaaatctgaa agtcttggtt gctggctcag atttgtcctg gagtggacgt 3960
agattggact tgcttatttg tagccctcca ccgttctcat ttgtgttctg gcctctggag 4020
ccaccctgcc agaccctact tggaagaaat gttgaatcaa gctactcaga cttcagcctg 4080
agatgtatta tggctggctg gaattctcag ccctttccac atcatgatcc cattaatctc 4140
ttgggtctat ccccacgccc agccacctct cctcctcact ataagccaag tctccctgca 4200
gttctctata aatactcaga tcctcttgcc tcagaccctg gtccctagct ttgtgggtga 4260
agaagatacc tctttagctt gtgccaaccc atccctctgg gctacaggcc attctgtagt 4320
caccttgcag tcacgtcttt ccttctagat aactggctct gggccttgga aaccttctct 4380
ttggctctag ctaattttgc tgttttattc cgtttactta aaacctattc acttggtcat 4440
tgaggtcaga actatcagga aagctgtgct ccttaaccca tcatctaagc taagcttggg 4500
ccaggttttt attacctctt gaatatatat agtgcaatat agtgtagtgg ttgagagtgg 4560

agtctgcata cctggatctg ccattctgca tgactttggg caaatggaca aactgcccga 4620
aacttgactt gtttatatgt atggggaaga catcatttac attgtcaggg taaggcttag 4680
cagaatgacc ggtccatagc aagggctcag tctttctgtg tctctcagtc tttacctctc 4740
ttccccatct agctttctgt ctaagttcct gtttctagtc tcagggcata tctagttgtc 4800
agctcctgag cagagcagag gtataagtgt caactcatgc ttgtgttacc agttcctacc 4860
cctgtcagta acctctgatg cttacaagga tactatcacc tatgccatca gtggggaatg 4920
accttccaaa aatattttta acaatactaa aaatacctga aacatactgg gtgaaaataa 4980
gacattggag ctgctgctcc ccaaggagat aaaaggacag ttcctaaaga ggcattgaag 5040
gtttgtatga gccagcagca cagtcagaag gcaagaagga gaccctggga tgtgcacaag 5100
gatgcaggag acgagattgt cacagaggct gtcatgctag taagagtcag aggttactga 5160
tgggtagaga ttgtcagagt ctgccttgct agtctagagg taggaccatg gatttaagaa 5220
cgttgcctcc aagtttttga attgtgaatt tttgatcata tttgaacaaa accccaccta 5280
cagtctgcat ggtcattgtt ctcacaaggg tttgtgtgat gcactgacaa gaacagaggc 5340
tttggaggtg actcctgggt ttgaatcacc atttgccact agctaattct aaccttaggt 5400
aagtcagtgt ctctgggtct caacctcttc ctctgtgagg ggtaggaaat agcacataac 5460
ttgtagcatt gttataaggg ctcgtgataa tgtttttaaa acacctggct caagcactca 5520
ggaaaatgtt ttattatgaa gaccaagtgt ctctgaaaag tgtttcctta gtgtctggag 5580
agactacagc agcaaggctt ttctgatcct actctgactc tcaagataat ggtgcttgaa 5640
gtgaaatcac agaagagatg actggggcag gaaaagcaag atggaaaaga taaagttgtg 5700
gaagggactg aaagtccatg aagtgctggg ctggggacag aaagggttta atagggcagg 5760
gtcttgagga acaggaacag agaccttggt tctgggttgc catctgattc ccagaagagt 5820
aaaggaaatg gggcaggagg atgtctgtgg aggacacagt gtggctgagt ggagagaaca 5880
caagcttctg gccagtgaac ctaagtgaga atgtcagtgg actttgcaca ggctgggtgc 5940
ctttgggggt gttcatctcg ctatgcttcg gtcccctcat ttaaaaaaat tggttaataa 6000
tatgatgacc tgccaatgtg tgtggaaata ctatgtaaac tgagcactat ataaatgctg 6060
gttgttgaga tattgggagt gggggtttcg ggagattttg tttgtttgtt tttttgagat 6120
ggagttttcg ctcttgttgc ccaggctgga gtgcagtggc gtgatcttgg ctcactgcaa 6180
cctccacctc ctccaggttc aagcaattct gcctcagcct cccgagtagc tgggactcag 6240
gcgtgcgcca ccatgcctgg ctaattttgt atttttagta gagatggggt ttctccatgt 6300
tggtcaggct ggtctcgaac tcccgacctc aggtgatctg cctgccttgg cctcccaaag 6360
tgctgggatt acaggtgcga gctaccacac ctggccctgg atttcttaat aggtcctctt 6420
atgtggctat tgagaatgaa tgagacaact cattaactaa ctaagttaat tcagaactta 6480
gtgcctagta agtgctcagt aaatgttagt ttcttttcct ccatttcttc ttaggaagca 6540
tcgtttaccc agcataaaca ggaggactca actgtgcatg tcttagtgtg aacaagtaac 6600
atcagatttc ccttttctga ggagagatca gttctgtaag agggagcatg agtgtgaatg 6660

gggaagaatc atggagaagg gagcaggact ccacagtact ggagatggag tagggacctc 6720
tgggaatcgt tggcatgggg gtgtggtgca gaaagggtga tgctaagagg tgagagtctc 6780
aagaaagaag atgggtgttc tagaggggaa agctaaacga tgataggcaa tgaggagcgg 6840
aggacaagac cctgaactaa gggtgaggag gccagcattc tgtcaggaga accttaggaa 6900
ttgtgaccag aagtcaaagt tgttacagca atcctgcatt caaccaggtt tcatctgaaa 6960
aacttaagag ttgctaggca cagtcgtgca cacttgtaat cccaacactt tgggaggctt 7020
gagtcaggag gaagcttgag gccaggagtt tgagatcagc ctaggcagca tagtgacacc 7080
ccatctcttg aaaaaacaaa cactcttaag agcagtggtt cacctagaaa taaaaagcag 7140
aaatcatcaa gatgagaaga cattttgtgt tttgacagcc aaggaaattt gccactgaga 7200
caaggtatct gacagcctag atgggattct agacttctgt gagaaagtgc agttaggccc 7260
tgaatagcgt caaactcagg gatgtgagct gactgactgc ctttttatgt aatggaggcc 7320
atgtgatagg gctttatgag gccacaaata gcactttctg ggtcctttca cagtctagtc 7380
agtggcccaa ccaacatatc tggttgttct gcaatctcag tcatctgaga agataacctc 7440
caaaatacat gcagtggcat caggtgtttc cttggtaggt gtgcatcttt taatgatgga 7500
ggacttagga tgaagaacag gtgtgtttaa aaacatcaaa cccccacatc attactattt 7560
gctgtaagga tctatgcagt ttgtatttta agtccttgac atatggaaga gcatttgtga 7620
gagtaacagg tttgctttgt aatgccaact aaaaagtgtg tatttatact cgaattttaa 7680
gttgaaattt tgctaagttt cttttttttt tttttgagac ggagtctcac tctgttgccc 7740
aggctggagt acagtggtac aatcattgct aagtctttct gtagttttag aacatttaga 7800
acaacttaag atttcctgta tttataaact taatttattg gaaatataag tacttgatca 7860
ggttttgtct tggtcagata aattaagtac ttaaggaaat caaatacatt aatcatataa 7920
atgccattta agtgtttaaa ggagtttagt taaatgtaag ttggatcaaa cattaaactt 7980
ttttgtttta taagcaaaat aataagattt gttagcagaa cttaaaagct tagaaaagag 8040
aggattttgg atatgtaact ttgaagaggt gaatagtttg ctcttttctg agacagggca 8100
ttgcttttcg acgcaggctg gagtgcagtg gggcaagcat ggctcactgc agcctcaacc 8160
tcctggactc aagtgatcct cctgcctcag cctcccatgt agctgggatc acaggcacat 8220
gccaccacac ctggctaatt ttttattttt tgtagagaca ggggcctcac ttcattgccc 8280
aggcggatct cgaacacctg ggctcaagca gtcctgcctc agcctcccaa agttctggga 8340
ttacaggcat aagccactgt gcctggcctt gaatatattt tttaaccaaa gtaatttatg 8400
aatataaatt gtttctatat aacccaaagt cactctcatt gacattaaga aactcagaga 8460
gtgggttcta aatgtaatat taccacacac ccaagtcact tagcctctac aagtaccacg 8520
taactagcag ccgttaaaag atggccaccg ctagtccagt cggccacaga catggttttg 8580
tttggccaat acaagttttt gaaagtttta aaaaattgac aacattaaag atcagaaact 8640
tgacctaaaa agatttctct ctttggaaaa tcagatctgg cagtaatgaa atcgcctttc 8700

ccatgtgaca gcagttggct ggagctgaag agcaactgcc cccttggctg tgttcctttc 8760
agtttgtcaa tctccactct tcactgttgt accctagatc tcactcacgt gacctgtctg 8820
gcccacatag atatttgagg gtatggccct gaacttgagg cactctggag ttgctaacaa 8880
ctgatgctgg gcagcctaga ataagggctc aggaaaagtg taggccctac ctctctctgt 8940
tcacatcatt cccccctcca gccaggctgt cttcccagct gacctggctc caggactgat 9000
tccaacccct atagcaactg tgtaagaagt ccccaggctc aggcttctca gtgttcactg 9060
ctcttgtctg tgaccaggca ctttgcctag gctctgacct catgtctcag tccttttgaa 9120
cattctggtc acctgcctag tttgaccgca gcctgacttt ggtctgtgag gggtcctggg 9180
ctctctgctt tatgcagcca ccatatctga tctttgtgat gtggttgtga atcttctgct 9240
tctatctcca ccatcactgt ccactgcttg ggcaatatgg ttgctcagct gagagattgg 9300
cagcccagat tgctaaaggc ttaggagaca tgaaaggtga gtttggaagt gtttggattt 9360
tcaaaagaag gacagaggca gaagccaacc acagtttaag ggggcagcta gtagagagct 9420
ttttaggcaa gggaggccac tgcacattta taggcagaga aggtggcagg acagagaatt 9480
taagagaatg aggtctcaga ggaggcatag gatgatggga tatgaaatag caaaggcaca 9540
cccaaggtga tcctgaagct cagaaatatt aagtcatgca ggagggggca ccattggaaa 9600
tataatcccc taaagttatg agtaaacaaa tgatggtggg gtgtggaggg tagaaagaag 9660
gcctaagctt gatgtggggg ccacagcctg ggcaagagaa atttccagca aagcctctga 9720
caaatttctg acaaagcctc tcatgttatc tagttgtggc agaagcatct gaaccaaatg 9780
gctaggaagc ttggtgaaca gtcagaccca gtgagggttg attaatggat ccatgtcagc 9840
ctagactaga gggaagtctc cagcagtatg ccttagggct cagtctgggc ctgtgtcctc 9900
tctgacattt ttattgacaa ctgaccgaag cagcaggctc atcaagtttg caggtgatag 9960
agctaggagg ggcagttaat actttgcatg aaagagtcag gattcaaaac gatttcaaca 10020
ggctggaaca ctgggctgga accaacaaga ttaaacttaa cagggataaa tgtagagtcc 10080
tgcacttagg ttaaaacaaa atcaatgaca taagtaccgg ttgtaggggt tggcccagct 10140
tgacagcagt tcatgaagaa aagacctggg gggtttagtt gactgcaagc tcaatatgag 10200
tcaactgtgt gatgtgactg ctaaaatcct aacacagcca tgaattgcat taaccaaacg 10260
gagcagccag atcaagggag gtgttacccc actgctttct gagatggtca ggccacctct 10320
gagcactggg tccaattctg aggtcttttc ccctgctaag gtcagtgaca aatgagaata 10380
tttttgatag gaagccacca tggcagtggg cctgaatact gtccgagact tgccagtgat 10440
tctatggtgc tgaaatttct ggaatgaagg acaggagtct agaatctgaa cccaggcaaa 10500
gacttttatc tggagcatat ttctaggatt taggggaaaa tttggggtct gcatttcctc 10560
acttcctatg gctgcccaga ggcattggcc tgtgactctc atccctggcc ttcctgaggt 10620
caggggatta gcgattagtt agacggggtc tagggtagag gtcagcctga gggggcggct 10680
ataaaacgag tcattgaggt gggagggaac ctggaaattg ggacctcacg caccatattt 10740
cccgcgctct cccctgggac gtgccgtccc tgctgcagtc gcagcgtggc gccctcgctc 10800

cacgcaggga tcctgagcca atggcggaag cggagtgcgc ggcgaggtcg cagttgcgtt 10860
tctcgcactc cccactgcgg ggagcccgtg ggccccgcac tgcagggaag ctgccggccc 10920
aggtctcctg ggtggcaaga agacgcctgt ggtgtggaag aacctcgccc gcagcccagc 10980
acgtgtagaa tccagatgtg gcttctgctg gagccacgtg ttccggcctg agcgacgtcg 11040
cacgtggcct cctggccgca gagcccatgg cgcggggggc cactcttctg acgcaagggt 11100
ctgtggggcc gcgcaagagc ctggggcccc cgaccttccg ccgactggcc gtgggctggc 11160
cgcagcccag ctcggctctg tctcgagttc cttcgctgtt caaagcagag tggcggccac 11220
gttcgggggc actggggcga ggggtcgggc gtcggacggg gccctgggtc tgtaccgttt 11280
cgggctcgcc ggaggggctg caggcagggc gggtttcggg ttccaccgcc agaataattc 11340
ggcagttctg gatggggccc aggaatttgt atttgtgacc agtttcccag gtgagtctga 11400
ggtccgtggt cctctgacct cgtttaggga tgagatctag tcctcagagc ccggggcggg 11460
gctgccgagg acaccggctt gctcttcagg cagctctggc ccgggtggcg ggaggaggcg 11520
gccggggctg ggatccgggc gcaccctgga agctccagat gggcgttgga gacctagggc 11580
gagatgaacc gaggaccgtc catgtgtgtg ggtgccgctc aggagcgggg aggtgggggt 11640
gtgggtggac gggcgtgttg agggtgatgg attgctgcag ggagtggact cagcctactg 11700
ggaggagtgg gagaggggaa agaactggag gaacggcgga gtccgttggc cttttcttgg 11760
agagctggag atggaggttg gttctgtcct gatgggggca ggaggacggg taatggaggc 11820
tgccatgctc agtgaagccc catgggggca gccatgcttt gtggtttgtg gtgaggcact 11880
gagccccagg atactgacta ttcccttttt tggagaaaga ggggaagagg atgctcctta 11940
agagcttcta ggggaacgtt tctgcctcca gggggtttag gtgaactgtg tgtgtgtgtg 12000
tgtgtgtgtg tgtgtgtgtg tgtgtgtgtg tgtgtgtagg ttagcatatt ggagattttc 12060
aagggagttc gtctccagga cacttccctc acccctacat ccacacacag tatcttatga 12120
tcctcttggt ttctcttggg cctcaactcc ccaaaagggg ggcactcttg tcagcagctc 12180
acaaacatgt ggatatctgc taactgaaga cactctgaag actttcccag aggaacgtta 12240
tagaaggaac cctcaaattc caaatccttg aatattggag ctggaagaga acttacaaac 12300
catctgatgc tgtcttcccc agcttggtct tggagagtag acaccttaag tgaaggccga 12360
gcttcgtaag cctctgagga tcccagtacc tggtgtggag acaggacttg tgcattgcct 12420
agagaaggga gagacacagc agagtcaagc ggtgggtggt gagctttgat ggtgaacacc 12480
tgggagctca gaggtgggta ggagcctata aaagaaggcc ccttgaagga gggaagctag 12540
agctgcatct ttaaatgggg aaaatttatc ttttgtgttt ttccccctga ttatgaattt 12600
atgaaaatga agaaaaaata tattggggcc gggtgtggtg gctcacacct gtaatcccag 12660
cactttggga agccaaggca ggcagatcac ttgtggccag gagtttgaga ccagcctggc 12720
cagcatggtg aaaccctgtc tctactgaaa atacaaaaat tagctaggtg tggtgatgca 12780
tgcctgtaat cccagctact tgggaggctg gggcacgaga attgcttgaa cccaggaggt 12840

ggaggttgca gtgagccaag attgcgccac tgcactccag cctgggcaac agagcaagac 12900
tctgtctcca aaatatgtat atatggatgg aacactgtga catgttggtc ttcaggggag 12960
caaagctaca gaggcctgaa tttcctgagg tttggagggc agtgaagaga tgaagaggac 13020
agctctgacc attgttggag cacagtgagc tcgtagggca gaggggtctg gctgcaacag 13080
aaagtatctg gcaacaacag gaggtatgag gctggaaccc aggggaaaga aacagggttg 13140
ggctcagctt catagaggta gtgaggatta ggcagatgag aacacccaag gagaaaagga 13200
gagaaaagag ggtttgggcg gggggtgggt aggtgtgaac atgcctgaga gtcatttccc 13260
aggtaaatag gagtggaact gagggtggag ttcagtgaga gggagggagg aagaacgaca 13320
caggaggggt gggaaccagg gtcacctctg aagaggggtt tgagtgcagg agacaggagt 13380
gaggccctag tgaaggtcat tagatttggg gatgaagagg tcagcagaat ggtaggggtt 13440
agagggtgag ttggtggaga ggaaatgggg gcatagcaag tataaactgc tataggttat 13500
ccgttgagaa gtacacagag gactggagac aggttggggt tttgaaaagg aaactgcagc 13560
aacgcttttt tagccagggg aagaggagca aatctgtaga cagaggacat aggagaaggg 13620
gccactggag agggggagat ggaagctacc agagagagca gacacttgaa ggagctgagt 13680
tccagaggga aggggggaca gggcctgcag gaggttgaag cctcagagaa atggggagaa 13740
ggaggggaag tcatggggtt caacctggac ccaggttttg atctcctaaa agtaagagta 13800
aaggttgagg ttggcttctt caggtaaggg tggccagggt gaggctgggc tggacggaca 13860
cagagggagg ggtggaaatg ctgttgtgaa gaatttgttt gggagtcaaa ggagcaatct 13920
aggcgtgtga tgtactgcct ggtagggcgg aggtgcagtg cagctgggct gggatttgcc 13980
ttgggtgaag ctgatgctgc atcttggggc aggatggagt aagcaagcca tggtctgacc 14040
tgattacaaa tgataggctc agtgccaggc tgaggggcct aggggccagg acacgatgag 14100
gacagtgagg ggcctccctg gggaagggag ggacaactgc cagagcctgg cccaggaggg 14160
cccgatagac gagtatgggc agccatgcag tgtgtggggt caagagcctg gaccctggac 14220
tcaagagcct gagttcaaat gtgaggcatt gtcattgtca tgatttttaa gtatctgagg 14280
taggctatga gtggagggaa ggacaggagg aggtacagaa agggagtgct tgcttcatgc 14340
caaggagtgt tctatattca cgagtccttt ctaaaccacc gcgaactagg tactgtttta 14400
tcattttaca aattaggaaa ttgaggttca gagaagggaa gtaacttggc caaggtcaca 14460
cagccagttg gtgacagttg gttttcgaaa ggcttttttt ctgttgcttt gataacagtc 14520
ccttctgaga agtggctcca aagtctgact ttctgctatg ccttttcatc agctacttac 14580
taggtaggtg accttgggca agttacttaa tttctccctc aattttctca tctgaaaaac 14640
aggaataata gtaacactat ctcatagggt tgtaatgata atgaatgaat atatgtagaa 14700
gatttgaaaa aaaaaacagt gtctagtaca tagaaagtgc tgcttaagag ggtgctgcca 14760
ttgccagcat cctcctcctc ctcctccttc tcctcctcat catcatcact ccttacagcc 14820
tccagccaga agctaaggac ttacaggcag gtgaaagaaa gtcctgaggc atattagtca 14880
tttagagaag gtcccatggg gaaaggcaag agagtagcat ccaaccagtg gttctcaaca 14940

agggacattt ttgctgtaac agtgtcctgg aggaactact gacttttttt gtgggggtag 15000
ggagagggag taatggggtg gggcagcagg gaattactga cttttggtgg caggagccag 15060
gagtgttaaa agtggccatt catacccagt tgagagtgtg gagaagttca gctggaaagg 15120
gtgcttggga tttaactcct cagaagagtt gcagctccag ctattggagc agctggaaaa 15180
ggtatggaaa tgagcttgga ggtctgtgag gggctggaaa gaatcagact taggaggggg 15240
caaggcttcc tggaggggag ggtgggggtg aggcaggctg agggatctgg acagtaagca 15300
ggggcaatgg agagccatgg ggttctggat gagggacatg atgtatttgg ggagatcact 15360
catgcagcca gggaagaggt tgggttagag agggataaaa agggggccca tttggggatt 15420
gggaacaacc cctaaggcgg taaagacacc tgggctggtc tggggtttgg ggttggagca 15480
tgcagataaa agaccacact gaggcacatt tgaaggaaaa ctccgtgtga agatgaagga 15540
gtggtgagtg ggctgggctg gccctagcag gtgcatgacc tgggaaggaa ttctgggaga 15600
gatgggctgg tcggggactc aaggtgatca cagtgaattt caggtgatgc tggggagtct 15660
gggagctgtc tagtgagggg ctagtgatgc cagttggaag ttaggactga agtaccaatt 15720
tgggagtcat ttttccacat ctattgacac tcaatacaca tataaaatag ggccccttgg 15780
agggtcattc ctatggggtt gctagttgaa tttgaagtct tgcttgcaga cgaatttgtc 15840
ataggaaaaa gcggaggggt aaagcactgg aaacctgcat cctaagcctg gctgggcccc 15900
ttctgcatag cgagggagtg aggagagagc tgctgggaga gtcagcgaga ggttggagaa 15960
ctaggagagg gcattgccag cgctgccggc tgcagggaac aggcctagga gctggcagag 16020
tagcagggtg gaagccagga acagtggagc tgctgagggt gaagatgact caggggcggg 16080
aggaatttgg gagttcaagg agagtggacc gagccgtggt tcaggaggca agttgaagag 16140
ggtgtgtaca tgagaacggg gcggtggtgg ggtgcggaaa cactgaggcc tgcagagggg 16200
aagggagcag gagaagagcg tttccctgaa ctagggttca cagggaacca cccccaacct 16260
cagccaacct caggtccaaa gtagtggatt tctccaggct ctattctctg acttccacct 16320
ccccgcaaca cacacacttg acccccaatt tgaggctatt tcctctgccc tggcctcaag 16380
gaacatggag aaaaatagcc tgtgtcctca ctcatctggc tccaggggat tccctgcctc 16440
ctctgccctc agcattccct tccctaggag aaagctctgc tcccgtaagg tgacctgact 16500
ttagagtttt tcctatttgc catcctcagt gcatatgggc tttcaaacag tgtgtgggcc 16560
accctgcatc tcccacttcc cccagtcatc atctgccact gctattcagg cccaactagg 16620
cttttcttgg ctgttgcttt gatcacagtc ccttctgaga agtggcgccc cgactccctc 16680
ccccaccagc tccctggtct ctttgagtct gtcaccggag ttaggaatct gcatgggatg 16740
ttgaaaactc ctctcctcta ggaatcctcc tccaattggg ctcattcaat tcttattgct 16800
cctttcctcg gttgagtctg cagatatgga gtccacttag ataactttgc agataaaaag 16860
cagctcgtgc tctgctatct gactgggaac catttttgcc tctattgcat tgtaatattc 16920
tgtaagggaa ctagagctca cctagtccag gagtttccaa ggatattttt aaccttagaa 16980

ccttcaattc tgaatgtaca cacacacaca cacacacaca cacacacaca cacacacaca 17040
tacacacaga gagagagaga gagagagaga gagagaaaag cagaaatacg ttggctgaaa 17100
tttacccttg ctctaacctt tacccatagc agccctagac acccctgaga acccttagag 17160
ctccatggag cacattctga aagcccaatt tcttcactta cagatgagaa aggcccagag 17220
aagtgaaggg actctcccaa ggtcacacag caggagcaaa aactcaggat tcccccaatt 17280
cccaattcta gcattctagg acttattaac atccagccaa gctctccctc tagtgttttt 17340
taaaaatgta atttgtgttt ttcattcatt cacacagcag atatttactg agcacctact 17400
atgtgcccag tacacagtct gcccttaaga ctattaggta caagtcctag gagtcaatgg 17460
gaagcaaaaa cagacactgt ctgccctcat agggctttca gtctagtggt agggactgaa 17520
aaacttcatg tgtgagaagt gccacaaagg agagatcaat ggtatgataa agagggctca 17580
ttaatggggg atttaactta gttagaaagt aacccacagc tgaaagctaa aggtaaacta 17640
ggagaagaga gaagagtgtt ccaacagatc cagcagcagc acatgcaaag cccctgtggc 17700
agcagggagt gagatggggt gaaggggtga gagaaggctg gtgtggctgg agctccagga 17760
gccatggcca gatgggcagg caggggccac ataccagatg ggctgcgcag gctgttaagt 17820
agtgctgggg ctttattcta ggattagatt tgtgtttcaa aaagatgtct ccagttgcaa 17880
agggtaactg aatgccctgt ccccaaccct gacggaggtg ggttcctttc attccgtctc 17940
cttcttttgg tctgcttctc tgactagtcg tgttctgctt gggacattct tagcagggct 18000
tgtttccact ttttgtgctg tatctatttc agctgtaagg tacatatgtg tgaatcttag 18060
tctgtgaatg tattgccact gtatcattca ataaatatta agttattaag tgccgctatg 18120
aagcagatac tgtcaaatta tcattatggg caacgaacat catgagtgat tgtgtgtctg 18180
tgtgtgcaac gttggtgaat tgtgtcgtgt gtgaatgtgt taatcatgtg ctctaagtga 18240
ctgaccacat gggtagctgt gttcttgtat ggttgttggt gtaagtctgt tgtaggtgtc 18300
attacgtgtg actgtcatag tgggttttgt gtgtgacttg atatgacagc atcacgtggc 18360
tgaggtgatg tgtggttgtg tgtgttactg tgcagtgtga atgtacgact gtcagcatgt 18420
gtgccctgac cgggtgacac ttggtgggtg tgaagcgtct gtgggtggca ccggagaccc 18480
catctcctgc tgtaaatccg gagctgaatg agctcccccc agactgttgg gggcgctctt 18540
catggcccct gggatgatta atgcaggcag tggagcctct ctgcatggga tgttggagat 18600
caggaacctc ccccgcagag ctagagggga gatgggggca acctgggggg ccagggctgg 18660
ccagacgggg ccagattaat aaagagggac ttgggggagg gaaggccagg gcgggcggct 18720
gcggcgggag gcggccgaga tgaaagtgtc ccggctggcg ggcaggcagg tggcctctcc 18780
tggccctgtc agcacttttt aatgtcccct tggttagaat gtgtgggagg agtggggcga 18840
gggaagcaag agctcacctt tcccaggccc tggaggagcg ggccgggtca gctgctagaa 18900
gtgggttttt ttggtggggc gagaggcacg cgtaggtgta actgagagga agagcctggg 18960
ccatgccacc tccctctccc actaaaacgt caccttagct cagtctgtct tggacaggca 19020
ggatagggca gcagctgggg aaacagaaaa gcagaacgaa cgtagaaagg gatggctgtg 19080

tgtgtgtgtg tattactgtc cagggtgtgg actggacgtg cgtaaattaa tgcatgtgag 19140
gccgagtgcc taagaatgtg tgagcaaggc agtatgtgtg tgtgtgtgtg tgtgtgtgtg 19200
tacgtgtgta ttcagctctc tgtgtgtggg aatttaaatg gtaaagcaag agactgggcc 19260
ctggcttggc ctgctgttca tgttcgactt cgggtagggc ctttatccgc cctgtgctgt 19320
gttgcttgtc tctaagtcga gataagggtg tctgtttcac aggatgagtg tgagtcttca 19380
gtcggtggaa atgtctgact gtaagtctct aagtatgcaa tgtcgttgtt aataattgaa 19440
tgcgggtgtg ttgggtgact gtgtgtgatg atgtggatgt gtctgggggc ctggggctct 19500
ttgtgggtca gtcacagtgg gtgtctgggt gagaggacaa ggctgttagt gtgagctctg 19560
tctgctgtga gtccatcgtg agtggacagg tgtgtagatg tgtgtttggc agttgtattg 19620
aattatgtgg atgtattggt gtggatccgg aagtggtgag tgtctttgtg tgcttgtgaa 19680
tgtctgtgtc cttatcattg tgtgtaacta tttgcactgg attcctttgt gtctcttggg 19740
gtacatctga gttgccctgg agactgagat ggagtgtgtg ctggctctgt ggggggtgtg 19800
tggggatggg gttggtgtgt ctttgtgccc tgtgtgtgtc ccaacatgag tgtgactgaa 19860
tgtgcctttg tctcagtgtt gtgagtgtga tggcatgtgt ggtgccttgg tgtggctgtg 19920
tgtggctgtg tgtgactgtg tgtgacagcg tgtgtgtgcg tgtgtgtgtt tggtgagcgg 19980
ctctgctgcc ttaagcacct ttcgaataca ttggccacag ctcattgaat cctttaacaa 20040
tccgactctg ctcttccttc cccgtttttt ctaattcagt tcctcttatt acaaaaataa 20100
aaaataaagc gctgcccctt tccagtggct ggccctacgg agcccccgcc tcgggaccag 20160
cctcgtttca ctccatttcg gagaaaacgg gaagtcggta attttaaaaa atattttcta 20220
ctttatttgg gatttaatgt tcttacagaa tcctgctgcc tccctctgcc ccccgaccca 20280
tccaccccca gccccgcatg caagaggcgt aatggcccgt tcagcatatt aggggagctg 20340
agcgcgcgtg tgtgtgcaca cttggataaa aacacacaga gacccgcagc agacacacat 20400
acaaatacac agcagctcaa acgtatactc agacacgaac tcaagcactc tacccttatg 20460
ccactcaata ttcacactga aacaccatac aaagacagag acccattccc agatactcca 20520
aagggaaaga gaaccaggag cactgtcaga ggagaacctc tggagacatg aaggaggcag 20580
gtggtcagag ataggaggaa ggagagagac atgttcacgg tgagagacag tttggaagca 20640
acttctatgc tgtaaaggaa tgtgaattga gtagtccggg ggaatgagtc aggaggttga 20700
tccctgggcc aggtgtgccc cattttgtac aagggaacac tgtatggtca agaaatgatt 20760
actcaaggtc atgcagggag tcgagaagac gtggggctgg gtgcagtggc tcacacctgt 20820
aatcccagca ctttgggagg ccaaggaggg cggatcacct gagaccagga gtgtgagacc 20880
agccttgcca acatggtgaa accccgtctc tactaaaaat acaaaaatta gctgggcgtg 20940
gtggcggggg cctgtaattc caactactca ggaggctgag gcaggagaat tgcttgaacc 21000
caggaggtgg aggttgcagt gagccaagat cgtgccactg ccctccagcc tgggtgacaa 21060
gagcgaaact ctgtctcaga aaaaaaaaaa aaaaaaaggt caggtgcagt ggctcacgcc 21120

tgtagtccca gcactttggg aggccgaggt gggtggatca caaggtcaag agtttgagac 21180

cagcctggcc gacatggtga aaccccgtct ctactaaaaa tacaacaatt agctgggcat 21240

ggtggcatgt gcttgtagtt ccagctactc gggaggctga ggcaggagga tcacttgaac 21300

ctgggaggca gaggttgcag tgagcagaga tcgtgccact gcactccagc ctgggtgaca 21360

gagtgagact ccctccccct cccccaaaaa aaagagtaga tgtgggaaca gaaataagga 21420

atcctgtgcc ctcctatggg ggcctcaggg cactaaacac tgtgtgtatg tgtgtgtttt 21480

tgtgtgtgta agatgtgctt ttgaacgacg ctggcttaag tgccatccac atactaggat 21540

caacaattgt ttgcagaggc tggcagtacc tgacgcggca gtgtatgtct gcaaatgtat 21600

atttgaggac ttgtgtgtgc acacgcatgg gggggatcat gcttgtccta ttcacttccg 21660

tatccccatc acttaacaaa gggctcggca catagtactc aatcaatatt tatccaagca 21720

tgaatgaatg aatagaaacc ctcagggatc tcaggtatag gagggcctga tcactggtag 21780

agaccgtggc agctcagcct ctgggtgggg cctgggaagg tgagacatag taactctggc 21840

caacttgctg ccatcgctgt tgctatgagg ggccctgtgg tctgtgctct gtctaaggta 21900

atgagtttca tgagttattt cagaccattc tgtgtggcgg gcatggggag ggggaaggaa 21960

acaaaagacc tgtccacttg ttaaacatgg gacaggtaag ggagagaagg gcagtagtag 22020

acggaaactc aagatggata gatagcgaca gataggcata ctgacgaaag acaccaactg 22080

gtggatatat ttatttctag atagaccaaa agacaaatag agacatagat aaatagatgt 22140

acagatagac acacagactg acatgaatag gcaggcaggc agacagtcct gaggagtgac 22200

tgagagatag accaacagac aggcagacaa atcaatagac agacagatgg acagctggtt 22260

gcacagagaa gatggatgcg tagctcagga cacagaatgg agccagctct gctgatggcc 22320

taccccacag ccaggcccca cattccatcg ttctttgtta aggaacccac tttcgtgggt 22380

ttgaagtc 22388

<210> 12
<211> 17306
<212> DNA
<213> Homo sapiens

<220>
<221> misc_feature
<222> (1)..(17306)
<223> chromosome 11 nucleotides 10581465 to 10598770

<400> 12
tttggaagac aatttttcca caaacctggt gggggcgatg gtttggggat gaaactcttc 60

cacctctgat cattagattc ttataaggag ggtacaacct agatccttcg catgcacagt 120

ttacaatagg gttcgtgacc ctatgggaat cgaatgctgc tgctgatctg acaggcggcg 180

gagctcagga ggtcatgctc tcaggcagct cacctcctac tgtgcagcct ggttcctaac 240

aggccatggt ttggtaccgg tttgcggcct ggcccagggg atgcggacca ctgggctaaa 300

tgtctgtgcc ccccacaaag tcatctgctg acttttgggg ggacatatcc taactcctca 360

atgtgatagt gaagtggggc ctgggcgagg tgattaggtg aggagagtgg agccccacga 420
atggaattag tgctcttata aaagggaccc cagagagttc tgttctcttt ccaccatgta 480
agatataatg gaaaggtggt agtctgcaag ccagagatgg ccctcaccag aacccgacca 540
tgctggtgcc ctgatcttgg ccttccagcc tccagaacta tgagaaataa atgtttgcca 600
tttaagccac ccagtctaag gccctttgtt acagcaattc aaactgacta agacacacac 660
caagcctaga ctcagctctc atgaaagcag ctctgggccc catccaggct gagagaagtg 720
gcctctgcag ggcctgggcc acagtttctt ctggtgcttg gagaagccca atgtgaacac 780
gctatccctg gcgcctgaaa gtaggagtca cacccaggcc accctgcccc cttctgagaa 840
gctcccaagg ctttggagga cctgtccaca gccctgaggg aaccaggcct ttgggatctc 900
tgcctcagtt ccccccaact ctctgaggaa cactgtcaga gtcttggctc aatttttcac 960
ttcaggagac tatacttggt acctgagaga agcatgacac cctgtatggc ccttggggac 1020
agtctctgct ccagggattc ctcacatcag gctccacaat taccgtaatg gaagctttga 1080
agttttccag ttctttctca gtgttctcct ctgtcaggtt gcgttccctt tcagcctggt 1140
taattctaga ttccagagtg tagctgtcat ttctaaaggc caaggacagt tgcacaaaca 1200
cgttctgttg ggaacaaggg tgtgagagag gtgctgggag gagttactgc agaggacaag 1260
gctgcagctg tgctccttgc acgagcgttt tgcaacggcc cctggaacag ccgccaaagc 1320
accaaagcag ccatgtgcaa gggaaacgct cacttcacag gaaaccacaa aaaagatggt 1380
aaagcaggga ccaaggtcca cgctaggccc ttcccagaac cggaggtcca aatgtattct 1440
accaaaaaat aagaagagaa taacactttg ctttttcctg aattttaaga aaataacatc 1500
ctgtatctgg gtttagataa actcaggcca gtacagctaa ttgtgtaagc atctggattt 1560
atgctgggat ttattgcatg aggggttttg gagattttgc aggaaaatac tgtattgaaa 1620
gaaactccat gtgagattta agcattgttg acgtaaagca aacttcaatc agcatctagc 1680
ccgagtattc caaatcctgt attgatagaa gctttaagta gctgccagga gattaaataa 1740
ggccaggtat gattctgttc ggttaagctt tctggaataa ataaatgttt catgctaaaa 1800
ttgcagcctg catgaacaga ctttgaaaag ataatattgg tcatacatgg aattcccaga 1860
acagtaggaa actactaggg agaagaaata gacagatgga cctcactcag gaaggaagca 1920
atggtcttca attttacagc catcatctgc cctgagtggt agcagattta taccctgcag 1980
ggttagtaac cacaggggct tctgaagttc atgtaacatg gcccatggaa ggcagtcatc 2040
tccagaagaa ttgaaggtac agaattgacc caatgatgat gatgaaggca atattgaaaa 2100
ctactcctta ttgagcatct actatatgcc agcactgctt ttcattctac aaattcatgt 2160
aatctcacaa taaccctctg gggtgggaac tgttatacct gtttatgggt aaagaggcca 2220
agaccagaac ggtcattaac ttgttcaagg tgacacggag taacggagcc aggatttaac 2280
ctcagactgt ctgatttgaa agcctgtttt atttccacca tgccgagagc tgtgtccata 2340
tacatctgcg gtcagcattt ggacattaaa caaaccgccc atggcccatc ttcacaaatg 2400
tcagtgggta ggcttagagc tcatagccca gtgatagaaa ttttctgcct tacccggcct 2460

tggtcaagtc tatgaataat gctcaatgct taattttcta taccttgatt atccccttaa 2520
cagcagtgtg acacaatgag ttctgaattc agtgagaaca cttggatcta ggtcttggtt 2580
ttgccaacta cagtatctag aaacttggtg tggttattta ctcactcagt tttctccttt 2640
gtgctgattc ctgctccacc tgcttttcag agctgctggt taggatcagc tgaaaggatg 2700
gctatgcgtg tgttatagac tgcaagtgca atgcctgtgt gcaagttata gatactacct 2760
gtggtagata cttcatactg tctgtcttcc tctgcttatc tggtccacat cagcagttct 2820
cattgtctca cctctgataa gttctctttg tccgctacag aatgaaatgc agattcccct 2880
cagctcactc acaaccccgc tgggacaagg aggccccagt gaggaaaccg gaaggctgga 2940
ttgctgacag cacagcttcc cggctccgct gagctggcat tcttgtagct gaaaccctga 3000
ggaggccagc cccctttccc actaaactaa agcttagaac ttgaaatctg accatttttc 3060
agtaattttg aaataaattt gattcaattc tgcagcctcc cagctggttt tgtgtatcta 3120
agtcgtacat ttaaagaata taaacaatcc ttacaggaat gtaaaatttc caactacctt 3180
ctcccttttg cccaaatttg tcaaaatctt ccatgaacgg acgtgggaat ggggatctca 3240
tggttcaaga gggctggtca tgaatgttca cattacccca cagtcactct ttctcatttt 3300
tgtctaaaaa tgtcagagct agagtctgga ggaagaaatg ttttgaataa gcccagtcaa 3360
gcacgggcac taaatttcat aacacacttg agtcacttac ctcaacttcc ttttcagtga 3420
gtggaggggc actgtgaaaa agaaaacaca caattgaact gtgtgcaacc tagtcaatag 3480
acccaaaggt gactctgaat gaccagcaga ccatgtgttt ccaggggtgg agtaagctgt 3540
gttggtgaga aggaaaagat gcaatgtccc cttgcctgtg ctctgtcacc tgtgagatac 3600
tgactcttac ggcaccatga ctatctctgt gactttggac aagttattta atctctctaa 3660
gccccagcgt gctcatctgc aaaatgggaa tcatatcacc taccttgcag aggtattata 3720
agcactatat gtactcatgt atgtatgtgt atgtgtttgt atgcataaat atatggcatc 3780
cacactcagt gggctctcgg gaaatgtgac cttgaactca tagccttgct gtgagaaata 3840
agaaaatgca tgtgataagg actttataac tcatacacat ctaagttgcc attataattg 3900
ttctggggat atccacccag gtctgcaaag cctcaccctc ctctgtctct gagggctgtg 3960
acaaaggtgc atgcgggcca gagaataaat ggtaggagag agtgaccagg gttcttctcc 4020
acagcaagaa cttctgatgg ctagaatcac agagtggcaa gaaagcccca gccgacacag 4080
aggcatcctt ttgagcagct cctcgtctgt ggcgttccca cagccagggc tctccagcta 4140
taggcagtcc ctgcatctgc atgtccactc gatgctgctg tggctgctgc tcttgctggt 4200
aactaaagct ggccccatct gaagtccaca caatgtaaat aatgcacagg ccctttcctg 4260
tgctggggcg gggaggcctg ggaggggtcc taccagcgtc acagaacttg gcaacagagc 4320
atgccatcaa taagcttctc aggaaacaca gcttcctctc ttgaacgggt gtgaagggac 4380
cccaagagca aaggtagggg tcagccatct ctgcaggggg ctggttctca cccctaggga 4440
atgatatgag tgccttcctg tcaggatgag aagaccccat ggccatgaag tcagcggagt 4500

ccagaacacg ggacaaacag ctgcagggat gtttgacact tacagtcctt gtcacaaccc 4560
cacccatcag gcccttgcct taactgtcat ctacagcata cacatatgat ttaaatgatt 4620
tggcaaaact tgccaagttt gtttctgact cctgggtttg ttgggatatt atgtgaaatg 4680
cacatatttt cataaattat aaaccaaaca tttttctatt tcaatgactc acattctgct 4740
tttttgctgt agaacacacc caccttattt ttattttatt tatttatttt tggggacagg 4800
gtctcgctct gctgcccagg ctggagtgca gtggcaggat cactgcagag gtggcttact 4860
acagccttga actcctgggt tcaagcaatc ctcctgcctc agccaccaga gtagctggga 4920
ctatgggtgc atgccactat acctggctaa tttttatttt ttattttttg tagaaacaga 4980
gtttcactct gttgtccagg ctggtcttaa actcttggcc tcaagtgatc ctcttgcctt 5040
gagcctacaa agtgctagga ttacaggtgt atgagccacc acacccagtt agaacatacc 5100
caccttaaaa aaaaagtggg ggtctggggg gaaactctga ggactcagag aagtatgatt 5160
taggcagaga agccacatgg tatgtctggt gtctgatgca gataactgtg ccttacaaat 5220
gggcataggt acccgggggc agagctgtga gcctctaaaa ggacacagca tcttttttaa 5280
gtgactattt gtatatcttc tctgaagaaa tgtctattta aatcctttcc ccattttgaa 5340
aaattggtta tttatctttt tattgagtgt aagccttctt tatatgctct agaaacaagt 5400
tccttttcag ataagtgatt tgcaaatatt tcattctgtg ggttgccttt cactttcttg 5460
acactgtttg cagcacagaa gtttttattt tgaggaagta ctatttattt ttaaaatctt 5520
gtctcttgtg tttttggtgc catatctaaa aaacccaaag acatgaagat ttacttatat 5580
tttttctttg aagagtctta tagtgttctc tctgacattt aggtctatga tccattatga 5640
gttaatttgt gtgttttgtg tgaggtaggg gtccaacttc actcttttgt atgtggctat 5700
ccagttgtcc cagcaccgtt tgttaaaatt acaatttttt tcctctgtaa atcttaccaa 5760
aacccttgca tgagtggctt tttgtcattt gttatttgca catctcaaat tattacttta 5820
tcacagaggc ctttccttga cctcctaccc aatgccccac tctcatgtca ttctctagat 5880
gtgccctatt ttatggtctt tctaccaact ttactctttc aaatcattca tttggttact 5940
taaatgttat ttgtgctttg ggaggccgag gtagaaggat cacttgaggc caggagttca 6000
agattatctt gggcaacata gcaagaccct atctctacaa aaaaatattt gaaaacttaa 6060
ctagtcatgg gtaaatggtg tatctgggat tgaaacccat gtctcctggc tcaaagccca 6120
ttctctgctg ccatgagctt ctggtgaccc ttatgccttc atgaactgtg ctcacctaag 6180
aaccccacct ggcctttgag ttgaaaacac ccagcctggc ctttcattat tctttttgtt 6240
tcgaatgcat tatccctatc tagacaattg gactttggcc cttctgggtt caagaattaa 6300
gactggtgtt cctctgtgtc ccacctagaa tccaacacag gggccactcg gtacagggcc 6360
ttctgtaacc cacatcctga ttttaccttc cagggagact cctgtggacc cgcagtttgc 6420
gcagcagcac atcagaaata ttaggcatga catctaagcc actctttgac tcttcttcct 6480
caatagctgg ggagagttca gaaggaagcc ctgaaaaaaa agtgcttact tataaaatgt 6540
ctctttgaaa tcacagtagt actgttggca gctagcttca ggagctttga catttataag 6600

tatctagttc tatgttaagg gctttttaat tgttatctta cattattctc acaatgagct 6660
agaacttagt catcccattt cacatgtaga taagccaagg ctcagcaagg ttaagtcact 6720
tgctcaagat tacagaacta gcaaatgaca tggccagacc tcaaggtcta cctgcttcaa 6780
atgttatgtt cttccctttg cttcccacag tattaagctg gcaattggaa gctggttggc 6840
tctttggcta taatgtttgg gaaaaaaacc tgctccttgg caggcttctc aggagacctg 6900
ttgttcaatt attcctcctc ggataaatta aaatgttcta tattatgtat cctacttaga 6960
aattaacatc tgattccata ccccatagat aagagatttt ccagagtaaa cctaaggttg 7020
ctagatcttt tacagatctc agaaacataa atgcctccta gtcaacagag acaatgaaca 7080
gctttttgtt ttttaaactt ctaaaactat cttgggtctg tgctttgaag acagaatgaa 7140
caaaccaagt attctctttt ccataaatct ttctcctgtt tgaaaacagc tcctgtgcct 7200
ctctaaagct tctcttctcc agactctagc tccatttttc caacaagctc actgtgctcc 7260
actgtggccc atggtctcta gataccatcc cattcctgca cacattctgg tttgttgctg 7320
ttacagaggc ccagatgttc tctaacaaga tcttagcaca ggaagacttt ggtaaatgtc 7380
cctggcattg atgtttgtta ttcatgcagc atagattttg tgtgcctgac tacgggctta 7440
cagtacactt agcattaagc aaaactctca acctctttag ttgacccaaa tcagttatcc 7500
tcatcctgta taatggttac tttccatttg catgtagaac ttcatattta tccatgtttt 7560
actctttctc ctggatttta acctggtagc tttccaatct gctcaatcat tttccaggat 7620
cctaggctag gctagagacc aggactatag gcatagttgt agccacctga agagttaaga 7680
ctgggaatca tgtgaataga aagaagtctc accaaaggag accatgaaga gacaagaggc 7740
tatctagttt ggctcaaggt aaaatatgtt taaatacttt gagcttgcca gtaagagaat 7800
ggtaggcatg gattccttgg tactcaaggt agccaagtaa gctggaggat catctgtcag 7860
agatgggtca gtcagcaatt cttctttgga tatgagattc aactagaagt tctcttagct 7920
acctgttacc tctctggttc tgtcctggta tttatagctc tctgacaagg tttctgtggt 7980
aaaccttgtg tcacccctgg catcattcta cctttatgta tgtatcccta ccacactcaa 8040
cagagagtca actgagtagc aagctcctga gatatctata ggatgatcga aacatatgag 8100
gtctaataat caagtgaaat gcatatcagg gtttttgcct agaaaagttt cccaaaatgg 8160
tgcttgaaga gtaatgccag aattggtgga ttctaggaac caaccacctt cctcctgaat 8220
tcaaccaaat taacaacaaa aaatttcaga acaaataaaa aatacacaag aaactctaag 8280
atacaagaaa aggcaatagt ctttaaaaat agtgactaga gaaagaataa aaattactag 8340
gaataaatgt agagcttcca cccatgataa ccctttcttc ttctccaaag aatcagtact 8400
gaaaggaaga tgggagagaa aaaattctta caaagagttc aataccacag aatagagagg 8460
gaaagggatg gagggggcaa gtaggcagtt gtcagaggag attcagaaag tttccctaga 8520
atagaaggtt ctccattcca tcttaccagc atttcgaaga gagaaaaaac tgcctgggct 8580
gcctgccatt ttgcttttct tactgatgag aagaagagag gaaaggcaga atgatcgcac 8640

agaacacaga gtggattttc ttgaatgata tgaagggatt tacaaggaag gaagactaac 8700
ggtaaagtaa ataaaacacg tccctctgaa ggagtgaatg gcaggagaca catggaaagc 8760
cccaaccacc atgttctccc ccgactcctg aaaaacagtg gctaaactcc ttggatttgc 8820
cgttttttcc ttcctttctt agcagaggag tagccgttgg gaagcattgg gtcaagtctg 8880
gaaaataaac ctacggacag cttgtctcta caattccaac ctaaattggt gaaacaatct 8940
gaaacccact actaaccacc tccaaaggaa taagatatgc ccccgatttg gtataacaaa 9000
ctcacgtttt ggactatgaa cagagttacg gaaaaagaga agaaaccaat tctacaaaca 9060
gctctatctt aacttcccct tttccccacc acctaagcag taacaaaaca aacacaaaat 9120
atacaatcct caactaaagc actaaaggga aggctaagag atcccagcta aggtaggaag 9180
gagtcaacaa aaactaacct acaatacaat atatctaggg aaagggaatc aaggtagaaa 9240
tatacaaatc agaaaaaaat aaaaggaaaa cagatggcat ggcaactata caaaaatact 9300
atagacaaaa gaggaaggaa ggtagaaagg aaggaaaagg gaaatgtgca agagacagat 9360
gtcaggatgg aaacatagtt gcaagaaaca catcaaaaaa tgcaaaagta tgcttctgtg 9420
caaaatatat accataaata ctttgactct agaaaaaata acctgaaaaa aaattaagaa 9480
attagagttg ggatgagaag atcatgcaaa gatggaagat gattgctatt taaggaaaca 9540
aattaaagac caaaataaaa ggactaatca agaaattggg aaatatacaa ccaaccaatt 9600
tattggaaaa atgaaggaat aaagcagata tagcttcaag ctgaattaga ggagggtggt 9660
agagattgag taatcataac gaatgcaaag aaggaaagaa agattgaagt atatggagat 9720
aaaataatgg acctggagtt gagactctga tgacccatct atgcattatg ttgtctccga 9780
agcagtgaac aaaacaaatg gaacaaagga catattcaaa gatataacag aagaaaaatg 9840
ttcctcactt gaggggaaag ttgaaccctt caggttgaca gaccgcatca tgttaaaaaa 9900
tatatttaaa tgcttcatac aaaaaaaaaa aaaaaaaccc agacatctac atgggaaagt 9960
gaagattaag aggtgctgga aatcagctgg tcccagcttt ctccataact ttaaatacag 10020
gggagcaaca tctacgcagt tccaaggaaa acagtcaacc aagaattttg taaccacaca 10080
agctgtcctt caagaaaaga caaaattttc aaatgtgata gaactcaggg aacacagcac 10140
ctctgaactc tttcaggaaa attgttggaa aacaaaatcc agctaaacaa gacataaatc 10200
gaataaattt gggaaaggga ccagtggtga acatgaaatc tttttacatt taagtctaag 10260
gctaaataac tgtggaaacc atggttacag aatatggaat gtaaatgtta taaccttaat 10320
gatgtacaaa gaagaattca agatgtgtag aggtagaaat ggaaatgtag gaagaaatgt 10380
gaaggtggga agatattcgg ccattctcat cttttcctgc aggaagtcag taggtaccat 10440
tgtagtgact acatgttctt atcttccgta ttcttgatgt tctgtcactt ggtgccttaa 10500
tcctggagtg acagcccctc tcaaggctag ctaattccta gaaacaactc tccttcaagt 10560
gtgtctttga aatacaaacc agccaatcca gaacccacac ctgtaaacat cacctttatc 10620
aaattctcac gcaccaagcc aatactcccc ctaaatcacc ccagggccag tacctgacaa 10680
ctacgggcca ccctatagcc taggtcctgc caaaattatt caaactagcc aaccctaaac 10740

atactgagga tacctaccct gcctctacct ttcctttcca agaaaacccc agtaaaggct 10800
tggggccatc tctcccctca ccacttctgc cttctgaccc accctagccc ttccccaagg 10860
ggctctgtat gatgtgccac acatcctgct attagggatc tgtgagtata aacctattcc 10920
ttcatgagaa tcatttgtgg gtctctgtgt cataccatac ctgattaaaa caaatgccag 10980
gtccaaagtt tagattaaat ttaaaaccta taatgcaaaa aaactacctc ataatttata 11040
gatttcatgc catccccatc aagctaccaa tgactttctt cacagaattg gaaaaaatac 11100
tttaaagttc atgtggaacc gaaaaagagc ctgcattgcc aagacaatcc taagccaaaa 11160
gaacaaagct ggaggcatca tgctgtctga cttcaaacta tactacaagg ctacagtaac 11220
caaaacagca tggtactggt accaaaacag agatatagac caatggaaca gaacaaagcc 11280
ctcagaaata ataccacaca tctacaacca tctgatcttt gacaaacctg acaaaaacaa 11340
gaaatgggga aaggagtcct atttaataaa tggtgctggg aaaactggct agccatatgt 11400
agaaagctga aactggattc cttccttaca ccttatacaa aaattaattc aagatcaatt 11460
aaggacttca atgttagacc taaaacctaa aaaccctaga agaaaaccta ggcaatacca 11520
ttcaggacat aggcatgggc aaggacttca tgtctaaaac accaaaagca atggcaacaa 11580
aagccaaaat tgacaaatgg gatctaatta aagagcttct gcacagcaaa agaaactacc 11640
atcagagtga acaggcaacc tacagaacag gagaaaattt ttgcaatcta ctcatctgac 11700
aaagggctaa tatccagaat ctacaaagaa ctcaaacaaa tttacaggaa aaaaacaacc 11760
ccatcaaaaa gtgggcaaag gatatgaaca gacacttctc aaaagaagac atttatgcag 11820
ccaacagaca catggaaaaa tgctcattat cactggccat cagagaaatg caaatcaaaa 11880
ccacaatgag ataccatctc accccagtta gaatggcgat cattaaaaag tcaggaaaca 11940
acaggtgctg gagaggatat ggagaaatag gaacactttt acactgttgg tgggactatg 12000
aactggttca accattgtgg aagacagtgt ggcgattcct caaggatcta gaagtagaaa 12060
taccatttga cccagccatc ccattactgg atatataccc aaaggattat aaatcatgct 12120
gttataaaga cacatgcaca cgtatgttta ttgcagcact attcacaata gcaaagactt 12180
ggaaccaacc caaatgtcca tcaatgatag actggattaa gaaaatgtgg cacatataca 12240
ccatggaata ctatgcaccc ataaaaaagg atgagttcat gtcctttgta gggacatgga 12300
tgaagctgga aaccatcatt ctcagcaaac tatcgcaggg acaaaaaacc aaacaccgca 12360
tgttctcact cataggcggg aattgaacaa tgagaacact tggacacagg aaggggaaca 12420
tcacacaccg gggcctgtgg tggggtccgg gggatggggg agggatagca ttagcagata 12480
tacctaatgt aaatgacgag ttaatgggtg cagcacacca acatggcaca tgtatacata 12540
tgtaacaaac ttgcacgttg tgcacatgta ccttagaact taaagtataa taataaaaaa 12600
aagtatatta ctaagtgaaa aaagcaaggt ttggaacacc ttgtataata tactacgatt 12660
tatgtttaaa aaatccactt ttggctgggc gcggtggctc tcgcctgtaa tctcagccct 12720
ttgggaggcc gaggcgggcg gatcacgagg tcaggacatc gagaccatcc tggctaacgt 12780

ggtgaaaccc tagctctact aaaaatacaa aaaattagcc cggcgtggtg gcgagcgcct 12840
gtagtcccgg ccgaggaagg agagcggcgt gaatccggga ggtggagctt gcagtgagct 12900
gagatggcac cactgcactc cagcctgggt gacagtgaga ctccatctca aaaaaaaaaa 12960
aaaagaaaaa tccactttta catatatgtc tatatttaga aatgcttgga atatatctag 13020
aagttacact aaaacatagt gacagtggtt gcctctgcta ctgggggaca gagatgtaat 13080
gagacttact ttttcctatg tctcattttg tactttttga attttgtact atggacatga 13140
ttatttataa aaattgaatt aaaactcata ccaaagcaaa aacaaaaaac aaaaaacaaa 13200
aaacaaaaaa ctacctcagt aactccaaac cctttatgca tttcataata cattttttat 13260
acttttagaa accaatttct cttctttgga ggaagcagtt accaaaattc agcaagtctt 13320
atagtttcac ttcaagttca attaaattct aatagtttta atttcaaaca agtatatcta 13380
gtataatctc cctctttttc ccttcctcct tctgtattag tggggaagga ggtgtttagt 13440
gtaagctatt tttactttct tccttgtatt tttcactatt ggttgaattt ttagatatga 13500
acataagcca ctttcaacaa aaagtaataa atttattttc aaatatatct cccaggacta 13560
gctcattcca atgaaaatat ttgtttctga ccatgtcagg gacttggaaa aattactttt 13620
ggagctctga aagatgctct gagtcccagg atggttccta aaagtcaaag ctgctagttg 13680
gggataaact tttttttacc tctttcttct cagggaggcc tctttcctgc ccaagacctg 13740
aggctctgag tctagactgg gggttttgga aggactgtcc attcgatgta cttctctgcc 13800
tcagcctacg tgtccctcaa aatccaggtc cagatagcca actaccttct agacgttgcc 13860
acctggttaa ggagaataaa aatcatttca agagccacca tttactgaac atcaactatg 13920
cagtagatac actttgtcat ttcttacaat agtactgcaa ataaaaaata gcttaaacac 13980
ctccttcccc acaaatacag aaggagcaag ggaaaaagag ggagattata ctagatatac 14040
ttttttgaaa ttaaaagtat tagagtttaa ttgaacttga agtgaagtga aactgtagga 14100
cctgctgaat tttggtaatt gcttcctcca aagaagagaa attggtttct gaaagtacaa 14160
agaatgtatt atgaaatgca ttttctggag aaactgaagc tcagctgagt taaagtgatt 14220
tactagggtc cagcttatca gcagtacagc cagaatgcaa aaccaggttt gtctgttgca 14280
ttcaagctgc tttccgtggg catttgacat gtccaaactc aaattcactg tcttccttct 14340
caagcaaact cctcctcctg tctcacaatt catcaccctc acaaccatgc agacaggtgc 14400
ctggtggtat tcctggtctg cctctcaact ccatctcctc atcaagtcct gacaagctga 14460
tctccaatag ctgtcaaact atcctgtcca catttactac cacctcatta cctctcacca 14520
ggctatttcc ctatcctttt acctggtgtc tccctgtctc tagcctctgc tgtttcaggc 14580
cagctctcac aatctgtcca gaagaattat tctaaaatat agatttgatc ctgatgcttc 14640
cttacctgca ggcttctctt tccaaagtgt cctaaatgaa attcaaactc attaattggc 14700
cttcaaagcc ttcaatttcc ttggtctcag cccactgttc ccaccttgtt gcccatctct 14760
ctgggacgtg tagctcacac ttagttcaca tgacattcct tctgttcctg aagtatgcca 14820
agcactagaa gatgcctaca tgcttttgct tctgctgtcc ccctgcttgg gatgactttc 14880

tcttccccag ctcaaggtgg ctcctcctga aaaccttccc tgactcaacc ccactgagct 14940
agctgcttct tcctctttgt tcccaggtac tttatggcct gttaaagttt tatcacaatt 15000
tatcgtagtg actttgtcta ttcttatgtt tcctctccta gacagtgagc ttattcgttc 15060
atacatttgt ttaatgttta ttgagtgttt actatgtccc tggcaatgag ctgggagcta 15120
aggatacaat gctaagcaaa actgacatgc cacatcctct ggagctcatt attcagggga 15180
acagagatta atcagagaaa catccaaaca atttcacact gagagatttt acaaaggaga 15240
agggatccga aggtaccagc aaacccttcc caagaaaagt gacatgagca gatatctaaa 15300
gattgagttg ggagttcatt aggcaaagag ggagagagga agaacattcc atgcagaaga 15360
cagaatgtgc catgcgtaag aagaggaaag aatgagctgc cgaaggcagg ccaaaatatt 15420
gtgagtgaga gtgagtgtgg ccctagaata tgctggggaa gtgggtgggg ttaactattc 15480
agggccttgt aggttctact gaggattcaa gtgctcttcc taagagcaga atgaagcctc 15540
tgaagatgct gaaacagatt tgccttctag agatccctgc ggctgctgtg ttggccatgg 15600
tgtgagctgg gttgggggtt gggagtgcag ggagagggga ccaggttgca ggctgttgag 15660
ttagccaagg ggactgataa tggtagctgg gacaaggcct tctccctgat ggtggggcct 15720
gaaccctttt cacttaatgc atccccagca cacagcctaa gaactggccc acagtcagtc 15780
tcacatattt ggcttgaaat aacctgcaac tgatcaaaga tcaaaacagc atacaatata 15840
gttcctgaga agggaactga ttgggttgtt ggaaagacta gatgcagcac agaaatgagc 15900
ctcccagtcc tcacaaactg aaggagagcc aaaattcttc agactccaga atgttctgat 15960
aagtatcttt tcccatttct caaaggaaca gccaacaacc atcataagag actggatttt 16020
tccccatgag ttcatatgca gggctgtatt tagtgcctat aaatacaatc tgagccagct 16080
ctcataaaat gctcccaaag ccttttatcc tgggtatggg aggggacggg gctctttgat 16140
ggggctttca gaatataaga aaagaaagtc acaagtctgc aagtattcat taaacacgga 16200
gggttgccca ccacatctct agcactgtgg agcacagtgt acaagaatat cccatttggt 16260
tggcaagata gagttctaca acctaaatga cagaaatgaa aatgtatgcc aggttcctgc 16320
tggaataaag accagtatca catgcaaaca ttacagcagc ttagtgaagt aggtactgtt 16380
attaacctcc tttaaaagat gaacaaactg aagcgtggag agaattagaa ccttgaccaa 16440
ggctatgcag ctggtaagtg atgaagtctg agctcttaag catccagacc ctagaactga 16500
gagaaaatga gagggagtgt atattgttgg acactgacca tggttaattt tatttttaaa 16560
atttggtgct tataggagtc acattcattt gcctgataat tttacttcta agatccatct 16620
tatgccctat taatattgga taataaagtt ttgctgtagg tagtggatgt ctaaattggt 16680
ataattttgt aagctaatct ggcagtgtgc acatatacat ttaaaagtaa atacttttaa 16740
ccaatcaatt gcaagtctag aatagtagcc catggaaaaa acagatttct ataaagatct 16800
atgtaagata gtattcatta taacagtgtt tttaatagag aaaaaaagtg gaaatattat 16860
tacaactggg gagaagtagt atttttataa tgattgaaat aatagaagac ttctcaaagg 16920

gaaagatagg caaacatgat tatgtaaaat ctttacaatt cctctaaaat aacacaaaac 16980

gtattaaaaa gcaaaaaaaa gattgaatga aaatatatta aaatacagcc ccccccaaaa 17040

gaaagaaagc caattaaggg ctaatatcta taatgtatca agtttacata tattaccaac 17100

aaaaaccaaa caaatcaacg aaaaacccca gacctaaatg ggtacacggg caaagatgta 17160

agaagttaag tcacccagta agaaaacaaa gtgactattt taacatttta agtttgagct 17220

tcctagcaaa ttaattgaaa taataatgaa atttcacact aataataata aacttaaaac 17280

aatgacaatt attgattatt cttact 17306

<210> 13
<211> 4784
<212> DNA
<213> Homo sapiens

<220>
<221> misc_feature
<222> (1)..(4784)
<223> chromosome 11 nucleotides 44240085 to 44244868

<400> 13
gcagtgcaga aggttgggcc tagccacctg tcttggaagg gactgggccc caaggttctc 60

ttctggagaa ctgagctgct gagtgaatgc ccatgaggca ttggagaggg gagctccatg 120

cagcaccggc cgcccctggt gccaacgaag cccaggccct cacaccctgg gtggcacgtc 180

ccaggctcct ctctcagcct agtctcaggg caagtacagg tcagggcagt catgtctcca 240

ctctgggcca gggtcttctc tgttgtgaag ggactggact aactagaggg tttctaagct 300

tcctcccaac tcggtggtcc tatcatactt agtgtggtta ggtggccccc aagggctggg 360

ccaagccaca gaggacaggc ctggggtacg tggctgcctt tgacgagatg aacaggaatg 420

tttcacattg gcatgtttaa tcctagacac ttcctgggtg tgttgggaca ggcagccata 480

gccacagagt cttctaatag acgccttttc ttctcaagct tttgggttga aatttgcctg 540

acttgggtct agcccagcca gagagcagac gcatggagga actgagtgcc gggggccact 600

gcggaagagc ccccttaaag ctctcactct ccctccctgc ctgctctgga gctacctgtc 660

tgtcctcctc tttgtcctga ttcccttgct ctccctcctg gcccactctg gagctacctg 720

gctctcctcc tctttgtcct gattctctca tgggtgagaa agacccaagg tgaaagttcc 780

ttccttcccc atccttcctc agccatgagc atttcagaag gaaagggggg agaaatgggg 840

aaactgttcc cagccttaag ttcttccttt ccctggctag attttggggg agtctgggtc 900

atttcagcga acctcagaac tagaaaaaac ttcccaaaag tcactccaaa tcctcaaata 960

tttgaaacac ttaaagccaa gcctccaaga acatttaatt caatgtccaa gaccctttca 1020

cctctataag atccatttta gagctacggt tcacaaagag gtctgactca ttaagggagg 1080

ccacagccag acccttcctc agcctggttt ctagagcaga tgagaacacc aggcaggcgg 1140

gagactgtca cccccaaccc cagagaccct cattcaacaa caccaagttt accctccgcc 1200

caacccagga ggcctttctg tgtccacgac cctgagcata gtgggcctgg ccctactcca 1260

tgacaggaat caagggagaa ggtagaaaag gcgagcgctt caatgcgggg cgctgcctgc 1320
tggagggacc ccccacctgg ggtctgacag gcctgcctct ggacggttac tgtgtactcc 1380
cagctgaggt gagggctggg gctcagggcc agacccaagc tacaggggtc gggcaggcag 1440
gcacagagcc ccgcttcctg gaggcaggaa gcctggtttc aggagggcat cctgacagcc 1500
ccatttgcaa tagcaaagcc acaccgagga gcccaggcca ggagcccttc cgcaggctgg 1560
agagagggca gagagtcctg gtccctgtgg aagaagtgta aggacggagt gtggaaagct 1620
gtgtggagaa gagcaacacc tcctagtgcc tggagggaca cagagccccc atctgccgtg 1680
gaaggctctg ggcagcgatg acactgacag atatggcagg gaggacagtc agtttgaggc 1740
catagcaggg cctggaggcc gagcatggcc acacgtcctc tagcaaagcc tgcacgcagc 1800
ttttgaccct ctgccacccg gcccccaagc tctagcttat gtgttccgaa gttgtgagtc 1860
cttccatgcc gggacactgc tgagcagccc cagggagggg ccagggtagg gcagggaaga 1920
gacgagcccc tccgcatcct cctggaggga acctgactgg gctctggatg gagaacaaag 1980
ctgcccagac ctccccgctg taccagatgc caggcaggcc ggagagcggg aggactgccc 2040
gggcccacgc cccacccgga tgcctccccc tctcccagga cctttccttc ttctagcacc 2100
atgggagccc ttgtggcatc tgtgttctca gcctccctca ggtctggctc ctcccctata 2160
gtgtccagtg aggggctggc ccgtgtctcc cctggggact ggcattgcta gatggcagca 2220
ggggtcctcg gctccagctc ctgtcatcct ctgcccactc tctccaggtg tatccagcct 2280
aggggacagc caggatgcag tcagccaggc tctgtcagac tcgtcactgt tcggggggag 2340
gtggaggctg gcgccttggc cccagcaggt cggattccgt gtgctttcag ggagagaaag 2400
atgggagggc aggaggctgg ctctgcaggg cagcctgtgc ttcagacaca cagccaccgg 2460
cctggtgacc aggggacccc actaggagcg ggaaggatgg acaagactgg gctggtgagg 2520
ctgctgggtc tgcatggaaa tctagcattg actcatggtc aactaggcag agcagaggag 2580
tgggcgggag caagaaagcg ctttcagctt atcatggatg cgaagctgaa aaacgtggcc 2640
acctggcttt ctccactgcc tgtggccggg agcaggggtc agggcctcag tgccagggag 2700
gggccagggg cctgctgccc cctccctccc agcagtccac ggggcctcag acttggggcg 2760
gctgaaagtg ctgagggtca ggcccctggc ccaggccagg ttcctaagag gaaagtcgag 2820
tgggaggctg ggggcctgga aaacatgggc gtggcccatg gtgtcccgag gtggggacgg 2880
ggcaggggtg ccctgtcatg tggcccagga aatggccgca ctgtgctcct tggccttcat 2940
gcggagggcc gcgatgctcg aggtcttgcg gtccggctcg ccgttgagct cgtagccatt 3000
gaggcctggg ctgaggctgg ctgctccaaa caggctgccc atgtgcgtct ggcccacgtg 3060
actgccagcc ccagacacac tcaggaagtc ggtgacgctg ctggccccag agccaggggg 3120
gtgggcatga ggggacatgc aggcaggcac cgggtcgcag gggaccacgc aggctggcac 3180
tggtgaggca gccccgttgt tgccgagcca ggacgggttc tgaatctggg agaggaaggg 3240
agagatgtca ccggctggcg ggcaggtgtg gtgtggaagg ggctcgcccc ttcccccaga 3300
gcacctctcc cctcactgtc catccttccc atgaagcccc tcttgagtgt ttagccttga 3360

tggacatcca gatacccctg tgggggtggc ttttacacct tggcatctct gttcccaggg 3420

ccaggctagc agcagaggga aattcagttc taaagagtga cctcaggctt cattaagggt 3480

ccagtggaag ggtgacaccc cagcaccccc cagctggctt cctattttta gactcaactg 3540

tagacttggc ctccatctgc ggatggcagc tgtgtctcat tcacctccag ctcctcccca 3600

ggcccctcac atccataggc acctgagcaa ggtgagcaga cctcaactcg gagagctttg 3660

gcctcgcagg ttcccttggg cccccaccag tagcagggaa gatggagggg ttgggtgagg 3720

tgggcggcag ggacctaaac ctctcagaaa ccaagggtca gtggctgagg ttccagggcc 3780

tgaactagga ggcttcagaa ctaactcagg atggaagggg ctgcctcagg agtggacagc 3840

cagatctgag accggacctc ctcgtgccaa aggggcgagg gcatgtgcag ggatgagttg 3900

gccatcttat cttccatctg tgccactggg cttcccttcc ctccccaacg ttcacctggg 3960

gctcccatct tcctctacct tctaggcacg tataaggagg tagaattagg gtgtggggga 4020

gacccgggaa cctctcagct tatgcacacg ggattcctgg taacctcatg tgggcttctg 4080

ctggcctgcc ctcctgcatg tgtctggagc acactcgagg gaggcatttt aaagcaggga 4140

gaaagagctc agcccccatc ctggagggaa ccgcatttct ctctgcggtg caggggaagg 4200

gggaccggtt ggcctccatc accccctctc agaagaggtc tgagcacaca ctatgtggtg 4260

ctgtaggtga ggtgggggta ggaggggtga acagggccct gtgttgggtt ctccctccag 4320

ctgtgcgctc aggcagcaga gctcaaagcc aaggagggag ctagagttcc atcctgggag 4380

gacagaggga ggctgagttt agggagggag aggggagaaa gagaatggaa gaacatagtg 4440

cgagctgatg ctgatggtgc ctgtgtgaga caccccatat tcctcagcct cacctccagg 4500

cctgtgccct gccctcctgc cccatgaatc ctgggctggg ggagagggaa cagagggctg 4560

gtctggttgg agagccagag accatttgct agcacagaac caggtcaggg agaagtaaag 4620

gaggcagggg cgcccgtgct actggctggc tgctgagcag ggccaggttg ggagctgagg 4680

tggaggccat gccagaatga ggcctgagca ggtagggagc agccgcttct gctgggcagg 4740

ctgagtgggg gtcaggagag gacttggact tggccaaaaa caca 4784

<210> 14
<211> 40837
<212> DNA
<213> Homo sapiens

<220>
<221> misc_feature
<222> (1)..(40837)
<223> chromosome 11 nucleotides 112797357 to 112838193

<400> 14
gttcatcttc tcaatctcct catctagaaa ataaatgcac acaggaagcc gctccttaaa 60

gggttgtgag gctgacatgg aatggcacat gtggacatca ggaactcttc caatagcatc 120

tagatggaaa ggaatggagc tgggaattga acctggtcct ctcactccaa aaccagcgtg 180

cttttcatac acttagcaag ataaataacg cacacttagt cctaagccag acagatgttg 240

gcatggcatt gctggaaatg gaacaggagg aaaaaatctg gatgacaaga gcatgtatta 300
gagaggggac agtgcgtggt tgtgagggtg cgctgatagg acatcagggc agggtgagac 360
gtcacctggg acgttctcta gggacaacaa atcgctgaga taatgtccat taaacaaggc 420
tcagtgccca gctctagttt gatgagatgc tgtggggaag tctgagccct gccctctgag 480
aatggcttct gcaccctcct cccctgacac actcagcttc acctttaaaa taaggtgaac 540
aagcatgttt aaaggatctg ttcgaaagac ttacagattc ctgggaaccc cctatagccc 600
atgtatggat ttgtccctac ccaagtgcct gggtagggac agggggtctg gaagctaaca 660
gaagccatgc ccagagccct gggtagaaca cacgttcctc tgggaagagg gtacctactt 720
gcctggggcg aacataggga gtactctctc cttcctcttt atcccaaggg ggcggtgaat 780
aggaaagaca gagtcctccc tatgtggagc agtgctggat gtctattgtc atcttcatct 840
ttatcatcat cgccatggct gacatttaca cagtgctcac tgtgagccat gtatcattct 900
aatcactttt catacatcaa ctaattcgat cctcaacaat gctatgatgt atgatactat 960
tattatctgc attttccaga tgaggaaact gaggcacaga gagtttgttt gctctaccat 1020
ttttccctat ggtctgagac ctaggaccct cctttggaag agggaagaag gaggggatca 1080
agcgtaagaa ttaagaaggc atggacagag tctaggggcc ctcataaaca tggatccagg 1140
gggaattgga ctcctggcct gtgtcaactg aatattttta tgtggttttg tatttgtgtt 1200
tccttttggt tcttccttaa tatccagtca ggtcattttg gaagttgcag cctgcattgg 1260
ctgtagtaaa ggaagccaag gggtgatgga tcagagctcc cttgggcccg gggtggtggt 1320
gagaacagta agcgcacgag ccccttgggg gcccagggac agggaggtgt gccactttcc 1380
accagtctcg cctcctcacc tgagcgtcct catctagatt tctcccctgt ttaaacctac 1440
aaagctttct cattttcttt caacaacccc ttccctctca ggcagggatg gcctcagagg 1500
cactggtcaa ttcctctcct gctcagggaa ggaaggtaac ggttcagtgg gcagagcacc 1560
ggcactttcc taaatgcctg atcccatgcc ttcccatgca cctagactcc cagattgctt 1620
gagtcctgga catgccatcc tagaaagctg ggcagagaaa ggggcaggat ccagcttgtg 1680
cccaccgatg atccctaaat gctcccacat ctgctcgctg gctctgaagg tgtcttcctg 1740
gagaataata tctgctttgg gatttctcca tcagcatttc tccctgtgac ctgggtgaag 1800
aaaggcaact tctccaaagc ttctatttcc tattttgttt ttgttttaat ttaattttat 1860
tttttttttg agacagagtc ttgctctgtc gcccaggctg gagtgcaatg gtatgatctc 1920
agctcactgc aacctctgcc tcccagattc aagcaattct cctgcctcag cctcccaagt 1980
atctgggatt acaggcatat gccaccacac ctggctaatt gttgcatttt tagtagagat 2040
ggggtttcac catgttggcc aggctggtct tgaactcctg gcctcgtgat ctgcctgcct 2100
cggcctccca aagtgctagg attacaggcg tgagccaccg tgctcggcct gtttcctatt 2160
ttgtaaagtg agaagagtag catggactcc cagggttacc aagagtattc actgggtctg 2220
tgtcagcaat gcaccaggca cagtgtctag actgtgctgc ctactcccca ttttgaggtg 2280

cagagctggg ccaagaaatg agtgactaca caattcttcc taacaaaagg gctacagcac 2340
agtccatggc agcctctacc tgcaggtcct ggacagggcc ccaggataag ctgtgggaag 2400
gagaggggat atggggagca taaagcagca gacccatttc tgaagtgggg agatgggagt 2460
gaaggtttca ggggcctggg agcataaaca tgcccctcct tggggatcct tgccggttac 2520
tcctatttcc aatatgacct aaggaactaa ctctgaggcc agaattccag cttggacttg 2580
ccagccgtgt gatgctgcag cagttactta acatctctga ccacctggtt tatcaactgc 2640
aaaatggtgg caataatgcc agcctctaag ctgctgtgag ggttatatag gaaaatgcta 2700
tatttaaagt ccctacgtga ggcccagcac ttagtaagca ctttacaaat ggtagttggg 2760
attattaagg aaacaatcta cccatttcgt aaggagaaaa agtaagccca gagtgaagga 2820
aaaaccacct ggggaagcac caggaaactc attggtaaaa gccagtgggg agctagagtc 2880
cccagtgtcc agtgcagggc cctgctggag aaagtgctgg agcaagcagg gggcccacct 2940
acctccaggt agacaaccca gggcatgacc agtgtggcga cgaggaggtc ggccactgcg 3000
aggctgacga tcaggtagtt ggtggtggtc tgcagcgcct tctcgcggga cacagccatg 3060
cacaccagca cgttgccgaa gacgatgaca gcgatgagca gggtgagcag tgtggcatag 3120
tagttgtagt ggggtctgtc cgccttcccg tctgacccgt tgaagggccg gctccagttc 3180
tgcctctcca gatcatcatc ataccaggac agattcagtg gatccatcag ggcggtggag 3240
ccactgggtg gccaggctct ggccaggaaa aatggacaca aggtgtcagt gagagggcct 3300
cttgcagagg tctccaggaa gaagaccaac tcctggcatt taataatgag aattttccaa 3360
ctcgggattt taaggtttac ggctaagaat tttctaaaaa tgttgggcct tctgccactc 3420
ttttgctagc aagtttttac agctggtagg acaaatgagc aaacacaatt ttttaaaaat 3480
gcataataag atgagcttga acagagctga tccatcattt atttattcat tcaacaaaca 3540
cttcttaagc acgacccatg caccacacac ttcagttaac caggcttgct accttctagt 3600
catcgaaaca gaaatgggta ttcattcatt cttgattcta taaatattta ttaagcattg 3660
ttctatatag ttctagaaat ataggcagga acaaaatata catggttctg tagctcacat 3720
tctaggagaa acaacagcca attaatcaag caattacatc taagggtgat agggagtagg 3780
aaatggaaca gggagagaga tacataacct agttcaggta ttcagggaag gctttctaga 3840
ggaagtgatg ttcaacagac aaacaactga aggatgtgta ggaattagcc aggtaaagaa 3900
ctaggagtca gcagaaggtc tagacattgg aagcagtata tgcaaagcac agaggtagag 3960
aggtagacag caggtggagg gcctcaaaga gattctggat agctgatgca gagggacctg 4020
gcaagggttg agtctgggga ggttgcaata ggcaagatca tagagggcct taaaagtcat 4080
gctaggagtt tatacatgat cctaagggca gcaggaacca catgatcaga ttcgcctttc 4140
gaataggtga ttctgacagc actgtggaga acggagagga ggggcccaga gtggaagcgg 4200
gacaccaact gggaggctgt agcaggaatc caggtaagag atgatgaggt ttggcaggat 4260
gcagggagtg aaagccctgc agaatgggca tctcatccca cagaagggaa agacttcctg 4320
aagtgggtat catctgggat gaagtagaag gaggacttag cctgctaaat gagaggggtg 4380

gggggatgtt ccagacagac aatatgacat ggtatcaaat catcaaacag caaacaattc 4440
tgtttggcca aaaatgtagg gtatggcagt aacgttgagg ataattaaac tgcagggaca 4500
tttacaggcc agattgtgtc ttgtcagctg gagagtccct gagaggttgg gcccagagac 4560
atgaccctat tggactggtg ttctagaaag tgtatggtgt agaagagagc tcagaggaga 4620
gtgatgtttt ggctgtaatt taggttagaa attgtggtca tctgaacgaa gatagtggaa 4680
acgaggatgg agagacctgt tcagaattag gagctaaact gcaagtcaat ttcatggtcc 4740
ttggtccttg gataggtgtg gggcaaaagt gggtatggga caagcaggga gataaggagt 4800
tgagggtaat gctaggattt ctgcgtgggg ctctgggagg ctggtgtcgc atattcacag 4860
agaaaaggga ccagaggggc tcaggagcag acttgggagg catcaggagt tgatgaactt 4920
gacagccgga gttttggttg tgactgaagc ataatgcatg gataatatag ttggcttttc 4980
agctaacaaa agtcttcctc ttttacagaa tagaattaaa cttatcagca ttccaaggtg 5040
tttcatacaa agcacatgac ttccaggtac tttgctactg caaattgaaa accaccaatt 5100
gatgccattt gggatgaatg tggtgccatg tccaatcaat gaacagggca cacctccagc 5160
aatgggtcag agaggatggt ggaagagggt ctgcccaatc tccacactct catccctgtc 5220
tgacacaccc actcttccag ctttagtaga agaatatgga tcatgtccta ccaaccacca 5280
tcactctgga gcctcgggga aaagcaaagt gcctaattcc atgtggggtg acatggaatg 5340
ccaccacgca gcaaatcttc ctgttctaag gtaatgaagc atatgtcata ttgatgctgt 5400
attctgcaaa accaactgag ccctcttccc atttggagcc cacagcggga ggcctgcatg 5460
tatgtattga tcaggtgcct ggggctgctt ctgacattca tttactcaat cggcatttat 5520
tgaacctgct gggcataaag cactgtgcaa agcagtgtca ggggcctgag gcgtgtgagg 5580
cacacttact gcccccatcc tgaccatcgc tctcttcttc tccccccact ccctgctgct 5640
gatgatagca tcatatactt gtaaagggtt tgatagtttg ctaagttttt tggatatact 5700
gtctcattta tcctcaaaac aaccctcgga ggcttttaat tattatccct gtctatggcc 5760
atactacctg gaacatgctt gatcttgtct aattattatc cctactttaa taagaaaggg 5820
atttggaaaa ctcttctgtt cacttccctt ccctctgctt ttccagtccc tatgcatcct 5880
tcaaagtgag tcttgcctag aatcctcctg tcttagaatc cacccatatc ctcctgcctg 5940
agccatcacc caccctcctc agggtcctgc agcactgaaa cacaccccag tgctgcagga 6000
gacacctgtt taggagtctc tttttttcac tggggtgtag acatcacaag agttacaaca 6060
tgtcttgctc attcctcctc ttgttcttgg aatgaacaaa taaaaaccat actatcttgt 6120
tctttcccaa ctcttgactc tggtttgtgg gcagtacgta ttcaacagat gtttgctgaa 6180
ctgaatcaaa ggctctgtta gtgcaactgg gaagaaaacc ccttcccact tgaaaggcac 6240
ctgctaacat aggctaccag attaacaagt gatgaaaaag taaggcaccc caagggtgcc 6300
ataggtgctg catgttgggt cctgttacag tctgcactat gctcttcccc caacccacaa 6360
aatgtatatg ttgaagtcct aacccccagt acctcagaat gtgactgtat taggatacag 6420

ggcctttaaa gagttgatta tgttaaaatg agacgcttag ggtgggccct aacccaatct 6480
gactggagtc cttataagag gagctttgga cataaaaaga gacatcaggg atgcgcgcac 6540
agaggagaga ccaagtggga atgcagcaag aaggcggcca tctgctagcc atggagagag 6600
gccttaagca aaatcagccc tgttgacacc ttgatctggg acgtcccagc ctccagaact 6660
atgaggaaac agatttctgt tgttcaatcc acccaatctg tggaattttg tcatagcagc 6720
cagagcagac taatacaggt ccctagaagt agactctgaa gaggaaatca gcctgcagga 6780
aatgtacagg ggaatgctct caggatcaag atgtgcaaag aagtgaggaa gcaggacagg 6840
ccagagggag aagtcacaag aaaggcatca ggtgacccca cagggcaccc tgccagttgc 6900
ctaaatgagg gcaacagggt agggccttta atcccctctc atagatgtca cagcttctgg 6960
ctgtcccagg gaaagcagcg aggccttgaa agaggcatct ctcctcagca gtgggcaatt 7020
gccaaaaact gtcagttgcc aacactccca gtagatgggg atgagggctg tggtcctgca 7080
gggggatctg ggtggcactc caccgtgtcc actacagttg atgttcagga gcatatgcac 7140
atgtgtgtgg ctggggcgtt tgtggagaac ttcctggagg agggaggatg taaactggga 7200
tttaattttt ttttaataga gacagaatcc cactttgcca cccaggctgg agtgcagtgg 7260
cacaatcata gctcactgca gcctcaactc ctggcctcaa gtgatcctac cacctcagcc 7320
tcctgagtag ctggaaatac aggcatgtgc caccatgcct ggctaatttt taaaaatgtt 7380
ttgtagagat gggggtctca ctatgttgcc caggctggtc tcacactcct ggcctcaaga 7440
gatactcctg ccttggcctc ccaaagtgct gggattacag gcactagtct aggatttaaa 7500
attatgagta caatgtagat ggcaaatgtg agcactgcag gtcaggtggg gttgagggtg 7560
agcattcatt ctctggagtg ctggttactt ctgcagggta agggagagtc cctgtaccca 7620
agttctgaga aaggcctcac acatttgatt ttctaatcta ttatttatta ttggttgttc 7680
tcgttgcttt ttgttgctaa tacaaacgta gtatatttac accataggaa aaaaaaggga 7740
aaacatggca aatgcctaaa tacctgtaac ccctataagt accttggtac agggctatct 7800
ttttgtctgt ttgcttgttt gggggttttt ttgttattga gatggagtct tgctctgtca 7860
cccgggctgg agtgcagtgg ggctatctcg gctcactgca agctccgcct cctggattca 7920
cgccattctc ctgcctcagc ctcccgagta gccaggacta caggtgcccg ccaccatgtc 7980
cagctaattt ttttgtattt ttagtagaga cggggtttca ccgtgttagt caggatggtc 8040
tcgatctcct gacctcatta tctgcctgcc tcggcctccc agagtgctgg gattacaggc 8100
gtgagccacc gcacccggcc agtacagggc tatcttttaa cacctggtgt cagtcctggt 8160
ttctatcgtg tcttcagcac cttatctact tggggctttc acctctcaca agcaggggca 8220
gcctagctgg ctctcaccat gtaaacatca agctgagcag gaattcagac atcagcaaat 8280
ccactgccac agagaaggta ctctagcctg agaggtgcta gtataacatt cgagttgagg 8340
actaaaaaac ctgggttcaa gtcccaactc taccatttac taactttggg gcctcagcca 8400
gattatttgg ctttcctgag cccagttttc ccatacacaa aatagagata acaacaattg 8460
tgagtattaa gtaagatgac tcatggccat gtgccaggca caggtaagcc cttgagaatg 8520

tcaactttaa aaatcaaacc aagcaaaaga atgaaatgaa acaagcaatc agaccacaag 8580
ccaacggctc agcaagatga agtgactgtt gctgttcaaa tgcagctcac tagggtctga 8640
ggtcctcctg ggcccactgc ctcctatggc ccatcctgaa aaataggtgg ctagatgaag 8700
ggagatggat ttcaagatgc agccaccaac tccttgggag gaagtaaagg aaggaaaaga 8760
caaagcctgt ccctgggccc tggctgtagg acaaactcaa gtgacccttg agatgaggta 8820
gcagctggcc tggaagatac agctctccca gcccagctgc tatagccgat gactgtcata 8880
ctaatcacca catttcatag actctaagac aaaatcgaat gaagatgcac cactatttta 8940
tatatcacta ggaaagaaaa aaatgccatc caaccccagt agagctccaa gatgtcatcc 9000
attgtaaaat gcattctgat ttcagagaca ttaaaatggt gggggcaggg ggaagtgcat 9060
tgtagagtgg atagcaatac agggccttcc cagcatttct aagctggtat tttctcaagg 9120
ggcctgagaa aacctgctgg tccccaggct tccctgctgc agctccagcc catccctccc 9180
ttttgtcctt ctgtctccgg gctgcacaga ttgttgggct ccaaggggca tcccaaaaga 9240
atacacaagt gagtagcttc tccatcctgg ctggagcttg tgttcatggt agcggagtga 9300
ggaaaggttc tcagtgctgc tccagttacg ctgggctggt tctggctcca aagaagcctg 9360
gtgagttctt tgtccactct ggagcttgtt gagggaaaat gcaaagccac ataacacatg 9420
gcaccttgta tttgccatga tggtcccaat ttcttgttct ttttttaaaa tggagattgg 9480
ctaaatatat aacccaaggt gcattattct tacatgagaa atttttttga gacaaataaa 9540
gtggcacagt atatttaaaa atctattgtt atcaaaacaa gcattttgat ttttgattgt 9600
gaaaatatga tcaacttgct caccaccctc caaatgtcat ctccatcttt ctcccaggtg 9660
gcccctgggc atgcataaaa gcttacaaaa ctccaaatca caatccccag tcaacaagaa 9720
cttagtgagc accaaataca tgcatggaca ctgttatttt ccagctgtta caggttgagc 9780
tacttgacag tgtttcctgg gtggaggatg aacaatctaa tcctgtagct gccaggcctc 9840
actccagggg tggctgcctc tcccctaagg cacctttctg tgcaggaaag tgggggtaat 9900
tgctgctcct cattccaccc tcaccatgca gatgcttgca gacaaggttc catgggtcct 9960
ctgaggcctc ctaggctttt ctttctcagc tgccctggtg gaagccagga gaaagagaag 10020
actgcatttg tgtggtgacc tgcaagctac tggtcatcaa agagccaaag atctcctcat 10080
ctgtctccag gactctttgc tctgccctag ctcagaaggg caaagatcta cagaaatatc 10140
tttcagagtc atccattcta gtgctcagga cccctgcctg tcaagttctt cctaacatct 10200
agcctaactc cagccttcta taatagtggc tctcttcaga agcttcagag tggaacttcc 10260
ccaatcagag gtctagaatg catcaaggtg catagaaata ttaggtcagt tgggatgtag 10320
aggcaggcac ttccaacctc agcagcttct gctattcacc ctatatgcct tacaaatgct 10380
actattttct gtgcgtgccc taatttggaa attagctagc tctcaagttg agaagtactg 10440
gaagagccca gatacccggg gctaagctag cccacccagg ctgccccagg caggccaccc 10500
tgctcaggcc acccactgca ctgcccccca ggagtagagg aggggacagg gagtgggcag 10560

gccttctggg gtatagatga ggtatggaag gggctgcttt cccagtcacg ccccaaaact 10620
ggcaggcgcc ccaacctata aagtgtattg gaagggaaaa aagctccttt aaggctcagc 10680
agtttgaggc tgacacccaa acctgacatt ttctctcagg agaaacttgg aaggtgtagc 10740
aacacttagt ccccaggctg atggagtggg gtgaaaaggg tcaatctgtt aggacagcct 10800
gggatccagg aagaaagctg gcaggtcctg ggagaacaga gaggattttc tctgatcctc 10860
tctttctctc tctctctctc tctctctctc tctctctctc tccctccctc tatccccacc 10920
acctcatttc tatttcatct ggggcacaga gctagaaagg tgggggacag aagccctgga 10980
tgcagcataa accccagcaa cgtgtttcct atgcttccgt ctcagcttaa gtcttggtgc 11040
tgccttccct gaaagttcgg cacttgaagg aatcaaattg tctaattaaa atttaacaat 11100
tccttatcag gcttgatggc gggtgagaca ctcctgtctt aaacacatca ttttttcctc 11160
ttttctgaga tgcaaagcat ttcaatgaaa aagtagaggg ggagaagaaa gcattttgta 11220
tttgcatctc aaaagcagcc tactggagtg acttgagttg gataggaatt gggagtgagt 11280
gggtaagagg ttcctttcca ggaccctggc gatataggcc acctgcccct acccctccag 11340
gcaaaggcca ggctctcact tggggttgga ccttctttta gcatctcttg caacttcaaa 11400
aaggcagcat ggaaatggaa gagggaggaa aggtggtgcg cccgttcctt cccacatcca 11460
agggaaaagg agagagttac tgaataacac ttttatgttc tctggccgag gaggcaacct 11520
cagcagatgg cagaggaaat ggagacctca ttcccaaagc cctaggccag gcggctgcag 11580
accttgggtt gccgagggtg ggagtgggta gatggacagg cacagtcact gtctggaggg 11640
caaagcctct aagccctgga gctggggtgt ggagggtctg tggcgaggaa aaaaaggagg 11700
gggaggagaa acagaaagga ctgtggggac aaagaggaac ctggagcaat ggacgatggc 11760
aacctcctgg gctcactggg aacggttttt cctgcatccc aggcccgagt gcacagggga 11820
gtggggtgtg aggctgtggg tctggccccg gtgtcagcag tcactcctga gggaagaaat 11880
gcagattgct ctttccctgt taaggggaag ggctcctcct tcttgagccc ctggactccc 11940
ccacaaacca cagcctcagc actactgtgg gggaacaggt ctggactaga gacctaggct 12000
gggggatacc cgttagtgga agcccaggga gtggcctgga ctaccatttg ggggtggcag 12060
aggctgctgt gggagacagg gggaggccac acactaatac cccatttgcc ccttctcccc 12120
cggcatgggg agcctccctg gaacctggag ttcttcagag gcagcgaggc agcagagctc 12180
agggaccgcg cagcagctct tggctatgtt atctgcaggg aacattgtct aggtttcttt 12240
agaaggcgtg gaacagcctg gatgaatctc tccactggtg gtctctggct gtgtctggcc 12300
ctatgcagct ggagccagag agtggctaca atctcgaggg ccttctgtgg cccagcttga 12360
ggttggcaac ccgggtcgcc tgctccaagc ttctccccct gctccctgga attaaacctg 12420
gccagcagtt cccagtctta cctcccacct cacagtaaaa atagaagcta gccttcggca 12480
ctttctcaat ttcctgtcac caaatccata aacttacagg cacaggcagc cacatgcacc 12540
ctccttgagt cccaactcct aaccctaagc cccttcctgc cacttgggct cagcatagca 12600
caccctggct ggcctcctcc tccttctcac ccttcaggac tatctttcca gccccttctc 12660

caaggaagcc ttcctgccac tccagctcag agcccgtctt ctcacttaat gctttcacaa 12720
cacgtgtgct cctcggtgac acccagcaca ggtgtaatga agtcagtatt tgtctagaga 12780
tttgtttgat gtctgttctt cttgctaaac tgtgtgcaag gctcacatgt gtcttgtctc 12840
tcctggtctt ctccctcccc agcacagcct tggcaatagc acatgctcca cacgtttgag 12900
agacgtccat ggagatagca tctcattcaa gggtgaaggg gccagtccct aggtcttttc 12960
ctaccctgca gtcatttctc ctcccagatc cttcgtgaag ggtacaatag cctcttctga 13020
gatctcctgt tgaccctctg ggtcccagtc acagtggtag ctggggttgg gtgcccagct 13080
ggttcgggtc cttgcagggt gtttcccagg tgggcatcac tgtggcacaa aggtgttata 13140
aggcattgga aaaagaagaa gaggcacaca gatgaaagtg agtgtgggga tgtggggagg 13200
tggggagagg tcatagccaa cacacagtct gtggaaagaa aagcacagag ctggtagaga 13260
tggcttctgg gcttctggaa ggcccagggc ttcagagtgt ccaggttggc agcaccctgt 13320
gggagggaag tcacagcagg ccaagggagc catccccccc catcagcacc agccagagac 13380
taccaagcat ccacagtgag tgagctgagt gcagctgaga ctcagttctc tatggagcac 13440
aagggtgggg tgggggtgtc cccaggggga ggctggagca cgcacagttc cttgcccagc 13500
tatcgaaagg gcccctgtgt gagactttgt ctaggagcag aggagaccac agctgagcat 13560
gaccaggcct ccccttctcc agggctctca tcccagacaa ccccgctctc tgcttcttcc 13620
caaggtggcc aggtggccct gggcctgctc aggtgtccct gggagtgtgg catctctgag 13680
aggcctgagc agcagcagaa tttgagtctt cccagcctta ccttgttgca ggcctccagg 13740
gtctgcagcg gggtgaggca gggtctggga gatgcagtga atgcaggaag gggtgtctgc 13800
ctgacagccc catccctgca gattttgatg gctccgtaaa aatgtctcct cataaactgt 13860
caaagtgtca caaattaatt aactgttagc tagagtgact cagtgtgggg gggggtgggg 13920
gagaatggct cagtgatgct gagcggggtg gtggcggtgc tgttctctgc gtgtgctgcc 13980
ttttgattaa aaatcctcac cgctccgata ctgtgtgagg gaaggggcag atccctgggg 14040
cagctcacgt gcactgcaga agactggtgg gtgggaggca ggtggaggtg tggggctggg 14100
gccccctccc tgagaaggga acacagtggg gagaatgaag cctctagaaa ttgcccagga 14160
gatctaaatt cagaatggag cccacagact ggcactgtat tcctcccccc gcccccccac 14220
tttcccttcc tcctcaattt gcccccacaa agaaaaaaat ctgggaagtt gagcttggtt 14280
tgccccaggg gctacacagg gtcacctgag ccaaggacat acttagctac caaactcctg 14340
ccagccagag cccagagagg aggaagggct gggtcttaga ccagcctcct tgacctttac 14400
tttaatttca ggaaagaatt gagaaaacat gctcttgaaa gtcaattggg agacaaatcc 14460
tgaaccttat tgctccagat ggaggaaaaa caatcctgcc tatttggcag aaaattgttc 14520
ttctttatta aaagacattc ctgttcataa aaacagagag aaggcccagt ccaaataata 14580
gcacaaccca cgatcacact gctggaaacc tccggaagcc cttgtcccca cgtttctcat 14640
ccttcggctc cagcaggctg acaccgggga cctttctttc ccatttggca aaggcgatgg 14700

gaggccttct tgcaatggag gggtgggcag gcaagatggg acttgaggac tcccacacaa 14760
atactgtttt ggtgtcaaga cctcaaagac gaattgaagg taaaatttca ccacaggaat 14820
gcaatcagta aaatccagat tgcagaaaat acaataggac aaatgaccca gtttcttcca 14880
aaaataaatt gcaaagaaaa aaaagagaga tggagtgggg agagcctcta gaataaaata 14940
aactttaaag ccatcttcac caattgcaat gtgtgggcct tagttggatg ctgattcaaa 15000
gaaaccaatt ttttaaaatc cctgaaattt aggggacaat tgaaaatcta aacagtagat 15060
tttttatgat attcagaatt attgatatta tggcattctg gtgaattttt ttagagtcct 15120
tatcctttag agatacacac tgaaatattt acagatgaaa ttatatgctg tctgggaatt 15180
atatgaaaat agcatagggg tagaagcata gatggaagaa aattggccat aatttgatcg 15240
tggttaaagg tgggtgatag atacattgag gttcatgacg ctattctttt taattttgta 15300
tatgtttgaa attttctata acaaaatgtt ttaaaatatc taatctgctc cttaaccctc 15360
accccagcgc ccatccctaa gaaaacagat gggtgcaagc caggcttcaa ggtatctttc 15420
tgatcaagtt ctcttattat gaacactggc ttccttttct gccagtctgg ctgccagact 15480
ctccctctgt tttgatcact ccattgccct gtccaggaca gatggagccc tggggacgag 15540
ccctggcaac cctttctatg gcctgggatc ctttctcatg acataacagc tttggcctaa 15600
taagtcatgt cctctctttc acagcaggtg gcggctcagc tggtagggat ggaggtggga 15660
gggtaaagcc cactttaatg acaaatagaa cagctactga gcacctattg caggccaggc 15720
accacgtgaa gtgctgggca cctactattt catttactcc gcatagcagc cctgtgagat 15780
tagatatgat ccccatgcta cagacaaaga aacaggcagt gggttgtgaa catcagtgga 15840
tctgcctgca gaacccagac tcttccttac accaggagtt gagtatggat ctagggtacg 15900
aagaagtaag gttttcacag tcctgtgtcc gaggcacatc tacacggttc cttcatgggg 15960
atgggcaggg tctcacccct ccaggctggt aggtgtcaga ctggtgtcca cagagccccc 16020
aaaggaactt ctcatataat tatatgaggg aagaattata tgagccttat aaagagggaa 16080
gttgaggtcc aggccatagc caagtttcct cagaattcta agtgacttag gcaacctcat 16140
gttttccaga aatgacactc ttagtgtctc caggacatgg ggctactctg acaagggagc 16200
tccgaggagc acagggcacg ctggagtcca ggtggagcca aggagcacag ggcatgctcg 16260
agtctgggtg gagccgagga gtgtgggcaa gagctggggt ccctcatcct ctctcagctc 16320
ctggggctgc ctctgggtcc atctggcagg ccaggctcct ccccaatgct ccctttcatt 16380
ccacagggtg tgcgtccacc accaagggtg gcactgtagg tctgggcatc ttgctttgcc 16440
tggtcttctg ctacctccct cagaggaggg gcaacatggg cacaacaggg aagagagcag 16500
agaggcagtc tcctcatccc gacaacagac tggggggtgc agaggagccc ccagctgcct 16560
ctcccagttg atggaagcag gttggaagag cagccacagt gctgggcacc atcctgaaaa 16620
caggctgtgc acccactgct gctgcatacc actccacatc ctggggccaa cattgaggcc 16680
ttcctaagca gtgatgccca ggatacagag gagtcacttc ctctgtccct ggaattgaag 16740
aaggtgtgtc aatgcgtcct atttttattg tttttttttt aatcagacag acaacagcta 16800

gatctgggtt ccagtcccac ttggtagcag tatggccttg acaatgaaat tgttctgggc 16860
ctcagtcttc tcatctgtaa catgggggca ataacatcct cctcatggga gtggttgcaa 16920
aattgactta gaaaatgtga ataaaggggc cagtaagtgg taggtgccca ataagtggca 16980
gctaatgtca ttacaaggtt gcaacagctg ccggaaagga gctgagctgt gtgagggtca 17040
caggcaaaag actcaagctg gaatgagggg cccaagggaa agaaactccc catttctctg 17100
tgacccccaa ttttctgcat gcttcagaga gattccagcc atgcctacca tgttgttccc 17160
actactcacc cctgaactaa aagccctaaa atatcagggc acctggcagg tgaccttggt 17220
taaagtcctt ttggggtgtg tcatgcagat ttgcaacaac cctgagcctt ccctctaaat 17280
ctttgagaaa aaagaagtcc tacctggcct gtaaccttcc tggtaaatct aaggatgcaa 17340
cagctatgag acatctctgc tactcaggac agaagtagga ccagcatgag atgtgataag 17400
gcttggcaga ctctatcagg tatcatgcaa tcatcagcac tgcattatta ttgtctcact 17460
gtgtggatgt ctgacatgca gaaggaaaat ccagttccct tggatgagcc acttctctaa 17520
ttctcaactt aatagttaat aatttaatga tgagagctgt cagacctaat ttcagagagt 17580
gatagatgaa tcaagtaaaa tgatatattc caaagtgtct tgcaaactct aaagtcccat 17640
aaaaatgtga atgtagtgta ttattatcgt ctcactcagg agcatgtagt tggtggtatt 17700
tctttgccat gcacaggttc ttcggtacct acattgctct gcatctgtcc tttctgtctg 17760
atgcatctgt gagccttaat gggcagagtt tgtcttaaac ctatgtgtat ctttagcatc 17820
tcctaggaag gctaccagtt ttgtaagtgc ttcctggatg tttgttatgc tggaggaagc 17880
tgacagtgac ggtgctcctt tgaacagcct aaaacaggac gagactccct tcttcgtggg 17940
gttatcataa agattaaaca agacacatca agccactagc acagtctctg agagagagaa 18000
ggcactcagt tgttcctatt ttaatggtag ctaacctgga aaaatataga atattgactt 18060
ttactgctgg aagaagcctt agagaatatt taatctaatc tcttcatttt acagattaaa 18120
aaactaagac cagggctggg catggtagct cacacctgta atcccagcac ttcgagaggc 18180
tgaggcagct agatcacttg aggtcaggag ttcgagacca gcctggccaa catggtgaag 18240
ccccatctct actaaaaata caaaaattag cctgggtgga ggtgggcacc tgtaatccca 18300
gctattcagg aggctgaggt aggagaatcg cctgaacccg gaaggtagag gttgcagtga 18360
gcccagatca caccactgca ctccagcctg ggtgacagag ggaggctctg tctcaaaaaa 18420
aaaaaaaaaa aaaaaaaaaa aaaaaaaaaa gctaagagta agaccaggaa agtgaaatga 18480
ctcacccagg ttcatagagc ctattgcttt cagaaccagg attgcaatta aggtctctag 18540
acaggtcccc tgctttatcc tacagggctc tctgactcct ggaaagggct caatccttgt 18600
cactaccatc ctggtcactg taggatcatc atcatctgca tcttcaagga aatgaactta 18660
aggagagtaa ttctggagcc tccatgagtg cctctcctga gtccaactta cctttatgca 18720
acttctgtgt atgctcgcct cgcccagata cctatcctca gcagagacag gctcaagtgg 18780
cactcaggaa caatgcatac atagaaagtc ccacctagcc agttcttttc caggccataa 18840

acttttcttt taacctttct ttgcaagggc tgttttccaa gcactccctc tctttctgtc 18900
ctctggatat tgataaaggc atgcacttac aggaatgtgc tcaaagatga ttccccccag 18960
actttcaggg agaagctcag ggccatgggg cctgttaggt ttctctgcga aatgctactt 19020
cttgaaacta tttctttggg aggttttctg tcccattctg tcccaggagc ctgggaaaaa 19080
ggctgcgcct ttggatgttt tgctcagcag cttgacatgg gccatgatgg ggataatggc 19140
agaggcaaat aatgcaaatc ccaagtgctt cattagatat gaagatgaaa gggcattccc 19200
aggcatgaga gctactctgc cacccaggat gatgggatgc aaccagggac cctgctcaca 19260
ccagtacctc tttggataag aaatcgagat attttactcc agagatccac ttatgagggg 19320
gcagaaagga gagaaatgga gagggcaccc cattgggaac accccagact agagccacca 19380
tgctggtttc tgatcttgag aatttcagag gcagaatata aacacttcgg tcttaccttg 19440
gggctcctgg gacacagatg agctaagttt tctgagcctg tcatggcaaa caacataatg 19500
ttgctaatca aacttcctgt gcgcccaccc aatttgataa atcagccttt tgatgcacta 19560
agtgaagatg aattagcagt aatgaggctg tgacagctcc ctgaggcaca ggttgacaga 19620
gagagctttg atggctgtgg caagaaggag gagaagctct tctcagggat cagttcaagc 19680
tgccaagttt gtgcaaataa ccctccaatg gatgctgaag tcatctgaga accagatgtt 19740
tgctgtcttc ctacaatgag ccacatgcca taagccccaa cttatgggca gaccataaca 19800
tggaatcaac ccattctgtt atgtgggaaa tggaggctgg caatggcagc ctgctaaaac 19860
ctttttcctt tgcaaggctg cctactacac agctagtctc caacagccca gcaatggaga 19920
gttggtactg gttagctaat ctccttcatg cccagggact gcaaactggt agcctatgga 19980
ccacatttag cttgcataca ggatttgttg ggctcacata gcattgttga aaaactggca 20040
ttttattgcc aatatttaca attggacatt tcacattaaa atacagatat ctagcttctt 20100
ttacaagaag aatcataaaa tcttggaaac actgggtcag cattcttgca tggcaacaac 20160
aagctggggt cgtccagata gtccccttta tacaaggcac acaccgattg aagaaagcag 20220
aggacagccc ggctcatgag gaatggactg tctccacact ccaaggatgg aaaaagaagc 20280
tacaccttgg tggctcacgc ctgtgatccc agtacttcag gaggccgagg tggaaggatc 20340
acaaggtcag gagatcaaga ccatcctggc taacacagtg aaaccccatc tctactaaaa 20400
atataaaaaa ttaccctgga ggctgaggca agataatcac ttgaaccagg gcggtggagg 20460
ttgcagtgag ctgagatcgc accactgcac tccaggctgg gtgacagagt gagactctgt 20520
ctccaaaaaa aaaaaaaaag ctacacctcc acgcttgttc cccacatccc ccgtatatgg 20580
tgaatttttt cagagtcctt atcctttaaa gataacgcac tgaaatattt acagatgaaa 20640
ttatatgcta tctgggaatt atatgaaaac agcatgggtg taggggcata gatgaaagaa 20700
aattggccat aattgcctta gcttggaagt ggccagaccc caaggctttg gcagaagcca 20760
aggcagagat ccctccctcc agtcattatg tccagttcca cacatgctct caagtttacc 20820
acaatcccca gccatcctta cagccctgca cccagctggt ttcactcagt ttacattaac 20880
taccaggtcc cagaatgtat ctaagtttgt gccccgtgtt ttataatagt tagcaggggt 20940

aactgagttg ctattatgtt gctgagctac aaaggctgtg tctgaatgat gctctcagat 21000
cctctcacct aatcctcccc atcaaggggt agcggcagga ggaggagcac tcttgaggct 21060
aaggaggctt gaggcaacac tggtggcttt tgagcaaagt gttgattttc tcacctcccc 21120
agccctgcct ggagacttca gccctgtgtc tggaggcccc tgtttcttca gaaacacagc 21180
tcctttaggt tccttttctt ccctgctgat attccatggg acacagcaaa cacaaatctc 21240
tctccctaaa ggttccattt gctttagact ttctacgatt gatcatctaa ttacctttgc 21300
agcccaggaa gcgctaatct cttcagcaac tgaggtgcgt gtcacagatt gaaacaaaac 21360
attccatctt cctggcatga ggcaaatcga ggtctggact ctaccgcatg catcagcttg 21420
ataggcaggg ctgctggtga cctgggagag gggcctgccc attacctttg tgcctttttt 21480
cctccatgca gtgctgggct ccccgaggga aggctggttc tgtccccctc ttttcttatc 21540
tgtaagccta acaaatatgc tagggtttgc gtgatggggg agaggaggat tctggtcccc 21600
ttcacgacca atgctgatga cagtgaacaa gtggatccag ctgctactgg tcctttcaac 21660
ttctatttca gaaaacacca gcatctctgt taccaggctt atttgtatgc ttgggttgtt 21720
tgagatgatg aggctcagtg gtctcagcct cttgcaaatt cctttctcac catggcctct 21780
attgctcatc tgaggccacc tcctccgaga agccttctgt gatctatttt tgggcaaatt 21840
atttattcct cttcaatttc atagtacact attggctctt cttggcactt gtggtaccaa 21900
aatctgcaag tctgtctctc tcctctccta tgctctttaa gaacaaggct tgtatcttat 21960
tcatcttttg ctcctggggt atccagcgca ttttagcaca gtgtggcgct cagcaaggac 22020
aactaacttg agaggaaaga tacatattag aagctctcta gtgacctcca agttttgggc 22080
agattggatt aaccatcgct ctccaatcac tgttttggga aaggacgctg acggatgtgc 22140
accgcacacc atatgttccc ttccaggacc ctgtgctcta tctgtcctcc tagcatacca 22200
ggagagctgt cagctcgcca ggcagttagt cctctgccaa acctgtgtac accagttctt 22260
gtcattgtca ttagtttagc tcactatttc ataaacaatg aaatatggat gccaagtcaa 22320
aatattgatc catgaaaact aagtggaatg cattgggaaa aattttgata aagaaaaatt 22380
gctaaaaaca aacaaacaaa aaacctaact agttatctaa tatgcaaatg agataactga 22440
aaattcttaa aatctaagcg tctacactat atcattttgt aagtttaagt ctttgctgtg 22500
ctttaaaaaa taaaactaaa aaaaactgga agctgtaagt aaagtactat gggcacactt 22560
aagcaaatag ggtgaggcaa gactccagtt gatgaatttg tttttgttgt tgttgttgtt 22620
tgtgtttttg tttttctttc tcactctgtg gcccaggctg gagtgcagtg gcacaatttc 22680
ggctgactgc aacctccgcc tcccgggttc aagcgattct actgcctcag cctccagaat 22740
agctgagatc acaggcatgc accaccacac ccaactaatt tttatgtttt ttagtagaga 22800
cagggtttca ccatgttgac caggctggtc tcaaactcct gacctccagt gatctgcgtg 22860
cctcagcctc ccaaagtgct gggagtatag gcatgagcca cacccagctg gtgtatttgt 22920
actcaaagaa atgacccaag atatgaacag aacgtttggt aaatgaatgt acatgaatgc 22980

cctaaggtaa aatgaaatga ttggagtatc atttttaaat gaattccctg tttaaagaac 23040
ttttgaaatc agtcaatcca ccatactgaa tatgagggct tctgctgtac tgactggcca 23100
gcatatcagc tttttctatc tatgttcagt ctctaacggg gctgtatgtt ccttgaggaa 23160
attacatggc ttacacatcc acaacctgaa tgactgtctt ttacactcca tgggccatct 23220
ataaatggtg gctgatgatg acggtgattg ctcctttatt caccacacat ttgctgagcc 23280
cctattatat gcagtgctga ttagggacta agggtacaac gttgaacaag agaggcaagc 23340
tccattgtcc tcacggaggc caccatctgg atagaggtac tagctcgaca gctggggctc 23400
tgatatgagc acaagccaga ccagtgagta ggaaagaggg tgcacatttt gcagagaggg 23460
taacgtggct aatgtcacac actaaacaga tgaacttcct gccacttttg tagctgcatg 23520
ggatctttca ctgcacggga cagatttttg gcagaagcaa aggctctggg agtctagcaa 23580
aaataagaca gcagaaaaat attaaagcaa gactgcaaaa ctcagtgctg cacactccat 23640
cacaagtgtg aaaatagctc cacattctga aaacacctga ccagcactct tcaaaaatct 23700
caaggtcatg aaagacaagg aaagactgag gagctgtcac agattacagg agactcagat 23760
gtgacaacca aaccatgtgg gatcctggat tcgatcctgg gccaggaaaa ggacattagt 23820
ggaaaaactg gcaaaatgca aataaaatct gtcatttagt taataatatt gagccaatgt 23880
tattttctta gttttgatca tcactctgtg gttatataaa acgttcactt taggggaagc 23940
tgagtgaagg gtatagggga actctctgca ctactttgct actcttctgc aagtctaaaa 24000
ttatctcgaa tggaaaacag gcattcacac aggcacctcc gatacccagg tatctgtgtg 24060
aaggggctgt ccaggccttc ctgaggcatc gtatcctcct cccacctgca ggcaaggatg 24120
cagaaacttt ttccaggcaa aggggagggt cctcttctcc ttcacccttg gatggagcaa 24180
atatcaagta tctgtcccac atggtgctga ctgcgcatgc aaggggcagg aggcagagaa 24240
gcagaaaagg aggagagggg aattaatatc tgtcaattga tccatttttc gtagccatca 24300
ttttaagcac ctactgtgtt ccaggcatag caaccctcct tatacatgtg ctctccatga 24360
atcctcatca cgtctccatt tcccaggaga cggaagccca gagaacttca aaaatttatc 24420
cacagttcta gctgctaggt gatagaggca ggactctaac aagctcagcc tgtctccaga 24480
gccactgctg gctacagaga tgctgggggt ggcctggcct tggaggcatt ttccagcctg 24540
ttctggagca tttccagggg cctgggtccg tgagttccag tgagttgcct gttgtcctgg 24600
cccagtaagt ttttctctgt gtctgacctg caattgtccc cttgcttcct gccagacctc 24660
agcggcatgt ttcactcctc ctgggacaca tggaccccac ttcatgcatg ggaagtacat 24720
ggagggtctt cagctccccc ttcccactcg cccctttcca gaagccctga tgacagatct 24780
gatacagtca ccccacctcc atggatccca ccctagttcc ccaggcactc tcttcatcca 24840
aactccttag agtcacgtct gctgaaaaga ccagtttcta cccccggcgc tgagtcagac 24900
acagagttgc tgtcagaact ggagaaaaca cagcagtcat agtaacggta gctcctgttt 24960
attgactgtt caccgtatac tggacactgt cttgcacact gtacacacct ttgtcctaat 25020
tctaaaagca accttaaaag ggaaacatta ctaatccttt ttcacagata atgccaatgg 25080

ggccaaggga gttcctttaa taacctatgt caactaggaa gtggcagtgg aggatggaag 25140
actgggtttg tctagttgca aaacctctgt ttccacagtg gcaatgaggc accccctact 25200
aacattaaca tactgcaaat gcaagatggg atatttggaa atagtttacc catgttgctg 25260
ctaagcctca taataatcct gtaaattcaa tcccatgtcc ccactgtaca gtacacattg 25320
atgcaagtag ttgtgaaaat gagaaacaga gagaagtcct gtggaggcac gtggctgtaa 25380
gagcaccatt taggaactca gtgttggtgg cgagtgagga tgaggccaac actgacagta 25440
aggcatattc cctgtgtttc caaagtggga ggggtcttca aggaaccatc acaccattgc 25500
cacaggagct gatggctgat aggacagaat tcctaacttg ctgctgtggg cttcatctgc 25560
agtcttgact cctaagcagc actcacacca gacaagaaga gggctgcaca aatgctgaac 25620
aaactcagat ctgtgtagat gccctcaggg aacttgggat cctctagcat gtcatagccc 25680
ttgcaagtcc aaacctgccc acttctgctc cagcctccaa agacagcagc cccaccctcc 25740
ccggggcctg gggtaaagtc gggaggcccg ctgggctgca cggccacact ctgttctgtt 25800
gtgcccatgt ctgtccctgc tgctgctgcc ctggaattgc tcaggctccg tgatgttgtt 25860
ctttaaatca taaaatcctc tccccgctcc tgtgcctttc ctgggggagc tctctcgggc 25920
ttttaattga atttgcagtt tctggcacac ccctgttaca cagaatgctt ggtatgggca 25980
gatggaagtg cccatgagaa tcaaaacaag gaaactttga caggattcaa ggcttccata 26040
ccaccctctg aaaaaaaaaa aaaaaggaga gaaagaaaga aaagaaagtg ctgcttgttc 26100
cagcacctgc agaggatgcg agccagccag caaattcact actcaccttg catatgaacg 26160
aacaaacaca cacgggtttg gtgggcaaca gagaaggcag gcggaactct ggggtgtgag 26220
tggtccatga agcaagacag acagacatgg ggcggggtgt gtagggactg aattctgaca 26280
ggcagtcagc atctggcttc tggcccctgc tctgccccac atttccaacc atgtgacctc 26340
aagcaagtgg cttccctctt ctggccctgg atgtcctttt gtaacaaaca tgtgtggcca 26400
catcagagga cacacaaggc ttcctcccac cagtcctaag attctaatct gccatgatgc 26460
gatctccagc cttgtccatg ttgaggggcc agtagctgaa ggagggagct caggtgcccc 26520
aggccctccc tgcttgtcag tctggggaca actgtctatt gtggttaaag caggtgtctg 26580
aggccatgat gaaggtaaga tcccacaaag ggatactgag ggggctctag atggtgtctg 26640
gcagcagaga atgtagtcag aggaccttca ggctaggaag tcatcatcac tctgcgagat 26700
ggaaccacga aacagggagt gagtgagtca ccagagataa tgagctccga ggcaggaaga 26760
tgcaggagtc taatgacgtt cttttcctgt cctgatgaga tctcttgtcc ataggccagt 26820
agagcaccca agcccagagc aatgggtaag ggggtctgac ttcaacctgg ggcagccgct 26880
ccttggaagg aaccaggcag caactgtgat tccagcatcc cagcccagcc cagatgcacg 26940
gtggctgaaa accacgcctc aagctccggg ggaactccct ggctattctt ggaaacacag 27000
agactcctgg ctccctgtca gagctgctcc ccactctaaa aagcccagga gtcactgcta 27060
caatattttt ccttctgaag gaggtggcat ctcagtaatt gcaacgtgat gctctcttga 27120

cagcagtatt aatcacctgt cttcatctca gccccagcca tgagagagga acaggaggaa 27180
gacagagcca gagggcctgg tgttgttcac tgtcctcatc caagggcacg ggtacagcag 27240
tctattctgt ctcttcctca agctcattgt cacttccgag aggggactgg ggtcaggcct 27300
cattcgggtt ccctagagtg gaaaggattg gtaaggagct gggtagctct ccttggccac 27360
ctctaaagga gctgcaccac actgggcctg cgaagaggag accaggccca cagacccagg 27420
caggatggag gagatgggag gggtcagcag ggggccaaat tcaagagttt cttctcaacc 27480
ctacctttag cctgccttcc tgaccatcct gcacatgccc accaagtcca cctcccaaag 27540
ccatggcttc aggctcatgt caccgcctcg ctcaaaatat cttctatgac tcccctgaag 27600
ggaaaagtcc agattcttta gctctctgtc tttgatcttc ccacattacc tgctggcccc 27660
tcctgtggac aggctagact gcagctggcg ttgatctagt ctgtgacttt tgtacccctc 27720
gcttcccctc atcctccttt gaatgctgcc atttctccac ctgcctcccc attcccaact 27780
gtccaaatcc ttccaactct tcaaagggaa aagcccacct cctagaatag ccttccctga 27840
accctctgag ctcatcaaga cttcttcctg ctgtgctccc atagagtctg atatgggagg 27900
ctctgccaag gtaatattgt aaagttgagg catgtcccag acccctgggg gaggagggaa 27960
actgagatgc cctgggaaat tgggcaggtg agacaggaag gccttggcat tcatgacgtt 28020
tggacatgtc aagcacctgt gctacacaag tgagttggga actgtaggcg tccttccaac 28080
atgagcagat catgtgagcc aagggcaggc ttggaaaagc ataatgctgc ttccccctga 28140
gcacatgcca tcataggtgc acagtaaaac ttctagatga ataaatgctg ggctagcaga 28200
gtgacggcca taacacggag ctgctcccac caagacggtg tcagccatgg gaggactgtt 28260
agccagttaa gctgtgattt taagctggag taacagttga agagagtgca gaaatggtct 28320
gaatacctag caagtggata tactcagaca agccggatgg gtcttccaag aattggagcc 28380
agagagagac taccagagaa gcgaaaattc accccaaagt cagagcaaaa tggctgagag 28440
ccaagaaaga aatcaggggg cggtgtttag tgagcagttg tgggtagaac tgattgctcc 28500
ctgagctacc gttcagatta gcaccagccc tgctgttcca cttgtctcgc tgtgcagttc 28560
atatctcttc cattttgtat gcacttattc ccagcatcca gagctgcacc tgccaccctc 28620
cacgaatgca atgttcacaa gtgaagtagt gggaggtggc tggtaaactg gagagaatgg 28680
gatccttagt ctattgttgc catttgagaa tggaaacaat atttcaaaag aagacagaaa 28740
ttggaacttt tatttgaaat ctccctatca tgaaatcccg gcaactaact catcacacta 28800
gctgagccaa agcacttcca gcttgcatcc tctactcaca ccatctaaaa caccaatgcc 28860
aaacaagcaa ccagagctaa caacattgtt gtgctttttg aaggcctcga aacagccctc 28920
tgaccaagca gggcagggtt attactccct ctttaggagt tgaagaagct ggcccagaga 28980
ggcttctcta tttgcacaag attactcagc tagttagtag caaaggcagg atcagaactc 29040
acatctcaac attctgcctc aaaggctcct ctcccaaacc ccaccttgtc tccacagcct 29100
aatggacagc agagccaaag cagttaataa aatagtctga gaccaacagt gttggctagg 29160
tgatcatggt atccttagca gagaaataga taggcagtct actacattct cactcaatct 29220

ctttaagtag aagagttcta tatcaagtga tcagaagtct aactttcttc accaaaacag 29280
agagttatgg cccctgatta attcccagat ttgagccaat tgacagatgc agaacccgta 29340
aatgaaggag agatttggtc ctcttgagga agacccctgc caaaaattta cattgttaat 29400
atttctccca gctttctcca aacggaccac tccttaccag ggtaactatg cattgggaaa 29460
ataaaataga cttttggagg attactagat taggtgaggg ctctgaccag gctctaatga 29520
cactaattcc tggagaccca aaacatcact gtagccccca gtcaaaggtg ggacttctgg 29580
aggtcaggca atcaatggag atttagctca gttccttctc acagtgggtc cagtgggtct 29640
gtgaacccac cctgtagtta cctctctggt tccagaacac ataattggaa taggcatatt 29700
cagcaacttg cagaatcgcc actgcacagg actggctaga ttcttttaag ctgttaagaa 29760
tatgtatcca aaacttgtta ataaggcagc aagcccacct caggctttga ttctccagga 29820
gaatctgttt tgtttgttta ctatttacca tccacaaagg gcctaaacct atgagataga 29880
tgctaacagt tagaaaagat ggtaacatgt gagtgatttc catccagtag agaagattgt 29940
ccaaagcctt atgattttgt tgccctgtga gacattaagt tttgcatcta atttagcaat 30000
cttattccag atttcttcca cgtatatatg tatatatata atttttcctg tatcctcttt 30060
tatttattat ttatttattt attttgagat ggaatcttgc tctgtcaccc aggctggagt 30120
gcagtggcac aatcttggct cactgctacc tctgcttccg ggttcaagtg attctcctgc 30180
ctcagcctcc caagtaactg ggattacagg cacctgctac cacgcccggc taattttgta 30240
tttttagtag agatggggtt ttaccatgtc ggccaggctg gtctcaaact cctgacctta 30300
agtgatccac tggcctcagc ctcccaaagt gctgggatta taggcctgta tcctctctta 30360
aacccagctt tgtcattctg ctagttgtct cattcatgag ttaaatgaat ctttgacatg 30420
taattgtttg agaaacagat ttttaatctt ttgaaaaata ttactttaac agtgtacatg 30480
actcaaaaaa ataaacattc acaggaaaat tccatagctg ggatggatta ggaagctgag 30540
tgagctccat tgtcccattt cttctcccct cctctggccc cctccagccc aacacattca 30600
actcacaccc cagcctgcct gccagcagcc caagaggcct gcaggaaagc ctgcacgctt 30660
cttcttttaa tggggatctg tgcgtcaagt tcggccgttg tggttgtagt acttactgct 30720
tgcaaagtaa gaggtggctg accccaaatc atattgggac acaggcatgt ttcaggtaat 30780
tgagagcatg acaacctctc caggagtcag ataaaagcca ggagaaaggg caatgttgtc 30840
tgacagccaa ggggccggtg gatcctttat gtaaataact tgaaaaataa atacacaaga 30900
aaagcagcaa ctgtgaagct gtggtttcca gaagatgggg aaagcttgtc tgaagtttag 30960
cccttgcttt ccatgttgag gagcaagggc tagggccact ggtgtgcatg cgtgtgtgtg 31020
tgtgtgtgtg tgtgtgtgtg tgtgtgcgcg cgcgcgcgcg cgcacattgg gggacaggaa 31080
atgagacact gctgattcag atccaccctc acctccccag ggtcctactc aaaccacaca 31140
tcttctggaa ttcttccctg accactgacc actgaccact ggatatccca gtagtatggc 31200
ccttctctga accagtcccc atcttttttt tttttttttc tgagataagg tcttgctttg 31260

ttatccaggc tggagtgcag tggtgcaatc tcagctcact gctacctcct cctcctgggt 31320
tcaagcaatt ctcctgcctc agcctcttga gtagctggga tcacaggttt ctgccaccat 31380
gtccagctaa tttttgtatt tttttttagt agagacgggg tttcaccatg ttggccaggc 31440
tggtctcaaa ctcctgaact caggtgatct acccgcctcg gcctcccaaa gcgctaggat 31500
ttcaggtgtg agccaccctg cccagccact atcccttttt gcctatagta cttcacacca 31560
tactttactt tatgctcttt attttattag gagttagcag gcctgtattt tagtcctggg 31620
gttctgcatc ttgttagctg agtgactttg ggcaagttac ttaatctcca tctccctctc 31680
taagccacag ttttatcacc tatcaaatga gtacgtaaga aagtttccta ctttctctac 31740
tccatggggc agtaaacatc aagtcctagg ttcacatcct ggctccatgc atacaatatc 31800
aacaacaaaa aatactgaaa aacccactta actgctgctg ctcattttat tcatctagga 31860
agtgggggtg atgataacag tatctacccc attggggtag ttgtacagat tgagttgttg 31920
taaattttca atgagataat gtatgttgag tgaggatact gatgcctggt ataccatacg 31980
cactcagtcc gtgttaacta ttttaactga gaaagctttg aagtagtaag atgcctatca 32040
aatgagaagt tttagaatta cgattgcctg ttttcttgta ttcttctctg tcttggattt 32100
gttagttatg ccttcttagg tagatttcaa gttcgtaaaa taattcgaca actatttatt 32160
gaatacttta gaaatgctaa gcaatgtaga taacaaaata agcaagatag agtcattatc 32220
ttccaggaga ccacagtgca gggagagaaa caagtccaca gatgattaac acgttccatg 32280
acggagatgc cggcacacgg aaggggttgt ccccccaggc tggactccct aaagacaggg 32340
ctgggtcaca tgatttttta aaagtcccct ccttattcct ctgcaccttt tactctactc 32400
ctaggacaga gctttgtatg tgatagaagg tcaatgtata ttagtcaaat aaatgtgtag 32460
aattgagcaa tcattcttat tatctaggtt acccagagtc cttttgatgg ggggctgggg 32520
ggaggatgga actaatatta ggaaggaaac tagtattaac agagaacctg gcacatccca 32580
aatactggga aagacatttt atatatggac agtggtgtgc tggggctggt gggtggctca 32640
ctcttcctca ctctacattt aaagaaggca ggttggtggc ttcaaatcag ctacagtttg 32700
agtctttaca caataaaatc attaaatgct tcaaattaga gcattattat ttttggagag 32760
ctagttgtta aaatatttac cagcggacac tatatggtcc tcttgcaata atctagttac 32820
aggtgaagga attaggattc acgcttgaac ctgggaggtg gagatttcag tgagctgagg 32880
tcaccccact gcactccagc ctgggcaata gagtgagact ccgtcatgga aaaataataa 32940
ttattatata tatacatata tatatgaatt aggattcaga gaggttgagt aatttgctcc 33000
aggtcacaca gatcatagaa ggcagacttg ggacttaaac tcatctgatt ccaaagccct 33060
ggcttttccc tctgtaactt gtgacccacc catccccctt gtgtcccagt gggcttgatc 33120
aggaacagat tagtgcattg tctgtacttc gtcctggctg aagggcagaa cagaaaagct 33180
tcactctcta ctcagtctcc ttgtaaaagt cagagctccc tggaccaggc acacccatgt 33240
tcactgcagc attattcata atagcagaag tggaagcaac ctaagtgtct actaatggat 33300
gagtagatag agaaaacgtg gtatccacat acaatgggat attattcagc cttaagagaa 33360

ggaaatccta atggaatcag atagagatcc aagaagtaaa cccatgcatc tatagtcaat 33420
caattctcaa caaaaaggcc aagaacacac agtgagaaaa gggcagtctc ttcaatgaga 33480
ggtgttggga aaactggctg tccacatgct gaagaatgaa attgtacgct tgtctcaccc 33540
tttataaaag aatcaactta aaataaatac ttaaatgtaa cacttgaaac tgtaaaacta 33600
ctagaagaaa atatagggga aaagctataa gatgttgatc tgggccgggc acggtggctc 33660
atgcatgtaa tctcagcact ttgggaggcc gaagtggatg gatcacgagg tcaggagttc 33720
gagaccatct tggccaacat ggtgagaccc cgtctctact aaaaaaatta aataaaataa 33780
tacaaaaaat tagccaggcg tggtggcaga cgcctgtagt cccagctact tgggaggctg 33840
aggcagagaa ttgcttgaac ctgggaggtg gaggttgcgg tgagctgaga tcacaccaca 33900
gcactccagc ctgggtgaca gagcaagact ccatctcaaa aaaataataa taaataaaaa 33960
agatgtcgat ttggcaatta ttttttgcat atgaccccaa aatcacagtc tacaaaagaa 34020
aaatcaacaa actgaattac atcaaactaa aatgtttcta cgtagtgaag gaaacaataa 34080
attgagagaa aatatttgca aatcatacat cagataagga gctcatatac aaaacatata 34140
aggaactcaa actactcaat accaagacaa aaaataaccc tgtttaagaa tgtgcaaagg 34200
acctgaatag gcatttgtca aaaaaaagac atgcaaaagg tcaacatata ttttttaaaa 34260
atgctcaaca tcactattcg tcagggagat gcaaaataaa accacaatga gatgtcacct 34320
catgctggat agaatggcta ttatcaaaaa tatggtagac cacaaatggt aacaaggatg 34380
tagagaaaag ggaacactta taaaccgttg gtggaaatat aaattaacat agccattttg 34440
gagaataata tgcaggttcc tcaaaaaact aaaaatagca ttaccatata atccagcaat 34500
cttacatctg ggtgtgtctc caaaggaact aagatcagca cattgaagag atatctgtat 34560
cctcatgttt atggtggcat tattcacaat agccaagatt tggaaacaac ctaagtgccc 34620
atcaacagat aaagattgaa aaaaacaacg tgatctatat atccacaatg gaatacaatc 34680
cagcctttac aaagaatgaa attctgtcat ttatgacaac ttgggtgaat ctaggggaca 34740
ttacactaaa tgaaataagc caagcacaga aagacattgt atgatcttgc ttatatgtgg 34800
aatctacgaa tttgaactca tagaagtaga aggtaaaatg gtggttacca gagcctattg 34860
gcagaatgag ggttgactaa gttttaatta ggcaacagga ataagttttg gtgatccatt 34920
gcattgcaac atgactatag ttagtaatga caagttgtct atttcaaaat tgctaaaaga 34980
atgaattttg aatgttctca ccacaaaaaa atgataagta tgtgaggtga tgggtacgtt 35040
aattagcctc atttgctcgt tccacaatac atacatgtat tgaagcatta cattgtaccc 35100
tataaacaca ttcaattatt atttttcaat aaaaatacaa ttttttttaa aaaaagaaga 35160
aaatcctgtc acatgctaca acatggatga accttgaaga cagcatgcta agggaaatag 35220
gccaggcaga aaaagacaaa tactgcatta tgtgaggtat ctaaaggagt caaattcata 35280
gagacagaag gtaaaatggt ggtggccaga ggctgcagag agagtaacat ggagaattcg 35340
tgttgaatga gtatagagct tcagtgtttc aagacgagaa agttctggag atctatttta 35400

caataacata aatattctta acattatcaa actgtacacg tcaaaattat taagatggta 35460
aatttcacat catgtatttt tttaaccaca gtaaaagaaa ataaactgtg atccccaatg 35520
gaattcacat gcacacatcc gacctgcagc aaaaaggaat gaatggaccc ctgggctcac 35580
cgtgggcctc catgtgcagc atttgagagg cagccaccac cataggagcc accaggctgg 35640
gagactgaca ccaggagacc caagcagctc cggaggcaat gggagagaca gatgacagta 35700
cgtaggtagc ggccgggaag acagacctgc atttgaatcc tgactgtgcc gcttccctag 35760
aggagtcact caacctctgt gaggtgcagt ggccacatca gatggtctct ctccctccag 35820
tgggtctggg tagttaaaga acatatgcct agtacagatg tcactttact cagtaaatat 35880
ccactcatcc aattacagaa agctacaagt aaataacaag aaggggagat ctcagcaata 35940
caaagcgacc ctgttctctt gttcagtgct gctttgtgga ctgtacagtt ctatttctgg 36000
gcagggccct caaggatgaa tctacaaagt ttatagtact tctgcttcca ccgagttgaa 36060
agagtgcaat agaaccggcc tcactggctt caaggtctcc tgccacccct tgccccagga 36120
actgagctcc agaatcccct ctccattacc acccccccga ccccccacta tttacatgta 36180
aatgaaaaac gaagaaaggg gagcctggga ccctgttgac tatgaatgaa aatttgtcta 36240
agttctccac cttctgcgag agctgcccct gagctctgtc cgcacagcaa aatccagacc 36300
tctgaagtat ctaatttgga aattatgggc tgtccgagtg gcagctctcc aagctgttag 36360
cttgatgctt gtcctggtcc ctctgcaggt agcagttgca atcagcgata ggctctgcca 36420
ccttagccct gctcattaga gccagggcca attatgtccc agccctggaa gctgaaccta 36480
acagcagcct cttgttccaa gggctgttcc ccagtgggtg ggacacagca ggccagcagc 36540
agcctgggct ggggaggggc atggaagatc tgtgccctcc tgggtggtag ggaagccaga 36600
ctgttgaaaa ggaattcaaa ggacagaaca agactttctt agctcccctt gggctcccag 36660
ctagctggtg acacagccga gacctgcaat tagaaaaaca actgcctgag gcaacagctc 36720
tgctggttaa atggtgccta acctgggtat ttagcaattt ttatatataa acacacacat 36780
ccctgtaaca cacagtgtat gggcttttgt ttttaaattt taaaatgatt ttctgccctc 36840
accttttgat aaatgctgtt atcagtaatt tcctcatttc gtgctttgat ttcctttttt 36900
attgcatatg taatctctga cccacatggg tgatatggga tgccaggtgt gggcctacaa 36960
atatcaccag aaaaaaaaaa atgagagaaa aggttgtgtt tactctttct gaaaacacag 37020
ccctctatgg tttctgttgg agaggccaca ggatcaccct ggcactggca tgtggatata 37080
ctccttcctt caaatgcatc agctaagatt aaagcactaa tttatttgaa aggctatgta 37140
ccagttttga tctgggggaa aaaatgctgg tccaccacaa cttatgctgt gtgtatatca 37200
tcacaaacaa gctttgatta ttgtgtgtgt gtgcatgtgt gtgtgtgtac acatttgcat 37260
ataaattagg atttattgaa tgcccactat tttggcactg tggactatac ttctatattt 37320
aaaagcagtc tccaacttac ccaagttcta aaattttgga atatattcat tcctttattt 37380
gtacatcctc tttacaaatg ttcagggtct actatgcact aaacaccaag ggcaagatct 37440
tatgtgaggt cattatcgac ctcacatgct gggggcagcg atggggcagg gggaaataag 37500

aggcaagcca tcacatatgt tgtgatatat atcataatga aagtgaagaa gataaaccta 37560
gcatatgtag aaaatatgat ctggggcagc atggaaatat tgtatcagac agatctaggc 37620
tcaaataaca gcttcagttc tcaccacctg tgtggccttg attaacactt tactttctat 37680
ctctgagtct ccgtttcctc ctctgtaaaa tgggagtaat aatttctatc ttttatggct 37740
gtcacagaga ttaaagctga tgcatataag gaagataata tagtcagtat ttgaaagtgg 37800
tagatattat tcacttggat aaattgaatg tactgtgggc aagaaagcat ctgcttgaaa 37860
aatgaacacc ttaatagaat atgtgtttta aagcaaggtt tgtcaccccc aatgcagctc 37920
tggatggaca aaggcagaaa agtgcgggtg ttgttggtta tagtctgtaa gggacttcat 37980
ccaatccaat accatcattt tagaataaaa aatcagagga caagaaaagt taactatacc 38040
taagctgcca gttagatgac agaactgaga ctagaaccta agactcctaa ttctagagac 38100
ctccataaag acccatctga gtccattttc cttagaaaga aagccgagca gtgagaacta 38160
aaaaaggtgg atctcctgga ggcagagaat agtagtagtt accagaggct gggaagggga 38220
gggggagatg gagagaggtt ggttaataga tacaaaaatt cagttagatg gacagaataa 38280
gatctaccaa taatagtata gtacagtgac taattagcaa taacatattg tatcattaaa 38340
aatagataga agagaagatt ggaatattct caacataaag gaaagataaa tgtttgaggt 38400
gacagatatc ccagtttccc tcatttgttc attacacagt gcatgcacgt atcaaattgt 38460
cacatgtacc tccaaaatat gtacaactat tatgtgtgta ctgctaatca gcaaattagc 38520
agattgccaa atcttcaaag aaactgggca gtgggcccca aggaatagtg ctgtgggtct 38580
gggaagaaag aaatctatgg gattccagct ctactttgca tttctttgct ttttgtgtcc 38640
attctattac attttcctgg atctctgtgt ctggcagaca gaaactcagc ggctatgatt 38700
agaggcacta tcaaattgac tgtcttcaga caagctgctc atttcaacca ttattccagt 38760
tccctcagtg gggctgctcc agggtcgaag ctgccagtgg accctgccct ctcctggctg 38820
gcattggtgc cctgccctcc ccagtcaccc ccagccccag cagcacttgt gtgctgccac 38880
aggctgatgg aacagggact gggcaggaca gggagggagg acgtggggat actcaggtgg 38940
gcagactttc ctgagccaca gagaatctgg gttctgcctc aagctggact ggctgtgatc 39000
ttgacctagg gagacactcg agctgccagc ctccaaaagc ttcctggggt cagaggctcc 39060
acctgttgct taagagctcc ctggctcctt ttctagacag aagaatgttc agaggcactg 39120
cctgggggcc ctctgccctg gcccctcctt gctgccaggc tttggggcag gcaccctccc 39180
tggcctgtgg gagcttggct gctcagaggc acatggacac tgctggccac atgtgcccct 39240
gtccctgccc tttgctcctc tcctcccacc aggattatgg actcctgtgg ctgtgacgtt 39300
accgtggaga cagggtgtgg ggagatgctg cagagggcag ggagctgccc agggatgaag 39360
acgagatgaa agccagagaa actgggaggc tccagcatcc tgtgcactca gggaggtagt 39420
gaaggcccaa tgccaagtcc caggtggagc ctccaaggcc tcaagtccca aagctggcct 39480
gagaacagct ccttggaaat cccctgtacc tccttctctg ataccttccc tcacctatca 39540

cagtgaagga gttaactttt tctccagact tactcaagtc attcaacaag taattactaa 39600

gctatggacc gggtcatcag agacatccag attggaaagc ccaagagggc aggctctgaa 39660

ggctcagacc tgctgggaga tattacagac agcaccaaat gttgccacct gtgtgctaag 39720

acaggcctgt tggaagatga atctgcgagg ccacaggatc ctcatttaca ggggcccaga 39780

acaccttgct agcaccatca gcatccccta tgtccttaca gactggccag ctgggcaaac 39840

ttaatgatgg ggctcccagt ttcagagagt gagtcccgct agttttgtct aagaggtcct 39900

tgatcttctt ctctatggag agtacaggtc ccaggcctcc catccaccac ccccagcaaa 39960

gctgaactca atggcaggag tcaggaagcc ctccttaccc ctgcctccac cccacacatc 40020

agggagcagc agtaaaatga cttctggaaa cacaccctgg gcacagtcaa gcaggggttg 40080

ttactattat tactattacc tggtggagat gagggatccc ccaaggcaga ctggagtata 40140

taagagaaga acattgcttt gtgtgaggca tggtgagagt taaagactag gattttagtc 40200

ctgactctgc ccccaaaaga tcctgggtaa ttcacttggc ctaccggagt tcagtgtgct 40260

catctgtgaa attagagcat tagtctgaga tcctggtgta taggaggcat ttaataaatg 40320

cttgtagaat gaacattgag taaacaactt caaatccctg agttccttat gactatataa 40380

agcacttaat atgcacatcc agctgccctc acggggagca attcctgagt ttcttatgac 40440

tataaaaggc atttaacatg cacgcccaat caccaccctc acagggcctg tgcccaagtg 40500

ccccagaaat ccaggttccc atgggccttc ccttgctgga acattcaagg gacccccttc 40560

cttcccctct cttcctccaa actcccttta ctttcccccc ttaccaacca ttgtgccagc 40620

aggtcagtga atccttttta atcctgaact cccagttgta ctaattgctt tctttcttgt 40680

cctcatttgc cttctaactc aggagatgag gcttcaatta gataagtggg cagctattcc 40740

caaacaggtg actaggaagg aaaacagtcc tactctggag gaaacaaaag atgtctataa 40800

ggaacaggat ccaccaaggc aggaagctcc cttacag 40837

<210> 15
<211> 3862
<212> DNA
<213> Homo sapiens

<220>
<221> misc_feature
<222> (1)..(3862)
<223> chromosome 11 nucleotides 131627190 to 131631051

<400> 15
tcagcctccc tcctacctct ccttttccct cccctcttag ccagggctgg ccgctcagcg 60

tcagttctac ttggcaggcc ttcttttcct catctttatc ttagcatttg tttcccatct 120

tatgaaaaat tgcttatcta cctacaatca acctttcagc tcagctccct atcagctcct 180

ttatcagctc taccatccta acttctcatt tgtttctgac aggcatcctg taacaaattc 240

ttaatttgat gagcttgata tagcccactg gctaaacagt gagctgaagc cagggctggc 300

tgcaggaata tcccatactg ttcagccacc cattttcttg agagcagaga gtccccaaaa 360

aagggtggaa atgggagagg agcaattctg tggggcatat ggcagcagga aaagaaagcc 420
tggaacaaca gttttccatg gggaagcctg gaagacagtg ttggacagag gcacctgagg 480
ccagagatgt agatgggtag atgaataaga agttggttaa taaaagagag agaaaaacaa 540
cttgcagctt tgagatactt cttggtaatt atagatggga aacacaggct cggatacatg 600
aatacctggg tctttttgtc ctgatatatg gctgggtaat cagagcagcc gtccatgtgc 660
accatgattt ctgaaaccat tgttcagagt gacattgact gtctctgaaa ggttaatggg 720
tatttacgta gggaggaaaa atgtaccttg gttaaataag tttaagcaac tgtgtgtgct 780
ccatttccca tttggagatc cataggatat aagaacttaa agattctaaa aatcctgccc 840
ttgatctaac gaacccagtt tccccagact tgtgtgacaa agatgtcctc tttgcagaat 900
actaattaca gtaccgtctt gcaggatact tagaagaaaa tgttgattct aaagccatag 960
taggctctgt tttacctctg ctgctattaa gggattctct gtccaacaac aatgtttcta 1020
gaatggtaac tgtgagcaaa atgtcctttc tggaacacat gccatgtcct ggagcttagg 1080
atctggtaat cttctatctc actatctgat gatgataatg accccccatt tattgagggc 1140
ctgttaatct cctaggtgct ttttctatgc atgacttcta atcctcacta tgatcccaca 1200
ggagagatag cattacccca tttcttctat gagaaaattg agagtcgggc ttattaaata 1260
atgtgcccaa ggtaagcggc taagccagga tttcatccaa ggtctggcta actctaacat 1320
aatgtttgtt ttcatcacac tatacccatt atgaaaatgg ggcagatttc aaaaaacaga 1380
gaagaatgat ttaaaaagca aataaattta gcagcctgtc tagttacata caaggaccac 1440
ttattgattg aatgctagct ctaggaaaac aaaaatctga aaattatcta gtcctattgt 1500
ttatttttcc ccttgtaaat aaagaaactg agatctgtag aaagtaaaat gacttgctca 1560
ggctcacgtg gccagctggt ttcagagtca acatgatcac taccaattac cctgacttcc 1620
agtccagagt tacttatacc catagtgact tttcccccct cctctcagag actgtttttc 1680
aggctaagac ttgccaaaat gctcaatttg cagtgtgaag atgataaaga cctcctcagg 1740
cctgccctca gctcggcccc tctatgagga ggcatcagga acaaaggctg tcctttccat 1800
ctgcatatac tgaggacctc gtgcagagga cataggagct cagcttgcca tcgtgctgca 1860
ctctcctgag ttctccctgg atatggagtc tggagaaatg aggcagtctc tgcaggttta 1920
gctgggaact caagccaagc aggggctgga aggcagctct ctggactctg ccaaagcctc 1980
ctctcacctg gtgttgtttg ctatccagac tcttcctgtc caccaactct tctagatttg 2040
agctgccaag taaagccggc agccaggcac ttagccctta gacgcactga cagggcctgt 2100
gtgttctact gagcacccgg ctagagacag accttatctc ctgcctgccc ctccctgggg 2160
agtgtctccc cgcaacccgt cttcatcccc agcattcttc aatctccttc tcgcccactt 2220
gtctcctggt tgcttctcct attatttctg tgattcctct cttccttttg tctttatcta 2280
gaactgttgc tttgtttccc cctcaccttt tcccatgttt cacagaaggc cagtctcctt 2340
ttccccacgt ctctgtacac acccactgtg aatgcacttt gttttcacct ctcctcagct 2400
gctcagtcct cctaagtgtc tctctgtgct ccgccgtggc attgttctca agtcccatct 2460

accgctggcc ttcctttggc cactcttccc tcattccttt aattttcttt gacctctttg 2520

tatatttttc ttctccaatg tgatcccttc tctctctccc acttctataa aagactcggt 2580

ttctgcatca ctggaagacc gcaccaccat tctgtggtga tggtgatggc tgctgggtta 2640

aaggaaatca gtgtctcctc ttctggatgg gtttggatgg tttccagtga ggtcagaatc 2700

ctgttgcccc tgctgggatt ctccagcctg gggttctcat tcttggcgcc attgatattt 2760

aggctggcga atttcttctt gtgggggctg ttctgtgtat gatgggatac cgggcaacac 2820

cccttatctc tacccactag atagcagtaa caagcacatc ccaattgtga taaacaaaaa 2880

tgtccccaga catttccaga tttcccctgg gaggcaacat cttccccatt tgggaagcac 2940

agccctagcc cacagcctgc agccttgctt ctagaaccac atgcggagtt gaggtttcta 3000

tgcctgttgg ataagcagaa aaagatgtgg aagtggtgga tgctgtcagt ggagacacaa 3060

gaagtgtgtt tgccaagata aatttgagcc tgaacatttt ttttatctct ttggtttgag 3120

tctgatctct ttctactcct ttcttttagc tctttagctc tcttttggct ctcttttagc 3180

tgtcttttag ctctttcttt tagtcttctt tcactgtaat ccaaacttct ttctctcctg 3240

acaatatgac gttcttgagt tgcagactgc taaaaacggc tctgattaag ccctttaagt 3300

gattcctgca gaagtggctc tgacggtgtt ccatctgtac cctcagaaag gtcaaggtgt 3360

cactcggctg ctcgagggaa ctctgcttct gggctcccag gtggttaatg cctgtttgat 3420

tagctgtaaa acaaaatcca gaaaatgcca agaggcaaat ctcaattcac ctttcagaat 3480

gaagctcact gcccagtcag agaagggggt gcggagtggg tggtgagatc cacagctgtt 3540

aaattcgcaa caggtaagcc agcaaataga ctcagtccta atgcggctgc cctcatctgc 3600

tgaaccacct gagtcacctt cacctttctg ctgctgactc ttggctggaa tgtggttcag 3660

tcctatttgc tctgctttcc atgcagctct gccttccact tgggtgccca cttccattcc 3720

agtcagcctc ccccttccct gctccactgc cgtcctgttg ctttccttcc aagctcacct 3780

ctacacccag gaagctggtc tcggttgcac atcttcacct cgctcctctc acaaatgcat 3840

ctcactccca aatctgctgc aa 3862

<210> 16
<211> 27590
<212> DNA
<213> Homo sapiens


<220>
<221> misc_feature
<222> (1)..(27590)
<223> chromosome 12 nucleotides 26159614 to 26187203

<400> 16
gtcctagatc ccttctgtta tagagaacct ctagggcaca accaggacca cacaggtata 60

gaggggcaga aagcccaccg ttatgtggag acttccaaac taggaaaggc acatttctgg 120

atgtctccaa agtgagtgga tgatggaata gtatggttat ttgtaggaga ctttgaaaat 180

tatttcccta gaatttcatc tctctcaaaa ttattgcatg ctctatgcca taatttagca 240

agattattat ggtgataatc tttccttctc caccagcaag tatactctgc ctttgaggat 300
cgcacatggg ctagctcaac tcattccatg agaaaacctt agcctgtccc tccccattac 360
taaaactatg gcataaaagt ataaagagca ctagactcag aaccaaaaat cctggttctc 420
ttactgagct ctatgacctt ggactagttc tttgcctcag cttcctccca aaggtttttg 480
aaaatcaggc cttaggatgc catgtttccc tctagaactg ctcagtctct ctcactccct 540
tactcgaagg gaaagaagtc accattagtt tcttttgtaa aacagataga tggaggaggt 600
gagacaaaga gctgctcatg aggtactgct ttcgttcccc taccacatga gatatgatca 660
tgctctgaat actatcatat ctcacttcgc agcaggaaat atcgtgactc cctagtccca 720
gcccatggtt caaatcctca ggaggcgatt ttggtgagag ccagcatagc agtcacgctc 780
taaagagact ggcaagttag gaaatccttg cttagacctc aagtcaaata tagccaatta 840
aactgtactg cagcttattg taaatggcac tttttaaagt ttaatttcca gttgttcatt 900
gctagtgtat aaaaatacaa tgtatatttg tttattgatg ttttcttctg tagcttttct 960
aaattcttgt atttgttcta gtaggatttt tttttggtag attgattccc tatgatttat 1020
gtacctgata atgtcatctc taaataaaga aaattttact ataaaaacgt aaataaagtg 1080
tagtgcagct tagcacggga gcagggtcgg ccagaacctt ctctttggag atggcctcat 1140
gcccttattg tgctgccgtt ccttctgtaa aacccagcta cttctcacct aggcctcctc 1200
acttggatgt aattagtagc tcccaccccc accacagcag tgtatccatc cctggataac 1260
acttactgtg ttgcaatgta atttatttta gtataggtct atcacccaca ctagactctg 1320
agatcttcag aggttaatct ttgattctct agcacttacc atggcacctc acatacagca 1380
agcaaaacaa atatctgaat ccccacacca tttattgaat ccataaactc aagaagaaaa 1440
ggaatatgct gtaatcgatg aggagtctga aacctctcac catgggtgtg tctaaattca 1500
gaggcagcat ctggacctac acacccatcc tgccagttcc ccagaatact tttgcattct 1560
gccggctgcc caagaagtcc aatacccaga ggcaggggag gttacatact agaagcagga 1620
gccagagtca tccttaaatc aaaatgctga gtcagtttca ctagtggccc tccctcccct 1680
attcttagca actagagaga ggtgttcatc taatcaaata acttattcct gacatgcaaa 1740
tttgtttatt ttgtggttag ttgtgggcta aaaccattag caagttgctt ttatttttat 1800
gtaataaaat gatggcaaaa tatataacgt atttgaaggc atttgcataa gtaatcttga 1860
aggcatttgc ataagaagca tcgctgagaa tctggaagtt aaaggaaata aaaaagctga 1920
gaatctatct ggaagttaaa ggaaataaaa aaggtcacag attctacaaa ttcacacaac 1980
ttacttgtag aacaaaccct caggtcctct gtcagagcct atttactcta attttagcac 2040
atttcaaatg ggcgtggtag tgtttaaatg gtttcccagc ccacagtaag tcagaggtag 2100
caaattagat aagagatgga ggaagcactc tgaggcttca agaagtgaca gctgacaggc 2160
tagctgtgcg taaagagact gaaaaaccct ataaaaagca ggtagaactc aaaaaaaaat 2220
ttggtgcagg tgcaattact cccagatgtc atgcatagtg caaaaatgat gtttacaatg 2280

atgaccctga attattgaga aaggttaagt atgtccaggt taagacattt agaggatatg 2340
tgtaatacat tttagtatgc tttccactaa aaattcaaaa atgaaaaaca gcactggcat 2400
ttgaacatag aagttttagt tttctaaacc actgactgtc cctcctgtcg gacaaactag 2460
gcatcactga atttagaatt ttaaaatatc aacatttaaa aaaggtggtt ttatatttgt 2520
ttggagtttt caatatggaa tctctgaacg agcataagaa acccgatttt aagacaaacg 2580
tacaactcct agaatagcat atttttaaag tacatgctgc gcatgttttc tagtataaat 2640
cccatcttaa tcacacaaaa tatgtgataa tttcccacgt caatccctta cttaaattct 2700
tctatgaaaa actagaactt ccatgggact ttagcatgca ctgtaaatcc atcttgaact 2760
tgtttaaaat aacagtgaga acaatgggtg ggtctaaata tattgagctg agggtccaat 2820
aggttaagaa tttttgttta atgaaaaata tcaggttgct acctgaagta aaataccatg 2880
atcacaacag tacagccagc ttttggcaat ataatggtct taagtcattc atgagagctc 2940
tgttcatttc tagctaatgt aaaagtcctt cgtgtgtttg cccagccatg tctctccaga 3000
ggctccactg gcaatcactt tccatggaaa ggttaagaat tgacctgtga catagaaagg 3060
agtaggctga gcctcctttt acataacttt tgaacaaatc tcgggtggtg ggcaataacc 3120
aaagtcatcg cctggatggc cagaaacagg gaactaaatg aaaaatagtt tcacaatgat 3180
aggaactaaa actccaaatt ttacatagct attaaagtgt tgtttctagt gccttgaaaa 3240
cgtgcttaag gttagctctt tggaggggag ggaagggcca ttttttttca attcaatata 3300
aagtttgcta agaaatgtgt tatataaaat taatcttttt aaagattatg taaaatatat 3360
gtatgtaaaa ttaaattttt atgagtataa tactatactc ataaaaacat tgactcaaga 3420
ttcatatttg cctgaagttg gaaaatcatt ttagctgaaa tttcaccact acaagtatag 3480
aagcctcttt agtgaattat gacttcagcc ttttaattca ttaaaataaa tcaatgacta 3540
aaataaagtt ttttcagatt ttcagataca atatagacaa tattattttt gcatttctgt 3600
tctaacttta tcttaccatg ttaccatatg ggcacttgga ctaaataact gacttgtaaa 3660
atctggccca actctgaaga tttttttatc tcattatacc aaaattatac acatgctaaa 3720
aacactgtat tatcctctga ttcactttta atcctttttg gttatgaaac taatattact 3780
acagtagtgg catcaaaata tagctcaaaa tatttgaaat gctgtttttc ccattattct 3840
gattcagata atttttctaa agttcaataa attaacaatg ctgaaaaatc ctacatagtt 3900
ttctagtgtt gatttcaatt tcctttgttc tatggtttgt tacgaagctt catgggggct 3960
gctcaatcaa gttcagctta cttggtccaa gatactgcaa tgggaagtag ttgagtgact 4020
gagggatata atagagctgg ccactagtgc tagtgctgac tctaggtagc tctgtgacct 4080
tgggaagata cttaatatct ctgggcttcc tttctttttt tcctatcacg aaatgcaggg 4140
gactggatga gctgtggctc cttctggctt catactgtta tgacttgcag ttcagttgcc 4200
ataaagtcag cagagggagc actttttcta catcttatac aatctgactc tcatttaaac 4260
attttcaaaa atcaaggggt ttgtgaagaa cattcctgaa actaaaagct gacaatcctt 4320
cactgtttgt atcatatcac agatttgaat ggaaatttga gaaaagtcta gaaattattt 4380

ctgatcctgg aattccatga taaagtccca tgacagaatt attttataaa aagaagccaa 4440
aacactctca tattaagcac ccctttgtga tcatctattt atacaagacc cagtgaaaga 4500
ggaacacctg accatactag agaggctatg tgatgtcaca gacgaaaact gaaaaacttg 4560
tcaatcatta aaacaataga aaattgggtc taacataaac aaaccacaca agttaattgg 4620
taggtttttc ttttgcactt ttcattaaga tacattggga acgagcaggt agacaaagcc 4680
agagcctgga ggcttgcaag agattggaaa tgtgtgacat caagccaatg acaaaaaaaa 4740
gctgtaaatc tgagaactgt agtgatttgt atccctatag ttatagatat cttaaaacta 4800
attgagcatc cattatgtct tttttaatta ttagacaata tcatatcgta tattaaagtt 4860
tttattcctt agtaagaatt tttaaatcac aaaatagtgt ggcaatattt aggtaataga 4920
atttatggag tgctgaaaat actagaatat aaaggtaatt tctagtttat cgtcacatac 4980
taaacttttc taaaccagct ggtttctcac agacagtatt tattttttta aaataattta 5040
agacagcata agcttgtacc aagcataagc attgtaacaa aagtgcaact tttcagcaaa 5100
tcctccccaa aagatatttt aactcaaaat atcattagca catattttct ccctacaaaa 5160
atagcatgtc agacatcatt aattggatgt attaacaact atgtacataa gagccacctt 5220
gtaggctaag agtttaacgt tgtttaaaca cagcgtttga ggcaaacagt agcaacagca 5280
gcagcaaatg caccaaactg acgaaaagac ccagatattt tcctcactca tagtcagact 5340
gttgtgtctc accccttaca taacatccaa gtgagatttc tcacagtgct accttggcaa 5400
caaactaaaa atatctagac aaggtcttgg tttaagcctt attaaaaaag ctttctttgt 5460
gattatctgg tatctggttt ggtctccaga aaatacatag acttggagat aggaaggcct 5520
cacaggactt cattctatat ctttacagca tttgcaatca aaactggcca gttaaatgct 5580
ttcgttcact tttgaatttt aacagaatat aggatatatc agtttggact tgttttttgt 5640
tgttgttgtt ttttgttttg ttttgaagtt cagtgcactt ttttttacag taagattaca 5700
aaaatttagg aacatgatgg attaatgaga gtgggaatag atgcacttga gatgtgtatg 5760
tatagatacc tatctatgga taaatagaaa tctgtacaga tagatatcta tctatatatg 5820
gacttagaag aaattaagat gagtccatta attcatggtc aatttctcaa taaagtgtta 5880
gcaaatgtac taggaatgat tctggccttc tcatccatct ccttccctga cttagttctg 5940
acgatggccc agctccgtgt ttctttaacg agtgcaagtt actaagagat accatttaaa 6000
ccacttcaga ataggttgca acgtgagaaa ctgcatcctc acttctttga aaaatatatg 6060
ccttttagaa agctgaacat tagaaaatac tgaagagtaa attcaattct aaaagagcct 6120
caatctgttt gtggaacgcc tcctaaaaca ggcagggcag ctttgagaac tagctctaca 6180
taacaactac ctgctgttcc atgtcaactc caaagacgtc aaaggtgtta acatctttcc 6240
cctaccccgg ggttcaccta gctcagccac agaacagacc cttcttactt cctctcgaat 6300
taggttccaa ttttaagaga taatggaaca tggcctaaaa gggggcaaaa tttatttggg 6360
ggattaaaaa aaagagccag tgtctttcgc ataggatctt ttacagctgg tggggggaag 6420

aaagggatgt tagtgtgtgg agggtggggt ggtgcgggat gagcaaaaca ggaactccga 6480
atgtacacat aacacctgtt ttgttgttct tgtttatgcc tgtcgtgcat ctattacact 6540
tcctccctta aagaccttaa gggtatttta acttctcact ctgcttgaac ctccttaagg 6600
gtattttaac ttctcactct gcttgaacct ccgtccttcg ggacgcaagg attcagggag 6660
cttcctttcc tggctgcgag ggatcttcct gagcagagct ctccgggttc cccggctcgc 6720
ggggcccggc gaagggcagg tgggtgcggc cgtgcgggtg ctgggggtgc ggcgccccaa 6780
ggggcgccac ctcgtgcggc aggagggtcg cggcggcggc gcccgccttc tcgggagggg 6840
gcgacaacac cgaggacagg caggggaacg cggcggcggc ggcggcagcg gcggcggcgg 6900
ctgccgcggc tgccgccggg gcggggatgc cggggtatag cagcgggaac ggggcggcag 6960
ccgccgccgg gtacagatac ttctccaggc cgctcttgtc caggaagggc tgcacgtagg 7020
cggcagctgc agaaggcgag aggaagcaga agggcaggca gaagggggcc gcggcggccg 7080
cgggctgcgg gaagggcgcg cctccgcctc cgccgaacgc caccagcgag ctgagcaggg 7140
cggcgtcggg tctcagcagc gcggccgcgg cggcagggtc gggccccaga agcgcggctg 7200
ccgccgccgc cgcgccgccc cccgggccgc cgccgctgcc gccgccgcgg gaatccagct 7260
tcatcctctt gggcgccggc gagtcctccc cgggaggctc ctgcttgatg gtgacgcggc 7320
tcgcccccgc gcctttgcct ttctcgcggt ccggccgggc ctcggcttcg ccgccgtagc 7380
cgctgtcggt gtccgtgtcg ttctcggcgg cgagctcggc gctgggctga gtccgctgga 7440
tgacgggcac gcagtaggcg aggggctcca gcttctgccc cgcgcgctcc aggcaggggg 7500
cggccgcgga cccggcggcc gagggagcgc cggtgccttt gctcagaggg acctgttgag 7560
tcaacagctg cggggtgggc aagaactggg tggccacggc gtgcaagtgg ttgatcagct 7620
ggacacaccg cggctccctg ggtgtccagc tctcaaaccg ggagaggtat tgcaagactt 7680
ctttggcgca tgtttgaaat cccgagtgga acgcatccaa gtcggactga atgggcgatt 7740
tcagagatcg ctcccctagg atgaggaagg gatgggggtg ggggacggag gagtggaggc 7800
aagaagaaac atacccacgt taaaacatct gcttatcacg tgggccctgc atcgactaaa 7860
gtgatctcgg tttttcccca gtattcaagt gatataatcc accagttgac gagagaagcg 7920
gggtggcgga ggtgcggagc ggggagaggg cgctctcctc ccagctgaaa accaaagtgg 7980
agcgggtgca accgccactt taaatcctga ttcctccgag ttctgctgaa agcctctagg 8040
atgcttcggg aaatgggctc gttccaatga agggaaagta taagcaattt aacaaatgag 8100
agggaaccca tgagcccacg gaaagagatg gggtgcgggt gagggagtcc tgacactgct 8160
cccctgtggg ctgccccgct tcatcagggt aggctggcct ccctgaactg acttaccatt 8220
ctgtaaagca attatcttct gatgctgttg ctcggttaag gcggttaaag cttttaagtg 8280
tttcaaagtt aattccaaga ctacagcttt ctccagatgt cccagagtct gcagtggtgc 8340
aaaaaagaaa cgggcacttg gttacttaaa aacacacatt tggcttaatt ggctgtccat 8400
tcagcacagc aatttaagca aacactttga aagaagtact gttcttttac ttcttgagct 8460
ttccacaaga caggttataa atgatttctt gccttcagct gggagcacct actccagttt 8520

gcgggaaact ctgtacggta tagcacaagt tttaagtcag gaacttatat ttacatttat 8580
tcttatattt tctgggagtc gcaccagact attaacacgc ccttggagag cagcaaagaa 8640
tgaaaatgtg catcttactg tcaatttcag atgttcaggc agtaaatctt tcagctgagc 8700
aatgcattca ttaattcggt ctcttctttt cttttctatt aatctgtgcg gtaatttgta 8760
ggtatcctta atatatgaga gtcatggaaa agagaaaaca gtaagcgaaa cattcactta 8820
ttggatatta ccctcgtctg ccccccccgc ccccccacca taaaacatac tatgtgataa 8880
actacaccca agaaacctct ttcctctgga ctgttctgca atgcaattta aattcaggta 8940
tgatgtttaa tatccccctg agagtaccca gcaagccact taaaattcac agttggggaa 9000
gctcaggggc tggaatatat ttgcgggcag agcttcactg tgaaatcaat ggctctaatt 9060
aactacccat gcaggttatg aggaatatcg ggaacttaca cttaccttgg tgtcgtctcg 9120
tttcatgctc cttttgggtt tacacatata caaagaggaa tagtccagtc tgcaaaacag 9180
aaatcagcat caggcaccca ttcggggagg ggctctgccc tcgccagctc cataccactt 9240
tctggagaag aaaaaaatca aaccaaagcc tagacagata ttcgcaaggg tgcgtgcacc 9300
ttaccctata aaatctctat gttccagtaa ctgtctctct tgcaaatgag gaattccttc 9360
gtccatgttc aactgctgtt cgtttcctct gtttcgattt ttggggctct gtacaataat 9420
ctgtgggacg gtaggcttgg gagaccttgg ggggatctgt gcgtctccag tctctctctc 9480
gctctccctc ttcagtgcag tgttgaaagt gtgaagcagt tggtcccccc cctccaccgc 9540
gctcgcacac acacacgcac acacacgcac actcgcgccg gccccactgc gctggtagtt 9600
tgctctcact ccaggcagtg tttggccaca gggcacgcgc gtcgccggcc agaccagcac 9660
ccagctcacg tgcggaacgt accatccgcg gtccagcccg gaggggggcg ggggaggagg 9720
ggccgggccg ggcgggggcg tcgggagaag gcggcgagag aaggcgaacg gcaggagggg 9780
gcggggacac ctgatcaggg ccaccggaaa ggaaaaacaa cttcggccaa gctattcatc 9840
ttcccaaagg cgatgcagtc ggcaggagga ggggggagtg ggggtgcagg ggtgcggggc 9900
agcctgagct tccttggctc caaaatgcga ctccctgggt tcgtccgttg ttacggtgca 9960
ggaatgtgaa cgtggctttt tgcttttcca ccgattgtgc tgttggtttg aaccatagaa 10020
aggaagtgaa aagagaattc gcggtgggta aacttgagtc ccaaaggaaa ttttggagac 10080
ttgtttgctc actgatcagt ggcctggagg gactccttgg cgctgggtgg agaggttgcc 10140
tcgagaagaa gcaacttgca atccgctcct tgaaggattt aagatacatg aaaacgttgt 10200
cactgggtcc gaatgtcgca atccaagtgt ctttcggtga cacggcgtcc ctgggccaac 10260
taaacttccc cgcagcagcc ggaattgcgg tgccactaat cacagcagca gatgacgacg 10320
tttggggccg cgtgtgctcc tggaggaacg cggcggggag cgagccgggg ttgagtgggg 10380
gctgggaagg gagaaggggg tggcacgaga gggggtggca cgagaggggc taccacagaa 10440
aagctatgca gcatttatca cccgcccgct caactctgcc gcgttctgag cctccggtga 10500
cgctacgtgc tttccccatc cccccgcccc cccgcgcgat aagagacgcc cggagtgtgc 10560

agagcccacg tttactaccg ctggcacctc caaagcctcc ctccttaatc cggtctcaaa 10620
cgggtaccag cacagggcca gcaacgtgat ccgacctgcc gctgctgcca catcactgct 10680
cggggaaccc gtgcgcgcgc gcgctcgccc tgcagccgcg gcgctcggcg ccgggaatca 10740
caggccagca agagccgggt tacctttccc cagagccacc ctgggagcat gactcactgc 10800
tagtgagagt taccgggagg tgcccgggga tgcgcactgc ccacgccgcc ctgccagccc 10860
aggggaaggc atgtcctgag ggactcccag gggtgggatg gaggctatat atatatttct 10920
ctagatatac ataaagacat agagaaaaaa accaaggttt ttcctgcaca gagagggata 10980
tttacattct ccccggggtg atctctgaaa cgttgattat tgccggtgat aagtgaaccg 11040
aggggagaag aacgccaaag gtacatgcac cagggcgcag cagggactgg ggcgcccgct 11100
acgtgtcccc cggcccgccc cgccgggcac gggttccggg gacggatctg ggtcgcggga 11160
agcggcggct gggaggaggt cacgtgtctg cgggagtcgc gtctcctccc cgactccagg 11220
agcagcccgg gccgcggcgg ccgctcgcgc tgctgcagtc tccggagtcc tgggcggaga 11280
cgcgcactgc gtctggcttc gtgactcggg cggcggcggc ggcccaggat caccccggcg 11340
tgaggagacg ctcgcggtcc gttcagttca tctgtttaga gggcctttta aggtctctcg 11400
acttcctcaa atagaggaac gtattttgca ccaaacaggc aatttaatca cgatggttct 11460
taatttaaaa agtattttta aaatgctgac tttattttaa atctcggtcc gacctctctc 11520
ctatttccaa aaggttaggt aattaagacc cttcgatggg gagggcacgt tggagcccta 11580
aaagcgacct attcctgcag tactgtatca tttttacctc tttttaattg actaaaaatg 11640
ggaatgccgt taggtgaata atttaaaaat taaaacacca ggtccccttc tagatttctg 11700
aatgtttgga gtagaaaggg gatgtaaata tttgctccag agctaaattt cacttgaaaa 11760
ggatgactgg ttagggaaaa tgaaataaag acaagatcat tgcattttgg cctcgtcggg 11820
ctaaaaacac aaggcagaca tctgaccagg gcaaacctta gaagctaagt ctggggcaga 11880
gattaaatgc aagttgaaat aggaggaaca cgatcaggag ccttgaggct caaaggtggg 11940
ttaaataagc ttgcagcacc ccataattta aagggacaaa aagctgcaaa tgacacatga 12000
agcttttttc tgttatctct ttgaggctcc tctctccccc catcctttct gccccaagct 12060
tccccacttc cgtacccttt tcccgtgata cctactcaaa ctgcaaatgg ggtcttttcc 12120
caatttttat ctcctttgtt gttcttgatc cccttttcct cttcttttct gtctttcctt 12180
tacatattca ccttttaact catgtttccc tttttcattt cttctttatc ttcctttccc 12240
ttatgttctc tgtctacctt ttccctgtta aatgccagct tttgccattt ctctgtagtt 12300
ctgtcaatct cctgtttgct ttttgccctt ttttttcttt gatctttcac cctggtgact 12360
cttcttggtt acccacttcc cttaaacttg aatcccattc ctgttctttc tcaaactctt 12420
ggcttctgga atattgggca tagtttgatg cctctgaata tccctttgcg caagaagggt 12480
gtaacaatag ctaacaacta tatagccttt aagattagag taccagactg cattcaaaga 12540
gctttttatg tattaagtca ttaatcattt ccacaaccct ctaaggtaga tactattatt 12600
gtttccattc tacagatgag aaaatgctta caaagtgaga ttgcaaaata tttccaaggt 12660

cccagagcca ggatttaagc ccagacagtg ggttcgagag tctggaccct ggacgactct 12720
tacacctcaa tgtgaatagt gagtggtaag gtataagtgt taactagctg cttcagggat 12780
gtttttctat ttaccagtat aagtgtgcac atttgtgtgt gtgtgtgaat gaaattgtgt 12840
ggggagtttt gcaagagaga tggaaaaggg agaggtgtta gcattggagc tgatgacaga 12900
gataagaaaa gctatggcaa gaagagaggc agtcttttgc catgtcagtg agtcattcag 12960
ttggcatcta gcttcaaatt tattgattta aatccaaata tcctaattaa ctcttataat 13020
tttgtacaga tggacagatg gaagtacagt ttagaaaagg ctgatggtga ccagctgttc 13080
accagctctg ctgagcaaac tgagccaagc tctaatgtga agacttcagg ttagtaatag 13140
gaaaagttca cctgtggtga ggttagaaag ggaaaaaaag tacgctaaca aaggaacaga 13200
aactagaatg ccatctttgg atatgtttta aaataggttg agtcttatct ttttaggaaa 13260
gcttaatgct atcctgctga aagccaagaa gatacaagat gagctcaaag cccctttgag 13320
cttataatta aagaattttg tgatgctttg ctttcaatta aagatatcca tctttctcct 13380
tttaataggc gaggggtagc tgcttccatt gcttttcttt ggttaagtaa ggaaatcatt 13440
acctatagag acactaatga aaggaaatac ttaggccaat tctcccccct gcctaaatat 13500
cctaagtcct gcactcatta tattcaccct gtgactttag aaggccttat cctgtttgca 13560
aagtcttgga tttccaacag aaacagaact tgcctgaaag gattaaagtt tccatgggga 13620
atagaggttt tctgcctctt tagggttctg caagccctac tgcccttctg gagaattaac 13680
tccagggtgt tttaggaacc caagtgtgat ccaaactaac tgtacatctc tgatggcccc 13740
aaaatctgct taggggcgta gagtctcctg cctgtgatac tgtgaaaatg gcccatctga 13800
agggcagaag ccattcttga tcacctctgt tttacagagc ggacaagaat ctgcagacgt 13860
tttacatcaa agttaagcag agtccaaaac aatctaaaaa agttttgggt tgatgctaaa 13920
atcaaggcct ggaggggata gcggggtggg atctggactt acaaaagctt ccctaggaat 13980
ttcggctctt ggcatgcaca cctactctca agggagaaga gttaatgttg tcaggcacac 14040
cacagaaaga agggtgcctc agagaaggat ttaaggaggg acggcaattc cactgctact 14100
caccagaagc cagtatgact ttaaaatatt gatgatgtta tttgctgaaa gtaccggatt 14160
ttaaatgcca gaatagaaga ttgggttttc cagttgcata ctcagcaaat gagcagaaac 14220
agcacacgtg tccccgttta atttgtaaag gaccttaagt ctttctgaag actatcaatt 14280
aatgtgtctg tagccataaa catacctatg aaccatgttt tacatttatg taacactgct 14340
caagaagttt atgcacttat tagtctttgc atagcttcaa ggagcagata atattgagta 14400
ttagaaataa attttatttc tgctgaaatc atttgtattt tcatatttat gtgatgatca 14460
aaaccattca tgcaggctgt tctgcatttc acgtgtgctc aaaattcctg gagttcttaa 14520
catacaaaag acaactagga gagagccaag cccaaagacc acaataggag gattccctgg 14580
cccttctgcc cctacgtatc tgcctggtcc ctcatggtgc ccatgtgctt ctcatactga 14640
ttgaggtgga tccataatga gaccggaaaa gtagcatgag gaggagatga caaccccatt 14700

aaggactttc acaaagtgtt ctctaccatt tctctggagt ctggactagt tggatggtgg 14760
aagtctgcag gaagaggggc cttttaatgt ctgtgtttcc tttgttactg ctgatgaaaa 14820
ttcttggaaa aatcttccaa gaaaaacatg tttatgcaat gtgaagcaca ttcaaatata 14880
aataagatgc actaaatgtc cacttctctc acccacccac tatgggcttc atacggtcct 14940
tacatcaatt gtcagatggt gtcctcctgg tgcagagagt tgtcagtaac agatatttct 15000
tctgacttca aaggtactcc tactttaaaa aaacaacaac agaaaactag ctaaggaggg 15060
ttaaatccag acttacaatc aaaactattg gtataaaact ttttatttaa tagctaaagc 15120
gaaaaatcat atataacaat tacccaacaa gtactatggt tctggtatta acttataatc 15180
atcattagta aggaaactga ctgaaatcca ggctgtgtat attagttaag gtctgcattt 15240
cagccaaaat aacagcaact gaaattagga agttcttttt tggtcacata aaagaagtct 15300
gaaggtaagc agtccagggg taacgtgaag tggtaaaagt gccatcagtg tcccacactt 15360
ctgcttttcc actctccacc agcgtctatc tgcaagatca ccccatggtc agaagatggc 15420
tgcaggaact cctgcaggga aggatgaaag ggtgcctgcc aactgagcaa ggtctcttaa 15480
ggagcttttt tttttttgca agctccaccc atctcattgt ccaatcccat tacaagaggt 15540
tacaaatgta gttttcaaag ctgaggctcc acagcaatat aggggttctt ttagtaaagg 15600
ggaagtggag actagatact gggttggcac tagccatctc tgcagtgatg tgactaaaag 15660
gaaatggctt gtccacgtcc tcttcttttt tcatcagtga gtctcttccc ctctattatg 15720
ccatatgagt tggcagcatc attttattgt attagggtat ttaattccaa tttcagaaaa 15780
atgtcaatct tcctgtctgc aattgcacaa acttaactaa ctccttaagc acatggtcct 15840
taacgtctgt atttttaagt tctaataatt gtagaaatct ccttagggag gaaatttaat 15900
agagtttgct cttcttcctg tagaatgcaa atttcagctg catgttttct tggaaatgat 15960
tgtctgtctt tttacgtagc agacctgtcc tgtggttgtc ctgtccctgc agctgtgcct 16020
tctgcctctg gcagttggct tgaagagagc agcttccagg agaaaggcat tctctgatag 16080
ttccttgaat gcatctagga ttttgcactt cattcacttc tagccacact tatgtagcat 16140
acaattgctt tagggtcaga agattgtggg agggctatgc tatccagttg ggttctggaa 16200
taaatgaggg aggaaagaag aggtaagtga gtgcagtgca atgggagaag agggactaat 16260
ttctaaggct gggattggga ggtgaatgct ggaagagaat ctatctttgg agcattttac 16320
tatggacagg tattaaggca ggtgtcttat aaacaatgtc cagtgctctc aattcttaga 16380
actaggcacc atcaactcta ccttacagat gaacaaactc aggctcagag accttaggta 16440
acttgtctgt tgtcatgtag taatggcaaa gccaggcaat agggacctag gtgtgctcca 16500
gggtgtcact ggcaagagtc ttgagtttta acagttaatt accttctccc ctcagcccct 16560
gagaggatct cctatccaag catttcacct tccttggtga agctctgctt caggtcatgg 16620
gagcccctgc cagagggttg atgtttagag gagttgttga gagatgagga agatggtagg 16680
aggggaaatt cttaacccca ttctagctga cttggaattt ctgatgtttg aatgggaatt 16740
ttgcatgtat ttccctacag acagacacaa gggtaagggt gtggttagct cactgaataa 16800

agctgccctg tgtctagcat cttgggatct cctatccaat cctcatccac tgaatgaata 16860
tttactgtgc ttatatgaac tgtgcactgt gatgggtttt agggattcag tagtgaacgt 16920
tgtcttcaat aagtttacaa tttaatggat taaatttctc ttaagagaaa agatcactaa 16980
gaaagtggaa taattatcct aaagccattt tatgttccaa aaaagcctgc tttttggtac 17040
atttgagata cacttaaaca gctctacttc tctagtattg cacgttagaa atcattccag 17100
aaaatgtagt cagtggcctt ggtctgtggg taaggcttgt cctgtaaagg ccactagcaa 17160
agaaaatgct ttctcccttg ctttcctgag ccatctaggc cttcattata tctacctccc 17220
caatttgccc tagctccctg atggtgggga tgcctgttca acacccaccc cttccaagca 17280
tatacaattt cactgtatgg ctcctgttcg gctctccacc ctcccctggg attctcccac 17340
ctcccttgac aagtatgatc ttctcatggc ttagtgttta aactttctct ctttggtgtt 17400
tgaagctaac tttgctctgg attgtaactt ggtctactcc ttggtgttcc acatcttctg 17460
tagtgtctgt taggaagctt cttctcatcc ccagcccctc agccctcacc ctgaccccag 17520
ttgcttggtc attgccctag caggtttctt ttagggggag attaggacag ggttagactt 17580
agctaatttc ctttgttcca gagaaggctg gaatggtttc atacaagaat ataactcatt 17640
acaatgaccc ataacctcca acacatagat tttcacatcc tgaaaaggac aacttcctca 17700
tcatcatata cttgactccc tcagctcttt gacctcagca gttcaacctg gaaagggacc 17760
tgacagggct ggcgaatttg gtcagcttaa tgtctacatc actttcaggt gcagggactg 17820
gatggcaaat ttagcaagac agcctgacct ttaaacgaag aatgcggtga tcattctcaa 17880
ccccttcatc tccctcactc ccccatccaa ttaataacaa tatctgagaa ttccacctct 17940
taaatatctg taactccatc cccctttcat cctaactgcc cacactccag accaggcccc 18000
cataatctct caccccaaat gttgcaagag cttcttccct gcttttaaca gttagggtct 18060
agtaaggaag ataaaaacca ccctagatat ttcaagtagg aggaatttga cagaggagat 18120
taaaggcata atcataaact ttggaaaggc tgggattatc agttttggga aaaactgtta 18180
ctgacttcca gaaaatcttg acaattggtt cagggaaacc tgaatcccat tgagctcagc 18240
tgcctgccat gtggaagagg ctgacttgca ggagcttgtc cagaagctac tgtaaacctc 18300
acatctgcac acagctgcca ggggagaaat atggtgtgtc tctgccactt tccacatctt 18360
gcacaagggc ctctcaaagg cagaatctaa gttagaaaga ggtggggagg ggattctgga 18420
aaaatgtggt tccaagtttt tattatgcaa tgaggacaga gtggagaagg cagtggagct 18480
ggtgctgagt tggcatcagg tatctcacac ttgtatccac tgtgtatgcc ttctatcccc 18540
ctccaccaca ctgaattcaa tcactaagtt atagccagag caatttttct gttcgtgtca 18600
ttctcttgat caaaactctt taatagcttc ctatttccat taagttaaaa tcaaacttga 18660
aactcttcag tggcctgaat atgacttagt tcttgcctac atctcagtct catcactcat 18720
ttctctgccc ccctttctag aatccagcat accagctttc tttcaaaaaa tattttttaa 18780
tgtaccctgc tcttgctgtc tcaggtcctt cactcacact gttttgattg attagaatgt 18840

tttgttccca cttccccaat gtctttccct tctcttcctc ctactcaacc ttcgggtgtc 18900
atctgaaaca tcactgtctc caagaagcct tctctgagtt cctggaccag atttgtctcc 18960
ttctatcagc tccctttgtg cctgtctttc ataacttctg ctgactgaaa gactcatatg 19020
acttttcagc atctgtcttc cccactagtt tgtaagctcc atgaagacag acacctgaat 19080
tgtttactgc tgtatcccca gtgtctagga cagtgttgag cacagaacag acacacaact 19140
cttctgaatg attcctaaat gtgctccacc aaatcatcta tacactgaag ttggctttgt 19200
tccaggaaaa cacttgaatg acaagtcata accaagccac ataacatttg aagtgtcctt 19260
gtacctgttg agggtgggga atataatgtt ggttcccacc ctcccatccc ccactaggac 19320
tttcctatat tggtgtggct tttatgggca gggacaccct cactatactt agagtactgc 19380
atatttactt cttggcttta ccaggcacat ggagattaga aagtaggtac catgaaggat 19440
cataaaatgc tcctgatatt gcaggaaact cagtagtctg gcagggtctt tagtgaagta 19500
gtgggaagtt ctgtcagggg gtagggcctg tggctgtgga attgctgttt gtcattgtac 19560
caggattgat gtgctcggtg cccagaaggc agtaagctct gctccctagc agccttggta 19620
agttgagttt ctcacttgca gaacttttac ttcctttcgc agtgtgcatt agcaaatcag 19680
agagactgta aaagctggag ccagtccagt cgaattccac aggtgctcca gagaccgtgg 19740
gttggtgagg taacatccag ggcatggctc tcagaggcca gaagtaggga ggggcgcaga 19800
gtttggcctg gcaagctgtg tttacaggtt tgctgggctg agtggatcac aggagggggt 19860
cctggggcag agccaaagag cttggttacc aagagtgcag aaagaaacaa gcactgtgcc 19920
aggatgtagg agtcaagcct cagactccgg aacaaagtgg gaggattgag agccagtgaa 19980
gccagtccat cagaatgcca agctggtagg cacagaggag gcaaggagca aagggtcagt 20040
acagacaaaa gagagctggg gcaagatgca ggaggcagga accaggtctg gaggatgcgc 20100
tacgaatgtg gaggaattct cctggaggtg gtcagtcagt cgtgcccttg ataggctgga 20160
aatagggtca gaaacacatc aaccctcacc ctcaagccaa ctttgcattt cctcctggca 20220
ctcgtacttg caaaaacaca tagctctcat ttctctaatt atgtattcaa aaagaaagtt 20280
ttgatttttc tggtcaaggg cttcttgcaa aatcaaacta cgtttgccaa atatagcccc 20340
ggtgaccaca agccatcagc aggcctgggt ctgagttcac acctgctcaa cattcctgca 20400
ttgccagtgg tgatccagct gagatacaat tctgagtcaa ttttgtcatt ggataatggc 20460
actactgatt tttcctaggc tttttattct gttttagaaa ttctgcttca gacataccaa 20520
aggtaatgaa atagacatat ctcatatttc tgtccttgtt tcatattaca gtccaccctc 20580
catatctgtg ggctcccatc catggattca accaacctta ggatataaat atttgaaaat 20640
aaattgtgtc tgtactgaac atgtacagac attttcccct tgtcattatt ctctaaacaa 20700
tacagtttat ttatatagca cttacattgt attaggtgtc ataagtaatc tagagatgtt 20760
ttaaagtgta tgggatgacg tgtgtaggct atgtgtaaaa gactatgcca ttttatatca 20820
ggggacttga gcacccaagg attttggtat ccaccggagg tcctggtacc aataccctgc 20880
agataccaag ggataagtgt gtttcatgtt gctgtgtatg gtggcagagt tggtatggac 20940

ttaggcttta agatcaagag gacctgagtt taaatgctgt tttcccctga gtagctgtgt 21000
gaccttggac aaattatgaa tgagtcggtt atctcaaatg taactacaga gaaatagcag 21060
tacctccctc tgtttattgc gtgtccatga caatgcgcag agaacacccc catcctagta 21120
ggtgagcaat aaatgatagc tattattttt cccatttgag ctgcttggtt aaaattatga 21180
gtgtcataga aaatatgggt gtatgaaaat aattgatgaa ttgagtaaca ttttaagttc 21240
aagaggtaaa cctattgctt tccattactc tttcctctag taatgggtga ataacaataa 21300
aacagaaggc cagacctgtc aacgtgattt tccattaatg agatatagtt aagagtccat 21360
ggtacttcgt gtgctacttg gaaataggca gtactggtca caaccttatc cttttaaatc 21420
atgctattta gacagctcct ctgagagctg tcacatagtt ccacatttat gtgcaaattg 21480
ggggaaatcc tgttagtcca tgctttgtcg taagttaaag ccttggtcag gtcttaaatg 21540
aatgacatgg gtgaattaag tttcaaactt tgttagctgg ccatgggagg catcttgact 21600
tatttcttct ttatttcagc cattctacta ataaaagcaa gacttttatg gcagttactc 21660
tgtgccatgc tctcttttac tgttttgcaa gtcttaactc atttaattgc tcaacaaccc 21720
tttgatatag atagtgtgat aatgccattc tgaaaaggaa aaaactgcgg cacaggttaa 21780
gtaatttgtc catgtcacac aaatagtaaa tggcagagcc aggatttgaa gaaaacactg 21840
cggctgcaga gtcattgccc ataatcttga ctgcctgtgt gcacaatggc ctcccaaaaa 21900
cccagacaaa aattcagcat ttctatttac ctccatgatt tttgtttaga gaagcgaggc 21960
ttcattatgc cagaaagagc atggctttgg ggtctgaaac tgtgagcata aggttaagct 22020
ttacgaatac ctgtttgcct agttgggaga ggcaaagttt ggaacttccc taaacctcag 22080
tgtccgcact tatagaatga ggatcttata tgcctgttgt gaggtgacta gtatttctga 22140
cacatagaag atgtccaata aatatgtcac taccttgaat atatatatac acatatacat 22200
atatatgtgt gtgtgtgtgt atgtatatat atatatatat atattttttt tttttttttt 22260
tttttttttg agatggagtc ttgctctgtt gcctaggctg gagtacagtg gtgcaatctt 22320
ggctcattgc aactgccacc tcctcttttc aagcgattct cctacctcag cctcccgagt 22380
agctgggatt acaggggccc caccaccacg cccggctaat ttttttgtat tttagtagag 22440
acgggtttca ccctgttggc caggctggtc tcgaactcct gtcctcaagt gacctaccca 22500
cctcagcctc ccaaagtgct gggattatag gcatgagtca ccgcgtctgg cctactatgt 22560
tgaatattaa cagttgtccc tcagtatcca caggggactg gttccaggac ctcccccgac 22620
cccatgatac taaaatctgt ggatgttcaa gtcccatgta aataaagcat agcatttgct 22680
tataacctat gcacatcctc ccgtatattt gaaatcatct ctagattact tataatacct 22740
aatatgacat aaacactata taaacactga atttttaaat ttgtattgtt atttatttat 22800
ttcgtttcac atatttttga tccacgattg gttgaatctg tggatgtaga acctgtggat 22860
acagagggcc aactgtattt aaagatatcc aaatatgcaa tcattttggt ggtttatttt 22920
ctcctgaaaa acaaattttt gattttgtta aacttttcac ctttccaaaa tagggtaaaa 22980

tttgatcact gagttaacaa taatagagta tcttctatgt gcaaggcatt ttctagatag 23040
gggctatcca acagaacttt ttgcagtgaa agaaatattc tataatctgg cctgtccaat 23100
gtggtagtgc tagccgcatg tggttattga gcagttaaaa ttgcttagtt taactaaggt 23160
actgaatttt aaattttatt taattttagt taatttacat ttaattttaa atagccacat 23220
gttagtgact atcacattgc acaaggaaag tctagatgct tttattaggc ccacatactg 23280
atcaatccat aaataattat ttttaaaaag ccaagacatt tttttaacct gttctttttt 23340
taaggtgctt atggtgctca gaatccgtgt gggaagaaaa aaaaatcaat gatcgagcat 23400
aaattcaggc caataaagat aaactatagt ccattttaat tttactggaa ctcaaatgac 23460
ataacttatc aatattttta aacagtgatc atgaatttgc tatgaatatt ttagttactt 23520
cccataaata aatagtaata taataatttc aattctgcat atcataccat atagggatta 23580
gttaggttta gatgtaagca gtagtgtgct ggtaaatgtt taactggttc tttatgaaaa 23640
atgtaggtgt gtgcctgtgt gcatgtgtgt gtatggttta ttatatacta tgttataaat 23700
gatgtgtagc aaaaacacac acttacacat aagaataaaa tatatattac tcctgtgaat 23760
cccatatagc caactgattt tcactgagtg cttttattga tttttgctaa attcttttat 23820
ctcttgccaa cctatgagtg caattaattc ccaagtgtaa ttctgacatt aatgttgatt 23880
gaaattttcc tttatgttaa tgattaaggt gaaagtcata cagtgaagac atttgttaga 23940
acctcattta ttcatcattg atatgagcaa tgttttcact aaatcaggta ataattttca 24000
catactggaa aaatatttcc ctatttttgt gtgctattca caatgtgata actataaatg 24060
tgacactttt aaatggaatc tgcctgatta acattttctc tatctctttt ttgagtccaa 24120
caattggcaa aataataaat ttgtagcact tgccaatttc catggtgtaa atactcccac 24180
cactactaat gtggcatcac tgacagtgga gttaggaaag tataatagat gtaataacta 24240
atctgaaaag caaatagtaa aatgtggtaa atagtaagat aatagtaaaa tgtggtaaac 24300
agtaaaataa tagtaaaatg ttgtaaaata tctaggaact gatgggcttt gattatttat 24360
tacctttgtt tttactataa tttacttagt ggtaaatttt tataacttaa ttcgtcatag 24420
tgtggagtcc cagaatgttg gtcctgtgtt tatcagcaat ttctgaagcg gacactcttt 24480
gccagcacta ggtgctaggg ctataaaaat gaataaggcc tgtcaagatt tgagttttcc 24540
tggatatgct cttgccctcc tctcttgtct ccttttcctc actgaatgat ctcatccagg 24600
ctcatggctt caatatcatc tttatgctga taatcccaaa tttatgtttc catctctgct 24660
tttctactgc aagctccagt ctcataaatt caacttccta cctgataact ccttttggat 24720
ttcatgtagt tatttcatga aatatagttg tttttatgaa ataactgtat gaaatcatga 24780
ccattccagc ctggtacgtc gtaatgtcca tgttttgctc caaacccatg tagtcttcct 24840
ccagtcttca tcattctcag tcaatgacac taccatgtac cagttaattg agctctgatt 24900
ttaaggatca tcctttgtcc tcttctttta ttaattttct acattgaacc tatcagcaag 24960
ttctgtttct tctattccaa aatacttctc aaatctggac agtcctcacc atcttcagtg 25020
ctgtcaccct agtcaaagcc actatctctc attgggacta ttgagagagt ggacctggtt 25080

tggatttgtg tccccgccca aatctcatgt tgaattgtaa tccccagtat tggaaaaggg 25140
gccgcggggg aagtgactgg atcacgaggg tggattttcc cctcgctgtt ttcatgatat 25200
tgggtgagtt gtcatgagat ctggctgttt aaaattgtgt agcaccttcc ccttcactct 25260
cttcctcctg ctacggccat gtaggacgtg acttcttcct cttcaccttc cgtcatgatt 25320
gtaaatttcc tgaggcctcc ccagccatgc tgcttgtact gcctgcagaa cagtgaacca 25380
attaaacccc ttttctttat aaattaccca gtctcaggta gttctttata gcaatgtgag 25440
tacgaatgaa tacattcata tttggtcttc ctgcctttcc tttttcatca cacagatctt 25500
tcacacagga actcttaaaa tataaagcag ctcctgccac ttttttgctt aaaatctctc 25560
cactagttaa cgtatttcag ttagatcaaa gtccaatcta tagtaggcag aataatgggc 25620
ctccataaat gtccatgtcc taatccctgg aacctgtgaa tatattacct tacatagcac 25680
aagaaacttt gtaaatgtga ctatattaag gatcttggaa aggagagatt atccaggatt 25740
atccaggtgg gaacaatgta gtcaaaagga tgtttataag tggaagcagg aggcagaaga 25800
gaattagagg gagaggtaac aatggaacag ccaggtagat gcagcactac tgtgtttggg 25860
gatggaggaa gggaggtaaa atccaaggac tgtgggtgcc tccagagacg ggaaaacgta 25920
aggaaatgga ttcttcccca aagcctccaa aaaggagtgc tcccttgcaa acatcttgag 25980
tttagcccaa tgagaaccat gaaagacttc tatctcaaag aactctcaga taataaattt 26040
gtgttgttta agctggtaaa tttattgtaa tttgttagca tggccacaga aaactaatac 26100
agtagcctgc aagaccctac aactttctcc tctagatcct cacaaagctg gttattcatc 26160
atttggatta ccagaatctc ctcaaaaagg caccaccacc acctggtcac tttttactct 26220
gtgctttatt ttctgaatag cttctgtgag aatatgaaat tgtcttacta atttgttttt 26280
gtctttatac cttcttacca gacacatatc attaacatat gaacttcagg aagtcaggta 26340
cctagtatat cattttcatt cagtaaacgc tcagtgaatg tatgtttttt aaaaaatgca 26400
tgaaaacagg atgtcttgct caggatatgc acagtctatg ggagagaaga tgagtaaagc 26460
aatgattaca gaacagtata atgaaggcta taaaagggac tcatatgggg agtcacaggc 26520
acccatagga aaagcacctt ggcaggctct ccagaggaat tactattggg cacaaccttg 26580
aagagaaagt aggaaagcaa aagcctggta aaaaggagca ggtggatttt caggcagagg 26640
ttgttggagg ttgtgaaaga gaatacacag gtattaaatc aactgcaggt gatttggtta 26700
tgctgggcct aaaatgaaag ttaggaaggt gtcttatcac tgggactttg tatgacttac 26760
caagaaatgt ttgatttgtc taggtggcat gaaagggttt taagcagtgc cagttttgca 26820
tcgtagaaaa tgtattctga tgacactgtt gatagtatat tgaaggggag gtaatgctgg 26880
agatgggaaa ccagttggga cgatatggcc attttcaaga tgaaaggttg taggacagta 26940
acagtgaaaa tggagaatga tagggagatc tgggaaagta ttttagaggt gcgttccatg 27000
ggattggtga ccaatttatt gcgtgagtga gagagaaaca gtattaggag ttgacactca 27060
gggtttggtt tgggtgactg gacagagagt agtaccttca ataagaataa ggaatatggg 27120

aggaggatat gagacagatt tcactgtgac aagtttgtga cgtaccatgg gtggaagagg 27180

catctcagag gaagctggat ttaagtctga atcttgggaa agatcagggc tagagattta 27240

tatctggaat gaatggaata tgtgatatga gtggttgagc tatttcagga catggagatt 27300

agaagagaag tgggttgagg atggaatcct ggggaatact gacttttaca cattggatga 27360

aagatgtgtg tctaagagtg agaccaagaa ggaatagtca gagaagaagg aaagaataaa 27420

gaatgtgctg tcatctacag aaatgcctgg ctccttcatc tgtacatgca atttagtgag 27480

atttccaatg tcacctcttc caggtcctct caaatggatt cagttgcttt tcactttact 27540

gtacctgtat tatgatgatt gtcacattgt atatacctat tttttacact 27590

<210> 17
<211> 4757
<212> DNA
<213> Homo sapiens

<220>
<221> misc_feature
<222> (1)..(4757)
<223> chromosome 12 nucleotides 103516177 to 103520933

<400> 17
ctggaaacca aaagccccca gtggtctaaa cctgagaggg tacagtggag ctcagcttct 60

agggagggtc cgaaccttcg cacagcatca gcagtaggag atgttctaca gctcttattg 120

tagatgctac agtgtcttct gaggtagatg gagccaggtt gtgtggaaat tgtttattct 180

gtaattggat gctcaagaat cagaaagcat aaagagtata atttgtgtct ctcttcccca 240

tggtggggaa gtcttattaa aatgcagatt cctgggcctt aacttccatc tccccctcct 300

tctgattcaa caactctggg atgaagccca agaatgtgcc cagaaatctg cttttttaac 360

aagcaatatc atggggaaag tgagtagggc acacgttgag aaactctagt ttttggaaca 420

tgacacggag gatttgggga actttatctt ttcttgactc tccttctcat ccttccacag 480

atcactgggc ccctcctgag tcgtctggct ccttctttcc tcttggggag agataaagtg 540

ctggcccaag cctcccaccc tggtgggtgg ggtgggcctt gactcagtgg tctgtatcct 600

cgtggccagc tggcctagct gagttgggag gtgaccctca cctcacaggt tgggcagcac 660

ctgtcagtct cgaggctcag cttggctcct ttccactcac atgaccttga ccatggccaa 720

gtgcctgcag ctaagggagg cgcggagggg ttatctgtaa aatgcaggta cttcctttac 780

cgtgggaagg atcacactgc accgactgcc tgttgcatag cgagcactcc agaaacggtg 840

gggttgttat cattcccata aaaggcacac aaggcccctt tagcttttgt ggtctgggga 900

gccaaggccg gttcccatgg cacccagtga agtcacgggc agggctgatg gctgtgggca 960

aagaacccaa ggttcaggcc ccacacggtc aaagcatttt aaaggccttg tattagacaa 1020

ataaaccaat taaataatac tgccccccat aaaagcagac ttttaatgga ttccatggta 1080

ggcttttcac cgcgcggtcc cggagccatg ggcctcgatg agcccgtctg ccgccatagg 1140

gagaatttaa tcttgcccgg catgggcata attttacgag ttttatggct gtttttactt 1200

ctccgaagtg ttgctatgtc attgtgcacc aagggcaact tgcgtgcatt tcacagttta 1260
cctccttttt ggtggaaatg aaaaatgaag tcataaatta aagaaaaaca caacatagaa 1320
tttttttccg tcttcttggg gcaactggag gaacaaagaa ccagtgtttc tctgtacagc 1380
ctgatcaaat tctaatggct ttaatgtatt gacatttgcc tgcgattccc tcctccccag 1440
ggaagactgt gggcatgtag ctatgtttgg agcttgtact actgaccccg agaagacctg 1500
gggcagttct ctagggcaag ggttggcaaa ctctagccca agggccaaat ctggcccact 1560
gcccattttt gttcattggt ggaaaaaact caaaagaaga ataatatttt ctgacacatg 1620
aaaattagat gacattttcc cagatttccc tgttcatcag tgaagttttg ttggcaccca 1680
gccacaccca ttcatttctg tatcgtttgg ggaggatttc acaccacagt ggccctgcgg 1740
aagagttatg acagagactg cagggtgcaa agcctaaata ctgtttggcc ccttacagaa 1800
aattttgctg acccctgccc taggaagtca aggaaaagga aagagaaaac atggcatctt 1860
gtttctgggc attgatgatt gactttcaga gtttccacgt ggctcaaagt gacaagtcaa 1920
cggaggctca cctgagcagt agtttttcct ccggagacct tccagatgtc atcagctgct 1980
tttggatgac tctcctccct ttcctgacct cagattgact tctaagcaga ggtccctggg 2040
ctaaccccct gcagccagcc ctcccagtca tttgagctgc accctgcttg aggtctggtc 2100
actcctcatc agtccccttg atagccacaa atagagaagt tcccgccttc ctcccagggg 2160
gatctaaaat agcttctgaa atttgtcacc aacactttta aactgtggct tttggccagg 2220
cacagtttta agcattctgc acatagtagt tactcattta atcttcacat ccaacccagg 2280
aagttagatg cccctgtcat tcccatttta cagacaaaga agcagagaca cagagaggtt 2340
aggtagtttc cttaaggtca cacagctggt agtggtagag ccagtattga aacccaggct 2400
atctgttcca gggaatgtcc tgctgtccat tcattgcctc tgtggagcag gtgaattctt 2460
taggctcatg aaaggatcta gttttccttg ttccactcat gccactgttg cacctcaaac 2520
cataagaaaa ctgcctcgta cttgagaaga aagccccatt tattcttccc ccatctctct 2580
tttttccctc tctccttctt tcctttcttc cttccctccc ttcttccctc cttcctcctt 2640
ctcggcttcc ttttttcctt tcctccttcc tttcctccct cttttcctct ctccttcttc 2700
ccttcccttc cttccttcgc ttgctcccct ccctttcctt ctcctttcct ccttccctcc 2760
ttccttctct ccctctctac tgctctcttt ccttccctcc taccttcctt ttttccttcc 2820
ttccttccct ctctcccaat ttcatccctc ccttcctcct tcctcctccc tccaacaaat 2880
aatgatcaag cccatgacta tgcgttacat aatgcatgag gggctgcaca gagtgaacaa 2940
gaaaaatgaa aaagaaagaa ttcattgcca tttaaaaaga gctcttggtg gcttcaatgt 3000
tggtggggtt tgaggagttc acttattccc cctcttttat taattgtaag taccatctct 3060
gtctggaatc ataaactctt gtcttttctg cttcatgctg atattttcac taaaagtaac 3120
ataatttagg agaggttttg gggaggctgg gcctctgacc tgtccagtgg gaggagcctc 3180
ctggggaagg acctgcccat tctcagaagc ccagctttca gggacaagga atgaaaggtc 3240
attgactgtt gctggagaag tctgcggaat cttaaaatgt gttaccaagc ctgctgctgg 3300

gtagaacttg ctgtcttgaa gcagccatta cgccatcttt tgtaagtggt ttcatttcaa 3360

tgagctttag atgcctgtcc tgttttctta aaacacttgc tggggagact ttttagcatg 3420

atctagatcc ttctgggtgt ggagatgcct gaatgttggg aagacagaca gcagctgtgc 3480

tgttggagaa gggacagtgt ggaaatcttc ctttgctaga agctggtttt ctctttcttt 3540

cctgcctgtt tcttcttccc tactctgtgc ctttttcttt gacagacaac aaatctttgt 3600

tgctcatttc tgatgagcct tcactttctt tcctcagtca tgcggaggaa tccctttggt 3660

gtggacatct gctgccggaa ggggtcccga agccccctgc aggaactcta caacccaatc 3720

caggtaagct tcaagcactc tgtcagcatg tgaatttttt ttttttgtag gtatggatac 3780

taaaaactct aaaattgtgg tctttggggg atttaaaact tcctttcaca gcctggcatt 3840

ttaccttcag tggttgcttt ctccgtcacc ctttttctat gtagcatctc tgaaaaatct 3900

agcaggggat gtttagaatt agagaaatgc ctaggtttag tgctaagcat tcagacctgc 3960

ccagggtttg aactcctgag attaacagtc tcaagttcga gcaatgggac tcgggaatta 4020

aaggtccacc gaagctgtgc agtcttccct ttggtatccc caaaggaaga gttgcacatg 4080

ggtacacttg acacccattc ccacatccca catattaaag cccagtgatg ccccacctca 4140

ctgtgccagt ttggcaaact cccttgcgag cggggacctt ttgctaagtg actgcctcag 4200

taaatctaac tcgttagttt aatgaggcag caaacgtggc ctctgtgcca tcaattatga 4260

aaagagctcc tctttgagca aaagaaaagc tccaaccaag gaaaagtgtc ttctgtggaa 4320

agctagtgca aagttaggag gcctggttgc catggcaaca gcctcagtgg tgtgtgtggg 4380

tcttgatgtg gagactctgg ggctgatcct tttgtaggaa cattaattag cacttgatgc 4440

ccagaactgg taagtgaaca actagcagca gcatcagtgg accttcgata gccgtagaga 4500

aagagggaaa aatgtactgc tttggctaca gtctctgact aaatttaata cttctttgga 4560

ccaaggtacc tggtggagga agataatgca cgggcctcaa aggtgtcgaa tttttagaac 4620

tcttgtaaag ggatgcactt gagaactatc tgatgccccc tacccccctt cccaatctct 4680

cacctgcact cccttctgga aaaatggact cctgaattta tgcatcatgg gtggcctaat 4740

taggttaact catgccc 4757

<210> 18
<211> 7824
<212> DNA
<213> Homo sapiens

<220>
<221> misc_feature
<222> (1)..(7824)
<223> chromosome 12 nucleotides 116622835 to 116630658

<400> 18
tcctgcctct gtatgtgctt aacttggtga agacgctatt aaccgcagta aagaaaacat 60

ttattcacaa gaaagccatg taatgaggag aatcctcaac gtgggagcaa atgtcagtat 120

ctctcagata agacagaaag gttcattcag acgaccgaaa catcctctta gcaggttttt 180

gtacaactcc caatgtcggt gctaccatgc agggcaacac cgaataaatg attccagatg 240
gtaaaaagga actcccattg tatttcctaa gtatttctat ccatttctca acattacccc 300
tctccatcac cccaggagat aagccaccat tatctcttgc tagatgactg gggaaaaaca 360
caaaccaact aaccttttag ctccacctga ctgctcatcc atcctccatc tgcacaatag 420
gaggaaaact ttgtttgttt gtttatttat ttatttattg agacagagtc tcgctctgtt 480
acccaggctg gagtgcaatg gtgcgatcta ggctcactac aacctccacc tcctgggttt 540
aaggggatgg attctcctgc ctcagcttct cacctagctg agactatggg catgcaccac 600
cacacccagc taatttttgt atttttagta gagacagtgt ttcatcatgt tggccaggct 660
ggtgtcgaac tcctgatctc aggtgatctg cctgcctcag actcgcaaag tgcggggatt 720
acaggtgtga gccactgcac caggctggaa gactttataa aatcataaat ctggtcatgc 780
caacaccttt gctgaaaatc cttcagcatc ttcccaaagc atataaaata aagatcaaat 840
tctttagcat catctctgta gtgggctgaa tggtgcttct ccaaaaaaga aaaaaaaggc 900
tactgggact tgtgaatgtt gccttatttg gggcaaaagg ggggtcctta tagacgtaat 960
taagttaagg atcttgagat gaatcaacct gcattgtcca gctgggtcct acctccaatg 1020
acaggtgtct ttgtaagaga aaggcaggga agatttgaca tagacacaag aggagaagac 1080
acagaacaga gaagaaggcc ttgtgaagac agaagcagag attggaagga ttcagccaca 1140
agccaaggaa tgtcaaggat ttctggagca acggaggctg catgaggcaa gggaagatcc 1200
tcccctagag ccttcggaag gagcatggac ctgctaacac cttgctcaga cttccagtct 1260
ccagaactaa aaaagaaaaa atttctattg ctttcagcca tcaaatttgg ggtaatttgt 1320
tataggagcc acaggaaact aatactgctt ccaaggctgc catgtgatct ggcccttccc 1380
acttgctctt tctcctcagc cgcaccatcc ttccttgtca ctcaagtgca actgcaacat 1440
gctcccttgc ccactccagg gcctttgcac atgctgttgc ccttgtctgc tccccagtag 1500
tttcttccac acttcagaca gcagttctat cctccttccc ctcagtaagc ctcccccaat 1560
gtcactgggc caacatgatt ccctgttaca ggccctccca acaccaagca cctcgcgttg 1620
gtagcctttg tcagttccat tgcaaaggtg tgagattcaa tcatcttatc agatctgttc 1680
cacgagggtg gggaccatgt gtgtcttctt cacccctatc accgagctat ctacacagca 1740
cctgatgtgt aatagacacc taaatattgg aatgactgtt ggaatgaaag gaagtgcaat 1800
ttagtgaacc tcaaaaattg agtctctaac caaatatcaa acagatccat caaactgaaa 1860
aaggaaggaa acagaaaagt ccctggggtg aataggtggt ttagcctgat tcatcttccc 1920
ataaaccata tcaaagtttt cagagacaac ttcagtgcct atggatggtg tcaaaccggg 1980
acattgactt acacactcct cagtgttgac agaactatct tccctattaa ggatcacttt 2040
tatccatgga tcccctcttc ccccaccaag ctggaatatt cccagacacc gagccaaagt 2100
ctccaatcac tttcaaatgg acagaaattc agtgaaatgg ctagagctgc tggccagtgc 2160
accgtggccc ttttgaaagc tccgctctgg ctactaggat acttagctca gaatgagatc 2220

ctccagagct aagttattac atgtttgggc catttttctc ttttcacagc tggacaactg 2280
actggagtgg aagccagcca gctcctcagg gagcccaagg gggaaaatag tgaggaagga 2340
cagttttcca gcaataaact tgtttccttc tcattctctc cagactcaga cttatcgact 2400
ctttggaaaa gcccacccaa gaaacgaggg ggaacagaag aaagccagga tcaaaccagg 2460
gtcagagaga gagagagaga gacaaagaga aaggcagatg gctctggagt caggcctggg 2520
ttcaaatcac acattcagaa gttgtcattg gccagatctt aggcaaatta tttcacctct 2580
tttagtctca ctttgttaca tagaagagga attgatttgt ccactcaaca aatactgaag 2640
gagcaacgac agtgtgctag gcaggactat gagtagggta aggtgcatga ggtgcctagg 2700
atactaaatt taagttgata ctctcagaga catacccaac cttgagagtg ggcacctccc 2760
taaatgccac ccctgatgtc cctcagttgc ctccccctgg ccctggtgct agatgttggg 2820
atccactaat aaaaacatca aggccatttc cctgctatga agactgcagg ctagaaggat 2880
aaaactttcc taaacttcat gacactgatc tcctggtcac aataaaaaca gcaaagaaat 2940
tgaacgttga agttcttaaa aggccaattc cacctcccac tgaaaaacca cttgtgtgtc 3000
ttaagtgacg ctgaatttat caaacacaca cagctaccca gcaatcctcc cagagagcag 3060
gattataggc caaaagaaat taaggcaaac agactccata aataccaatc tcttccagac 3120
ctgctctgcc ggggcatacg tcctcccaat caggatgctg agaactccaa cttcatgagg 3180
tatggactgt gagccctgaa attaccccct ctccatgcag tgtaaataaa acaggatgaa 3240
atcagcaagt ctctttactt tgcaaagaaa ctggggagaa aaagggcaaa agcacatacc 3300
cacaactggg atgcattaac ataaacagaa agcatttggg caatttctgg gtaagagctt 3360
agccttattt tcagggcatt atttgatctg tgcactgctt cctatggggt tccaatagaa 3420
gacctataat cttcaagatt atctgaaatt ggggagggca tttcatattc atattcgtgg 3480
atggaacaaa tgcatattca tgtcttggtg gtgaatggtt cctgagctgc aggtgcccct 3540
gaaaagtctt tcgtatcaac agtctttagt tatggaaact caactttcca aatagctgca 3600
gccactaaga ttgtatccca cctaccgctg tctctggcca agcaggcaaa tggaaaagca 3660
tgttttcagt gtggaaggag aagctggtaa tgggtaaagc caagaaaatc caattgctac 3720
tgatctcaga gcttacttct acagtctctt taataattga tcaacacagc ctcacctgca 3780
acccagacca ctggggagag aagggatccc ccaaaaccct ggtgaaacag agcctgtgtg 3840
acccataaag cctaaaatat ttactctgtg gtcctttaca ggacaagtat gctgacctct 3900
tctctatacc aagctcaaga cataatgaag ggccaggtgc ggtggctcac agctgtaatc 3960
ccagcacttt gggaggccaa gcaggtggat cacttgaggt caggagtttg aggccagcct 4020
agccaacaca gtgaaacccc atctctacta aaaatacaaa acattagctg ggcatggtgg 4080
cacacgcttg tagtctcagc tacttgggag gctgaggcag gaggatcact tgaacccagg 4140
aggtggaggt tgtagtgagc caagattgta ccactgcact ccagcatggg tgacagagtg 4200
agacttcatc tcaaaaaaaa aaaaaagtac acaatgaaga aggatctggg gatgagatta 4260
ttttaattag gagctattga ctctaaccta aagagtgcga caaggtggga aggactcatt 4320

tgtctgaact tttattcagt cgcctgcccc acaaatattt attaagggac gcctttgtgc 4380
caggccctgg gctaggatat gccgggatat gactatgagc taggcaggcc agggcctgcc 4440
tccaagggac ttagagtcca tcctataaag gatgagttca ttacagtgaa tgtgaatgct 4500
acagaagggc agtcctgggg actctgagag ctgataagat gcccacagcc tagcgaaggg 4560
atcctgggaa gttctcccag gaagctaaga ccatactaga gagcaggaac tggtcaggca 4620
acagacgctc ccagaggaga agatccatct ctcaacctgc actgcaaaat gccagctcca 4680
ttcatttgct ccaacactct tctggttatg cagcaggccc acttatttac tgagggccta 4740
ttaggagcct ggaatcagag acacaagaca ccggacaatg ggttcagaga caccctttcc 4800
tcaccccact aggcacagtg gcaggtgcac tgattgggcc cccaagggac acctagcaga 4860
ggtctctaac ccagtcctca tgccaataac ttcaggtcct acagccccag cctcaacttg 4920
gccttggctt gacatcgaac agctgaatgt gccttgcact gcctggtgac aggttccatc 4980
tgcttcatta ctgtcagtcc ccagaaacca ggcttgtagc taagtgctct gggccctaat 5040
tggttcctga tagattgggc cataacgaag cacttagaac ctgccagaga agttgaggat 5100
gaagaggggg tgcccctgca tagactggga atgctcccct ctttgggaat tcttgctggg 5160
aaatctgggc agctggagag gtggatagga ctcaggctcc tgaatccacc aatctggaat 5220
gcattcccag tatcgtcact caccactgtg agcctcagtg tattcttctg taaaatgggg 5280
ctggtgacac ctgcctcaca agccgttcag acaaagtgag gtgctgacca tggtgtttat 5340
ggttaactga gtgcctgccc tgtgcggagt ggttggctga gtgttgttgg gtcttttttt 5400
tttttttttt tttttgagac ggagtcctgc tctgtcgccc aggctggaga gcagtggtgc 5460
aatctcggct cactgcaatc tctgccccgc gggttcaaac aattctcctg cctcggcctc 5520
ccaagtagct gggattacag gtgcatgcca ccacacccag ataatttttg tatttttagt 5580
agagatgggg tttcaccatg ttggccaggc tggtcttaaa cttctgacct caggtgatcc 5640
gcctgcctcg gcctcccaaa gtgttaggct gagtgtttgg aatgcatccc ctctttataa 5700
tttccaatct tgttaatcac ctataattac caaccccatt tcacagatgg agaaacggag 5760
cctcaaagag gtgaaatcac ttggccaagc tgcctgtctt ctgttcacat tcccatagtg 5820
gacattcaga aatggtaacc tcttctctct ctgcattagc cattctgctt gggtaacaga 5880
tataaaagta gctcagtcac aaatgtagac agacagaaag ccgatcagtg gttgcctgga 5940
gctctgctgg aagtggggat tcactgcaaa cagacatgag agatcttttc agggtgacag 6000
acatgttcta aaactggatt aagacaaagg ttgctcaact ctgtaaattt actaaaatgc 6060
attgaattct atttcttaaa atggtgatct ttatgccata taaattatac cttaataaac 6120
ctgttaagaa ataaataaat acaattatac ttcaaaaaag tggcctggtc aacatactgt 6180
ctataacaat aaaatataca ttgtaataaa caggagggtt acaagagata tggaaaaata 6240
tgtccacaca aaatcttgta catgaatatt cattgcagca ataattgcta aaaaattgaa 6300
acatcccaaa tgcccatcaa ctgaggactg gataaacaaa atgtgctatg catgtatgta 6360

tatatgtatg tgtatgtata tttgtgtgtg tgtgcaaaat ggaatattat tcagccatag 6420

aaaggaatgc acactacatg aataaacttt gacaacatta tgctgaatga aagaagtcaa 6480

acacaaaata ctacatattg tatgattcca ttcatatgta atattcagaa tgggcaaatc 6540

cacagagaca gcagattaga gattgccagg ggctagttgg gagcagggag gtgtagggat 6600

gggggtaaaa taggaagtga cagttaagat atttgaggtt tcttttcggg gtgataaaat 6660

gttttaaaat ggattgtggt gatggttgta caactttgtt aatatacgaa aaaaccattg 6720

aattctatac tttaaatgga tgaattgtgt ggtgtgcaaa ctatatcttg ataaacttgt 6780

tttaaaaaga atagaataaa aaggaagggt tagttgtagt cttggacagc cagggttcaa 6840

agctgagctg catcactttc tagctgtgtg accctgagct actgccttgg cctctctgag 6900

ccttggtttc ctattatata gaaaggggtg ggatatgagg aataagagta ttttggcatg 6960

tgggttgtgc agaaaacaaa tgaagttact gcacagagag tactaagcat tgtgagccag 7020

gcgcacactc cggccttgag aaacagagga aaatcttgaa gacaaacaag agaaaatgag 7080

gacacggaga gtcatctaga ctagcctcac agtaacccat ctatttcaac tggtattctg 7140

actccaaaac cagcaccaag gtgtggctgt ctgggagggg ataggggttg caaatatgcc 7200

taagcttgct ctctaaggct tcctggatca atgatctctg gtagtatttc ccctagggtg 7260

atccttacca tactggatcc aagagatgct gatagatgtt gcatgaaaaa ggagtccccc 7320

atgcaaatta tcttgggaaa ttcaaagtgt aaatgaagtg aaatggacat ctctgctgcc 7380

agccttgtca gagcctttaa ttctcctatt tgtgcactgg atatctatac aaggatatag 7440

cacccagaat tttcaaaatt aacatagtga caaatcattc ttgagcgttt catgtggtat 7500

gacagtttgg caaaataaat gttaggagac agcggatcca tggctttaat taaaccattg 7560

tgattctgct tttggctgtt aagtcagata gtgataactg aaactgttaa gtcagatccg 7620

gaacttgagg actccagggt ccagtggagt ccagtggttc tagtgtcagg ctctctgtgc 7680

cttatctcat gggaaccttc tgagaaccag gaggtagtgg ctgagatttc ccccactaga 7740

gatgaaggga cctgcctcgg ttaatgagta gtagcaccag aacctgaatc tagcccagac 7800

tagacccaaa gccagtgatt ctaa 7824

<210> 19
<211> 37248
<212> DNA
<213> Homo sapiens


<220>
<221> misc_feature
<222> (1)..(37248)
<223> chromosome 13 nucleotides 18870123 to 18907370

<400> 19
   catgctccta actgttataa actattttgt aaactagtgt actaatgtaa taattatgaa 60
   agttatattt gaatggaatt gtatttaagc attaaaggaa atgggagatt ggcttctagt 120
   cacattttta ccactaacta gggtattgtc tgtgcaaact tattagcata aatagcacca 180
   attgttcagt agcttaacat aaccaaagat tactttcttg tccatatcac agtcaaatat 240
   tggtcaggaa gaaagttcca cttatgtggt catttagaga ccttgactcc ttccatactg 300
   tggctccacc atcttcaata tatgtcttta aaattatcct ggtaagagag aagaacaaag 360
   attaccctgg catcatcccc ctgctagaca agaggaagaa tactagtcac atagccccat 420
   ccatgtacga aggcctgaga aatgtactgt taagctgagt gtccaggagg agaaaagagc 480
   tctgatgagc tcctagccag cctctgccag aggcagttgt tcaacctctg gacctgtttc 540
   ctatcatgac aacaagggga tagatgagga agtcctgagc atccttgacc cagcaagtgg 600
   tttgaaggtc aaatgctagc cctaggacag gagctgtttg tttgtttgtt tgttttgaga 660
   caaggtctgg ctcccttgcc caggctagag tgcagtggta ctatctcggc tgactgcaac 720
   ttctacctcc caggctcaag ggatcctccc acctaagcct cctgagtagc tgggactaca 780
   ggcacctgcc accatgcctg actaattttt gtatttttgg gtagagatgg ggtttcgcca 840
   tgttgtccag tgtggtctcg aacttgtgag ctcaagcaat ctgcccacct tggcctccca 900
   aagtgttggg attacaggca tgagccacca tactcagcct ggagattttt tttaataaaa 960
   tacacattat taaacaacta gccaatattc atgaatagat agaacagcag tgtctcactg 1020
   gccaccatca cctctaaaat gccagagttt tcagattcca ggctgggtgg gtgggcaaat 1080
   gcatagcctg tttgacctgc actaggccaa aaagggatga tgacaattta cgtgaggagt 1140
   gagtgcttga tcctggaata atagaagagc ctctatctga ctaactctcc tgccaataac 1200
   agttattaac tttgaataaa atgtttaaaa ccgttctttg aaggctagga gaataatcaa 1260
   aaacaggcag aaactagagt gaatataatc tttgaaagaa agcaagccaa ctaggtgggc 1320
   tccacatata tccagcttgt cacctaaagg catttctcag ctcatgagac acagtgggag 1380
   gtttgaacta aaacagaaag tcttaatggg atgaggacac ggggttggag tttgaggttg 1440
   cctacgtggc tggaagttaa ggattaaaat cctataaagt agggaaccac agagcaggga 1500
   gctctgaaat ctgcatccaa atacccctca gattcttggc tgtctcctaa cctctacgta 1560
   tacagggtgc aacctcaagg aatccagaaa aaagccacag ctgggaagct acagagctaa 1620
   gcagagattt cagctgctgc ctagtactgg ccagagttta cagttcaagt ggcaccaaat 1680
   tagagagact ttatgaatat ctttccattg aagccataaa agggtcataa ggatcatatc 1740
   tcatgatcaa ggactatgcc ccaggacaaa aggataaacc aaaatagacc catcctaata 1800
   aagcctaaaa caaagcctac acaggaccca ccagaacaaa actgaacact ctttagagta 1860
   agatagcata gctgagagtc tctacaaggt gtcattttta atgttcagta tggaatgaaa 1920
   atttaataac atatgaagaa gcaggatatg acttgtgatc aaagggaaaa tgggctacaa 1980
   atgctgaccc acagatgacc cagatgttag aattagcaga cacagacttt aaagctgcta 2040
   ttaaaaatat agtgaaggat tgatttacca gaaaaggatg gaatgggtga ataaatggca 2100
   gaatctcagc aaatagatgg aaactaaaaa gaagaaccaa atgaaaatcc tagaactgga 2160
   aaatatagta tctgcaatga aaaggtcatt gaatgagctt aacaacagaa tggacacaac 2220
   aggaaaaaaa tcatttaatt caatttgcaa tgtactcaat aaacttctct aaactgaaat 2280
   atagaaagaa aaaaatagaa tgaaaaaaga acagaggagc caagactgtg gaatgatatc 2340
   aaaagattta acatttgtgt aactagggtt tcaaaaagtg aaagggtaga cagaaaaata 2400
   tttgaacagt tttgtaaaat tgatcaaaga cagcaccttc cagatccaag aaactcagca 2460
   gaactccaag cacaataaat acaaaaatga ccacatttag ataggccaga gtcaaactgt 2520
   tgaaaaccaa agataagccg ggcacagtgg ttcacaccag taatgcctgc actgtgggag 2580
   gccaaagggg gcagatcact tgagcccagg atttcgagac cagcctggcc aacatggtga 2640
   aaccccatct ctacaaaaat tagccaggca tggtgacatg cacctatggt cccagctact 2700
   tgggaggctg aggtgggagg atcacctgag ccctgggatg ttgaggctgc agtgagccat 2760
   gattgtgcca ctgcactcca gcccaggtga cagagtgaga taaaaattca agataaatat 2820
   attgtcatgt gaggctagag ctcagggaag aggtcagagt tggaaaagac tgggagaatt 2880
   ctcaatgtca gtagtattta aagcctggca ctggatgagg tcacccaaag aaagaaggtg 2940
   gtctgagcag agcaggtgct aggaccagcc tgccacacag cagggtttag tgttcagact 3000
   gcacaagaac cagcaaagac gctgagaagg agtggcctga gaaacaagag gaaaactgga 3060
   gaaaattgaa tcacagatgc aaggggatgg gatttcatgg atgtaccttt tccatgggct 3120
   tttcctgtgt ttcactgtat tactccctat tatccatgtt atttttctgt aagcgtgtgc 3180
   ttttctttct tcctctacgt agagtctcaa gctgattttt ttctctgact tgtttttata 3240
   tctctgtatc caactttgtc ttttatcctc ttcttcattt ctcttttatc gtcccttctc 3300
   cttctgaatt tgttttttaa atgacgctga tacacattag gcatttcatc agtttattat 3360
   ttattatatt attcttggag ttgtatgttg ctattattaa tattgcttaa aaattagtct 3420
   aacattttgt aaataatacc caaaaaataa gccacaaaag aatccagcaa attaaactgc 3480
   ttttgtctcc attgattttc ttttgtggga caatgtatta gttttcttat tttgttttta 3540
   aaaatttatt caatcccttt ttcctctatt aagaacatta tattatttaa taaaagccct 3600
   cttatgagca gttttggcta cctcaatgat gtaaaattaa tggtgtatat gcagatatgt 3660
   gcatactctg ccaggtcctt ttgtctttac tcaaaaaaaa gtaattgcat attcttaatt 3720
   ataagacaaa gatttggttt tttttttttc ttccctaaaa ccattttttg gctgtgtgct 3780
   gtggctcatg cctataatct caacactttg ggaggccaaa gtaggaggat cacttgagtc 3840
   caggagttgg aggctacagt gagttctgat cacacccact gcactccaac gtgacagagt 3900
   gagcccgtct ctcaaaaaaa aaaaaaaaat gaagcttgta tttttaaaat aaaaatgggt 3960
   tgttaattct gagttcaaca catgtaaaca ttcatttttc aattttgata tttcataata 4020
   tagtaaaata tcttgattag gatacctgta ggtactaacc actagaaata atttgataca 4080
   ccagtgtaac tggtcataac tgttgtttat ctgttgaata tttaggttta ctcttccaag 4140
   ctatttgtaa caggagccta aaaactgggt ttctttcatt gcacttcatg gctcactggt 4200
   cattgaagat ggcaagcgag gggcactctg gatacaggtc agtttcctga caaccatcag 4260
   gatagtgtga taggcacacg gttccaactg tcctcattta tttaaatagc aagctaatta 4320
   aggaccttga agagtattag agagtttcac gatctgctca tgtcctgtga tttaaccgta 4380
   ttataattct ttaatgggaa ttttataaac aggcagtttg agatgcagag tttccaacag 4440
   acttaacgga gttttttccc ctttaggtgt aaaaggaaga gaacatctcc tggacctaat 4500
   tgccacaatg cgatactaca gtttattcgc accaggttgg aaaaaacggg aatagtgttc 4560
   aaatcactga tggaactgga tgacgcttct atttccaggt ggctttaact atagtattaa 4620
   ggaacttatt tatattttca actatttatt taataaacct tacaaacaag ctacagggaa 4680
   ttatctttca tgtgtaggct actttataag tatgtaggaa gataatttgt ttgcaggcga 4740
   cagcttgccc acagtctcct gtggcgggca gggctgatgt ggtatttgtg ccttggcctg 4800
   cacaggaaat gggctcaaag aatttcagtg cagctccccc tggagcccgg cgcttctggc 4860
   tctggttgtg gtactcttca cagcagtctg ctgatcctgg gtgcctccag aagacccaca 4920
   gtgtgactgt tgggcccagc cctgttccca acaatggcag ggcctgggac aagaatacaa 4980
   tacaagtatt taaagtttat aactttatag tttataacaa tacaagtatt taaaaagttg 5040
   taaatcaaac tagcaaacta ttagattaag tgtgtcctaa tctcctccct tggcaaatac 5100
   aacttcataa ccacctggaa ggccaggttc aaattcagcc cttggctgac ccccagcacc 5160
   ctaagaggcc ttgcctgcac acgtgaatac cccaccccac ccatcaaaat gccatccaga 5220
   ccccatgcca gtgccccctc acaccagcca ccttgtaaca ccagtgagat ggcagcactg 5280
   ttcaaactca ggatgacaga ccagggaaga ccctggaagt ggactcaggg ccatgtggtc 5340
   agggacgctg gagttgcagg gtctaaaaag gaagatatgg gctccggggt gggcacattc 5400
   tcttgtccct acagaccctc caccccatgg cgagaggggc atgaccagag taggatccct 5460
   ctgacataca tatatatgta tatacataca catatgcaca tacatacaca tatacacata 5520
   tacatagaca tatgcacata catacacata tacacatata catacacata tgcacataca 5580
   ttcacatgta tgtgaaggca cacatgtgca catacacata tgcacataca ttcacatata 5640
   cacatgtata tatataagta tatacataca catatgcaca tacatacaca catacacatg 5700
   tatatatata aatatataca tacacatatg cacatacata cacacataca catgtatata 5760
   tataaatata tacatacaca tatgcacata catacacata tacacatata catacacatg 5820
   taattggctt atgttctttt aaaggcaaga taaggggaaa actaatcaaa ctaataaaaa 5880
   tactattgtg agggaggttt ggtagagagg acaaagatgg aagctaggtt tctcctagtg 5940
   tgctttgttt tgcagttttg actttggaac tatgtaaata ttttacataa taacggataa 6000
   gattgatatt tatttattta tttgttgaga caaagtctgg ctctatcgcc caggctggag 6060
   tgcagtggtg caatctcagc tcactgcaac ctccgcctcc tgggctcaag ccatcctccc 6120
   acctcagcct cctgagtaag tgggactaca ggcatgcacc accatgccct gctaattttt 6180
   gtaatttttg taaagacagg gcttcaccat gttacccagg ctggtctcga actcctgaac 6240
   tccagcaatc ctcctgcctc agcctcccga agtgctggga ttacagacat gagacaccat 6300
   gcccagcctg ggaattaatt taaggtgact ttacaacata gtaaataatt tgactgtgtc 6360
   aaattatgtc aaatccccta gtgggatagt gcctaaggat taaaaaatat tacaaagaaa 6420
   tctcactggg agtagccatg gtgttaataa cacttttgat ttgttatttt ggaaggtaga 6480
   tactaatatt gttagaaacc aatatgtata aaatcaaata agttaaacga aaccccttga 6540
   tttctaaact tgaattagat cagagaatag aggaacatga tatgtgacac catgcaaatg 6600
   cagtcagcaa agtctggaat gtggggaaat aaatggcaag agaaaaaaag gtttgaggag 6660
   cagaacctgt aaataaacct ttagagacat gtcaaccaaa agcaagggta gaccttgttt 6720
   agatcctggt ttgaacaaaa cccactgcat ttaaaaaagt gagacaggaa aaattaaaca 6780
   ctacctggat attttatgat atttagagaa atattgataa ctatttttta ttttttgaga 6840
   cagattctcg cctgtcgccc aggctggagt gcagtgacgc aattttggct cattgcaagc 6900
   tctgcctccc aggttcacac cattctcctg cctcagcctc ccaagtagct gggactacag 6960
   gcacccacca ccatgcctga ctaatttttt tgtattttta gtagagacgg ggtttcaccg 7020
   tgtctgataa caatttttta agtgtaatga ttaagaaaaa gttctcgaac acaaaacagg 7080
   tgttcaggaa gttctggact aggcacattg gctcatgttt gtaatccatg cacttcggga 7140
   ggctgagatg ggaggaccac ttgagcccag caagatcaag cccaggagtt taagaccagc 7200
   ctaggtaacc aatgacaccc ccatctctac aaaaaaactt tttttaatta cccagtcatt 7260
   gctgggcaca gtggctcacg cctggaatcc cagcactttg ggaggccaag gcaggaggat 7320
   tgcttgcttc caggagctca agaccagcct tgaccacata gcaagaccct atctctacaa 7380
   aaaaaattaa aaattagccg agtgtggtag tgtgtgacta ctcaggaagc tgaggtgagg 7440
   gggattgctt gagcccagga ggccaaggct gcagtgagtt gtgattgtgc cactgcactc 7500
   cagccagggc aacaaagtga gaccctgtct aaaaaaaaaa aaggagaatc ataatttact 7560
   atatgtaatt tatacttcca ttttttaaaa agcaatacct tctcattagt ttgccaagga 7620
   agatgagaat ttattctttt ttctatttgg ggggctttct tttttttgta tttatttatt 7680
   tatttattta tttatttatt tattttattt atttatttat tgaggcaggg tctcgttctg 7740
   ttgcccaggc tggagtgcag tggcatgatc tcggctcact gcaacttctg cctcccaggt 7800
   tgatcacttg agcccagcaa gaccaagtcc aggagtttaa gaccagcaat tctcatgcct 7860
   cagccttccg agtagctggg attacaggtg cgcaccaccg tgcccggcta atttttgtat 7920
   ttttagtaga gatgaggttt cgccatggtg gccaggctgg tctcaaactc ccggcctcaa 7980
   gtgaaaataa accagtgcca cttgccgaga gaattaatta aataaatatc aaagaaaaaa 8040
   cactttgaac agctagggga tgggactaaa aaattagtat ttgacttaat aggcacatcc 8100
   aaagaggtag ttattctatt tatattgttc ctaattagta atattatagc tgttagaaca 8160
   agtataaact ttgaacctgc ttttgaactg taataccaca aagtattaaa gcaagatttt 8220
   caaaattgat caagtatatt ccagcatata gctcttgcta cattactgct atggtgctgg 8280
   caaaatggtt tgctggcact tgaggacata agacaatacg atttcaggcc gggcgcggtg 8340
   gctcacgcct ataatcccag cactttgaga ggctgaggcg ggcagatcac aaggttgcag 8400
   tgagctgaga ttgtgctact gcactccagc ctggcaacaa agcgagactc tgtctcaaaa 8460
   aaaaaaaaaa ttaaatattt tttatttcct gtttattgca tgtttgaaac tttgttctgt 8520
   attttctatt tcaggatgta cgtaacattg atgcaggagt gcattacatg ggggtgtggg 8580
   cacaagattt tcttactagt ggaatgtgaa ccaaaaagtg tagaggccac tgcactaaag 8640
   gaggccggct caatcagtga aaatattcag agccagtttt gtcgttttca gcagtcagta 8700
   actatcagta gacaaacatt taccaggaaa tgttggtctt ttaaacactt tgggcatgct 8760
   tcttatttag tatgttcatt ctatcatgac attcagtgaa catttattga gtgcctactg 8820
   tgtaccaggg attagtaagc acgttaaatt tataagcttt gttgatttcc accacaaacc 8880
   cataggacct catgttattc tcataattga ggaaactgag attcccagtg ttgaatgaaa 8940
   gccacacagt atcacatggc caatatcatg tgattgcaga gtcaggactc aaacccagct 9000
   cttcaccatc acgctatact gacagccctt tcccagttca cagggaaaaa tcaggaacag 9060
   ggagagaatt ttcaaaatat taagtttccc catagaattt tctgaagaac tttggtgtat 9120
   gttgccactt gttcactaac aagttctagc agatgacaga acaaatgagg aagtagctaa 9180
   ttaatattaa tgaacaacct cagaattttt ctgagtgttg aatagacttg aatattcaac 9240
   agtctcaaat atttgacacc atttagtgga cacagacttg actcgatatg cttattctac 9300
   caagttgttc ctgatttacg tgacacaact gaatgtatgc cagtttcatg ggggactctc 9360
   agatattctg aaggtaccta atcaagtcag tacagtggga aggagagaga aacagaagcc 9420
   tcatttgtga aatagtactt gagtttgtgc gtgaagaatg gataggattt agactgagaa 9480
   gcgtggaaag gggacaacca taaacaactg tagagaggct ggaacgtgag ctgtgctgca 9540
   ggcagcaggg atgcctggag gcacgtggaa gccaggaggt ggcgggcaca ggacacttga 9600
   caatgcagtg cataagctca ggtgagacgg gcagaagaac ttccgtaaga tgccaaggaa 9660
   aactgctttt attaaatatc ttaatttttg gcagaatcat tttgtctgat ataatgtgat 9720
   gggtttagta ccacattaca tttcacatgt tatattctga aatctgaaac tttttttttt 9780
   aatctaggaa tattccctgg gcttttgagg caataaagca agcaagtgaa tgggtaagaa 9840
   gaactgaagg acagtaccca tctatctgcc cacggcttga actggggaac gactgggact 9900
   ctgccaccaa gcagttgctg ggactccagc ccataagcac tgtgtcccct cttcatagag 9960
   tccttcatta cagtcaaggc taaatcaaat gaaactgaat tttaaacttt ttgcatgctt 10020
   ctatgtagaa aataatcaaa cgataataga tacttacaat gaaactccat taaggtttta 10080
   ttcagtgtag caattagtgt ctttaaaaat aaagtggaag cagaattact ttaatcaact 10140
   aacaagcaat aatgaaactt aaaatatttc agttttctgt ctcatttttt gttgttgttg 10200
   ttttattttt tatatttttt attttttgag acaaagtttt gctcttgttg ctcagactgg 10260
   agggcaatgg cgtgatctca gctcactgca acctccgcct cctgtgtaca agcaatgctt 10320
   ctgccttggc ctcccgagta gctgggatta caggtgcgtg ccaccatgcc ctgctaattt 10380
   tttgtatttt tagtagagac aggatttcac caggttgccc aggtaggtct caaacttctg 10440
   atctcaggtg atccacctgc ctcggcctcc caaattgctg ggattacaag catgagccac 10500
   tgcacctggc ctgttgttgt tgttttagag atggtctcaa tctgtctccc aggctggagt 10560
   gtggtggtgc gatcatacca cactgcagcc tcaaactcct gggctcaaag aatagtccca 10620
   cctcagcatc ccaattaatt gagactatag gcatgtgcca ccacacccag ataattttgg 10680
   ggtgtacttt ttgtagagat ggggtcttgc tatgctgcct aggctggtct tgaattcctg 10740
   gcctcaagca atcctcccac ctcagcctct gaatgtgttg ggattacttg tgtgagcctc 10800
   tgcacccagc cagtgtttca gagctttatg gtgatgagag atgcttctgg atggtcctgg 10860
   gagcctgtgt tctcctcaca ttcaggagtc cagtggaaag ctggctcagc tggactgagc 10920
   aagctgagaa gaaacatggt cttttgtagg ggatctgtca gggtggtggg agaaattata 10980
   aaaattaagt tgtaggaaat agacacaaaa cttcttggat ggctgggagg tttgcatagc 11040
   ttctgtaaaa gatttggctg aaggcagctg aattctctta aaagcttagg gtgtagatac 11100
   atacgaaggt ggaggagttt atctaaatag cttgtttact catgtggtcc taaaacagac 11160
   ctttgatcat ttgcgggcgc atgactgctc tctactcagg aggtcagcaa tgttaattac 11220
   cctctagtgg tgtggtgttt acttgagacc tttgtcatta aatctctact gaataaatgc 11280
   ctggagcccc agcctgacag ggctgtgcct gctgactctt tacagcaccc tcctcagtgt 11340
   ctgggagctg cccggtcccc taccccactc tttcatggga tacctgtgtc tgagtgcatt 11400
   tcttcatctg tcatgcagcc agggtctgca ggttggaccc agcaagttat tgacagagtt 11460
   tgaaaattat acaggattgg tcgttgagtt ggatatttta gtcaacattt attaagccca 11520
   tgcctcagca gtagttgact tttgtctaaa gggagacaaa gaaattctag ttttcccaaa 11580
   tgtccaaggc tccaaacaaa acatactgaa tcagaatttc cagaagaaag tcctggcaac 11640
   ttggatattt aataggtgct ccaggttatc aagcacatta gaacacaatg gcccaggtgc 11700
   tgtatctcaa agtgtgagtc tgtggactgg cattgtctgc ctcactcggg aactcatcaa 11760
   gagaggcaga atctctggtc ctgtttgaga ctattgaatc agaagctgca ttttaattag 11820
   caaggccctc aggtgactcc tgtgcatgtt tatagttgaa gggctagtgg aatgtggtga 11880
   caggaacata tttggtgctt gttcagggct ttgttttaag aacccatgat gcttgtgccc 11940
   agtggagcaa cccatcagtc caattgttgg gagaaaagct gaggcaagac ttgctatctg 12000
   acataatatg aaaagagcct tggaacatgt cctgggtcca gagtttaaaa cttcttgtgg 12060
   cctatggaac accaggctct gtgcttaagg gtggaaggct gccccactgc actacaacct 12120
   aagcccaggg cataaaaccc ctcgtggctt ggatggaatc caggactcag tacataaaac 12180
   ccctcacggc ctctggaatg tgtccagact tgctggctcc ttgcttcttg ctctcccagg 12240
   atcataaatt gattgtatct tgaggtagaa ggaaatgttc aacattatct tgagtagcag 12300
   aacatgttcc atatgcttca aagaaaatgc tagactgaca cagctgtaaa ctaaacactt 12360
   gatgtgactg cttcctttca accagcacat cctcgccacc tgcttctttg tttaatcacc 12420
   aataaatggt gtgggctccc agagttcaag gccttcacag cctctatact agcgttggcc 12480
   ccctggaccc actttatgta ctattagctt gtcttgtctc attcctttga ctccgccgga 12540
   ctttgtagcc cccacggcct ggtgttgggt ttgatcacct caacaccaat gtggatattt 12600
   gtgactgcct ttgttttata atatttttat tattcacctt gtacagatga agacttccag 12660
   ctgtagaggc caaaggggcc acactgcagg tagagcaggc attcaccaag gcgtttgtga 12720
   tcctggggtg cagctcccac ccttggctgc ctgggtgctt ctttccacac ctactgtctt 12780
   gtgacatccc cttaggcacc cagcacaggc tgctgtctga tttagaactg agcctgttca 12840
   gttgatggct cagtgcaggc ctctaaaaaa gcaagcctga tgtgggttag tacatctgtg 12900
   gttggcttac ttatttggtg ttcatttttt cagttttcag aaaggtcagt tgaagtgctg 12960
   ctggtggctg tagaattgat acgcagggga agtttttgat ctgtgtgctt tctaagggac 13020
   aattgctaag ccgcactgat accttgagcc cctttccttt ggaacaatgt agagcccagt 13080
   tcatggctcc cagaagttta ggtccagtaa tttggctgca ggcctagaga atgggaggct 13140
   gcagacttgg actaatggtg aactcttgcc tccccccagg gatatgtggt gcctgtcata 13200
   agctccagag agctgccttc catgagacca gcagaagagt gggcaaacat gaagtccaat 13260
   ccccctatcc aggctgccat tgacctcatg gcaggggctg caagtacagt cctgtgcctc 13320
   ataaccatgt ttctgttggt gatggatggt gtatctgatg gtggtcccat gagattatac 13380
   catcttttac tatacctttt ctgtgtttat atacatttag atacacaaat accactgtgt 13440
   tacagttgcc tacagtatcc agtacagtaa cgtgctgtac aggtgtgtag acttgagcaa 13500
   caggccatac tgtacagcct aggtgtggag taggctacgc catctagggt cgggtgtata 13560
   tgctctatga tagcacaatg atgaaattgt gtaatgacac atttttcaga acatgtccca 13620
   gtcattaaga aatgcctaag tataatccca gagaaaaccc agtcttctct attcctcaag 13680
   taccttctaa atgagatcaa atgaaagtgg tatgaaaaca ttgcctttct ccctctagaa 13740
   gtgatgggct ttacctttgg gatgaatcca tttgatggaa gtgttgttca gagtgtggca 13800
   gaaggtagag gagtgtccat tattgctaac accttgtgag tcagggtgag ggggggaatg 13860
   tgcccagtac agctggaaaa cagctcagag gtgaagcccc ttgtccactg tcacctggct 13920
   gggataggca ggtgacatgg ttcttcaatt caagtctggc agtcttcaga gcccttgtag 13980
   tgcaaaaata acaccgtagc atctcccagg aaacaccact tactgtccct gctagtgttg 14040
   gcaaagaaag gaggacactc tttagaagtg tcgtttacaa tgagactgat aatggactgt 14100
   gtcttagagc catgaaagga aaggagaagt tgtaggattt cctgggaatg tcagctaacc 14160
   tgagcctagg ggcctgagcc caagggcaga ctgaggctcc cccagcacag ggaggtgcta 14220
   cctgtgacaa gggggtagtg ctggcacagt gcaggctact ccctagaaag atcagcttga 14280
   acatgcagga agagcagaac cctcgggctg aggcacaggc ggaataggaa gtgcatggtg 14340
   gtaatttagt tctcctgggg ccagaagtag gaggagcggt tggaaagctg attgcccaaa 14400
   gggaaaccct ggactaccct ggcctcccac aggactctca tagtaattgt ggctccctgc 14460
   agtggggagg ccagaaggag tgttgcccaa tgctgtcctc atccagtcca ccccccaccc 14520
   accaccaaca gatgagtatg gtcatgagtg tggttacctc atcagtcatt tgctcagctg 14580
   tgaaaaagaa attgttcaga gaagagcaaa gtgtttttcc atgagccaga ggtcagccaa 14640
   ggtatgctaa tgaggaggac tggagacagc gcatcacaga cactgataag gagcactggg 14700
   caagggcact tctcccaggg cagagcccac aaggagcgtc ctggcaccag atgctcaggg 14760
   aactgaaggc tggcaggggc ccgcccagta gtctcttgcc cactgtgtgc ctttaggcta 14820
   cagcctccca ggccccgtgc cccctccctt cccccatgcc acactgggga agatgctgac 14880
   cctgagagtg gtgcaaccat ctgcctgccc ctggccacct ctcaccagca cgtgaatgct 14940
   cttgggctca gttctgtctc tgcagtagga gggtgtttag ccacatgaca gagtctcacc 15000
   ttttgacatt ttatgtcttt cagtctgtca aagatctaga acatattttg atggaattaa 15060
   aaagtttact tttggctggg caaggttgct caagcctgta atctcagcac tttcggaggc 15120
   tgaggcaggt ggatcacttg aggtcaggag ttcaagacca gcctggccaa tatggtgaaa 15180
   ccccgtctct actaaaaata caaaaattag caagatgtgg tggtgcacgc ctgtaatccc 15240
   agttactcgg gaggctgagg caagagaatt gctctaaccc agaaggcaga ggttgaagtg 15300
   agcgaagatt gtgtcattac actctagcct gggtgacaga gcgagaatcc gtctaaaaaa 15360
   aaaaaaaaga gtttaccttt tcctgtttat gtgcatacag gtatttgggt accacatttg 15420
   taattcacag ttagtttgaa agatagaaat tgaaacagta tggttgggag cctggtatgt 15480
   aagccgtttg ctggtgggca tgtgtgtatt gggcccttgt atgtacgatc tctgtgatcc 15540
   catcattact gttcatttct tccaggagag gaatgacaca cttctcactt taattgccag 15600
   attatctccg attctgaatc agtggaatat aatttaatat gattttacta tgattaattc 15660
   agggtcattc tctcccaggt gctgctgggc taggataaaa tgagatctga attacattaa 15720
   aataattaag tattccaaga gaagaggtga cagaatggtt taagtggctg cgtagtttag 15780
   ataacctaga tgcagataag ctgaatgaag catttaatcc agcacattcg tattttaact 15840
   tgatcatcag caataggagt ttgaaaccca gttgcagctg caggttccac acaactgaat 15900
   tcggttcctt gtgcatagta gtgttcactg gagatgcctg gaggtctgaa ggtgtgccct 15960
   tttataggca ggaattccac caggatgggt atagcttgtt ttttttaaaa ggcttgggac 16020
   tggatacgat tgcccaagaa gacagtgtag acagcaagga gggctgagca tgaatgggat 16080
   gacctaccag cacaaaaagg gcaggcagga aggcagtaaa gaaatgagca gggcccaaac 16140
   caggagagtg tggcatgcca cgatggagac tgaacagaag caaaacgatg ggcgactgta 16200
   gtgttgttcc ctgttgaagg agagacactc tgttacagaa gacctggact tcagttcagt 16260
   tcagtttcct tagcaaagga caaaatgggg agagaagaca gggaatcagg gaagaaaaac 16320
   tcactcactt aatgtgacgc aaacatcgca tgcagtcgta ccaaccaagt aaacatgata 16380
   agctacaaag aactatggtt ttcgctgctc tcaaaatgcc actgagatta ccaagtttta 16440
   agcttatttc aagattattt ataggtgatt ccccaactgg gaaagtcatt tctgaatttc 16500
   ttctcttcta aaatagatct agaaaaatta acaaacttta ttccagtaag agagtttggg 16560
   atgtactgct tcagcccctg tcctaaggaa attatcaagc ctggagggtg gggtgctatc 16620
   aactgcctgc caacatgtta ccttctggca tccagaagct tccctgtgcc agatggagcc 16680
   accaatgtgg ggaggagagc acaaagagca gagaatccac agaaaccata cctgcaggga 16740
   gcaaacacca tcacacccca ctgctgtgaa tgcaaatgca tttacctgtt cttaccatct 16800
   ccctcagcat gttgaatcta ttctgggatt agacaagctc ccagacttac ctgctatgtt 16860
   taataaaaac acggtaaagg aagaatcggg gctattaaaa tacttaagaa tgagaaagtt 16920
   gaaaaaagaa aaaaagacgt agattgtgtt tggcatgtct tccccccacc gtaagggatt 16980
   gccagtcagc tggaggcctg ctggttcatg tgggccagcc ctcgacacct cagtgaccaa 17040
   acgaaataca accctcacta gagcttcttg tacttggcaa gacccagaca tagacctggg 17100
   ctaccaaaca aaaccttgtg ttcttagagc ctgagaacaa gagctttgac agtttccaca 17160
   aagaaccctt ccgcagcatt agcatttcta tcttcaccta cctgcagcca ttactgcatt 17220
   gaactctgga aatgtcacat cagctgttct taagataaaa taccatttgc tatttttttt 17280
   ttcctctagg ccctgtccct ttgatgttaa gtggtggagg gagttggcgg gatttgctct 17340
   ggcttgcggt atatccagtg gattgtatca aatccagaat tcaagttctt tccatgtctg 17400
   gaaaacaggc agaatttatc agaaccttta taaatgttgt gaaaaatgaa gctgaataaa 17460
   cacctgtttt tccagcatct attatacatc ttaaaatctg agatatgaac ttctctgttt 17520
   tgattgtaaa aggaaagaga acagtaacac agaaactttg tgtcccaaat agagcacaga 17580
   taccttgtct attactcata gtaatcctcc cttaaatttg tataacatgt gaaattttac 17640
   aaagtactta tacctccctt atctaattca atcttcataa ccaccctggg cagtactaat 17700
   attttaaacc agtctccaac ctcaaggttg gtgctcttgc cactgcccag gtgaagctgc 17760
   aaagctgtgg ccacctctgt ctccatggtt gcctctgtct ggccatctca ggcctcaatt 17820
   ctgccccatc catatactaa gagttaacca agcatggaat tggggtatgt tacatgagca 17880
   tcagtcagtt ttgccaggag acctgatact tgtccttgtt tctgttggtc tatttaggga 17940
   aagctgggaa tgcgtccttc tatgacttgt tggtttttag cactcttgtc tggatttgca 18000
   tgtgtaaata ttcctgtact atgcagctgc atgggtgtcc ccaaaggagg gattgttgca 18060
   gtctttgctt gcccttttgt cttctaggaa taatggcctt atattctgga ctgaaaccta 18120
   catgattcga gcattccctg acaatggagc actctttttg gcctatgaat atagcaggaa 18180
   tttgatgatg agccagttgg aagcatactg aagtgtcttg gagggcctga gccaagcaca 18240
   ggtgtttgag gactacagtt catttcaggg tttcttgtag tacaagacca gcgtgaagtc 18300
   attctgattt cttgggaatt ttgcctttct gtcttctctt ataccctata tcttaacttg 18360
   tttggaagaa cctctatttt gcatcatgtc atttctgccc ataattgtac tgaaatagag 18420
   aagtcactgc tctttccctt ggtaaaatag agagtggtca gtaggcttat gcacctaatt 18480
   caaaagactg agtacagttc tgtcagggct tttacataaa cctctacttg cacattcaat 18540
   ttggacagtt atgtgttgag ggaaatacag tttggttcca tgtttatttc aaatattgcc 18600
   agaaaaaccc agaggtgatc gtttttcatg aagatgctta caaatggttg cttaacccat 18660
   tctagatgta ggatctgctt aatgtgtgta ctattctaag tggttgattt ttttttttta 18720
   aatatggagt ctcactctct tgcccagtct ggagtgcagt ggtgcagtct cagctcactg 18780
   caacctctgc ttcctgggtt caagcaattc ttctgcctca gcttcctgaa taagtgggat 18840
   tacaggggtg cactaccaca cctagctaat ttttgtattt ttagtacaga cagggtttca 18900
   ccatgttggt caggctggcc tcgaactcct gacctcgtga tccacccgcc tcagcctccc 18960
   aaagtgctgg tattacaagc atgagccact gcacctggcc aaataaaaca tatttttaac 19020
   caataaaaat taaggacaaa gaagagcatt ccttatagaa tgataaaggg cacaattcaa 19080
   ccagaagact tagcctaaat atgtacacac ccaacattgg agcacccaaa ttcataaaac 19140
   aagttgttct tggcctacaa aaagatttag acacacaata acagcaggag acgtcaacaa 19200
   cccactgaca gcaatagaca gatcattgag gcagaaaact aacaaacact gtacttgcac 19260
   ttgacagttg accaattgga cctgatagac atctatagaa cactttgtcc aacaaacaca 19320
   gaagatacat tctcatgtgc acacagaaca tattctaaga tcaaccacat gcttggccat 19380
   aaagcaagcc tcaataaatt caaaaagttt gaaatcatac caagcacaca ctctgaccac 19440
   agtgagacaa aaatagaaat cagtatcaag aagatctctc aaaactacat aaatacatgg 19500
   taattacata atttgctcct gaataactcc tgggtgaaaa tctaaattaa ggcagaaata 19560
   aaaaaattct ttgcaattaa tgaaaatagg gacacaacat accagaatct ctgggatgca 19620
   gctaaagcag tgttaagagg aaagtttata gcactaaatg ccttcatcaa gaagttagaa 19680
   atatctcaaa ttaacattcc tactttgcac ttaagggaac tagaaaaaaa gaataaacta 19740
   actccaaagc tggcagaaga gaaataacta aaattagaga agcactgaat gaagtgcttt 19800
   ttgaagtgct gtttttttta gactagatac aaaaatccac acaaaagatc aatgaaacca 19860
   ataattagtt tttcaaaaaa taaacaagat ggatagcctg atagctaaac aaagaaaaaa 19920
   agagaagagc caaataagta caatcataaa tgacaaagat gacattacaa acaatcccac 19980
   agaaatacaa aagatcctca gagaatacta taaacaactc tatgcacaca aattagataa 20040
   tctagaggaa acggataaat tcctggaaac atacaatctc ccaagattga attagaaaga 20100
   gattgaaacc ttaaatagac aaatgccaag ttccaaaatc aaatcattaa tataaaatct 20160
   accaacaagg aaaagccctg gactagatga agcaacagct gaattctact ggaggtacaa 20220
   agaactggta ctagttctat tgaaactgat ccaaactgag gatgaggggt tcctccctaa 20280
   ctcagtctat gaagccagca tcagcctaat accaaaatct ggcagagaaa cacacaaaaa 20340
   gaaaacttca ggctaattac tctgattaac atagacacaa aaattattaa ccaaatacta 20400
   ggaaacagaa tccagcagca catcgataag ttaacacacc acaatcaaga aggcttcagt 20460
   ccagggatgc aagactggtt caacatacgc aaatcaataa atgcgattca ccacataaac 20520
   agaactaaaa gcaaaaacca tatcatcatc tcaatagatg cagaaacagc ttttgataaa 20580
   atcaaacatc ccttcacatt aaaaacctca acagactaga catcaaggga acatacctca 20640
   aaataataag agccatctat gacaaactca cagccaccaa cctactgaat gggcaaaact 20700
   tggaaccaat ccccatgaga aatggagcaa gacaatgatg ccgactctca ccactcctat 20760
   tcaacatagt actggaagta ctacctagag aactcaggca ggaaaaacaa agaataggca 20820
   tccaaatagg aaaagaagtc aaattatctc tctttgctga tgatatgatt ctatatccag 20880
   aaaacgaaag actctgccaa aagcctacta gaactgataa acaactttag caaagtttca 20940
   ggataaaaaa atcaatgtat aaaattcaac agcatttcta tacaccaata atgtccaggc 21000
   tgagagttaa atcaagaaca gaatcccatt tatggtagcg acaaagaaaa tgaaataact 21060
   aggaatacag ctaacaaagg atgtgaaaga tctctacaag agctacaaaa cactgcagaa 21120
   ggaaatcaga taacacaatt aaatggaaac acattccatg ctcatggatt ggaagaatca 21180
   gtatcattca aatggccaca ctgctcaaag caattgacag attcagtgct atttctatca 21240
   aactaccaat gtcattcttc acagaattag aaaaaagtat tctaaaagtc atatgaaacc 21300
   aaaagatggc ctcaacagcc tgagcaaatc taagcagaag ggcaaagcca gaggcttcaa 21360
   actgtaaggc tatagtatcc aaaacagcat ggtactggta caaaagcaga catatagacc 21420
   aatggaacag aacagaaaac cccgaataaa gccttacact tacaaccacc tgatattgca 21480
   agctgacaaa aacaagcaat ggggaaagga ctccctattc aataaatggt gttggggtaa 21540
   ctggctagca atatgcagaa taatgatact cgacccatac ctttcatcat atacaaaaat 21600
   taaccggaga tagattaaat atttaaatgt aagacctcaa gtgataaaaa tcctagaaga 21660
   aaacctcaga aatacccttc ttgacattgg ccttgggaaa gaatttttaa ctaagtcccc 21720
   aaaagcagtt gcaataacaa caaagattga caagtgggac ctaataaagg taaagcgctt 21780
   tggcacagca agagaaatga tcgacagagc aaacagataa cccacagaac aggaggaaac 21840
   atttggaaac tatgcatcca acaaaggtct aatatccaga atctataagg aacttacaca 21900
   aatccacacg cagaaaccaa ataaccccat taaaaatgtg gaaagaacat gacacatact 21960
   tctcaaaaga agacatacaa gtgaccaatg agcatctgaa aaaatgcttc acatcacgga 22020
   ttgtcagaga agtgcacatc aaaatcacag tgagataccc tctcacacta gtcagaatgg 22080
   cgattaataa aaagtcaaaa acaacagatg ctggcaaggc tgcagagaaa agggaacgtt 22140
   tatacattgt tggtgggagt gtaaattagt tcagccactg tggaaagcag tctggagatt 22200
   tctcaaagag cttaaaacga gctaccattc aacccagtaa tcccattatt ggatatatac 22260
   ccaatggaaa aaagaccatt ctaccaaaag acacacacac tgatatgttc attgctgtgc 22320
   tattcacaat agtaaagata cggaatcaac ctaggtgccc atcagtggtg gattggataa 22380
   agcaaatgtg gtacatatac acgatggaat tctacgcagc cataaaaatg agcaaaatca 22440
   tgtcttttgc agcaacatag atggagctgg aggccattat cctaagcaaa ttaatgcagg 22500
   aacagaaaac caaataccac atattctcac ttatgagagc taaacaacag gtatccttga 22560
   acataaggtt gggaacaata gacactgtgg actactagag gggtggaggg gagagggctg 22620
   cgggttgaaa aactacctat tgggtactat gctctctacc agggtgcaat atactgatgt 22680
   aacaaacttg cacacatacc ccctatattt aaaattaaag ctaaaatttt aagacatgag 22740
   tatcattctg atctaagttt cttgagttct tcaaagaatc attcattttg cattctaaga 22800
   aagcaatacc caaagaggcc ttaaatgagt tttctttgaa taagcgaaag cagaatagtg 22860
   tgacaactca taatttccag aaattttggt gggtcttgga ggtctgggga agagaaacct 22920
   tactggtaat ctctatttca aaaagggaaa ttcttaaaat aaaaaagggg gtttccttcc 22980
   ttcctgtatt tctggtactg aaaaagacag atcttaatct tcactgagcc agctaagtat 23040
   tttcctccct attcacctaa acccaggaca taatctgccc tctgtagatt atggctgtgt 23100
   gaattttaaa ggaaatagtg taacctatgt tgtgcctaca catgtttctg tcatatgaat 23160
   ggcagggcgt aatattaaca tgaaaggaat ttgaaagcat gttctatgat cttttcctca 23220
   aagggggagc caggtcaggg ggagtaaagg aaaaaggcct tagctgggct gctgcatggg 23280
   cagtcggaac gtttttggaa tatttagagg agaaaaaatc acctgcagcg aggtggctca 23340
   ccagcctagt ggggctcttg gctcctggca ggttgctgcc ttccgtgccc accatggctc 23400
   agggctctac tctcatctgt gccactctgg tacccacagc cattatagca agtatttttc 23460
   tatttccact caaaaattaa aaagctctct ggcaaactcc cgacattgag ctgctgatgt 23520
   gagtggtccc agcggttagt gtgttggtta gaaagttgct atgccagaca ggtttgccct 23580
   ggactctcaa accctgtatt ttcatgaact gcttcttttt ttagtccttt ctgtagagtg 23640
   ccaggttttt caggaatgag tatatcgtgt tttagcagtg acacttttct tattagttgt 23700
   tatctttcat cttggtctag attgttgagg gccttgccaa taaaaggaaa atcattcaaa 23760
   taagaataat ttcaataaag ccaagataga aggttttcaa ggacaatcat ggcctgaaga 23820
   tacagtggca ataaaggtat tttgaataaa gtaacaccca cggactgggc atggtggctc 23880
   atgccttaat accagcactt tgggagactg aggcaggcag attgcttgag ttcaggaatt 23940
   tgagctcagc ctgggcaaca acagcaaaac ctcgtctcta ctgaaaatac aaaaaaatta 24000
   gctgggtgtg ctggcatgcg cctatagtcc cagctactcg ggaggctgag gtgggagaat 24060
   cacctgagcc tgggaggttg aggctgcagt gaaccaagat cacaccactg cactccagtc 24120
   tcgacaacca gagtgagatg ctgtctcaaa aaataaaaaa tacaaataaa aaatttgtta 24180
   aaaaaaaaag tagcacccag caccaaagag gaaataataa tgattttctg tagacataag 24240
   gctaaccagt gtttataacg caaagataac ctcccttcag agtcccccca agcttaggtg 24300
   aagttggttg tgagcaggag tagtgaattt caaaataata cacctagatt aagaatattt 24360
   gacttcactt acaattttat acaaaagcag aagttaccaa agattactta tcttcatatt 24420
   taccacagtc ttcttacccc aacccccatg tctgtgtgta attaaaggaa gcttgtcaag 24480
   taagctacct atttaatgct cggaatgaaa gagagtgtgt gttgggagtt ggggaactgc 24540
   ttatgtgaaa catttctctc ttctggattt aaaacttagt cttggttgcc aatctgttaa 24600
   cagactgttt gtaaatgaag gggaagaaaa atatataaat tagctttcta ataaatctga 24660
   aattacaaat gtgaaccaaa gcagggaata aatacttgac caaaaatatg taagtaagtg 24720
   ggtgttgggg aatcacaatt tttaaatatc tcaagttttt gccatgaaag ttctatttca 24780
   aagttcttca aaatgatgcc tgatgttcct gcatactgtg ttccaaattt atgtaaatac 24840
   aagatggaaa gtgtgtgaag tatgtgcctt caaaaaagaa gaaaaacact gacattttat 24900
   ctatatatat ttaatagatt tatgaagaac atatataaac atatataaat atagatgcat 24960
   catcaaggaa gacttaggac acatgaacat gtggcagggg ttggaaagaa cataattctt 25020
   tcccagaagg ggaaggggga gctatactta gtcggatcca gctacaacat cattggaagt 25080
   catttctcgc caaaaagtat ctccacagca aattctggtg gataaatttt ccatgctttt 25140
   tgtttatgtg gattatccaa ttcatttctt ggtagacaaa gcctaagaat agaaaaaaaa 25200
   aattacattt tatattgggg ctcaccaaaa agccacgcag ttgggtaccc acgttagagc 25260
   tggaagaaac aaaaaaaaac ctcaccatga atagaacccc agaccctttt ttgcatttca 25320
   gttggctcct tccacctttt tagtgagact taaaaatact ttaatcttca gcaacacatc 25380
   agtaacacat gctgactctc ttagcattgc tttatatgga attaatgaca tccaagttta 25440
   ataaaatatc taaatttctc tctgtgacag aaatcagtca ggatagacat gaatgaaaac 25500
   ccaggaataa aatactatat ctcttttaat ttatgtgtga agttgcaatt ttttgaattt 25560
   ttacaatcag accttggaaa tgaccttgag cagtaggcta taactcccat atgcttagcg 25620
   ttccaataat ggaacactag gcataaattg gttaacccat ttatgcctgg tgttctaaat 25680
   gatagaagtt agcatttttg gctaaacaac aatctcataa ctaacaaaaa cagctttacc 25740
   aagtagtatg taaatttaaa tgttacagaa atctttagaa atttatataa aagtaagaat 25800
   aaaagtgatc tagcttatca cttctccaaa atgaacgcag tgttttaaag gaaaaaaacg 25860
   gtatccttta gcaagaacca cttttgagga gcagcatcaa atgaagctcc acccaggtct 25920
   cactttttga gggactttgc tcatgttaga agaaaaagct tattgtttgt atgcatctaa 25980
   aaaaaatact tgtaaaaaat ttccatcaaa tccaaagttg actctatcaa aatccattaa 26040
   atgttttgca ttgcaagtgt gtagaccaga ggtttaattt cctgttgcct tgctggactt 26100
   aaggagttat tcgatccagt tcacgtttga agaaaagatt aggactggat gtaacaataa 26160
   ctatcaattc atgccacata taatcatagc cacttcttca actctgacct aaatcattta 26220
   aaaaatattt tgtccttttg tattgaagag tatggttgat acaaaaaaaa atctcaattt 26280
   ttcaccatca caaaacaaat gctacttata gtggagaact tctagactga gaaatgtggt 26340
   tccaaatatg gcaggttttt ttggaacaat aatctccaaa cccaattagt aatttttcag 26400
   aaagttttcc caattcagtt attagaaagc cacatttgaa tggctattta aatagactat 26460
   tacttaatga tatttcttag ctgtattcct taatactgtg tcttgtaact tctcccagat 26520
   ataagtgtgt ccgtcagctt ttcagctagc tgaagcctcc ctaggtcctg ccttacttta 26580
   gcacaaaagt tgatggtggt tcgtcattgg taaatcagca cctactgaca gggacctgac 26640
   atactgaagg tgctgagcag actcatattg aacttctctg ggaggaattt actaccaaac 26700
   ttaagatctg atgataatac ttttgaactc ataacacagt cctatggcat ggaccctgag 26760
   gatactgcaa cagtgggtct caagaacaaa tgtattttta aaataaccaa agtaagagga 26820
   gtaaggcaag gaaaaatggt ggctcaggga ttttttgctt tctataaatt aagtttatga 26880
   caaccagcaa aagacttgcc attacacctt atttatagac attgaagatt aggctgtttc 26940
   tatgggtttc tattatattc atacctgtta ttttgaataa aagacgtgtt gaaccagaag 27000
   aaaaatggac aattgtcata gtatttagga agattctaaa aagaaaaaaa atttcaaagt 27060
   aaaactgaca ccaggaacta atcattccaa agaaattaaa gttcctttct ttattccttt 27120
   atatatttat caaacaatta taaccattaa ataggtagca ctctgcaatc tataacaata 27180
   cactcaagac acaaaggagg aggctttaag aaaatgctat tgcattctct tgctgtttct 27240
   atcaaaattt tcaaggaata gtgtttttcc catgataggt ctttaattta aatgctgcta 27300
   tttgaatctg gtttagatag tgccaaacag atgctactaa ggacctatgc ctatatatac 27360
   ccaactacat tgtagtgaat tcttataatt tttttgttgc ctcagcatcc cttttaatta 27420
   taaattgtat tttctgatct cagaagcggg gttcagtcac cctcagcagt ttccagttca 27480
   cctcctccca gtttctccat gtgactgatc ctgatatctg ccttatacaa ccttctcctg 27540
   atgaccactt cactatggga cagctagata taacctactt gactcatccc acagaccccc 27600
   atactccgca gggaccatgt ggatatatca gtgaccacct ctcagtcaca gcagagactc 27660
   atggctgctt gcgttaaacc aaattagact tcccctcagg aaacctgctc aggtagcacc 27720
   ttacatccca ataaaggctt ccactctcag gtccctccct ctctctcgct attgctccca 27780
   cccatcagtc gagcacaggt ctcctggatg gctccccctt ccagttggcc ctgtgtgctg 27840
   ccctcttctc tctggaatta ataaaaaact gctttggtta tttcgtgtgt tgtattgtgc 27900
   tgccttctct gtttcaccca actgagtcac ccaaacctaa ctctttctag tcagtgctcc 27960
   cagctactcg ggaggctgag gtgggaggat tgcttgagcc caggagggtg aagctacagt 28020
   tagctatgat cacgccactg cactccagcc tgggtgacag agtgagaccc tgtctcaact 28080
   tcttaatttt ttaaaaaaga tgcaaaacaa aatgtaatca ttgctatgca gtaaaacgta 28140
   cctttgggtt tttttttttt tagcatcttg gcttttactt tttttgtgtt tattttgtat 28200
   tttttgtatt tgagaataca ggtaaattta gaaaaattta gaaaatttac aatttagaaa 28260
   aaaacaaatg aacacaaatt atttaaatta catacgctct tttaaaaaaa tttgttatag 28320
   atctgattta ttgttttcct ccccctcccc ctcccttgtt gggaattgta gtaaagcaaa 28380
   tctgaaataa aatcattaga ccagacaaga acaatggcag aggctatttt gtgattactc 28440
   accgaagaga aaaactgcac tttcacatca tcatacagag gtggaccgtc atatacatta 28500
   attaatattt tgtctgtttc aatgtcatgc aatatctatg aatgaacaca tggaattgag 28560
   aactacaaac ctagataacg ataacaaaag ttccttttca cttatcatga cttcctgtat 28620
   ttatcaagta cttttgctct cacagcccaa tcactacatg cataaataat tattaataat 28680
   cttttctagt tgcaatactg ttgtttatgt gtgtgtgtgt tttcatttgt tttttgtttt 28740
   ttgagacaga gtctcactgt cacccaggct ggagtgcagt ggcgtgatct cggctcatgg 28800
   caacctctgc ctcccaggtt caagtgattc ttgtgcctca gcctcccaag tagctgggac 28860
   tataggcaca agccatcaca cccagctaat ttttgaattt ttagtagaga tggggtttca 28920
   ctgtattagc caggctggtc tcaaactcct ggcttcaagt gatctgcccg cctgggcctc 28980
   ccaaagtgct ggattacagg catgagccac tgcacccggc ctgttgttta tgtcttttaa 29040
   aggccaacat gtttagggtt atcatgaacc acttttcttt ttttttcttt tcttttcttt 29100
   tttttttttt tttttttttt gagacagagt ctcactctgt caccaggctg gagtacagtg 29160
   gcatgatctc ggctcactgc caacttctgc ctcccaggtt cagctgattc tccagcctca 29220
   gcctcccaag tagctgggac tacaggcacg tgccaccatg cccagctaat ttttgtattt 29280
   ttagtggaga cggggtttca ccatgttggc cagaatggtc tcgatctctt gacctcatga 29340
   tccacccacc ctggcctccc gaagtgctgg gattacaggc atgagccacc gtgcccggcc 29400
   gtgaggcact tttcaaaagt gctgcacttt taaaaagtat tgaattttcc attagacttt 29460
   aatcaattaa atattattat ttaatgtgtc tcatttgatt ttcatgtgtt agggcctttt 29520
   ttgagaatac attaagaagc tcaattttaa agctagcatt gacattcaaa aagcaatgca 29580
   taacttatca tggtttttta ttttctaaat ttaaaaaata acaacagggg tctgtaacac 29640
   ttgggggttg ggcctgcatg ctcctcccaa gggaggggga gagtggggtc cccctgctca 29700
   tcatgtttta tcagaactct tgtgtttgtc aatgggatct ttgaacttga ccagtgcagg 29760
   ctgcaggatg gagtgaccaa agatagctca agttttggaa tgaatgactt tgaagccgta 29820
   ctagttcttt gactcattct catagaaata atggcaccca caataagaaa ggtctatgct 29880
   aggccctgtg aaaaacacaa aaggaggtgc agactcagtt ggccccttca gggagctgta 29940
   catttaagaa acaatagtag aatggtacat aactcaatct gtatgacaaa gactgggtgt 30000
   gctgttatgt actactgggg gctagttaac agaaggtgca gaatgcaaaa atttcattaa 30060
   ggaggtagaa attgaattgg gcatgaagca gtttagataa aggaagaaga gctctgaaaa 30120
   aagaatttag tccaaacgga gagaaaaaga cgagcaataa cacaaagtag aaattagaaa 30180
   aatgggccag gtgcggtggc tcaagcctgt aatcccagca ctttgggagg ttgaggtggg 30240
   cagatcacct gaggtcggga gtttgagacc agcctgacca acatggagaa accccgtctc 30300
   tactaataat acaaaattag ccaggtgtgg tagcacatgc ctgtaatccc agctacttgg 30360
   gaggcggagg caggagaatc gcttgaaccc aggaggcaga ggttgtggtg agctgagatt 30420
   gtgacagtgc actccagcct gagtaacaag agcaaaactc catctcagaa aaaaaagaaa 30480
   agaaaaagaa aaaacttgga aaaacaacaa caaacaatgt cgggcagtgt ggttcatgtt 30540
   tataatccca gcatgttgca aggctgaggt ggacaggctg cttgagccca ggagtctgag 30600
   accagcctgg gcaacatagc aagtcccagg ctctacaaaa aataaaaaaa attaggggat 30660
   tgcttgagct caggaggttg aggctacagt gagccatgat catgccactg cactacagcc 30720
   tgggtgacag aatgagaccc tggctcagga aaaaaaaaaa ggaaaggaag aaataaccaa 30780
   ctaactaact aactaaatta gagttgtgtt ccaagagcca aaagggatga cccagtttat 30840
   ttggaatgaa gtctgctggt caagctactg atggaaaaaa gctcaggtat tatgttaggg 30900
   ctattacatc ttcagagctc gagccaagga aataagagag aagttgttga aaaatatggt 30960
   ggggcatttc ctatgtgaca aagaggcctg ataattatca catataacac ttatttagtg 31020
   atttatagtt cacaaagaaa cttcatatat attatctaat tgaattccca ttacacttgg 31080
   atgtgttatt tattttacag ataaagatgt tgagacaaag gtgttaactg gctctgacca 31140
   tgggcacaca gtgtgtcttc tgctctacct acaatttttc acaatgagag gaatatttca 31200
   ggatgcatag ttcaggattc tatacattgc attatagaag gagaaagaaa gggtaaggag 31260
   accactgcag aaatctacgc tgagcaaagg agattctgga caattagtgg cagtgagaac 31320
   agaaaggaag gagaacatag aaaactgtca aaggcataat caacagagat ggatgactaa 31380
   ctggatttag ggggctgagg caaagagaat caaagcccaa ggcgtgagtg gaagaatgct 31440
   ggtgccattt gcagaggcag agagaaagct ccttgataac tgagactggc gaatgcattt 31500
   gagttggagt ggaaactgtt catgttgtag ctcctgcatc taccactcag tgggtgctca 31560
   taaatagctg ttcagtaaat gaacaaataa tagttgaagt aaggggaatg ggtcattctt 31620
   taagaataaa gcatttgcta agaacagaaa gccaagggtc aggcctgggt gaatgccctc 31680
   agttgggaga ggccaaaaaa gccgggggca gagtcgggag ggggtggcag ggaacagggt 31740
   tagtaatagg agaatttgag acaaaggcct gggtaggaat tctaaaattt agggttatta 31800
   gaccatgaag gatgctttct tactgtttca gaaaaggtat atagttttag gcatttcttc 31860
   attgtagcaa aacattgact accaggtccc ttttgctttg gataaagagg ttcctcccca 31920
   gttcattcac ccttgcgttg tttctcacag ggtcatccca cagaacgtct gacttagaat 31980
   cacttgggac attgctaagt ctatagattc ctgggcccca ttccagattt cccgaatcat 32040
   actttggggg tacaacctag gaatgtaaat ctttacaagc tcctcagtgg ttttcatgca 32100
   cactaaactt ttagaatcag tgctatataa taaaagatca aatgactgag aagaagagaa 32160
   tatggctaaa aatgaccaga tatgtaaatt aaaatcctta atacattcct gggaacatgc 32220
   tcagcatttg tggaagtgtg aaaggcaaag caacactgac cagaaataca ttctggaaag 32280
   caccctgaaa aaactgtttt gaacaaacag gttaatttat ctctggtttg catccaaacc 32340
   cagtgacacc aagttgtatg gtaaccagaa agcgttatgg aatctcttta tggtgagtga 32400
   tagtttatgg aggtgaacat tgctggtttg acattcagcc ttaaaacaaa catcagattt 32460
   tttcaattca catgttttct cttaccgaac aatttcctaa tgaagtactg gaaaagacaa 32520
   cctttttctc cattactact tggactttta gatcacatac atcacctgtt ccaacaaaat 32580
   gaaattaaaa agttaggttg gttagcatga agatcaatat gtctcaccac ttaacatcat 32640
   ggattaaaaa gagaaaaaat tatccaagct aacaacgtat caatagttac ttatttggtc 32700
   ttgcatggat tttgtctttg aaagacaagg cttcaagccc atttctgtct cctaaagcag 32760
   attttataag atgagagaag agaaaagaat acatatagtt cagcttccaa gacttagaat 32820
   cccatcactt tatctttcca gaaaaaatat ttagggaagc taggccgggc atggtggctc 32880
   acacctgtaa tcctagcact ttgggagggc gaggtgggtg gattacttga ggtcaggagt 32940
   tctaaaccag cgtggccaag atggtgaaac cccatctcta ctaaaaatac aaaaattagc 33000
   caggcgtggt ggcacatgcc tgtaatccta gctacttggg aggctgagac aggagaattg 33060
   cttgaacccg ggaggcagag gctgcagtga gctgagatca caccactgca ctccagcctg 33120
   ggtgacagaa catgactctg tctccaaaaa aaaaaaaaga aaaagaaaaa gaaaaaaaat 33180
   tcaggaaagg tgacttgcaa cctttcttgc taatatgttg ctttcttcag acttctaatc 33240
   ataatgattc ttaataataa atactgtcat ttaactactt tggccctact ttaaggccag 33300
   ttcctgatgc aatctctata aaaatatagg atagttatat ccaaaaattc ctgtggtgtt 33360
   cagtatatat ttacagacaa aggctcttct gttccttttt ttcaattcaa atgacccact 33420
   tcccttagcg tttcctcaaa gacttggttt ctcagccaat taatttttta ttgctttcct 33480
   ctcaattcta cattaatttt aatttaaata ttcacaaaac taagtagaga attaaaacaa 33540
   atattttaat aaactagtgt attaattatc tttgattgtt catctcatat gctgtttgtt 33600
   ttcatttaat tttgttttca ctaacaccat gatattcatt tgtcattaga aatcacagtt 33660
   tcttgcctca gttccctctt tcccattcca ggtgcagaac ttccttctat tgatgtctac 33720
   cagaaatcag tgaaatgtag aactaaactc agaaggctaa aaatactagg ctgtctgtgt 33780
   ggacaccagc atcctggccc catggcacct atagcaccca gcactggcac catctcaggt 33840
   ccacaagctg gctgctagaa gaggtttgct ttaggggttc cgaagcatcg cctccctgct 33900
   acctctctgg cagtgggagt ggtgaggccc cattccttcc acacagacac atcctgcttt 33960
   cccgcctccc ctgagtgtaa gctgggagtg agagggttac agatggagga gagaggggaa 34020
   agggagagcc cagggtgatg agaaaaaaat atgcctctaa aagaaggtga gaaaatgata 34080
   cgatacggag acacctccac caaggcaaac tctgaactag aggtggttct gagaaataag 34140
   caggcttgag ggcacatggg tcctcctcct ccctcatcag aggctccctc tgcccggaaa 34200
   agtccacctg ggttgagacc ggaggagcaa tgagaacagg agagatgggt aggggatgaa 34260
   gtcccccaaa ttggtgatgg aaaagaaaaa gtgaagaaca tctcagggct ggcaatcaat 34320
   gggcaattgc aggaggagcg gcataaataa ggaggagttc ctgtgtgagg atatgtaaaa 34380
   agacaggtat gagttaggat catctgtata tacgactatg agcaaaaccc tcccagagtg 34440
   gggacattgg ccctcctgcc caattcttca attacacata aagcacttac cacgaatcga 34500
   ataaataatg aatcttttta taaagagtat ccgtcttgga gggagattcc agttgtagag 34560
   atgtttcact tgtgcaaaat atccaacata tctattctga aaagcaacag aaatcttcct 34620
   ttaagtggag atgaaaagtc taaagtcagc attccttttt tttttttttt ttgcataaag 34680
   cttgttaatg gaggtaagta attaagaaaa catttactgg atgccattat ataactggac 34740
   atttttaggg atgctgcccc acaactatct accttgaggg aaaaatactg aattcagtgc 34800
   aatggggaag taacaaggat ggagccaggc cctgtttata ggctgcttat ggttgagagg 34860
   gcaactaatc agaagcacaa caaaacagtt acagcatcat cagtgtccag acagaagctt 34920
   agttgtgact gagttagagg aaggcttgtg gtttggtgaa gagcagcttc ctggacatga 34980
   gagaatggta taagtgaaaa ctccagagga atacctccaa acctcagtag caggtttccc 35040
   gtcactcttc tcttgttcct gacagctaga atttcttcac tttgcatttc ctcaattctg 35100
   ttttctcaag gaggccctgg aattctctca gccttgtcct ttccctgggg caagctgttc 35160
   acaacggctc tcccctgaat ctttcttcaa ggtattgaag agctctggta ctcaatcaac 35220
   tggttcagaa ttcaaaagtt ggccatcatg gtggctcccg cctgtaatcc cagcactttg 35280
   ggaggccgag gtgggcagat cactgggcgt caggagtttg agaccagcct cgccaacagg 35340
   gtgaaaccct gtctctacta aaagtacaaa aataggccat gcgtagtgat gggcacctat 35400
   aatcccagca ctcgggaggc tgaggcagga gaatcgcttg aacccaagag gtcgaggttg 35460
   cagtgagctg agattgcacc actgcactct agtctgggtg acagagcgag actctgtctc 35520
   aaaaattaaa aacaaaacaa aacaaagcaa aactcataag ttcacagact gttccttacc 35580
   cattgggaaa tgttcattaa agtaactaac tgccatactt ccctttagaa agggccagtc 35640
   atgagacagg ttcatgaaag tccttaactt aggagtagat gtagtcagta gaatggccca 35700
   caatgcagga tcagaatgtg atggtgtctg agaatcacca ccagacatag agggcactct 35760
   gtcctgaggt cttgccaaca cagctctcaa acatgcagct gagtccctgt gttgcttttt 35820
   cggtgaatca gaagagattc agttgcagcc tttggtgtgc tggttctgga ctaaagttag 35880
   ggaaactatg catgactgca gaagaaatgg ggatggggag atgtgtgggg acagttgttg 35940
   aaggagctcc atctagcatc cccttagttt ctgccactag attctccacc tttccctaag 36000
   ggctgtaaac caccagggcc ctcaagctac ttgtgctccc acgcttcctt ctcctacaac 36060
   ttaaaacaac atggacttgt agtagaaatt aatacaacca acacatagga ggcactcact 36120
   acaaacaaag aaaatattat gagaaaaggg tggaaagatg ggtaagccac gggctttgcc 36180
   tttaaagggg atacagaagt caccacttca aataactaga gaatgatgca aagtgacgta 36240
   gtccctaaaa gaaacacaaa gggctctaag aattaaaaaa gcaagagact acttcaggct 36300
   gagaagggaa agggactcta ggaaaagggg catgagcaat ggcatgaggg tatcaagtgt 36360
   ccataggctg ggaaagagcg cacagtctaa atgtgttgga gtatgggatg tgtgcatgca 36420
   catagtggaa gagaaggttg gaaaagtcaa agtgtggctc ttgagtgcca ggttgaaacg 36480
   tttagtttat tgccattggc agtggggaca tagtatggat tttgagcatg gaaatggcat 36540
   gtttagggct gcacttcagt aaattcacct atcactttta gcttagggtc tatgactatc 36600
   tttgttttgt tttgttttgt tttgtttggg gcttgggtgt gggagaaaag tagcagtgga 36660
   taagggtgag ttcttctggc ccaaactaaa ggtttagggc cggtagttag agtgttcttc 36720
   taacggggcc aagagtactt actcagctcc caccacttaa agaaaaactc atttacagcc 36780
   acatccaaat ccatttcgga attcccaacc cgctctcact tcggtttcag gggcaatata 36840
   gaaaaggcaa tataaatgag aagctgtgca cagcaagggc ttcagagact gacatctaga 36900
   tttcagtcct tggttcaaaa gttactttct ctgaacctca attttctaat ttataagatg 36960
   agaatcatag tagtgcctac tcatagagtt actgagaata ttaaatgaga tagtgaatag 37020
   agaactctta gaatagttca gcaaagtcta gaatgttgta aacgctatag gataaatatc 37080
   ctggtttctg caataaacaa attacaagta ataaaaaatg gtgagaaatc tatgaactta 37140
   gattcttaaa agagacttag gagacacaga aaccaaatga aatgtgtgga ctttgtttga 37200
   attctgatta aacaaactaa attttcaaag attttaagac aattttgg 37248

<210> 20
<211> 4714
<212> DNA
<213> Homo sapiens

<220>
<221> misc_feature
<222> (1)..(4714)
<223> chromosome 16 nucleotides 7525911 to 7530624

<400> 20
tgaatatttc ttgggttctt actatttgag aggcgcagaa ctgggccttg gagatacaag 60

agtgaataag acagacatag cttctccttc aaggaatgta cagtctaccg taacacagaa 120

tacacacaca cgtcactcac acagttgaat gcagccagcc atctgtatcc accaggttcg 180

acatcccatg gaaataagaa aacactgttt cttccatctg caattggttg aatctgtgga 240

tgtggatctc gtggaaagga gagctgactg taattacatt tgcaaaaact tctgtgaagg 300

aaaggtagag ggtgatgtgc aaacaagcaa cagggagaca taaatcaaag cggagatgca 360

ttcaagactt ccttaaagaa gtgactttta acatgaaaat tagaggctgg gcgcggtggc 420

tcatgcctgt attcccagta ctttgggagg ccaaggtggg tggatctctt gaggcaagga 480

gttcaagacc agcctggcca aaatggtaaa accctgtgtc tactaaaaat acaaaaatta 540

gccaggtgtg gtgctgcaca cctgtaatct cagctactca ggaggctgag acaagagaat 600

cgcttgaact caggaggcgg aggttgcagt gagccaagat ggcaccactg cactccagcc 660

tgggcaacac agtgagactt cgtctcaaaa aaataataat aattaataaa taaaagaaag 720

acatgcagag aaaggtgggt ggatgtcctg ggtaggacag acagcagatg tggaaacaag 780

atgcaggggc gtgtggaagc cagctcttaa tggcctgtga ttgtcgattg tatatgtctc 840

tttccaacag gacagggatg ccatggtagt agcttgttac ctccagggtg agagtattta 900

cactatgggc attggcaatg ctacagatga gggcttttct gtctgctttt ctggagagct 960

ggttgttgat cagttaccag cactccagtg acagaaagga agttggcata ttcctgtgac 1020

aggatgaata tcagtgtagc tgtgtggacg ggcaggggga tggtggccca agttgaggat 1080

aaacaggtga gcaagaaaca ctcatacaga gccctgctgg cctcatctgg gggctttgta 1140

ccatgggagg ccattgaagg attttaagca gaaagaatcc catcattagg tttgattttg 1200

ggaatgatct gaccaccatg tggacagtgg attggagaaa agcaataatg gtgttgggaa 1260

gtggcagaaa ggactgccag taagctcttg ccatgggctg gatgagagat gttgatagga 1320

actagaatgg tctggaacat gggttggagt caatatcaaa tgaccaaata atctgccaga 1380

gctctctgta atatacagta ttcacacctg tggacttttg tgacaatttg ggacggtata 1440

gtcccttgta atttaattct gagagtgacc attgaggatg tgatgatgct ctctttacag 1500

tgaggaaact gaggcgtggt cagttgatgt gactcagtgt gttccctgag gtcatagctg 1560

aacaagtctg acttagtaat atgtctgact ctagacctac acttataacc acaacttcac 1620

actctttttg ctgccacctt cttctaaggg gctggaagaa tggagatgcc actgagctgg 1680

gggtgaagac atcgtaaaaa gatctacctg gttctatgag atgcatccat cctgaccttg 1740

ccaagggaag taggaatcag gaaatggtgg cttctaacgt gatttcatcc aggcccttat 1800

gcttgggtcc atctcaggaa tagtgtttgg aattaacgca ggagccagag actaagcaaa 1860

ccccgcctcc accccagtgc agatttcagt tgaatgcaga ctagagcctt tgaaaattat 1920
gcattggaaa acaattctgg ataaattaat ttccatcata cataatgcat gaggggaaat 1980
ccgtggtatt tttaaacaat ggaaatgaga gaatctgcct tttgggcatt gatcccaatt 2040
aaatttattt cagcaaacat ttatcaacac ctactgtgtg ccaggttctt actggaaaac 2100
agagatgagt cagacattgt ctgtgcccgg aaagagtgag aaatcaatgg aggtacccgc 2160
agagtgatca gctctgggtt agaccttgag actgggagac aaagagttgt gagtattgcc 2220
ctggagggac atacatccta gtagaggaga acgaggacct gccagaagat ctgagagtga 2280
gagtttgttg aggaaggcta tctcgtgcct gggcttcact tgaatggttt tctttcatga 2340
gtccttccgc catcaataag atccttattt aaagcctcct attgtccagc aatggtatgt 2400
gctgtccatc tgccccatta gcccatagct gctactatta tgctactttt cctatggtgt 2460
ccactgttat tactgccaca actacctcaa cttactgttg aggaaaatgt aattctcctc 2520
aacttctatt actattatag tgttgctgtt attttattta atgatagatc atacattcga 2580
aattatttgt tgagtgctta ctgtgtaact ggcaccgaag attgaggaac agataaaaaa 2640
tagctatggt ttcttcgttt agaatgttat cttgtaggag agagagatgg aaacaaataa 2700
acaatgtaac taaataatta caaattgtaa tgtttgcttc agttatgcat tgctgcctaa 2760
caaactaccc caaacaaaca ttttatttac ttacaattct atgcttcagg tatttgggtg 2820
aagtttagct gtagagatca gcttggtaca tataccactt ggaactgggg agtaagttcc 2880
aaggtttccc acagatgtct ggaacttggt gattgtctgt acaacacatc tcctcccccg 2940
cacccccacc cccaccccgc cactcaatct ctccacaaga ctagcttgag cttcctcaca 3000
gcatggagtt ctatggtcaa tgagactttc atatgggcat catcttggca tgtacacact 3060
tcagtggtgg ctgaattcga aaaaggtgtg tctcaagaaa tgagagtaga agctgcatat 3120
ctgataaagc tcagccttgg catttgctat gtgctatgtc tgcctcagtc tgttgattaa 3180
aataaggtca tctcagattc aaggagaggg gaaaagactc cacctttgac taggagatat 3240
gacaaagaat gtgaaaccgt cttgagtcca ccatgatact ttttgaagaa aacacatatt 3300
aggttgaaaa agagaagatt aaggagggag ggacatgaga tcagggaagc cttctctgag 3360
gaagggtcgg ttaagctgtg ttctgaagga tgggagagtc cagcaagaga agacagtggg 3420
agcagcattc caggaagagt gaagagtatg tgcaaagcca ggtgtccctc atctctctct 3480
attcccagtg cagcttcctc accactcccc aaccaaatag gagtctcttc ttgtaggtgg 3540
aacacgggat aatataaaga gcgtattttt gcacatctga gaagcccagg tgacatattg 3600
tccatgatct gaagcaacag ataatgtgtt tcctcatgta tttcccaatg ggatcaccca 3660
aagtaacatg cttaaatgga tgagttgtaa tcttctacat agaggtgatg ccaccaggca 3720
ttggtgatcc agataaatga aatgcaggaa taaggatacc gagactgttt ctggccacaa 3780
aggaccaggc agagtagtct attgcttggc gaccaagtgc atgatagcac tgggcttcct 3840
gcttctgccc tccccagtga acttcagatg aggcttggga gatgaaagac gagagaggag 3900
tcattatcag aatggtaaga atatagcatc cagatagatg agaagagaca aaaagataac 3960

tccgtcccca tactggctta tttctccttt atggagaagg cagccccttg gaggtaggca 4020

tttcctgggt tcaaatccca acgttgccct ttattcactg catgactttg ggaaagctat 4080

tgaacctcac taagccctaa agcatagaag gtgccctata aattgtagct gccactactt 4140

ctattattat catgtatgtc attattgcta ctgctttgca gaaagacatg tgcctcattg 4200

gtggtgggat tcttctaacc cccgtctcct cctttcaaag gctcactctg ctactttaga 4260

gttatctttt atagtgtact cccaagcttg cgatgttgct tccctgcctg taccattttg 4320

ttttctccag atctcgctgt ttccctcgct ggaaactcct tggtctctga ctgtagcaat 4380

atgtttaata actcaaggta cctgaaaata atgcaatatg gcaccatgtc ggcaatgtgg 4440

ctgagagagt ggctcctgta cagattgcct gacttcacat ccaggctccc tcaggtgtta 4500

cccatgcaag acttggcaag taacacaact actctgtgtt atttatcatc agttatacaa 4560

ttaggataat taaagtgttt gtcacagagg gtagttatga agacaacaca tgtcaatgat 4620

tgcagagttc ttagcctggt gccaggttta tacaacacat ctgatgatca ctgactgctc 4680

tgtctctgct gtctctatgg ttgttgtcat ggac 4714

<210> 21
<211> 11007
<212> DNA
<213> Homo sapiens

<220>
<221> misc_feature
<222> (1)..(11007)
<223> chromosome 17 nucleotides 8906153 to 8917159

<400> 21
atcaatcttg catctcagta gatttttagt ccccgaggct cagactgcca acaaaaaatc 60

tttcagggca ttgtaatatt ttgatgacca cagacttcct tggaatgaag ttgtcccaat 120

tccgaatgag gggccacggc ctcataaaag gtttttattc ccctttctgg gctggagaag 180

ccatctagga aaatgctgtt tctgtaccta aagtgttggg attttcagat tgttttcatg 240

agttacagac acacggagca gcatgcttga acttagaccc tttctgcaaa tgtcaggctg 300

tatgataatt gagccagaat gttactcgag ctttctgaca cattttttga gacctcccat 360

ccagtattct catacagaaa tatggctcct ccaggtgtgc acttaacact cgcttctttg 420

cagccagggg aacctttagc taaggaaaaa aaagccctcc tacccttggt ttcaaaaggt 480

tttgaaatgc taattcacaa ccatgaagta ttctgacagc ctggaatgtt attcagggca 540

atcaggcacc ttgattatta cagaagtgtg aaaggtaaat tgctgctacc caagaagtca 600

cccagctttt caagagaaag ctcagaaaaa tagcacataa aattatgttc ggggattaca 660

cttcatgtat cgtgtgatgg ggtgctctga cacccagcgt gtctgtaggt gtcacaacgt 720

cagctcgtag tagaaatgta acagcatttg ccagcgtcgc ccttaagtaa aaaaaaataa 780

taataataca ggattgtggg aaacattttc agaatgtgtt gtatgcctat ctaaaatctg 840

aaagtacatc ttttttgagc ttctgctttt cctattttcc actgagaaat cctgtcccag 900

agatgagact atcttttctt tttctttttt ccaaagggaa agactatttt ttatttatcc 960
tatagtcttt tataggtatt atcattaatg actttttttt tttttgacgg agttttgctc 1020
ttgttgccta ggctggagtg cagtggcaca atcttggctc actgcaacct ccacctcccg 1080
ggttcaagtg attatcctgc ctcagcctcc tgagtagctg ggattacagg tactggccac 1140
catgctcagc taattttttg tatttttagt agagacaggg tttcatcatg ttggccaggc 1200
tggtctcgaa ctcctggcct cagggaatcc acccacctcg gcctcccaaa gtgcagggat 1260
tacaggcgtg agccaccgct cccagcccat taatgacttt taaagctttg ttgtattttg 1320
ccacaataag cacaaaatgg tgtttctcca tggtccaaac tcaaagtcag gattcagtcc 1380
ataatccaaa agttccacat tgagctatga aagaggtaag tgcaggtggt tagcagagca 1440
aagttttttt ttttttggtg acagagtctt gctgtctccc aggccggagt gcagtggcgc 1500
aatctcagct cactgcaagc tctgcctccc gggttcatgc cattctcctg cctcagcctc 1560
ccgagtagct gggactgcag gtgcccgcca ccacgcctgg ctaatttttt tgtattttta 1620
gtagagatgg ggtttcaccg tgttagccag gatggtctcg atctcctgac ctcgtgatcc 1680
gcccgcctcg gcctcccaaa gtgctaggat tacaggcgtg agccccaaag attttttttt 1740
tagccaaggc agatgcatgc cttgaggaag gagctgttgt cactaccaac tgctcatgag 1800
aatgagggaa acccatctca ttcgcttttc cttactgggt tttccagtat gatttattta 1860
ttttttcaat tactaagtaa gaaatacaag catcctaact gtaatggtcc ctttttttta 1920
ctatagcttc ttcaaggtct ggtgggtagt tgtagtttat tgctctcctt ggaggaacag 1980
gaaaggggtt atattgtttg taggtaagtg gaaaccaaac attccatggg gaggaaagtg 2040
aatagttgtt tagggtcgtt tgtatgtaaa tccttgcatc cattcattca tcggtccatg 2100
ctctatccat gcttccatcc atccacccac ttcctggcca gccttctcct ctaactgact 2160
tccacatcca ggagctcgcc ccagggttct cccttccctg tacacattct ctctgtaaat 2220
gaccgctgcc attttcatgg ctttccataa agtgatgcca agcctctctg ttttacctct 2280
ctggccctga cttctctttt gagcttcaga ccccacagct ccccatttct cacttctgcg 2340
tcgatgcctc atgggcacct caagctgctc aggccagata cctaggaggg ttgttccaaa 2400
tccctctttt tctttcactc ccccatcccc aatcagttcc tccctgagtc tgatgagtac 2460
catcccctac tcggtgtatt tcatgtcatc ctgcacactg tcatcattca cacatcgttc 2520
atagtgtatt tcatgacatc attcacatca ttcacactgc cgagtcacca tccccctgga 2580
tggctctggt agttccttcc ttggtttcct gcttccacta gctcctcagt ttgatccatt 2640
tccacacaac agcagtgtga tctctttttt tttgagacag ggtcttgctt tgttactcag 2700
gcaggagtgc tgatcacagc tcactgcagc cttgacctcg caggctcaag caatcctccc 2760
acctcagctg cccaagtagc tggcactaca ggtgtgcacc accatgcccc attcatttta 2820
tttttattaa ttaatttatt tattgagatg gcgcctctct ctgtcgccca ggctggagtg 2880
cagtggtgca atctcggctc actgcaacct ccacttcccg ggttcaaaca attctcctgc 2940

ctcagcctcc tgagtatttg ggattacagg tgtgcaccac cacaaccggc taatttttgt 3000
atttttagta gagacagtgt tttactatgt tgaccaggct ggtctcgaac tcctgacctc 3060
aggtgatctg cctgcctcca cctcctgaag tgctgggatt acaggtgtga gccactgtgc 3120
ccagccttat gtgatcttgt taaaaagcaa tacccttgct ataaacccag agtggctccg 3180
tcggacggtg gcacagattc cttccctgcc agtctgaccc cagcctgcct ctcctggttt 3240
cctgcccaca ctgtggccac attgcccacc tgttcttccc actcttctgt ctgctcttgg 3300
ctggttcttt ctcatccctc aggtttgggc tcaaattgcc ttgctgagac aggcttttcc 3360
tgacacccgg tctgaagcac tgaccccaca cacccatatg gccacagtct cttgctttca 3420
caatctgaaa tgatctcgta cttgtccact cactgaatgc ctgtctcctg acattgaact 3480
gtgaacgcca ccagggcaag caccaagtct gctttgcgat gccgtgcccc agcctccaga 3540
ccaggcctgg gtatagcccg tgagctcagt aaaacggcag tggatgaagg aaggcatgaa 3600
tgaccccaag caggatcctg tgctgggctc tgtggcctct cagattgtgg actatgctca 3660
agtgttaggg aagtatgtag aaacttaatc actttaccat ctactgatcc aagaaaagaa 3720
ggaaagctta atttaaataa attattatca ttaacagagt tgaccataaa atgaacaaaa 3780
ccctcattta tcattttgct aatcctggct tccccactag aatatctacc tttagccaga 3840
gattaaatcc attcatatgt gtccggagaa aatataaaac atatatacaa taaatatgtg 3900
tagaggaaag cagtaggatg gacactgcaa tgtaattgta ttaataagac attaaaaata 3960
tagttaaatg caattatgta tattattgtc cggtcaagaa gatcactagt tgtattaaag 4020
atgtcataag cctgggcatg atggtgtgca cctgtaatcc cagctactca ggaagctgag 4080
gcaggaggat cacttgagct gaggagtttg agtccagcct gagcaacata gcaggacttc 4140
gtctccaaaa aataaaaata aaaaagatat cataaagaca tcataagctt ccattggtta 4200
cttttagtat ggatcaagta gaaaattcaa tagcagagcc gggcgtggtg gctcacacct 4260
gtaatcccag cactttggga ggctgaggtg ggcggatcac aaggtcagga gtctgagacc 4320
agcctggcca atatggtgaa accctgtctc tactaaaaat acaaaaaatt agccaggtgt 4380
ggtggtgggt acctgtaatc ccagctactc aggaggctga ggcaggagaa tcgcttgaat 4440
cttgggggca gaggttgcag tgagctgaga ttgtgccact gcactccagc ctgggcgaca 4500
gagtgagtct ccgtctcaaa gaaaaaaaaa ttcaatagca gatatacaga atggtagaac 4560
ttaaccatgg atcatgttag taagcaatat tacaaatggc acgttgtggc cagttgcagt 4620
ggttcacgcc tttaatccca gcactttggg aggccaaggc aggcagatca cctgaggtca 4680
ggagttccag accagcctgg ccaaaatggt gaaaccccgt ctcaactaaa aataacaaaa 4740
attaactggg catgttggca catgcctgca atcccagcta cttgggaggc tgaggcagga 4800
ggatcacttg aacccaggag gtggaggttg cagtgagccg agactgcacc attgcactcc 4860
agcctgggcg acagagtgac tccatctcaa aaaaaaaaaa aaaaaggcac attgagccgg 4920
agtcggcact ttgggaggcc gaggtgggcg gatcacatga ggctgggcca acatggcaag 4980
accccgtctc tgtaaaaaat acaaaaatta gccaggcatg gtggtgcaca cctgtagtct 5040

cagctacctg ggaggctgag gcatgagaat tgcttgaacc caggaggcgg aggttgcagt 5100
gagccaagat tgtgccactg tactccagcc tgggcgacag agcaagaccc tgtctcaaaa 5160
aaaaaaaaaa atggcatgtt gttagggatg tggacatgat aatgtctcct aaggctatta 5220
aatacagctt caagtgtgga agttgagtat gaagcagggg tatgtgttct gggtgagcca 5280
tagaagataa taaatgtttt ggatctctgt ttctttgagc ctcctgttac aataagataa 5340
cagttttcac tgtattaaat acatttgtca gtggaataag gtccatttga cttaaatgca 5400
cccgggatac attaatttac acagccattc acacagccac cagagctgcc ttcctttatg 5460
actccattct ccactcctgt ctcttacaga attaagtcca aactccttta cgtggaattt 5520
gaggcccttc ttggtctgtc ccaacccact catcttcctc acttcttcag tcgcctccca 5580
tgccccatcg tgtttatgct gtgcaagcat cttcaggcct ggatcgggag ttagttcttc 5640
caggcatctc tgcccatcta gcccctcaac aggtgactca gggtgagggc tctgggcttg 5700
ggggatggga gctaggtcca tccctcggca gccaggaggc cctgggctca ggtgctagaa 5760
gcctctggtg caaactcaca cattagcctc tgcctccata gagttcagat tgtattgaag 5820
atatcataaa ccgggcatgc tggtgtgcgc ctgtaaaccc agccgaggtg ggaggattgc 5880
ttgagctgag gagtttgagt ccagcctgag caacatagag cagtctccaa gcacggtgcc 5940
ttagggcctc ccagaatgtc taactcaggg cagacaatca ataaatgttc attggatgaa 6000
tgaataaact accaggctag aagggattga tgagtttagg gaaacgtctg tttggtgtga 6060
ctggaatcag attatgttct tttttttctg agacagagtc ttgctttgtc acccaggttg 6120
gagtacagtg gtgcgatcac agctcactgc agccttgacc tcccaggctt aagtgatcct 6180
ccctcctcag cctcctgagg agctagaacc aaaggcatgt gcaccacagc tggctaattt 6240
ttaaaacaaa tttttgtaga gacgaggtcc tactatgttg cccagactgg tctcgaactc 6300
ctaggctcaa gggatcctcc tgcattggcc ccccaaagcg ctgggattac aggtgtgagc 6360
tgctgcacct ggccagatta tgtgtgtgtg tgtatgtgtg aatttttttt tttttttgag 6420
atggagtttc tctcgttgcc caggctggag tgcaatggtg cgatctcggc tcaccacaac 6480
ctctgccctc tgggttcaag cgattctctt acctcagcct cccgagtagc tgggattaca 6540
ggcatgtgcc accacaccca gctaattttg tatttttagt agagacgagg tttctccatg 6600
ttggtcaggc tggtctcgaa ctcctgacct tagatgatcc gcctacctcg gcctcccaaa 6660
gtgctgggat tacaggcata agccaccgcg cctggcccga ttttttaaaa aataggcctt 6720
ttcttaagtt agacccttgg gagggaacag tgtgtcatcc tcgaatttcc aaaaggggct 6780
ctaagatgca ggaaagagag tggctggtgg ctggtgtcca caccatatgg cggcagtaca 6840
gactgaccca cggctctctt cccagctggt gccttgcact cagcatgcat ccacccctcc 6900
atgagtacag gaatcagctc atctgctctt caaagtatgg agctgaattt tcctatcctt 6960
gcaaatagcc tcttgcctcc tagcctggct tcctccaccg ttcatcaaga cttcagtacc 7020
aggagcactg tattgatgaa tttcacaatc ccgtggggat aaaagctgct aaaacacttt 7080

tcccaactgc taaattggaa ccttttagac accgagatct cacttcccct cctaggaatt 7140
ttcctattaa aacattttaa aggtctattc aactagttta tagctatggg aattaaaaaa 7200
aaaagaatgg aaattaagtt ctagggacat gtctttttgg tcttctttaa gaatcagtct 7260
aatttccact tcattggctt tttgttattt caaagcagaa gaatgaacat tttctcattt 7320
cgtatttcgt aatgaggaaa ccttagcaga acctgtatat aaactatttt ggtttgacta 7380
tggattaatc actttcactg cgttaaaaac aaaataatga agccaaggag gttatttcct 7440
tttcaagagg aggttgaatt ttctgagcag ctttctccct caaacttctc agagtcttca 7500
ccttggaggc ctcgccctgc ctgcttcttg ggactctgct tcttgtcacc aactgggatt 7560
tggggacaga aggaccagta ggtgggagca cctcaaagta aggagtgtga agtgcctgag 7620
gacaaggtgg caggggcttc cagttcctcc ttaggataag gctagtgagg ttgaccccgc 7680
attaacaaca catttaaagg tttcatctcc tatttgtgat cttgatgtat tctacctttg 7740
cccacttggt gagggtacca agaccccgtg tcacagcaca ggccaaatga accacctgtg 7800
gctcatcctc ctgggaagag tttggtgggt aacccagata ccagtgggta gccttcggga 7860
gaaaagggtg agctagtctg gtaatgttcc ccagcacgtg gctggggatg gtcctggctg 7920
gagcacatac ccgccttctt cttcgtaggt tgtgtagact agtggactaa gaggttggac 7980
cctgtaccac actgagtggg ttcaaactct agttctacca catatcaact ctgtgacctt 8040
gggcaaatgt cttaggtgca ctgtgactca gtttctcttt ctgtctatta ttattgccct 8100
cttcgtgtga ttgttgttaa atgagttaat tcaggggctt agaacagtgt ttggttcaaa 8160
gcaagtgctc agtaaatgtc agccatgagc cttccgggtg gatctcactt cagccactgg 8220
ttaggacaac tcagaaaatg tcatgcgtgc ttctttctcc cgagaaagga aattgtttta 8280
ctcttgtcca aactgtcttc actatttaga ggtcatagag gaacaagaag tgactgagag 8340
cagtcatggc tctttgagcc taaacccccg agactttgga aaagtgaagt gtgatgaaaa 8400
gatggaatta ggaccttagt ctcctgccct acttgtcggg tgctgttgcc actcatctaa 8460
gctgccactg gtttggagct cacattttta ttttttttga gacaaagtct cattcttgtc 8520
tgccaggctg gagtgcaatg ttgcgatctc tgctcactgc aacctccgcc tcccgggttc 8580
aaatgattct cctgcctcag cctcctgagt agctgggatt acaggcacct gccaccatgc 8640
caggctaatt tttgtatttt tagtagagat ggggtttcac catgttggcc aggctggtct 8700
cgaattcctg acctcaggtg atctgcccgc ctccgcctcc caaagtgctg ggattacagg 8760
catgagccac cgcacccggt cctggagctc acttttaagc tacatgtgct ttgtggagca 8820
aagtcatcat ttggtagggg ctggacccat agttcggtct tcccaagact tttggggact 8880
gtttcagacc attcccatgc tgcgtgtccg ccacgttgct tccacagagc ctctctgtgg 8940
tccatggctc ttcccattgc ccgcagacct cacttcctcc aacttcttgg aactttccag 9000
attgagagaa taaccacaaa ccactgaggc cggagcactg aagagtccag ctctttttca 9060
cctctgtcag cggtcatgag ggagcctgct ctctgtgggg acagatctat ttttggtgta 9120
acacggatcg tgtgaacact gataacctca gacagagtga ttttcttcca tgtttctcaa 9180

gcatttaaag ggaagcctct ggaaccaccg tgttaagctc ctgacacggt ccacagcttg 9240
ctctaagcct gttttgatca cttttctaat acaatgatat cgtttcacat tctcagtaat 9300
tagactctgt aatccctcca ttctactctg tgtgaccttt atcttaaatg acatttttaa 9360
aatatgaagt ttaaaaatta aaaaaaaaaa ccataaaaca atgtttgcaa aaaatgtact 9420
aaaatgaatt atagtttcta agccctaggc tgtcatgcct ggtggaagcc aaaggacttg 9480
gctcagaccc acccctgggg ccagtcctac agctgcaggt aagttatgcc cacctgcctg 9540
cccatgggcg ggagaccctg cccgcatgcc ctttcgccag cagcatcaaa tggaggctgg 9600
gggtgatgag cttgtcaagt ggtggccgat gacacttgta ttgttttccc tacaccttgg 9660
aaagtgactc aaatggcaaa catatgtctc caaagtcatg cccagggttt ctttccctcc 9720
agaattccta aagacactct gagtcccagc ttgcagtatg aatggaggct ggggtctgac 9780
caggtgatgg gggtgtgaga agttgcctta tatggtggcc cctacatttc acagcccctg 9840
gcagatgctg cagtttccac atccctgggg aggggtgaga gatgctgtag atgcctttgt 9900
gggatctgag cacacacgca gccccgagag tcgggaggga agccggggct gggagtagga 9960
tctctgccct ttgcacagct gaggtctggc aagatgcttc tcgggcagga accccaaggg 10020
gagcactttc tccacgtgat ccatcatttc ctgggggtct tcatgagtcc ttagccccag 10080
ctttccagct tcacaccagc cccctcccgg gtggccctgg agcctgggtt atttgctgtt 10140
tttttttttt tttttcaagc gggcaccaca cccgtggact gtctgccaag aaggaccatt 10200
gttcgtgaga attcctccct ggctccagct aacccttaga tgcactattc tgattttgtg 10260
ggcttgaatt cttattttct taagcctcac ttctacccct accccccaac ttattcttgc 10320
cttctctcaa cccggccttc tcatcaaacc tcattttgtt gcccaaaaag ctttgagagg 10380
tgggcgggga tgtggaggtg aagcccttca ggaaaattga aggtggctgt ttgacctggt 10440
ctccggagta gctttgcgtg cccgcctcgg agaggaggca gggaggcccc ctgcattctg 10500
ggagaggagg ctgtcgggta acctaagttg gggaatgcag cggtgatggg aagtgagtgg 10560
gaggcagggg tgggaataag cgggaggaga agataggcta aagaatgaaa agcaatatct 10620
ctccccccag aattgagttg attaacctgt agaaagggag gccctgccaa gcccaacttc 10680
cttgtttcca gaaaccttgg cacggaggcc ccgtttctgg cagaggcctc tcctagtcgg 10740
ggaaccagca aagcagctgc tgtgattttc tttttcctct catttctggc aatccggaat 10800
gggactgttt acttttttct tcctctttcc ttccccaccc tcctgtagca gggagttggt 10860
tctggttaat gttgaagatc ataacatgat taccccaaag tctcctgaaa ttaaatcact 10920
tttcttcaaa gagaaagggg tggggggacc gtctggctgg taggccagcg aggacgagcg 10980
gatattgaaa gcagcctggc agctgtg 11007

<210> 22
<211> 35893
<212> DNA
<213> Homo sapiens

<220>
<221>  misc_feature
<222>  (1)..(35893)
<223>  chromosome 17 nucleotides 9517630 to 9553522

<400>  22
gaaagaatat aaatgaacat tatatatatc tactctgaac caactgacac ctcaccgact      60

tctacgaaag aacgcaggaa tgatatgagc acggagtctg gaaactgagc ctagctttct     120

tacttaagag ctgtgtgttc ttcagcaaat tacttaacct ctgtgtgctt cagatttctc     180

ttctgtaaaa tgtgtataat atcagttgct atgggtggta aatgggttca tctatgctaa     240

gtgctcagaa ttatgcttgg ttcttagtac atgctatttc catgttagtt attactttaa     300

ggagctattt cttatggttt cttcttctgt acaataagga gggtgggcag tgtcagtgat     360

tctctactat gggagaacac tagggtcatc tggggagctt aaaagctcag gattttagaa     420

agattttgat gtctggaccc ctctctaccc accttgaaac agaatctctg ggagtggggc     480

ctggacattg gaattttaaa aaagccttcc tgtggttctg gggcacagtg aggctgagaa     540

gcaggggaag agatggtcct gagatccctt cagcttggaa gttggcatgc tgggtttgta     600

tggcaggggc gggttatagc ttcccttccc atactcagtg catttatgtg tgtcactaaa     660

cattcttttc ccttttaaaa aaacggaggt gaaagtcctg taacataaag ttaagcattc     720

taaagcgtac aaatcagtga catgtacatt cacagtgttg tgcagctacc acctcctagt     780

tcctaaacat ttcatcaccc caaaataaaa cctggtaccc agtaagcagt cagcctcatc     840

ctcccttccc tcagctcctg gcaaccacca atttgctttc tgtgtctatg gcattacctg     900

ttctggatac ttcatataaa cgggatcgta caatatgtgg gctttcatgt ctggcttctt     960

tcacttagcg tgctgttttc aaggttcatc cacgttgtag tatggaccag gggttcattc    1020

ctttctatgg ctgagttgta ctctactatg tggacgtatc atattctgtt tacccatttg    1080

tctgttgatg gacggttggg ttgttcccac cttaactatt gtgaatagag cagctatgaa    1140

cacttgtgta caagtatttg aacacctgtt ctcaattctt ctgggtatgt acctacgagt    1200

ggaatcacag ggagtggaaa tgcaaggata attctgtatt gaattttacc cccaaattgt    1260

ttacgtgtga ttaaacgttc gcctctgttc ttcaccctca cagatcttcc ttgacttgtc    1320

cccactgcct gaaacagagc aacacctttg atcctttcct gtgtgtgtcc ctacctatcc    1380

ccttgcgcca gacgaggtac gtgagtgtcg cggcttgccc ggtgaacttg acttccatcc    1440

ttactcatta cggggaagct tctggcctca cacctgccat tttctctcct ttgcttctca    1500

gccagcattt ctctggaaac cagccttata gctggatgac attcaactat tgaaattgac    1560

agagtgaaag gttactgtgt tccttctcta gctgaggaag gaattatgaa tgtttggcac    1620

aattgccaga cttagatctg tggcccattc agaagctggc acggacccat tggtctcact    1680

tagtgagaca ggggcaaact agagaaaatc cgtggccacc tgcttttttt tccccagctt    1740

tacttcaact tcaaaaatct attttcttct ttgagaaggt gcaggaaaaa gatttcttta    1800

aggagcaaag aaagaggaaa ggggttgttt ggggaagatg agaaaaaaaa agaaaagtgg    1860

aggtagggct gggtaacagg caccatgggc cgagttctat aaaaataagc ccaaatctta 1920
gcagtgacca ggaaggcaaa tggccaatgt cagtgtcctg gcatgtgggt gagctcctat 1980
gtggggtgtc ttctctggca ttggaagaga ggtatttcct ctttatgttc cccacctcct 2040
taagtgccat ttctcttacc ctcttcctat tgacatcccc tgcctttagg atgcagctga 2100
agacataaca tcttcagagg gccttgcttg gcaactcaat ctccctttct gctctaaaat 2160
tgtgtgctgc tgatcttctg cactacaaat ataactactg gcttatgcta taacatggct 2220
tatgctgtct atcatcagtg acaccatcag ataatgtcaa cttatagatg tgatgatcta 2280
agactcccag cctgacacag cacagcagag tggtcaagac cactgactct ggagccagac 2340
ggcccaggtt cgaatcccag ctctattaat taattacctg tgtgacttgg gcagttattg 2400
gagtacagat caagagaaac agtagctgca agagtgggcc ttcatatgcc ggtgggaagg 2460
ggaagcagag atttccaaag gagactctca ttaaggagaa tgtcatcagt agtgttgatt 2520
tgtactgaaa aaaacacctt gactgcttta attttcaaac tttggagttt gtttttgttt 2580
aagcttatct atttaacaac tagagccttg agatcggaca tctctatggt gattcttaat 2640
caaatacttt ttcccccaat tctctggaaa agtaatttag aagatttggc tctaccctgc 2700
cacctggtga caatgtacca agtacaggga cacaaccagg aagcggagat taactatgtg 2760
ctgaacttga tccattatct ttctttcttt ctttcttttt gagatggagt cttgctgtgt 2820
cacccaggct ggagtgcagt ggtgccatct ctgctcacta caagctccgc ctctcaggtt 2880
caagcgattc tcctgcctca gcctcccaag tagctgggat tacaggcatc cgccaccaca 2940
ctcagctaat ttttttgtac ttttagtaga gatggggttt caccatgctg gccaggctag 3000
tctcgaactc ttgaccgcag gtgatccacc tgcttcagcc tcccaaaatg ctaggattac 3060
aggtgtgagc cactgcgcct ggccttgatc cattatcaac aatgtcattc acagttcatg 3120
tcagtccttt aagggtgttg ccccttccta ttttccaggt tcttgagtgt caccttggtc 3180
ttcccctcta agagccagcg gttcctgcgg gttggcctgg ccgtgccgat cctcagcaca 3240
gtggcagccc tgaggaagat ggttgcagag gaaggaggcg tccctgcaga tgaggtgtga 3300
tagaattgca ctggggcctg gtcattggat gatagaatgc taactgagcc agctgtctag 3360
gctgtttagg ccaatttgtg gttcccaggt gactccagta tgactcatgg gtttctggtt 3420
gattagtaat gcctgctgtc tgggatgact gggccttgac ttttccctgc attgacacag 3480
aatgaccaga aaagaacacc tttgccttcc gagaaggcaa tgacaattat gatcataata 3540
attccagcaa tagctacccc taggtagtgc ttactaagtg tggggcacta ttctgtgaac 3600
tttctattca tgcctgattt aatcacaggc gtcctgtgaa gtagacatta caattatcct 3660
catcttacta atggggaata aagaagccct gagaggttaa gtgacttgcc caacgtcact 3720
agctagtaag tggctgagcc aggatttgga tttcgatggt ttgacttcat agcccaagtc 3780
accattgtat attttcagtc ttggcaatat aaaccaggac agtaaaggaa cagtaaatga 3840
agcaggagga cctgctgaga gaactgagga tgtataacat gagagagaac atctggcgga 3900
agctgggatg gctgtgttct acctgttttc ttttcaaacg agtcttagta aagcgattgg 3960

attcacagtg cctggagggg ccctcatgag gaatagggag gaggccgtgg tggcgccaag 4020
ccacatggca ggatgggagc ggccctcagg gtgattcagt agctccctag gtttctgacc 4080
ctggacacag tatgtgattg gctctgtgtc cttaactgtg acatggaaat tgacacttcc 4140
ctgactatct catgggattt tttcaaatca aaaaatattt ttaaccttgt tgggaaggca 4200
tgccacacat acaataacgt ctacctatct gaagattgtg gtttgctggc tttctctacg 4260
ttcatagcta cattcctata acctccacgc ttcctgagcc tccccatcac cccagtgggc 4320
tcctttctgc ccctgcccaa tcaatccctc tgacctctat cgctacctca tagaatattt 4380
atgactctct caaatagcag gggaaatgtg tgtatcttgc tttgtaaact ttaaagtgca 4440
ctatggggag ttactgaaag agacggagag agagggggac agtgggtgag agtcctgggg 4500
agaaagtgac agtaagaaaa gatcaaaagg gagaagtcta tgagagggag cgtttgaata 4560
attaagagtg tgaaaaatct ggcaggaatt tgagaggtgt gatgtgttgg ttcagggaga 4620
tgggagtcag ttggggatgc cagtgtggga cgggcatttc cactgaaact gaaagtcatc 4680
tattttgatg ctttgttttc tttaactggc aagtgtatta tatttatgtt atataaaatc 4740
atagatatat gtacttggat ttatttttct attttattta tttatttttt gacggagtct 4800
ccctctgtcg ccaggctgga gtgcagtggc acgatctcgg ctcactgcaa cctccgcctc 4860
ccaggttcaa gcgattctcc tgcctcagcc tcctgagtat gcatcaccac acccagctaa 4920
ttttttgtat ttttagtaga gacggggttt caccatgttg gccaggatga tctcgatctc 4980
ttgaccttgt gatctgcccg cctcaacctc ccaaagtgtt gggattacag gcgtgagcca 5040
ctgcgcccgg cctgtatttg gatttattta tgtcatctta tttatttatg tcatcttatg 5100
ctatttaccc tgttttccta ttttgtaccg cccctccccc cccattttta gattgaacag 5160
tctttttctt ttagaagatg attcttttac attgagcaat ttttctttat ttcatctttt 5220
ctgggatgaa gtacatccag aagatgtact tcatgtacat gtcaatatgt atatgtaaat 5280
ataatatata cattatattt ccattctttt agtggttact aaaatgtcaa cttgcataat 5340
tacgagtcta aagccaatct tcccatgtaa cacaaggatc ttggtatgca ttaaatcccc 5400
ttataccttt ttgatttaga tattattgtc cagtatttta atcccttctc cacctccaac 5460
tgtaatagta ataatagtta ttattttagc tttatatagt tgataatatt tacctttgcc 5520
aatgtttacc attttctttg gttcatcatt tcttcctaca actcaagttt tctctttgat 5580
tcaggttttt aaatcctgaa atctatgtat ttatgaatga atgaatgaat gaatgacagg 5640
gttttcctct gttgcccagg ctggagtgca ggggtgtgat catagcttgc tgcagccttg 5700
aactcctggg ctcaagggat cctcccacct cagcctcctg agtagctggg actacaggct 5760
catgccacca tgcctggcta attttttaaa aaaattgtgt agagatgtgg tcttgctgta 5820
ttgttcaggc tggtcttgaa ctcctgggct caagtgaccc tccctcctca gcctcccaaa 5880
gtgttgggat tataggcatg agccacggtg cccagccctg aaatgcatct tttttttttt 5940
tttttttttt tttttaatga cggagtctcg ctctgtcgcc caggctggag tgcagtggca 6000

cgatctcggc tcactgcaag ctccgcctcc cgggttcaca ccattctcct gcctcagcct 6060
ccgtagtagc tgggactaca ggcacccaaa atgcatcttt ttaacgagaa cttcccaggc 6120
agtgtacctt gaataggtta tgtgtgctga gatattgatt tcttcagcat cagggtaaca 6180
gagttgggtc tagggcagtc agggccccca cagtagtcac ccctctactc tgctacaaaa 6240
ccatctccta tttacttcaa tgtttggtaa aaattttttt tatgtatgca atcacgtgaa 6300
aaaggttagt aaacctattc agaaattctt tcactctgta gtaaacactc actttttgtt 6360
cacatgaaga gggctttatt ttgttgtcat ttttctatgc ttgtttagct gggtataaaa 6420
cttagatttt tttttttttt ttcagtttct ttttttgttg cacttgagat gtctgaggac 6480
aatctaatgg ttgtatctac tatgctgcct tattactaaa aataagtagc caattggaag 6540
gaaaaggtga aaaatgggtg ttgactatca gtcactttta gatttcttat ctgttgattt 6600
ctctttcaga aatcaagagg gaaaatgatc tttctcattt caggtgatct tggttgaact 6660
gtatcccagt ggattccagc ggtctttctt tgatgaagag gacctgaata ccatcgcaga 6720
gggagataat gtgtatgcct ttcaagttcc tccctcaccc agccagggga ctctctcagg 6780
tataacaggt gctttgcaca attttatttg gctatgatga gttacaggtt tcagaggata 6840
ctccttgggt gcaaaggcat aaaacatcca attttagcac ttggaacagt cagacttatt 6900
atccctggca ttcgttgtgt gcagatggtt gatattaaaa attattcacc taacacaagt 6960
attctcaaaa ctaagcttag tgaatattag aatcacctgg agggcgtgtg aaagcagatt 7020
tctggaccct acccacagag tttatgattt gggtggcctg gggtggggcc taagagtttg 7080
cacatctaac caagttccca ggtgatgccg atgctgctgg tccaggaacc acactttgag 7140
aaccactatt tggtatagat gctggagtca tctttgccgt caggattcct atgggtcaag 7200
atttgtgtgt gaagtaaaat gtctacaaat ttgagcagta cgtggttaaa gttttattaa 7260
atgacttttt aacaaaatag ttccacttct taatgcttgc ttttcctatt ccatggactg 7320
gctaacatgg tcaatttcta acagtccttt ttatttgtag atcaggatac attttggcca 7380
agttagtgaa gaagacccat atatatatta tatattatat gtaatatata aaatatatgt 7440
tatctattac atataatata taatatatat aaaatgtata ttatatatca tatataatat 7500
aatatatgac atatactata taatatatac tatataatat atactatata atatatacta 7560
tataatatat actatataat atatactata taatatacta tataatatat actatataat 7620
atatacatta tatagaatat attatataat atatatttaa tatgtattta atatatttga 7680
tatatattta ataattatat atgttatata taatatagat aaatatatat aaatatatat 7740
ataaataaat atatatataa ataaaataaa taaaataaat aaatatatat aaataaatat 7800
aatatagata aatatgtaat atattatata gctaaatatc tatattatat atagataaat 7860
atatataaaa tatattatat atatatatat atatatatat atatatatat atatatatat 7920
atttgagatg gtttctcact ctgtcaccca ggctggagtg cagtggcgtg atcttggctc 7980
actgcaacct ccacctcctg ggttcaagtg attctcctgc ctcagcctcc caagtagctg 8040
ggactacagg tgtctgccac cacgcttggc tagtttttgt atttttagta gagatggggt 8100

ttcaccatgt tggccaggct ggtctcgaac tcctaacctc aggtgatcca cctgcctcag 8160
cctccgaaag tcctgggatt acaggtgtga gccaccacac ccggccagaa ggcccatatt 8220
taaaggaccc tgtgccagtg ttactttata ccccagatga ttcctacgcc ctggccccac 8280
attttggcca gcctaggtag caggctatgg gaactggatg tttgaagttc tctgagttga 8340
cagggagatg ctgggcattg gaatctagaa caagacatta actgacactg actatattac 8400
aacccagtga aacaagttct cctttcaaat tcaatcatgg tggacacatc ttttccttga 8460
aagtcaacat ttcagaactg attgcaaatg gaggctagtc caattcctct acttcctcac 8520
tggtaaagat tctcacccat ctctatatac ttcttatgtt ggctttctaa gagagtagat 8580
aaactgggga ctctgtaatg aagtcataaa agcaaccaaa attattttgg agtatttttc 8640
aggtgacttg aaatatatta gttaaattct tgttcctgtt ttaaaactat agggtgatgg 8700
ccaggtgcag tggctcacgc ctgaaatccc aacactttgg gaggccaagg caggaggatc 8760
acttgaggtc aggagttcaa gaccagcttg gccaacatgg tgaaacccca tctctactaa 8820
gaatacaaaa aaattagctg ggcgtggtgg tgcacatcca caatcccagc tactggagag 8880
gctgagacag gagaatcatt tgaacctgga aggtagagtt tgcagtgagc caagattgtg 8940
ccactgtact ccatccagcc tgggggacag agcgagactc tgtcttaaac aaacaaaaaa 9000
ctattgggtg aacacaattg caaattcact accatattct tgatgttcgt atgccaggcg 9060
tcagatggca caaatgccat gaacctcatt atctcctccc ctaaagccct ctagtggccc 9120
cccattggtg gttaatagat gctcaaagag aaagagggac tagagaagct aaggctctga 9180
cccaggaaag cagctccttt aggaatatga acaaatgtca ttctctccct tctagctcat 9240
ccactgggtc tgtcggcctc cccacgcctg gcagcccgtg agggccagcg attctccctc 9300
tctctccaca gtgagagcaa ggtgctaatc ctcttctgta acttggtggg gtcagggcag 9360
caggctagca ggtatgtttc actttattct tttttcatgt ggtgtctggg atttgtagac 9420
ctgtacttgg gagcctgtct agagtaaaat taaacattta ttcccaaggt taataaacta 9480
ggagccagaa gtagggtcct ttctgattaa gaggattaga aatatctgcc ttaagcattt 9540
ggctaacgtc atcattccta accatcagac atcagaggcc ttcctattaa tcggaggaac 9600
aatttttact gccttcacca ctgttatttt atattgtaaa acctaggcgg tgcagtgcta 9660
aaagaaactg gctataagaa aagaggtgat aatttggttg tctgtccaga aaaccaagaa 9720
ctggaaagtg attggcagta aggaaagggt tcaggaaatt gatgggttat ataacaaata 9780
tacaaaaaca gatagctttc ttatatagcc caaataaagt gtttaaaaga cagaattaca 9840
ttaagatata tttagatgga taaaatgttt ctaaaaatgt atcttgaata ataaactggt 9900
aaaaacaaaa agattattta ataataatta aactattatt aattagctca gcttttaaaa 9960
atcaattagc ccagccttac ttattgaata attcattaac tcttagtaat atgaaataca 10020
tcatttttat catgtaataa atacttacag atagttggtc tattttgttt gatttaattg 10080
catattcttc tgcctgtatt acaatatttt aataatttca gaattatggt atatttcata 10140

ttactacaga gtgagtgaat tgttcaaaag taaggctggg ctaattgatt tctttgttgc 10200
tttttatttt aaatagtcaa ctttttactc ctgctatgaa ttcacacgta taaatttcag 10260
gttgactaga gaggtaacta gagaaaagga agccatgaaa taacttaaag atggccgagg 10320
tgggtggatc atgaggtcag gagttcgaga ccatcctggc taacacggtg aaaccccatc 10380
tctactaaaa gtacaaaaaa ctatccaggt gtggtggcac gcacctgtag ttccagctac 10440
ttgggaggct atggcaggag aatcgcttga acccaggagg caaatgttgc agtcggccga 10500
gattgcacca ctgcactcca gcctgggcga cagatcgaga ctgtctcaaa aaaaaattaa 10560
taaataaaaa ataaaaaata aattaataaa gacaacatgg gagaatattt atgtccttgg 10620
gtgggggcta gtttaacatc aaagtcagga ggctgtaaag gaaaaggctg atgttttaaa 10680
tatatttaaa aatttaaact aaaagcaaat ttgaaaggca aaggacaaat gaggaaagat 10740
atttatgcca tgtgagaggt gaaggattta tgtccatcct cgaattttcc agctcttgaa 10800
aattaaggag ataaataccc ttggtgaaat gaaccacaga gatgaacatt agttcacaga 10860
agaaatacaa gcataggtta aaatattcag catcagcttg gcacggtggc tcacgcctgt 10920
aatcccagca ctttgagagg ctgaggcggg cggatcacct gaggtcagga gtttgagacc 10980
agcctgacca atatggagaa accccatccc tactaaaaat acaaaagtag ccgggcatgg 11040
tggctcacgc ctgtaatccc aactactcgg gaggctgagg caggagaatt gcttgaaccc 11100
gggaggcgga ggttgcggtg agccgagatt gtgccactgc actccagcct gggcaacaag 11160
agtgaaactc catctcaaaa aaaaaaaaaa aaaaaaaaat cagcatcatt tgtagtggga 11220
gaaattctgg ttgcaatagc aagatactat tcttaacctc ttagattgtt aaatgatttt 11280
tagaaattga taatactcat tctgggccag agtccgggga atcaggtaaa ttggtatggc 11340
ttctttggag gacagtttgg gaagaaagag ctttagtctt gtctttctac ttctggaact 11400
ttaccctaag ggaataattg tgcgctcaaa ggttatgtcc aaggttatat ctagttcctg 11460
cgataagcaa gcattgttta aataaaaact tggaatgcct cacagtacag gatggattaa 11520
gtaaaccgat ctgtccatac aacaatggac gcacagtgcc cttgcctgtc cagacagatc 11580
acctcatatt tgtgttctgc tttagccaat tttattcaac aatttcacat tcttacctca 11640
ttgggtcttc acacaggctg cagcgaggcc aggaagcagt gagtcgctag tgatttgtcc 11700
aggagtaccc acggcagagt tagggcccat aggcgcaggc tcacccagca ttctgtacac 11760
catgagctca ctgctgtgcg cttactatgt tgggacatgg ataccatccc agtgatgaga 11820
atattctcga taccccaacc aaagttgtgt catctggatt tatgtctctg ccttgccttc 11880
ccctggctcc aagcttgtct tactctattc cctgctgtga gtaagagatt ttgccagcta 11940
gctggtcatt ttgagtaatc tgtttacaca gcagcatcta cctggctttg tgtcactggg 12000
cctctgctta cattgcagtg tctccatttg ttacaaggtt ctctgattgg caattttgta 12060
ttgtgtttgg ctttaaaaag catttacatt atttttaatc gacacataat tgtacatatt 12120
tatggggtac ggagtgatat tttgatacat gtacatagtg tgtaatgatc aaatcagggt 12180
aattggcata accgtcacct caaacattta tcatttcttt gtgttgggaa gattcaaaat 12240

cctatcctct agctattgaa agcatacaat aaattattgt taacaatagt cactctatat 12300
tgctatagaa cactagagct tatttctcct atctagctat aatttttatt cattacctaa 12360
ccttttccta gcccccactg gcgcctccca gcctctagta gaaccactat tctactctac 12420
ttctgtgaca ttaacttttt tagattccac atgagggagt acatgcagta tttatctttc 12480
tgtgcctgac ttatttcact taacataatg tcctccagcc tcatccatct tgccgcaaac 12540
aacaggattt catttctttt ttatgggtgc atagtattcc gttgtgtata tagaccacat 12600
tttctttttc atttcttttc cattcatcca ccgatgtaca cttaggttga tttggaatct 12660
tggctattgt gaatagccct gcagtaaaca caggagtgca gagatctctt tgacatgatg 12720
atctttcctt tggataaata cccagtagtg ggatggctgg gtcacatggt ggttctattt 12780
ttagtttttt gaagaacctc catgcttttc tccacggtgt ctccattccc accaacagtg 12840
tataagagtt cccttttctc catatcctca ccagcatttg ttatttcttg tctttttgat 12900
aatagccatc ctaactgggg taagatgata tctcattggg gttttgactt gcatttccct 12960
gataattagt gatgttgggc attttttcat atgcctgttg gctacgtgtg tcattttgag 13020
aaatgtctat tcagaccctt tgcccatttt taaatgggat tctttggttt tttttgctgt 13080
tgagtttttg tgcttagctc ttgtcactta tcctattgac aggaagccag ggttagaaca 13140
accactcatg actggtggat gttgctggtt tttcctttgc tgggataacc cgatttcctt 13200
ggattttcag gtttgggcca cccttcctga taagggaaga cagagctgtt tcctgggccc 13260
agctccagca gtctatcctc agcaaggtcc gccatcttat gaagagtgag gcccctgtac 13320
aggtcagtgg tgtgcatgcg tgtgtgtgtg tgtgcgtgca tgcgcatgtg catgcgtgtg 13380
tgtgggtgtg tgtattggga ggggtggaat ttagtgtagc ccaggagagg ataatttgct 13440
gttaaagagt gttcatactg ttttataaaa tgacttagat ttatttgcaa ggaagtaacc 13500
atcttatagt ctgtagtgtt ttacttttgt gccattacaa aatacctttc atgggcagag 13560
ctttttctta ttaaatcttt caaaacacca ttgggaaaat ggcatttaca ttaaaacgaa 13620
attttttttt tcacattttc catcattccc taagcattta cattattatt cccaattttg 13680
tatgtttatg gcttaccata gtttttccta gaagatagca tattataata aattgtaact 13740
tttatgttta aatcaacaat ttcaagggac ttgagataga atttctggga aaattaaaat 13800
ggatgattat gtccaaatat gaaaatctaa atgttaaaat aagtttgtcc agttttttaa 13860
aaactaagat aactcaaagg aaaacatttg ccttatttat ttattctcag attggtagtt 13920
tccaaacaaa aacagcatgt ttaaaaacta ttgatttttt gtttttagct cctataatat 13980
cagaaactat tctgatggtt gcttgacaca gaagatgttg aaatttttag ggacaggtca 14040
gttgtgtcag attcctattg tcccctaact attcccatag taaaagcttt ttatttgggg 14100
cacacacaga tcttatctca gagataattc ctcataattc atgttccttc ctgctcagca 14160
aaaaccatat gtttgattta ggatttatac caaataacat tttgttaaaa accttcacta 14220
catttgtccc aacttttatc tctaaaggat tttgttcaat gtactgtaga tcagatccac 14280

caaagtgccc aaattagaaa ctcctctgca agtaggtcag tctatccggt gagagttttg 14340
ccttctccca aaccttgagc tactgattat cgcaatggat gaaactatgc tttttttgtt 14400
tgtttgtttg ttttttgaga cggagtctca ctctgtcgcc caggctggag tgcagtggcg 14460
tgatctcggc tcactgcaag ctccgcctcc cgggttcacg ccattcttct gcctcagcct 14520
cccaagtagc tgggactaca ggcgcccgcc accacgccca gctaactttt ttgtattttt 14580
tagtagagac ggggtttcac tgtgttagcc aggatggtct cgatctcttg acctcgtgat 14640
ccactggcct cggcctccca gagtgctggg attacaggtg tgagccaccg tgcccggcca 14700
gatgaaacta atttcatgtg agcatttaac tcagccatcc ttactcttac ctgctcacac 14760
tcctgtcctg tttttttttt ttgagatgga gtctcgctct gtcaccaggc tggagtgcag 14820
tggcgtgatc tcggcttact gcaacctccg cctcccaggt tcaagcgatt ctcctgcctc 14880
agcctcccaa gtagctagga ctacaggcaa gcgccaccac acccagctaa tttttgtata 14940
tttagtagac acggggtttc accatgttgg ccaggatggt ctcaatctct tggcctcatg 15000
atcctcccac ttcaacctcc caaagtgctg ggattacagg cgtgagccac cacacccagc 15060
cacacctgta cttttgcctc catacctttg cagtcatttt agctttattt tatttttgag 15120
gcagggtctc actctgacac ccaggctgga gtgccgtggt gcggtaatgg ctcactgcat 15180
ctttgacctc ccaggctcaa gtgatcctcc tgcctccgcc tcctgagtag ctgggactgc 15240
aggtgtatgg taccgtaccc agctaacttt tttttgtttg tttgttagag acagtttcac 15300
cattttgcct agagtgatct tgaactcccg ggctcagttg atccatctgc ctcagcctcc 15360
caaagtgttg ggattacagg tgtgagccac tgtgcccagc ccaatttcaa ttttagaaat 15420
ggatcaagca atcaatgggg ccagcaaaat actaatacgg gcaacctttg actcagccac 15480
agatgctttt tcacagattc acgggtgaat gtatggtcag acatgctttt ctgtaggagt 15540
aatgtgttct agatggttgt gaggcttcca ggctagccaa cccatttaac ccataattct 15600
tttaatacta tattaaatac tggttcaaat atagtcattt gaagagaata tattcaggag 15660
tctcaacatt gaactgaaga gattaaacat attgtattga aggtttggac atgaacttcc 15720
ctagccaaga attgagaaca ggaaagtttc ttacaaaaat gaccagttta ctggatagga 15780
ttaatttgca tgtattatga aaaaaataaa agctatcaat gccaattttt ttttttttta 15840
aagagataga gtcccactct gttggccagg ctggagtgtg gtggtgtgat catagctcac 15900
tgcagccttg aactcctggg ctcaagtgat cctcccacct catccttgca agtagctggg 15960
actacaagca cctgccatca tgcctggtta attattttaa tttttttgca gtgatagggt 16020
ctcactgtgt tgcccaggtt ggtcttgaac tcctggcctc aagaagtcct cccacattgg 16080
cctcccgaag tgctgggatt acaggcatga gccaccatgc ctggctaccc aaacactttt 16140
atggtatttt gtaaacgggg gtgtgtgagg ccagcactat ttgtgtgcag gcagagtagg 16200
ctgcctctgt gcagatgttg gtgccgtaat gatggctgac ttttgctgtc caaacaacaa 16260
ggctccatta ggggcggagg aaaaagagtc ttctccccca gatttaaatt aacattctcc 16320
ttagggagaa tacccttccg tggccaaccc tcaatcaaag cccctgaagt catcttactt 16380

ccagacgttg atttcgtctt tgttgaagcc gtagacgaac tggaacgtgg ttagagtctg 16440
gctagacgta tgagctccct gcccttcaca ctcaagaccc cctgcagccc agttccagtt 16500
tacccaccag ccttgtggcc gatgctccct gcacacgtca gctcccacag ccaaaggggt 16560
cggctcacaa ctctctgcat ctgtttcggg tcattctgtc ctaccatgtg tcattcctct 16620
ttcctggaat tatgatgctt ttctcttgtt ttcctctcat aattcaacct aggtttcctc 16680
catggttttt aagaaaacat gtctgccttt tacctttgtc agagtttgga agaaaatttt 16740
attttatttc tggttgtcaa gatcatcagt gacaaattta agcacctatt tcttttattt 16800
gaatgaagac cccgttgatt ttttaaaaaa ttttggtaaa agatatatac atttatttat 16860
tttaaccact cataagtgta tattaattac atttacaatg ttgtatcacc atcactacta 16920
tgtatactcc cgattttttc atcatcaaca aaaactttat taaacattat actgattaaa 16980
acaaaaacct tatacctatt aaaaaactaa aatgttgcca gacgtggtgg ctcacgcctg 17040
tactcccagc actttgggag gccgaggcag gtggatcacc tgaggtcagg agctcaagac 17100
caacctggcc aacatggtga aaccccatct ttactaaaaa tacaaaaatt agccaggtgt 17160
ggtggcaggt gcctgtaatc ccagctactc gggaggctga ggcaagggaa tcacttgaac 17220
tggagaggag gagtttgtag tgagccgaga tcgcaccact gcattccagc ctgggtgaca 17280
gagcaagact gtctcaaaca aacaaacaaa ctatacctat taaacaacaa ttccacgttt 17340
tctcctctgc ctagctcctt gtagctacca ttctactttc tatctgtatg aattttcctg 17400
ttttaggtac cttatataac tagaataata taatatttat ctttttacat ctggcttatt 17460
tcactaaatg tcatgttttc aagggtcatc tgtgttgtag catatctcag aatttccttc 17520
attttttttt ttttcttttt ttttgagatg gagcttcact cttgttgctc aggctggagt 17580
gcaatggcgt gatctcagct cactgcaacc tccacctccc agattcaact gattctcctg 17640
cctcagcctc ctgagtagct gggattacag gcacccgcca ccacacctgg ctattttttt 17700
ttgtattttt agtagagatg tagcttctcc atgttggtca ggctgatctc gaactcctga 17760
cctcaggtga tccatctgcc tcagcctccc agagtgctgg gattacaggc gtgagccacc 17820
gcgctgggct gccttcattt ttaaggctaa ataatattct gttgtgtata tatcccacat 17880
tttgtttatc cattgttggt ggacactcag gttgtttctt tcttttgact tgtgaatcat 17940
gctgccgtga acattggtgt ataaatatct gttcaagccc ctccttgcga ctcttagata 18000
tacacctaag agaattaaag gttatatggt agttcaacat ttaacctttt gagaaactgc 18060
tgaactgttt tctacagcag ctgtaccatc cattttatgt tcccaccagc aatgcataag 18120
ggttccagtt tctccaaatt cttgccaatg cttgttattt tccattaaaa aaaaaattag 18180
gccaggtgcg gtggctcatg cctgtaatcc cagcactttg ggaggccaag gcggatgagg 18240
tcaggagatc gagaccatcc tggctaacat ggtgaaaccc cgtctttact aaaaatacaa 18300
aaaaaattag ccgggtgtgg tggcgggcgc ccgtagtccc agctactcag gaggccgagg 18360
cagagaatgg caagaacccg ggaggcagag cttgcagtga gctgagatca cacaactgca 18420

ctccagcctg ggcgacagag cgagactcca tctcgaaaaa aaaagaaaat taaaacaaat 18480
ttaattgtag gctggacgtg gtggctcatg cctgtaatcc cagcattttg ggaggccgag 18540
gtgggcggat cgcgtgaggt caggagttca agagcagcct ggccaacatg gtgaaacccc 18600
atctctacta aaaatacaaa aattagccag gtgtggtggc aggcacctgt aattccagct 18660
actcaggagg ctgaggcagg agaatcacct gaactcagga ggcagaggtt gtagtgagct 18720
gagattgtgg cactgcactc cagcctgggc gacagagtga gactttgtcg tctcaaaaaa 18780
aaatttaatt atagccatcc cagtgggtgt gaagtggtac ctcagcgttg tcttgatttg 18840
catttctcta atgactaatg atgttgctga tcttttcatg ggcttcttgg ccatctgtac 18900
atctttggag aaatatctgc acaaatgttt gcccattttt taattgagtt gttcattcca 18960
ttgaattttt aagaggcatc ttccgttaaa tatgtcattg taagcctcat ttcaatttca 19020
gaaaagttaa aatataataa acacatatct tagaataata actaaggtgc tatctcttta 19080
tgtggcttat tttaaaatgt ataaatattt actttgtctg taaaatatac atgaactggc 19140
ttttagaaaa ttgcctcatt gtggggatga taatcatctt aataaagtag gttaagttac 19200
ctattaaaaa ttaattaaca tgattgattt atgttccatt acatgtacca gtatctgctc 19260
cagttaagca tttactattt aaacacactg tgttcatttt tggggggaag atgtaacata 19320
ttaaacacag tattagatca ctgcattaca gggtatagtg tgaatctgaa aaaaatgaag 19380
tttttgtctc ttaattttga attctaatta ttacgggaat attcaaaata cactatggaa 19440
aataatgatt atatttttta atgtatgcgc ccctttagag tctaatttaa atcaatatag 19500
gggctacgta atgatccatg tctgtttttg tcttcgcaga acctggggtc tctgttctcc 19560
atccgtgttg tgggactctc tgtggcctgc agctatttgt ctccgaagga cagtcggccc 19620
ctctgtcact gggcagttga caggtaaggg ggaaggtcca ggttcagtca gctaatgaac 19680
cctttcttat tttaccagag tcctttgagg gtatatgtca atgagattgc tcatagtatt 19740
tgttattctt gatcacttac ctctccagta ccagccgcta tgttaggtca ttgggcatgt 19800
tctgtgcttt gaacccgtat gtctggagag gcactattat gtccgcttca aagatggggg 19860
ggacaccaac gttctcagag gttagttaac ttgatcacac ttaaaaagct agttaatagg 19920
caagcctaaa tttgaaccca gattgatcta actaaaaccc aaattcagcc aggcatggtg 19980
gttcacgcct gtaatcccag cacttttgga ggctgaggtg ggtggattgc ctggggtcag 20040
gattttgaga ccagtctggc caacatggtg aaaccccatc tctactaaaa atacaaaaaa 20100
attagccggt cgtggtagca cgcgcctata atcctagcta ctcaggaggc tgaggcaggg 20160
gaattgcttg aaccagggag atggaggttg ccgtgagccg agatcgcgcc actgcactct 20220
agcctgggtg acagagcaag actccatctc aaaaaacaaa aacaaaaaca aaccaaaaca 20280
cgaaattctt tcaattaggt catgctcctg aattcaacag gaatatctcc ctagggtatg 20340
tgaagattct acagctgatt cattggtcct aaactgtaag agattgtgga gtgtgcctga 20400
ggcttaaagc cagtgtggct gtgtctctgg tagctacctt gatagccttc tggtgcgtgt 20460
gcccttgcaa agcaatcccc aggtcattgc aacatctatg gggcagttct actctataaa 20520

cgaactgtct ggcacttctc tttattttcc catagacccc caggttctca gtaaggtcct 20580
ctgatttcct gacttggtca gccaaattca acctgactga gatgggcctt tccccttgta 20640
aagagaccag gaaccagggt agcaggtgag ccagaaggtt ctgtgaccta caccccatcc 20700
atatcctcct tggctggcag atgcttttgc tttacagagt taggaaggca ggagaagggt 20760
catcaagggc ctgtttgggt ctcccaggga gaggaagccc cttcccacac tcagtcttgg 20820
agataaccca aagtggttct gtagactgcc agcctgtgag ctaattgtca ccagcccgtg 20880
acaagaactt gagaggaagc acttacgaat gttaaagcaa tatgacagag caatctataa 20940
tatgatggat atgataattt cttttaaaaa tggaactttt attttatatg tctttcctca 21000
ccccccatta cttttttatt atttattatt tatttattta tttattcatt tttgagatgg 21060
agtctcgctt tgtcttccag gctcaagtgc agtggcgcca tctcggctcc ctgcaccctc 21120
cgcctttctg ggttcgagcg attttcctgc ctcagcctcc ccagtagctg ggattatagg 21180
cacacgccat cacgcctggc taatttttgt atttttagta tagacagggt ttcatcatgt 21240
tggtcaggct ggtctcgaac tcctgacctc aagtgatcca cctgcctctg cctcccaaag 21300
tggtgggatt acaggtgtga gccaccgcac ccggcctatt atttcttttt ataaaagtgt 21360
tggtctgcag tggtttggaa acaaaccatc aaactggtct tttaccatag gtagtttgag 21420
aagcgctaga attgagtaga gttctacagt aagctgacca tcctaaagga tattctcttg 21480
aggtcacacc cctcctggcc cgcaccttcc attcactgga catcttacct cagtctggcc 21540
cctgttcacc ccactcccat ccccacccca ccccacaatc ccagtcccag ccctgctggt 21600
acagctctct ctggcatcta atgtgacctt acctctttca cagggctctc tatttagccc 21660
ttgccctgcc tcttccatgc ctatcccaac atctccttgt tctataggaa attctttttt 21720
ttctttttgg taaaatacat agaacataaa attacttttt ttttgagatg gagtctcact 21780
ctgttaccta ggctggagtg caatggtgta atcttggctc actgcaacct ccgcctcctg 21840
ggttcaagca attctcctgc ctcagccttc caagtatatg gtactacagg tgcgtgccac 21900
caggcccagc taattttttg tatttttagt agagacaggg tttcaccatg gtagccagga 21960
tgggcttgat ctcctgagct cgtgatccac ccacctcagc ctcccaaagt gctgggatta 22020
caggcatgag ccaccactta gggccaaaat tacattttaa ccacttgaaa gtgtacagtt 22080
cagtgggatt gttacattta ccttgttata catccattgc caccatccat ctccaaaaat 22140
ctttcatctt gcaaaatgga aactctgtac ccatcacaca atatccctcc ccttcctctt 22200
cctcagtctc tggaaaccac cactccactg tctgtcactg tgaatttgac aactgtagat 22260
gcctcaggta acaggaatca tagtgtttat ccttttgtgt ttggcttaat tcactaacca 22320
ccatgttttg aaggttcacc catgttgtag cacgcattag aatttccttc cttttgaagg 22380
ctgaataata ttccattgta tgtatacacc acctagaaat atatctccta gaattttttt 22440
tgtctgcttg aaatcttcct ctccctctct gggatgctca aatattattg ggtctcagca 22500
ttctacccct ggccctctgg gttccattta tgggtgtttt ttatgttttg ttttgtttgt 22560

tttttagaca gagtctcact ctgtcgccca ggctggagtg cagtggcaca atctcggctc 22620
actgcaaccc ccgcctcctg gattcaagaa attctcctgc ctcagcctcc cgagcagctg 22680
ggactacagg tgcacactgc cacccctggc taatttcttg tattttagta taaacggggt 22740
ttcaccgtgt tgcccaggct ggttgtgaac tcctgagctc aggcaatccg cctgcctctg 22800
cctcccaaag tgctgggatt acagtcgtga gccaccatgc ctggccccat ccctgggtga 22860
tcctactctt ggttctgtct actgccttta ggccaaagtc tcctagatcc atatctcctg 22920
cccagatttt tctctggatt gcagacctgc ctgtcctact taaatgcctc atagacactg 22980
gtaactaaac acttgccagt ctgaactctt cttgcttaaa gctttccatt tcccatcttt 23040
gtgtttaaat ggtgacacca ccatctactc aggacctcaa gctaggaacc tggcatcatc 23100
cctgacccct tctaatctac gtgttataaa ccactcggtg gcctcccagt gcccacggga 23160
taaagtccac acttgtgaag gtggcctaca aggccctgcc tctcccatgt ggcccgtccc 23220
tcactgtgct gggactttcc attagagggc acacatcctg ccctctactg gtgagccctc 23280
cacctgtgtg tccctttccc ctctggttcc cctggctgac ttttttaaga aattcattaa 23340
atttttgggc caggcacggt ggctcacgcc tataatccca gcacttgggg aggccaaggc 23400
gggtggatca cttgaggcca gaagttcagg cccagcctgg ccaacatggt gaaaccgcat 23460
ttctactgaa aatacaaaaa ttagccaggc atggtggcac acacctgtaa tcccagctac 23520
ttgggaggct gaggcaggag aatcgcttga acccaggatg cggagcttgc agtgagccga 23580
gattgttcca ctgcactcca gcctgggcaa cagagcgaga ctctgtctca aaaataaata 23640
aataaataaa taaaataaaa aagataaaaa tttcttaaag ttttgtttat tattcattta 23700
tttttatttc tataactttc ttgggtgaat gttgtcttct cacttatata attttttttt 23760
ttaagattca tgggtacgag tgcaggtttt tacatggata tatcatgtaa cactggagtt 23820
tgcatttcta tataacccat cactcaaata gtgagcatag tactcaataa gtagcttttc 23880
aaccctctct cccttcttcc ctcccccatt ttgcagtgct caatatctat tgttcccatc 23940
tttatgtcca tgtgtaccca cggtttagct ctcacttata aatgaggaca tgtggtattt 24000
gattttctgt ttctgcattc attcacttag gataatggcc tccattttca tccatgttgc 24060
tgcaaagaaa caggaattct tttttttttt ttttggctgt gtagtgttct gtggtgtaca 24120
tgtaccacat tttctttatc caatccacca ttgatggata cccgggttga ctccatggcc 24180
ttactattat gactagtgat gtgacaaacg tgtgagtgca ggtgtctttt tgatacaatg 24240
atttattttc ctttgggttg atacccagga gcagaattgc tgagttgaat ggtagttcta 24300
tttttagttc tttgagaaat catcatactt ttttccatag gagttgaact aatttacatt 24360
ccaaccaaca gtgtctaagc attctctttt ctcttcatcc ttgccaatat ctgttatttt 24420
ctgaccttct aataatagcc atctgctgat gtgagatggt atttcactgt ggctttaatt 24480
tgcatttcct tgatgattag tgctgttgag cagcttttaa tatgtttgtt ggccacttat 24540
atgtcttctt tggagagatg tctttcatgt cctttgccca ctttttaata ggattatttg 24600
tttttgttga cttaagttcc ttatatattc tggatattag tcctttgtca gatgcatagt 24660

ttgcaaaaat tttctcccat cctgtaggct gcctctttat tctgttgata gcttcttttg 24720
ctgtgcagac ctctttagtt taattaagtc ccatttttca atgtttgttt atgttgcatt 24780
tgctttcgag gtctcagtca ttcctctggc caagtcctgc cccatctgtc ctccagagca 24840
tcctggggtg tgtgtcccca gacacctgag tgcatcagaa tgatctcttt gtatgctcct 24900
cccattagcc tatcagtccc ttaagggtgg ggattgttag gtatatacat ttcttggagt 24960
agcatcgctc catacacatt gaataaggcc ctctttcttt ttgtatcaga gctgtggtct 25020
gggcagataa tctcaccctc tagtgacaat ctgatttcca tgtaaagaag gaagttgggt 25080
gacagccaca cctctttctt tctgggacct aaggagtttt gtacaataaa cttggagcat 25140
gaaggtacca gttttcattt tccactccca tgtttaaatt ccagttgaaa agtagactgt 25200
ggattgaggc agaaagagat gtaaatttta ggtttattca taagactgat taaggatgca 25260
gcttagatgt gagtccaatt tgggctttca aatgtgtttc cccagaatta aattacgtac 25320
ccgcagacct ctgccaaggc agatctcccc aatggcaggt ctccctcatt gtggaaatgg 25380
agaatttggg ggacctaaag aaagaagctt agatctaggg gtcctcagtc tgtctcctca 25440
gaatggaata tttgtcacat acttccagtc ttccacctgc aaggcagagc tgcttccagg 25500
aaacgcaagg catgggtggg ggagaatggc aggtggctag cttgtaggac ataggtaggc 25560
ctaatgacag gacgtagagc ccaccttctt ctacaatccc accaatcaga taaggtgctt 25620
ctcctccttt ggaaggagtg aggatgagga ggcagcatgg ttctccccgt ctacaggaaa 25680
ttgagggagc aatcagtgtt tttcccaatg catcagggct ccctgggctg ggagaacttc 25740
tcccagacac gccttgttcc tcactccctc ctggctcacg caggaaggtg ccacaactcc 25800
agctcagacc tgtccatgct cagccacagg actttgtgcc ttccatgagc tcaaaacctt 25860
ttatcagatc atcttagtgg tagtttggga ctcatctgac atcccccctt ttcttgcaac 25920
acatttgaaa cactcctttg ggtgtggacg ttgccatcac ttgtatctgt tcctattgat 25980
ttcaggataa aacctcatgt gctccacttt gctctcttcc catctcttct gtattgaggc 26040
tgatccaccg tttctgaaat gggctgggcc caagtagttg gaaagagcta caggttgaca 26100
ggcactggga ttttctatgc actggagggt gtggaaggag aagacttctt aggttgtgga 26160
ggggggctta aaggtgctcc ttctgcattg cagttggggg aaaggtagca gctctctttg 26220
ggcatcatca gtattgggta tttgtgtagg atggtcaagt ggaagtgccc tatagacatt 26280
gagaaggtag ctggggctgc agtggaacag ctgatctatt tcctcaaagt aaccccaaat 26340
ctacagagct cacaagctcc catgggaatg aggggaggca ctgtgaggtt ggtctgaagt 26400
tctgagacgg agccttcagc agacttagca gcctccgagg atagctgacc ttggcttact 26460
ggcagttttg ggtatttctg tttggaacat cagtccttgg gttgtggggg agactgctga 26520
gccctgaaga gctgtgggaa ggaggccccg tgttttctga tgggctccct tgttctcttc 26580
tcagggtttt gcatctcagg aggccaggag gccctccaca tgtcaagctg gcggtggagt 26640
gggatagctc tgtcaaggag cggtgagttc aagggaattt gccacctgca gagctgagac 26700

agggcctgtg tgtgcccagg tttccctgga cgcagcttcc cccagcacgg ctgcaggcag 26760
ggtgcacaca tctttgagcc agtgtaaccc gttcctgtga gatgcctgag tgagaatttt 26820
ctgtatgtaa ataacagtga caggaatgga ggaatggagt gatggtggtg gtggtgggtg 26880
atgtcagaag tagtaactgc agtcatagta gacagttacg tagcacccac ccactcaccc 26940
catgccagtc actgctctgc acacttcatg tgtgtatagg ggatttattt aatcttaatc 27000
acaccctatg tgtgggtggt ctggggtgga tactcctgta atccacattt ctcagaggtg 27060
gaaactgagg cacagagagg gtaagcagtt gctggagggc acacagcttg tacattggca 27120
gagctgggat ttgaacccag accctctgac tggagtccat gctcctcatc accatacttg 27180
gctacctctg taacactgag ggcatcagcc ttactttaag aaaggatgtc gttcagtcca 27240
gcaagtattt attgatcaac ttctaggtgc caggccctgt gctaggatga ggaaaacaaa 27300
gatgaataag ccaaggacct gccctgtagg agctggtggg ggagagagaa ttccagtaaa 27360
atatagtgct gagtcagaag gatgcctgca gctctccagg aacccatagg cagggcacgg 27420
tagtgtggcc tggccaatgt ctgctgacgg gtttgctgtg agtagagaca tagacgaaac 27480
ctgtctggga gcaggaaagt cctgaacgcc gtgggtgtcc tctccgtgca gcctgttcgg 27540
gagcctccag gaggagcgag cgcaggatgc cgacagtgtg tggcagcagc agcaggcgca 27600
tcagcagcac agctgtacct tggatgaatg ttttcagttc tacaccaagg aggagcaggt 27660
cccgccctgg gggtccatgc cccggccggg aagggggctg gctgcctttg gtgggttggc 27720
cacgtgctgc gggggcctca cagtccgggt ggtttgcttt ccagctggcc caggatgacg 27780
cctggaagtg tcctcactgc caagtcctgc agcaggggat ggtgaagctg agtttgtgga 27840
cgctgcctga catcctcatc atccacctca aaaggttctg ccaggtgggc gagagaagaa 27900
acaagctctc cacgctggtg aagtttccgc tctctggact caacatggct ccccatgtgg 27960
cccagagaag caccagccct gaggcaggac tgggcccctg gccttcctgg aagcagccgg 28020
actgcctgcc caccagttac ccgctggact tcctgtacga cctgtatgcc gtctgcaacc 28080
accatggcaa cctgcaaggt gggcattaca caggtgagcc ttgccttgcc tttctgcaaa 28140
tgcagctgtg gccacagcct cgagatgtcc ctggaatggg tgttgctcag atgctgagct 28200
ccctggggca cttattgaga tccgacccct cacccaccgc acaccaggaa gatgaggatg 28260
aggaaaacaa agatgaataa gccaaggacc tgccctgtag gagctggtgg gggagagaga 28320
attccagtaa aatatagtgc tgagtcagaa ggatgcctgc agctctccag gaacccatag 28380
gcagggcacg gtagtgtggc tgtctcgcac catgtgcgag agcagaaggg accttgtccc 28440
agctatagag ttgacattga gttaggttgg aggttggaat atgtaaagtt aggcctgatg 28500
cagtagctca tgcctgtaat cccagcactt tgggaggctg aggtggaaga atcacttgag 28560
gccaggggtt caagaccagc ctaggcaaca aagtgagacc cccgtctcta caaaaaaaat 28620
gaaaaaaaaa aatgagctag gcattgtggc ttacacttgt agtcctagtt gctcaggagg 28680
ctgagatagg aggctgaggc cgtggtgaac catgattgca ccactgtact ccagcctggg 28740
caacagagcg agaccgtgtc tccaaaacaa ataaaccaac aaacagaggt tagttactgc 28800

ccaaattttc aaccatatta aatatgggag gtcattattg gaaccaagag agtgacattc 28860
attctcatct gtgattcagc gtgaggaatc ttacttatcg gatttggtat ccttttattc 28920
tatggggtac ttttcatgtt tattcctctg gggaatttca gagcagtgga tgaagggaag 28980
ggcataaaca aatggccaga ttttctcaat cgttcataat catccctttg agtgttttca 29040
gagagtgtca tgagggatga tttttctcga ttatgtcatt gtttgcttct cccccaccag 29100
ggcctctcag tgacggtgag aagcctattg gtagctgcgg caattgagtg tctccaccgt 29160
gcagatagtg tggagggtca tcggggtgag ccttaggagt ccagggggtt tagggcatga 29220
tttccgccat cggagaatgg atggaacagg cccccataga gaaagagaaa tgaggatgca 29280
gttacaggct ggggaccacc tgagtggaca gagatgcgct ggatggtggg ccagcctagg 29340
ctggtcatgg tgaaggttat gggagccacc aaagccgctt tcagctgaca gtggccgcct 29400
tatgctgctg tgcggtgcca gcctgttctt ccacagctgt cctgctccag atgagggctt 29460
gccacctttg taactgctgg gaccattctg tccagccact tcccctgtct cacttctctg 29520
ggccgaaaat agctcagcag gaatcccttg aggcccatat atatattggg aggcatcttt 29580
gttactcaga tttttttttt tttttactaa gagcgttatg gctgaatgag tccccgtgca 29640
cacatgagtg gtgaaaataa ggacagctgg aacctgggcc ttgtcatttc ccatgctgaa 29700
cgtgctgcct ccccatctat aaactcacag ggtggcccca cataaattaa gggcagtgat 29760
gtaaaaccac agcagggagt tagaagtagc ataggcgtga gaagcgttgg catctgcctt 29820
atcaaaagcg agatccagac ccctcctggg gtgaacatta aagagcagaa tgcccgattt 29880
tgggggtggt ggtatggcag cagaagggac gcagttcctg gaaggttccc cttgcagaga 29940
tctcctgggt cagtgtttgc acacacagtg tgccaccacc tgccaggaaa gagcctgttt 30000
aatactaaaa gcaaagggag aggagcccaa gggaaagagg ggactggcga ggcggtaggc 30060
agggcagtgt gtgcagccaa cacagccctg ggtaagacct gggccacttg ctgtcatcgg 30120
tgccaacata cacaatcttt tccctgaaga caatttgcac tgccagttaa atggcagagt 30180
ctgggcttca aacctgggcc accctggctc cagggtcagt agcttctgcc ctgccgagat 30240
gagtgattcc tgatatgaac aagtccccaa gccatgtagg gtattgtaga tgagtgtcaa 30300
ctccctcctt taggtgaagc tcaccctcag tggcttccta ggaaagggtg atgggagctt 30360
gacctttgga gtgcttacgt gtcaggaaat gcctttattt ccacacctga ttgatagcct 30420
ggtgggacat aaagtccttg attgaaaatc agcaatgttg ctccgtgtgg aatggggtct 30480
ggagtagggt tttgctgttg agaatgggtc aggccagttt gatttctgtt cctttgtcta 30540
ggataggata tatttttctc tctggaagct ttcaggttct ttttcacagg tgctctggaa 30600
tttcatagag tgtgcttacc tgtaggtctt tttcattgct tgtattggga cctgcattta 30660
ggggacctgt gtgtctgcag acgtgatctt caggtctggg aaatatcttt gtgctttttt 30720
ttgttttgtt tgtgagatgg aatcttgctc tgtcacccag gctagagtgc agtgatgtga 30780
tctcggctca ctgcaatctc tgcctcctgg attcaagtga ttctcctgcc ccagcctcct 30840

gagtacttgg gactcagggc tggccacggt ggtgctcgcc accacgcctg gctaattttt 30900
gtatttttta gtagagacag ggtttcacca tgttggacag gctggtcttg aactcctggc 30960
ctcaaatgat ctgccttcct cggcctccca aagtgctggg attacaggcg tgagccactg 31020
cgcctggcct agtcttaggt ttctttttca gagcatcctg gttttgtttt gggtcctcac 31080
ctggcttggc ttcttgtggt ttctgacacg cccagtaccg agcctctggg gctgtagctg 31140
gtgggtggcc ctcttcctac cccctgcaac ccccccacct cccccaggca ctctgtgttg 31200
aaccccttcc ttgcagaggc gagtgtgtga cctccactcc atctgcttgc tgggccctca 31260
aagctgagct gttgctttct atctcacgtc ttttgacata tttgttcctt tttagttatt 31320
tgcttcttgg aagtgtgata attctaccac atttcagatt tttagaaatc tcaaatgcct 31380
tttgtttcat tttaaatgtg gtaatgtggt aaaatttcat tttaaatata ggtaatgcat 31440
gcctatattt caaaattcac acggcctaaa aaagatggac agtgacaagt cttgattcta 31500
tgtttgttcc tcatctaccc cctcctcatt tctaggtaac catttttagt agtttcttgt 31560
gtactattct tccacaattt ctttatgtaa atacaagcaa ttgtgagtat atttttctat 31620
atacatatat atgtgttgat tttatatatt tccttcccac cccctaattt aagtaaaagt 31680
actatttact gtgttgcacc tggctttttt ttttttttct ctctgaaaat ctgtcttgga 31740
aatcttttca tgtcctcatt tctagacagc ttctccatct tcttttataa atgtaaatgc 31800
actaaataaa tataacaatt tattaaatac tctgtagatg gacattggac tgactcattc 31860
ttttactatt acaaatgttg ctgtcggccg ggcgtggtgg ctcacgcctg taatcccagc 31920
actttgggag gctgaggcag gcagatcact tgaggttagg agttcaagac cagcctggcc 31980
aacatggtga aaccctgtct ctactaaaaa tacaaaaatc agccaggcgt ggtggcgtgt 32040
gcctgtagtt ccagctactc ggggggctga ggcaggagaa ctgcttgaac ccagaggtgg 32100
agattgcagt gagccgagat cacaccactg cactccagcc tgggtgagag agtgagactc 32160
tgtctcaaaa aaaaaaaaca acaacaacag caaaaaaaaa acccaaaaaa caaaaaaaca 32220
caaatattgc tgtgataacc ctttagatat gtcattttct acatatggaa gtttattggc 32280
aggataaatt ctcagaattg gaatagctgt gtctgtgttg taataagcac aaacaagatg 32340
tcctgcacaa agggtcatag cagtttgcac tctgttaaga gcaagagaac acctggttct 32400
ctatagcctc tccagtagag tgtgtgatca cacttttgga tttttgccca tctaatagta 32460
ggaaaatggt atctcagtgt aagttgaata tttaggttct taatagtgtg agcttgagca 32520
tcttttcgca tgtttgagcc actatggttc ctttcctgtg aactgtctat ccatatcctt 32580
gactgttttt caaatgaatt attagtcttt ttcccatcaa tttctagatg ctctaattct 32640
taatgtacta tgagattcac ctcttttctg taatataaat tgcaagtatg tgttccctgt 32700
gtaacatctg tcagttggct cttcctgtgg aaaatttttg ccatgcagaa agtttttatt 32760
tttgcttttc taggtaaaat tatcaatcat ttcttttaag tcttctggat tatgagtcat 32820
gattagaaag cctttttcac tctaagatta aaaagtgttt ttcttgagtt ttctacttgg 32880
ttttagctta ttacatttaa atccttgatt tggagtttct taattttctt gagagaaaga 32940

gatccaactt tatcctccgg gagttgccaa tttgtcccga gatcactact gatggaaaag 33000
ccaatcttgt tccttgagat gccatctgcc ctatcagata ttaatttttt tttttttttt 33060
tttttttttt ttttgagaca gagtctcgct ctgtgtccca ggctggagtg caattgcacg 33120
atatcggctc actgcaacct ctgcctcccg ggttctagtg attctcctgc ctcagcctcc 33180
tgagtagctg ggactacagg cgggtgccac cacacccagc taattttttg tatttttagt 33240
agagacgggg tttcactgtg ttagccaggc tggtcttgaa ctcctgacct catgatctgc 33300
ccgcctcggc ctcccaaagt gctgggatta caggcgtgag tctccgtgcc cagcctggga 33360
tttttatgac tagaactaac atatgtaaga tgcttctcat attgtaggcc ccaacaattg 33420
gtaactttca agctgccata tacttttaca taaatgctat cataccatag atgtattttt 33480
tgtttttagt ggatttttgt ttgccatttt acatatcaaa ataaacttaa aagatgttaa 33540
atgaaataaa atatgatgat gaggcacttg actaaatggc aaaatgaaac actgggttcc 33600
atcatgacct cctgatgtgc gcatccattt acgtacgcgc acatgtgtgt ttatgtgtac 33660
atacatagat taagactaaa cagaatttca tgagtcgata attaaattta ctacatgtga 33720
tataccttgg tccattctat tccagaataa catattactt aattttaaaa atgctttggc 33780
caggcgcggt ggctcacgcc tgtaatccca gcactttggg aggccgaggt gggcggacca 33840
cgaggtcagg agatcaagac catcctggct aacatggtga aaccccgtct ctaataaaaa 33900
tacaaaacaa ttagccgggt gtggtggcag gcgcctgtag tcccaactac tcgggaggct 33960
gaggcaggag aatggcgtga acccgggagg tggagcttgc agtgagccga gatcatgcca 34020
ctgcactcca gcctgggcga cagaccgaga ctctgtctca aaaaaaaaaa aaaaagcttt 34080
aaaatatttc attttttatt tattaaacat ttttttttaa tgcactgatt accagtgtcc 34140
cgatgggctg atgactggca atttggaaga caccaaacta tatagaaatc caggatcttt 34200
gctcattaat tcattgcaca aatgtatatt aagtacctgt tctatgccag gtcctggagc 34260
ttcagtgatg aacaagacaa atacaaggcc tgccctcatg ggacttcagt ctcactttgg 34320
agacagacaa taaaatgtgc ttatcatttt agcactttca ctacctgaca ttttgaaagg 34380
gcgactgatt gactgtgtct gtgatagaca taaggcctct gcaaggatgt cacatgtagg 34440
ctcagagctg aggatgagat agaactagct atgtgaataa tgggatgaat tgcattatga 34500
gcagagctcg tagcatgtgc aaaggccctg aggcagaaag cattcggtgt gtttaagaca 34560
tgatgacagc ctggatgaag tgtagccatt caggggtaat tgatgaaggg tggggctgga 34620
gaagtgggca ggacactcta ggccacggat gggagtttgc attttttcca aaggggtgat 34680
aaatcattta aaggttctaa gcagaggagt gaagacataa tctagtgtac aaatcaaact 34740
agttgctctg ctgtatggag aatgtgtaga agggatctag agtggaagct ggaattccag 34800
ttaggagggt gcttcagggg ccgagtgaga gacaccactg gctgagatgt tggtgttgac 34860
aatcaagata ttcgagacat agtttggaag tagaatcagt aggtctggct tttggattag 34920
gtgtggggcg gaacggaaaa gcaagagcaa agatgaactc ctaggtggct ggcttggatt 34980

agtgtggagg tcagcaaact gtggcccacg ggccggatcc aatctagggc ctgttttttg 35040

ttgttgttgt tgttttttga gacggagtct cactgtgttg cccgggttgg agtgcagtgg 35100

cgtgatcctg gctcactgca acctctgcct cctgggttca agcgattatc ctgcctcagc 35160

ctcccaagta gctgggatta caggcgtgca ccactacgcc cagctaattt tttgtatttt 35220

taatagagac ggggtttcac catgttggcc aggttggtct cgaactcctg acctcgtgat 35280

ttgcccacct cggcctccca aagtgctggg attaaaggca tgaaccacca cgcccggcct 35340

gttttttttc ccccgtttat taataatcta tgggttaaaa atggttttta caattttaaa 35400

agtgggagaa agacacgcag aatatgcgac agagaatata tgtggccata tgtgacctgc 35460

aaagtctcaa tatttgtcat ctggtccctt atgggaaaag tttgcaaact tccagattac 35520

tgggttatgc tgtcatttgc tgacacgtag agggcaagga aacggatatt ttgttgtcgt 35580

tacttgagaa atggtgagaa tatttatgaa ttttccttag acgtttgact tcagttctta 35640

gaaagaggta tgccataagc ccaattgtga ttctcatctt cacttttatt gcatgcttaa 35700

tcagagatat gcattttgaa gcttctcaga agaaatgggg tgaacgctga cattattgtt 35760

tggctaccta acttgggagc tcccttttat ttgagatttt gcctgatgaa ggggtagaat 35820

attcaactta aaataacagc actcttggcc gggcatggtg gctcaggcct gtaatcccag 35880

cactttggga agc 35893

<210> 23
<211> 2014
<212> DNA
<213> Homo sapiens

<220>
<221> misc_feature
<222> (1)..(2014)
<223> chromosome 18 nucleotides 41490240 to 41492253

<400> 23
agatggatag aacttaggca agaagaggga tggagggaga acagagggcc agtgataagg 60

ccagaacacc ttggcagggt gtgcgttggg aaagaaaagg gtgaagcttg atgaagttgt 120

tgtaaacaca tcaggaaagc caggcagagg aatttgagca caatattctt ggcaataggg 180

agcatgtagg ttattgagta agagagtgac ataataacat ggtctagccg ctgaagctaa 240

gaatggattg aaagaggaaa gatcaaaatt aggaagactg atgatacaga catgcaattc 300

tgagggtgtg ggccagggta ggaacagtga gaagagagtg caaagtcatt ctggagaaaa 360

atcagtctgg tgtcccgcag ttagagatgg ggtgctctga cctttgtact ctctttaacc 420

tcatttatga ggctttatca tcaggcatca tgaggttcaa taaggcatca acttatttat 480

gctaattaac gtcttcccca tggaataagc caaagacacc gaaagaaatg agatttaggc 540

tagtcctttg gccaacaagt gagctgcgtg ggaaggagaa gagaaagttt aaagcatttg 600

ctgtgaggat gagtctcgct tctttcaagg taacagtgac tcataccccc accttcttaa 660

aaataaaaaa ttggctaatg gattaagaag ttgagccttc caagtaattt tatctccaag 720

tgttcccatg gacctggtct tcctcttcat tccatgcatc tctcatccac ctcccagtcc 780

agataggaag tctcaggaag actggggttt ttcttgagga aagggagcca tggtttaaag 840

agaaatagat tcgagaatac ctaccttagg ccattatatt ccaaagcaga gttgaattga 900

aagattttga ctaggccggg cacagtggct cacgcctgta atcccaacac tttgggaggc 960

agaagcgggt ggatcacctg aggtcaggag attgagacta tcctagctaa caccgtgaaa 1020

ccccatctct actaaaaata caaaaataca aaaaaaaaaa attagctagg agtggtggca 1080

tgcgcctgta gtcccagcta ctcaggaggc tgagacagga gaattgcttg aactctggag 1140

gcggaggttg cagtgagccg agatcacgcc actgcactcc agtctgggtg acagagcgag 1200

actgtgtcaa ggaaaaaaaa aaaaaaagaa aggaaagaaa gaaaaggaag gaaggaagga 1260

aggaaagaaa agagagagag aaagaaagga aagaaaagaa agaaaagaaa aaagaaagaa 1320

actgaacttc caatggggag taaggtttag ggaaataaat cttagactac aagataagag 1380

agaagagaca cctatgtctt atctacactc agctctgcca atagctgtgt ggcctagggc 1440

aagtcattcc atttcttgtg gcctcagttt ccaaatctgt aaaatgggag agctggacaa 1500

gatcatctca aagccttttt caattctaac atccaatgcc ttagagagtt tagagaatga 1560

tctttgaaaa tttcaactaa atataacttc acagaaaaag gtaccatccc acttaccacc 1620

cacaagtatc catatcgcac acacagagaa tagcctagga aattacaagt cttagtacaa 1680

agctcaactt ccatgtggag aaacattgtc caaactgtgg ccaaagcata gtcagtcaaa 1740

gcatagaact ggttgtagaa aaaaaaaaga tcaaattata cgtgatgtgt cacttcaaag 1800

acagtggttc tggtattgca gcaaggaaat tgccttgtgg tgtgtgggtg tgtccacaaa 1860

caacagatca gaggagtaag aggcccagga agctggggag ggatggcaaa taggtcttga 1920

tggtagcctc tcatgctcct tatgttggac attgcaactg aaagatggac aacatcaatc 1980

cccccctagg agagagtaaa tcttgttcaa aacc 2014

<210> 24
<211> 332
<212> DNA
<213> Homo sapiens

<220>
<221> misc_feature
<222> (1)..(332)
<223> chromosome 18 nucleotides 68579550 to 68579881

<400> 24
tatgtttcaa ttctacaaga aaagcagaaa acataaatta tgcatattaa ataaaataca 60

aatacgtatt ggaatgtgtt tgtactactt aataggagga aggtttagct gtgtaagata 120

tttagctgtg atactctttt tttaacagtt taatgagacc atcaagataa caggtatttt 180

ctaacatcca attgttaaga gagacattca gatcccatct tggaaaagca agccaggcca 240

tatgtagaac agaatagctc tcaaccttgg gctgcaatct aaaagaaatg tttttgcaga 300

catgtaaatg atccgataga attgaggctt gg 332

<210>  25
<211>  11675
<212>  DNA
<213>  Homo sapiens

<220>
<221>  misc_feature
<222>  (1)..(11675)
<223>  chromosome 1 nucleotides 33725161 to 33736835

<400>  25
   ctcagtaatt taatgaagat agttgttatt attttggttc aaaaaaataa gactcagagc      60
   agtatatcta gtcataagtt tttgtataaa aaagagggaa gctaagaata cacatttaca     120
   tttgcttgta tgtagaaaga aactctaaaa gaatgcagaa gagcatagtt acctgtgatg     180
   aggagagggg aaaagaacgg ggtcgatggg atgaagatgg atggaggtgg ggcattttca     240
   ctgaatatac ccatgtatgt attttctgtt tttaaaccat gtgagtctat tatctattga     300
   aaaaaattga aatatgtttt taaaagttag gtgtttctat aaagtattga gattgttata     360
   ttttggaggt tttatagcct gggtaacacc ttgacctctc tacctgtgaa cttggaacca     420
   gctgagaaat agagataaga aattagctgt gtgattgatg aagttgattg ggtaatgtta     480
   ttttagttga ttactccctt ttattttaaa tatctatatc tctgagtacc tttttttttt     540
   ttgcctgaaa tccaacattg aaatgaaaat tgccacttcc tttttccttt tgagttagag     600
   tgccttgtct tttgcatgtc cttctattgt aagattaaca acacgtactt ttatctcatt     660
   ttaaaattcc atctaatttc tttttttgag acaagttctc actccattgt ccaggctgga     720
   gtgcagtggc atgatcatag gtcactggta cttcaaactt ctgggctcaa gtgatccttc     780
   tgcctcagcc tcttgagtag ctgggggtac aggcatgtgc caccatgcct ggttaatttt     840
   atttatttat ttatttatta tagaggtgag ggtctgctat gttgcccagg ctggtcttga     900
   acccttggca tcaagtgatt cccttgcctc agcctcccaa aatgctagaa ttacaggcat     960
   gagccactgc acccagcctc catctgtaat tctttgtcct ttttaaaaat tttattttaa    1020
   aaatatcttt ctctttcagc agagatattt agagcaattg catttggggt tataatttta    1080
   tatttttttc ctgtttttat gcctcccttt gccttttaat atacaacttg ataactttta    1140
   tctttttttg atcatttaaa aagtaagcaa cctatttttg atgcttatgg gttatttttt    1200
   aatgttacac aaattttaca taatttatct ctttatgaat caaaaatgtt ttgaacagtg    1260
   ggactctcat tgaaacactt ggatcgccac cctgtgttca acagatggta ctcatttggt    1320
   ggtataaatg ctggtctacc taccaaactc actctgatgg ctttctcatc ctatttcaag    1380
   ccattttgga tcttaaaaga gcctgttagc ctctggactg cagttgtact caaaatacca    1440
   aagtgggtct tactgaattg ttgattggga attttgacta tttgtgtgta gggactggaa    1500
   ttgcatacag aagagaccag gcccttaaag acaggggaag aagctcagag cttccagctg    1560
   cagccagtgg atccctggcc tgagggacag tcccagaaga agggggtgaa gaatacatgc    1620
   cctgaccttc ccaatcacct aaatgccgag gtggcaccac agcctttgaa agagagtggt    1680
   gagtacatct gagaacatta gggaatgagg ccttctgcag gggagaggga tgggaggtga 1740
   ggaagggtgc atggggaaga aggatgtgca gaattcaaag caagggatag gctgcagtgt 1800
   ggtgggcaca cttactgaga agtaagcaag cctcgtaaag aggctgtgcc tatcgatgat 1860
   gtcatcatgc cctgctgtag ctggagtcct gcagcaggtg caggctggga caggggtggg 1920
   tggtgatcac aggaggtcag ggctggcatc tcccacatgg cacaccactc acagcagctt 1980
   aggagctaca cactctagaa gggaagtaaa ggggaagtct gcactggcca tagggatagc 2040
   cctagcttga tccaagtgtt acttttggaa agccaatctc aaagggaggt gattttttgg 2100
   gaaccagagc ttgggtttca gtgacttttt cttttcctca gctgtcctca ctccccgagt 2160
   ccctactctc ccaaagatgg ggagcgttgg agattgggag gtgacagctg agtcccaggt 2220
   aagctgttac tcgttcttcc tttcctgtca ttgctgctcc tgtgctcttg cccccacagt 2280
   gttcctcctc tctctccatg acctctgctc agcaaaaggg catgaggccc agggtgttag 2340
   cctttgccct tcacccccag cctgctgggc cccaaacatc cccaaacaca tgtatgtacc 2400
   aaatggccat cctgctcctt atgatgccag cttcacacat ggtatccctc tgttcccatc 2460
   atctttcctg ggagcctgct agggttccta ggctgttgtc aaaggtcagt gatgtttggg 2520
   gtccttgtct tgtgcatctc gcaggaagcc ctgggccctg gcaaacatgc tgagaaggag 2580
   ctctgtaaag accccccagg agacgactgt gggaacagcg tgtgcctggg taaggagacg 2640
   taccctctga aggtgatgtt gtcatgcttc tgatacaccc agttcccaaa gagggttttg 2700
   ccgctccttt aagaatcatg gctctgcagt ctctggaatt tcagaaatag gaagaagact 2760
   tgccctttgt agccagcact ctcatggcag ggatggagtc tgcctggaga cggggtggca 2820
   ggatggcttc caagagcctg ccctctctgg gcacattacc ttccttacct cagtccattc 2880
   accacagtcc aggggccagg cctggactgc catctcagaa atctgatttt ttttagcact 2940
   gtaaatagga tctctctctc aggcagactt aagtgaacta aacccatgag accaatgaat 3000
   taaacccaat aggaataaat atcacagagc ccaaaatacc tgaagcccat ataagcagca 3060
   cagactcctt tctatgtggg cactcctatg tggcaagcca aataataggg agccgtttcc 3120
   ctgtggagcc cgggatcact gtgtggggag ttagacatgt gaatagctga aatagaaaag 3180
   actctgatag gcctgactca tggtaagttt agataccaga agctcgctgg ctacttgctt 3240
   gacagtggaa ccccttttta cacatgatat ctgggggaaa tagtgttcaa aggaacagat 3300
   tttgggaaac ggtgaattaa attatcctta tacctcttta aacccttttc atctagtttt 3360
   cttcaaatat ttatttcaat taaaggcttc ctattttgtt agggccggga gctgttggga 3420
   tagcagtcca ttaaacatag ctaactttta aaaataagga aatttatttt ctcacattat 3480
   tagaagtcca gaggaggaca tgctccagcc acagtacaga ctagctgagc aatgtcttaa 3540
   tgggtctgtg ttctttattt ctgtctgcca tcctcagtgt tagcttcata ttaaagctgt 3600
   tttgtttcat ggtcaaaaga tagatgccaa tggtggcaat ggatgcctag caggacagag 3660
   actcactaca cacacaaaca cacacacaca cacacacaca cacacacaca ccacacaccc 3720
   ttttcctcca gccatggagt tcagtctaat tgggccaatt taggccatgt gcccatctgt 3780
   ggaccaatga gttactctta tatgatgttt catgtttcat aacaacaaca acaacaaaca 3840
   aatgaacaaa gtaaacaggt cacttaacat ttctcatata ttggtcgtgt tacctcttct 3900
   ctctccctat tcctgtaaac attttatggg atcaacttgg gaatttctcg ttagcctctt 3960
   gcaaggaaaa gatagaagtt tcagggagat tttggacaaa catgccgctc aacagaccat 4020
   ttctactctg tcatttctct tccttttgta ggagttccag tttcaaaacc aagtaatacc 4080
   tccgagaaag agcaaggacc agagttttgg ggtctaagtc ttataaattc tgggaaaagg 4140
   agcactgcag attacagcct ggataatgag ccagctcagg cattgacctg gagggattca 4200
   agagcctggg aggaacaata ccagtgggat gtggaggaca tgaaggtgtc aggtgttcac 4260
   tggggctatg aggagaccaa gactttcctg gcaattttga gtgaatctcc tttctctgaa 4320
   aagctccgga cttgtcacca gaaccgccag gtatatcggg ccattgcaga gcagctaagg 4380
   gcaaggggct tcctgcggac actggagcaa tgtcgctata gggtcaaaaa cctcctacgg 4440
   aattaccgga aagccaagag cagccaccca ccaggtacct gccccttcta tgaggagctg 4500
   gaggccctgg tcagggctcg gacagccatc agagccacag atggcccagg agaggccgtg 4560
   gcacttccca ggctcgggta tagtgacgca gagatggatg agcaggagga agggggctgg 4620
   gatcctgaag aaatggcaga agactgtaac ggtgctggcc tggtcaatgt tgagtctacc 4680
   caggggccca ggattgcagg ggccccagct ctgttccaga gtcgtattgg taagaacatg 4740
   ggggtttagt cagattcaga tttccaaccc tcctaccagc tagactgcta ttccctgagg 4800
   tcagggcaca ggctgcaagt ctggattgaa gccactagag tgaagagcta cattccttag 4860
   gtcatcctca aactccaact ttcttttccc atgaccaagt ctctttgcaa aagtcttctt 4920
   agttatagaa gcaaacattt ttaaagcctt aaggggcctc aaagagtgaa tccagtattt 4980
   ccaaaagtgt gtcactggta gtatgggatg atcatcgacg ttacatgaat gaactttaaa 5040
   ttcttgtatt tatatatttc tcttaatttg tatgagtgca tcaacctgtg atttcacaga 5100
   taacaaagtt aggtaaagag tgatggcgag tgaaatattg aacagataat ggcagaaatc 5160
   ccgacagtgt agacttgaat aatcaatgtc tgggaaacat ccaatcattg taaaggtgca 5220
   aacatggaag tccatgagat gaggcgtctt atagacgcac aggtaggagc agagcccgga 5280
   tcagagcctg tggctcctgc ctgctggttt cacatggttt tccctccacc atgttgcctt 5340
   cctgggctac atcactttcc tgcagaggag ctgagactag accacctcct tttcattccc 5400
   ctgcaaagga ggaaagtgtg tcctcatttg gcctttcttg caggactatt tgaaactcat 5460
   ttatgcctct aatctttagg aattctcagc tttcatgttt tgttaaaagg agaatttatg 5520
   gccaggtgcg gtggctcacg cctgtaatcc caacactttg ggaggctgag gtgggcagat 5580
   cacgaggtca ggagatcaag actatcctgg ccaacatggt gaaaccctgt ctctactaaa 5640
   aaaaatacaa aaattagctg ggtgtggtgg catgcacctg taatcccagc tactcaggag 5700
   gctgaggcag gagaattgct tgaacccagg aggcagagat tgcagtgagc caggattgca 5760
   tcgctgcact ccagcctggc ggcaaagcga gactccgtct caaaaaaaaa aaaaaaaaaa 5820
   aaaggagaat ttataaaata aatagagtac aggtattgag tgaatatggg gaaaatcaca 5880
   tgcatcccaa ttacaggcct ttccctgaac taatcagcta aagccaggca catgggatca 5940
   cctgatataa ttgatataac atggctactt aagtcctcac cttcagccaa gacttgaagt 6000
   gttaatagaa tctcttagaa gagggtgagg gtgggaagct ctggctggct gtcctagcat 6060
   gcccctgaga agcaaaggga tattctccag gtacctggat agtttctgac atgcctatgt 6120
   tctaggtatt ttgaagggca ggtgacttta ttctctgatg acctaggcac atctcattaa 6180
   gtctcacagt tctcaactct tcactcctga cagcaggtgt gcactggggc tatgaggaga 6240
   ccaaggcctt cctggcaatt ctcagtgagt ccccattctc ggaaaagctt cgtacctgtc 6300
   accagaacag ccaggtgtac cgggccattg cagagcggct gtgtgctctg ggcttcctgc 6360
   ggacactgga gcagtgtcgc tacagattca aaaacctcct tcgaagctac cggaaagcca 6420
   agagcagcca cccaccaggg acatgccctt tctatgagga actggactcg ctgatgaggg 6480
   ctcgggctgc agtcagggcc atggggactg tccgagaggc tgcaggtctc cctaggtgtg 6540
   ggcagagtag tgctgagact gatgcccagg aggcctgggg tgaagtggcc aatgaagatg 6600
   ctgtcaaacc ttcaaccttg tgtcctaaag ccccagacat gggtaagcct ggagtaattg 6660
   gatgttctca aaatctgtgg gcatgtggag agaatgagga agccaggctc attatctttt 6720
   gtctcttaac taaaagagag aatgggattg aatgggaaaa agtatttctg ctttgctcag 6780
   attatccagt ctctagcttt gtgtgatccc ccgaatcagt cagtcagtca gtcattctat 6840
   cttttactag gtatatctgg gcggttagag actttattca atccagtatg acacaggata 6900
   tatggaatgt actagaagaa ggaggtgttc atgattgagt actaagatag aggagtttga 6960
   tggggaaaca ggttggctgt ggctgctggg ctgagcccca tggctcactt ggtggggtca 7020
   ccatgtccca gagtccattc ctcagctttg caggtcacaa acacttgtgt gtaccaaaac 7080
   ttgatcaatt tcttgttagt cacatttata agatgtgcga tagaagatat aagaatcaaa 7140
   ttcatgttag ccaaattcac agatgtacaa tacagacaca cacaaacaca cacacacgcg 7200
   cgctaatgag tgaagaaaaa ctgcatttct gtccattttt tcaggttttg aaatgaggca 7260
   tgaggatgaa gaccagattt cagagcagga catttttgag ggtttgcctg gagccttatc 7320
   aaaatgtcct acagaagctg tttgccaacc tcttgactgg ggagaagaca gtgaaaatga 7380
   aaatgaagat gaagggcagt ggggaaatcc ctcacaggaa cagtggcaag aaagttcttc 7440
   tgaagaggac ttagaaaaac ttattgacca tcaaggcctg taccttgcag agaaacccta 7500
   caagtgtgac acatgcatga agagcttcag tcggagctcc cacttcattg cccatcagcg 7560
   aatccacaca ggtgagaagc cctacaaatg ccttgaatgt ggaaaaaact ttagtgaccg 7620
   ctctaacctc aatacccatc agagaatcca cactggagag aagccctata aatgccttga 7680
   atgtgggaaa agctttagtg accattctaa tctcatcact caccagagaa ttcacacggg 7740
   ggaaaagccc tataaatgtg gagaatgttg gaaaagcttc aaccagagct caaaccttct 7800
   gaaacatcag agaatccact tgggaggaaa tcctgaccag tgtagtgagc ctgggggaaa 7860
   ctttgcccaa agcccatctt ttagtgctca ctggaggaat tctacagaag agacagctcc 7920
   tgaacaacct caaagtatca gtaaggactt gaattctcct ggaccacaca gcacaaactc 7980
   aggggagaaa ctttatgagt gttctgaatg tggaagaagc ttctctaaga gctctgccct 8040
   cattagtcac caaagaatcc atacgggaga gaaaccatat gaatgtgccg aatgtgggaa 8100
   aagcttcagt aagagctcca ccctggccaa ccaccagcgc acccacactg gagagaagcc 8160
   gtataaatgt gtggactgtg ggaagtgctt cagtgagcgc tccaagctca tcacacacca 8220
   gagagtgcac acaggagaga agccctacaa atgccttgag tgtggaaaat tcttccgtga 8280
   ccgttctaac ctcattactc accagaggat tcatacggga gagaagccgt ataagtgcag 8340
   agagtgtggg aaatgcttta accagagctc cagtcttatt attcaccaga gaatccacac 8400
   aggggagaaa ccctacaagt gcacagagtg tggcaaagac ttcaacaaca gttcccactt 8460
   cagtgctcac cggagaaccc atgcaggagg gaaggcgtcg taggggacag tttcctcaac 8520
   aacaaaggag gactcaatgt atatatctta tatcataaga tgtatgctag agataaactt 8580
   tccaattttt aagcttggtg tgtacccagg gaagttatct tggtataaac caggtaattt 8640
   ggaagtgaat tacaaatact aaggatccag atttgaaggc acttttaagt gtaatttgtt 8700
   tttcttctgt aaagacccac acagaatcct gactgtcctt gtatttgcta tcatgtaaga 8760
   gctgtgtcag tatttgagcc aaactggcaa cttacgagat tagagttaaa tcagtggtcc 8820
   tgagcagtga ttccaaactc tcactgtgcc ttcacaccat ccatgtgtaa tccaccccat 8880
   cagcagtggt tctcctactt tctcagtggg ggcatatcct caagggagaa tgttgtgact 8940
   ctggtgagag aggagttggc ctagagcagg ccactgtgag ctcagcacag agtaggaaga 9000
   acagtgacat cattacaaaa tcatcaatag cagcaatagc tggtgtttat tgagctgttg 9060
   ctctttggca agctctgtgc taagaacttt gtatacatca tctcatttaa tcttcacaac 9120
   ggccccagga gataagtact aactttctcc ccatttccta gaggcttggg aaattaagta 9180
   actcgcactg gtcacacatc tgtaagtggg agaggcagga ttcaacagat ctgtctgtct 9240
   tgagtctatc gtgctcttat tgctatactg aattcccatt catgttaggt aacttagggg 9300
   ccagaatctc catgcacttt gtagaccaca tttcctgtta tgaaatttcc acattttgaa 9360
   ttttttaaac aaatatggaa actgagaatc cctaaggatt aaaggacttg ggatatccag 9420
   aattggtgaa gtgatttgag caaggatgag ctattatcaa aatacttgat gtgagtttcc 9480
   atcttcgcaa taatatgggt gagagatata ggtgggttga gattgggaaa aatatgcggc 9540
   ctaagtaatt tttcttccaa taagatactg ctttggctaa ggaaatggga tgtaagagtt 9600
   ctctttttct caggggacag tcctacttct tgccatgctt ggctgatcat aggttaaccc 9660
   taacagaagc ctggggtccc tacatttttg tctgtctggc ttttcctttt ggcaagcaga 9720
   agctttgttc ttggtcccca tgccaagact gaggtgggag gtgctggtgt atatggtcat 9780
   ctactgagag ggccttgacc actgttctgt gtaccaggtt cttggcacaa ttttaccctg 9840
   agccatcctg tcccaggcct gcccagggct ttctgtatgt tgacccttaa gactcacctc 9900
   ccacatggac aggctaagtt agaaaggttc acataggatg gttttaacat ttgcagcact 9960
   ttggcctcca gagtttctcc ttagaagggt ttaaaactgt tgaccaagga cttcggtttc 10020
   cataggactt taaaggaaaa tccccctcct gccatcctct tatgacacta ggatgtattc 10080
   ataacctgct tggttcatgg gctggggaag tacctatcag atagtggatg tgaagcagcc 10140
   ttgaagaatg gaacttttat taacatgtga ggaagccagt ggagagattt gctactccca 10200
   cagagtgata tcccttctta accccttccc ctgcttcaga acctcctttc ccaagaacca 10260
   gccccactag gatcaccgtc aactccctcc cagtttacct tttctggccc acgttatctc 10320
   tgcggggcag gggtcatggg atgaaccctg gatagaagtc agcagatgtc agtaactgca 10380
   gtaatacagt gatattgtct agtaatgtga gctgagagga aggatcctga ggtgagggag 10440
   agggagggaa ttccagggaa aggcactgtt gtgtggaata gcatggattc agggtggggt 10500
   tgtatgatgg gaggttggga acagggagct gatattagag gagtgctgga gcaaggtcac 10560
   tgaggacctc acatgtgaat ggagtgtcaa caccaggccc ggggcaatgt tcagatgtgc 10620
   atggttgaaa tctgacagct gaatggaact tggacatgct gcaattagga tggccttttg 10680
   ggacactgtt gtggtatttc agctaaaggc tagggtctga actagagcag tggtgacact 10740
   gaggaggagg gaatgaatgg gaaaggtctc agaggcagag gggaggacat cagaggaaag 10800
   ctagaatgcc tttggtgact gggtagagca tgtcctcatt cagaattggg agcacaagga 10860
   ggagaggctg atagagcagg agggcagaag atgagaagtt tagtttggta tgtctagaac 10920
   ttgaggtgct taagggaaat catggtgatg ctatgcattt ggatgtcggc tagcagctag 10980
   gggagaggct aggactagag atctgtgtat gcgaagtagt taaactaatg agaaaggaag 11040
   aatttcagtt tcaaatattg ggcctaggac agagctactt gatagtgctg aatggagagg 11100
   aagatggccc tcaaaggaga tctagaccat tccccactcc cacccactac catcatgttc 11160
   tggaccctgt cttctttgtt actgacccct gaattcttca tgtagccctg agttttctgg 11220
   agccctcact gtggaagtca agctgagctc tgtgtggatg tgaggtggta gtcctctctc 11280
   cacttggtca ccctttcccc tgggtttggt agtgtgttag tgtctcatat ctttagtgaa 11340
   cagttttgat gtttattatc tgccattctt gtctcatgaa tcttaataga ggttcagtca 11400
   tgaaagcaag gccagtgtgg gccaggtgag aaaatggccc aggtggtccc ttcctccttg 11460
   tctacctgac ttcctttgga agaagcactc tttcaggggg cttcagtgtt ccaggcttgc 11520
   tgtctaccca agagcttgct gggcacccag gcgccctcca gtaggtctgt gctgctcaga 11580
   taaactgaca tctggtttta cacaccctga gctttgctag cgtgattctg ctcttgggaa 11640
   gtagattcac tctgcagaat gggtggacag tttct 11675

<210> 26
<211> 32722
<212> DNA
<213> Homo sapiens

<220>
<221> misc_feature
<222> (1)..(32722)
<223> chromosome 1 nucleotides 33741252 to 33773973

<400> 26
ctggaagaca ggacagagat cacagactcc tcatagtcca tgctcttgct gttagctgta 60
gtttgaccac aaatgccagt aaggcataca ggtcagttat aaatgtcagg gtccactcag 120
gctggccagc caattcaggc aggaacaaga actgttacct gaaggggtcc aacataggta 180
gccagagagc tggccaaggc cagtacaact gagaacgtaa tactaggaaa aagccctacc 240
tgagggagat gcctcatggc tggggctggc catctgttgg ccccatgtca agaagctgct 300
ggggctgggt gcagtgactc ccacctgtaa tcccagcact ttgggaggtt gaagtgggtg 360
gatcactaga agccaggagt tggagaccag cctgggcaac atagtgagac ctcgtctcta 420
ctaaataaat aaataaatcc agaggatccc acctccctgc agccctttca aatggaggct 480
gtttatttgc atacagccca agttcctcct ggtgggaggt ggggttgctg ttctttctcc 540
tcactccttg aggctgcctc cagatcatag cttctttcct ccctattcat ttgaccagta 600
attgacagcc aatagctata aagagcctag gggccaggca tgctggtaag gaccttgggt 660
ggatgacatc atttcagcct cagcacgact caatgaaata ttgttgttgc tatccccatt 720
ttaccacaga gcctcagagg ctcaggttaa taacttgcta cttcatatat tgtctcaaat 780
atatgaatgg aaccaacaat atatgatgtt cctacttcat atattgtctc aaaattttta 840
agaacagctt gtttgaattt gaattcagtt aatgtcatac atcgcaactg gatttaagat 900
gaggtcattt tcactgagga gttttccaga atctgtatgt tgtcaattac atccttatgg 960
tatcattgaa cttgtttccc tgtcatctgt atttcctgta actggtattt agatccaaag 1020
gtttaaacag attcaaattt gatttttttt ttggcaagaa tacttcatag gtggttgtta 1080
tgtacttcca tcagaaggcc tataatgtct ggttgcctcg ctttttgtga tattagcagt 1140
cattgatgat cattgccaga atttatttta ataaaggttt caaaatggtg ataaattcta 1200
tccttcattt actggctgca atacttttgt aaagagactc cgcttcttaa tatttagtta 1260
ccctgaaata cattgcatac aaaaagcagg atgaatgctt gctcttccct ccttttactt 1320
actggttatc aaaatatagc tggtttctta atgttttcca taagtgacta atgagttttt 1380
aaaaaaatgt tatttattat ttatgattat ttcaatagct tttggggtac aagtggtttt 1440
tgttacatgg atgaactata tagaggcgaa ttctgagatt tcagtgcacc tgtgtgagtt 1500
agtgtacatt gcacctagtg tgtagttttt atctctaggc cccctcccag cctctgctgt 1560
ctgaatctct gaagtccatt atatcactct gtatgccttt ggatactcat agcttagctc 1620
ccgcttataa gtgagatcat acagtttttg gtttttccac tcctgtgtta cttcacttag 1680
aataacggcc tccagctcca tccaagttgc tgcaaaagat attatttcat tgattttaat 1740
ggctgaatag tatttcatgg tgtacgtaca ccacattttc tttatccact cattagctga 1800
tggacagtta gggtggttcc acatctttgc agttgtgaac tgtgctgctc taaacataca 1860
tgcgcaagtg tctttttcat ttaatgactt attttccttt ggatagatac ccagtagtga 1920
gactgctgga tcaaatggca gatctacttt aagctcttta aagaatctcc atactgcttt 1980
ccgtagaggt tgtactaatt tacattccct ccagcagtgt ctaagtgttc tcgtttcccc 2040

acatccatgt caacatctat tgttttttca cttatccata atggccattc tcgcaggaat 2100
aaggtggtat ttcattgtgt tttttttttt tctttttttc ttttttgagg cagagtcttg 2160
ctctgtcacc tacactggag tgcagtggca ccatctcagc tcactgcaac ctctgcctcc 2220
tgggttcaat cgattctctt gcctcagcct cccgagtggt tgggattaca ggtgcctgcc 2280
atcatgcctg gctaattttt gtatattttg gtagagacgg ggtttcacca tgttggtcag 2340
gctggtctcg aactcctgac ctcaggtgtc cacctgcctc ggcctcccaa agtgctggga 2400
ttacaggtgt gagccaccgt gcccggcctc attgtggttt taatttgcat ttccctgacg 2460
aatagtgatg ttgagtattt tttcatatgt ttgctggcca tccatatatt ttcttttgag 2520
aaatgtctat tcatgccctt tgcccacttt taaataggat tatttgtttt aaaaatatta 2580
ttacgaactc atagattcaa acacagttac tatttttcat ccattgcctt attattatta 2640
ttattttatt tttctagatt taggggtaca agtgcagtca tgttacatgg atatagtgtg 2700
tggtgataga gtctgggctt ttagtgtatt cattgttcaa atagtgtatg tcgtcaaatt 2760
ctcctctgtg agttggacca gcagtgacag tgccagtttc tcccacagtt ttgtcaacag 2820
tgtgttgcct agcttttgga tttttgctga tttaataggt aaggaataat ggagtgaaac 2880
tgagtacctt ttcatatatt tcaaggtttt tgcatgtctt ttctttcaca gtctttgaat 2940
gtttgcttat ggtactttta gacttatttt tctcaatttt taagagttct ctatgtataa 3000
gggatatttg catgaatgtt ccatgtatgc ttcaaaagaa agcatattct ctaatattgg 3060
gttgcagagt ttgacagata ttcatattca taaaaacttc cttatgatta ttttggttag 3120
gtcttctaca gccttacttt tgtttccttg atttgttatg aactgagagt ggtgagttaa 3180
aactgctgtt attggcatgc tccaatttgt ctttgcacct cctggtttct gctttatgac 3240
acatctgctt tcttattggg tgcatagtta tcgatatctg ttatatcttc attgtgaata 3300
atctccttta gtgctgtaaa gtgtccttcc ttgtcctctt tcatgccttt ggccctgaat 3360
tctaacttat ctggtgtgag gatggtgatt cctgttttct tgtttgcgta tgcctgttat 3420
tcctttgctt tgctttgctt tagatttcaa tacatggtat ttaagccatt ttgaatcact 3480
ttgtttaggt atgcctcttg tgatagcaca gagtcaagtt ttattttgtg agataatctg 3540
aacatcttta taattaaacc taattatatt taataatata gtggctacgt ttgttttcag 3600
gtctgtcata ttattttgta ttgtatttac ttggtgcatt tgtcatattt actggttctt 3660
tcacagtgtg tttttttgga gtacttaaaa atatatgtat tagtatttgg caaaatttat 3720
attttcttct agtaattact tttatattaa tatatttata aatgtcctta gtcctctttt 3780
ttctacttag gttctaactg ttttattaac tggttctcaa ccagtgcatc tgttgatcaa 3840
tgactttcaa cagaggtggg gggagtgtat ccattttatt ccccagaaga cactaggcaa 3900
tgtctggaag tatttttggt tgtcacaact gggagaggtg tgcatctatt gggcagaggc 3960
cagggatgcc gctaaacatc ctataacaca caggacagcc aggccctaca acaaaaaaat 4020
catctggccc aaaatgtcca tactgccaag tttgggaaac ctttagatgg tctttgactc 4080

ccacctgtta tctgtgtggc aacgagtggg gtcactttac tttccccttt ctctttctct 4140
tcccattaaa aaatcatgtt atctccactt tgtcagaacc taggacattt acatactatt 4200
cttcggtctt tattttcatc ttcattgtag atttcaatat gcagttttca attcttaaca 4260
tcagtcctgt tgttgaaatt ccttaactat catttggttg gttaaaactt gtcctcccag 4320
tagattcttc aggaagggct cctgaggaaa atattcctga attcttgaat attcattact 4380
gtgttctcat tgtcttgatg cataaaaatc actgcatata aaattcttgg tttgtacttt 4440
ctttccttca gttttgcttt gtgtgtggct gttaagaaat ctgatagcaa cctaatttaa 4500
tctggaggct cagagaatgt tttatttttc tttaaaatct tacagtttga ctggggtttg 4560
tcttggagct gagcattgca ggataatttt cccaggaacc tggtggacct ttcaatatat 4620
atgttaagtt tttcttttag ttctaaatag ttttcctaga gtataatttt aaatattaat 4680
tctgtctaat ttatttaggt tttcttcttt agggactcct attgtacata aattggcttg 4740
ctttgcctgt cttttatctt tctttctttc tttctttctt tttctttctt ttttttcttt 4800
ctcttctttc tttcctcttt ttctctttcc ttccttcctt ctttctttcc ttctttcttt 4860
atttttcttt ttcttttttt ttgagctggg ggtctcgctt ttttgcccag gctggagtgc 4920
agtggcacaa tctcagctca gtgcaacctc cacctctcga gttcaagcga ttctcctgcc 4980
tcactctccc agtagctggg actacaggca cgtgccacca tgcttggcta atttttgtat 5040
tttttggtag aactggggtt ttgccatttt ggccaggctg gtcttgaact cctggcctca 5100
agtgatccac ccgcctgcct tggcctccca aagtgccctt ttatcacttt cagtctcata 5160
ttttttagct ctttacataa cttctgtttt cttagatatt taagttttct tcctgtatgt 5220
gctttattat actttctttc tgtatgttac ccattgaaca ctttgcactt tgctcttcat 5280
ttctgagata tgcttgttct tttattgtaa gctgtcattt atttcttcct gttttatgtc 5340
tatttgtatc ctgagttttc aagcttctgg tttgtattac atgttaatat ttacaattgc 5400
ttgattatat ttaattaatg ttgggatgct ttaaaaaaag ttttcctttc ctttattttt 5460
taggggagta ttttaattag ttacaatgtt ttcttttctt tgcttttttt ctcttttcca 5520
ctcttagata tatacccaag ttacaatgtc tttactactc attttctgtt ttattctttt 5580
tttttttttt tttttttttt tattgatcat tcttgggtgt ttctcgcaga ggtggatttg 5640
gcagggtcat aggacaatag tggagggaag gtcagcagat aaacaagtga acaaaggtct 5700
ctggttttcc taggcagagg accctgcggc cttccgcagt gtttgtgtcc ctgggtactt 5760
gagattaggg agtggtgatg actcttaacg agcatgctgc cttcaagcat ctgtttaaca 5820
aagcacatct tgcaccgccc ttaatccatt taaccctgag tggacacagc acatgtttca 5880
gagagcacag ggttggagta aggtcataga tcaacaggat cccaaggcag aagaattttt 5940
cttagtacag aacaaaatga aaagtctccc atgtctactt ctttctacac agacacagca 6000
accatctgat ttctcaatct tttccccacc tttccccctt ttctattcca caaaaccgcc 6060
attgtcatca tggcccgttc tcaatgagct gttgggtaca cctcccagac ggggtggtgg 6120
ccgggcagag gggctcctca cttcccagaa ggggcggccg ggcagaggtg ccccccacct 6180

cccggacggg gcggctggcc gggcaggggc tgaccccccc ccccccacct ccctccggga 6240
cggggcggct ggctgggcgg gggctgaccc tcacctccct cccagacggg gtggctgccg 6300
ggcggagaca ttcctcactt cccagatggg gtggctgccg ggcggagggg ctcctcactt 6360
ctcagacggg gcagctgctg ggcagagggg ctcctcactt ctcagacggg gcggccaggt 6420
ggagatgctc ctcacctcct agacggggtc gcggccgggc agaggcactc ctcacatccc 6480
agacagggcg gcggggcaga ggcactcccc acatctcaga cgatgggcgg ccaggcagag 6540
acgctcctca cttcctagat gggatggcgc cgggaagagg cgctcctcac ttcccagact 6600
gggcagccag gcagaggggc tcctcacatc ccagacgatg gacggccagg cagagacgct 6660
cctcacttcc cagacggggt ggcagccggg cagaggctgc aatctcggca ctttgggagg 6720
ccaagacagg ctgctgggag gtggaggttg tagcgagccg agatcacgcc actgcactcc 6780
agcctgggca ccattgagca ctgagtgaac gagactctgt ctgcaatccc ggcacctcgg 6840
gaggccgagg ctggcggatc actcgcggtt aggagctgga gaccagcccg gccaacacag 6900
cgaaaccccg tctccaccaa aaaaatacga aaaccagtca ggcgtggcgg tgcgcgcctg 6960
caatcgcagg cactcggcag gctgaggcag gggaatcagg cagggaggtt gcagtgagct 7020
gagatggcag cagtacagtc cagcttcggc tgggcatcag agggagaccg tggaaagaga 7080
gggagaggga gaccgtgggg agagggagag ggagaccgtg gggagaggga gagggagaga 7140
gagagggaga gggagaggga gagggagaga gagagggaga gggagaggga gagggagagg 7200
gagagctgtt ttattcttat agaagctttg aagatatgtc catgaaccct ttttgtccgt 7260
gagaaaagaa atgtgtagga ttttcctaga ccagcaataa caggtgcttc tgctggagtg 7320
agggaatgga gtggtgttag tgtttatttg atttcttagt tcagggacac tctgttctct 7380
tggtataggt agatcttgtg gggtgcagct tgtctcacag gcattgtagt ttgggcttgc 7440
cccctctatc tatgccctac ggcttatgaa aacttgtcct ggcctgatgt ttggaagtct 7500
ttccccactc tatgtggtgt ccagcaatct gctcttttct tcctttcttt tttctccttt 7560
atttttagta gggacagagt tttgccatgt tggccaggat ggtctcgaac tcctggcctc 7620
aagtgatcca ccagccttgg cctcccaaag tgctgggatt acaggcatga gccactgtgc 7680
ccagcccatc tgctattttc ttataaagtg tttgcatatt tgcatgctgg tatgaacttg 7740
tcttctgaac ttagacttaa tatttgctcc cttgctctct tgattcccaa tccaaccgga 7800
gcttgtgttg gacaacagtc caattcctga ccctgaatcc ctgccaagct acctgaggtt 7860
ccagtgggag gactacaccc accctgctga ttccctgtgt ccaggagaga aatgccaggc 7920
agcgtgtcca agatggaatg cctttattaa aactgggggc caggcgcggt ggctcacgcc 7980
tgtaatccca gcactttggg aggctgaggc gggtggatca cctgaggtta ggagttcgag 8040
accagcctgg ccaacatgct gaaaccccgt ttctacaaaa aaaaaaaaat acaaaaatta 8100
gccaggcatg gtggcgcgtg cctgtaatcc cacctactcc ggtggctgag gcaggaaaat 8160
cacttgaacc cgggaggtgg aggttgcagt gggccgagat tgtgccactg cactctagcc 8220

tgggcgacag agtgagactg tgtctcaaaa aaaacaaaaa caaaaaactg ggaatgttgg 8280
tctaaatcag gttattgagg tgacaagggg ggcagctgga gccgaggtac agtgcaaaag 8340
tgaagggtag tgggagtcag aaaactgaag gtagtctgga tgaattttcc acctaaaatg 8400
atgactgcta acaaatgcat agtacattta agaaaggata ggctcctact agctcttgcc 8460
agttctttcc tcttcttcct caccactcct ctcatgactg tcttgcctgg atgaccctcc 8520
ttgatttggc ttttctctgc tatggagttt ctactgccta agcccacctg cccttagacc 8580
gcggtcctca gatcctgagc tcctcagtcc aagccaaact ccagccctga ggggagcaac 8640
ttctttgtcc ccaggttctt ggtctgagct gatgtggcct ccactataaa ttaaaaaaaa 8700
ttgtattgtg gtaaaatata tataacataa aattgatcat cttaaccatt gttaagtggc 8760
atcaagtaca ttaagtatat tcacgtaatt gtgcaatctc cagaactttt ctttctcctt 8820
tttcacaata caaacattta atgaaaattg gttagtgagt gtgtataatc cacagggggc 8880
taaatgttaa cctgggactg tagcagaaat ttaaaataga atagatgctg gagtaaatgt 8940
tatatattgt catcatctga ttcatcttct tccttcaaag attccttggc atgtttctct 9000
tcttcttccc ttatatcttt tggaataatt tcatgtcttc ttcctttagt taattcacca 9060
accctgctga gttctaattt aaaaggttta ttcttaactt catcatatac tatgtaaata 9120
tttttgcaac ttcttaacaa taacatggca ggtcatttct ttcacctgaa gcttttctat 9180
tttctatctt tgcagcgtgc ctggctttgc caagggtaca gccccaataa ccatacgaaa 9240
catcttgcag aattgtgccc tgttgttcac gctgtaagac cctactgtaa aactgcagcc 9300
aaaaggtcta acagcactgc agagtgtata tgcatggcaa ctgtgtctgc aagatgtttt 9360
tgtggaatgg tatagccaaa gctagatcta aagttggaag cgtcttctct tgttatatct 9420
gccaaagaag gggcatctgt caacaaacct gctaccacca ttccaacatg ccaatcaata 9480
ttaaaaagtc ttttgttgga gccttcttca taaagtttag gaagactccc ttttctaccc 9540
caaagccacc accatcttta catctgagtc cagtggctgt actattttcc acagccttca 9600
cagcatattc aactcaaaaa actcttccat caggagagaa tgtagagact gacaagtcat 9660
acctggtgtc aattgagctc tttgttttaa aaccagtaga acgcgtaggg cttccaggcc 9720
tccagaaact tttcatctta taaaactgaa actttatact aattaaacag taactcccca 9780
tcctttcctc cccctagtcc ctggcaacca ccattctact ttctgtatga atttgatgac 9840
tcaaggtagc ttttataaat agaatcacat agtatctggt tgtttttttt tttttttttt 9900
ttttttgtga ctggcttatt tcacttagca taacatcctc aaggttcatc catgttgcag 9960
tatgggtcag agtttccttc ttttttaagg ctgaataata ttccattgta catatgtacc 10020
acattttgtt tatccattca tctgttgatg gacatttggg ttccttctac tttttggcat 10080
ttgttaataa atgccgctat taacatatct ttttgtgacc ctgctttcaa ctcttttggg 10140
tatatattca gaagtagaac tgctagacaa tatggtaatt ctatttttga tgttttgaga 10200
aatcgccata ctgttttcca tagtgactgc acctttttac attcccacca atggggcacc 10260
agggttccaa tttctccaca tcctcaccaa cacttattgt tttttgatag tagccattct 10320

aatgggtgtg aggtggcatc tcattgtggc tttgattatt tccctaatga ttagtaacgt 10380
tgagcatttt ttcgtgtgct tgttggccat ttgtatattt tctttggaga aatgactgtt 10440
caagtctttc ataagagttc tttatagagt ctcaatatta accccttatc agatatatga 10500
tgtgcaaata ttttctccca ttctatagct tgccttttca ctctgttgat tgtatctttt 10560
gatatacaga agttgtgaat tttgatgtag tccagtttat ctgttttttc ttttgttgcc 10620
tatacgtttg atgtcatatg caggagatca ctgccaaatc taatgtcatg aagttttcca 10680
cgtacgtttt cttgtaagca ttttataatt ttagggctta tgtttaggtc tttgatccat 10740
tttgagttaa ctttttttat gtggggtatg cactatcatt tttgctcaga gtttccaaca 10800
tgtagcagtg ataacacaat gcagttaaga aatggactcc catatttcta cccttggatt 10860
ttaggcgaaa atatgtcctt ctttcccttt cccaacaaac acttgcccct agtcatgaaa 10920
gaggccataa aaatgtaaga aaaccaatgc aaaaatttat attttatttg aattcaaaaa 10980
atttaaaatt gctttcaaat tcaggaaagt accataatgc aggaccccag ggggaagcct 11040
catatacaga gacagataaa ttaaatgtat atactgcaga caagccaaga aggtgtgtag 11100
attacatatt tacagcactt gatgttttct gaaaaccata tttatacatt ttattacatt 11160
tacaaaaaag gcttccacta ccgtttacct acaataatga aaaaaaaatt tacacctttt 11220
tttttttctt ttttggtact gtacaaactt tgtataataa aaatatatct ctgtacaaaa 11280
gatttttttt gtctttgttt tacttttttt tttttcctca tgggatggtg atgggtatat 11340
ggcgtagatg gtgggtgaag ggcctcatct gccctgttcc cttccaagcc tctggaggtg 11400
gagagaaatg tcaggggtgc ggagggtgat gcccagcaga gtgctgggtg ccccaccagc 11460
aggtgctcct cgagcatctc atggaacttt acaaggagac gcaggggagg ggtggggggc 11520
gggaataggc atccgctggg tggtctgaga ggggcataag aagggggaag ggagcagtcg 11580
aaggagagga gtgtaagggg gaagggagca taagaaattg ggaggggagc ttcctgcact 11640
tgtttccagc caaggaaaga gatgagagtg tgagtgtgtc tggattggcc ctctccctcc 11700
aggcccaggg gggtgccgtg tcccagaggt ggtgtggcca ggggtaggct cctgcctctg 11760
tcagcctcca ttgtgtctgc tggaaatgga cgcctctcac tttcctctgg ccactcttaa 11820
catcagtcag atcttctaat tgttccaatc tagaatttgc ttaagaacca ggtgtcgctg 11880
tccctccgct cctgcacatt tctatttgct atttcaatat gtgacatgaa tctctgaggc 11940
cagcatgtca cacgtataca ggggcttaag cttgtggggg ccttcccgag gacagagaaa 12000
gtggccctaa atgctatact ttgattccta aactcatgaa cttcccaaca attctcatgg 12060
aattcatgaa cgacccaagc atgatatagc tgtcaaaatc aatcaaaccc actcgcaagg 12120
agctggagag gtgtgacttt cagccactct gttgttttga ttcaggacct gccaggtctt 12180
ttgctggcta tggtcagcgg ctgctgggag ccaaggagcc agcaccaacc gtgggaggcc 12240
tggggagttg aatccttgcc ccgaagctgc ctacgcggag acagcagaac ccagatagga 12300
cacacaggtg tcttgcctgg ctttccagac ctggccacag tgctactgta gagattcatc 12360

ggctcctccc ttcttagctg ccccttctag aacagggttt ctggcagctg gtgtagtgga 12420
ctttggggca gactgatctg ggtttgcatt ctgactccac acttcctaac tctgtcttta 12480
cctttgcagg cttctgtttt ctcatctgtc aaaggggaat aacaaatcct acattttggt 12540
gttattgtgc agactagaaa tactacaggt aaagtgtcag gcacagagaa taaccccagt 12600
acatgatagc cattgttttt tgggtactgg ggcaggcttg ctcccctgtt ctctcaagga 12660
ccctgctgct gcccccagac ttggcatcct gtctgggcca cagacttgtc ttctcttctc 12720
tttgtcagtt gacagccaga cttgaccttg ggctcctggc cagactcctg ccctatcctc 12780
cccggctctc ccttctttcc cagtgcttcc caggacccaa atgacttttt aatccactgg 12840
gtttagaact caataaaaca gtgtttgaaa aaaataaaga aactgatttt caatctattt 12900
ccaaagactt ttttcctgcc cttggggtaa tccttgctgc cgctgtgcag atgggccgtt 12960
catcagcaga cagctggttt ctgcacgctg accgggtttc tgcactctga ccgcatgatg 13020
ggatgtgtcc tactgttaca gtcaccgagt caccatgtcc tcacacatgg gatgggcggg 13080
gggcatattg ttttgaaagc tgaccgtacc tcccctcttt catctcttct ctgtgtgggg 13140
aacagggcat gaagcatcct tcatgcgcac ttctagtttt gaagccttta tggtctcttc 13200
cataggacgt gaatgaagga gaggtgtgtg tgtttgtgtg tgatcaggcc catacacaca 13260
cacacacaca cagatccaca agttgctttg ctattttaaa tccataaact ttcttcctct 13320
gtctgcagcc cctcttcctt ttctgccttt tcaggacacg gcaggagtcc tctccagggt 13380
aactggcata gatggcggca gggcgagggc ctgagtccca gcagtagcag acacaggcaa 13440
ggagacgaac gaagatgggc actgggcact gtgctcaggg gagcctactt cctgccccct 13500
ggggaactgg gaacaaacag atcacacctg agtttgcaaa gaaaaaagag gcagtgggag 13560
gggaggggcc gggagcagag gaggaaaagc aaagcggcaa agaaaagaag gcacaagcgg 13620
gaaagagatg agtcacctca atcttcactt tgatttttaa tcttcccagg ttggggatgg 13680
gggtaggtag ggaatacgtg cagcagaaca tgtatttttc tgtctttttc ccttgcaggg 13740
ccaacttcgc cattagacct ggaggcccag tgcttggggg ccaccatact tttaggaacc 13800
caggaaaaca ttctaatttt agtttctttc aaaatcagat gaaaaaaaaa aaatgactag 13860
gataataatg gctctgtaat aaagaatcca ggctggatta tattcatttt tgtagtgaca 13920
cagttgtgaa atctcatttt tgatactttt ttttaatgga ggaagggttg gcaaacatca 13980
tcacacagcc ctgttccccc gccctgttac tcactgaccg cacaggcctc gttgcttcct 14040
tagtagggtg tttattttaa tttcagcttt tctccttggc attatttaaa aaaaaattac 14100
catctagtca ctcatttgca atgtgtgtgt gtctgttcag ggtggctctt gctttcttta 14160
tgctccagct gtgggcagaa gtggtcagag gggccctgct ggagacctgt gggctgggca 14220
gtgggttcag agcctgctgg caaggggcca atgctgaggg ctggccagat gtggagccac 14280
caaccccagc cgacccggag gtgacagcat ggcgcacacc agcacctgga acccagcaga 14340
acccaggtgt ggagcaagag tgactgtgtg gaggtcagcg gcattcttgt ttctttctta 14400
cactgttcaa taattcatgc agtgaatcag attcatcact gatcatctcc aagccccgat 14460

ttacatcatc agaggggatt tttctttttc tcttttgcct tctattgata agagttctga 14520
gaaggaaaaa ataaagattg ggcatacaga tgaaacttga ggagtaattt tggcatgcta 14580
aaacaatccc actggaataa gggggaaccc ttatttaaat gtttgttttg gagagacgta 14640
aaaagatggg gaaaagctct ttccataaat tgtttgtatt caggagctgc atctgctgag 14700
tccctgactc actcaccatc tgggactcag gctgaaactg tgaaatcctc caggagttgt 14760
gaggcagaaa gggagtaaag cacgtcaggg gctgggcagt gataatgaca tttgctgctc 14820
ttgtaacccc ctccgcagga actgtcccca ccatctctgt tggtggcctt tgcaggcacc 14880
ctcataaaag cctgtttcct cgctgcactg ccatttgtgt gtggctggga gaatctgcca 14940
cgtgagcggc catgcacagg agcgtgggga gggagcgcaa cactagccct gggtttggct 15000
ggtggcccac ttggcggatt ttccaccgcc ctcactggga atccctacct ggctggctct 15060
gtgactcttc tccccagagt ccttcagcac aggacctgag agggggcacc tgcctatccc 15120
cagaaagcgg gatgtgtggg agaagacagt tctcagaggg gtgcagcttg gacatcgtgt 15180
tggggcaact cctgaagggt cataggtccc agaggtgagg gtgtcatcga aggaggacac 15240
agtgaggaag aggcagtgcc ttttagattc atcagttcct tcccagtggg cctgtcagat 15300
gcatgcctgt gtgcatgtat gcgtatgtgt gtgtgtgtgt gtgcatgacc gtgtacacgt 15360
gtgggtgtgt ctgccccctg tctatatttc cgggagctcc tcccctctgt tgtgctcaat 15420
gtggggaatt ttgtaagtaa acctgcctag gctatggtgc tcagttgatg ggtcaaacac 15480
caatctggat attgctgtga aggtatcttt ttagataaaa ttaactttga aatcagtaga 15540
ctttgagtct ccataatgtg tgtgggcctc gttcaatcag ctgaaggcct taagaaaaag 15600
actgaggtgc tccaaagagg aaggaattcc gtctctgact gcttttggac tcaagatgac 15660
agcatcaact cctgctggaa tttctagcct gctggcctgc cctttagatt tccaccttgc 15720
cagccaattc cttagatgaa tctctctctc taaatatata tttatttata tttctatctc 15780
tatataacat ttacatatag ctctacggag atgcgtatct atatatagat ggataggtat 15840
ggatatatct tgatatgggg tatatgtatt gcgtttatag tgtatgaatg tatatatata 15900
cacaatatac ctatatacac agatatatgt gcatctctgt atagctctac agatctccag 15960
ggcttgactt cgacccagag gatgtatata tccagttggt tctgtttctc tagagagaaa 16020
cagactagta catgccgatg ttctgccaag cccaggggtg aggagcagtt tctggagcca 16080
gtcagcctgg gattggattt cgaccccacc ccttctgagc tgtgtgacct tgctcaagtt 16140
agtttctatg cagctcagtt ttctcatctg taatggggct cccacaagct tctacctcac 16200
agggcgtgca gggattcaat atattagatc cacaaagggc ctggttcagt gcctgttaca 16260
caatgatgct caatgcacag ctctcctctt actgggtgtg tctatgtggt gtgtgcgact 16320
ccgttgggtg gagcccctgg cacgcatagg tccctgtctg tgcttgtcat ggcctgtctt 16380
cctcccacac ccctgctcac cggctgctgg aggcggggct ctcggtggcg gtggtggcgg 16440
ccaggccggg tggctatact gctgtgcaca ctgtgctgac tgtgaactcc gcctcgctgg 16500

ccatgatgtc tgtgggctgg atgttgcggt cgtacattgg gttctcaaat gtggcccgaa 16560
cattggtgtt ctcgtggcca gcatagccat tgaaaggaac tttgggtctt ctcctggcga 16620
taaaagagga agtgcccacg gcatgaaaag agcgacacag agaggcttag gggtctggtg 16680
cggggggccc tggagggaga gagggaggcc tgcctgatgc ccgccctgcc tccttcctgc 16740
acggtacctg tgcttgtaga gatagagcac gaagcccgca ataatgaggg cgatgaaagg 16800
caccaggatc gcggctgcca ctgagctgct gttggaagca aagtggcggc caatggactc 16860
ggggtctgac tccagcagcc tgaggtctgt aaagtcccaa agcagaaaag tcaagtcacg 16920
agacacagtc tgaggctccg ttggctaccc tccccgacta tacactcttg ggaagcaggg 16980
ctgccactca gggtgctcct cactcctgct cagcacggga ggcaggtgtg cccagggctg 17040
cccgcctgcc tccacgtgca cactcagtcc ttgggggcag ggtcctgcat gcatctacct 17100
tgtgctgctc agatggcacc cagaggacca tccgcagagg agcctgaaac catggctcaa 17160
cagaaactct tacaagggtg cagcccttta gaagtctcta aagttctttt ggctgtagca 17220
gccttttatc agatgaaacc ttacaggcac aacaaaatat aagacagatg aaggtgaaac 17280
tgttctggtt gaagctgaag catctccttc tgaaaggctg cctctcctcc ttcctcaccc 17340
tcccctgcag actgggaagg ctcagaagaa cacaagtaga aactgtggcc ccagtaggca 17400
ccctgctaac tgaggggctg gggctctgag aggggacagg atgtctgctg tggagccaga 17460
acaaaaactg tgattttcta aggcctttgc agcgtgcctg gccatcttgc agtaggtgaa 17520
gggtgctgtg tggaagacag tggtctcagt ccatgcagct catgagggca gagctgggac 17580
aactccgggg gaggagcagt gtgggtggga aagggtggga gcagaccgtg gctcagtcca 17640
gagaagcttt caggagcttg gagccagagg acatggagtg gactgccttg gcaggcaagg 17700
gccccggcga tgggaggtgt cagtgcctgg ggtacagaga ggctgtcacg cacctgcggc 17760
tgcctcctgt ttccccctcc cctgcttctg agaaaccaga cccctccccc caccagagtc 17820
tgaggctggg cacccagagg ctctgggcca ggacaaggct ccaaggtgtg gatgtttgac 17880
atggatgaca ggaagcacgc taggtaaaga ggccagggaa ggaccaggga gggaagtggc 17940
cttaatgctc agttttctta tctgtaaaat gggcacaaaa ccagtcttct agggctgggc 18000
tagggcagaa acgacatcac agtcaaggca ccctgagagc tctaagacac tccacagtgt 18060
cgttgtttgt tgaggcaggg gtgacaccca gggctggctg gagggtgtgg ctgggggctg 18120
tatctaagag ccaatgtccc tggagttggg ggctctcctc tccaggtctg aacaggaaac 18180
tttgtcaaag gttgagtggg ttatgcacat gtgtttgctt ttggagagat attccatggc 18240
ttttgtcaga atctcaagag gcatccctga tcccaaagag gccaaagcac ccccgcctca 18300
gactgttcaa cttccccagt cctctacctg accacggcac acacggtttc tctcccaccc 18360
acccgggccc acacttccgg gggacaagca ctagttaccc agtctttgaa agccgaactg 18420
cccaaagcct tggcccttga cagagccttg gtagatgaag gtggctccgg aggactctga 18480
cgagacctgt gatggtgggg agcacagaga gcaggtggga ggctgctgga aggatctaga 18540
actcctctct catcatacac gctgcccagc aggtcaccca ctgacctgct gctctgacac 18600

acagggtttg ttctctgccc acacctaggc aagggtgggt gtgaccgggg agcccggggt 18660
ggggcagggg caccagctac atgtatgagg taggaggaag attttatttt ctactggtca 18720
aatcacttaa tctctctgtg cctcagtttg tcatgtgcaa aatgagaata gtaacagtac 18780
ctatgtcata gggtggctgt aaggattaaa tgagttaata aagatgaaat acttcagaac 18840
acttaattta taggccttac atgcatggta gctgttatta ctgtgatgaa aaaccgcctt 18900
ttggagcatt atggaacaca cagaaccatg ctgaaaatgt cccttgcaga ttaaaggtat 18960
tggagagttc gtcacagttc taagagctat taatttttta gggggcgtag ctggggacat 19020
aactcctgct actgatgctc atgtgacaga tgcagacccc caaatgtgca gtctggggcc 19080
gcagcggctg agggcatgct gtgaggatgc ctgtgctcct gggtactccc aacctatgct 19140
ccatggctag gagcagcctc tgtctgccct ccgcagctgt ccccgcaaac acaccttgct 19200
ggctatcaaa aggctcgacg cttgtctggc gtcctaggca gtagctccag ctgttcctgg 19260
ccctctggat ccccatcctc atcttcccct ggcctgcact gctagctcca tgtcccaact 19320
ttgaccctcc tggatggtgg agtctattgt tcctgcacag cttttgggcc acgatgattg 19380
aagctggccc atacttggat tatgaagggg acctacagat acccttgggc ctaccaagga 19440
actacctgta tgtgccagcc cctggagaag ccacttggtt ctcctgatgt gtttataaag 19500
caaagtcaac tatgccacag tcctccacca gctggtcctg tccatgagaa tgcactgtga 19560
ctccctagct ctctgctact ctggtctacc atgacctctc gcttcattct atcctttctc 19620
ttctagctcc agcaatccaa gacagagaag tggaggacgt ggtaggggat gctatgctca 19680
actccactat gtgtgaaata ggaaggtatg gccagagaag tgtcactgga gatccagctt 19740
ccacctcaag aaagccctag aatagaatgt cttttccaca agggaatgca agaaagtcct 19800
atccctctgc catgtcctgg tctctcaagc tgtgttaatc actcacaatc tgcatcctca 19860
agtgtgagga gaagttcaat gtagtcatgt gagactcact gccttcttcc agctcccacc 19920
caccacatta ataccttcaa ctagcctgaa ttccctctgc ccccaacctc catcccttgc 19980
tttctgttca aacaacagaa cttactccaa ctctcttgaa tgtggtgaac ctctggactc 20040
tgagtcttct gtcttttaaa atcttttcct ttgcctattt cattagcaga tgaggatcct 20100
cttgctgctc ctaggcaggg ccccacagaa aacctcctcc caagttgcca gattgatcct 20160
aggggtttgc acaccctttt gtcctcctct tccctgccag tgatctggga gtcaggggag 20220
gaggtgagtt tgctgaactt acatggccat ctaaagccca gtgatcatct ttgaacttat 20280
tcataaagat ctccacaggc cctgtaatct ggtacacctg gagtaggaga tgaaaatctt 20340
ctttcttgta agttcctggg aaataaagtt caggtgttac acatgaaaga tatgggaaaa 20400
tttcaaaact caaatttgtg ggtttgtggg tgtaagtttc tggatggaat ttcatgtgta 20460
gatgttgata taaacattct ccacatccct ttaagtcgta agtgtagtgt tgagtgtggg 20520
tttgtgtagg ctgaacgtgg ttatatgcaa gaattattaa tatatgcata tgtatgttaa 20580
taattggatt accgggttga ggttgtgatg ctattgacac aggcatgtgt gagaatatga 20640

ttttgtcttt taatctgtcc actaatgcat agatagttca atgggtgcaa tagttcatga 20700
ggtcctgggc atgggagcat gtgaacatgt ctattatcat gtgtgaaatg agcaaatgtc 20760
atcctgcagt tgcgtaggtg tgtgcacgta tgaatgcgta gataggattt tatgtggggt 20820
tgtgcattaa tatgttcatt cactcacaca tttgcttatc tatccatcta ttcaacaaac 20880
acttattttc taaggtactt actttttctg attatagaaa gattgaaaat tgggaaaatt 20940
ttagaaaatg ctgagatgca tgaagaaaaa atgatctctc ctgatcttac taagaaataa 21000
ccacgttact gtaaattatt cctgttttct ttcttatgaa tgtaatattt tcctttaaaa 21060
cactggagtc atattttaaa tacattataa tgaatgcatt tccctcaaca ctggcataat 21120
atttcatcta gtagatatac tatcattaaa atatccattt ctctatttta gggaaatttt 21180
ctagttccag tttttatata ctacaaatgg ccttgtaatg ggaacagcct ttattcataa 21240
atgtttgact ccctccgtat cttcttatga taaaaattgt tagaaatgta attaatgggt 21300
ccaatcattt tcaaggatct tgacacaaat cgccaaattg ttttcccaga atggtaccat 21360
tttagaattt ggcgttcatg gctgaacccg acctgcacta agtgttattg tttaaaaaca 21420
ctctgagttg atggtgtaag tggaaaatga cattttgagt ttactttttt tttgatgatt 21480
gctgaaaaat attttttctt ttgtttgttg actgattgta cttgggatcc tgggaagtct 21540
ctgcccctat ttttggttca gtttagggat atcagtcttt tctctttgtt cataagagtt 21600
ctttatgtat tcaggttatt aatatttgcc atttaaacat ttccctttag tccacagtgc 21660
tttatcatgt aaagatgaat agacacttcc tcttccctga ccaccagaca agtctgagcc 21720
ttgcatgaca gtatgatgca atgccattag gctcatcctt gccaagggtg tgtgcccatc 21780
catcctcccg gctttcatag aggggcctgc tccttaccag ccaagtgcag ctccacgcca 21840
ctgtggtcga tcatggtggc attgaccttg ctgttggcaa cttggaagcc agtcactctg 21900
agcatggctg gctgcttctt cccctggtat tcataggccc ctttccacag ggaattcttg 21960
gcaaaaacat ccccaggaac tagaggaatt gagaaataga tgtgagaggg actttgtgtg 22020
tgctgccaga attggggtag cctgatgact gatcccagcc cactcactgg gattgtttcc 22080
tttccccaat gtctaccatg tgaggaaggt ggtagaatgc tctggcctct tctcgtcttg 22140
tgtctcagag tggatggata gagggaactg ataaatatcc actcccatgt tcatcgcagc 22200
atcattcaca atgtccaaga tagggaaaca acccaagtgt ccgtgaatga atgaacaaat 22260
aaagaaaagg gtatatatac atacactatg gaatactaga cagcttttaa aaagaaggaa 22320
attctttcat ttgcagcaac atgtatgaac tggaggacat tatgttaagt ggaataagcc 22380
aggcacacaa agataaatat catatgatct cacttatata tggaatctaa aaaagttgaa 22440
ctcattggag tagagagtag aatgatggtt acagaggctg gggcagagtg gagggtgatg 22500
gggagatggt caaagggtac aaagtttcca ttagactgga gatatgagtt ttggtgatct 22560
actgcatggc atagtgacta tagttaataa gaatgtattg tacatttcaa aattgctgaa 22620
agagtagttt tttcgttgag atggagtttt gctattgttg cccaggctgg agtgcaatgg 22680
tgtgatctcg gctcactgca acctctgcct cccaggttca agcaattctc ctgccttagc 22740

ctcccaagta gctgggatta caggcatgca ccaccatgcc cggctaattt tgtattttta 22800
gtagagatga ggtttcacca tgttggtcag gctggtctcg aactcctgac ctcaggtgat 22860
ccgcccacct tggcctccca aagcgctggg attacaggtg tgagccacca cacccggcca 22920
aaagagtaga ttttaaatgt tctcatcaca aaaaaatgat aattaggtga gatgatgggt 22980
atgttaatta gcttgattta tttattccat aatgtataca tatagaataa catcacattg 23040
tactctataa atatgtttat tatttgtcaa ttaaaattaa aatatttaaa aagatataga 23100
gagggaacta gaaaggagtg acagaagaag ggaggagaaa aggaaaggtg tttaaaagat 23160
ggagaaatgg aagaggggag aagagagaga aaactgacat gtcagtatag catggtgaat 23220
tggaacatgg gcaaatgaag tcagacagac ccagagtcta atcttagctc tgtcacttat 23280
cagttgtctg atcatgtgca aaagccttga ctttctccct gtataggcta tgcctgtatt 23340
cctatacatg gtggccatcc catctacctt atcagggtta ttctaggggt taaatgacta 23400
atgttgtaaa ttgtatgtta catgatgttg catgtgtaaa agcacctaat cagtgatagc 23460
tactaaaatt gtcatttatc cttgtaatca ttctctatgg caattattat gatctctatt 23520
ttactgaagc ttaagaggcg aagctcattc gtccaaggac acagctggtc agtgaccaaa 23580
atggtactca aattgagttt ctgatgccaa atctagcctc gtttcctgca taatacacat 23640
gttaatgtgg taaataaaag tagaaggttc atgaaaggat gtgatagtct ctaagaattt 23700
ggtattggtc aggccacatc tgcagtctgt gtccagttct ggatgtgata cttggaaaga 23760
ccccagacct atcttaaact gtaaccattt ccaggaagct ccctgggtat ggcctgggtc 23820
aatgtgtttg tatgaccaca cactggccag tcctatgcca ggaggggctg tagtaatcaa 23880
cagttccaga gcttccagtt ggaagttgct ggctctttct tccagatgaa gtttctgctt 23940
ccttcaggct cactttcttc tgaagtctgc ctgggctggg gaggtgggta gggaaagcca 24000
tccagattta tagatagttt tcatgccctt cctctaggca ctcagcaact ctcagcaaca 24060
cgagttttct ggctaactgt gtgaaaaaag taacaccagt ttcttgagcc aatgatctgg 24120
accatacgta ctcaaggctt gggtgagcgg tggctcccgg gcagtgttga tgggtctccc 24180
actgggccgg acctctgctg gggaaaaaga gtggaagaaa acaggttcag cttctggttc 24240
acactctgtt ggaaaaataa tgaccacctg tctgacctta ggtcaatggg ttctttgaga 24300
tcttgctttc cccttatttt ttttagacca ggaggtagac tgctttgatg ctctaatgat 24360
gctatggatc cctctcagaa taaaaaatac aaagaattac agaggaagtc cattttatta 24420
gttggagttc ttacaatatt agacaagaca ttaggatata gtggtatata tgattcttta 24480
ttactgcatt aaataacagg atctagcagt gagtcaagaa ataaccataa tcttgaagta 24540
gtcaggaaca tgaacaatat tcatgatatt ggtagcaact ggaatatata caaaagtctt 24600
ttcaatttct actggtgaca aagtcacagg tgctaacact cctaagggtt gttgtctaca 24660
ttccaaatta aaggagaggc tacatttcag taagaagtaa aggaataaaa ggtgtatatt 24720
ttcccatcca agtgcacaga ctccctgaat gtcctcattg aaaactttgc ggaggctgag 24780

gcaggagaat ggtgtgaacc tgggaggcgg agcttgcagt gagccaagat catgccactg 24840
cactccagcc tgggtgacgg agtgagactc tgtctcaaaa aaaaaaaaaa aaaaaaaaag 24900
tcctgccctc gactttgctt ttcttccaga tgcctcgcat gaaggaagaa cttctaccca 24960
tcaacggtga actctgtgag tcctggtggg ggttgcagag gagcaccttc ctcccttgga 25020
atgatgagat taagaccaag aagccctcac aggcaaagca aaggcaaaag ctttgggcct 25080
gggccagaca gccaaggttc aggtcccagc agtgccactt aatgactgcg tgaccttgac 25140
tagtaacttc accactcaga gtctttgcct cctcatttga aacctggaaa aaactgaaac 25200
atacctcaca ggtctatgct gaataaatga ggttatgaac aggaaagtca tgaccagtaa 25260
gtattaatgt cttttcttct tttgcagaga aacaatagaa aatctgcctt gtcgccatta 25320
agggcagact ccccactgtc tgccttggcg gggagcacag ctttagcaaa tctccttcaa 25380
ctctggaggt cagcctatgc ctcagtacac agaatgctgt ggagaaagtt tctgcagtga 25440
ctcaatgcaa gtgtacttct gaacaaaatg ctttatcgtc ttaggtttca taagatgaat 25500
ggagcaaaga aggagaaagc atcttatgtg cgaggtgctg tgataggcac ttcttcattt 25560
cctcctgctc ttgaaactct gaagtagaga ttattttccc cattttatag cacagaaact 25620
ttttgagagg cagagaaatg aaagggcttg ctcaagatca tacagtaatg ggttgaattc 25680
agctgcctct aaaacattat atatttttaa ataaaagtag ctataataac agataaaaat 25740
atttaagaat aaatgtaacc aaagaaatgc aagacttgta cactgaaaaa tataaagcat 25800
cattgaaaga aattaattat ctaaataaat ggaaagacat cacatgttca tggtttggaa 25860
accttaatat tgttcagatg gcaatactcc ccaaattgat ctacaggttc aatgcaattt 25920
ctgccaaaat tccagctctc tagtttgcag aaattgacaa gccaatctta aaatctgtat 25980
agaaacaaaa ggcaaaacaa taggcaaaca gtcttaaaaa agaacaaagt tggataattg 26040
atacgtccca atattttttg ttttctttca tttaaaacaa tttattttta tttagagaca 26100
aagtcttgct ctgttatcca ggctggagtg cagtggtgta atcatagctc actgtaacct 26160
ccaaatcctg ggctcaagtg aacctcctac ctcagccttc tgaatagcta agactacagg 26220
catacaccac cactcctggc taatttcttt acttttattt ttttgtagac atgtagtctc 26280
cctagggtgc ccagactggg acacttgcca atttgaaaac ctactacaaa gctaaagtaa 26340
tcagggctgg gtggtaatgg cataaagaga gtcatagaga tcaatggaat agaatagaga 26400
atccagatat aaactcatac atttatggtc aaatgatttt tgacgggagt gtcaagacaa 26460
ttcaacaggg aaagaatagt cttgccaaca aatggtgctg gaacaactgg atagccacat 26520
aaaaaagaat gagtttggat tctaacctaa tatcataaaa aattaattca aaatggatca 26580
aagacataaa tctaagaagt aaaactagaa aactctaaga ataaaacata ggcataaatc 26640
tttgtgacct tggatgtatc acagtttctt agatatgaca acaaaagcat atgcaacccc 26700
caaaatagaa aagttagatt ttatcaaaat taaagacact ggtgcttcag agaacaccaa 26760
caagagagcg aaaagacacc ccgtgaaatg ggaaaaaatt tgcaaatcat gtaccttata 26820
aaggccttgt attttagata gagaaagaac actttcaact caacaataaa aagataagtc 26880

agtaaaaaat ggacaaagga tttgaataga catttctcta aggaagacat acacataaaa 26940
aagtgctgag caccattagc cattaaggaa atataaatca tttttgcaca atgatacagg 27000
caacaaaagc aaaagtagac aaatgagatt acatcaaact gaaaagcttc tgcacagcaa 27060
aggaaacaat caacagagtg aagaggcaac ctgtggaatg ggagaaattg tttgcaaacc 27120
acacatctgg taagagatta acatctaagg aacatataga aggaactaaa acaactcgat 27180
agcaagaaaa tagataacct gatttaaaaa caggcaaagg acctgaatag acatttctga 27240
aaagaagaca tacaaatggc caacaggtat atgaaaaatg ctcagcatca ctaaccatca 27300
gggaaatgca agtgaaaccc acaatgagat atcacctcct acctgttaga aggactatta 27360
taaataagat taaagataac aagtgttggt aaggaggtgg agaaaaggaa actcttgcac 27420
attgttagtg ggaatttaag ttaatgtaac cattatggaa aacgtatgaa ggttcctcaa 27480
aaaattaaaa atagaactac catatgatac agcaatacca ttatggagta tatatccaaa 27540
ggaaacaaaa tcagtaagtc aaagagatat ctgcactctc atgtttattg aggcattatt 27600
cataatagcc aagatacgga aacaacctaa gtgtccatca gtggatgaat ggataaagaa 27660
aacatggaat atatacacaa tggaatacta tgcagcctta aaaaagaaag aaattctgcc 27720
atttgtgacc acagggataa cctggaggac attatgttaa gtgaaataag ccaggtacgg 27780
taagacaaat actgcacaat ctcacttatc tgtggaatct aaagtaattg aactaattaa 27840
tagaagtaga gagtagaata gtggatacca ggggctaggg tggggtggta ctggggggtt 27900
ggaattggaa agatgttggc caaagggcac agaatttcag ttagacaggg aataagttca 27960
agatctcttg tacatcatgg tgaccatagt taataacaat gcactgtgta cttgaaaatt 28020
actgagagta gattttgtgt tcttaccaca aaaaatggca agtatatgaa gttaatggat 28080
atgttaatta acatgacttt gccactccac aatgtataca tatatcaaaa catcatgctg 28140
tacactctac atatatacaa cttttatttg tcaattaaaa aataattgga tgaaaattca 28200
aacaaacaat aaaaaacacc acaatgagat accactttat acccactgat catggataca 28260
ctaaaacagg atggacaagt gtggggaagg atgtcgagaa attggaccct gatgcattgc 28320
tggtgggaat aacatggtgc aagagttttg gaaaactttg gcaattcctc aaaatattac 28380
acatagagtt actgttttcc tagatatgtc cccaggagaa ctgaaaacat attaaaacaa 28440
aacatgtaca tgtatgttca cagaagtatt atttgtaata gccaaaaagt ggaaacagcc 28500
taaacagcca tcaacaggtg aatggataaa caaaaagtga tgtccatatg atgagatatg 28560
attcacgcat aagaaggaaa gaaattccga cagatgccac aacatggtta aatcttgaaa 28620
acattatgct aagggaaata agccagacac aaaagaacaa atattgtatg attccactaa 28680
catgaggtac ttagaatagg caaatttaca gaagcaggaa gtatgatagc agttactatg 28740
ggctaagaga agggggaaat gggcagttat tgtttaatgg gtatggagtt tctgttcgag 28800
ataatgaaaa aattctggaa atggatagtg gtgatggctg tgcaatatca taaacataac 28860
taatgccact gaattgtaca cttaaaattg tatattatgt atattctatc acaataaaaa 28920

cttaaatgta ctttaataaa tgtactttat atcactactg tatgtggaaa acaaaagaaa 28980
tttccaagtg ataccatgaa aataaaacac cgttagtaag ttctagctct gatatagtct 29040
ctgaccctga aggcttccct ctgagtggca ggatagcaaa atagtcatag atcctaaagt 29100
tagaccatca gggtatagat cttcagcact taacctttct cagctgggga ccttggacac 29160
attaattaac ttctctatgc cctagtttcc tcatatatta aatagcctca taggatcttg 29220
tgaagattaa atgagttgaa acatacaaag catttagcac aatgcctggt atctggtgag 29280
gtgagtgcct atcagagagt ttaatgctat tccagcactg aactaatcgt ttaaccttaa 29340
tcctttccct caagactgga ctcagacatc accttctcca ggaagtcctc cctgacttct 29400
cctttccccc actttagtgc ccttcctcct cattcctaca gcatttggca catctccatg 29460
ttcctccaag tactccaaat ataaaatctg ataacgtgtc tgtcttccac atggcagggt 29520
tgtgtctttc ccctctgaat ccctcatgcc tagcgcaagc atggggccac atgaactctc 29580
aataaataga gaacgagtga atgaagaaga ggggaaggga ggtctgggac atcctggtaa 29640
ccccaaggga cctacaggcg ggtgctgttt tatcaagggt gcaatatgtg taatgcgtca 29700
ctccagtttg gtggaagaaa gcactttcct ctgcctgatg atgaaatgtg atcctgtgag 29760
tgtttcagcc caggcaaaag gggcaaccct tggggtgggt tgagtatgca cctttttaga 29820
ccgctctgct ttgctcttct agactcaaaa ccttagaaaa tcagaagcgg gatccagctc 29880
cttgaaacgc aattccttcc ttctcttttg ccctctttca gctcaagttc agccagcact 29940
ttcagcctcc cgccctggag agcttccccg agcaggcaca ctcacccagg cagatgggcg 30000
gcttgcctgt ccagctgcca tccgccttgc aggtgcggtg ctcggagcca cccttgaggg 30060
agaagccctc ctggcaggag tagatgagcg tgtagcccat ggagggcaaa tccagggccc 30120
cgacgttggc atgcgttggc gtctctggct gcctgcagtg gtgggctgga tgagaggaaa 30180
gaccctgttg gactggagga gattaggggc ttcaggggcc ctttcgacca ttcccctgtt 30240
cctagataga atatcctctt cctttccctt ccagtccctt tcatgccaag catccccctc 30300
cctaatcctc caccctaacc caagctctgt gtctagagga atggccaaga tggagatcat 30360
gtggatatcg gcttggttga ctctgagttg gggaacttta agcctgcagc acttcctgag 30420
gggtaaagga gtaactgggt caaaagcttg atatctccat cctgaggcct ggccttgcag 30480
ttgttagttt ttgctgctgc cactggatag tttttctttt gctaaggcat agggattgcc 30540
ttccaatatc agaaaggctt ttgtgtggaa gtcttctgtg aggccccaaa gtgtaaggac 30600
tacaaagaga ccgatgtcgg ttcgcatggg gagttgtgaa ctccctgtca ttcagagcat 30660
tcaaacatga cccagatgcc cagctggggc aagaggaatt ttcttgggat gtgggtgaag 30720
tctgcactca cggacacagt caggtggggt tccactccag gtcaggtttg ggaggcaggt 30780
cctggtggtg gagccctgaa gcaggtagcc tttttgacaa cggaagagga ctgtgcttcc 30840
aacctgcggc aagatacaaa gtcccatcag ccccttcctt tcagcggtgc ttcctacgtc 30900
tgtcccttga ccactttcac atggcccaac tcctcccgca ctgttgcctg caaaactctt 30960
tttctgattc tccgcctggt taaattttct tcttcctttg aggcagttga taggatgcct 31020

cctccacaag gcctcatcta ataccctgaa caaagttgta atattacact ctgttgtgga 31080
cacggtggct gattcagagt cagaagaccc tggatccgag ttctggcttc tgcatttact 31140
cagggcaggc tgcatggaac tctgtaaacc ttagtcttcc taaggataca gtggggatga 31200
ccatccttgc cacaatcaca ctggaatgtt gtgacactca aatgtgatgg cggatttgac 31260
agcattttac aaaatgtgaa gaagagtgca aattaaatat tgtgtgtgtg ttcctttcct 31320
caatgaaatt gcaaattctt taaggatggg agacttgtct ttacagatat ggcccccata 31380
gaatctgttg aacgaactgc caccctccct ccctgctctg ctcagttatg ttccctacct 31440
taaccctcca ccagcccctc actgtacaac tcgacacccc gcaaccacgg gctccatctc 31500
tccctaccca atccacttgg tgtaatgcgg ctagatctgt ttcctatact gcaactcgga 31560
ttatactact tcccagttca aacacttatg tgtccctatt acctactgga gcatgtgcaa 31620
atgcaatgtg taactcttaa attccactcc cttcttccat gctgaacact tttttgtctt 31680
ggatttcatc atttgactta gcaaatggca cctttatcca tgcagttgtg caaataaaaa 31740
acctcaagtc atctttgatc ccttcgaccc cacaatccaa cccatcaatg agtcctcttg 31800
gttctgccta caaaatgtat cccgaacaag cgttcaggat cacttctcac cttctttttc 31860
cctggagtca tgacaacagt gtcctccttg cctaagtgca tctattctca actccctgat 31920
attcattctc tactcagcag cctgagtaat atttcaaatg aagaatcaga ttgtgtcacc 31980
ttcaaatggc attttagata aaatccaaaa tacttatcat ggcttcaagg ccctgcatga 32040
cctgtcccat gctgccgcct ttgcaaaaat tatgtgatat ccgacataca caaaataata 32100
tatggaacac atataaagta taataataaa atggatattt tagattgaat ccaccatgca 32160
acttaagagc tatggacatg gttgtaatgt tctaggggct ctttccctgc tgtgttcctc 32220
aacacccctc cgtagtaatg gctgtcttga aatttgtgtt aatttccttg ccttgttaaa 32280
aaacaatcct atcacatatg tgcatacata cctctaaaca atttagtaag tttttgctgg 32340
ttttaatatt tgtaaaagta gtgtcatgcc acctttgtca cttgatagta tgtttctaag 32400
attcatccat gtcatgcaga gctgtaattc attcattttc tttgctgtat aataatccat 32460
cacgtgacta tacccacaaa tttcctgttt cttggtgaca tgtgcaagag tgcctctggg 32520
atatgtttgc agaggtggaa cttttgggtc atagagtgtg tgaatgttta actttacaca 32580
ccaggaccaa gctgttttcc aacctagcct cactcttgac tacatttcct tatcctccct 32640
cacggggctg cagacttgct agctggccta ctttccaggc ccccaggctg ctaagcactt 32700
ccctcagcac tcctttgcac ca 32722

<210> 27
<211> 13229
<212> DNA
<213> Homo sapiens

<220>
<221> misc_feature
<222> (1)..(13229)

<223>  chromosome 1 nucleotides 224909378 to 224922606

<400>  27
aggaagaaga ggaggtgggg aggaggggag gaggaagagg agggcaggag gaagaagagg      60

gaggaaggag gggaaggaga gacccaggtt taccctactc taggctgtgg ggatgtgtgg     120

gcctctggcc accccactta caaaatgctt tagctatcat caggatcatc tacataacat     180

gcaaaatgaa aatgaggggt cccttgtcca aaattcagta agaatgtcat gatggggaaa     240

gcagaacttc accaagctcg gggcccttct catgccctgt gactgctcag ccaggaaatg     300

ggccctgcct cctggggagg aaatggcccc cgttaggaga cagcaggtga ccccaggcct     360

gcaggcctct gtggcctgct cacagccaat gaccagaggc tcctcaatcc tagggcaggg     420

gagggctcaa ggccaccggc acctgtggct ctctttttgg ggctcagaga agtcccctgg     480

aaaggcctgg caggagcggt gaggatgcaa ccagacctca gccttctgca tgttgttcag     540

agtctggcct tgcttcctgt ccctgcagtg ctgtctgagc cagtcagtgg ccacttgccc     600

tgcccagccc agccttctcc gcaggaggcc aagccaccca gggccagcac tgtctgatcc     660

tcagctcagc ccacatgccc cctggggtct gcttcctgtc ttctttccag ccaattcccg     720

cccctccaaa ccaatgttat atttctcagt gcaatttggg gtgggtagaa gaaagggttg     780

tgacgctggg ggcagacact tgtatgtcgt gaggactgca ggtcactggc agccctggtg     840

acccccactc ccatccacac atggcaccat ctgcagcgca cccgggctca tattctctca     900

ggcctgtgga gttctgcagg caacttcttg ctccacacac ctgtccccac gccaccttag     960

gcccgcaagg gtgtcagctt ctgctattag ggactttgca gaagaatgac tgaggatgct    1020

caggacttag ctaactcctg tgtgtatttt aaaatgcaac ccagaaaccc tcttcccagg    1080

aagccttcca ttgcccctcc tgggtgcttc caaagagccc tgggcacgct tccatcccag    1140

ttcttctctt taacacaaga aagggacctt ggtttaccct ggtgggcgct cagagacccc    1200

cagcaggttt cctgagtgcc tggctgggca ggtgccaagc ctcacagcat ccaggaggag    1260

ggagatcaaa ctcagcaggt gtgggagggg ctgcacttgc ccatctggag cccggtcaga    1320

ggctgctgaa gaaatttcac attctgggca ggtgcgggct ccacttcatt tccttatcag    1380

ggccctgaca ttcctctccc ggaagccaca gctgtgctgg gacaacttca gagaagcaag    1440

gaggacttca ggaaggcagc tccttccagt cccagtgtac cccaagacag ctgcccttcc    1500

tccagccccc ttcttgctga tttggccagg cccatagtgg tttcaccacc ttgcaaatta    1560

agggctgaag caacatacat actcctctcc ccctccatca agattttaaa aggcagccca    1620

cggagttgga tgttgttcta gaaggcagga tgatgacgca gaatgcaatt caagtgtaga    1680

ggttggaacg aacctttgtt ctgcagcctt ccagctgcag catgggcaaa cctcggcgta    1740

cactgggcac ggcaggtatc aatgcacaca ccctgccctc cccatgcagt caccgtgaga    1800

gcctgagcag gtgacaggca caaacagctg ctctgtagac tgtcacagac aggcaaacct    1860

tccaggctca ctttggtttt ttgcttctgt gtctattttc ttaaattgaa atgatttttt    1920

cctccaaggc atagcagatt ggcaagcatc caactaggca ctttcagagg gcctgatcgt    1980

ggccagtggt gatcagatga ggctaactgt gtgattaatt gcttacaaag cttcccgtcc 2040
catgcccatc gcagctccgg gaactcatgg ggcagttcct taggccccca gatgtgtaac 2100
ttggaaagag tcagagccca agtgagtcaa tatcagtttc tggttcaagt cactgacgtt 2160
gcaaaaagaa gaatgatttc ccaaagcatc ttatttcatg tatgtggaac tctgctaggc 2220
actgccaaag aaagatattt tcaaggtgaa taataagatt gcttggcaaa tttcattttc 2280
tagagggggt ttacattctc ttcttagcct ctttttcacc tgacttgttc cctcaatttg 2340
ctactttttc ctccagcact tctcttttta tttaaatagt ttgatcactt ggcttttatc 2400
tttagataat cgtaaagaaa gagtctccct ttgttgttgt tgttgggtaa aagagatgag 2460
gggaaatctc taaaatatca gaaatgggaa atgatccctg gaccctctaa cactccgaaa 2520
acggtattgc cagccgaact ctgctgcgtg tgtgcaaaga gatcacgtta catgtctaac 2580
atagtgttag atactaataa gctaatagct caggtaactc tcacaacact gatattgttg 2640
tctccatttt gcaaatgatg aaacatgttg agaggggttt agtaacttgc aaaaggtcac 2700
acagcttcca ggtttgtgcc acttaattcc aaggattttg tctcagccaa caggattact 2760
gggctcccat catctctctc ggacctgggg ctatctgctt cccccctatt tataaaatag 2820
cctcacataa acagcaaggg ctttgtcaga agagtgacct ttcagatggg ggaggcctga 2880
acaaaggcct actctcccct ccctccaagt ctgttcccag aacgtcctta ctgacacacg 2940
ctcggggaag gcacatctaa caaagcgtcg aaagtggatg aagaacgccc ctgccagggg 3000
agaatttgtt ctgtgtcaaa gcctttgttt tgctcttgca aaagtctgag tgagagtcat 3060
tgagttttcc atgtttggaa aaaatccttt tttccttcct tcttcatttg gtctttctaa 3120
aaaagcagtt agggtttccc tagtgatcat tatttccttc actggggaaa agcctggtgc 3180
ttctcctagg ctgcagagtg gaggcggtgg ccaactttgt tcattgtatt ccccctggca 3240
gggcccagct gggttggcgg cagctgggta caagcccagg caggtagggg cccctggctc 3300
aatagctccc tcctgtagcc ctggttagaa acctggctgg ggccaaaagc gaaacctgcc 3360
cctgccaaaa aggagctggg agggaaggag gggaagggct ttcaaaacca agacaccaag 3420
gatcggaaaa attaagccca aggacttggc aggacaggta gctccagcta aaaataaaag 3480
tctggatgta agactaactg cctttccccc tttaacaaac aagaaaatcc ccctaaatgc 3540
caaatgcaaa agcactttca gaaaagaccc gagtaaggct ttatttcatc ccccgtctcc 3600
acccgccgtg tgatgttccc gcaggctgga tggcaccgaa catcctccac agcgctgctc 3660
accaggcacg aggttcttct cccccgccct ttatggaacc catgagcgta gggtagggat 3720
tactgagcgc tgtcgggttg gtgaagtccc tccttcagaa gcaactgctg ggcctggggc 3780
aggagaacga ggatgatggg tccttaccag cccctgcaga ccgggacccc ctgcaggggc 3840
tacacgaacc agctcagtga actagctagc taaactagct ccaggtgagc gctctgtccc 3900
ctccacaccc aatcaggctg cgagggctgc cgggtccctg gtttgcagtg cgtagacctg 3960
gtcgggctga aggctggagc tcccaacctg gctgactctt tttgagggac gccaccatgg 4020
tcaatcgacc cctagcagca cattctggcc aaaaccagga aaaccctgag tgactgccca 4080

gctgtctcct gattagctgc cttcttcctg ttgcaactag aggcctagtt tctaggccaa 4140
gggcagcacc cagactggca cccaggggcc ctccatcatg gccactcttc ctcttccacc 4200
ataagcccta aaccctgcca aaggaggttg cccaaggccc agacggcaag gacagggctc 4260
caggagcagc ccagggcctc accaaggctc cctgaccacc cataagaggg agcagggtcc 4320
ctccgggata cagagccacc cccagatacg gccccacact cactctctgg cgtgcccact 4380
ccaatccgtc agggcaagcc atcccatacc tgccccacct ccaggtctgc tgtgcaggtg 4440
ctctgcccag aaagctctcc ctcctccctt ctgtgtatcc acatcctacc tctccttcca 4500
gcagatcccc tggaatctgc aagctggccc taccttgtcc ctgaagtctc tcaggccact 4560
ctagccccac gccactcctg gaatcctccc tgaagctgga gggagatgca ggatgtggat 4620
acgcagaagg gaggagggag agcttcccag cagggcacct gcacagcaga cctgcaggtg 4680
gggcagggaa gggaaaggaa gctggggtgc ttcgtgattc ttgtgtgcca taccatgtct 4740
cccagtggca ggaaggctcc tggggaactg gggatctgtc ctaaccttct ctatgtcccc 4800
caggctgcac agccggcagg aatccaatac tcccggcatc tggctgataa gaggaccctg 4860
cggacccagc acatgctaag ggcccaggcc tggaagataa agcacaccta caccaaggct 4920
cacactgctt tatgttcacc tgctcacacc tggatttttc tagagacttc tccaagtaac 4980
aagagggggc atatttgtgg gaatttcctg tttggtctgc ttgggtttct ctccaggacc 5040
ccacggctgg cagcctggct ggccccaggc cccaggggtc tttttcactc aagggtttgg 5100
agggcaacct ttcactcagg ttagtcactg tttgctcact ctcccatccc tcaatctgtg 5160
atgcccttgc tggcaggcag gggacatctc ttcgtgggca tccccagtat cctgcaggag 5220
ccagggacac tggagacacc agcaaagaat gctcttcctt agcgactctt tcaggtctgc 5280
actccttccc tcatccacac tctataggag ctggcatcaa aggggcctgc aatcttgcta 5340
aaccagagtc tttttttggt tactcaagaa tcgaggtgtc cttcacccaa aatggaaatg 5400
ttcctaaccc tccctattgg gcagggccca gcaaatgctc catctctggt gagaaggtca 5460
ttaacttggc tcttccctcc tcctctctcc tggattccat cctcttaaga actaaaaata 5520
atgaagggct agaggagaga ggcgagagag gaagaaggca caggaaatgc tgtgctgaac 5580
agaggcctcg cagagctcac accaggggcc tcgcagagct cacaccaggg gcctctccag 5640
gcctcaaagg ctgctgggcc cggaggctag gaggggacca acaataggag gaaacaggga 5700
gtggccaaag gggctgggga agagcacgag gcggatgggc agcctttcga ggctgacttc 5760
ctaatggata aagcccagag gacagaaggg ctcagccttt cctctgggaa agggcgcctg 5820
ggctctgccc agctggttaa gggccacccc tgcaacacgc agcagatcct ctcccgccct 5880
gcgctcatcc actcctcccc agagatttta tttttacttt ggtaaatgca aaacaacaag 5940
gccaagttac agagggtgga aatttaccaa aggccttcac tgatcggcag acaattggca 6000
ctgaagttct ggaacctccg agcacccgca gagtccatct tgggccgctc tctcagaggc 6060
gccagccagg ggactcagcc ccatctccac ccccaacaca ccttcttggg aacagcttcc 6120

tggtctgggg agctagagtt gcttaatcct tcctttatag cccacagaga ggtaaggcag 6180
ggcaggagcg cagcccatag catgcgagct gttctagtgc cagttcaaga aatagctcgt 6240
gtgcttacca gagccagaca cagggctgag tatttgctat gtgtgtgtga taagagtctc 6300
tcaacccatc acagaggtgc tgtcactgtc ccacttcaca gatgggaaca ctgaggcaca 6360
gagtcatgag acaacttgcc caaggttctg tacatgtgta gtcaggcagg ctggctgcgg 6420
atccaagctc ttcatcactc tgctgggctg cctcatttcc tgtcatgtgg aacaggtaac 6480
attatagcca gataaactga gatttagatc ctgccactcc tggctggtgg cactaggcaa 6540
gttaccaaac ctctctgagc ttcagtttcc tcatctataa aatggaaata ccatgtatcc 6600
taaagtattg ttttcagggt taaattaaat tctctgtgtg cagtgcctgg tacatacaca 6660
agagcactca acagaggcag atacattttg catacctctc acgtatactg tctggtctga 6720
ccccctcaag gtcaagagcc tatggatcgg atgaactgta tgggagacag ctctgtatga 6780
gcccagcaga tgttagcagt agggtctgac cacctcctct tctatcccta atcccagcct 6840
tgaatcccac ctttccacaa ggaaagggca gaaggtgtag gccacagcca ggaggaagtg 6900
gatccccaat gaaaagtcct attccttgac agaaacagag tctttctgga actccaaaat 6960
gcccaggagc tactttctag tcgggtgtga gagggagtgc acggacgatg aggcagcctg 7020
gcaggagagc cactagaggc tctcgtgttt cctgaaaatc cccccaccca aacctccccc 7080
agggacctgg gatctgaaat aagtaactct gcctgttccc cgggagactg agctgagcaa 7140
cagggacaca ggcagctctg ccaggtggcc acaagtggaa acagacttct tcatcccatg 7200
ctggaggatg acatttacaa agccaggaat ctaaaacgaa gttttctgca gaaagttcaa 7260
gaatctaaaa tgccatcttc tctccccaaa gctctcccag gcacagccct gctaaaggaa 7320
acacacttac ttctgtgtga acagctcttt agtaacccct ggttcttttt cagagattcc 7380
tgagattgaa aaaacttcag gttatacatt ttaaaacctt aggatactac tggatatagt 7440
agaagggaca agatttcctc aacctccaat tgctaaaagt ttacctctct aggccaagaa 7500
cattaatgga caaaacactg ccttttaatt agtttctggg aatgcaaaaa agaccttgta 7560
agagatttgg ttaagggggt catcctggtc taaatccaaa gccagagttt tgggaactcc 7620
cacttaagga atttccaaca aggaaacttt tgagggagga cttgaatacc acatgacggc 7680
ttataagcag ttatcaagct acactggtgc aattcatatt tctgacagat tatgctgatg 7740
tgcctgtggc taaatggcac tcagcaagtg agaatgcaga ctcgcagaga gaaccagaaa 7800
ccccaagctg ctttccaaag aagcatctgc agccctgccc cagcagtggc caatgcgcag 7860
ctctgaattc tgctatgagt cacccagctt ccctctcctg accagagctg ttcagggtag 7920
agaccctgtg tctcggttca gcagtcatgc atctgttcac gtgcagacct ctggaagcac 7980
aggaaacggg ttgccaaaaa gggacaccag agaaccccca agtatgagca agactcatca 8040
gctctcaggg ctcagctgtc ccccgggtca tcttgacggt atgtgtgacg atatgtgtga 8100
ttttcataaa cagattgact catcgccctt ccccgtgcat ctctgtcaac caggccctgc 8160
tagtggcagt gggtgcttac ttagcacttt ccccacgtga caggagcctc ccagggcccc 8220

agaaaggcag gcaccagtct cgcctcctat taccagagca gcaaacagag gcaaaggtgc 8280
gctcctaggt ttgtgtccca atcggggcag accttggggt cttgcccaaa ctcctaccct 8340
ggaagcctgc acgccctact gcagccaggt tctcccgccc accgggagca gggacaccct 8400
gaaactgttt ccttcacttc ttgctttagg ctacaatgat tcaacatttc tttcttttct 8460
ttttttttta tgagacaggg tcttgttctg tcacccaggc tggagtggag tcagtggcgc 8520
aatcacggct cacctacagc cttaactttc tgggctcaag caacccagca atcctcctgc 8580
ctcagcctcc ctagtagctg ggactatatg catataccac catgcctgaa taatttttgt 8640
attttttggt agagatgggg ttttgccgtg ttgcccaggc tggtcttgaa gtcctggact 8700
tcaggtgacc aaagaagtga tccacccccc acggcctctc aaagtgctgg gattacaggc 8760
gtgagccacc acgtccaggt taacatgtct tttatattga ggtattttgc agcagctttt 8820
gggatcatta cagacagcat gggttttcac aaagtgaggt gaggatctcc tatgagacag 8880
gcaaagaagg gctgcacccc aggtccttcc tgtcttctcc ctcagattta cagagaagca 8940
aaaccaagaa acagagcatc gacatccgta gaggctggtc cagttttcta tctaagagat 9000
cttaaagctt ggcccccaca aaagtcactc tcttgcctgc acagcttaca gaaggctcct 9060
ggttctctta gacttgtttc atctccttcc tactgagtta gaggaaatga aacattattg 9120
agggaatgca agcaggttat ctgaaaaatg tattctggaa tgatagaggc tgaagaacgg 9180
gcttaaatga cttctgggag tccttccagt cagtgagcct gaacacaggc atgagagagg 9240
cacttggagg aatggtctgc tttgtgcagt caggatttag gtctcactaa gcttggggcc 9300
tctaggctca ggaggcagga gctgaacgag agcccctgac catctccaaa gagagcaaac 9360
ctcctttgca atgtccatct gctgtgtgcc gggcagtaca cgtatcgtgg tgggacgagg 9420
ggagagcctt ggggactctg tcccagggag aaggactcca gactctcaca atgaccacgg 9480
cctcttgcag gctcagcccc tccaaggtct ctggggagag ctcacattta ggaactgtta 9540
acatcatttc tagggaacag aaccagctgg ctgacttatt ccccttaact ttcaatgcta 9600
ttattagggc acatctgtct cggagtgaag tcaccaagga aaaacccagc tgaagaaaat 9660
attttcaggc agtcgtcatt tgaatcttcg gaaaaggaag cgagccgata aactgccaaa 9720
gagccccggg attcaaggtg gccaaagagg cgcatggaaa gtatttgcct tgggaatgtg 9780
ggcagctgag ctatttacac agtgtgtggc cagcacgacg caaatggacc agaagctcct 9840
agaactgttt cctcttccct ttcctttccc cctttccctt aacaggatag ccagccagca 9900
gaggctccct gtaaacctga gtcaggagga aaggccgtgg gtgagtgact agcagccagg 9960
tgatataggg cagaggccag cagactcacc ttgtttggct acattttttt tctaaattag 10020
ttgctaacat ttaaaaatca gattccacac aaaaatccga attcccaact tctctttgaa 10080
atcctgggcc tgtaatcccg gacagcggcc cctgcctggg gctgagcagc agccacttaa 10140
gatgggacct tctcttcccc tgtcactggg tcccgctgac acaggtcacc tgcccggccc 10200
ctggtggcat ttgagtttga gccgctggat ttagagacaa aggggaaagt gctgaggaaa 10260

gccaaagacc tccactgtag tctactcact ggccatgtca acgcgggcaa gtcacctact 10320
ttgctgagct gctgtttctc cctctgcaaa cagaaatgaa aatgcacagc aaggcctcat 10380
ccccaaaact gtagggagga ctaggtagga aaacacggga catgcttgaa aacctgaaca 10440
cacaccatgc aagtaagatg tggcccttcg ggtctgaaag atgaaataga acaccaagac 10500
aaaacgggcc tctctgatga gatgagtagc ggaggaaacg acagatcaca caaaagctac 10560
ctcggtggtc accggcgggg gctgagatgg aagccaggat ttctaaacca cccagcccca 10620
ccaacctggc cctgcctgcg gtaccacctc cccacccaga gaagtggaac atcactgtgg 10680
tcgtggctga gcagtacctg actctgacca acctctgcct ctaattagtt acaacacaat 10740
ttagaacccg ggatggttgg cgtttgggac ttgcagccac ggcccagccc aggtgaggcg 10800
aggagacact cacggaagca cctgtggtgg ggccaggagg aagcctagca tcctggcaat 10860
gaaactcaac actcaaaatg ctaggagtaa atcacagggc actgtggcct ctggggattt 10920
cacagtgtag cggccaactg cattttgagg ggtgacccaa aagctcctgg ccatctgcgg 10980
tcttccccac cagcctgcct aaaaatgaaa gaaatgacag acatgaccgc agagggctac 11040
caatgggctt ctcagaagat aaagagaggt ttaaatgata aaggcatcag tcctatatct 11100
gccccaaagc cctccttaag gccacatgtg gagagcgagc agctgtgata ccagaaggga 11160
gcacatggca cggtgggatc tggtgcctgt cgtggcagca atgactcagg ccacccaatc 11220
agggcaagac tcagcaaggt ctgccagctc ccatcaagcc agaggcgctc agagggagaa 11280
gctgtaagtg cttgctgctc tcaggagcaa gaggtcatct tgcttcttac cagctcctta 11340
ccaaggcatc atatgtgacg gacatggctc cctggtgtcc aggggctcca ggggctctgg 11400
gaagatagag catgtgcagc gtcatgcccc agcacactga ccccacagat ctaatgggac 11460
catgctgaac atatgaggat cacaaattcc atgggagacc ctaagatcta ccctcaggga 11520
cagctaaggc gattctggaa cagggctgcc aaaacagcca gtcaatcact aaagttccta 11580
atttaccacc ctgacaactc tccctacggc tagcacccct tgctcttcct atctctccag 11640
gatcacaaca gcctgtcctg ccttgggtgt gccttcctgc aacccactct ccactcatca 11700
gccttgcaga tctttctaaa acatcaatct gatcatgcca atttcatcat gtccctcccc 11760
tgctttaaac cttctgtggc ttccctaaag cctgcaggac aaagaccaca gaccacacat 11820
ggactctcaa gggctctcgc ctctccccac caatgcgtcc cttcactccc tgggctgttg 11880
caaaactgat ctggttgtgt ttgccaacac acaccacact gcttcatgct cctgaacctt 11940
ttgtgctggc ccctctggct ggaagaccct tcccctaacc taccgagaga actcttcttc 12000
attctttacc atgcgcaatt atcacctcct ctttgcactt tttgcccaat gcactcaaaa 12060
aggctaatcg cttctcccct atacctcaca cacgcatctg cttctctgct gagaggacat 12120
tcacgtgatc tggtgatgac atgagtgtta cccccactag agtgagcatt ccttgaggac 12180
tggaaatggc cagttcattg ctgaacccct aggccctacc tggcacactg ctttcccaga 12240
gttgggaatc gataaatata ggctaacata aacatgtggg aaccccgcca agacccaaga 12300
gtctggttga gaaagcggaa gagtgctcag aatgcctagc ccttactgtg gggtctggct 12360

taagggcggg gtttctgaag cagaaaggta agttaagtaa gggttgcctc acacccagag 12420

gccaagaacc agaggcagca gcatccagac tcagtgaggt cccacaacca gggccagccc 12480

atcagggaga gaggcaaaca agaggagctc tggaaccagg acccaggcag gtaacctggg 12540

ctggctcctg gactcctttg tccaatcaga ccacccacat tttctgacat cagtgctgtc 12600

cctggatgca ctgggcacag ctgtgagtgc tttctcctgc agatctggga tagggctaca 12660

ggaattcaga agaggaagct attactttgg gagaaaaagg gaaaaaatta aaaattaagg 12720

aagcttcatg gaaaaggtga catttaagct aagtcttaaa aagatgagga aaatgtgtgt 12780

gtgtgtgtgt gtgtgtgtgt gcgcgcgcgc gtgcgaagtg gaggttggta tttcaggatt 12840

gagaaaagca taagcaaaag cctagaagca aaaaagcacc aacagcacag ccaacagcac 12900

agccaacagc acagccaaca gcttggctca gctcagcact aagggcctgg cacggagcag 12960

tttgccccag agggaggcaa aaagagggaa aattgtctgt aaggaattta aaacaatact 13020

gaaactgatg ggctttttac catgaccatg agctggcgat tctgatttaa acataatcca 13080

acacctcccc caacaacctt ttgtctaatt ctgaacaatt gctgcattat tcttgacttt 13140

taataatata tgtaagcttc aaattcacac agactaatta ccttttcata aacattacat 13200

tctctatgga agttaattca aataactca 13229

<210> 28
<211> 1976
<212> DNA
<213> Homo sapiens

<220>
<221> misc_feature
<222> (1)..(1976)
<223> chromosome 1 nucleotides 228390036 to 228392011

<400> 28
aatcaagttg tgcaaacaaa ttgaaggatg gtgaatgtgg aggactttat tgagcactgg 60

aagtggctct cagtgggatg ggaagctaga aagggggtta agtgggaaga tggtcttccc 120

ctggagtcct gctgtcccca gccaaactct tctccaaagt cctgctgtca agctgtccct 180

ctgaaatcaa gctgattctc tccaacatcc ggctgtttct tctctccttc tctattgctt 240

tgctggtagg gcctggggtt tttatgggta caagatggag ggtggggcgg gccggggtgg 300

ttttggaaaa ggcaacattc gggcaggaaa acagaaatgc atgttctcac tttgggccct 360

acggttccag gcttgagggt gtggcgtttg ctagggacca ctctctccta cccagtattt 420

ccctgcctcc tgtttgtatc ataaagtgta tttaaaataa gctattataa aggatgagga 480

aaatacttga caatgtctgt cattttcttt ctttgcccca gaagattgtc ttatgtggcc 540

ccactttgga gactaccact cttggggctg ccgtggtcac aaaaacccag attattgttt 600

taactctatt tcttgccttg gttcatcgta ctttggggaa gaatctggaa tttgtataaa 660

gtataccacc caagtggcag tcccagctgg aagtcatgga atgggggcaa gaacaataac 720

ctgggagcac gggatctggg ttcggaacaa ggatgatggc cgggagctct aggctcagct 780

gtgccaccag tcctgtggcc ttagacaagt taaggagtat ttgggttttg ttttccttgt 840

ctgtaaaatt tagatacatt cttgctctaa aactctttga ttctgttaac tgtgtcttac 900

catttccctt taatctctat tctcagattg gaatcaccaa ttttattttc ttttacatgc 960

tgtaaaaata tatctctgta gttcccccca ccctcatacc acatttcagg tctactgtgg 1020

atgagttttt gcccataaac tctgtgaccc ttgcttctat ttcagcatca tgctttctac 1080

ttggaaccac tgaatttttt ttttcagtgt tggtttttaa aatattgtct tcaaatagag 1140

tatgtatatt agtcaatatt ttgtattttg gaaatctatc tgatagagaa gaatgtcatt 1200

ttttcccctt ggtgtggaaa aacttctgga ttgaaaaata agtttctccc tgtaattgtc 1260

tctgaatctg accagccagc cagctggcag aaggactggc aaaggagcgg gggccggaag 1320

aggagcaggg gccagtagag gagtggggcc ggaagaggag caggggcagc tgtaggggct 1380

agaaggccga ggtctgcctc tgaggacagc tgttgatgag gttcttgttg gagatctctc 1440

accctttggt caacgtggcc ttttggagca gattttctct ttgcctttat gagaccttag 1500

tgtcattctt gtattcctta gtaaccttgt gtaaagggct gaggcttagg agagacgagg 1560

gtagggagag agttcagaaa ggagagtcct aaggccgtct ggattctcct tgagccccct 1620

ccccatcctg gtcctgggct gtgtcttcat cacagccacc cctccccgat gtgctgttgg 1680

ttcagttacc ccagacagtg gatttctcag aacagaaatt gctaatgttc acgtaaagat 1740

gaatgaaatg ggaatgatga aatgtgttaa gagaacttaa atccagggtg tgaattgcca 1800

ctgatcctaa cagagacgtt ttagcttttc tacttcccac ctgccggaag aaagatgaga 1860

taaaccactt gtgttatttg ttcagcattg ctgcagcatc agagaggagt gttccccgac 1920

cctttgtttt tctaggtgac ttaatgggtt ctggcattgt gcatttatct ttttaa 1976

<210> 29
<211> 2120
<212> DNA
<213> Homo sapiens

<220>
<221> misc_feature
<222> (1)..(2120)
<223> chromosome 3 nucleotides 68637763 to 68639882

<400> 29
atacttgtac aggacatgca ggtttgttac atagatatac atgtgccatg atggtttgct 60

gcacccatca acccgtcatc tacatgaggt tatttctcct aatgctatcc ctcccctacc 120

tccccacctc ctgataggcc ccagtgcatg atgttccccc ctctgtgtcc atgtgttctc 180

atttttcaac tcccatttat gagtgagaac atctggtgtt gggttttctg ttcttgtgtt 240

agtttgctga gaatgatagt ttccagcttc atccatgtac ctgcaaagga catgaactca 300

tcctttttat agctgcatag tattccatgg tgtatatatg ccacattttc tttatccagt 360

ctatcactga tggacatttg ggttggttcc aagtctttgc tattgtgaac acagtggcat 420

tcccatcacc agtgccacat tctatttctt aaaactgact cacaggtcct gcccacactt 480

aagcagagat gacccaagga cataaatcat taggggtcac cttagggtgt gttcaacaca 540

cacacacaga gacacacaca cacacacaga gcaaatatgc tttatcagtt tacaatccag 600

gtttgttacc aggatctgaa agctggtgga aacactgtgt gggcataatc agggcaacgc 660

tgtgctccac tggtatgcat ttgttatggt ctgattggtc aaaatattag tcattgaagc 720

ctgataactc aaggtgaaat agctgctcta gcattgagaa aatgttaaat atattttaac 780

atatttcata gaagacattt ttcctggttg ctctagctga gaacctaaca atgcccattt 840

caacaaattt gtcagatgaa atgttcccca aaatgccctt cttttcaaaa tgactaaaat 900

ataccctttc atttcatttc atttaaatta ttctcaaagg cagccacagt gcaaatgaat 960

cagaaataca tattgtatgg caatagaata ttctacatta tttttctggg ctggaagggt 1020

gtttattatg gcctcacgca aagcctgtga tgcaggaagg tcttcgatat tttaagttct 1080

gggccatgca tagcaaattc atagctttcc tgctgccgtt ctgtccttct caccagggca 1140

gacattgata atcaatcaca gttctcttgc tcgtgaaatc tagacacagc ttcagaacct 1200

taaacaccat gccttaggca gccactagca atggctggat tagaacttga attaaacctg 1260

ctggccagcc cttggatagc cagttagctt attttaccat agagccacca ctgggcccta 1320

aaactaaact aaagatgaaa ttatctgctc aaaagcaaat agaaaacata gactaatgga 1380

gaaccatgat gagacaggat agggaaagag atccctacta ctgagacaaa aaatttaaaa 1440

ggttatatta aaatattatt tatttatatg gaaaaaatga aatatattat tatgcttttt 1500

aaattatata acatgattac caggttgtca gatcagggca aaaacagttg attaaaacta 1560

gcgtctagaa ttcttcaccc cttctcccct ttgtagtacc ttctacctat acttaagctg 1620

tgtttgcaat gaagaaacag gtattgttct attaaatgat tatggagctc acaagatttt 1680

taggctctaa agttgttata ttttcaataa gacttggtct tgggaagcat cgtagtgtga 1740

taaagacatc tttgagtcag acacttaggt tcttagcata tctcactgta tgattttatg 1800

tacacctctt aaccaaccca gacccatttc tccacatgca aaatggcaat gaagataaaa 1860

tacaaaaacc gcacaacccc tattgtgctt gggatgatgg tgatagtata taacctaagt 1920

aatgagaaag tgttttgacc tcacaagaaa catgatgtca tgttattaca aagtagtatt 1980

tatttagatg tcagttctcc tttgcaagat ataccaaaaa atctaaacac gtgtatttgc 2040

cagcccatgc tgccataaca aaataaccat agactgggca gcttacacac aggaattatt 2100

ttgtcacagt tctggaggtt 2120

<210> 30
<211> 22849
<212> DNA
<213> Homo sapiens

<220>
<221> misc_feature
<222> (1)..(22849)
<223> chromosome 4 nucleotides 6138398 to 6161246

<400> 30
acagagcaag cacagcccgc agcccagggc ttgttccccg tcactcccac aagacagagg 60
agacagagct aagggttgaa gcacatacat attcaacctg acatgcatac tgacatcagt 120
gtatgctcct gtctctctca ctttaatgag atacaataaa acactgccgg cccccagcca 180
ggcctctgca gctcagcttc ttggcagagc tggcttctct ctccgtctcc aggaccatgt 240
ctccctctca gtcattgacc ctctcctgcc tggccacaac tccccaaccc tcaccaccag 300
acagctcctg ccaaggtcac cagtgctctc ctggccaccg aacccaccgg acaacgttgt 360
cctcccgccc tgactccgtc acccactccc agactcacga cctcctgctc cctctcccca 420
aggccacttc ccatggtttc tatcttcttt atagactgtc ttgctctggc tctcattgaa 480
gcctggagtt tccaaggctc gggcctcttc tcactctata tcctctccca ggcagccagg 540
tcctccccga cggtatcaac cccacatatg cagcagatct taaacacatg cctcagccct 600
ggcttctctc tgagctccac aatcacagaa ccagctggct gcctgacacc tctgcttgga 660
tctcggaggg accacagcct ctccacgtac aaacctgaac ttaatcttca gttcaccccc 720
agccctgtct tgtccagcct tcctatgggg gagtgtcaga aaggacacag gctcaacaca 780
gtgggccggg tgacagtgag gaaccacacc aaaaaaagta tgcagaggaa gtgtcagccc 840
caggtcatcc actctggctt tttctggatg ctccagtgaa ggcactgtcc tggttgaata 900
gcaggccccc aagacccatg gccttcctgg aacttcagaa tgggacctta tttggaaaga 960
gggtaactgc aggtgcaatt agttaagatg aggtcatcct ggagcagggt gggcccttat 1020
gactggtgtc cttatgagag gagaagagag acacagaggg gaaggccgtg taaggacgga 1080
ggcagaggct ggagggacac agcctcaagc caaggaactc aaggtcagcc agcaacacca 1140
gcatcaggaa gaggctggga aagatcctct tccacagcct tagggggaag tgtgggcatg 1200
cagacacctt gatttcaatc tcctggcctc cagaactgta taggaacaca tttctgtcat 1260
gttaagccat gaagtttgag attatttgtt gcggcagctg caaggaaaca gatgcaaagg 1320
ctcctcctgc tcagctcact cacgtagcaa gttacatttc agtggattac aatccaaagc 1380
tcttacttct aaatcataaa ccttgatttc agacacagac tgccacagca gagaattggg 1440
gtgaagggcc ctcttctgat agcagagaga agcaggctaa aatcccgccc accccccaac 1500
cctgaccata tgaccatggg caggttgggc cacctctctg actcaacttc tgtctctgta 1560
aaatatggac aataggaaga ctttagaacc aactctgtct aactgttgag gagctttaga 1620
gcaaccatga gaagaatttg gtacagtgcc tagcatatcg caagcctgcc atcagtgttc 1680
actgccactg ttaatctctt ctagccccta ggtaacacga ggctatttta ttttaaaaga 1740
agaccactaa aaactgaaaa aaataacata gagatgagag aagaaaggaa atggtactcc 1800
tagattccag actgagaggt tttccagcca cataccacgg tgctacagtt ctgatgtgga 1860
atttccatca cttgcaacca aaataatcct aattgacata gagttcagga aagaacaaaa 1920
aacgtgccag aggtatcacc tctctcttat gtgctagttg tgagctaggt actaatttgt 1980
acttaagagc tctctgaggc acctttcttt ggaagacaaa aggctgtttc catctgccag 2040

gatgatatcc tggcccctgc aatgggtgct ccatgcagtt tctcctaagc agctgctgaa 2100
tgtgcccact gctccccctg gtgatgatcc accagcatct acctgcttgg gccaaatccc 2160
aaatcccgag tgttgcaatt tccaaatggg taggagcact gtcccttcca caggaggaat 2220
ctttaaacat gctgaaaaca ccactgccaa tgtagatgcc aaccccaaag caaaaatcat 2280
tatgcagaag gagtttattc atttaattca ctcaagcacg ctttcattaa ctcaacacat 2340
gctgagctga gcgctatcag tgggtgctga gaaacattag agagcaaaag agaccagccg 2400
ccctgcccta ggggcttcta ttctgctagt gggcggagca ggcaataaac ataataaata 2460
agcagacatt gtagtgattg agagtgattt gtgctatgag ggggaaaaaa aacaataaca 2520
acatcaaagc aaaggaaatc ggtaggggca gtggagggtg ttgcaaacca acagtttgtc 2580
catcacgggt aagggagggt tcttgtctgc ccctcagtat cagctttgca gtgaagcccc 2640
cgccactgcc ttccactagc tccccacaaa agacagcacc gtcgcgagca agacatctcc 2700
aggatcccaa attctccacc tggaagagca ccaattcccc cttgtcctag taagtgagac 2760
agagggcttc tgccccagtc atcacaagaa agtttcctag aaaatattct caaacagcag 2820
agatggcatc tagaaacgga cggagggcca atgcagaaca cattccggag ccagcatgag 2880
agtcctgagg ccccagaggt taagacactg gccacgctca cgtagcaaga ggcaggcaca 2940
ctcacagttc actgacttta ctcctggatc acaagctgag ctcttagtga tcctatctcg 3000
ctgcccttgg gatgctgatg aaatctcaag cttcctggaa ctgctcacct gaccatatgg 3060
aacatccctc agcttccttc ccgcatccat ttccccttct cccatacgcc cagtctcagc 3120
atgtgcagta ggaaaggggt ggaacgaaca tcactgcaag ttcccagggc agtccccaat 3180
tggctcatgc cacccaaatg tccctgtccc aacagtaatt gcttcaggga tggatgcaca 3240
atccaatcag aaccaatggc cagaagtagg cagcaccagg atcctcctgt ttttctttag 3300
gagctaagaa agggactctg ggctgtgtgg tgtgagaatg tgggacctgg cacagtggtg 3360
gcatcccccc agcacagcat gagagcccac agcctgggaa tgacacagca ggaggcagag 3420
cagagcccaa gaacctggga tcctggatga catcgttagt gcacctggag ctgcacctac 3480
agcaaatccc atgacttcag ttgggggagc caacagacac ccatggccta agctggtttg 3540
atggaatttc tgtcacttgc gaccaaaaga atcacaatca acagagttca gggaagaacg 3600
acaaacttga tgttggctat cactcctctc ttaatgctag cgtgagctag gtactcattt 3660
gtacttaaga gacactctct gagtctttaa aagatcctca aagtggacat catttacccc 3720
aagttacaca caaacttgag gcccagaaag gggaatgtgc taacactcta gttagcagca 3780
gaaccatgac tcaagtgcag attgtactct ccaagccctc cctgtcccct ctgccccaaa 3840
ggaacagagt ccggttgttt ctaagcagtc aagtccacca ggaaatgtat cttagtgaaa 3900
acaagcccac tgacccatga ctgatttggt gagctccact ttattgcaaa gtttggggga 3960
atcaaagact gaagcagagg ccactacctc aacatcaatt ctcattcttg gtacctgtag 4020
tgggatgaat aatgtccccc aaaatgtcac atctaccccg aaaatcagaa tgtcacctta 4080
cttcgaaata gggcctttgc agatgaaatg aagatgcaaa ttaagatgag gttataccac 4140

gttagggtgg accctaaacc cactgactgg catccttaaa agaagaggac acctgtaatc 4200
ccagcacttt gggaggccga ggcaggcgaa tcacctgggg tcaggagtct gagaccaacc 4260
tggccaacat ggtgaaactc tgtctctcct aaaaatacaa aaattagcca ggtgaggtgg 4320
tgcatgcctg taatcccagc tattagggag gctgaggcag gagaattgct tgaacctggc 4380
aggcagaggt tgcagtgagc cgaggtcacg ccactgcact ccagcctggg tgacagagtg 4440
agagtccatt tcaaaaaaaa aaagaagaaa aagaagagga caagctgcag acatgcaggg 4500
gagaaggcca cgtgcaggca gagactgaag tggtgcgact gcaagtcaag aaatgccgag 4560
gactgccggc cacaccagaa gctgcaagag gccagaaaac actttcaccc tagagctttc 4620
caagggagca tggcccagct gatatcttga tttgggactt ctggactcca gaaccgtaga 4680
gaaaataaat gtcattgttg taagcctcca agtttgtggt aatttgttac agctgccccc 4740
aggaaactaa cagagcacca caccagggtt tctcagaagc cacaggccac gcagtatggg 4800
ctccaggatc actttaaact agaacatcac tgtcccatgt tgaccatgat caccttaaac 4860
tagagtgtca ccgcctcatg ttgaccgtaa ccaccttaaa ctagagcgtc actgccccac 4920
gttgaccatg accgccttag aatgtcaaaa cgccatgttg accgtgactg ccttaaacta 4980
gagcatcgct gccccaggtt gaccataacc accttaaact agagtgtcac tccccatgtt 5040
gaccataacc accttaaact agaacatcac tgtcccatgt taaccatgat gcccttagag 5100
tgtcaaaacc ccacgttgac catgacgtcc ttagagtgtc aaaaccccac gttgaccatg 5160
actgccttaa actagagcat cgccacccca ggttgaccat aaccaccttt tcttcttgac 5220
ttttactaag atgcatccta atgtgtagac acaagtccaa aagccttctt gcatttctaa 5280
tccaagccag ctgacaaaga aatgaaacca gcagcccatc ttctgaatgt gttgccatgg 5340
gcaacaggtt tgcttaagga gtgacattta tcagacaggg tgtgggtgaa taggagaaat 5400
gaacgtttga ttttttcact ccttctccta agtggaccat tttgggtgaa aaccatccac 5460
acaatgagat acctgaatgg ggggagattt ttgtttggca ctgaaatgga aacggagcct 5520
ttgtgatact ggatccctgg cacattttaa attaagctca aaccgtctct tgtgcaaata 5580
cacagagtat tttttccccc attttacttc tcttacatat acatttttcc tataacagaa 5640
gaacacattt tacaagtatc ctattacaga aactataagc tgctacatgc tttagctcac 5700
ttgcttgttt aattctaact ctcggcagat atgggaatac cccccattta taggtgaggt 5760
gctagaggct tggagacagt aaccacctgt cccagggtca catggcaaac aaatggggag 5820
cccagaatca ctcagggcag cctctctcca aaggcttctc ttcccaccat gtgatgacag 5880
ctcccctgaa cacaaaccca tgaacatacg tgattgagaa aaggggctgg agactgttcc 5940
tgaggactca gtattggtgt gcttgcactg tctataaatc agaaatttta aactgacaca 6000
cagctcttct gccattcttg gcacagtatg gggttaaggc atgaactggt ggtttgtcac 6060
caactgtggg acccaggcta tagctggatg aggactttaa aagggtgcct tgtcacctgc 6120
ctccagtccc aaaatctctt ccagcatctc atctcccgca gagggccccc agccccagtt 6180

tacactctcc ccatgctggg acactcacta cctctccagg aggttcttag tttctaagaa 6240
cctttggaat ctcagagtct ttccaacatt gagggtgcgg aataaatgaa tatatcatca 6300
aatcatcagg actttttaaa aggtaaggaa aagaaaagag agaactccct tctaccccag 6360
caacaccctc agtgcctttc actgggtgtc ccgccccctc cctcccagcc gtcctcacct 6420
gaagagcaca ctccaggtgt ggtggaatcc cggccagccc acagcctgca tgcagaggag 6480
ccctgtactg tcagtgagag ggggccagtg tgggtacagg cacttcagct ctctgaagtc 6540
attcactttc cctagatctc cttcctgtgc tacaaaatga gacactgggc gaagaaccat 6600
ttcctgccaa tgggcttgtg taccgatgcc cagaggaaac tggagcacca cccacctgca 6660
gccgcaggct cccctcccca tggccacacc cccttcctgc tcctctggga atgcacgggg 6720
cttggcagag gtgggtttta agccattgag agaacattgt tggttctgcc cagctatgaa 6780
agtctatagg aggaaaagat agaatataag caaaataccc ttcgtcgtta ttctgtcact 6840
gaagctctca gtgcacagca tagtgaggtc tgcagggaag catggcccct tctaagaggg 6900
tccttgaccc caagtcattg gaaggcagag aatagaaaac cttttcagtg ttcatgcaga 6960
cccactatat cggtcatcat cacatgcatg ggagatagaa acagaaacgc attcctggaa 7020
atcattcccc cagattcagg tgcaggggcc tcgattctac ctgtgatgct tcatctactg 7080
tgtggtgggc acaaggtgtt tgcttgtgat ctcatctata ccttttgcca gaaatagttc 7140
acacttttag aactcctatt aaaagtaaga aaataaagtc tccagtactt gacaacagga 7200
ttccattata ttttcatttt gtattttact gggaatccaa gtatcacata tcatcatggc 7260
tattttagag atatggttaa aatgatcaca tcatgcccca ttacttccaa acctttgcca 7320
gtgtttctcc ctctgcatag gatcacctaa caaactccta tgcatccttc aaaacccagc 7380
tcacacctca cttcctctgt gaaatgactc ctgcatctac ccacagaagt gatcacatcc 7440
tcttctttgt tgctgcctct ttggcttgca tggtcctatt accaaaccgt cctgctcaca 7500
ctagaataat ctgctcacac tccagttaac tgtaagcgct ttgctgcctg gaatcgtgtc 7560
ttatttttct ctataactca gtaaataaat attcaactga gggggagctc tcctgcccag 7620
ggctatatgt gttcttgggc cattctgact gacagcaaat ttcatggcca tggatgacta 7680
taaacagtgt ccagctccca ttaaactgat tgtttactat taacatgcag caattagcag 7740
cgagtccaga tcatgtgctg cttccaaggc tgataaccaa cccaacgaga tgtcccaaaa 7800
agacagcttc agaaacaatc actcaaattc ttatgcaaaa cagtcacctc ataaaggtca 7860
gcagtctctt gaagttcagg gtcctgaacc agacacctcc tgtattacaa tgaatgatgt 7920
ccacctgtac agggaggtgt ggctgccgtg ccctgaaaag tatcctgtct ccagagagga 7980
atgatgctta cttgggaaga ggtcactttc ctcaaagaag gagccaggtt tgcccgtggt 8040
aggagtcggt ggggaacaag tcctggccca gtgcctctgc ccaccaccac tcaatgtgtc 8100
tggtcaacct gtgtgcaggg ctccagagtc ccacctgccc aggaggctgc aagttccctc 8160
ttttctgcca ccctggctcg gctgggcagt ccccagatgt cctgcccttc acctcccaag 8220
cagagatcac atgggagcgg gtgtcacctg tcaccccagt agactgcgag ctccctgcag 8280

atggccaggc agggttttag ccatggcccc ccaggactcc cccgagaggc tggcccagca 8340
ggcatctata tagggaacat gcatcgaaca catgccactg tcagttcatg agttttaaaa 8400
acaaccatta tggttttata aaagcataaa ataaaatctc aaacaatctg tgctagccct 8460
gggtctagct cacagcaggt gctcaccaaa tggagtgccc ggaaaatata tagcctctct 8520
tccgtaattt tctggcaaaa aaagaaagaa aacactctcc tttatctccc agctgcctga 8580
agcttgatgc gaaagggcca ctgagaaggc cagaaatggg ccactcctac ccaactgaca 8640
ggaggaatcg tgctaagaaa acagcctggc cccagtgaga gggcttggca gacaggcatg 8700
ggagtaaaca aacctaccaa cctccggggc ccctggggtc tcaggaactg cgggcaccca 8760
gcctaggctg gtccatcggc ccctgttctc ctagttgaga tcctgaggcc caaggcctta 8820
agctgtgcct tgattcctcc taccttgtca aactagttcc caacagtggc actctttcaa 8880
aaataaagtt ttaactctta ttaggaaagg aatgcagatc cattatagaa aatgaaaaat 8940
acagccagac gcagtggctc acgcctataa tcccagcact ttgggaggca gaggcaggac 9000
atttgcttta gctcaggagt tcaagaccag cctgggcaac atggagaaac cttgtctctg 9060
caaaaaatac aaaaaaatta gctgggcata ggtggtcctg gctattcagg aggctgaggt 9120
gggaggattg cttgagcctg gaaggtagag gctgcagtga gctgtgattg caccactgca 9180
ctccagcctg ggcgacagag caagaccctg tcaaaaaaga aaaaagatag aagaaaaata 9240
aaaaggaaga gggagggaag ggacagaggg agggaggaag gaaggaaaga aggaaagtgg 9300
aaggaagggg gagtggggga gggaggaagg ggagagagag gaagggaaag aaaagaaaga 9360
gggaaatgaa agaaaaagaa aggagcaaag gaaggaagaa gggaaggaaa ggaagaagaa 9420
aggaagggag gagggaaata gaagaaagaa ggaaagaaaa gaaaggaaag gagcgaagga 9480
aaaagggaag gaaaggaaga aaggaaggaa gggagggagg gagggagaga gggaagaaag 9540
gaaggggaaa gagagagaca gcgaaagaaa gaaaggaagg gaggaagaaa ggaaagaaag 9600
aagaaaggaa ggaaggaaaa ttaaaaatac aacaaagaac agagaaggaa acagaaagcc 9660
cccatgatgt caggatccag aggcagcccc tgccaacctg tcacctgctt cccctcattc 9720
tacttctgca agacagagtc ctgtttgcag ccatacatcc tgttttttaa ccttctgtgt 9780
tcactttccc aagtccctat agcttgtcat aaatgcctga gtcattgtcc tgactcagaa 9840
actcgaggca gcaaacgcgg atgcaataga ccactggcag aacacacacg gtgccatctg 9900
atgtgtacgt aagatttgtg catgcttttg cacatttcct tcggtagact cctggaagtg 9960
gaattagcac actaaagggc aggagcgtgc ataaagctgt tgtttgcaca gaggcggggg 10020
gcgtctgcta tgcaccacac tgcacaggct ctgggctttc acagtgaaca agacagactc 10080
agtccctgtc cttacagaac ttggatccta gtaggggaaa acacacaaga aataatcaaa 10140
gagagagaga gaaaaatatc agatgagaag tgctgtggca aggatgggaa aaggtggcat 10200
ataactgagt gactcggggc tggcctagat gatgctcagg caggcctccc tgaacaaggg 10260
acactgaggt gggagctact cagtgagaaa gaaccagcgg tgcaaagatc ggggctggag 10320

cgctcccggc caaggacaca ggagtgcctg tgcaagggcc ctggggctga gaacaacctg 10380
ccaaaggcac ctgctgtctt ctcttttgac acttaaatta caccctacct ttaaaaaaaa 10440
atcctggcta atttgattgg gtatgatagc atcctgctgt tttaatttgc atttcatgac 10500
cggtgaggtt aaactaattt ttatatttaa taggcattgc ttttctttgt gaactgtgtg 10560
ttcccatgcc ttgactaatt ataaattccc attgttcctc ttgtaaactt gacaaacttt 10620
ttatacactg aggatgttaa ccctctgcca cattgtttaa agactgtttc ccaggctata 10680
tcttcactct ccatttttct aaaggcctgc atgccatctg tgcatcaaat actctgttga 10740
ggaaactggg ggtaaaataa tatttgtcat tatcattata acaaattcat tttcagatcc 10800
cattggacat tgcttttaat cctgtatcat tcagaattga gttttgaaag aggatccatg 10860
gcctgaggct gagtataaaa ttcccacata acttttaccc tgtaaaatgg gagtacgggt 10920
tgagtatccc ttatctgaaa tgtttgggac cagaagtgtt acagatttga gggtttttgg 10980
attttggaat atttgcacat acatagtgag atatcttggg gatgggaccc aagtctaaac 11040
atgaaatcca tttatgtttc acatacactt atacatatag ctgaaaggta attttataca 11100
atatttttaa taattttgta catgaaacaa aagtctgtgt acattgaaac atcggaaggc 11160
aaagttctca ctgtctcagc cacccaagtt gacagtctgg ggttgtttgg catcaccatc 11220
attcctgaca atgaatgtat ttatttattc aagacagact ctctctctgt cacccaggct 11280
ggagtgcagg ggcatgatcg taggtcacag tagcctcaat ctgctggctt caagcaatcc 11340
tcccacctgg gcctcctgag tagctgggac tacaggggta tgccaccatg tccggctaat 11400
ttttaaattt tttgtagagg caagacctca ctgcccagga tggtctcaaa ctctcagcct 11460
caagcaatcc tcccacctca gcctcctgat gtattgggat tacagtcatg agccatctcg 11520
ccccgttctg actctgaatt tatatgctac caataagtaa tcacttctta cacttattca 11580
cacataccac tgatacagtg gagaagataa tgtgctcatg gtaacatgtg agcttgcttg 11640
catggggagt ctgggtgtgt atggaagctc tatcacagct gatgggggct agggggatct 11700
tttttcccct tgggtatgct aaatacactg cgttgtacac ctgcattttg actgcgatct 11760
gtcacaggag gttgggtgtg caattttcca cttgtgactt catgttggca ctcacagagt 11820
ttcagatttt ggagcatttg ggactttgga ttagggatgc tcaacctatt tctataccac 11880
ctttggaaaa ctccataagg atgggggaag ctttacagat cacttcccca ttttgcaaca 11940
aaaaatgcaa taagaagcgt tcattctcat catcatttga caaatggtgt gagtcaacca 12000
ttccatggga aaagccaggc ccacgcttcc ttaggcagct tggagaaacg caatctggtt 12060
cccttgatca aaagcaccca agaccaggat gtcaggtttt tgtctggctt tgaatatttc 12120
agaggtagtg aaagctctct ccgtggaacc ggaaagaata attaaaatgc aatgcccttt 12180
atggctcact tacattgtgg gacagaaaag cggtaactgt gacttgaatc agagataaca 12240
aaagtgtagt gtcgagtttg gagttcgaga acatggttct gatacgcatg agtctggttt 12300
aacaaggtgc tgtccaaaac cacgcctgcc tgagcacact ttacacaact accacccagg 12360
tcagaaaaga aacattcctc gtcgcgtgcg gtggctcacg cctgtaatcc cagcacttcg 12420

ggatgccaag acaggtggat cacctgaggt caggagttcg agaccagcct ggccaacatg 12480
gcgaagccct gtctctacta aaaatacaaa agaattagcc aggcgtagtg gcaggcgcct 12540
gtaatcccag ctacccggga ggctgaggca ggagaattgc ttgaatccag gaggtggagg 12600
ttgcagtaag ccgagatcac agcattgcac ttcagcctga gcaacaagag tgaaacttca 12660
tctgaaggaa caaggaaggg agggagggag gaagggcagg agggaaggaa agaaagaaaa 12720
gagaaagaaa agaaaggaaa ggaaggaagg aagaaagaga gagagagaga gaaagaaaaa 12780
gaaagaaaga aagaaagaaa gaaagaaaga aagaaggaaa ggaagaaaga aaggaagaaa 12840
agaaagaaaa agaaaaagaa agaaagaaaa gaaagaaagg aaaggaaagg aagaaagaga 12900
gagaaagaaa gaaagaaaga aagaaagaaa gaaagaaaga aagaaagaaa gaaaaagaaa 12960
gaaagagaag gaaggaagga aggaaagaaa gagaaagaaa agaaagaaag aaagaaagat 13020
tgattcctga tgctcgagaa ggctggctcc tgccgcttcc tgatcaaccc cacacccgga 13080
ggtcatccac actcttattc tagcacctta gattagtttt tacgcttctt aaacttcatg 13140
tgagtaaaat catatagtat gtagcttttg tgctcatctt ctttggtttg gttctgacac 13200
cacggctggg aaattcattc attttcttgc ctgttttgga aattcatttt ttcccagtgc 13260
tgtgtaatat cccattgact aaatacacct ttatccatcc tattgctaca tttattgctc 13320
acagagcatg ttttctatcc ttttccttca gggaaagtgg gtctttatat ttaaagtgtg 13380
acttttgtat ccactctgac aatttctgcc ttttaactgg tttagtccat ttacatttaa 13440
tgtaactact gatatgactc ctttaattcc accgttttgc tgtctcttct ccatttgtct 13500
cgtctgtttt tttcccctgc tttggtcctt ctttgctacc ctcttttagt gcaaacagat 13560
ttctttagtc tctcatttta tttccccatt tgctttttat ctgtgcctcc ttgtgctgcc 13620
tttttaaacc ttgatttgtc acagcctcct tgaactcata gcgtcacgca ccccctcaga 13680
caagactgaa ggactgtgcg agagcacaga tccattttcc accaaccacc cccaccccat 13740
cctttctaga ttgttttatg cattttacgt ctgcatatgt cataaacccc acaatacaat 13800
ctgattcttt tgctttaaac agtcacttgt gttttgaata cactgagaaa aagagatcgt 13860
ctcagacatt gactctcctt tacccacttc tcctgttctt tattcccttc caagagaact 13920
aaattcctat ctggcattgt ttctcttcac ctgcagaacc tcctttagca tttttggtgg 13980
tacagcctct ggcgacaaat aatcttagct tccttttacc taaaatgtct ttatttcact 14040
cttattgtaa aataatattt tttgcttgat gagaaattct gagttaacag atgtctctgt 14100
ttcgttttag ggcactttag tgtatgggat gctaccgagg ggcacggctg ctccacgtcc 14160
ttgccatttc tcagcactgt tagcttttta atttagccat cctggtgcct gggcagtgtt 14220
gtttcattgt gtgctgttct tcctcttata aatatgctcc ttttaaatgc aaatacaaga 14280
tccaccatta aacttttatc aatcctattt aaatttccaa tttttcttgt tttaaacatt 14340
ttagagttta atttataaat catgaacttc gccgattttt agtaagtctt ccaagttgtg 14400
gagccatcat tataatctgt ttcagaacct tcctcgccca gtaaggtccc tcacactcat 14460

ttacagctaa tctccattcc aactagtccc cgtcagccac tatagatttg gatatataga 14520
tttggctatt ctggagattt catacaaatg caagcataca atatgaggtc ttttgtgtct 14580
agcttctatc atttagcata atgtttctga ggttcagaaa tgttatagat attgaagcat 14640
atatcaatat ttcatacctt ctcattgttg aatcacattg tgttatatag acatgccaca 14700
tttatccatt caccaactga ggacatttgg gcttctccca ttcgttgact attataatgc 14760
tataagcatt catgtacaaa tctttatata gatatgtgtt ttcatttctc ttgggtagat 14820
acttaggagt agaattacag agtccgtagg tgtccttttt ttagactttt taagaaacta 14880
caaaaccatt tatcaaggtg gctataccat tttaaagtcc tatcagaatg acggtaccca 14940
ttcctccaca tgctagtcaa catttactat tgtctgtttt tttttaatag tcattctagt 15000
ggatgagaat ttatatctag ttgtaatttt actctggttc cctgatgatc aatgaggtag 15060
aacacctttt cacatatttg ttagtcattt aattatcctg tttttgaaat attctttcaa 15120
gcttttgccc atttttgagt tttcatatta ttttatagtt tttataatct gcacaaaagt 15180
tctttgtggt tataggtatt gaacatgtct tctctcagtc tgtaatttgc cttgtctctc 15240
ttttattagt gtcttttgat gaacaatgat tatttcatgt ccaatgtaca accctctctg 15300
aatagtatat ttatgtaaat agtgtactgt gtcctgctta agaaatattt accatgtccc 15360
aagatcatga aaatagtctg ttttctacta gtagctatat tgttttactt tttacatgta 15420
ggcctatgga ccactttgaa ttaatctttg tgggtagtat gatatagact tcatactcta 15480
tatacagaga gtgtgtgtgg tgtgtgtgtg tgtgtatttc ctgttttttt ttcttacaga 15540
tagctgacca gtccaatatt gtccattgaa agatcatcct ctctatagtg aactgccacg 15600
gtatctttgt ccacacacgt gaagatgtac ttctgggctc ttctgttcca ttgacgtgtc 15660
tctctaacct tcagcctact gtcttgatta ctgtagcttt gtagtaaact ttgttatagg 15720
ttgtatgaaa cctccaattt ttctcttctt caagattgtg attccacatc ctttcaattt 15780
ttacatcaaa tttagaatct gcttgccgat gtctgcattt aaaaagtctg ctggtggctg 15840
ggcgtggtga ctcacgtctg taatcctagc actttgggag gccaaggcag gtggatcact 15900
tgagatcagg agttcgagac caacctggcc aacatggcaa aaccccatct ctactaaaaa 15960
tacaaaaaaa aaaaaaaaaa aaaaaaatta gccgggcatg gtggcaggca tctgtaatcc 16020
cagctactcg ggaggctgag gcaggagaat tgcttgaacc caggaggcag aagttgcagt 16080
gagttgagat catgccactg cactccagcc tgagcaacag agcaagactc agttaaaaaa 16140
aaaaaaaaaa aagagtctgc tgggattttg gtaagggata gcactgaatc tgtagatcaa 16200
tcatggaaaa actgatatct atactaagtc ttctaatcga tgaaaacgat atccctttcc 16260
attgatttag atctccttaa gtttttctca gcaatgtttt atagttttca ggggagacat 16320
cttacacaac tttccttaga tttattccta ggtatttgaa ttttgtggtg ctctactgta 16380
aatgatattt tttaagattt cattttctaa ttgttcactg ctgatatatt gaaacaactt 16440
atttgtgtgt atgtaccttg tatatgtaga ctttgataaa ctcacttatt aattctaata 16500
gtttgtaggg tcttgccatg ttctgaatgt gtctcacaaa aattcataca ttgaaactta 16560

tcatcagtgt gatagtatta ggagatgggg tctttaagag gtgatgaagt catgagggca 16620
aagcctttat gaagggagtt agtgacctta taagagaaat atgagctgcc tgcctctctt 16680
ttgtcattcc actccttctg ccatgtgagg acatggcatt caaggcactg tcttagaagc 16740
agaaactgga cccttgccag atagtgccta gatcttggac ttcccagcct ccagaatggt 16800
aagaaaaaaa tttctattat atataagtta cccagtctgt gttattttgt tatagcaaca 16860
gtaatgaact aaaacaggtg tcttgggttt gctacatcac aaccatagca cctgcaaata 16920
atgagtttcc cttttttctc cctctctaaa gacttttttt tttttttttt tttttttttt 16980
ttgagaggga gtctcactct gtcgcccagg ctggagtgca gtggcgcgat ctcagctcac 17040
tgcaagctcc acctcccagg ttcacaccat tctcctgcct cagcctcctg agtagctggg 17100
actacaggcg cccaccactg caccccgcta attttttgta tttttagtag agatggggtt 17160
tcactgtgtt agccaggatg gtctcaatct cctaaactcg tgatctgcct gcctcggcct 17220
cccaatgaaa cttttattta ttttatttta ttttatattt gacatcctgg ccaatgcaat 17280
gttgactaga agtggtaata gtcaacatct tgttttgacc ttgaacttgg gtgggaaatg 17340
ttcaatattt catcattatg tatgttactt gtaaaagatt tttgatagaa ccatttatca 17400
tattagagaa attccattct tttcctggtt ttcggagagt ttatatcatg aagttgaatt 17460
ttaccaacag atttttctgc atctattaag atgactgacc acgtttttct tttttatcat 17520
attattatgt tgaattatat tcagtgattt tagaatatga agccaatctt tatattttca 17580
gaataaaccc agctgggtta tgacttatta gccttcctat gtattgctgg atttgatatg 17640
ttaatatctt gctcgtactc ttgtatctat gttcataaaa ggtaatccaa aagttttctt 17700
atcctgtaat gttattgctt ttggaatcag agttacactg gcttcataag ttagttaaaa 17760
tgtgttccta attctctatt ttctggaaga ctttggctag aattagtttt acttctttct 17820
taaacacttg aaagaattta cttgtgaagc catctaggcc tgggcctttc ttcataggaa 17880
aattattcat tacagattta atttatttag gagctttagg actacttaga ttttctattt 17940
gtttcatgtc agttttggta cattgtgctt attcaaggaa tatgttcatt ttatcgaggt 18000
ttcaagttta ttggtatatg ctgtttgctg tggttttatt gactgtagga gctgtggtat 18060
ttcccccttt acatttttga tattagtaat ttattgtctc tgttttgctt tatcaatatt 18120
gctaaatgtt tgtcaattta tcagttttaa ttaactcttg aatttgttga ttttctttat 18180
tgattttatt tcactgcttc ttgctcttta aaatttcctt ccttctactc tctggagttt 18240
aatttgctgt tctttttctt tcttcttgag atagaagttt agatgcttga ttttcagcat 18300
ttcttctttt tgtctaaaat atgttttgct ctaaggacta attacaaccc acaaattttg 18360
aaatgcggtc tacaatgata aaagtatgtc atgtccacag tattctgcag ttgtggaata 18420
gagtgctctg tcaggtaagt tcacgttggt taattgtgtt ggtgaaatca tgtctttatt 18480
cattctttgt ctgtgtgttc tactgtttgc tgagagaagc gtgttcgtcc ccaaccgtaa 18540
ctgtggattt gtctgcttct ccttttagtg ttgataattt ttttctttta tacattttga 18600

ggttttgtta ttgaatgcat tcccactgaa ttgacccttt attattaaga actgtccttt 18660
tgtgtcttta gtattacttc ttgccttaga gcctttgaca ttaatacagc cagaccagct 18720
ttgcttttag gaagtgcttg cacagtgtat ctttccccat cctcttatgc tccatgtcac 18780
catgtcctca taattaagat gtctctcttg taaatatcac tgttactctt tgatccagtc 18840
tgaaaatctc agcttacttt acttcttagc ctcctgcccc acacagcttc aaagtgtgac 18900
aaacgccctg aggggcagac tggtcatgtg tttgtggcgg gccccctccc tttgtctcct 18960
acgtccagtg agggcatggg agctgtcatt ctgcctttcc agctgggctc gccagcctcc 19020
cccttcactc caggactcag gaggatctcc tgtgcagcaa gctggcctag gtggggctcc 19080
tctggtttcc attgtttcat gcccactctg agatggcaga aaagtcctac tcatctctct 19140
gaggacatcc aatcctcagc ccgcacccag aatccacaaa tgccccaagg gaagaaagca 19200
gctggcaatt aggccgccca ggcagggctc tttccccttc cctttggaat tttagttcat 19260
ccacactcca cagcttccac agatctccag tatctttcaa aacaggacta tttgcaattt 19320
atatgtttcc cttgctgttg cagccagttg gccggcctgc cctgacctgt ggcatcttac 19380
ctggagatgg aagtctgctc atggttgttg aagtaaatga atcaataaat gcccgacact 19440
tagatacgct atcacgaggc atttgcaaca accctcgcta gtctccatta agctcctttg 19500
acaaatgggg aactgttctt tgagcggatt acctgcagcc ccaaagcctc cttaccacac 19560
ctaagacggg agggaggttg ctggggcccc agtgctttga acaatgcctg gagcacagca 19620
ggtcctcgga gctccgcatt tcccccatgc gtgcagggag ggaaggccgc gtgcccgcgg 19680
gcgggggagg gggcggcacg taccagcctg cggatgtcgc gctcgcactc gcgcttgatg 19740
cggtgcacct cgtcttggtg cgcctggtag gcggcacgca ggtcggctgc cttggtcttg 19800
tcagcctgca tgcagttact gagcgcctcc tctgcctgct tgcgcgctgc cttgagctcc 19860
aggatctcct gctgcagccg caggcgctct ccatcgaagg ccctgcgcgc ctcctcgcgc 19920
gcctcggtca gcagcgccgt cttgaccttg tcggccgcgc cgtcgcgcag cacgttcagc 19980
gtggcctgca gccgctgcag ctcgccctcc ttgatcttgg cggtgcgcgc cgcctcctgc 20040
tcgtgctgcc ggatgagccc ctcgcgcagc gcctgcagct ccttggtctt ctcctcatgc 20100
agcttggcct tgagctccga aatgtaggcc gtgtgccgtc gctgctcctg ctcgcgctcc 20160
agcttcgcct cctgcagccg ctcgcgcagt ttgcccacct gcagccagag cggcgagaga 20220
gccggtcagg gtcagggtca gggtcagggt cagggtcagg gtcacagctg ggggagctgg 20280
ccacagcctc cccacgccca agacacccac ctgggcccac gttcccatgg attccccctt 20340
cggcagtgcc ctgcaggcct gaccctcctg ccagccccac ttagaatctc tcctgctagt 20400
tccctgagac tttccctggg ggtggaaacc ccctgaggat gctggagagg cattcaagcc 20460
tttgacatct cttccaggtt gtaaattgag acgactgctc aatcacaaaa cacaccctgc 20520
agaaatcatc accaagaccc aaaagattgt aaaccagaaa tatatttcca gggccccatg 20580
cgcggactgt ggagttggaa aagacagacc aggcttctaa gtcctggggg gcagtgagca 20640
gggggcacca ttcttgttga gtggggtccc tagctgggct gggggctcca atgcatcacc 20700

cacattgcca catgaacccc tcacggtctt gggaaggaag tagcgtgctc cctctctttc 20760
tccctctctc ctctctctct ctctctcttc ctctctctct cctctgtttt atcatgaggc 20820
agccaaagat cagagacagt ctggtgagct gcccagggtg agggatgagg accctgtgct 20880
accaggcaac ccaccccgcc ttgagcctat tcattttatc agcaaacaat tcaaaacatt 20940
acttataaaa cgtcccaagg gagtgtgttc ttaaaacact tcaaaatgct ttctttaacc 21000
tttttgtttc aaatatcact caacacacat tccacaggaa cgcctgacaa gcccttattt 21060
cctgaattct gcccgtctct gaagacctga aatgaattgc ctctggtgga caagctgctt 21120
ccagggaaaa tggcaatgtt tttccccaga ccacactgtt gatagcatag tcacatgtta 21180
aaattaattt taggtgagaa gttttacaat ggcacctgag tattgataaa tccttactta 21240
aaacagcagg taaaaaagca aataaaacca acccaggcaa acacacaaga tcaacatctc 21300
ctttcaccag atactcacaa tcataggaat gaagccaggc gaccagtcca tgttctgtcc 21360
accttaccct gggggcagca atcagcatct tttcctagtg gatttagcag caggaagcca 21420
gaactttaag tatttaacag aattgtccca gttatggaca aaatggtttt gactgttctc 21480
cctcctgcga ggcaacagtc aagtccctgg ttaatgccga cagccatctg gggccagcag 21540
ggtcagatcc aatgagatcc taatctgctt tgatgagctg cgtttcccca ggcaaatttc 21600
tctgcctcta tggtggctga tgttgtgggt cgacttggct gggccacaat gcccagtcgt 21660
ttggtcaaac accagtccag atgtggctgt gaaggtacat tttataagag attaacattt 21720
acatcagtag actctgggtg aagcacggtg ccttccacaa tgtaggtggg cctcatccaa 21780
tcagtcgaag gctttaagag aagaccgagg tcccctgcgg aagaaggaac tctgcctccc 21840
ggctgcactg ggactcgaag ctgcaagatc aacccttgcc tgaatctcca gcctgctggt 21900
ctgccctaca catttcagat ttgccagccc cacaaccgca taagccgatt ccttaaaact 21960
aacgaataac ctctcactct gtggggggtg tgtgtgtgtg tgtcacaacc tattggttct 22020
gtttctttga agaaccctga ctacacaacc ttccagagct ccagtttcct ctactaggaa 22080
acagggatgg caacagctgc ccctcaaagg cccgttgaga ggaggaagat gaggtgactg 22140
tgggtgggtg aatggaagtc tgttgttatc gtggttctga ctttctgcat caggggtggg 22200
cagcctcgtg tgagagacac aggtagaaag ggggtgagga acatggaagg gtgggcagag 22260
gcctgtgcag ggcagcccgg ggcgtctagg ggctgcttcc tgggaggggc agaggtgctg 22320
ctgaggctgg tgatccaggt gtgtagggca gggatggtga cagagaggca ggggcctggg 22380
gacagggccc tgctgggtcc ctccagctct gcccaaagct gctgagagcc aagcccaggt 22440
gacatgacct atgccacaga agcagcagag tgaggctggg gtgacctctg atgggcacag 22500
agcactccct cccaccacca ctccatccag ctgagcaggc caaaaaaagc cgcccagcac 22560
tttacctcca ggtgcctcca agctacccca agtgtagagg cacctacacc agccgctcct 22620
cagcacttgg actctaaatg cagccacggc acaaaggcca agagagggag catgccctgg 22680
gggtgtgggg gctggcgtgg agaaattatc tgaaccacca gcaccaccct cacttgccac 22740

tcccactcag agagtgagtc agattcaccc ctttccatcc tcatcctcat ccaatccagc 22800

agcactggct ctccatgtcc aggcctggcc acttccccac ttccccctt 22849

<210> 31
<211> 19938
<212> DNA
<213> Homo sapiens

<220>
<221> misc_feature
<222> (1)..(19938)
<223> chromosome 4 nucleotides 24575658 to 24595595

<400> 31
aaaaaaagac tgcaaatttt gtctgatgaa aaaatcctta aggaataaag gaaaaattaa 60

aacattttca gataaaagaa tttcttcctg actgtcctta aaaatggtaa aaggaagttc 120

tctaagcaga aagaaaatga taatagaaga cagttttgaa cttcagaagg gaaaaaacat 180

tggaatttgg aatggataaa attggggata aataaaacat ggtactatat tagaagcaat 240

agattacttc ttatgaattt cttaaattat atttgatggt tgaagcaaga attataacat 300

tttatgtgtt aattagtgta cgtagagaaa aacatagcac aatttttttt taagtggaga 360

ggataaagag acctaaatgc aagtaaattt tctactctca gatgggaaaa cacctatagc 420

agcagactgt gataagctac atatatattg taatacctag aacaactact aaaaatctat 480

aaaaagattc aagtcatgcc aaatatgttc tctgaccaca gtggaattaa atttgaagtt 540

aacgacagaa aaatctctgg aaaattcccc aatgtttcaa actaaataac acacttgtaa 600

ataacatgga tcaaaattaa caattaaatt agaaagtatt ttgaactgaa tataactgaa 660

aacagaacat ataagaattt gagatattcc taaagcaata attagggaaa aatttgtaac 720

actaaacacg tactttagac aagaagaaag aagatctcaa atcagtgact tcagcttcta 780

ccttgagaaa ctcgaaaaac aagggccaat taactgaaag taatccagga ctacctccct 840

gaaaagctgc gctctagtca ggagtaggcc gaagcggcct tctgacacag catgactcag 900

caggtttgga gtgcaggcgc aaaaccccgc acgttatgta accatgccat gtgaggtgca 960

ttaggtgatc acccacgtaa gctcatgctt ggcttggagc cactattatc tgtaaattta 1020

taattaccct gctaatactg tacatatggc tcaattacag ccagagagag agtaaagcca 1080

tgtcaaaact gtctacgatt cctcaagtgt ttttccagct acccaccact tgcccaccga 1140

ctcccctcgg acctcagttg gaacctaaca attggcatca caaacaggat cccgagctgt 1200

tgcaggtgag tacgtggttg gcacaagggc aacgtgacca caccctggtt gagtggctat 1260

gggcagctat atagactgtg tggaacgaca ctggtgaaat acccaaaatc atgagtaaat 1320

ggcagtcata tgccaaattg gtgcaagtca tccaggaaat gggtatgcgg caggctatgt 1380

ttgacctgaa tacccagggg ccagatgatg aacgtgtcac ctcccacaga agggatctca 1440

tgtggggttc tgtgtcccca agtgcctttg gctccctagc tgttgtcctt accccatata 1500

tagggcacca catacatgaa gtgaccactg ctatggtggc tcttgggcaa gcagaagtct 1560

gtcagtggga ctgaggggtc tgggatgtaa agaaggggaa gatgctccac ccacagtggc 1620
cccctgacag gagaaaggag ggccccaaca agtgacacac tcacagatgt ggatagattt 1680
gatttttgtc agggttgacc aagagaaaat cggtaagcag ttcaatgaaa ttctcttaaa 1740
tttgtggaga cagttgtctc tggagcagca atttcagaaa atgcccaagg gggagaagga 1800
cattgctgca tgacctggtc ctgcccaggc acttcagttc aaagacaact tgctgcagcc 1860
aggtagaaat gtagagcctt ttctgtttga ttagggaact ggccaaagtg cctagctttg 1920
gggaaacact gggcgactgg aggccacatg tggaattagc aatccactgg tcccccacca 1980
atgtgcggtg ggtgctgatg ctggtggata ctggtgcaga ttgcagcctc atttatggga 2040
acccagataa gtttctggga aaacctgctt tcattgacag ttatggagga cggtcagtga 2100
aagtgaaacc tgtatctcag caccctgaca tcggctgttt ggctgcctgt ttatatactg 2160
tgtatgtctc tcccatacca aaatacattc tggaggtgga tattttgcat ggcctggcat 2220
tacaagccat ggccagggaa ttcggactga gtgcatgtga cggacatatg catcaccagc 2280
ctcaggtcct gccacaacca cgatgggtta cttccaccca tcaataccat ttgctgggtg 2340
ggtatacaga gataactgag acaattaaaa agctggagga ggtgcagaaa atgtgtggca 2400
cccacagcac ctacaattct ctggtgtggg cagttagaaa gcctgatgga acttggcaga 2460
tgacggtgga ctatcaggaa ctgaataaag taacaccccc ttcgcatgca gctgtaccat 2520
caatcatgga tttgatagcc aatttgatga cagaactggg acagtaccac tatgtagtgg 2580
acttggccaa cgcatttttc tccacagaca tcagtccaga gagccaggaa cagttcacct 2640
tcatgtggga tgggcaacga tggacttcta cagtgttgct gcagggctat gtgcatagcc 2700
ccaccatatg tcatgatcta gttgccacag acttagtcac ctggcaatgt ccagaagggg 2760
tctagctatt ccattatatt gatgatatta tgttaacctc tgattctctt gcagatttag 2820
aagtggcggc gcccctcttg cagcaacatt tggcagcatg tggttgggct gtcaatgaat 2880
ccaaggtcca agggcctgga ttatctacca aattcttggg agttacttgg tcaggtaaga 2940
caaaggccat cccagaggct atcattgata agattcaggc atatcccagg cccaccatgg 3000
tgaagcagct gcaaactttt ttgggcctcc tgggttactg acagtcattt gtgccccatt 3060
tagcccagat gataaaacca ctgtattggt taacaaagaa gggagttatc tgggattggg 3120
atgatgcagc tgagactgcc ttcctggcag ccaaacgggc tattcaacag gcatgagccc 3180
tacaggtcgt tgaccagggg ctcccatttg agctggatgt gcatgtgacc acagatgatt 3240
ttggttgagg cctgtggcag tgcacggggt gcttgaaaat gctaggaggc ttttggtccc 3300
aactatggaa gggagctgag ctccagtatt ccttgaaaga gaaacagcta gcacctgtat 3360
atgccgtcct tgaggcttgt gagagtgtga caggatgggc tgcagtcatt gtgcagacga 3420
cttacccaat agcaggaatg gatgcattca cgggtaacaa attcccagac tgggacattg 3480
cagacatcca ctttggcaaa gtggggtacc tacttggagc agcagagtac gctgagtaca 3540
agtcccttag caacagagtt gtaagaggtc ttgggacctg tagtcctaat gcaagataag 3600
gccatggggc ccgaggcacc cctagaccct gagccttcgc catttaagga attgggtccc 3660

ctcattccca gtgggtcatg gtacaaagat gggtctagcc aaggtgttac tgctgcctag 3720
actgctgttg cagtccagcc tagtactaac accatacggt ttgaaaccag gtgtggacag 3780
aatagcctat tgggcagtat ggatggtgat caccaaggag gtgacaccta tggtaatctg 3840
caccgatagc tgggttgtct attggggctt aaccttgtgg ttaactacct ggaaaataca 3900
gaagtggcta gttggccacc aacccatttg ggggcaatcc atgtggtaca acctctggga 3960
aataggtcat cagaaagaga taactattta tcacgtgtca ggccatattc ctttggccac 4020
cactggcaat gatgaggtag atgccctggt gaaggtccaa tagttagagc tggcacctac 4080
atgagatatg accttgtggc tacaccagaa actgggacat gcgaggggta aactgatgag 4140
acagatcaat aagcatttga gcctgtccct gcccatgcaa gacatttggg aggcttgtta 4200
gaaatgcctg gcatgtgctc aggcataccc taaacagagg cagctgccca gtgttacaca 4260
agtaacgata gggtgagtgc ccttgaccag gtggtaagta gactaaatcc agccactgct 4320
gaagtcacaa gggtatatgc atgcgctaac agctgtggac acaggcctgt tgttcatcta 4380
cccttgcagg gtggccaacc aacagaacac catctgggcc ctgcaacatt tatgtggcct 4440
gtatggttgc cttctggcca ttgagagtga taggggaaca catttcactg gaaaacaggt 4500
acaataatgg gcacaacaaa tggacataaa gtggggattc catgtgccat acatcctaca 4560
agccccgagt ataattgagc gatataatga actcttgaat aatgggttac cctcatatgt 4620
cacacccctg tctttgcagg gctggagttc caagctggac ctggtgctcc taaccttgaa 4680
cgaacggcca cagaaaggca gccagacccc agtggaggct ttgttacacc agggcactgc 4740
ccccactcag ttgcagatac attccaagga tgacctcctt cgaccaggta tggggacgaa 4800
tggtaacctg ctggtggctg ctccagctcc cctgaaagca gtggaacaga aaacctggct 4860
gtggccctgt accctccaag cctgcacagc cagtggttgg ccattgtagc tcccttgggg 4920
gagggcctac agtgtgactt gcatttcact ccttcggtgt tcaatacatg gcccctgcag 4980
ttgactgttc ataggggaaa ggccagggaa agaactctcc tctgggggac atatgtactg 5040
tctgtgccac ctattatgag ctccccatga ctttggcaca gatacaggac tcaaaggaac 5100
cgtggggagc tgaggtgtgg ttttgcccat ggcaaaaacc cttggtggct gcactgttat 5160
ccagggatga aaggttagcc tatattttgc ctgagggacg tgctttacct ccatcagtat 5220
ctgtgcctac tcagtcattt ccgtcatagg ttaacatgct ccaactgcat tgtggactca 5280
gcccacacct acactgagat gaccggtgtt tccagctgtt ggatctgcac cacctttcca 5340
gcaacagctg tggatggctg gcctcggcac atacattcag catctgcaga caactgcaca 5400
tggctggaga cttggacacc tgtggctgat gcctggaacg caatgcagca aattttggac 5460
aaaggacacc tcaagaccca aggtgcaccc accccctggc tagcctgtag catttatgat 5520
gggtggggct ggctactggg gggacgtgta gtacctccag cccaggcatc atggtgcact 5580
gagaaacact ggggtaacac cactgaggag tgggtacccg tcatggcttg tgtgaacata 5640
acacatgtca ctacaccaaa ggtatagtga caaggtggtg gaacaagaag ccccaccaac 5700

atcacgccct aatggatttt gtgccccctg gcagtctatg ggtctgtagt gacacagggt 5760
ggccttacca accagtggac tggactggat gttgtacctg ggggtggcct catgtacctg 5820
gcactgttct ccccatgttg cccagactcc cacatacctg ggaggcacta ctctcttggg 5880
ttttgtgagt gcaatgagcc ccctggtggt cctacccctt ggcaatgact atccctggag 5940
tgggtgtcaa aactgtagaa gcacaagtta ctgctcttgc agagcacacc accagggctc 6000
tgaattacac ccgagtggcc ctcatcctgt tatggatgag gttgatcaga tgagaaaggt 6060
ggtgttgcaa aactgaatgg cctttgacat aataactgct gcccaaggag gcacctgtgc 6120
ccttttagga acacaatgtt gtacctttat ccctgacaat tggcagaata taacagcagc 6180
cctgcaaagg gtctcatggg agattaaggt ggtcaagacc cttactgacg accccctaca 6240
gagttggtgg gcatctctag gctctggcct atgctgggtc ctaatagtca taagtagcat 6300
agctgggatc ctagtagtga gttgttgctc tctgtgttgt tgttgtgggt tatgaattca 6360
gggctctgcc ctatgggctt gtgtccttgc ccgggggacg ccctcggcct gggaggtgga 6420
gtatgaggga gatgactgca ctttagtcag gagtaagcca aggcaggctt ccagtgcagc 6480
atgattcagc aggttaggag tgcaggtgca caaccctgca cattatgcaa ccttgccaca 6540
tgaggcacgt taggtggtca cccacatgag cttgtgcttg gcttggagcc actattgtct 6600
gcaaaaggta taattaccct gctaatgctg tacgttcggc tcgctcctgc ccagggagag 6660
agtacagcca tgtagaagct gtctatgatt cctcgaatgt tttcccagct atctgccact 6720
cacccaccaa ctcccttcct cttggcaagg acattctaaa gttaacctga aaaacaaact 6780
agttcagccc atgatgggaa gagggggcca gatatgcctc attataccat cctctctttt 6840
agaactactg atagaacaga ctctttaagt ctgataagaa acatttacaa tctattatct 6900
ttgaaacata ctacctagag gcttcatctg cataataaaa ccttggtctc cacaacccct 6960
tatcataatc cagacatttc tttctattga aaataagtca accagttgct gatcaaaaaa 7020
tctttaaatc ttcatgactt ggaagctctc acttccagtt gtcccacctt tccagaacca 7080
accaaggtac atcttacaca tatcaatcgg tcccttatgt ctccctaaaa tatataaaac 7140
caagttgtgg cccaaccacc ttgggcacat tctcagaatc tatggaggac tggtcactca 7200
ttttcactca gaataaatct cttcaaacat tccacagagt ttgactattt tcacagacag 7260
agggaaggat gacaaaggga cacaggaaaa ccctggctat catgaacttg tttgctgtgt 7320
tgattgtggt tgaggactaa attctgacct ttttcttttc ttttcttttc ttttcttttc 7380
ttttcttttt tgagacaagg ctctctgtca cccaggctgg agtgcagtgg tgccatctca 7440
gctcactgca gcctcaacct cccttgctca agccatcctc ccacctcagc ctcccaagta 7500
gctgggacta caggcacgca ctaccacgcc cagataattt ttgcattttt tgtagagatg 7560
gggtttttcc gtgttgccca gtctagtctc aaacccctga gctaaagcaa gccgctcccc 7620
tcggcctccc aaatgctaga attacagaca cgagccacca cgcccagcct tttttcctct 7680
cttgcccaaa ttcctaagag gcctggggag tcacacacta caaactgtaa aatctcatca 7740
gagacatttt atttaaacct gtctaactga ctgactttcc aatctgaccc tggcataaca 7800

tcacatgaca gataaagaag gaaatcaaaa cattttaccc caaagtatgt ttatttgtca 7860
cactttgaaa tagcctgcaa ctttgttttt gtggtggaat tgcatctgta acgaatctct 7920
gttaactaga tatttcccct tccagaccct cccaatcctg aagggactaa ctgagggtct 7980
agcacttttt aagggtttga gcaggaaaca ttcaccatct tttgcctgta agggtgggca 8040
cccatgaagc ttcatctatg taacaagaac cttggtttcc acaacccctc atcttgacct 8100
agacactctt ctattgattc caggtcttta gataataact taattctttt aatcaattgc 8160
caatcagaag atcttggaat ccccctatga cctgtaaccc tccccttcaa gttgtcccac 8220
cttttcagac caaaccaata tatacctcac atgtattgac tgaagtctta tgtctcccta 8280
acacatataa aaaccaagct gtaacccaac caccttgggc acatattctc agcacctctt 8340
gggactgtgc ctcaggcctt agtcactcat atttggctca gaataaacct gtttaaaaat 8400
tttatggagt ctgactcttt gtgtcgacat ggtggtggtc ccatgggtgt acacatatgt 8460
caaaacttat ccaattgtac actttaagta tatggagttt actgtatatc aactgtacct 8520
caataaagtg catttaaaaa aagtctggag aaaaagaagt ccccttttgt ttgcacttgg 8580
agctgtgatt aagcatgcct gaggctactg gcagctatct tgacattgtg aggagaaaac 8640
atgcttgaga gtgaggccaa cccagaggat aaaaagaact gaaaaaggcc agagttagac 8700
cagattccag ccatccatcg gtactggatc cagctgtgcc tacagcagtc tctggaattt 8760
ttgctcattg agacaatacg tgtgtgtgag tgtatgatta acccggtctg aactgagttt 8820
tgttggttac aactgaggag ttctgtgcaa cgttatccac agtccaccag gcaggctgag 8880
agctgcttct taaccagcaa gtgcctcatg gagggattgg gtttggggtt atggaagtga 8940
acagagctgc ctgccttaag gcaagtaggc cttgctctgt tccccttgca gtccccgagc 9000
ccgggaagcc cgagagggag cccttcagtg cttgtgcact cggctgtttc gcaagagtca 9060
cggtcagttg cgcctctgct tcggaagagt atttttagtg gcaaataaag tggtgcgcaa 9120
ataaacaaac gcctggaaca aatcctgcaa aggagagcgc ctgtgtgcgg cccactgtct 9180
aactaggtca gcctcgggca cagctccttt tgacagtttg ctccccaggg cggaaggtga 9240
cccgcaggtg ccggctgctt tccccaccaa tcagccatct gtggtttgag actcacaagc 9300
ttaggagaga ctcgggaggc atcagttccg catcccgcac tcccacgtga atttgccccc 9360
gccctgagtc agaaccctag aggggtgaaa tccctgaatt ctgcagagga aacccatact 9420
tctttgcttt ttcagcaatt ccaggagatg tcgtttttca agtggcaaat gtgtggctct 9480
agaggacaga gtaaccacct tgcagccaag aagccctgat gtcaactccc agctctgctg 9540
cagcacccag aaatcaccgg acctttctgc atctttctgg gtgactctgt catgaggatt 9600
tcataatgta attccctagc acataagaat ttctcaacca atatggcttt cttttttcct 9660
tctcactagt ctttgagctt cttaaaggga agacatgtcc tattcatctc tacacccccc 9720
acaccagtct gcattcagta agtgctcaat caatgtctgc tgaatcacat cttcccactc 9780
aactgcatgg cctcggatga tccaattctg tgggactcag ggtctccctg attctgaaga 9840

tctccatttc agccacccca tcctggtgct ttttctggag agactgggaa aaaggtgagc 9900
agaagagaat agaaccagga gagggagtga aagaggggtt gaaagggggt tgggggaagg 9960
aaggggaggg aagggaaatg ctatagtttg aatgtgtccc ctccaaaatt cagatgatgc 10020
caatgtgata gtaggaagag gtggtgcctg taagaggtga tgaggctgtg aggtttcctt 10080
ctttgttaat gggattaaag gtcttataaa ataggctttg cgcagcattg cactagcttg 10140
ctctcttgcc ttatgccatg tgaggttgca gcaagacgcc cctcatgaga tgccagcacc 10200
ttgatcttgg actttccagc ctccatagct atgagaaata aatttctgtt ctttattaat 10260
tacccaatct caggtattct atgataacta ttctgttata gcagcacaaa acagactaaa 10320
acaggaaggg aagggaggaa gacaaaacag aagataggag agaaagggaa aagaagggag 10380
gcaagacaaa aggagaaggg ggaaggagag ggaggggagg ggaggagaag gcagagaaga 10440
ggaggggatc ttgaggggag gggagagaat gtaagagggg agggaaggag aaggaagggg 10500
gaggaggggg acaggagagg gaggggaggg gaagagaaga aagcggggag gagaggggac 10560
aggagaggga aaggaaggaa aagtaggata gggaagggaa ggagagaaaa gatgaaagga 10620
gaggagaggg gaggggaaga gaggagaggg aaggggagga gagaggaagc atacgtcctg 10680
gggaaggagc tcattgatgg ggcttgggtc catgcccatc cctgaaccag tcactgtgca 10740
ctatcactaa gtcccgtgtc cacccctgca accatgaaag taaacacagt gtcaagggtg 10800
gtcccccaaa gaaaggagaa gtaacgggtc ccaggcaagc agaaaccaca gatgtcttct 10860
ctgccaatcc cgcatctgtc tttgaatttt gtaattagaa atgagttttt ttttctgatc 10920
aggcccatca tttaactcaa gcctggttgg gggtcctggg aagggcaaag tctgtgttcc 10980
agtgtggtcc atgcatacaa ggaagagaaa agaagagaaa aggagggtag aggaggagag 11040
gggacaggaa aaagaaggag aagggagagg gaaaagaggg gaagggaggg aaggagaggg 11100
aggggggaag ggagggaagt ggaaaagaga ggaaagattt ggtggcaatc atttgagccc 11160
cgtgtttagc tgtgcctact ttagattttt cagttatttg agccaataat tacctttttc 11220
cttaaatgag tttaattctt tttaagagta cgtggagctc aaagagatcc ttactaatct 11280
atctgggccc cttagagtaa ttccagggtc catttatcaa ggaaagcaac atggagcaca 11340
ggcagagtcc cccacttgag gccatctgca ggtatcctcc ttgcttgggc actgtgtggc 11400
cctggaacca ggcctgtgtc agggtgaagg gctggagaag gtggaaatgg gtatagaggt 11460
atcaccagga catttatcca aagtgtgtca tcaggctggg tgcggtggct catgtctgta 11520
atctcagcac tttgggaggc tgaggctggc gaatcgcctg aggtcaggag tttgagacca 11580
gcctggccaa catggtgaca ctccatctct accaaaaata caaaaattag ctgggcatgg 11640
tggtgcatgc ctgtagtccc agctactcag gaggctgagg caggagaatt gcttgaaccc 11700
aggaggcaga gattgcagtg agccgagatc gtgccactgc actccagcca tctgggcaac 11760
agagcgagac tgactccctc tcaaaaaaac aaaaaacaaa aagtgtgcca tcatctggcc 11820
tgcgctggta tagtgtcctt tctgaaggat gttcccagaa gtgttgtcag gtgtgtgcaa 11880
tggactccag ctctggagcc aagcggggat ttggaagcta tgtgcgattg tggagaacat 11940

ctggccgcta gaggagtgtg catggaccac actagaacac aggctctgcg cttcccagga 12000
tgcctatcca ggcttgatta aaggatggac ctgatcagga aaaaacaaac aaacaaacct 12060
catttctaat tacaaaatcc aaagacaggc atgcaggatt ggcagggaag acatctgtgg 12120
tttctgcttg cccgggatcc attatttccc ctttctttgg gggaccaccc ttgatactgt 12180
gttgctttca tggttgctgg ggtggacatg ggacttagtg acgatgtaca atgactggtt 12240
catggatggg catggaccca agccccatca atgagctcct tccccaggac attggctgga 12300
gctttgggaa agaggtattc tttttccatc gaggactcca agctggcagg ttggagatcc 12360
tcttagctat cttgtgtgga gatgctgcct aagatctact aagcacagac aaaagcagag 12420
ccatgcgtca ggaaatatcc atttctccct tgatccagcc attcctgaaa ccacgtgcat 12480
tcattttaca aactaacatt tcactcccca acacctctgt tatttatagt taagccagtt 12540
tgaatcaagt ctctgtcatt tgtggccaaa caaatcctaa ttaatattat ctgtgaatca 12600
acaagcctgt ctagctaata ccagatgttg aagatgataa aaaagatgat gtcgtggacc 12660
caaatgaaat acctaaggtg tggtagaagg cgcccagcac tggagtccag ctctagtccc 12720
agccctgcta cccctaaaga ggataaaata gcttaacctc ccctaaggca ttaccaatac 12780
agggatgata ataccacata ggtctgcctg ttaggaatat tgaccagatg ataatatgca 12840
tgtgaaagca ttttgaaatt caaaagctca atacacacat agtaacaagg tatgctataa 12900
atctatgcta cctggctggg cacagtggct catgcctgta atcccagcag tttaataggc 12960
caaggtagga ggattgcttg aggtcaggag ttcaagtcca gcctgggtaa cataaccaca 13020
atcccatctc tacaaaaata aaaaattaac tggacttgat ggtgcacacc tttagtccca 13080
gctactcagg aaaccgaggc agaaggattg cttgaaccca agactttggg gctgcactga 13140
cctatatttg caccactaac tccagcctca gtgacagggc aagaccctgt ctcaaaaata 13200
aataaataca tgaataaata aatctgtgct gtccaacaca gtagctacta actacctgct 13260
gttactgagc atgtaaaatg tggctagtct gagctgaaat gtgctgtaag tgtaaaacac 13320
acatcagctt tcacagactt gattttttta aataatataa atatcctatt aataattttt 13380
atattaattt catatcgaaa ggatatttgt gatctactga gttaaataaa gtacattgtt 13440
aaagttaatt tcatctgttt ctttcccctc atttaatgta gacaccagga aattgtaagt 13500
tgcatgtggt ggtttgcata tgtggttcac attctatttc tgttggcagc acttgtctaa 13560
attgtgaggg atggttcgtt gttggcgtgg gaggtcagtg aattctggag gagtcagaaa 13620
aatcagaaaa acctcagatg aagagacaag gatgggggta ggatggcatt tggaacccat 13680
tctccatggt tgttgtctct aattttccct gtgaataaaa tgaacagtct tccctttccc 13740
catcattcct cccaccattc gccacccacc gcccaccaga gaatgagatg gttttagcat 13800
cctgtgtgct tttcacagct gaaatgctgg caattttgcc acagattcag atctactcgg 13860
actcactggt ttcctttggt aaaatgcccc atatccagaa gggaagtctg ctggcttctc 13920
tgacctgggc gtgtctttag gatttctcaa taggatgaca tctgtggctg gttgtccaac 13980

actcatggct cccttctgga agcggtgcac tttgttctta ccctgagtta ccccgaaaag 14040

gagaccctga gaaggactgc atgcaaatgg tttgggggca caggaattgc agggaacact 14100

ttgagagagc agggaagtga gacagggaac aaatggaagc cagtaaaggg tgaattaatg 14160

aggccaccat gggggacatg gattcagtgt cttgggaaat accttgagaa atactgtgca 14220

gcatatctcc ttactgtccc tgtgtaagga acctgggata tttattaact aacttttatt 14280

ccttagtatt gagggtcaat cttggggtgt taactttctg gtatttccag cttgggtgta 14340

caccaccagg aacagggaat gccctcagac agaaagatgc aggcaatctt tgaagtatac 14400

aggaaatgtc tgcaagtgat cttcaggata ggctgcaggg aaatggtcag agcacagaca 14460

gggtctgcta taccttagaa gaaagagaga agacatggag agaagtgggg tccttggcaa 14520

tagtcctgat ctactggatc aagccttact tgaagcctac ctctagactt gtcattacct 14580

gcccctatat attccatatg ctatttaaac tggtttgagt tttctgttat tcacaatgta 14640

atgatttcta actcagtctt ttcctaacct ggttcctaca attgagcaga ttccctgtgg 14700

tttcaaaatc aggaaacagg gctgatgggt aagtcatgtc atcatttcct tcctgaaacc 14760

agagtgcaac ggaaagaaaa ggaggagttg catgggatgg tggccaagtt ctcattgtga 14820

cccagggacc attacagtaa aaggaacctg cctagggaac ttgctcagct ctctctaaac 14880

acagtcagct ccatgttccc aactgaatgt cacgtcaatg cgtcgatgtt aattccggta 14940

aactgaatcc acccgagaat cttgttgtgg aagcaaaatc ggcttaaaag gcttctttga 15000

tgcacaggga attctcacaa gcaataaatt aaaaataatc aacgagttat tttatataat 15060

ctgtatataa tataaagtaa ttgtgtccat aaataacgag gcagttaaac aggaaaagaa 15120

aggattttta cctgacgttc acatcgcttc acaaggttta gtggattcag aaggacatct 15180

ggactccagg aaaatttaag cgtagcattt tgagacttcc ctaaggcccc cttctctagt 15240

acgcaatcgc ggattcacca gcttgagtca gttggcctca gttttgcttt tggaggttta 15300

agtcgggtgg tgggaagaga tacatttcca ttcctgatat ggccacaaga gggagctccc 15360

aatggtcggc tgcacctgaa agtcctgtcc gtgaaaactg acaaagaaaa cttgatttaa 15420

aaggcgtctt ctgataaacc tcagcatctc cttactaaca tactaagagc aatcactgaa 15480

cacttttcgt atgttcaatg cgtcctcgcg ggatctgaga agcattgtcc tattaaatct 15540

cagtcgtctg attttcagca tcaatttatc ttgggagctg ctcctgcatt ttataaggtg 15600

aattctacag caacgagatt aaagattcag aaagtgagta aagttaacac tgagcccttg 15660

ggcctggtaa attgacaacg gagccagaat aagtatttac cattgtaaag ctctgatcct 15720

catggcacag ttgtttctga agaatgctac cagcacttcc ctgccctgca tttgtctggc 15780

tttttccatt catatttgaa tcgattctct ctctctctct ctcttttttt ttaattatta 15840

ttatcatttt ccagggactt tgggtcatta taagattgca ggtaatgaca gactgtaatt 15900

aagcctaaaa cagatttttc ttttaggaga cagaagaatc cactctagct gagactccag 15960

attgcctggg ctgtaattta cggtgctatc acaaaggggt cctttttccg catggtgctc 16020

tgcggtgatg agccagaagc tgaagctgag gggtgtgtga ctctcccgca cactctctgg 16080

agtgagcaga atggacgctt aaatggctaa gtatagcttg aaactgatta caggccagat 16140
cccactattg tgcatcatca gttgtcattg ttgagactga aattgcactg ggatgtttct 16200
ctttgtcacc ggctccaagc tcatttctag gacaggagtg aaaccgtatg gtgagcctcg 16260
ggcccaagca cagggagaat tgttcttctg gtgtgtcccc caaggaagag cttggcaggt 16320
ctttggcagt ggaagatggg ctggcttcag tcatgatacc tataacattg gtattgctag 16380
ggcttccaga tttatggata gtaaactcta atctctagca aagaaggttg aaattgtagc 16440
tcttcctcag ttgctgtgtg aacttaagca agcagcttaa cttctctggc cttagttttc 16500
ttatctgtac aacagaacta attatagtta ctctctgata tggtttggct gtgtccccac 16560
tcaaatctca tcttgaactg tagctcctat gattcccacg tgttgtgcga gggacctggt 16620
gggagataat tgaatcaggg gagcaggtct ttcgtgtgct gttctcatga tagcggacag 16680
gtctcacaag atctgatggt tttataaagg ggagtttccc aacacaagtt ctcttgtctg 16740
ctgccatata agacgtgcct ttcacctccc accatgattg tgaggcctct ccagccacat 16800
ggaattgtga gtccattaaa cctctttttt tgtaaattgc ccagtcttgg gtacgtcttt 16860
atcagcagtg taaaaatgga ctaatacact ctcctattct caaggtagat ttgcagagca 16920
atgaaatgag atatatgaag accccaagat atatatatat atatatatat atagatagat 16980
agatagatag atagatattc acacacacac acatacactc aggcactgta cacacacatg 17040
cacaaacata ataggcatag aagatataga aactacccat atgcccatat gtattattat 17100
gggtatgtgt gtgtgtgtgt gtgtgtgtgt gtgtgtgtgt gtgtgtgtgg tttcttggta 17160
aaagcactgg ggtaagacta tggagcgttc atatgctagg agatgaagtc tggacctgac 17220
tgcatagtaa cgataataat aataatcttg cattcagggc ttattgtgag ccaggcactg 17280
ttctaagctc tttgcatgta tgaaagcagc ggttttcaat agaagcatta tcagcattca 17340
ggcagaacaa ttcttcattg tcagtgggac tgtcctaagc cttgtgtgat taccaaaggt 17400
ctctcaagac cctcatcatc tctgctaacg aaaggcagcc caactcccct gcacttttaa 17460
aaacacccag ctgagaacca attgcctaag tatagatatg tactctccaa tatggaagct 17520
actggccacg ggtggctatg aatggcttga aatgaagtta gtctgaattg gagtgtgctg 17580
caagtggaaa aaaaacaaac actggatttt gaagatttgg catgaaaaaa atcgtattaa 17640
tattttaaaa gattgattac atgatgagat gataatattt tggatagatt agagtaaatg 17700
aggtatttaa attcatttca cctgattctt gttgcttttt tctttttctt tttttttttt 17760
tttgagaggg agtctcactc tgttgcccag gctggagtgc agtggcgtga tctcagctta 17820
ctgcaacctc tgcctcccag gttcaagcga ttctcctgcc tcagcctcct gagagctggg 17880
attacaggca cctgccatca tgcctggcta atttttgtat ttttgtagag atggggtttt 17940
ccccatgttg gccaggctgg tcttgaactc ctgacctcag gtgatccacc tgccttggcc 18000
tcccaaagtg ctgggattac aggcgtgagc cactgcaccc tgcctcttgt tgcttttttg 18060
atgtggctac tagagaatgt taaaggacag ccagagcttg catttgtggt ttgtgttcta 18120

tttttattag acagtgctgg aatggacagt gggatgctgg caagggcttt tgagccaaaa 18180

tggtgtcaga gtgactgtgg gggacactgc tcggccagcc cagccaatat tcgtttctgt 18240

ttcttctata ctagcaaaat cctgattgta tttgtgatgg tcttgtgttc aaatactctg 18300

ctccttagat cccatgtaac ctagtttttg tcaaaaagca taaatggaag tcaactgggt 18360

gggccatctg gggaagctgt tgttaacatg agaaaaagac ttggctgaca tgtgcctttt 18420

gccctttgcc cttccctcct tttgctgctt ggaaagcaga cacaatgtct gaaggtagag 18480

cagcctcttt gggaccatga ggttgacagt catattatag gaatggtgaa aggagggagg 18540

agtctagttc cttaagtagg actcttcatt atgtgagcag aataaacacc tcgtttggtt 18600

aggccacagt agtcggattt ctgttacaag agttgaacat gagcctcact gatcattatg 18660

gtttcgtttt gaagtcaatg cactggatgt tttgcttttt cagtctgtct caagcatttg 18720

agcatcttag ctaagtttgg ggaaattctg acgttaagag ttgggcctcc acatggcaga 18780

actcagaaac caccaatggc caagtccctc atggcatgag gctctgccag tcagtattcg 18840

attccgagaa actggagaag aggatctagt gtttagcccc aatagccttt taataaactc 18900

cttgtctgct tagccagccc aagccagcat caattgcctg caacctagag cactaatggc 18960

tgcagctggg agttagtaaa ggccctgctc ctcaacagtg tcttattaaa atcctattcc 19020

ccaggaaaag acacatgaat ccttaacttc tcagcaggaa acactgctct gagatcaggc 19080

acttccttac gttggaaggg cattgtaatc tgtgccattg gcaaaatgca agacaactct 19140

tattatcccc acttacctct gtctctggag gctgctgcag tgctgggttt acagggtgta 19200

ttgacaatca ctttttgaat tcaagttgct tgcgaggaat ctgggtagac caaacctggg 19260

tagggaaaca caacgagtga ggagttgctg gaatgccagt catctgcctc tggatggagt 19320

gtacttccca gcacacccct ctgcttcagc ccaagaatgt ttatgtagaa gtgagaccac 19380

ctggattcaa atcgtggcac taatacttac ccgctgtgtg tgatcttagg gaggttactt 19440

aaggtctctg agctttggtt tcctcctctc cagggttaaa gagtctgtga actgtgcaga 19500

cacttagggt ctcccccact tggcaatgtt ccctacttag tttcatgctt tgctgctacc 19560

atcttaaaat ttgtaataat ttttaacaag gagccctgca ttttcatttt gtcccaggac 19620

atgtaagtta tgtagccagt cttgcttctc tataaattga gaatactatt tatcacaaat 19680

atttgcagtg agaagtaaat gagaaaatgt cttttatgtg ctttgccaag tgtctgaaac 19740

tagtatgttt tcagtgaatg atggctgttc catgtcagcg cagctaattt tctcacagca 19800

tcctccctgg caggctaagc tccctgcacc ccctttacta acctccatgt actcagggcc 19860

catgtgagga caaattggac acccgctgca gcatcaggaa gtgggagacc ttaaggagcc 19920

aagaattccc agaagtga 19938

<210> 32
<211> 16921
<212> DNA
<213> Homo sapiens

<220>
<221> misc_feature
<222> (1)..(16921)
<223> chromosome 4 nucleotides 143690864 to 143707784

<400> 32
   tttcctgtag tgccagaagg aagctttgct ctgctgcata gtctggaaga caaattgata 60
   ttctccatag gaacagacag gatgattcat gctgtgtggt acataattaa ggtctgtgaa 120
   ctcaaaatga gcaaaccagg atgttagtaa tcttggtaga gaagtggctc aagagggcag 180
   gcatacctta ttctttattt taatctcttt gaggtaggag agttcaactt ctctgagata 240
   agggaactta tccagaatag aaattctttt caagtttttt ctctcctatc tcttgatctc 300
   tcactttcat cattatatat gtaatatata cacacacaca cacacatata tatatatata 360
   tttttttttt tactttactg gcaagaaact ggattttctt ctcatccctt gtacctttga 420
   agtgttcatt caattcaaac aattactgag gccctgcaaa gtactagggg accaagtact 480
   tcaaagccac atattaaaat gtggttctta tttccacaaa agcccagtct agttagggag 540
   agagatatgc aaataaatta tctgggtaat tatctggctg tgtcgctatg ggtaaaactt 600
   aaattccata attccagtct cctcaagtat aaaatgacaa aaatactact taccttgctg 660
   atggtgttgt gagtatgaaa gagttaaagt acataaatca caagtaattt gcttggcaca 720
   tatgaaataa cttgatacaa aataggaagc gataacacaa actagtagat attctaacca 780
   aggaatcgta aagagtcatg gaaacaaagg aaagaggagg cgactctgcc cagaagaagc 840
   aaccatttgt ggttttatga agtggccatt tgttgtttta tggctgccca gcaagcacac 900
   attctttctc tggcaacagc atccaatttc cttggacgac gcccttgtcc ccatactcaa 960
   tccatgtgtt tctgagggag ttgactccat ttttggttac atggcatggc aagaagcctt 1020
   ttcaaagctt cagcagagtc tgggcttaca aagggagcct aaatactaaa ttaaagtgaa 1080
   ctaaaagatg gaaagaactt gtggggccat tttccttttg tgatttcctt tctaggactg 1140
   cacagaaata aaagggagga gagagggagg gtaaagagac aaggcaggaa cctgggtctc 1200
   aaggtgtaaa aatgtccaaa agcacatttt aaaacaatga ttgcctaaat gttttactat 1260
   taaactgttt aaatttcagt gatgtaaacc ccttccatct cctcaaactt attataattt 1320
   gtgatataca caaaggtctc gtgtgagtgt ttctgtttat ttgtttttct ggaaagtgag 1380
   ataagtgttg agttctgttc tgtgttatca cgcagtagaa ctgtagcagg atgctagact 1440
   ggcaaactga tacataaact aaaccaactc agtggaagca gaagctcaga gtgacatatg 1500
   gccaacagaa tgtcaattac atatagaaaa ctttaaaaaa ttggatggca agaaggcata 1560
   agacttccct agggtatgga atagtaattt gagccaattc tatctatttt tgaggttaga 1620
   aatactctag ctgacactaa ggctatattt tagtcctgtt gtggttcatt tgaactctga 1680
   tgcaatagac agtcgcacct tgtttaaatc cctgctgtag ttctgaacta gacgcatata 1740
   aatgtcaagt ggcgggggac ctatttcaga gaagtagcaa ttgactcagc atcaaccaga 1800
   gtcctgggat ttctaccaca aagtcacagg aaagctgtgg aaagtactct gaaatcactc 1860
   ttacagaatt tgagtaagga ggctctttga aattgaatgc ttttggcaga aaaccaagtg 1920
   taagaaatac taaatgagct ctcaaaagca atgcttttta aattttgatg tgcgtgtgaa 1980
   tcaactagaa ttcttgctga agggaagatt ctgattagta ggtctgggta gaacctgaga 2040
   ttatgcattt ctaactggct ccccaatgat gctgaggctg ctaatctggg atcctcataa 2100
   tatagaacct tgaagtttat taagtaaact aacctaatta tatagagaat tccttaaaac 2160
   ttgaagtctt aaaaaaaaaa acaaaaagaa gaagaagtta cttactggga ccctgcactg 2220
   cctatctgag cacatgctta gagaaatgaa ttcctacatc agattggtga ttaggtaggt 2280
   ttttgagtct tttgtaggag tctcatatca ccaaaaccta agaatattct tggaaagtgc 2340
   aataccaagg atcagagaga atttggagaa agaggcaaca ttgatctctg agaatataaa 2400
   gaataaagtt aacaattgct accatttaat atttaccatc tgccagacac tgctgtaagt 2460
   attttattac attatgtctt aatattacag cagctccaca ggtaggaata attgtcatcc 2520
   caattttaga gatgagaaaa ctaaaactca gagagggcaa gttatttgcc caaagtccta 2580
   cagctttgga cagacatttg aacacaggca gtcctgctct atagtccagt cataatcatt 2640
   atgctctcta tatctcctac cttcattgca ccaccaaaat agcttttgtt ttggagatca 2700
   ataaatttcc attatgcaag acacaataga catttttggt cctctaaaca acactgtatg 2760
   ctgaagacct ctccttcctt ttctaagtat taccctatta tcttcccttg gtttttaagg 2820
   tttgaatttt tctggtttct gttctgtttt tctaaaactc ctctcagctt ttttttagta 2880
   ctatattccc ttacttggcc cttcaatgcc cagatttctc agagcagagt cagaagctct 2940
   tgtttcctca ccctgtagtt tcttccaata aacaatatta accactgctt tcattttcac 3000
   ctctacacag aagactccac attcccactc tcttttgaaa cctagactta tgtatccaac 3060
   ttgatgtctc tatttatatg tctgaaaagt taggtcaagt gcaataaatc taaaactaag 3120
   ttcaagatgc tggctccatc ttcaccttcc acaaagcagc ttgtagaaaa gattgctttc 3180
   tatgcccttt catatcccct tggctttatg attttgtgat taccccacat gacaagccaa 3240
   agggctagag aattgttgcc tgctagtcaa ctcatagttg acaggagagg agctgtggaa 3300
   taaatacccc agttctcttt tccttcagat gggatatgct gagacatagt ccatgctggc 3360
   ttctagggtt ccccaacagg catgagctac tgtactccac agtggtaaat accttgataa 3420
   tgtaacttgt attgctgttc ttcccttctc agtcctactt ccttactccc cattggtgtt 3480
   tcctggtccc aacccccaaa taaaacattt gcccttaaac ctgtcttggg gtctgcttct 3540
   ggaaaaagac caggtcaaga cataccttcc ccttgatttt cttatgtgtt catttctcaa 3600
   tgaatggctg caccgtcagt cttattccac tagctaaaca gccaggttgg ctgggagcgg 3660
   tggctcacac ctgtaatccc agcactttgg gaggccgaag tgggcagatc acgaggtcag 3720
   gagattgaga ccagcctggc caacatgctg aaactctgtc tctactaaaa atataaaaat 3780
   tagctgggca tggtggccag cgcctgtaat cccagctact ctggagggtg aggcaggaga 3840
   attgtttgaa tcctgaagga ggaggttgca gtgagccgat attgcccagt gcactccagc 3900
   ctgggcaaca agagtgaaac tccatctcaa aaaaaaaaaa aaaaagaaaa agaaaaaagc 3960
   cagagtcatc cacttccttc cactccagat ccaccatcac ccagaatgac aaatgttttc 4020
   tattaaaaag cttcaaattg cccacctttc catctcctcc accaactcca tagtccagta 4080
   ctattatttt ctcacttgca atagtaaaaa aaaaaaaaaa aaaaggaaaa aaaagaaatt 4140
   cctctaattg gtctcccata cctactcttg atctcctcaa atcagtagga atataaagca 4200
   actctgctca tgtcacaccc tccaagtgcc ccctatttaa accttttagt ggatttcagg 4260
   ctaatgaaag gcaagcttaa ctactggaca aggtactgtg agatttgttt tcgcttccct 4320
   ttctggcttc accttacatc aaactacccc tcataccctg ggctctaccc actctgaacc 4380
   ttaaactagt gtctttgtgt tttcaacctc actaatggat tttagtaagg tgtgaaagca 4440
   atttagtttg tcaaagtcag aatttcattt taatgtaatg ggagaaaaaa gagtgttatt 4500
   tgttagaact tttctttcaa atgtttgtgt actgcattca tgatgaacaa catatttgta 4560
   atcaaagacc acatccaaaa agagtttgaa aaatgatgca ttaaacatac catgtttttc 4620
   ccaccaatga gcttcagcgg atgctattcc ctctgtggga aaagctgtgc cccaaactct 4680
   ttgcctacaa attcctatat actctcttaa catcaactgc atccatgctg gggtcctaag 4740
   ggcctggggg atggcaagcg atcaggattt aaaagactgg cccccaggaa gcaccacatg 4800
   tagctgagga gaatccaaca catccaattg gagaaagtta aaagtaaaag ccatgaagaa 4860
   tccaaatatc agcctgcagg atggctgtga accatagcaa cccaaaagag atgggttatg 4920
   agacagagtg ggaaatagaa cctgcagatg tgcttcaagc catcagagtg tagccactgg 4980
   gacttctaca ggtcactcgg gcacactgtg aagggcaaat tgtagactga aaaagctttg 5040
   gcaaagatac catatttaga aagctagttc agcagccctg gtcagaggtg taagcatatt 5100
   tatttgtaag gagagcagct atagcattgg agaaaagagg caaacatgaa agatgttaag 5160
   gagggagaat ctataagatt tggctacaat gcagtctatc tcatgtattt attagacact 5220
   gaatagttgt aaatgggtat tttacagttg atttatacag actccagagc taactttcat 5280
   agggtgagga ctttgaaact ggttaaaaaa atttaaaaag catttcggcc aggcgtggtg 5340
   gctcatgcct gtaatcccag cactttggga ggccgaggcg ggtggatcac gagattagga 5400
   gattgagatc atcctggcta acacggtgaa acccagtctc tactaaaaat acaaaaaaaa 5460
   attagccggg cgtggtagtg ggcgcctgta gtcccagcta ctcaggaggc tgaggcagga 5520
   gaatggcgtg aacccgggag gcagagcttg cagtgagcca agatcatgcc actgcactcc 5580
   agcctgggca acagagcaag actctgtctc aaaaaaaaaa aaaaaaatta aaaagcattt 5640
   ctatgtttct gtctactagc agaaagtgca tgctgtgtgt gtgtgtatat acagatatag 5700
   atttatatgt atcttttcag caagcgagga gagttcttag ctgtgggtaa cttatgagga 5760
   tacagacaca agattgtaaa gatgggataa atttcaacct tttaattgac aggtaagaaa 5820
   ctgaggctca gaacttcatt gtgttacaaa ctaagagcca aagctgcaat cagaacccag 5880
   atctttagcc aggtagagga agactctgtc ctatcttcta tggttggtca aaggtgtggc 5940
   tcctgagcat gagtcacatg agtctcttct ttttatacta gcaagaccaa gaaacttctt 6000
   tggtgcaagt acactgtgtt cccactgtca ccaactgtgt gggaacaaca ccacatcact 6060
   ccttggtaac gaaggtaagt cattactggg tccctgactc cagacaggag ttctaattat 6120
   atgtaagtga agaaaaactg ttatttttaa aatgaggaca tgggttagaa ttatggtaat 6180
   ttaaatagaa gaaaaggaag tttggggaaa gaaaaagata gtttaatact tgggtagttt 6240
   tgggaataaa ggagaaagag gcataaagaa agaatctgag gcttggaggc tgggatgaaa 6300
   gtgggcacca atgaccaaaa tggggtcccc tgggaaggaa agtcagctca ggtcagagag 6360
   aaaaagacca ttagtccagc cttggatgtt aaccttgaga agagggggag gtatgcatgt 6420
   gatgatttcc tgctgatgtg ttatttagga aaaaaaatgt tttaaatcac ctaagacagt 6480
   tttatttcca atttgagtca gaaagacaag gttagcagtt gcagtagcag ggataggggc 6540
   ctaaaagaag cacgcaaatt gcaggggaaa gtatcgattg cagtttttat catctaagct 6600
   gatacctagg aaacctcgct atctcctcat atattcagtc aggagaatag aagacagagg 6660
   tctggaagag gctttatctt ctctttatta gatacatcaa aaccgataca gcacaaagct 6720
   gctcctcaaa gctgccctgt cgtgaccacc ggaggaccag tagccaacct ccaatattat 6780
   atccctagcc aacttttcac acagctgcac ctaagagatg ctttcaaata ggctgcctgg 6840
   agaggtttgc tggtgcagga gtgagcgtct tttaagcact cacaactgat gacaatgctg 6900
   gagaaggaag gccggcagtt ctccagggta gggcagctga tgggaaattc ggggaaacag 6960
   ttgtagagag gagaacataa agaatggata aaaattgaac taaatttaga ttcggatgtt 7020
   aagagcaaca tgaataagag gagaacaaga agagtatgta aaagctcctt tagttcccag 7080
   aaatgaaact tctatgaaag tcagactcac tgtaaagaat tacttcaaag acctggagag 7140
   acgcacagac cttgaagttc acctagtcta ccatctccca cctcccactc acacacagac 7200
   acacagcctc cacaaagaag aaactgaggc cagagaggcc acctgtccag ctggcagtcc 7260
   atgggcaaga gttagtggca gaatctggga atacactcag gattgcagac acatgctccc 7320
   gatgtccagg cacagttcca cactacagtg caaaacctgt ttaagataca gccttggtaa 7380
   acattaaccc caacatgtcg gaagttgtta tagatatcta aaaaatagaa aaaatattag 7440
   aggtaacaat aagtaatacc agtgatgaaa tataaggtgc aactaaaata atttatattc 7500
   catataaaaa ggaattgagc cccctaaaat aggtctatca gaaagggtag ggttccccac 7560
   tctaatgagc tgtctgttct tgcagagttt ctagttctat ctatgggctc attcacctat 7620
   taccccttct acgttctggt tctgcccctg agggccataa ggctgccaca aaacgaactg 7680
   ttcaacgaca aagtcaatcc tgtttctaaa ctctgttttg acctcactcc tagttctata 7740
   tctaaggctt tacttccttt gcagctcttg acctgcatct ctggtgccaa ccatggtaat 7800
   cctatttgcc aatttccatc ctaagacacg ggtcccattc tttcatttta aattctgtac 7860
   tgtacctatc atctcaaact tctggaagag cccacaagtt ctcatcatgg tatcattcct 7920
   cagtccaagc tcttttcaaa accttgaccg ctagattttg agctcagtct gtgtactcag 7980
   gtcacttatt gtccattttg tctgttgtgg cttgacagat atccagtctt atcttgctct 8040
   gttctctgga ttcatagcac ttatttgtcc tgcacccaat tccttacatt cccattgact 8100
   tccaaattta atcatcctga ctgccccaaa taagactttg acaatttggt tttggcagca 8160
   aaaaaaaagg aaaaaagaaa gacaaaaacc aaaagttagg gaggaaatag atcttctttt 8220
   agaaaaagtt attactaata tatctaaata taaagcctaa aagtgaagaa attctataat 8280
   ccataggtat tattcaggct atggtgtgcc actgttaaca aattcatctg gaaatctcag 8340
   tgggttaaca caagatacat ttatttctcc ttcttggtat aattcagtgg gtgttggggg 8400
   ggcagtgtgg ggaaaaatct actatgtgta gaaactggag gcctgggtta aaggaagcct 8460
   caattcagag cttgcagtga gcagagatca cgccattgca ctacagcctg ggcgacagag 8520
   caagactccc tctaaaaaaa aaaaaaaaaa aaaaaaaaaa aaagaagctc aattttttga 8580
   agcttcatca ggagtatgtg tccttgatcg cctgtcaggt gaagactgaa caatcacatg 8640
   gggtttttac tgcctcagcc tggaactgat atatatcccc ttctgtctca gctcattaac 8700
   tagaagtaga cacagagtcc cacccatctg ttagaacgct gcgaagccca acttctccat 8760
   gcagccaaaa gtggagaact gtaggtcagg aatgctaaaa atgtctggtg cacaatgatt 8820
   tcagtcgaca caactctgga atttttgcaa ctgtgtcagt taaaggatta atgcactctc 8880
   agttgttata gtgcttctca gttttagttg gtggtaaaca aagtgatttg caataaatgt 8940
   gataaaaagg aatgaatatg gactaatagg gtcataaaat ggcagtgcat tcatcaaaag 9000
   ctgagagaac ctaagatgtc agagagtggt ggtgctgttg caagtttttt tcactgttat 9060
   gcttttgttt atctttgtgg cttgagaatt attcaaattt aattcttaat atgtaccttg 9120
   tatttaatga atcaagattc attaaaaaat gaaacttgag attcatttga gactcacttg 9180
   aaattcattt aaaaaatgag aggcaaaatg gggcacttac ctctcatttt tacatgtaat 9240
   agcatataac attcaaaagc atcaaatagc aaggatgtct tgctattttt attatttcaa 9300
   catgacagca agtttgcaaa cagcaaaatc atatgcataa gtttttatca atattgtgat 9360
   ttaattttag agtgtcaccc acacttctac acatatttat cttgtaatca tagcccttta 9420
   aaaacaaacc tatgataact atatatatta tttatgtcac tagtttaact agacatgtca 9480
   ttaaaatact ttaaactctc aatgtataaa ttgaacattc tgccaagagt atactaaaaa 9540
   taataagaag agaaagccat tgaatttccc tttattcttg cttgatcttc ttcctattat 9600
   tatgttgaaa aaaagtttca gttacttatt tacatttcac tcatgaaata gtttttatac 9660
   actttaaaaa tctacgataa gaatagagct aatgccattc tgtccctctc acttgcctag 9720
   aggcatctaa gtggtatatg aggactggta tctctaaaag cctcatcaca tctcaggaca 9780
   tcacatcaga taagcagcag gaaataaaca taaacaaata gttcctctcc aatttcacag 9840
   aagggggttt atcaagacat gttttctcta ttgagattgc tatggaagtg aggcgctata 9900
   gtgagatcta atcctcaatg ctaacagcct gcgctttcct tccccgcctc agccatgtgc 9960
   ctctcctaag catgctgagc cggaaaaggg tcagcctaga catggccctc ttttctaaag 10020
   ctgtgacact gcatgggcac aagacaatca ggctcctctg tggaatttaa gatacttctt 10080
   aattctgaaa gctcacaaat actactacta ctgtattagt cagagttctc tagagggaca 10140
   gaactaatag gatagatgta catatgaagg ggagtttatt aggagaattg actcacacga 10200
   tcacaaggtg aagttccaca ataggctgtc tgcaaactga ggagcaagga agccagtcca 10260
   agttccaaaa cctcaaaagt agggaggcca acagtgtagc cttcaatctg tggtcgaagg 10320
   ctggggggcc ctggcaaacc actggtgtaa gtccaagagc ccaaaagctg aaaaacatgg 10380
   attctgatgt tcgagggcag gaaacatcca gcacaggaga aagatgaagc tcagaagact 10440
   cagcaagtct gctctttcca acatcttctg cctgctttat cctagctgca ctgacagctg 10500
   attagacggt gcctacccag attgaacgtg tgtctgcccc tcccagtcca ccgactcaaa 10560
   tgtatctctt ttggcaacac tcccacagac acacccagga acaatacttt gcatccttca 10620
   atgcagtcaa gttgacactc aatattaacc atcacaacta ctaattttta aaaaaccagt 10680
   ctcacatagg ttgataaaat cttgactgtc attttggtga aaattaaaag acctttttat 10740
   tatcatttgt tttttccaca gggaattact gccaaatcca tatcatgaat tccccaaaaa 10800
   agtatttaat aagacagcac ttaaatataa cagaaaatat atgttagagc taaactcact 10860
   aatagtataa tttacacttc tgcaaaagat cataatttaa tttttttatt caatgtggta 10920
   gaagaccaaa ttaataatta taaaattctc tactcactga tttttctgga tgtagcctct 10980
   gttatggaag aataaaatat aatactgttt taaaagatac ttgagattaa aaaaatactt 11040
   ttgaggaaga aattaattca caaaattatt tcacttgtct agatatgcat tttataaatg 11100
   gaaacattga aggagaaagg ttacatgact tgttaaagtt tctttgtttt tttgtttgtt 11160
   tgtttgtttg ttttttgaga cggagtcttg ctctgttgcc caggttggag tgtagtggcg 11220
   cgatctcagc tcactgcaac ctctgcctcc caggttcaat cgattcttct gcctcagcct 11280
   ctggagtagc tgggactaca aatgtgagcc accacacctg gctaatattt ttgtattttt 11340
   agtagagacg gggtttcacc atattggcca ggctggtctc gaactcctga actcatgatc 11400
   ctcccgcctc ggcctcccaa agtgctggga ttacaggcgt gagccaccac gcctggctga 11460
   cttgttaaag tttctaaagt tgatttgtga atggagaaag aaatagaatg tctttcttgc 11520
   aatgtcaatt caatatcgaa ttcaatattt tttttggtgc tcatcttctt ttaagatctg 11580
   taagactgaa atgtttatgt gcgcactgta aaaaaggttc tgtctaaagc ctgaaaaata 11640
   attttttttt gcaaagagtt tctatgttca tttcaaacct ttggcagtat tatatctaga 11700
   attttttttt gttctctttt tctttttctt ttttcctttt ttcttttgat taagatcctc 11760
   acttctcaca gggggaaaaa gaggaaaagc gaaattttat aaaagggcag ttctgaccta 11820
   ggaagaggaa agaaggtttt aacatagagg aaagctgagg tatatcttta aagtagagta 11880
   ggaggttctc actacatcat tttgctttaa gtggatgtaa aggaaaagtt accaagtgtt 11940
   ttcttgcttg aacaaagata ggtattttct catgaagctt tgaaaggttt tctagttgaa 12000
   aagtaatgtg aaaaacaata acttctgcca ctgaatgatt gttataagcc tcagtacctt 12060
   caataaaaag atgcttctgt tcaatttcca gtcacatcaa attctcatta cgagaagcta 12120
   aatcccatct ctccatgagc cagactgtat caaaagtagc agatttatgc attaagtttt 12180
   gtaacatatg cttacatccc atatagccca ggtcacaaag caaattctag gactttttcc 12240
   cctcaatgaa taccaggatt ggatgccaag cttcaaggag accaagagca ggaaagtact 12300
   tgtggacaag gtgtccttga aaaataagaa agacatccta ttgctttttg aaaacaaaat 12360
   tgtccagttc ccaacaggtt gcacaaaacc cattttgtgt taaagaaaac aaaagattta 12420
   taagagtatt atttagaatc tataagaaaa aaaaatagag agataataaa ataaagagaa 12480
   ataaagagaa aataaagtcc ctagggtaga aaaccaaaag gtcttcttaa taatttccac 12540
   gccattctct cttctcatgt tacttgcaaa atgttagata ttgctatttt tttttctaag 12600
   gtggataaga aaatcagact aaatgccatt gtagttgaat ttcccagaat gactgtggaa 12660
   attgggatac aaaggtacag acgtcaataa taaatgctag ggttcaacac acaaacttaa 12720
   gtcaaaacac ctctcaccct accagtaaag aggccaaggg acatgcccct aagatggctg 12780
   tgcctttaaa gacagctgtg tttagcagtg aagagtacct ctgctaggca gaaagatgaa 12840
   cactgctttt acaaagaaaa taggtcaact gatatgacag aatgaaagca ggattttgtc 12900
   aattttttta aggcagaaaa actgacaatg aaaggaaaaa atcaaggtta caaaaatgat 12960
   gtgttttctt attggcaaca tgtccactgt cgtacttaga gtccacagtt tgctgtgttt 13020
   gagagtgttg gcttcatttt aagatgtaac taaatccaag tatttaatgg aatggtacat 13080
   ccttacctgt gtttcccaaa acagatgcaa agggttctcc agaataggca aattgaaaag 13140
   gaataaggtg acagggctgg aaaagcagca gcaacatttt gtaacagaat ggtttgaaga 13200
   ttaaaatgaa atgaggccta tctataaggc aatagcagtg atagaaatag aaaattagaa 13260
   tccagtttat agttacactg taaattacaa tcatcaattc atatatatta gagccatgga 13320
   gttgattgca aaaatggcca taatacattt gagtatctcc aatcaagaac ttgagtctat 13380
   ttgcccattc ttgaatttgg ggtggtgttg ggacttgctt tggctaaaga aagtggcaga 13440
   aatgatattg tctagctctg agcataagcg tgaaaataac ttgcagtttc tttctcactt 13500
   ctccctctct ctctttctga gaacccgcca cctccatttg cacaagccca agtccagtct 13560
   agccttccag tggatgagtc atggcacagc tatggctatg gctctaccca acagtcagct 13620
   aatcccccga agcagagcct tttcactgac cagcagctgc ccacctacac atgggttagt 13680
   ccaattgtat caacagaaga actacccagc tgatccaaac caaatttcca acccacggat 13740
   ttatgagcca aacaagcagt tgttatttca agaccctaag tttagtaatg atttctcaca 13800
   caacaaaaga tagtagatag aatccatata gaaacttccc gcaagtcagt tagttattaa 13860
   cccattttgc taaaacttta ctgaatagga actatgtgca agcacgctaa caaagcttat 13920
   tcatagtcct ttggggaaac agaactccct ggtgtgtaat gtgaggcttg cagtttacct 13980
   ggggaagcca ggaaggcttg gagcagagaa cacctcagtg agggcttaaa atagattgat 14040
   gagtggatca agtgaagtaa ggtgggacag ttatgtcagg cagaggaaat gataaaggta 14100
   taaagaagta aaatagcaag gcataagcag agaggtacag gaatttatag tagaaaaatg 14160
   tagtgtctta aggaatggca aaaaaaaaaa aaaagaaaga aagaaagaaa gaaagaaaaa 14220
   aaagaaactc agcagttaat aagaaccaga ccatggaaga ctggaggctc ccataaatat 14280
   cttggattga agtatattga taaaggctta ggccagtagt cctcaaacag tagggtacat 14340
   gaaaatgatc tgaatagaac ttttgaggac tctcttaccc ataaattaag attcagcaag 14400
   tttttgatgg agcccagact ttgggcttta aatttttaaa ttttacattt ttaatatgga 14460
   atgttggagt atctgatgca ggttgttcat ggaccacact ttgaaaaata ttactgtagg 14520
   gaatgggagg agtccagtag tggtttaaaa aaatggagta actcaggcac tctcatatta 14580
   ttttcaggtc agtatttaag actgattttt cttaaatcag tctctggtat ggacaaaata 14640
   tataaggtta taatataata tggaatatta cttagtctat agaacatatt caaatttctc 14700
   caattgttcc accaatgctc ttttttggat caggatccaa tccaaacaca cacttcgcat 14760
   ttagttgtca tgtttcctta gcgttcttta atatgaaaca gttcctcagt ctttctttgt 14820
   gttttatgac cttgacgatt ttcatgagta ctggccaggt attctggatg cccttgcacc 14880
   aatgagccca gctgattccc agactttggt ttctaaatac cactctccaa tataagaaaa 14940
   ctatggctcc ttggagaaac agctgaatcc agggctgaag gagaaaaaat aagatgagcc 15000
   tgaaatattt tgtgctagaa aataccaaag agttaaaaag aaagaaagaa agaataggca 15060
   catttcaaaa agacaaagta gccaacttga ttggtgatcc taaaggtcaa atacagaaac 15120
   actgaataac aaaataaatc ttgatcataa gggattataa cctgtggaat aacgtaaggg 15180
   ttcatgtgtc tgtactaaca caaataagtg agtgaacaaa tgaatgaatg aataagtaaa 15240
   ggagaaaaga aaacttttcc ttactataaa actacaactt gtttctaagt taaggaatta 15300
   ttgaaccaga aaattaccat ttggcaaaca ccacagtaat agttgtttca ggcaagaatc 15360
   attaacggat actaaaatta gtggcaaagg tgtgataaat tacaggatat ttaaattctc 15420
   tcaaattatt ttcccatgag atacttatag agtacaaaga aatatagtgc ctttgtagtg 15480
   aagaaatctg gcaaacacca ccctccccaa gtgatcaaag ttaacatttt cagtaatgga 15540
   ataaattgac atcaagagtc ttcggataag acatgttgat aaggtcacaa cctcactttc 15600
   tggtatcctt gccaaaaatg caaaactgaa tttaaatatg agaaaacatc agaggaagtg 15660
   cattgactgt cagagcctca tcgacctagt ataagagaca caccaacgta cgtacactca 15720
   tatgtgttag aggcactttc agtttggcat tcattcagtt cacaaagaga tccatttaaa 15780
   gactttacaa cgtacgtaca ctcatatgtg ttagaggcac tttcagtttg gccttcattc 15840
   agttcacaaa gagatccatt taaagacttt acagaagaaa aatgacagga tgaaaactgt 15900
   gcctcgagga gattaaactg gaagcagaag gtaggataca aaatgaaggg ggtggaaatg 15960
   aaagctatgg aaggcattga tgcctgtaag tgatctatta cattatttca ggcacatagt 16020
   cataataatc tcaaccagag taatggccat agaaatggag aaaaggaaag ttaacacaga 16080
   aataatatga agaataatgg caatgatttt tgcattgaat gtgaaggaga tgtaaaagga 16140
   ggggaaaaaa gagtcttatt tactaaacag ggcacctggg tgtctatgca gaaggtgagt 16200
   caatggaagc agaaggaatg tgatcctgta ctttcaaaga agggagttct gtttcaggca 16260
   gatatttgaa ggtattattg agatatccta aagagaatgt ctagtaggca gcagaaaagg 16320
   tgagattgct atctgggaaa gagggataac acataggaat gattgccttt gatgtgacac 16380
   ttggtttaca gagaatatgt aacttgtcca gtgttacaaa gccagtaaaa ggcacaatca 16440
   gtatttaaac cccacttcta ggttcagaag tttctttggc ccatataatg ttctgtgtgt 16500
   attgggactc tccaggaggt tgaaaagaac tgtaaacctc tattactatc agtttctaag 16560
   tgcacaatta gacttatgtg caaagttata accagctcat tttttaaaaa agcaaaagaa 16620
   taagtgaagc tctcttttca tctgtgagtt ccattgggct ttatatagaa gtaaggacta 16680
   cgttcagagc cttgtaaggt ttttgatagt cacgtgattg actgtcagag cctggtccat 16740
   ctcagagacc atcccctcaa acgctatcct tttacacatc aggtatctga tcctgcacat 16800
   tcaaatattt atatgtaggt tagatgggaa agacattggt ataattaatt taattttcac 16860
   taggtctcag cctattttaa gagcagtcag gagctagagt tttcggtctt catttcctat 16920
   a 16921

<210> 33
<211> 34083
<212> DNA
<213> Homo sapiens

<220>
<221> misc_feature
<222> (1)..(34083)
<223> chromosome 5 nucleotides 166750597 to 166784679

<400> 33
ctgtgtcgtt gttcaccctg ttctttttgg ctgcagtggt atccccaaac ccacctaccc 60

atcacatacc tatccattat caaagaatta tctaaggtgc catgtcctct atgaaatcat 120

tctgttttcc ttaactacag gtgtactccc actcctttga aatttttttt aacattttat 180

ttatccattt catcaagaat ttaggggaaa tggtgtagcc ttcaccaaag tagagaagta 240

aatgatttga gatgaatttt ggagctagag tatacagaac ttgatgatag tttgaatgta 300

gtagttgaga aaagaaaagc aagagctctt gggtgggcat agtggctcac gtgtgtaatc 360

ccagcatttt gggaggctga ggcgagcaga tcacttgagg tcaagagttt aagaccagcc 420

tggccaacat ggtgaaacac cgtctctact aaaaatacaa aattagctga gtgtggtggc 480

ttgtgcctgt aatcacagct actcgagtgg ctgaggcagg agaactgctt gaacccagga 540

ggcagagttt gcagtgagcc aagattgtgc cactgcactc cagactggga aacagagcaa 600

gacttcatca aaaaaaaaaa aaaaaaaaaa aaaagcactc tcaaggattt ctaactttag 660

ccactggatg gattcctaag tattaagatg agaaatactt gaagaagata ggatttgggg 720

cttattcatt ttagacattg ttaagtaaga caactaagta gaaaggcaat gtggataatt 780

gtgtaattat taagactgga acctaggaaa aagatctgga ctggagatgt caatttgaga 840

atcagcagca taggagtggc ccatatatat ccaggaaagt agatgagctt acccaagggg 900

aaaggtagag agagaagagg agggagccag gagcaaactc tgagtagtcc ttaagtgttg 960

ggtggagaga ctgaaatcag tggtttgcag aaggtagttc tttgttcatt cttgttggtg 1020

tgatttttct ttagggttcc ctgatggctt ccggtgttgg tgtggctttt atcatctctc 1080

aacccacagt cttttttcct ttccattatt ctgtttcaaa ggaggcaaga ctggctgagc 1140
ctcatcagta caggagaaag agaaaaggaa tttaaaaagg gatagggtca caggaacaaa 1200
aataaataag ataagagttc tcatataatt gtaaactagt ttgatggttg attttatgtg 1260
tcagtttgac tggaccgtag gatgtccacg tagctggtta aatgttctct gggtgtgcct 1320
gagaggttgt tgctagaaga gattagcttt tgaattagtg gactgaggaa agtcaatggt 1380
ctcccccagt gcaggtctgc atgatgccat ccactgatgg cctgaataga acaaaagata 1440
gaggaaggtt gagtttactc tggctgattg tttaaagtgg gacagcggtt ttctcctgcc 1500
gtcagcattt ctgtctctca ggctttcaga ccaaatctat accataacct ctccaactct 1560
caggccttga aactacacta cgggttttcc ccagcttgca ggcagcagat gggatttctc 1620
agtctccaaa atcatgtgag gccatatttt ataatatctc ttcattagat atatattcta 1680
ttatttttgt ttctctggag aagcctgact aatatgttgt ctgtaatttc aaaatgtatc 1740
gagtaattca tggcaataac atttaactac aaacttttat tgtactcaga atatttattt 1800
agttagttat tgggcatcta ttgtctatta tgcttagagt atattcatta acaaactaga 1860
aacacatttt cctctccatg cgggtggatc aggaggtcag gagttcaaga ccaacctggc 1920
caacatggtg aaaccccatc tctactaaaa atacaaaaat tagccaggtg tggtggaggg 1980
cacctgtaat cccagctact cgggaggctg aggcaggcta attgcttcaa ccaaggaggc 2040
agaggttgca gtgagccaag atcatgctac tgcactccag catgggcgac agagcaagac 2100
tccatctcgg gggaaaaaaa aggaaaaaaa aagttaattt ttattaggca ataaaaaatg 2160
aacttgaaat aaagaaaaaa gagagaaaat agaagagagg gagagataaa tagaagggag 2220
ggatgaagga aataaagaaa aaactatgct atgtagtgat aaatggtatg ggaaaaagaa 2280
gacagtagaa atagatagga gtttgtgatt gtgagagttt tgcagtagta aacgggagtc 2340
ggagggccaa ggaaaacttt attaaaatgc tgacatttga atgaaaacct caaggaacag 2400
agaaactagc tgtgaaaggg aattgactat acagatttca ggggtgtggc atttcagtca 2460
ggtggaagag ctagtataaa gattttaatg caggcacact cctgctgtat ttaaggaagt 2520
taatgtgatt gcagcagaat gcaaattggg cagaatagca ggaaaccaga acagagaaat 2580
gaaggtgaag tcaggtcata gagggtgtca cagattattt aaaatggtgg cctttgctct 2640
gagggaaata agcatccaga cagagttttg agcataagaa tgtcatgatc tgacttccca 2700
tgtaaaagga tagctctggc tattgtgttg agaatatagt ggaagaagac aagagtaggg 2760
agaacaggag gctattgcag taaccaagac aagagatgac agtgtctgtc tgggacaata 2820
gcggtaggaa gaagtgatct gattctcaat gtattttgaa ggtaaaggta ggagtatttc 2880
tggatgtatt ggctgagaag tgtctgcaaa caattccttg aagattactc tcaatcctgt 2940
gcacatttta aaataaggct atatggtttt ctgatattga gttgtaggag ctccttatat 3000
attttggata ttaactcctt aactcatata tggtatgcaa atattttctc tcattccgta 3060
gattgcattt tcattttgtt gctagtattc tttgctgtgc agaagctttt gagtttgagg 3120
tagtcccacc tatttttgct tttattgctt gtgcccgtga tgtcatgttc aagaaatctt 3180

tgccaagacc aaagtcatga agttctttcc caattttttt taggagattc acagtttcag 3240
gtcttacatt ttaagtctgt aatcatttca catttatttt tgtgtatggt gtaaaagata 3300
aggatccaaa ttaatctttt tcacatgtga ctgattcttc tgacatcatt ttttgaggag 3360
actatccttt cttctttttg tattcttgga tccttgttga agatcagttg actatataaa 3420
tgtggcttta tttctgggca ttttatttta ttctattggt ctatgtgtct gtttttatgc 3480
cagtatcata ctgttttgaa cactattttg tgtgtcagtt tgacacatag acttataaat 3540
aaatataaat aaatgtatgt atgtatttat ttagttagtt agttagtttt gttctttgga 3600
gacagagtct cactctgtca cccaggctgg agcgcagtgg tgtgatctcg gcttaggagg 3660
caacctctac ctcctggggt caagtgattc tcctgcctca atctcccgaa tagctggaac 3720
ttcagactca cgccatcatg cccagctaat ttttttttct atttttacag acagggtttc 3780
accatgttgc ccaggctggt ctcaaactcc tgaacttaag tgagccacca gcttcagcct 3840
cccaaaatcc tgggattaca agtgtgagcc atcaggccag gctgtaatgt attttaaaat 3900
caggacatgt gatgcctcca gctttgttct tctttctcaa gattgctttg gctttgaacc 3960
aagattatgg ttctacgtgg attttaggat tatttgttct atttctttaa aaaatgtcat 4020
tggaattttg gtagggattg gatgcattgg aggtgtagat tgcttttggt agtatggaca 4080
ttttaacaac attaagtctt ccaatccatg gacatgggat gccttctcat ttatttgtgt 4140
ctcttttaat ttcttttata aaagttttat agttttcact gtacaagtcg ttcactgcct 4200
tgagtgagtt tattcctaaa tatatttttg ctgctattat agtgggattt tttcttagtt 4260
tccttttcaa atagttcagt gttagtgtat agaaatatta ctaatttttg tatgttgatt 4320
ttgtatcctg caactttact gaatggttta tcagttctaa caggttgtgg ggttgggata 4380
tttttctttt gtctttaggg ttttctgcat ataagattgt gtcatctgga aacggcgata 4440
gcattacttc ttcctttcta acttggatgc cttttattcc cttttcttgt ctacttgctt 4500
tgctaagact ctcggcatta tgttgaatag aaatggtaag agcatgtatc cttgtcgtgt 4560
ccttgatctt agaaggaaag tttttggttt ttacaaagaa gacattcaaa tggccaaaaa 4620
tatagcaagt cttcacttaa tctcatcaat aagttcttgg aaattgtaac tttaaggaaa 4680
acaaagtgca gtgactcttg aatgatgcca ttttgtttga catccctagg gtataaagtt 4740
gatgagaaaa aaaatgatgt ggttatacat cctttcactt aaactcagtt tccaggaacc 4800
tatagataat tttaagtgag gacttgctat atatgaaaag acactcaaca tcactaatag 4860
ccagagaaat agaaatcaaa actacaatga gataccatct catacccctg ttaggatggc 4920
cattatcaaa gcagtaacaa aagagataag tgttgtcaag gatgtggaga aatcgtagct 4980
tttatatact gttggtggga atatataaaa tgatacaacc actatagaaa aaaatatgga 5040
gtttcctcaa attaaaaata gaattaccct atgatctagc attttcactt cttggtattt 5100
attcaaaaga attaaaatca gaatctcaaa gggatagatg cacccccaaa gtcattgtag 5160
cattattcac agtaaccaag atgtggggag aacttaaatg tccatcagtg tatgagtgga 5220

taaagaaaat gtggtgtata catacaatag aatattattc agccataaaa ataagaaaat 5280
cctgccatat gatacaacat ggatgaacct tgggaacatt atgtgaaggg aaataagcca 5340
atctcagaag aacatataca gcatgcattc aattatatga agtatctaag ataatcaaac 5400
tcataaaagc acagagtaga atgaaatttg ccaggggttg aaagaaggag aaaatgggaa 5460
gttgttgatt aaataggggt gtcagtgaat gacagcaaga ataagaggta atgtggtgat 5520
aaaatctgat ggcaagagat gcaaagctga ggatgtctac agagcagtga agcatcatgc 5580
tgtatcaagg acaagaagca gtgtgtatct cttttatata ttcagtgcca gtggcatgag 5640
ggcacaggga ggaaaaactg ccaccacctg taactactcc agggaaagta gtgtccttag 5700
gaagaacaag atttctactg gagcgagacc atgaaatgag catttggaga aatggttaag 5760
acttttttca gtgattgaca tacagaggga cagtagacat gacaagggcg aaggcagaca 5820
gtgaaggtaa ggctttgaat gtcaagggct agggagagat gcatcttggg cacctttctt 5880
aactcctgat gatggatggg gttagaagaa ccagcaaaag tgtggttcag tcctggtgga 5940
gagtttcatt gttgatcctg gagaaatatc cccaatctat ttctcaatga aggcagtgat 6000
aaacttttga tgctggagaa gtaatggaat cttcctttaa taaaaagagt caagatgtcc 6060
ttggtgagtg cagagagaaa gaagaaacat tttctaacac actgtaagaa atcttgaata 6120
tagggacaac atcttattta tgtgtgtttc caaaggctac cacgttgctt gtcagaatga 6180
gtcacaaaac catggaaggg agggagggag aataggagag aatgcaggaa aatgaagaaa 6240
ggaaagagaa aggaaaatag aaaggcaaac tcaaaaaaaa tgaaagataa gaaaatatta 6300
tcttgttaaa accatactca tcaggagact taagtcctta ttcatttaat tcaacaaagt 6360
ttttctcaaa ataatcacaa aaacattctg aaaattaact aatgaaagaa aatgatagtg 6420
aaatcattta taaaacaatg tatatggacc cgtgattgat aggttttgtt gcaaaccgag 6480
cattgagtat gcgtgtgtcc atgattcaat ataaaactga cccttctcat tttgaagaga 6540
taacaatttt cttattttga agagattaat aatgtggtga attaacaaac catcatggaa 6600
gagattgaga gaaccatggg aaggtgatta tatcatacct tgtaaagaaa tatgtatctt 6660
attcataggg tacaagatac tgtttctatt cctgctgaaa gagtaggtta agaaatggta 6720
tctgcaattt gaagatacca gaatagcaac ttgatctcat atatctttgt ttgcttctct 6780
gaaattcgca tcaagaaaaa gtctctaaaa catgtctcag aatcattttc tctagaatcc 6840
actaagtggg ctttacagct ttaaaaatat caaagattta tattactatc tctcctagga 6900
aaactgtgcc tatgaccata gtcttgtttc aacttccaat gtatttttat gaagaaaata 6960
tgagaactat atatactcca tgcactgtga tgatgtaaca tactaatggg agaggtccaa 7020
gggcttagta gaaaaagtct agagatgtaa tttcttgtcc tagctctgcg actaactagc 7080
tatatggcca ttactattcc aagggcaaca gacctaaatc tctctgggtg tcaatatcat 7140
aatttttgat atgaagacaa tcgaggctat agtgcagaaa ttttggagtc agatagacaa 7200
gtgtttaaac cttaattctg tcctttatca gctccatgac attggcaaat taccaataag 7260
atctcaaaac actagtttct tctgcaaaat gtggataata attgtcccct tcccttattc 7320

tttccttctt tctttccttc tttctttctt tcctttcttt cttcctttct ttctttcttt 7380
ctttctttct ttctttcttt ctctctctct ctttcttact attttttttt tttttgagac 7440
agggtcttgc tctgttgtgc aggctggaat ggagttggca ccatcttggc tcactgcaac 7500
ctctgcttct cagattcaag tgattctcct gcctcagcct cccgagcagc tgagatgctg 7560
agattacagg catgcaccac catgcccagc taattttggt atttttagta gagatagggt 7620
ttcaccctgt tggccaggct ggtctcgaac tcctgacctc aagttatcca cctgcctcgg 7680
cctcccaaag tgctgggatt acaggcgtga ggtgctagga ttacaccagc acccagcccc 7740
ctttccctta ttcctgttat gattattaaa tgagatgggc tagtatatta agctcttagc 7800
gaagtgcctg gcacgtagta aacaattcat gcatagtacc gattattttt ggtgttactg 7860
tttttaaaat aatttttgcc atatttagtc aatgtagact gctttgaaag cttatagatt 7920
tatcacatgc aaatgattaa atgtgatttc tgtgtctcct tcataaccct tggctgtcag 7980
gtccttgggc agatatgtaa atccctgctc ttgaatcgat ccgacattaa aggcgatggg 8040
gattagataa ttttcactta aagtgactgt cctctttttc tgggaacctg tcacaagcgg 8100
gagttctgcc tcgtatatca aaaaggaatt ctagaaggtg atttcatttt tgctttcatt 8160
ttgttcttca aggaaaggga gttaaatatt aattaagaag tctaatccag taaatataga 8220
gatgcaacca tcccagactc acagagttga tgataaaagg agctgtgaaa gggatcaaag 8280
ccaataataa gccaggatga gcagagcgag gagcgagcga gagatgtggg atcaaaatat 8340
gcaagaagcc caactgcagg ctgtagtgca gagcgccttt aactacagca gaacgctgca 8400
gcatcactca ttctgaacac aagttatcaa acatttattg aatatctact acatgctgag 8460
aactagaaat acaaagataa atttcacaaa atccctgccc ccaagtaatt cacatcctca 8520
gaagaaagtc ctaaatggtg acaggaggtg gcatagcaca ataattaagc acatttcttt 8580
ggaatgagac tgtctgggtg taaagctcag ttttgtcact tcctagcttt tgggatgtgg 8640
aaacgacttc atctctctga gtagagtttt ctgcactggt atgatggaag aaccaagtca 8700
tgactttatt gtgaagagta aattagtaaa atatttgaaa tgggctcaat gtagtatcta 8760
ctacctggta taggcaacat tttacatagt atgccatgat taaaggttgc acaaagaagt 8820
taatatttgg gcctggcttt gagctagaag aacattgttc ttgtaaagaa acagaataga 8880
tcacatctta agctgagaat acaaagatac tgtaaattca gagctcttac atggtatgat 8940
cgggaaataa agaaatagtg gagtgagaga gaaattgaaa gtgagaaagg aaaatactag 9000
aaagttaatc ctgagccagc tcttggaaaa tgtagaattg catagcaaag tattttgact 9060
ttaccatgga agcaaatgtg ggtcaccgaa ggttattaac aaaggagtgg attgattaaa 9120
tttgcattta gaaaaattat tctaaatgtg gaatctacac tgagagaagc aggacttgaa 9180
ccagaaggag gaattagaat gccaagagtt gataatggtc ccatttgggc aatgagagtg 9240
atgggaagga gtgaggtgga ggagggaagt ttcagaggca acttgacagg gattagcaac 9300
tggtccaagt gggtagagac aggttcatag agagagggag gattctagag tgggttttaa 9360

ggactacaag taggatgact aggttagagt atagtcaata aatgagatgg agggtaggag 9420
gagaacatct gactgggaaa caggaagttt ggttttgaaa aatatgaagt atatattttc 9480
ttgggcttct gatgaggatg tggaaatagg cacttggaaa tgtttttatg gaaataatca 9540
gaacgtgaat ctggaaatta agataagaaa accatcaccc atagattata aatggggcta 9600
catcagggga tgagatgact ggcagaggga atataaaggt accagggcca aggacacgac 9660
cctagaaatg ctccctttta ggggacaatc caaggaaggt gagtcagtgg aagagatgga 9720
gaaaggatac tcagagaata gaagggtgaa tctatcctct tcttggcaga agggactatt 9780
aagtagagca aggtcaacca tgccataagc cacaaaatat ttaattagga tgaagaatga 9840
gaaaatgcct ttggatttga cactggtaac atctgatgga atattctaaa gtgaggtcct 9900
tgtaggtgct tgattaaaat gcctaacaaa ggctgggcgt ggtggctcat gcctgcaatc 9960
ctagcgtttt gggaggctga ggcagaagga ttgcttgagc ccaggaggtt gaggctgcag 10020
taaactgtga ttttgccact gcactccagc tcggggtgac agagtgacac tctgtctctt 10080
aaaaaataaa gaaattttta caaaacctaa caaagaggag ggtataaaaa ataaaagctg 10140
agcgaacact acgtcctgta catatgtaga gatcttgcat tttctgtact aatgcctcat 10200
atacagtaga caatcgaacg gtgtctgtcc catgctgtac atatgttgaa gttttcaact 10260
tcactccttt gcctaaggag tgatgctaac tgacaatttt catcatattc tgtccaggag 10320
gaccttgatc cagggcaagg atgcttatgg acaggagctg atgcttggaa ttcctcaaat 10380
tataattcta aaaattgtgt ttgttgagaa tcatgagcag ccagtaagat tctcataatt 10440
tatatagcat ttgaggatta ttattgaaca agaagtcttt aaatttttaa aaggacaaag 10500
tgtaaaatgt tctgaataaa aggctaacac acttagctta aaaatgtagt gcctctgcaa 10560
gcttaaaccg tgaattttaa tcaaagccac tgccctcttt gagggagtta ctcagaagtt 10620
gcatcagatt gttgatgata aataggcttc agcgattcat ttaaataata tttttaaatc 10680
taagaaaacc cctattttcc agctttaggt atggaaataa attatcctat tagaacactg 10740
acattctaaa ttttgagaaa tagcataaaa ctcacggacg aaatcagttt gtattattgt 10800
tggatagtta gggttgtttt cagttgtaac tttctcttgt ctttgtcatt tccctaagtg 10860
acagaggaga aaggaaggtg gataaaaaca gaacttatag aggatatatt ctccttccca 10920
ccccatgtca cctccccaac caacaaatgc atatttaatg gcccttccct cctttacttt 10980
gattatttaa tcatcatata gtgctacagc ttttttaaca gtccctttaa aataagagaa 11040
tattgtcagt accatttttt aaaataagag aagcagttgg ttttgaattg agacagacct 11100
gaatttgaat cccatctctt ctaagtaaaa gctgggtggt cagctgattt tttaatcttt 11160
ctgagattct ccttggtcac cagttaaatg acaaaagaga atataaatat ccattttaaa 11220
aggttgttgt gggattatat aagctaatgt attatatgag cactgcatct tccttgggtt 11280
ctattctcaa ccaggaggta aaatcataat cataggtaga aacatttacc cctgacaaat 11340
cccttaaact ggctctaaat ttgagaatgc atgggttcat gcatgcagtc ctattcagtg 11400
agaagtatgt atgaaggttt aatgcttata ataatacaca gaacataatg atgtgaacat 11460

gtatcaagta tggatatatt tagagttttg agaaacacat atggtaagca agagttagaa 11520
ggaaatgaca tgtctgactt atgaagagat taatgtctcc catccttcat tttggctgaa 11580
gtgtagattc tctctttctc cttctcccat cccctaacat gagggaattt tccagtgcac 11640
atagaatgga catcatgata caccaaaggc atgccacagt tcctagcaca taataaatgg 11700
taaatagcca ttcgtatttt taatgtcttt tattctccta tagaagggat tttcaaacga 11760
aaactgctct gtagcttaac aagggtttaa agagacttca gagccagcag catcagaaca 11820
atgagacatg cagccttgtt gcctaattca ctcttgggtg accagtctta aatgatgcaa 11880
tgtgaaatca tttcaattaa ttttggtctg tctctgtagt cttctgaggg ccttataaaa 11940
gaagtatatg cttaaagcta atacacagga aggcctgaaa gctccaaact gtgcagcttt 12000
tggtggattt gaggtaaacc ctttatgagg aacaagagag aatggatttg cttatggtta 12060
tagtttctct ctctaaagat ttaccagctt agaaaatatg tatatatgca catatgtgag 12120
tgtgtgtgtg taagtgtgtg tgatagttat tatttttttt aagcaaaaca aaatttcctt 12180
aattcatccc tgatattgag agaataatac taatacattt gggccatttg tgacttcttt 12240
ctcaaaaatg ctttttggga tgccaaacca agctgaaatg tctgccaccc ttgatttttg 12300
gtagtaaaaa aaaaacagtt tgaagtagcc tgaagacgaa acataaatca atgttattca 12360
tagcttactt atgctatgct aatatatttg ccataggcaa gaagtactcc taactaataa 12420
acttacttta aatcattagt tgaaatgtct ggatattcct tctaatgatt tccccagctt 12480
agttttgtca atgcttcgca ggtcttcatg ccttctacct gaacgtatgg gtcatggtag 12540
atagacagaa caaaaaactt ttataacatt ttaaaaactg ggattagaag atcctaaaac 12600
cttgtctgag tgctctgctg ttgatttctc agaggagtaa aatatacgtg gcaacttctt 12660
cagaaagaac atattaaagt tgaagtataa ggatttttag atcaaaagga taatgatttt 12720
ttaaaatctt tactatgtga taaattattc tgaaggcatt ataaagatgt acaagcttgc 12780
accagggaat aacacagaaa gttggtggtg actttgaaat tagtatagac ataacactaa 12840
cctccttcct tccttggcca agtttcttta tagcatgatg ccacaactgt tttttttttt 12900
tttaagtaat tttttcttat caccaaaaga ttttcttttt cttctttaag tgatatacac 12960
aagaacggct gcatgtttta aaacatgtgg aacatgatag gctcacctat tttccctcta 13020
tttaaaataa tggtttcata gagaattgta aattatttct cctcttaact ttcatgaaga 13080
aaatgacact tatattttaa accaatttaa ctctcaagat gtgccatctg tgcctctcct 13140
cttgtctcta ggccaagaga gtgttattaa gcttgaggat gggtagaaga gaagagtggc 13200
agagagaggc actggaggtg tttgagagag gcatgagggg agatgaaagg cccgtgcaat 13260
tggggaattt cctggtgcat ttccctgaga aaaaaactaa tgatggtgat ttggggttat 13320
gaaaaatgac tgaccgtcca tcagcatagg ataagatcag aagattcttc ataacgctag 13380
atacaggact cctgaaggca aataatatga ttgactatca catgggtctc atgcaaggag 13440
agttcatacg agttaaaagg ataaaagccc tggtgtagcc cactggggag agataaacct 13500

ttgaaggaag cccttttaac cacattcatc agtaaggcca taagggactt ccttggaagt 13560
ttatcacaat tgcttttgca tgaaactgta tttttcacca tggtctccaa tgaagataaa 13620
cccagtgctg ctcatatcat tgaaatctac agtggagatc tacaatgacc ctctccatgc 13680
gtgttggtca tctctttctg cttcttcatg ccctgctcat gtttcttcat gttcctcctg 13740
cgacttattt tttgtaacag catgattaag acataattca cataacgtgc aattcactca 13800
cttaaaatac aaaattcaat ggtttctagt atatcacagg cttgtgcaac cttcagcaca 13860
atcaatttta gagtattttt atcaccccag gaagaaattc agaactctta gcagtcattc 13920
ccattcccct ccacaactgc cagtcctaag cagcatccat caatctactt tgtgaccata 13980
gatttgccta ttttagacat ttcatgtcaa tagaatcatg caatatgtag tattttctga 14040
ctggagtctt tcatttagca tgatgttctt gaaattaatc cgtgccacag aatgtgtcag 14100
ttctttattc ctttttgttg acaaataata ttccattgta tgaatatagc atgtttattt 14160
tttttcattc atcagttgat ggatatttgt gttgttttcc ttatgggctg tttctttgat 14220
tttttttctt cttggcatcg tgtatgattt ccgaatagca tggtgcaaaa taggccttga 14280
agtttcctgc atctgggtgc aaactccagt tctgctgctg ataagcaatg gggctctggt 14340
caagttatgt gacttttgtg atcctttatt tctcattttg cataaatgga cagcccacag 14400
ggtactgctc atattgcccc ttggcccaat gtgaggatat aataactatt gaacattttt 14460
ttcatttatt gaatgagtaa tcacataata tgagccttcc caaggagctc ttatatattg 14520
tttataaagg taatctgggt aatgccgggc tcttacactc tattttaaat caagcaagtg 14580
aaggcttcag agacatctct tcgtattgac agtgtgctct gcaaaagatt cagcaaagct 14640
ccatgagaac tgtttctgtt catctcttcc taactcagcc tcaccctgta attttaggac 14700
ataagcccca cagactttta ccttagatat agtgcaattc aaacacacac acacaaacac 14760
acacacacac acacacacac acacacgcac acagagatgc caaccattcc tgagaaggaa 14820
cttgcagaat cttccatttc atagcattat ctgttgagac agaaataatg tgattgattt 14880
gccaggtcat ccaggatttc aaaaaccccc aaactaagcc attttacatt ttagatccaa 14940
agtgttgttt atcataggct gttaagactt tctcatttta atttcaacca gagaagaata 15000
cattgaatat aaacagggcc tcttagagca gtcagagagc atactgagag tttctctagt 15060
aatagagtca aaaatagtga ctaaccaagt ctttaagtag aaatgctacc cctaaataaa 15120
tgcttctaaa tgagatggca aaaacgaata gactcttgtt taattaaagg tacttgtaaa 15180
gtttgcttag agtttgcagt aatccacctt gtcagttctc atccgggatg tttaatgatg 15240
ggtttgcttt agtttccaaa tggaaatcag ctgctgcttc tttacagcct gacattaggc 15300
accctgtgga aattgtatta ttctccacat gcatattcat agaattaagt attatcaatg 15360
gacttaaaga gctcaattat tttttgcaat tgcatgctta gttgcttatt ctgaagtaga 15420
gtagattgag tatttcgggg cctggtcaaa tccttgaaat ataaatgtga atgtatatca 15480
gcagaaaatc accagcttta ctttatcagt tggattgtta agacaattgc caacaatatt 15540
atcccttgat ttatagttca caaaatgtct tctgttataa tgagcatagt ttttttcctc 15600

acagaactct gtgaacaaaa aagcattcta cagatgagga aactgattgt catccaagaa 15660
gaatctctta tctaagattg tataagtact gaagcaagat taaaatctag acattcctga 15720
cttctagttc tttattgcat tttctgcatc ctattgcttc cctggggaag gtcttatttc 15780
tgtatgtttg gaaataccta cttactttag atttttattt tagcttgtgt atgtgtcatg 15840
gtggtgtcat tttaattacc aggccactga accacatgga catttggtaa tagacagaat 15900
gctgcatttc cagatgctag agttagtgta attcttgatt aataatttaa tagaaaataa 15960
ggtgtttgtt tacaatgtta actaaacatt caaggaaaac acacagcagc aattgagatg 16020
agaaaacaca gaatgcaaac aggaaaacaa aagaaatgag gatggattaa ttctacatgc 16080
agttgaaata taccacctgc tttgcccttg cttttgggac attcaagttc atctctcttt 16140
ctccagtatc attgtcattg tgttagatta ttactgtggt ttgtagagct atagggactt 16200
tgtgagcagt gatctcaaac tcaaatatct ataagaacca gactaaaaat ataaataact 16260
gggtatatgc tggttataga tacctgtatc tatgttctgc aagtgatggc atctttagca 16320
aactagaaca taggtactag ttaaaggagc tgagctgtgt tcccagctgg ttgttgctct 16380
cttggaatta gtacagaatt tcccagatct tgtaattttc caagtaagtc cagatatcaa 16440
tatatttttt aacactgtga agtctcttac tacctgaatt aattaaatct aatgaaaaag 16500
aatcttttgt agaaaaaaaa atggccagat gtggtggctc atgcccgtaa tcccagcact 16560
ttgggaggct gaggcgggca gatcacctga ggtcaggagt tcaagactag cctggccaac 16620
atggtgaaat cccatctcta ctaaaaatac aaaaattagc tggtgtggtg gcacacacct 16680
gtaatctcag ctactctgga ggctgaggca ggagaattgc ttgaatgtgg gaggtggagg 16740
ttgcagtgag cagagatcat gccagtgtac tccagcctgg gcagcagact gagactctgt 16800
ctcaacaata accacaacaa aaaaccacaa actggcagaa attggcctac aggccattta 16860
tttccaactt cactcaaatg gaccttcctc ctagaaagga aaaccatcct catttaatat 16920
taagggacta aaaatgaaaa gaggtgtcag tatatacatt ctgatcacat ccgtacttgg 16980
aagttcatac aatgaattca tcaaactctc attcttcttc tggggaaata tctaaactcc 17040
attgcatttc cttattccac tgtggcccat tgtggattgt gtgttatatt tgaatatatc 17100
aaatctagca aattgacatt gaaaaccaaa catatcagag acaaactata gaaacacaat 17160
agagaaaata aatccacctc tgccaaagta tgatctttcc aatcatttca actcctaaaa 17220
gtagaaggaa gcttggaact ggaggtgtta aggagtttgc ttggaaaaag caatgaaata 17280
aatattgctg tctttcaaga atatatatgt ctaactcacc tcagggtttg gacctgggtg 17340
ttgaatgaac tgaggatctc tttggcaaga agctctagta gatgacaaat tcttactctt 17400
gttgtattgc tcaaagaatg ccccctactt cttgatagca tttctgaggt gccagagctt 17460
caggtgataa atgacatttt tattcttgac atgttactct ttattatatc attgagaaat 17520
aaagttcaat aagtgacttc tgataatact ttactgcggt ctttatgaaa gatcgattcc 17580
attaagtagg tacatttcag ttgctattac ttcaagtgat gccataatgt acttgaaatt 17640

cagtctttag ttaataatag caagcttatt gagtgtatta gagatgtgct aaagtactta 17700
cttttgggcc tggtatcagc caagacatag tattttagac ttgcaggctt tccatgtatt 17760
tcccccaaaa tcttatacat atatatatat aatttaatat gtatctatag tgtatatata 17820
tgtctaatat atatatgaca gagtatctta catatggcat agcattttat tatatatgtc 17880
atggtgtttt agacctgtat gctttccatg tattttctcc caaatctaat atatatatat 17940
atatatacac acacacacat acaatttttg aaatgcaaat atgtgtgcaa tcatttttta 18000
gggtattatc atttaatata ctaaggtaac ctcattgatt aaaacttgaa atatgacttt 18060
actcgaagtc acttgggaaa tatattgatg gtcatgctat tcataaaaat gaactcaagt 18120
ctacctgtct agaaactgag caatgtcact tgaaaagaat acagcagtaa gaataagaaa 18180
ctaaaccaat tatagttttt acaaataatt attcttatga tcaggagtaa tagttgtgtg 18240
cagtgaaaaa gggtgtaata ttaactaaag actagagttt gatttctctc agcttttagc 18300
actcaagaaa atgtgatgag aatgtctggg ctaagcagga tggataaact ttaacaaaaa 18360
agttgtaaaa acagtacaag gaactttcat atcctgaagg ggcaatttta aaagtaattg 18420
tgaaatttta ataacatagg gaagtactca ttcatctatt gttaagttaa taagcagcct 18480
aaaaattggc ttacacatta tagaaccaat taagctttaa aaaaacaaag agaaactcac 18540
acagaaagga gactggaagc atgtgctcaa aaatattaac agcggcatgc atacataata 18600
ggattataga tgattttatt tgtgtttttc atttttctga tttgttaaat aataaaaaat 18660
tcctgtatgc aaagaaagca tgtttaagta aaagtatatt gtattggtag tgttaaatgt 18720
gcttgtgcca tttttattca ggtaggggtg tgtgtgtttg tgtgtgtgtg tttctgcaca 18780
ttgataatag caaaatacca gttgtatcag tgatccatga tgaaattgtt aacactgatg 18840
atgctatttc actgtggaat tttcttgtga tgttgagaat cttctttgtt agttttttta 18900
gaagtaaaag cttatcataa ggaaagtatc aaaaaattag gatttgaaat ttgtaattaa 18960
tattaattta ataactcaac taaacaaatt acaaatttta ggatttcatt atgatgtagc 19020
ttcattgctt tagcatgaat ttctctctac catcagaaaa taaacttctt acctgtccag 19080
tatggagcat gtagacaatt ttgtggccag catgaaattt ttacagtgat gataccagca 19140
aacacctcca agatataatg tcagatctgg ttaatcagta tgttaaaata tgtttgaatt 19200
tgtcttgatt agaaaaatag caatcaaagc attcaactct tttcacttgt tgagacagat 19260
ggcctatctt tatgaaggca aatatccaaa aacaacaacc cactacctta tctttcaaaa 19320
tatggtgctc agaatcatct tggaaaagag ctatttaagc ctaataagcc atatgcttga 19380
tgaattgatt tattccattt tgtagtatag acataaaaat cttctttgca gcatctttat 19440
catatctgca ttctctgcaa ctcatttata aaaattagca aaacttccaa ataaggaata 19500
aattattata gagaattcag cttctgtgtc ttacaaaaga aaaaaaaaaa tgtgtctaag 19560
tgaacagtct ctgatttgcc agatattctc cccataaagt tctgtgtatt tgaagagcaa 19620
ataaactgac agcttccttg gtatgcataa agcaggtcta aaattattcc agaaagaatc 19680
cctcagagaa atgcaacttc cctgtcattc ttatgtatta tttaaggtag agattgacca 19740

tgggcataga agaaatatac aatgtttctg ggtcctaaaa gaagagagtt catatacttt 19800
ttaattttat gtattttaag atatatgtca gtgcattgta tttagcatgg aaaagaatgt 19860
cctatcaaaa taagcagcta ctcctgcatg tagtttagtg gcaaacccac ctattaacct 19920
ctagccacaa ccaaataacc ccacacttga aacctgaggt tgattagatg aacctttgtt 19980
ccccataaaa ttctttaaaa ttatacattt cattaaaaga ggaacatatt tggtcaactt 20040
agaaacacat tgttgtgcaa tagaagagca tggctttgtt attagatgga gatgcattct 20100
aaatcggaat ccaccatttg cttaactggg aactgctggg tacattatat aaactccctg 20160
aggctgagag aggataggaa actgtgatga ggtaaaaaat caaaactaaa ttttaaaagt 20220
aaagtatact tgacttttgc atgggatatt atacttacat aaatgtggct atttctgatc 20280
atctgaggta ctggatttcc aattattaga tcaattacaa attcctttgc ccaataaaca 20340
cataatattt gagaaagtct ttctttccac tttaattgtg aagcataaac aaaagtgtaa 20400
cctttgtgga gtacgctgag tctttaactg aaagagatgg cgtatcattg ttagaagaga 20460
gaccaagaag ttggtctaaa ttatcagtct ccaaaggcta tgatatttgt tacccaagtt 20520
gtgtctgtgt ttgtgtgtgc atgtgtatat gtaggtgaga gtgtgtgtgt gtgtgtgtgt 20580
gtgtgtgtgt gtgtgtatgt atgtgagaga gagagagata atgaggacca atttaagact 20640
tagcttaaaa tatctctaca ggattccagg agattggatg aagtttaagt tatgaagtaa 20700
tctaggaaac attaataact ctctctgttg gtctgcaaaa tcatgttatt aaaactcaaa 20760
ccatttggca agaatcatca tcaatgtatc gaatgcacct gaaacaatca cgagttaact 20820
tttccatctt tcaattatca tattcatgtc taaatacatt tgtgttgact gactgctgat 20880
caaactacat gtgtgcggaa aaggacaaaa gatagaaatg tgaatgttgg aaaaatactt 20940
actacttaaa tgcaagtatt ttgataaaaa acattatcag tgagctcacc aaggaaatgt 21000
tatctgggtg ctggattggt aaagaaatat ctagctatca aattgggtac agtgggattt 21060
aagggttcac ctggaagctc tatcaaaagt ttgcttaact ctagcagacc accaggatcc 21120
ctctggttcc agtgggcttt tagaatctcc aaagctctta gacttgaacc acatttcgtt 21180
tttggaaaca gactcagaaa gaaagaggaa caggagcagc taggaagtgt ttttagtaat 21240
ggttcagaaa gtggggtccc aggagcagca gcattatagc ctgaggagaa aagagaggga 21300
cttgttagat atggaaattc ttcggcccca ctccagacca cctgaatcag aaactagggt 21360
tagcaggccg ggcaaggtgg ctcacacctg taatcccaat actttgggag gctaaggtgg 21420
gtagattacc tgaggtcagg agtttgagac cagccatctt tcagtaagaa acatgattac 21480
tggaggatgt ggtataccac ctaggtttgt gtgcaacttt ttttgccgtg ataagattat 21540
ctattactca atcttagaag tagcaaaaga aattatggaa tgctttttaa ggcatacttc 21600
ttccttgaat cagtaatcaa ggaaaaacta tggcagggaa aatagaaagg ttaggcaatt 21660
tgtatttccc aatggtttga aaaaacagtg gctgttgcat ttctgctaaa ggctgctaaa 21720
tccaaattga tgaaactttt ggcccagcct tgcagaattc cctttgaaat gttttgtcat 21780

gtctgtcagt ttagtcatag tttaagatgt tacatgttta acttagggag atgagtgcag 21840
agtctgaaaa tatgtcatcg aaagcaaaaa aaaaaaaaag tgacttcaat ttatgacaaa 21900
aatacagcag agggactgaa ttggaatctt cagagagcat gagtatttga gcccctgcca 21960
aatgttaatc cctttcaatg gaaagaagga ttagaaatgt acattggccc tatatcttca 22020
tttaattcag gactactagt tatctagaaa catttttgtt attgtgtcta atttcgcact 22080
atttttggca tcattttcaa aaacctttac cctggtttat ttatttattt tttggctctg 22140
atcatcagta ttaattcaat tctatttgtt ttctacataa ttaaattgtt ctatgtgagc 22200
atcaatctat tacagtgtta caagttagtt cattgtttca aggaggtaat gttagtatat 22260
ctctgaaata acatgtaggt ctctgattga gtaatgaaat acgatgtatt caatttcttc 22320
tctctattta acctacctgg aaccatgttt gtcagactaa ctcaccacag agtgagcatg 22380
gataaacttg tatgaggtca aagcccatac cactgaggat actaatataa aagattacca 22440
atttgtgaca ccttgctatt gcattattat ccatgcttac gaggctggtt gcagtgagat 22500
gtctaatttg aattttctga accatgcctt taaggctcac atacctgagg tatttatcca 22560
aacacattgt aggtttacct ccctcaaata tccagccatc catccgtttc cttctgagca 22620
gagcaataaa aatcaagcca gaccctatgc tttacgtata gaaccagtaa gatttacagc 22680
caggctgagt ataaaatcat aaaactttca gtacgtggtt aatatataac catcaaaata 22740
acagagtgca attctaaatt tatgaggctg aacttaacac tgaatatcaa ataagaatca 22800
cattgatctc tgtagcggat gacttaaaaa cacattaggt ggcataaact gcaagacagt 22860
acattgattt aagcataaaa tgcatagcgc tcaatacttt accacctaac gtttctcagt 22920
aatgaacaaa gctgttgtat attgatgaaa ggaaaattat ttgttttgtt atattacagt 22980
gctcacagag atgccttgta tttaggctag gcaaacatgg tttgaatcac atttagcaaa 23040
gggtcacatt ggatggacat tttactgaat ttatacttgt ttctcaaaat aactgtacag 23100
aagcattatc acgatgacag ttttctcatt ttctctttta acagaggaaa gattcatacc 23160
aggtagatac ggactttttg tagaaataca ctttactgta agttttctga taaaggcaat 23220
tacatggaat tttaaggttg atctctctaa gtgcatgaaa tgatcttgta aaattaccat 23280
attcagaaaa tctaaaagca tattttaaaa agaaacaata attttatggt aaatacatat 23340
tttataaaaa tctttactgt agaaggatct ttaaaaaatt gattggaatg aggaatatta 23400
ggaaatgtta atagctacag gtgtgtccat gtaatataga tagcaaaatg gataaaattg 23460
gttctgatat ggggtgaaaa acaaaagcta acactcggca agtgatttag ggtgatttat 23520
ggcgtgattt atggaggtag taggggccct ggttcgctag agagagagca cccacttctc 23580
agcttcctct atctgattgt tgtgtctggg tgtatgaaaa tatcagatct tgggatctgc 23640
aataaaagtg agacatacag atttatctgt gaagtctcct gattttaaaa cactaacaga 23700
tgatttaaat gggaaataag aggtactgat caaatgtaac ctatcttagg tcgatttcca 23760
gcactcacac tgctggttaa agatcactca tcttcaggct tctgggaacg ttgcttcatc 23820
cagaggagca agagttatcc tttctgttgg tggaaaaaat aatcagtgga agaatccgat 23880

attcaaattt tcataatttg cttgggaaat atcaccttgg tacatcagtt aatattttac 23940
taaatgagct gtgttgtatt ataagtttcc atcaggcggg tggtagcttc aagactttgg 24000
tgagaaaagc tcctttgcaa gttggttctc tttcctcaca agtatccaca ggtaaagaat 24060
tttgccagga ctcagagaac agcattcact cactgaagtg ttaatggcaa agcaaatcta 24120
ggtgcaaagt tggctgacag gtccgccaca ggaagatttc tgtctatcac catttcaact 24180
gtaggatagg taagtccctt ttcatgaaac agtgctggga ggaaattgta tcattctttc 24240
caataagtga ctctttaaat gaaaaaaaac tactacagct ttaactttac attcaaataa 24300
atgatatatg catatttgta gcatgtattt tacctgttgt acactttcta gcattatgtt 24360
ttcaggctag ttttccttca taataaatta acttattact cttcatgaca tgcaagatgg 24420
actgtgatgt taaagaataa tgttttctaa ttatcattga catttgcaga gaagattaaa 24480
atttaataaa attaattgtc cattcttgaa tggcttactt tgcagttaat catagaactc 24540
tgatttctct tactcttatg aaaacttctg aatttcctgt gtcttattaa tatccatagt 24600
aaacccagca cttagaaagt aagttagatt ttttccttct gttgacattg ttgaagcaaa 24660
aaaaaaagta ctgattttga aaaaattcta tggttgaagt ttttcaactg aaacctttac 24720
cagtaatctc acccatctta ttaggaaaac ctaacttctg gaggttatgt tgaatggtga 24780
ggaatgtatc ctcttcgccg tggtacatct ctagtaatat aatttgaagt gctagggccc 24840
cttgcatcca tttctgaatc tattattagt tgattaaatt agctggctgg atcagtacag 24900
tgagaagaga cgcagtaatg atattataga attagtatgc tatgatgaat tgtcaggaga 24960
agtaattgtg acagccctgt ggccatatgc acttagccat gcaatttata gtcatttgga 25020
gagccaagga cttttcagtt tgggttagga aatttgctcc tgcttttgac atagtttgag 25080
ggatatgtgt gtaagagtga agggggtgct tagcagcgat tcaggaagaa atcaaaagtc 25140
tcacttctta gccattggca tttggatgat tttcagggaa taagccagtg tgtggatgcc 25200
tttattgtaa cagtgccaat tcctttaaat agatttatat gatagtgccg ttactttatg 25260
gttgctgaag ataacgttac tttgggtaag ataaaagtaa atgcaatctg gtatcacaat 25320
ttcccttgtt atggaacact gtaaattctc atccatgtac tgcatttaca gcaatcctga 25380
aaacattgct ggggttgttt tgatttaaat acatacgggg ctcctaattg caagtagttt 25440
ttaagcaact taaaatataa cgttactttt tgtttgtttg ttttgcaact atttgcaaga 25500
taaacggact gtcaggtgcc tctaaaaggc ttatgatgac agcagttggc aatgcaaata 25560
ttggcacgac accacattag ggcaaattta ttgcagaatt accaaaacat gtataactgt 25620
gaagtcacct cagtacaact attatatgag aattactctc ttgctgtttt tgtttttgtt 25680
tttgtttttg ttttgttttt tcattttatg actaccaggc tacagtaata ttcagtagta 25740
caatgaacat gcttactgac attctaactc caaaccagat tcatgcagaa aactactcct 25800
ttttctttta gctgtctata gatattttct gtgtataatc gatcgctttt atttctcaaa 25860
caggattttc atttgattac agatgattgt ttcgagatca ttcaaatcaa tcaaatgatg 25920

tttgattaca tagattgttt cgtttttctt ttcttctctt gtcattgttt tatccattgt 25980
taactcaatg tcaatatttc aaagataatc tataatcaaa tattgtctta ggatcttatt 26040
ttatgtctta gccccttctc catgtcccta atcttaatca cccagaatta cacatttaga 26100
aacacttctt cagtaattct gtagttgcac atttcacttt gaaggtaata gaagggtgtc 26160
tgtcacttct tcatttctgt gtccttccag ctcactagca ctcttatctg ggagttattt 26220
ttgactctcc tccttcacct tccaagtcta atttaacacc aacacttgct gattccttcc 26280
cagcacagtc catccctggc ccctgccatc attctatact ggagtgcagt gctcccctct 26340
tagtccatct ctctgccatc tcttcccaga ccagtagaac ctatatatac tgaattgcca 26400
gatggatctc ttaaattttt attatagccc ctcatatttc ctccatgtga catgaggcct 26460
tttgcttatt cttattccag cctacagaac ttatgtgctt tttctctcct ctatcttacc 26520
ctttccctag cacacataga tggctaacta taattgtaat ttataaattt ttgtgccagg 26580
tactgtgcta agtgctttcc atacattatc tagtttaagc atcatggcat tcctacgtgg 26640
taagtggtgt gattcccttt ttatagtcaa agaaacagag gtgtgaaaca acttgcccac 26700
caaagtgaag gtgtaagagg aagagccagg gcccaaaagt aggtctgtct gactttgtag 26760
tgaaaccatc aactaccgtg cagcactggc accctggcta cacgtggttg tgttgctgtt 26820
tccctggtgt ggatttgcat tcaatattca tctttttgat aatttgtgtt cctcctcccc 26880
tttgtgtaca tttggagact gaccccacct ttcattttgc attttcatat ttgttttact 26940
gcattaattt gcattgaata atgtgtcact aagtatttgt gcttgtacac gtacatcctc 27000
acctagacca caatcaaatt aaactgcttt ggagagctgg gcacggtggc tcacgcctgt 27060
aatcccagca ctttgggagg ccaaggtggg tggatcacct gaggtcagga gttcgagaac 27120
agcctgacca acatgatgaa accccatctc taataaaaat acaaaattag cagggtgtgg 27180
tggcacatgc ctgtaatccc agctacttgg gaggctgagg cgggagaatt gcttgaaccc 27240
cggaggtgga ggttgaggtg agccgagatt gtgccattgc aacaagagtg aaactgtctc 27300
aaaaaaaaaa aaaaaatatt gctttggaga aacgtataga atcaaaatat tttccattct 27360
tcaatacaat actttggact tacagtccct aatgaaggtg gacattcaac ttactaattg 27420
ttcctctttg tcagggtgac tttgttgcta tattatattt taatattttc atttggtaaa 27480
ttgcaggaaa attagattgt cttctatgaa gcttgagaaa agctaagttt tgcaaacaag 27540
ctcctgacca tacagcagta gccagaacaa tgaacaggac atgaacaata aagaatctgt 27600
gaccaacagg aagatatgga gatttgcata cttttaaaac cttatgtgat taatattaga 27660
ttcaatcttt acatttcttt ttacaacatt ttgttccata aaaaatgtca agcgtgtgtt 27720
tgctgggaag aggaagagtt cattattctt atggttttct tcattatatt ttgaaaagcc 27780
aaggatgggg aaatcaacat tggtaaaagc aattagtgat gtgggttgag gtgcgggaat 27840
tacatcctat cccttggcat caagaataaa taccaattat tatcatatat agaagtgtgc 27900
ctgtgtcgct taaaaaatta tgattgcttc aagatagttt aaacattatc atctaataaa 27960
agcatattga gaaattagat aatttgacta aagtatataa ctatactaag caacctaaac 28020

ttaaaattat gtatatattt acatatacgt ttcatatcat aaaatacaac atactatatg 28080
tatatacaca cacacatgaa aacttagatg tgtatgtaga tagatagata gatagataga 28140
tagataatta tttaaatatg ttggtaggcc aggtaaggtg gctcatgcct gtaattccag 28200
cactttggga ggctgagatg ggcagatcgc ttgaggtcag aagttcaaga ccagcctggc 28260
caacgtggtg aaaccccacc tctactaaaa atacaaaaat tagatgggca tggtggctca 28320
tgcctgtaat ccctgctact tgggaggctg aggcaggaga atggctgagc ctgggaggtg 28380
gaggttgcag tgagctgaga tggtgccacc gcactccagc ctgagtgaaa gagtgagacg 28440
tcgtctcaaa gtaaataaat aaatatagat agatagataa ttgggcagat gggtatttag 28500
aacgacaaaa tttcatcagc atcttttccc gtttcactta agcttctgaa aagcccttct 28560
tggtcagaat gactgaaaca aagtgatggc cttttcaaag ttacataata tattttcagc 28620
acatcccctg actaacacta tgaaaagtgc atccattcta gagtcagaca ggcctaagtt 28680
acaattctgg atttactcat cactttctgt atgtttttaa atgagttatg caacttctct 28740
tcagtaaacc aagaataaat atacttatga tatgaagtta agattatccg aaaagggaaa 28800
actcagccct catttgaact aaaatcagct taaaaggcaa attgtaagta acaagagggg 28860
acttccaata gtgagcaagt tctaccaata tagtaagcat tcctggattt tgcagcagca 28920
gagagcatga ttggaacatt tcaatgagaa tttatttggc ataaagggag ttagaattaa 28980
gttgaacttc gaagtaagtg taataattaa aataataaac gaaaagaagg caaaaggaaa 29040
agatgtaggt atagagtttt atactttgca aaaggtcacc aaaattcctg gtgaccagtt 29100
gcttaatagg aatagggaag ccagatgcag gagtagtttg caacttggaa agcacgtgtc 29160
cgtgaaaagt ggagagctta ttgttaccct gtgtgaagga aattgtaggc tatgagacct 29220
tttaattttt ctagactatc ttaaaatcca gttctcaaaa acaatctgat ttttaaatga 29280
tgtcaaatta ttcaagcttt ctgggaccaa ataaactatg tggactccgg gatggtggcc 29340
tctctactgt actgtgaatg aggacatgta tagctggtaa taagtatgat ggcaagtgga 29400
gtggagtggg ctttacaccc ttctcagcag gggagccctg gaagttctat gacctcacct 29460
cctaccagta ttcttctcac tcactcactc acttccagcc atgcggtctt tgaataagcc 29520
aatcccattt ccaatttagg gagttttgca ctaagtattc ttcctgcctg aaatgttctg 29580
cctcaggtat cttcatgtct aatgttgtct ccttcaagtt cagatgtcac cttctcaata 29640
aggcccaccc tgaccattgt gtaaacttgc aactgccttc ccatccctgt tttatcatga 29700
tacctcgtct ttttccgaca gcaccatcat tctctactat agaatttact cccctatcac 29760
aatttcttct tattatcacc ctgtccctac tggaatatta gttccagagt ggcaggaatc 29820
tttgttttgc ttactaatat acctagaact aagcctgata tgttgcacac actcaaaata 29880
tttgttgcat aaagaaatga gtgaaggaag gaattaattc aattgttatt taaagaccat 29940
agtaaaaacg tggacacata cttttgaagc acaagactaa actaaattaa ttggagacaa 30000
gaggacttta acatttagga ttggaggcac cagcatagta agaattggga aaggcttata 30060

aaattgtaaa ggtgttttga ggtcgtggtt aaacagtaat acgtctatgc taaaacttta 30120
agagtatttg ctaattaact aacctcatat ggaacaaaac ccccacattg ttgatatctc 30180
aaaagaaata ttagtctgac ttaggccact gatccatgct actcagtgtt gtttttctga 30240
caatagcaat aaaggtaatt atatttgaat attaatggtt gttttgcaaa gcattgttct 30300
gatagagtag tcacttatat tgaaattaac atttccagtg tacacacgtt aaccttgtcc 30360
tcagtcaaca gctaacttaa attaagtgcc tattagagct ccatttaact agagatcaga 30420
ccagcctcgg catgggtgtt gtccctgagg tgtttaaaga gagccaagct tctgctcaac 30480
aggtggccct cctctttgaa cttctagtga gagctccagt gaatatgtgg agatacgttc 30540
aatgggcatt gtaatgtggc aggacagagt gtggacgttg gcgactaata aactggattt 30600
aagatggctc ttccacgtcc attagctctg tggactccta gatagttcac aataaacctc 30660
agcatcctta cctataaaat agacaatagg aacatctact acataagatt cttgtgaata 30720
taacatgagc tagtatatac aaaattcctt atcacactat tttgttggta gtattctgtt 30780
ttttgtttgt ttgttttgag atggagtctc cctctgtcgc ccaggctgga gtacagtgcc 30840
atgatcttgg ctcactgcaa cctctgtctc ccaggttcaa gcaattctct gcctcagcct 30900
cccaagtagc tggaattaca ggtgtctgcc accacaccca gctaatattt gtatttttag 30960
tagagacgga gtttcaccat cttggccagg ctggtcttga actcctgacc ttgtgatcca 31020
cctgcctcaa cctctcaaag tgctgggatt acaggcgtga gccaccattc ccggccggta 31080
gtattcttaa gaatgttttg tctaccctct ccattaatag gatatattta tgacttttac 31140
aagtaaacgt cgtatttgtt tggcacttgc aatagtggtg attagttcga caaatagcac 31200
aattattctg cttcagtagg atgtaacctc atatcttaac caaaatataa tgtggctagc 31260
aattttacaa tgttcaaaat acttcagaaa tccacaatgt gcagttcaac ataataattt 31320
ctcattttct ccaatcagca tcccaggaga tctagttatt catgcagatc aacacgtcca 31380
tgatttaagc cgtgagcttt taggaacttc actggcccaa agctaatagg tgaaaagata 31440
tatacctaac attttaaggg attttcaact cttgatgtct tattttgcaa aagagctttg 31500
cagcaataat ttatcaggca gcctctctgt ccacattgga gtattctcta gaatgattct 31560
tatgtttatt ttacatttcc cttccctgca agaagacaac tccaggtaag gagaattgtg 31620
agttaataag atgcatgcca ctgatagcaa cagtctattt gatgtctatt tccaaaagaa 31680
aaatgcaaag tggagtgtgt aatcccttaa cgtaagatac ctccatgaat cccaatgcct 31740
ccacgtgaat cctgatgcca gagctgcaag gagcatacac tgtcagggta cagaaaatga 31800
agacctctct gcttgaaagt ttaaatgccc tgctcagcag gaagtctaga aagatttgtt 31860
caactgaaat gagcttataa tcaggctttc atccaaagat aaggaaagac aacatccagt 31920
agttttatga tgcactctta ccaggaaaac acaaatagtt ttgtccatgt tgtgcagcgt 31980
tgactcctgt atagcatatt tcaaactgca atcatgaaaa tgttccattt ccgactaaga 32040
agaaaaagtc acccatgaaa cttgacaccg taccttcatt ttttttttag caatgaacaa 32100
aaaatacaca ttgttagtgt cacccattct attttttcat tttcttttta tgatttagag 32160

gtgttcctaa gacttgcaat tatttaaata aaattattgt agcattttta ggggtcattg 32220
gtacatttac tcagcaaagt tagctgcctg aaagacagat taactgctaa tcagtgctta 32280
atgagggctt tgccacgcca tgtttgagca cttgtcagta taagaatatc gattagtaaa 32340
gccacctttt ggacttattg ttggtaaact gggttgattt ctgaggtgga taaaatgcat 32400
ggctagatat tctgtccact gtgctgttgg caggctgttg tttatcacaa ggttttagtg 32460
ttcttaatga actgttaata agctgtcaat tatagtgttt atgcaaatta gggaaagaga 32520
cattgaagga ttgatgtgcc tagcattaaa aatgccttct tgatacgtta taaagtaaat 32580
aattcccctt ttaataattg tccttaaaca tttgctcttc tttgccactg aagcattccc 32640
acgttgggca ccctggaaaa caatagtcca ggacttcttt aagcattata ctgagaaaac 32700
aagacacatc tatctctagg atgtactttg ctttatgtta gcaattctgg aatcattttc 32760
attcaccctc ggccgtaagt gaagttcaat atcaagtgtt ctgaaattca tgggtagcta 32820
ttctatgatt ctcatgtggg gcatccattt ttgcctttct tcccactgga gaccatcgac 32880
catggcgaag caacctcctc aaccccttaa atgtcctgcg ctaatggtag ctgtcccaag 32940
tcccatgtgc tttgtagtgc ctcagattat ctctttagta caaatccttt gtgtggttat 33000
aaattgtcaa ctgaaactat ttcttttaaa aatctgcttc tctgcctctg tcccggcaga 33060
aatacctgtc tttggccaga aaatccatta attttctatt ctgggttttt gtttttttat 33120
ttcatatgcc cttgaatgtg ttgacctaac atgccccacc tgacgtcctt caaggaccta 33180
tacaattgtc ttgttctctc agtcattctt ccggccccgt ttacccaggc agaataaatc 33240
agttcctttc tgcagcagcc ccagagtact ttaaatgagt tagatatatt ccagcacccc 33300
ccacccctat tctgcacaca cacacattca gaaattgtga gctctttcag ggcttggcct 33360
gctatttatt catcttcaga aatcatgtca ttcaatgtta gaaaatagct acctcaagac 33420
actctggaag aaattctgct cagatttcca ttttataata aaactaaggt tattaaatgg 33480
ttcccaatat ttaaaaggat aataattttg ccgcaatctc gtcactttta tttttaattg 33540
ttgtgatcag aattctatcc agatagttat tatgggagaa caattcctga agaatgctta 33600
acctctcaat acaattaaag tagacatctt ttaaaggctc ttaatattcc ccccagaaat 33660
gtaaaattac atcttgtttt gtattgtgga agtcatgttt tgattatatg aaattggtat 33720
tttacgtgaa aaaaaaaatc ttctgttcag cttggagcat gaaggtttgt gcagttgtta 33780
gcacattact tattccaaaa tcattgaaaa tattattctt gcaggtttgg ggttttttct 33840
aacctcattc gctcagctct gggagttata atgtttcata aaggaagtat cataatctac 33900
agcaaaaatc atgcatccat atcagttgaa atgatggatt aagctgtttt attataagta 33960
tatcacaaat gcaataaata tttgccaatg aaatgcagtt atcataaggt tttgcacagt 34020
gcaatttcat gttaatgtcc aagtagaata tagatggctt tatatgtttg gttatagtac 34080
ctg 34083

<210> 34

<211>  16484
<212>  DNA
<213>  Homo sapiens

<220>
<221>  misc_feature
<222>  (1)..(16484)
<223>  chromosome 5 nucleotides 170305146 to 170321629

<400>  34
ataagtacta gaggcaggat ttgaacctag gctgtgttct tattagctaa attgtactgc      60

ttcccttttt ttaaaacaaa tatttatctt ccagtagaga aatataaaaa attacatgca     120

cggtgaggtc tttgtctgtt ttcaggtagg catgttttat caagggctaa taggcaggta     180

tttctatgtt tacattattg gtaagcccaa aaaattaagc ttgcttattt taaattcagc     240

tatatcaaca agtattcatt ttatgtgtct tgttttggga atcaaaaaaa gtgtgaaatg     300

tctccttggt aatgatcccc aaagcatatg cagtctcatc tgtgataaag actcaggaga     360

cagctaaaga acagttcagg gttaggactc ataatgtgtt aaacgcttaa gagattgttg     420

gattataaga tactgttgga tcttgaaggt ctgtctagct aattgacaac tgtattgctg     480

gagcattccc ttttaccaat cttgagagga agtataatag tttgagaatg aacttttgga     540

aattcattgc ctagatttca gtcacagctc tgcactttta aatagggact tgtaatatta     600

aatgaatata gatacagtac ttagaacagt gcctagtgca gagtaactac tatataaatg     660

tttgctatta tttttaatag taataaaggc ttgttttaag aaaccagtat ctaaaaatct     720

aaattgataa aacataacag gaaggtaatt atttacattt aagtgttttt cattttgaga     780

aataatttac aataggagat tttaaaaaga atgactccta cagagagtca ttacatatgc     840

attcaccaaa agtcatgctt gcaatgtttt agattcacat ccattacatg aagtgtataa     900

catatcctac acacaattgg agcatagtct gttaatagcg acctctgtaa aattctaaga     960

aatactacta attgtcattg tttggtagtt atgttatata aagtcgctgt gaacaataaa    1020

ttagcaaata ccgaaccatt gctcctgtgg gcctctgacc acaacatttt catcagctaa    1080

ccaacacatg accttttttt aagtatgttt ctgtagaaag acaccttatt cagtatatat    1140

tgtcgattta ttgacactga actcatagcc agcagcacta tttttcatgc tggaacaaag    1200

cttatctaac atgtgttttc tatataagac acataacaag ctttttttct ttaggaacac    1260

tgcatagcat ttcagcatta tgcttagatc atcttaaaca gtgaaatcac taacaaaaaa    1320

cacaaaaatg tggcagtaga tagaccatta aaaacatact tgtttacatt atgaagctga    1380

aacaagaagg tagagtttca ctttgtttgc tctgagcttg aaacatgtgt actggacaac    1440

tacagatttt cactgctttg cacattagca ggtgactgag agtattgatt ttgggattat    1500

aaataaattt tatcaagtag gcaaattcac aaataatgga atctgtgaat aatgaggatc    1560

aactatatca tatcttatat tgagtattat tggagaagtt gctttaattt aagcaataga    1620

aatataatta ctattaccat tcattattaa taatgggtct acattttaat acatttatta    1680

aattggtaat gttaaccttt cctttccttt ttcatgccat gttttctata taatgatttc    1740

ttaattcagt aacccatatc tgttgataat gtataatata cccagcgttg ttttaggctc 1800
tggagataca atagagaaca aaacagacaa aaatacctgt tttcatgcag tttacattct 1860
gttggaatac acaggtaaca taaaagataa gtatgtaaaa tatgttagaa aatgttaagt 1920
gctaagattt aaaataatga agcaaggaaa gaagatagga aatgctgggg gggattgtat 1980
tatctattac cagaacttaa tggcttatca ggtaacaatt gcattattat ctctcagtgt 2040
tctgggggtt gataagtctc agatggttct cttcaggggt ctttcagtca ttgtaatcag 2100
atgacagctg gggctggagt tgcctgaatg cttctttact cacctgtctg gtgtgtgtgc 2160
tggaatgctt agagtgtata ctctatctag ttgggcattt ctctctctgt tcaatctctc 2220
cagatggcta gttcgggcct cctaatagta taatggtctc agggtatatg gtcttcttaa 2280
atggtggcag gcttccccag agtgaacatt ctctgagaga ctggaggaat ttgccagttc 2340
ttgtaaaggt taggcacagt actaggatgg cgtcactttc accaaattct attggtcaaa 2400
gcagttacaa atttccaaag attggaagaa taaactccac ctcttgatgg catatacata 2460
catggaagga attggtgatg agtatttgaa gacagactac catgctaagt attgaaaatt 2520
gataggatat cttggtaagg tctgagaagg tgacatcaag taagactggt cagcatagtt 2580
gtatctttcc ccagttatat taaactgtgt gagtactggc aaagagtagt ggtgtatcgg 2640
acataaacag tcatggtttt gtaagatgag tatatcatag caaaagagga gctaaatgta 2700
agtgcaaggc agagatgata atgattgacc atataattaa agctaagtac ctaggataga 2760
taatatgtga agggtataag agagagtgaa aaaatggcag attcccaaaa actaataaaa 2820
gagatcagca aacttgcagg atacgggatc aataaataaa atcaatggta tgtctatttt 2880
ttaacaatca gcaattgaat atgaaaggaa gaaaggaagt ccatttatag tattatcaaa 2940
aaggataaaa tacttaggca taaattttaa aaagaagtgt aagaattata taccgacact 3000
gtaacagtgt tgaaagaaat taaagatata aataaacaga aagacttctc atcgtcgtgg 3060
atcagaagac ttaatattgt taagatggca gtactcttca gattgatctc caaatccggt 3120
gcaatccact ttaaaatccc atgggttttt tgcagggatt gacaaactgt tcctaaagtt 3180
catatggaaa tccatgggac ccaaaatagc caagataatc ttgtaaaata atgaaattgg 3240
aagactcata ttttttttta tttcagaatt tactagaaaa caacagtaat caagactgtt 3300
tggtaccagc atacaggtag acagacatat aggacctggg tgcggtggct catgcctgta 3360
atcccagcag tttgggaggc caaggtagga ggatcacttg agcccaggat gttcaggaac 3420
agcctggtca acctagtgag acacctcatc tctacaaaat attttaaaat taggtagcta 3480
tgttgatgtg ggcctgtgat cccagctgtt cgggaggatc acttttgcct gggatttaga 3540
ggctgcagtg agctatgatt ttgtcactgc cctccagcct gggtgaccga gtaaatctta 3600
agtgactttg ggttgggcag ctatttctta gatatgacac ctgaagcaga caacagaaaa 3660
caaattagat agaccagagt ttatctaatt aattagacaa attagataaa acagagttgc 3720
aaattttgtg catcaaagca tgccatcaag aaagtacagc ccacagaatg ggagaaaata 3780
tttgcatatc atatgactga taagagatta gcatctagaa tacataaata attattacaa 3840

cccagtaatg aaaaagacaa cccttaaaaa tgggcaatgg atttgaatag acatttctcc 3900
aaagaagaca cacaaatgac cactaaacat atgaaaggat gctcaacatc cttagttatt 3960
aaggaaaggc gtatcaaaat tataatgaaa tacctcctca caccaactag gatggatata 4020
ataaaaaaga aaactacaag tgttggcaaa gatttggata aatttgatct tcatacattg 4080
ctggtgggaa tgtagaatgg tgcagccgct tttgcaaaca ttatggcagt tcctcaaaat 4140
gtgaaaagta gaggtgccat gtaactcagc aattccactt ctaagtatac tcaagagaaa 4200
tgaaaacata catccacata aaatcttgta cacaaatgtt catagcagca ttatttataa 4260
tagtcaaaag atggaaacaa tccaagcttc catcaactga atgggtaaat gaaatgtgat 4320
atatccatag taaccaaaac agcatggtat tggtataaaa acagacacat agaccaatgg 4380
aacagaatag aaaacccata agtcgtatgt ttacagccaa ttggtttttg acaaaggtgt 4440
caagaacata cattggggaa aggataccct ctttaataaa atggtgctgg gaaaattgta 4500
tatccagatg tggaagaata aaactggact tctacctctt accgtataca aaaatgaaat 4560
ctagatggat tagacttaag acctgaaact ataaaactac tagaagaaaa catggggaga 4620
cacttcagga cattaatcta tgcaaatatt ttatggctaa gacctcaaaa gcacaggcaa 4680
caaacataaa aatagacaaa tgggattata ttaagctaaa aagccccttc acagcaaagg 4740
aaacagtcaa cagagtgaag agagcctgtt gaatgggaga aaatatttgc aaactgacca 4800
gggaggaata gaatatgcaa ggatcacaaa taacagtaaa aaaaacaaaa acaaaaaaaa 4860
aataactcaa ataagtggca gtcaaatgac atgaatagac atttctcaaa aggagaacat 4920
acaaatggcc aataggtata tgaagaaata cttaacatca ctaatcatca gggaaatgca 4980
aatcaaaacc gcagtgagat gccatcttac cccagtcaga atgattatta ttaaacagaa 5040
aaaataacag aagctggcaa agatgtggat aaaagggaac tcttacacac tggtggtggg 5100
aatgtaaatt aaaacagcca ttatggaaaa tagggagatt tctcaaaaat gaaaaataga 5160
actaccgtat tgtccagcaa tcccaatact gggcatttat ccaaagggaa agagttcagt 5220
atgttaaaag aatacctgta cttccatgtt tattgcagca ctgttcacaa tagcaaagat 5280
atggaatcaa cctacattcc cgttgatgga tggataaaat aatgtggtat atatacatag 5340
tagaataccg ttaggccata agaaagaatg aaatcgtgtc atttgcagca acataggtgg 5400
aactggaggt taagtgaaat aagcctggca cggaaagaca aataccatgt gttcttatac 5460
atatgtggga cctaaaaaat tcatttcatt gaggtaaaga acagaatgat aggagaagct 5520
gagaatggtg tgtgggtgga ctggggagtg gggcaggatg aagagaggtt ggctaatgag 5580
tacaaacata cagttagata aaaggtataa gttctaatgt ttgatagagt agggtaacta 5640
taactatagt taacagtgta ttccatattt caaaatagaa ggcaggattt gaattattct 5700
caacacagaa atggtgaata ctcgaggcaa tagatatgcc aaataccctg acatgatcat 5760
taaacattct ttgcatgtaa caaagtatca catgtacccc acaagtatga acaaatatgg 5820
tgtatcaata aaaataaata agatctatac aaacaatgga atgttattca gctataaaga 5880

aaaataagta ttgatatgtg ctaaaacatg ggtggtcctt gaaaacatta tgctgacttg 5940
aaaggagata gtcacaacac tcacttattg tatgattcca tttatatgaa ttgcccagaa 6000
tgggaaaatc taatgagaca taaagtagct tagtggttgt ttagaagcta ggggcatggg 6060
ggtaggatgc agaatgacag ctagtatgaa aatgttctaa aattagattg tggtgatgct 6120
tgcataaccc tgttaattta ctaaaaaaca ttgaattata tcctttaagt gggttacttg 6180
tatggtatgt gaattatatt tcagtgaagc tgttcaaaaa gtgataggct catggattga 6240
agattcccag taaaacaaag gattatggag ttgtgagatt catctttgta gatattgaaa 6300
tcactagtga ttatgatata aatattgttg gaaagactga cagccaagaa ccaatacttt 6360
caaaaaacag ggggaaataa tctgcaggag agtagataat tgcaataaga agggatattt 6420
gttagtgttg ttgatgatgt aagatgtaaa ggtgaatgct ttcatgatga agaaagagaa 6480
aatattaagt agaaatgagg aacgtgaaga tcacctactc catattcaag gcccaagata 6540
cacgagaagt ggaagaaaaa gcagctacca cttgatagag cctacagaga aagcagggtc 6600
atcagagtag agcttctatg atagactttt atattgaccc taaaacactg aaattcataa 6660
ggtaactttc tggatacttt cagcttccta catgaagttt ttgatctgtt acgtttttca 6720
tgaattgtat gtcatgaaac ttgagtctgt ttttcagtgg attacttgat caaattagtg 6780
ttcttactct cctacttaat ctatttcaca ttcctaatgt ataaggctgt ataattggtc 6840
agttctggtt tatttttgtt ttgtgagaga ataaaggatg tgaatacttg ccaggttaga 6900
tttctaattt acaggaatct tgtcacccta ataggttgat atgtctcacc aaaaattata 6960
aaagagttgt tgattaattg tagtataata atgactgcta gtatccatgg gagaacagaa 7020
agaatttgtt ctttttctaa cttttatttg tcctttctga gtcttctaac aagcagatag 7080
cctcttttct tcattattca gttccatacc ttttaaaaaa acatttttac ttgaaagcgt 7140
ttcttaatca gaaacaattg atttcagtat tatgaaatag ccctctcatc tttgaaaaat 7200
cagtctccaa gaatagcata aaatttgttt ttagcaagat aattttatgt tgttaattat 7260
ttgtaaaggt ctcaaaagta ctgaaggatc acctagatca atgaatccag agatataaaa 7320
taatatacct taagcaaaaa tcattgaata aatacctcca tttctaaatt agtcaatttc 7380
agctgccccc aaagtaagat ttatgaaaac tgtagatgtt gccatttgtg ccactgcctt 7440
aaatctattt aatcctctac tactatcagc tgtgaagtta actctaccat caaacaatgt 7500
gaaatcttgg attttttaat acatcatgca taaaaataca gaaggaacaa atttattact 7560
atctattatg ttccaagcag gggaaatatt taaaatgatc acactggtac tatcatgaca 7620
ttgaccattc agaaaagatg ctggtccttg gatcatgaga gtacatcctg actgaataac 7680
agatctggtc ctagacagtt tagtgttttg tactcaaatt tattttcagt catgtcttca 7740
tggtagacat tgttttttcc attttacaaa tgaaaaccct aagacttaaa gagataagta 7800
acttatgcaa gatcacatag caaattagta gagctgggat tcaaattagt tttatttggc 7860
tctaaattct atgccatttt cagtgttcta ctgccttgat aaacttaaaa ccgatctgct 7920
ttctatccag aactgagttc acaataattt atcactcatt tggaagaaca gatcattgtg 7980

ctgattttgg aatttccccc aagataagct acgtttctat gaatacttac taaacttttt 8040
tcttccttat tctatattag gacgtcttgc atggctggta tacttagttg ggacagttgt 8100
aggaggaaga ttaacatata ccagtacaga tgagcatgat gctatggatg gagaattatc 8160
ctgtcggtaa gtaagagcta tgtaatttac tgaaattcac taaatagtta aaaaaaatta 8220
gttattgaac tagtattatg agtgaagaca ttttgtatgt ggccttttga ggaaggtatt 8280
atttcccttt ttattggtga gaaaaccaag gctcaaaggt taaatgattt gtagaagtca 8340
aatctctaat aagtgataga ctcaaaatat tttgtacatg tttgtgcaaa taatgtgata 8400
aagatttagg ttgcttttta ttacttagga tgtgagcaag cacatacagt tggtgtgtat 8460
tggaaaggtg gcaaaattgg gtgacataca tccaatcaga aagtattagg tagtcagtta 8520
cccatcgcag gttttgtcac ctattggcca cagtcttagg aaaaagcagt tgaattattc 8580
tcttcaagtt tctattctaa cctgaggaaa atttctatat atgaataata caagtgaatt 8640
gattttccat atattttaat ttatttttaa taacaaatct ccttataaca gcgtcataca 8700
tgtttttatt gaagacttaa acattcaaat gaaaataaaa attatatact aatttggaga 8760
taaccactac tgttattttg taaagtccgt ttgagtcttt ttaattaacg tgtatgggtg 8820
tatgtttact gcatgtgtgt ttttcaactt ttcttaaaca ttacaaaaca atgtaagatg 8880
gagctaataa taatacctaa cttattttgg gctattataa ggaatttatt tttttaaatg 8940
cttagaacag tgcctggcaa ttagtggtat attttacaca cacacacaca cacacacaca 9000
cacacacacc ccaaccccac ccacccacct caacaccact ttttaaaatc aaatgcaata 9060
tcaacatttt acctggacat taaattttct aaatttagaa aattccttgt ttttggaatt 9120
ttaggttgtt cctcaatttt ttttattata aattactttt agatatacta aattgttttt 9180
agttatatct ttatgtagac tgaaataatt tcattagagt tcgttcctaa atgcagaata 9240
gctggtcaaa agttatgtag cattttaaag actttcaata ctcaatacca cacggtgttc 9300
tgaaaaagga ccaatgtatc ctctgaacca agggtcccca acccctgtac tggtccacag 9360
cctgttagga actgggctgc atagcaggag gtgagctgca ggtgagcaag gattactgcc 9420
tgagctccgc ctcctgtcag atcagcaggg acatagattc tcataggagg gtgaacccta 9480
aactacacat gtgagggatc taggttgcat gctccttacg ggaatctaat gcctggtgat 9540
ctgaggtgga acagtttcat cctgagaccg tcaccccaca ccaccccaaa ccccatccat 9600
ggaaacattg tcttccagga aactggtccc tggtgccaaa aaggttgggg accactgtgc 9660
tgaacccaaa ttgtatgata gttaacattt gttagtggtt gaactgccag atagtagggt 9720
tcatattgtt gccagtttat aggtaacaaa tgtattattt caattggtat ttatttggtt 9780
actagtagaa tcaaacattc ttttccactc ctagctttgc cattttttct ttgtgatttt 9840
tatatctata ttgattatat tatttacctg cttttctttt gtgatgatct cttctaactt 9900
atttgaaaga gctcagtata tatatatatc tatatatata tatatatatg ctcagtgtgt 9960
atatatatat atgtatatat atgctcagtc tgtgtatata tatatatgct cagtctgtgt 10020

atatatatat atgctcagtc tgtgtgtata tatatatata tatatatata tatgctcagt 10080

gtatatatat atatatatat atatgctcag tgtatatata tatatatata tatatatata 10140

tatatacact gaatgaactc tgtagaggaa cgaactctaa tgaaattatt tcaatctaca 10200

taaagatata attaaaaata atttagttat atctaaaagt aatttataat aacaaaaatt 10260

gaggaacaac ctaaaattcc tgtatatata tatatatata tctcagagat cttaatattt 10320

tgctcctcat tttgaaaatg ttttctctgt atttttactt cagaaatttc ttctttactg 10380

gctttaaata ttatgtattc agatctatgg actttttcct tcatatttct ctgctgctta 10440

tatttagaaa gatttttttt ccacttctga ttaatgaaaa tatccacctg tattttctcc 10500

cagctgtctt tggtattaat agctttttat atttattttt aatatatctt gatatttggt 10560

ttcatatgtt ttttccaaat tttccaatta atgccaatcc taccctctac tgatttgaag 10620

tggcacttat atcttacaca aaattatttc atcttttttc aggcctttca tacttttcca 10680

ttgatcttag aagtatattg ttatatattt ttaccctatt aaagacctta aaattgttat 10740

acctacttgt gatattctcc ttcattttta ttattaaata tatttctaag tgcttaatga 10800

atttattgta cttttggata aaaaatttca ttttgttcca tttccttttc aacctttaaa 10860

ctatttcttc aataaaatgt gaatcttcat ggaccatgaa tatttttagt ttttctaaga 10920

cttattattt tctatatcaa attgaattac ttgaaaatat gaataacagg tgtttggggc 10980

ctccttaaag tgctttagtt acttttcaac caaacaaaaa atttgctact caatctctcg 11040

agtagctggg attataggca tatgccacca cgcctggcta attttgtatt tttagcagag 11100

atggggtttc tccatgttgg tcaggctggt ctcgaactct tgacctcggg tgatctgcct 11160

gccttggcct cccaaagtgc tgggattaca ggcgtgagca actgtgccca gctgattttt 11220

ttttaagttt aatcatcaag cagggaaata aacgactggg cgcagtggct catgcctgta 11280

atcccagcac tttgggaggc caaggtgggc ggatcacgag gtcaggagat cgagaccatc 11340

ctggctaaca cggtgaaacc ccgtctctac taaaaataca aaaattagcc aggtgtggtg 11400

gcgggcatct gtagtcccag ctactctgga ggctgaggcg ggagaatggc gtgaaccccg 11460

gaggcagagc ttgcagtgag ccgagatcgt gccactgcac cccagcctgg gcaacagagc 11520

gagactctgt ctcaaaacac acacacacac acacacacac acacacacac acgcgcacac 11580

tgccactatc tcgtgagaaa acgtatatta ggaaataaag taggtatata tttgccaatg 11640

agaaacaaat attagttttt tatttaagca gcaaacgtat tgaacatgtt gaactaagat 11700

ttgtgttttg gcctgcattc cctggaaaac agagccttgg gtgaagttca tgggccaata 11760

tttttctgga ggttagggag aagagtacat ttttagagaa gaaagaataa gaggaaaggg 11820

aaagtaagtg agacaataag agaaaacaaa tacagtcaat ctttattatt cctggattcc 11880

attttttgtg aatttgccta cttcataaaa tgtgtttcta atcccaaaat cagtactcat 11940

ggtgcttttg tggtcatttg cagacatgtg cttagggatg aaaaaatttg agttttgtgt 12000

tgcactttcc caactgaggt tgaacaaggt gatactctgc cttgtttcag ctcttgtact 12060

gcaaataatt gcccttttta tattctgttt agagccacat ttttcatact gttgtgcttt 12120

ttgttggtgc tttcagtgtt taaaatcgcc cagctgtatt gttaaagtgc tatttagtgt 12180
tcttaaatac aagaaggctg tcatgtgcca tacatgggtt agattagctt tgtttgggta 12240
ccagtgggag tactgtgaat actgcagtag tctgtgagtt cagtgttaac aagtcaacaa 12300
catatttgaa aaagcctctc taaacagaaa tacacatgaa acaagattgc aatccattga 12360
ccaaaatgtt gtcaccagag gcttgcagga acctaatcct gtattttacc taggagcaat 12420
ggttcattat tcactaattc agtgttcaca gtgactacag aatataacta ccatgaataa 12480
tgagaaccaa ctatgtaagg tggtgtatta ctgagaaaat acaactggtt gcttgctttt 12540
ataggaattt tccctggagg ccacacgaaa ccacttgtgc ctcagaacaa ctcatgactg 12600
ggggagtaag gacaagcagt atatccattg gcttctttct gtgtctcgtt tcttactggt 12660
taatgtctac tcctggggta tttcactttt ccacacttct tagttatgtt tcctggcctc 12720
tttgaggagc tgctgggaag acagagcttt aattggtcct gttagagtta tgaacaggaa 12780
tggtggtttg tctctctttt ctgggaagat acataactcc agaaactgtg agagtctttt 12840
ctggggctac aagacaagtg gctgaagcca ggggtgaggt gagaggtgat agtgtggttg 12900
gtgggtgtgc aagcagggcc aagcaaattc aaggtggggc atattctagg acttacacaa 12960
cagtctggaa ataaatatgt attttagaat attgagattc taaactaaga atgatgtctt 13020
acgtaaaatt acaaaaaaaa tcactttcat atgaagatgt tccaaataat tggaactaca 13080
gtactgtgac tttctataag ttatatataa tagcattcca actcctcaga gaacactacc 13140
aaattagaag atcatgaagc tcctgccaaa acttcatgtg ctattttggc atgaaaagtt 13200
gaagagtcat aaaagctcct aacattgtga tgatttataa ttatgactga aacatgctac 13260
tttaaacata gcaaataaat atatttacac tcaggccaca taattctaat taaataaaga 13320
catttatagt cattttagtt tcattcatca tgactaaaat gaaaacctct tgagagattc 13380
tccagggatt ttgtcttatt catcatgcat cctgttaact catttatgta tgcagttgca 13440
atttttaaaa tttttgctat cagaccttgg cgatgacctt gagcagtagg atataaataa 13500
ctcccacatg cttagcgttc caataatgga acactaggca taaatgtaat ccactagtgc 13560
tagtagattg agtgctagct tctttcagac ctattctact tagctgtgcc accctgtgtc 13620
tctcattgct ctcacctacc catctcccag tcatccctca ttatcaagac tttaataact 13680
agctcagagt tttcctctct ctcccaaacc ctgctattat ataaataact tcagctgctt 13740
tctgatcaga taatatttct cccattttct tacccataag cctccagtgg cttcctatca 13800
cacttgagac aaatttcaag gcccttgtga tggcccagag tatcctgtgc tacttctgac 13860
ctcatttcct accctttccc atttaacact ggcctccttg ctgtcctttg agtattacca 13920
gtgtgttttc actttagggc ctttgcactt ctctttcttc tgctaagaat actttcttcc 13980
agatgtttgt atagtacatt tggcccttga acaatgtggg agttaggggc accaaccttc 14040
cacacagttg aaaatctgcc tgtaacttta gactcccccc aaatttaact actaatagcc 14100
cactgctgac tgtaaacctt attgataaca taaacagtca attaaaacat attttgtatg 14160

ttatatgtat tctatgctgt attctaacaa aggaagctag agaaaagaaa atgttactaa 14220
gaaaaccata aggaagaaaa agtaaatttt ctgttcatta agtggatctt cataaaggtc 14280
tttatcctct tcatcttaaa cattgggtag gcttaaaagc tgagaaggcg aagttggtct 14340
tactctctca gggatggcag aggcgcaaga aaatttgcat ataagtggac ctgcacagtt 14400
caaacctgtc ttgttcaagg gcaaactatt cttcctcact tcagtcaggt ctgtactcag 14460
atgtcttttt caaagagggc tcctctgacc tccctatcca aaatagcatc cttaccccac 14520
tttatttttc ttgatagtac ttattacctc ttgatgttgt ttgttctact agagtaaggg 14580
aaggagtaag ctttgttttg cttactgctg tattttatct cctctgtgta gcaaaacctt 14640
aaccctgagg gaatttaggg ttttctctaa tttgttgagt gatagtaaca gaaattgcac 14700
acttctgtgg ataggaccca ttgtcacttg ttgtttttaa cctcaactag gccctcagca 14760
ttgctcagtg attctatttt gtttcagtaa ttcttgtctt ctgacttcta aacacctatt 14820
gggctgtttc actgggatgt tgtatcccac atgcacctct aattcagtga ctaaaattaa 14880
actcatcttt tccatcacct aaccctggct ctcttcacat gtctcttcat catacactca 14940
gtttcccaag cctgaatcgt gagagttatc cttaactttt tccctttttc tctctagcat 15000
tcatatagtc atcaagtcct gtcagttcta tcttttagtt acctcagtta gtccacctat 15060
cctatctcct ctgccagttt agctactatc atctttcacc tggattagta gtaagttttt 15120
aaacggactt cttgcttcct tttgcagcca gagtaatctt tctaaagtac agatctgatt 15180
atgtcaatgc ttggcttgaa ctatgcaatg acgtcattgt ttttaagatg aacaacatgg 15240
cttatcaggc tgttcattat ctggctcctg cctgtcccat tagcctcatt ttctgccatt 15300
cactacatta tagccatgtt gaacattata cagctgagta gctgggatac aggctcctgc 15360
caccacacct ggctaatttt tttattttta gtagagacag ggtttcaccg tgttggccag 15420
gatgatctca aactcctgac ctcaagggat ctgcctgcct tgggctccca aagtgctggg 15480
attacaggcg tgagccactg cacccggccc cctgcccctt tagatggcta cacatgcttt 15540
cctttttgtc gcaacactat ggctttgcct tgttcgtcct agaatattag gaaatagcat 15600
ttgttatagg acttgtcatt ctttattcca gctgcttctt ttatcttcct cttgatagat 15660
tatagatttc tagaggactg gggatatata ggtcttgtac tttgttgtat tttgaatgcc 15720
tgacacatat ctgacgtata gtgggccctc aaatttgttg actgaataaa tgcttattta 15780
gttggatacc taggaaaatt cagcacagtt atccaaaatt catttcattg tgtttgaaat 15840
ggcatgctta tacttggatc aaaaattatt tatcttttta ttctgtgctt tttaccagaa 15900
ataatttaag ataaacgaat aatacatgta ggacaataaa ttgaaactgg gaatggatat 15960
tatatacaca ttgaacatcc ctaatccaaa aatctgaaat ttgaaatgct tcaaaatctg 16020
aaacagtttg agcatcaaca tgaatctcaa aggtcatgct taaaggaaat gctcattgaa 16080
gcatttcaga ttatggattt ttggattcga gatgctcaga tggtaagtat aatgcagata 16140
ttccaaattt caaaaaaatt aaaaatctga aacacttctg atctgaagca tttcacaaaa 16200
ggaatactta acctgtatat attggtatgt ataggcatta gtcttataca cttgttagag 16260

gtggcagcac atcttcagag ggcactgtgg tagagactgt caattgtctt tcatctcttc 16320

ttcctttccc ttcccataat aaagtgcttg attgttttct tgagcaaatg gccactctga 16380

ataaagacca gattttctag gttgttttgc agctaggtat ggcaaagtga ttatgttctt 16440

agtgtaaatg aacgtgttat gtgtaatata tgtagatgta atgg 16484

<210> 35
<211> 18401
<212> DNA
<213> Homo sapiens

<220>
<221> misc_feature
<222> (1)..(18401)
<223> chromosome 7 nucleotides 37873471 to 37891871

<400> 35
gcacgtttgc ctgatggagt tcccagcttg acttttccct ttggcttagt gattttgggg 60

tccaagagat ttatttttct aaggatacaa atttaccctg ctctggttat tatacatttt 120

ctttattgaa acatcactat gtcccccatg aatatatgca attatttttc aataaaaagc 180

aaaaataaaa acccccaaac aataaaaatg tattgttaac aggagattta taagagtaac 240

attcctttgc ccagggacca tggagaggga agatttcatt tgacttgggt agtgaccacc 300

attttagtcc atccatttca ggagcctgaa ggaaacagtt tatcatttta ttttgtagtg 360

tgacttcgaa gagtttgtct cttttatgac aagtggctta agctatattc tagttgtatc 420

tcaaggaagt aaacacaatc ctccctctga agaaaccgaa ccacagactg acaccgaacc 480

taacgaacga tctgaggatc aacctgaggt cgaagcccag gttacacctg gaatgatgaa 540

gaacaaacaa gacaggtata gctcaagacc aggaattgtg ttacttgcaa tgtgttattg 600

ggtaatgaag cgtagtgtag gcacctcagt acctaagggc aaatgttttt atgttaaagg 660

cataagtatt tcttccagtc aacctaacat actgtatatt cagaagagct gtgtgccagg 720

tgaagtgtca gatacctggc attggaaaaa gtagttggct tgtgggttgt agggtagtaa 780

gaatttacag atgacagtgt aggtttgaaa gaaaagtttt gttagataga aagagcactg 840

cagaagagtg cagcggggct cctcagtgag agaggactga gctcgccatg gtggattttt 900

gtttagggat atttatggac cttaaagctg gagcttaagg gtaatttgga ccttattagc 960

cacacaggtc atgataaata attgcacttg tagacatgtt ggtgccttga tgtcagcaag 1020

ggttgcacaa tgagtttgaa catgcatgca ttccggagat gtatagaaat tctagttact 1080

tataactttt tttttaagcc tggtaccaga tgccagcttt aaataatagg gaagtctagt 1140

tacttctaaa ttcctcagat aaggagtttt gcctctggat agtcagcttg gtggccctca 1200

ggtgatcttt gctcccctca ctgtgactgc cctgaaagtt tatgtcaaga acactagaaa 1260

gggaggcctt ttccctgttt gtcttcctag agctgggcat accagtctgg ccacaagggc 1320

tggggcttga agggtgtatt ggaaccatgt ataatccctg ccaactcagt ctcctctccc 1380

atctccccac cttccccttc caccttcccg gtgccctaaa aacatgaaac atgcactttt 1440

actttagatg gtttgtggtt acaaattact gagaacaaaa cggttgtata ttttttgttg 1500

atggatttta aaatattaga aaatggatat aaataatttg tagaatgttg gttttatgcg 1560

ctgctgtttg cgcctgttat tcctctgaag actcagagaa tggagagtgt aacaaagcag 1620

ttgtcaggct gcaccggaaa gaaagtcagg tgagcaagaa catacttaga gtatctgagc 1680

cttgaggctt cctgggatgt gtctccccgt gcttggcagc ccatctccat tgcattttct 1740

ggctgagact atccagaatg tcccttgttg gtccttcttg acttattaat gaatcatagt 1800

ggcttttctt gggtgcctac ctctgttggc tcctagtgat gccatatttt taggaagtgg 1860

gagatcctaa tatcattaat tccaagttgt acgcaggata aaggaactca taaggtaaat 1920

cctgctggca aggagctttt taaacagtag cagttacttt cagggtttta agggaaaata 1980

ataatgagtg ggctgaggga aggtttgttg ggtagggtgt gttaagttta aaaagatacc 2040

agattttatc ctagaaattt tgagccattc aaaaaacaaa aagcgttcta tgaagcactc 2100

attaatgtga cgagtgaagt catcattcca tggggctatt gtttaggtag taaacctcca 2160

ttacaaccaa ctttgagaag ctccaattgt ccaatactcg catgacacca tggacaattt 2220

aggcatgtta attggcatgt cagaaggaaa tgccagcagc tctccctcca gaacaagggc 2280

aagcactata ggggccagat cagagtgaaa tgaaatgtgt acaccttgct ctggcacagt 2340

ctacagtatt tcctttctaa acttagagtc cccatgtgcc tttggcccat gtttatccca 2400

agctggccac agcagacagc atgggacagt gtggagcatg cttgtttggt gtgctcgggc 2460

tcagaaactg atactcaaaa tatggcgctt tgacatgctg aactgaaaaa gcctcacaat 2520

ctctctgact tgccccagca ctcccggcca gcagtctctc tcaaagcaca ggatgaagct 2580

gaatttcttt tatctgccta agatcctcag atccgacagg aacaattatt tttcttcctc 2640

tccctataag accaagaatg taaccacatc tgaacggaga tagtgtacaa gttaatttct 2700

gtgcgttggt ccattcattc tccctcgtca tcctttactg tccttcaaca gaattcttct 2760

ccctgctccc attacctgtt ttgccagatg gtctataaca ggggtgtcca atcttttggc 2820

ttccctgggg cacactggaa gaagaagaat tgtcttgggc cacacataaa atacactaac 2880

actaatgata gctgatgaac ttaaaaaaaa aaaaagaact cgcaaaaaat atatatcata 2940

atgtttttaa aagtttacca atttgagccg ggtgcggtgg ctcatgcttg taatcccagc 3000

actttggagg gccgagacag gcagatcatg aggtcaggag atcgagacca gcctgatcaa 3060

catggtgaaa cccagtctcg actaaaaaca caaaaattag ctgggtgtgg tggtgcatgc 3120

ctgtagttcc agctacttgg gagactgaga caggagaatc gcttgaacct gggaggtaga 3180

gattgcagtg agtcaagatc gagccactgc actccagcct gggtgacaga gtgagactcc 3240

atctcaaaaa aaaaaaaaaa aaaaagttta ccaatttgtg ttgggccaca ttcaaagcct 3300

tcctgggcct catgcagcca gtgggctgca ggttggacaa gcttggtcta taggcttctg 3360

aacctcattg cagggtgggt actcactctg tggttctctc tgtgtacatg ttaatcaaat 3420

ttgtctgctt tttctccaac taatatactt tttgtctgtt gatgcccgtt ggtttttcag 3480

taaaacttga gaggacagag gggaagcttt cccctgactt ctataatgct tgttcttatt 3540
ttcacagctc ttgctctgga gggagggcct gataggcact gtcacttcca tgtgacatct 3600
ttcaagtagg tccagaatct aaaggaataa ggacaaaaaa ctctcatgcg taatttattc 3660
agttgttcac tttcactctt tattgccccc tctcccctat ctcccagcct gctcactctc 3720
ctctctgggt ggtggggatg gagagggtga gacaggagag aagttaaaag cagagcaatt 3780
gaactgggag tgattggtga cccctctgcg tacagagcag cggagtccca gagctttatt 3840
ggctggttat tcctgtggag acaaacactc acagcttatg aattcacatg ctgtggggaa 3900
actctctcca tgccatcgat ggtctgcctg ctccagtggc tgcagaaggg tgggggaaga 3960
gccagggaaa cagggcagag gtacccatgt ttgtcattca cattccagct tctttcatga 4020
tggcttttta ttacattatt tacatgtctg tctctttaat cagcctgttt taagaaatca 4080
aaggatagtg taatttatct ttgctttcca gtctttctac agatcaggaa ggtgtggttc 4140
cattagctac attgctcaga gtcacacagg tagtaactgg cagagtcttc tcattcattc 4200
acttattctg ttatgtgttg attcattcat ctagtattta ttaaagacct actatatgcc 4260
aaatcctaga gatacaaaga tggggaagaa ttagatgatg acctctgagc tcactttcag 4320
tttaagtctt tatgacagag tgaaggtgac atttccaacc acctctacta tattgtattc 4380
ctctgtccat agaaaatatc tattctcttc tcagtagcct ctctatttgt ccctatacat 4440
gtcatagcta tagggagctt aattttttcc tttatgatat tctcatttaa attaaacata 4500
atagatacaa ttcctaaaat aatccttaaa agtatttccg ggatcaggga agtctgaatg 4560
ctataccctg actctactcc catctgcaaa gctttctttc ctctccatag tcaaagggaa 4620
gcttaactga taagcagctg gctgtcagtg aggtctggag ctctgttcag gtccctaggg 4680
caggagaaga tgagggtggt gtggtgtttt ctccaccgag agtttgggga gctcctgaac 4740
tccccagaaa acaaggacct gcagacaacc acagaccctg agaataacag acttgtggac 4800
ttcactgaga cttcctgact cctaaagggc caggatagaa ctgagtagaa ttattgaatt 4860
ccttgcccta gctaggtctc tgggggcttt tcctgattgc ccctgcagaa gaataatgac 4920
ccactgtctt ctaaagcaca agcttgtaga ggtcatcttg ccttccaact acagggtttc 4980
caacacatat caaaaatcag acctaagccc cagtgagcca tcaaggaaaa atgagtacct 5040
gcctaaggga gactggcagc cagagaaagg aaggcctgta caaaaatcac aacacaagtc 5100
tagtgaaaaa gaactgctta gagacagaaa aacccttaaa taaaataaca ataaaaaaag 5160
gacttgagat tcaattgcta ggtatacatt taaaaagcct ttcaaacttg aattatacaa 5220
attgtgattt gaattataag ttcagtggag aattggtatg atcatcattt gagactcaaa 5280
ttaatgacat ggaaagtcag atgcaagaaa tatctgaaag tacacacaaa aaattagaca 5340
ggatgagagc agaagtctgc aatctggata atatgctgag gggatcaatt gctaatgaga 5400
aactctgcac agagaaagaa cagatggaca aactaactca acaaataatg ggggaatttt 5460
tttcccagaa taaatgcatt tgtctgccaa ttgcaagggc ttacaatagt ccaccctcaa 5520
ctgatgccac cacaccccca ccccacacac aaatacagga acatcctggg aagaaacact 5580

gcacactgtg tccagacaga cagagtaagc acttgtatac ttcatatgtt tgaaacccag 5640
agcagatcat cttaacagcc tcttcctctg tatgatgaaa ttatttttaa gtactagtta 5700
ctgaatgagg caaacaatta tgctagaata aaaatacaga caatgaaaat agtcttttat 5760
gggccagatg ccgtggcttg tgcctgtaat cccagcattt gaagtcagga gctcaagacc 5820
agcctggcca acatggtgaa accccgtctc tactaaacat gcaaaaattg gccaggcgtg 5880
gtggcccatg cctatagtcc cagctacttg ggaggctgag gcaggagaat tgcttgaacc 5940
caggaggcgg agattgcagt gagccaaaat tgcgccactg cactccagcc tgggcgactg 6000
agtgagactc tgtctcaaaa aaaaaaaaaa agaaaagaaa atagtcttta tgtacctttg 6060
aatatataaa catatgagta taaaataaaa ataaatgtta cagtgtccca aaggaaacaa 6120
gtgatagatg aattgtttaa ttaatatttt taacaataaa acacatgtat gtctgtatat 6180
attaatctgt cacagaaaat gaagtagttt ttgctttttg aaagctttaa tttttgaaga 6240
tttgtccatg atgttctcaa aattgatctc cactctatgt ttaatagaaa aagataggtg 6300
aaattcccat tttacagtac attgcagaaa ttgcaataat gccaatagct ttgttgtttt 6360
tggattgatt ctatgtagag caccatgtac agcacactac ttatgaaagg ataagtaatg 6420
aatatgtgtg aactccaaga gtacataggc gcattatcac taatgttgat taggagtgct 6480
ggtacatggc gctgataaaa agcaaccaga cttttaattg gttttattac tgatttaaaa 6540
ttgcacttgc actttaggtg gatcccttcc accaactgca tacttggcag caattgagaa 6600
taagttactt acaagggaaa aatacttgac tgccataggg ctcattttag gactactgga 6660
agaaaaaggc tgcaacccct gcattcttac aaagctaaga tatttacctg gtagagcaaa 6720
tgaagatctt caaggacata cttagcaata tatcacctat atactccttc tgtggaaaat 6780
aagtgaggat atactcaacc aaatcaaaaa tatgggagga aaaaaaagaa aaacagttct 6840
taacagtgaa ccttggaatc tctgaacaca cacacagaca catacagtcc ataattctgg 6900
gttttataat ataacagctg atgaaggact ctagaaatag aagaaaattc ttctagattc 6960
taaattatct caacaaaacc tagaggtttg tgggtgtatg tggcaaaatg gttcaaagat 7020
caagaaggca gtattttgac agttgtattt tgaggttctt atttgcagaa agggcataaa 7080
tggagtatta aagttgggag atagtgtgtc agaatgcaag aaatatttgg gacatacgca 7140
tctgacaaca ggggcaggaa aaagacagta aacattaatg cagtggataa gtatagtaaa 7200
acttccacat taacaaggga aaaacatgga atgaagagca gtttgctcag accagcaaaa 7260
taaggggaaa aaagaagaaa cctcaattta aaataaatag tctagataaa gtaagatgaa 7320
aaaaataaag tcaagaatat ggctattagg ctaactatga aaaggttaat tttcccgtca 7380
catatagaca ttactaggta aaggttaaaa aaacctaatt taaaaaccat catattaatg 7440
atgtgcacaa gaatcatcat tgggtttaaa aatattgtgt gaaaaatata tacacagttt 7500
catataatgt ccttagagat tacttattaa tttataaggg aaaattgtag cttttcagta 7560
gataaaatgg ggggatatca actacattaa tatcaccaat aatgggaaaa actgacatca 7620

tacacctcct atgtgatgga gcaagtaacg gaccaagaag gacgcaggct catgaagata 7680
gttttcctgc caaaggttca taaccagaat gtaataggaa agtactgggc catccaatag 7740
tttgaaacca tccaaaatga agggaagtct acaaacaact gtcctggact attcaacata 7800
tcaatatcat gaaagacaaa agaggttgag tcgctgttgg agataaaagc aaattaagga 7860
gatgggacaa ctacatgcaa tatataatcc tggattaata taaaaattac tacagataag 7920
atggtattag aaaattggtg aaaattgaat acggactgtt tatgaggtaa taattttgta 7980
ttaatgtaat atttcctgaa gtatttagag gtgattggtt aaaatatctg cagcttactc 8040
tcaaatggat ctgccaagac acacacacac acacacccac acagcccccc atagccccca 8100
cacacattgg gggatgaaag caggagacag agagaacaaa tatgggacag tgttaacagt 8160
tggtgaacct agatgaaggg tgtaaatggg agttctttgt cctagtcttg caacttttct 8220
attaatttaa tttttttcaa aataaaaaat ttaaacaact tttctcatct atattctgtt 8280
tagaaaaaaa aaagatgaaa ataaagggat ggccaaaaaa tagccaggaa aaataaagta 8340
ttaacactga tcattaaaat tgctaaattt gataaagaca cgatgtagaa ggcaacattt 8400
atgacaatac ggcacccaaa aaaagaaaaa attgtggccc caccaacata acggctaagt 8460
acctaaggag ggaattactg gaaaagtaag gagaactgga aaaaatacct gctagcagac 8520
aagaataagt aaggatgaga ggaattaaaa atacatattc cataaataat atataataaa 8580
ttgactgggc gcggtggctc acgcctataa tcccagcact ttgggaggcc gagacgggct 8640
gatcacaagg tcaggagatc gagaccatcc tgggcaacat ggtgaaactc catctctacg 8700
aaaatacaaa aaaaatatta gctgggctgg gggtgtgcgc ctgtagtccc agctactcgg 8760
gaggcgaggc aggggaatag cttgaacttg ggaggcagag gtagcagtga gccaagattg 8820
caccactgca ctccagtctg gtgacagagc gagactccat ctcaaaaaaa cactatatat 8880
atatatatag atagatagat agatagatag atagataata aattatctat aatatatgaa 8940
acataaacat atatacaacc ttacacatta caaataattt tttcttttac acaaagaata 9000
cctcaacaaa tttttaatca aaattttacc acatatattt ttctgattat aatccaataa 9060
aattggaaat aaacaataaa aaggtaattg gaaatgttca tttgaaaata tgaaatgcac 9120
ttttagataa cccttagttc aaagacaaga ttaaaaagaa aattaaaaac tatctggaaa 9180
gttctacata gggaaaagaa aaatttttcc ttaaaaacac aaaattttat tttgatcttt 9240
gtttattctc tcataaagtc aaacatagat aatatcatgt aagtaatttt tgtagtgggc 9300
tcaataaaaa gttgaattaa gtatcatatt cttggatata atagggagat gcaatttatc 9360
tttacatgac atttaatttt tgttttatta aacttctagt aatttctgaa catatcagat 9420
ttaatttgtt ggcatggtta taaacctcag tttataataa tcttaaatta tattttggat 9480
tttgacagtt tacaagaata tctggaaaga caacatttag ctcagctctg tgacattgaa 9540
gaggatgcag ctaatgttgc taagttcatg gatgctttct tccccgattt taaaaaaatg 9600
aaaagcatga aattagaaaa gacattggca ttacttcgac caaatctctt tcatgaaagg 9660
aaaggtaggg aatcaagcat aaattgttta agtattttat atagatgaga tttgaataaa 9720

ccctagtata attgcattta aagacattgt tttaaaattt aacattgaag aaaacgcacc 9780
ttaagaaatt aatgtattaa cttactaata tcttaatagt gaataaaaca tatttgtagc 9840
tgaagagttt acaaggtatt ttctaatctg tgaatttgtt agagtagatt ttaacattgc 9900
aggaattatt acataataaa gatttaaagc ttctatgtct gaaatgcccc aattcaaagc 9960
ttggatctac cataactgat agtgtgactt gaacaagata ttttaccaag tttatttctt 10020
cacgtgtaaa attcagaaca ttaaaatgag aaaacacaaa taaagtacct ggcacataaa 10080
gagttagttc tttttaatct catcccatat tcttatagca gagtgaatat ggcagaacaa 10140
gaacacaagt cataaattaa cactattcat tcttttttta tatctaatat atggaaacat 10200
aattaccatt aatatttata tattggcctt ttattctctg acttgtccaa ctacctagga 10260
gctttctagg agctttgacg tagtttcctt ggcatttcta tgtagaccag gatttctcaa 10320
cctcagaccg gcttacattt tgggccagat cattctttgt ccagggtgga ggattgactg 10380
tgttatgtgc attgtaaaat gtgtagcagc atccttggcc tctactcact aaaagtcagt 10440
atcatacccc ccttcccctc tgccccacct ctcaaattgt gacaacgtca aaaatgtctc 10500
tggaggttgc caaatgccct ttgtagggca aaatctctcc ggctgagaat cattgatata 10560
cacagtaata gggtttgcaa agtgagatac ttgttcctct tcatttccat ctgtatgcct 10620
tttatttctt tttctttcct cattgagcta gctagcacat cctgtatgac gctgaattgg 10680
agtgatgaga ggagacatct ttgccttgta cctgatctta tagcaaaagc atttggtctt 10740
ttaccactaa gtatgttagc tgtgtatgtc ctttatcaga ttaagaaaat taccacctct 10800
tcctagtttg gagagaattt ttatcaggaa aggatgttgt gtttttctca atttttttgt 10860
atctattgat gtgattatgt tttttccttc attttgataa catggtggat tattttgatt 10920
attgatgttg aaacagcctt gcattctcca gaaaaatccc acttggctgt catgtattat 10980
cctttttaca cactgcaggt tttgatttgc taatattttg ttgagaattt ttgtgtctat 11040
attcaggagg gatatgtgtt gatttctttt cttgtaatgt ttttatctgg ttttggtgtc 11100
agagtaatgc aggttcataa agtgagatga gaagcagtca ctcctatttt atggacgaga 11160
tagtgtagaa tttgagttat atcttaaatg tttactggaa tttgtgagta aaatgattta 11220
gacatggggt tctctttttt ggaagatttt gttttatttt tgtctttttg ccattttcaa 11280
tgtcttttaa tatttaaaaa agacaatatt ttcaaataca tgttattttt aaataaatag 11340
actttacaga acagttttag atttacagaa aaatcaagcc aaaatattaa accattgtag 11400
acttcgagaa aaattaagaa aaatactaca gagttcccat atacctgctc cctcccacct 11460
tctcctatca acatcatgta ttactgtgat acacttttac agttaattaa ccaatattgt 11520
taaaatatca ttaactaaag tccatagttt acattaagtt tcactcttta ttttgttcat 11580
cctatggatt gtgacaaatg cataatgtca ggtattccca ttacagtatc atacagaata 11640
gaattgccac cctaacaatc ccctgtgttt tacctactaa tctctttctc tctcctaacc 11700
cctggcaacc actgatcctt ttgttatttg atatgttatg ggatgtctta gagttgatat 11760

catacagtat atagcctttt cattatatat atatatattt gtttttattt atttttattt 11820
tttgagatgg agtcttgctc tgtcacccag gctggagtgc aatggggtga cctcagatcc 11880
ctgcaacctc cgcctcccag gttcaagaga ttctcctgcc tcagtctccc aagtagctgt 11940
gattacaggt gtgccaccat gcccggctaa tttttgtatt tttagtagag agggggtttc 12000
cccatgttgg ccagggtagt ctaaaactcc tgacctcaag tgatccacct gcctcgcctc 12060
ccaaagtgct ggatttacag gtgtgagcca ccacacccgg ccagtagata gccttttcag 12120
attgacttct ttcatttagc aatgtgcatt ttgtgttcct ccatgtcttc tggtgacttg 12180
attgttcatt tctttttatc actgaataat attccattgt atgtatgcaa ctaagtttat 12240
ttgtgtagtt acctatagaa gggcatcttg gttgcttcca gttttcagcc attatgaaga 12300
aaattgctgt aggtgttcat atgcaggttt ttgtgtggat gtcagttttc attttaattg 12360
ggtaaatatc taggagcatg agtacaggat catgctgtaa aagtatgtat agtttgtaag 12420
aaagtgccaa aatgtcttcc aaagcacctg taccactttc catttttgtt agaaatgaat 12480
cagagttcct gttgctctac aaccttgcca ggatttggta ttgtcagttt ttgggagttt 12540
agttacatta ataggtaaat aatattacct tagtgttgtc cttgtttaca attccctaat 12600
gacatattat gcagagcttc ttttcatatg cttactaatc atctctgtat cttctttgat 12660
gatgtgttgt ctctttatac tttttgccca tttcttaatt gagttgttca ttttcctcat 12720
tgttgagtag aaaggttttt tttgtttgtt tattttgaat acaagtcttt tatcatgtat 12780
gtggtttgca aatattttct ccaagttttt ggttcttggt ttcttaaaag tggctttcac 12840
agaaaagtct ttatttttaa tttagtccag cttattagtt atttcttttt ggattgcggt 12900
tttagtgttg tatctaaaaa ctcattgcta accacaaggt cacctagatt ttcttctgtg 12960
ttattctcta gaaagtttat agttttgtgt tttacatata ggtctatcat ccattttgag 13020
ttatttttat gaatagtata aggtctgtgt ctagacttac tgttttgaga gggtatttct 13080
aattatttca gcaccatttg ttgacaagac tgtccttcct ccattgtatt gcctttgcaa 13140
ctttatcaca aaccaattga ctatgtttgt gtgggtcttt ttctgggctc tctgctctgt 13200
tccattaatc aatgtggcta ggtctggtag tatcacttct ctaactttgt tcttctcctt 13260
caatactgtg ttacatcttt ggccttttca tataaacttg agatcagttt gtcaatttcc 13320
atatccacaa aataacttgc tgggcttttg gttgggattg tgtttaatct acagaccaag 13380
ttgggaaaaa ctgacatctt catattaagt cttgttatct atgaatatgg aatatctctc 13440
catttattta gatctttgat tttttttcgc cagagttttg tagttttctt catttagatt 13500
ctttacatat tttgatagat ttttgcccat gtattttctt ttttagtgct agtataaatt 13560
gttttgcttt aaatttcaaa ttccaattgc tcatttctgg tatatagaaa aaaattgact 13620
tttgtatatt aattataacc tgaacctttg ctataatagt ctcttatcag tgcccgaagt 13680
tttgtgttgt tgttattgac aatgatgatg atgattcttt gggattttct acatagacag 13740
tcatgccctc tgtgaagaac aacaatagtt tcatttcctc attctgaatc agtatacctt 13800
ttatttcctt ttcttgtgtt atttctttag ctaagatttc cagtatgaat ttgaagagga 13860

gtagtgagag ggagtagctt tgcttttttt tccgatctta gaggcaaagc atctagtttc 13920
tttccattaa ggacagtgtt aactgtagga tttttgaaga tgttctctag taagttgaga 13980
aagtttccct ttattcctcc tctggtaata atttttatca tgaattggta ttaaatttgt 14040
catatgattt ttctgcatct actaacataa tcatatgact tttcttcttt atcctattag 14100
tggcgattat gttaattgac tttcaaatgt tgatccaccc tggcatactt ggaataaatc 14160
cagcttggtc atggtttgta atttttttat acattgttaa atttgaatgg ttaatatttt 14220
gttaagaatt tctgctccta tgttcatgag agctattggt ctttagtttt ctttccttgt 14280
aatatctttt tgaatattat ggtaagtctg gcctcatgga atgagttaga aagtgttccc 14340
tctgcttcta tttgctgaaa aaaataaatt gtttccagag aattggtgtc atttattcct 14400
taaacgtttt gtagaattca ctagcaaaat aatctagtcc tggtgctgta tttttcagaa 14460
tgttattaag aattaattca atttatctaa tagatatagg gctaatcata ttacctagct 14520
ctcattgtgt gagttttggt atttaatatt cagtgtattt caaggaatgg gtcaatttat 14580
ctgaattatc aaatttgtga gcacaaagtt gtattgcaga gaaaaagtct gctgtcagct 14640
tggtttttgt tcctttgtag ataattcagt tttgttgttt ggtggttgtg gcttgattta 14700
tttagatact tgcaaaatat tttcccttta tttttgctca attgtttttc aagattgtat 14760
agatatctaa gaatgaactg atctttacac atttatttaa ctattaagca ccttacttag 14820
aaatttgcct gtgattgatg agaagtaagg tatcaaggtg tgggccaggt gctgtggttc 14880
atgcctgtag taccagcacc ttgggaggcc aggcagccgc attgcttgag cccaggagat 14940
caagaccagc ttgcacaacg tggtgaaacc tcatctctac taaaaataga aaacgtagcc 15000
gggcgtggtg gcccatgcct gtggtcccag ctgctgggga ggctgaggca ggagaatcac 15060
ttgaacctgg aaggtggagg ttgcagtgag ctgagatggt gctactgcac cccagcctgg 15120
gcgacagagt gagaccctgt caaaaaaaag aaaaaaagaa aagaaataaa ggaaggaagg 15180
aaggagggag agagggagga agggaaagaa agaaagaaag aaagaaagaa agaaagaaag 15240
aaagaaagaa agaaagagag agagagagag agaaagaaag aaagaaagaa agaaagaaag 15300
agaaagaaag aaagagaaag aaagaaagaa aggtcaacaa ggtgttcggg gctttgaagt 15360
atgatttttt tatgcatcag gttattccat gaaacttcta tgcaattgct agacaccttt 15420
atctagaacc aggaattctg tgtgtcttca tacctgtcac caaatatggc atcagatagt 15480
gtcactggaa tgcttgaaag gatgtttgaa aattctcatg gacatagtgc aagcaaaata 15540
aaatatttat tattatattc atcatggaat ttagagtgaa gcaaagaaac tcaatgccgt 15600
cttgcaaata aggcatactc accaatattt tttcaattta tccgttggac ggtgaggacc 15660
ctctctacac ctggtttata attcctttgt gatcacctaa tgagagagtg caaattaggg 15720
aaggcaatct ccaggattct caaggattga caatcgacct actctcccat taaatctact 15780
ttctttagct ttatgcagat ttaccttccc tgaatatttt tcctgagctt ttatcgtgag 15840
tcatttcaag aagctgttaa cattatatgg caattgcttg gggattggtt gtcacttttt 15900

aaactgtggt catcaggtac cctcatttga gaaatctttt tacaaaatta gtaatttaaa 15960
aaaaacttca gtgtcttcta agaatttata tgattccact ttttcaactt tcaagagcta 16020
tatatcctgt gttttccttt cttttctgtg aggtagttct gaagcaaatt atttcttaaa 16080
ctgtattaac atgcatgata ctttgtttta ttctttcctt acaattgttt gcttagttct 16140
actttattca ctctatttcc tgttttttgg cctcgataag gagcttaagg ctgttcaaga 16200
tcttagtcaa tatttgtgct tttgacttat ccttctttgt cacaagatga cctgcacagc 16260
tccaaagatc atgtccctca aacattcaaa agcaggaagc agaagatggt gtaggacaga 16320
ggttctcccc gagtgcctct ctcagaagag tccccagtgt atttcctata aatatcattg 16380
accatagctg gatcacactg ccacactttc tgtaaggaag gatggaggtg tgagtattgg 16440
attttctagg cttctgtagt gggaagtagg caagagagaa gaaggttgag aaataccttc 16500
aggtattcaa tcaccttgtt tatccccaaa tgggacaaat caaatcatgg ttattgaaca 16560
taaaaattat ttgcaatgac ttccataatt gtgttggttg attatttagg ttactgtcta 16620
cacacacaca cacacacaca cacccacaca atcactcaca ttcacatgca tacacccaag 16680
aaaaagattt aatgagatac ctgtgtgtgt gtgttgtata tgtgtatgtg tgtgcatgca 16740
tgattcctat acgatgaaaa attggaaata gcatcataat gagatgttaa gagatactat 16800
tcaccttaaa ttaaaaccta agaaggtatt tctgcaaact ttcaaaattt cgtatgagaa 16860
taaagtgcat tgtatgccct catgatagta tattatgtat tgtgtacata accagtaatc 16920
taccagttta aaacttatta tgtaactgtt tttatttaga tgatgttttg cgtattatta 16980
aagatgaaga cttcaaaata ctggagcaaa gacaagtagt attatcggaa aaagaagcac 17040
aagcactgtg caaggaatat gaaaatgaag actattttaa taaacttata gaaaacatga 17100
ccaggtagaa tccaggttga gaaaattcag tctgatttat ttttgaatca tgtaaacttt 17160
ctcttaagtc tggtttctat ttaagctgcc aaactcctca agtctgtaaa catgttctga 17220
gtttacttct ctaccagtcc tgctcccatc ccaatcctct gctcacctgc ctgtcttctc 17280
tagtcttcct ggaatgttac tggaatagta ggaaggtctc agaggaagtt actggagtcc 17340
cattgagttg aacaccagaa gtccagggtg tacaatacta tgaagtttaa gcagagaact 17400
gactgactct caagagcttt tgtaatctta tagttaacta gttctaggaa agaatgagtt 17460
gctagaaatg tgctgggttt ctttctagaa gaatgactga aatagcaatt gaggctgaaa 17520
tcctgcaagg gcatataaac cagcaggacc atgggaacaa tgaaagaaac atctacaaga 17580
atcatggcac tccccttccc ttcctttccc tgcttctatt cttttataga tatgctcttg 17640
aaaacatatc aacggaaaaa aatcacagac ctctgttaaa agattatttt acatgttaca 17700
tgtttttgat ctatttaaca atatgcattt gtattgccta tacataatta gctactgagc 17760
tacacttctt attacctctt ctttgttttc ttttctaata gtggtccatc tctagccctt 17820
gttttattga gagacaatgg cttgcaatac tggaaacaat tactgggacc aagaactgtt 17880
gaagaagcca ttgaatattt tccagagagg taggattcat accatgggtc actatctgtt 17940
ttcgtgggct ccattttaaa caccttttta gatgtagagt attggaacca cagctctagt 18000

ccaagggagc ttctcaggaa agctaccttt cctggggacg gagtggactg cttatgactg 18060

gaggtccaac ccagtgacag cctgaggatg gccaaagatt ttcaagaaac cactgtgagg 18120

tgctaggctt ggggctggga gctaagaggt gtatatttca gtcaacaggt catctcttgg 18180

gagaaatatc caccctaccc gctatttagg agaaattcct ttgagaaggt tatggtggca 18240

gctgtagctt ctttaactga tcatgagttt taagacaatg atgactaatt gaatatgtac 18300

tctacaattc agggggaaca tttattgact tagcttactg attaatctta gaagtcagct 18360

aaactttgta atcaacttga cccctggcat attacctggc a 18401

<210> 36
<211> 20197
<212> DNA
<213> Homo sapiens

<220>
<221> misc_feature
<222> (1)..(20197)
<223> chromosome 10 nucleotides 73954184 to 73974380

<400> 36
aagtgactca attaattaag tatctcttat gcttaacaag agtgattaca acaaaatttc 60

ttaacctttt gtgtaacaca taatgttaaa acttcctttt aaatcatgct gggtatcaaa 120

ttctctggga cttgtgcagt gcttggattg tcatattaaa gataaagtat gttaatttaa 180

agtgacagga acgaaatcat tctgaaggga gcagggcaag gacatatgca tcatgcgctg 240

aacctttgtt cctgaagcat tactatggat tttggagcat taagtaattt aaagcacatg 300

ctgctgagtc agtacctgcc agaaatccta atcagaagat gagaaaaaga aaaaacattg 360

acattgtttg attatatgaa caaactaatc aaatatacat atcacaaaac ataatataca 420

aagaaacagt taaaacctgt aataatatca ctgaacacct ggaagtagga aatgaggaat 480

acatagaaat gttttagata ttatctgcta ggcacaaaac aaaacaagga tgtctcatta 540

ctgtttctat atataatatt tttttaactt catggcaaat agttgctttg ccactcatat 600

taatataggc aatcaaatat ggatgagttc agataattag taactactct aagaaagagt 660

acataatggc tccttagaaa aaggtctcct ccaaaattat gctgcattaa ataaagtgat 720

acccacattg gtattaaaaa aaaaaaagca gtcatcctga aggctggcat ttggacaagc 780

agttgttgtt agctaattta gctgggtacc actccaaact attttcaaat atcaaatggt 840

tttaaagatt tttttcaata aataaacttc actgaccagg aaataaaatt tgacctatat 900

ttaccatcct attctaacca cactgaatag cattgtaaaa tttcttgact gaaaaatgga 960

tgctattaac aattcacaag cctacagctg gaacttatgt gacagagccc aagggatttt 1020

acggcacagg aatgagatga atcactgtga acacaggaca actgagacat gtgaaatcaa 1080

actgggggcc tctctttagt taatggactc ggtatttaac ccgggctagt tgctcattac 1140

agagatagca caggagattt ttatagctaa ttaaactggg aaggaaagga ctgtggagag 1200

aacaattacg cagagggtat actagtctgc agcttttcaa gtaaacgaag aaagaaaatc 1260

atatatatga tagcagagaa ctcctttctt aaactgagta aagggctcaa agtcatgcaa 1320
aacagctata atttgtagag ctagatactt tacctattgt cccaatttta ctgagaatgt 1380
aactgacaga gaggttaact ggtctcttgc tcaaggttac ccatcagtta tgtgggagag 1440
cctatattta aatacaggac tgtctatatc caaagtccac actcttcatc ccactctatt 1500
acacataaag aagttcagca atccacaaga ctatgttttt cataccaatt cagcatactt 1560
tataacatct actaaccatt tacacacagc tgccaaacag agtactgaga tactcaaaat 1620
ggaaatgcct tggaaaatat ttcagatacc aaagccagat tttttttttt agggttatgg 1680
tatataaaaa agaactaaag ttgctaattt gaaagaggag ctaaaactga gtaaatgttt 1740
taaaaataat ttaaagaact ttggatggga aagaaggaag atggcaaaat ctgtaactaa 1800
aaataaaaca aaattccagc aggtgtgaaa ggatatgaaa tgttgaattt gttcctcaat 1860
tcacttttgc aaattaatta cattcttttt tttttaagac agagtctcac tctgttgacc 1920
aggctggagt gcagtggcac aatcttggct cactgcacct ccgcctcccg ggttcaagtg 1980
aatctcctgc ctcagcctcc caagtagctg ggattacagg cgcctgccac caagcccagc 2040
taatttttgt atctttagta gagttagggt ttcaccatgt tggtcaggct ggtctcgaac 2100
tcctgacctc aagtgatctg cctgcctcgg cctcccaaag tgctgggatt acaggcatga 2160
gccactgcgc ccagccagta aattaattac atcctaaatt ttgcttgtct aaagactata 2220
atgagcagct ccattcaaat gatgctatat ttaacatata attaatcaat atttgaaaga 2280
gttaataaat tgtttagttt ataatagtaa attgtagaga caggattcca aattatctta 2340
aatactacat gataccacct ttcctatgtc taatccagtt gtgtatacga cctcttaaaa 2400
attttactac cttacctaat actgtttgct caaaaaaaaa ccttgattat aatcagcaaa 2460
gatccaagtt ttcaaataca ggtgacagtg cttactttaa gagtaatggc ttcaaaaaaa 2520
aaaaaaaaaa aaagaaagca cttctgaaga tgtcaagtgg tactcatggt atatgaaaca 2580
ataatggagc cagtaacagt aaaattgata ttctgatttt taaattcagg tattaaaatg 2640
tatatatccc atattttctt tctataaaat gaagtgttat gtggtttctc aggttagtat 2700
gaatttctag ctgtacttta tcaaagcaga aaagattaac ttcaactctt agcaaaaaga 2760
aacaaacatc aaggtttttt tttgagcaaa cagtattaag gcagtaaaat tttaataaaa 2820
ggtcatacag acaactggat tagacattca gaaggtggta tcacatagtg ttcaagagaa 2880
tttggaatcc tagctatatg attttaagct agctacttat cttttctgta ctttttcttt 2940
tgtttttgtt ttgttttgtt ttgttttttt gagacagggt ctcactctgt cgcccaggct 3000
gaagtgcagt agtgcaatct gcagcctctc cctgctgggc tcaagcagtc ccccgacctt 3060
atcctcctga gtagctgaaa ctataggcat gcatcaccac acctggcaac cttttgtatt 3120
ttttgtagag aagggctatt gctatgttgc ccagggtggt ctggaactcc tgagctcaag 3180
tagcctccta aagcactggg attacaggca tgagccaccg tgcccagccc ctattttctc 3240
aatccgtaaa atgatgataa aaatagacag cttaagatag tggttgtgtg cactaaatga 3300

gtaattatgt ataagcctgg aacataataa gcactcaaaa agcttcttat tattgacgat 3360
aattcccaca agacaaatat agtcaagcat ttttcattta gaaatttcac ttttcttttc 3420
tttcttgaga cggaatcttg ctctattgcc cggctggagt gcagtgatgc caatccaact 3480
cactgcaacc tccgcctctc aggttcaagc tattctctgt ctcagcctcc caggtagctg 3540
ggattacagg tgtgcaccac catacccagc taatttttag atttttagtg gagatggggt 3600
ttcaccatat tggccaggtt ggtcttgaac tcctgacctc aagtgatcca cctgcctcgg 3660
cctcccaaag tgccgcgatt acaggcctga gcctctgggc ctagcccaga aatttcactt 3720
caaaccaaaa aaataaacca ctgacaacca atgagtgaaa atgaggtttt aaaaaagggc 3780
ccagtcatca tgcccaaagt aacatgtgac tttgaaatat ctgtgaggaa cttctggctt 3840
caaggaaata cttttattct cttggcatct gttacagtgt ttttaaagca agaatttccc 3900
tatataaaga aaaagaaagc agaaatgata taccctactt taaaaatggg atagggcaaa 3960
tgaaaacaca gataacaaga aagactaata aaattatatt tacatattca tatttagcca 4020
aaaacctgaa taaatatatt aattaaccaa gaatttccac ttgagtggtt tcaggtctct 4080
ggaagccaag agagagaggg aaagacatgg ctaattaaca atgcagaatt acatgtcata 4140
ttaacctgtt atctaaattt gactagagta gttcaaataa atgtggagaa ttcatcaaac 4200
cctgtcccat gagactttta gtaccctatg tcgtaattta catttcctca atcttcaaaa 4260
catttaactc ttaaaagaca gaaaatttct taacaactta ctatatatgt caaggagtaa 4320
aactacactt gaccttattc tagggtctta ttttatctga gaaataaagt aaataattag 4380
aactcgaata aagcacgaag aagtattaca ttataggaaa aaatgtgttc atattctctt 4440
tagaagaacc aacaatttcc cccaaaagga atggtatttt aaagttaatt tgttaatatt 4500
ttcaaaaaga actttcacct agtaaaagag aatgtagtaa tattaaagct ttgattttca 4560
actatgactt ccatctgttc ctctactaat catattgtct gttataaaca ctataatcaa 4620
tacttccctt tcttggtttt gactatactt ttatctcttt acagataaat gtggataata 4680
tttcattttt atttgatgta aaatgttttc ttgttccatg taaacaaata ttcttgtaga 4740
taacataaag gaggtggaaa taaaatacac ataaattttt tcttccaagt ttgagagact 4800
gtcaaggttc tgaggggact gtagttacaa ataatggaaa aactcaattc agaggcagta 4860
tttctgaaac gttcagggag aggttggggg aaaggggaat caataggtag gaaagaataa 4920
caaatctatt ctctaaaaaa gaaatacatg gtggtatgat gataatgaac aattggtttc 4980
aatagacaat tttattgctt gaaaatagtg tataaactat agcttttaga agacataagg 5040
ggactgaatc caacgttgcg gaaattggta ttccttgctt aaggaacaat ttcatctata 5100
ccaagaaatt ttattctgtg actttataaa aaaacatggt gatgtttgat gtggagaaag 5160
ttgtctatta catgcaatag aaacaggaaa tacaaaatat aatttctatc tttggtttgc 5220
tgaaggaaaa aaggggtaag agaaaatggg aaaataaaga aggctacaaa ccacttgaaa 5280
taaatcatgc aaataaaaga gagcaactaa atgattacat gaaagaaata tttaacaata 5340
atgtaaaata aagcctccac ttagaaataa ttgctttgtt ttatatgcca actagatggt 5400

aaaatcataa aagatgataa atgaaacaaa gaaaaaatat agttttcatt aaataaataa 5460
tccaaattca tgaaatgact gtattgacaa attgttagca gaatacatgg atactagggg 5520
aaggtgcttt tttttttttt tttttttttt cttttcacaa gaaggattca acacaggaag 5580
gtgcatttag aaagaatcta tgacatatta cattaaaaca aaagctggca ggacatggtg 5640
gctcacgcct gtaatcgcaa cacttcggga ggccaaggtg ggcagattac ctgaggtcag 5700
gagttcaaga ccagcctggc caacatggta aaaccccgtc tctactaaaa atacaaaaaa 5760
atttagccgg gtgtggtggt ggatgcctgt aatcccagct actcagaagg ctgaggcagg 5820
agaatcgctt gggaacccag gaggcagagg ttgcagtggg ccgagatcac gacattgcac 5880
tccagcctgg gtgacaggag cgaaactcca tatcaaaata ataataataa taataataat 5940
aataataata ataatgccag aatatatttt gtattaccat ctctataatg ctaataagct 6000
atttataata attttcaaat gtcttctaag agaaaatccg aactctttca agatttgaag 6060
ttttaaactt ttaaacctag tctcatattt tcaaaataat atcacttatc tacgaaacat 6120
attcttaaaa ccagtggtaa ccatagctgt taccattctt tatacgagac ctatgatagt 6180
agatggtttg gaaatgcaat agactatgca agtgccaata ataatgtatt tcataacttt 6240
tctacaaggg aaatttctag tattctaaaa gtattagtgt ccattaacat aaagtaataa 6300
tgggctctgg aaaaagattc aactacaaat tcaaaagctg cttaattaaa ttcagttctc 6360
actgactatc agttgaaata gtaatgtctt atttacttct tacagggaac cttctagtta 6420
attaaacaga tcaggaaaaa aatattttag caaaacaaga aataactatc caattatttg 6480
tcaatatcag ttgcaccact ctttcctatt ttgcatctgg aggttccatt attcaggtag 6540
aaatttaaaa catcccagga aaatattatt tttatatttg ttcttacttt caatgacaaa 6600
aaggctgaag tttaattcaa ttagaaggca ataaaataag ctagtgtttg ttactggtca 6660
aattttggac tttgttaaat ggtgtgctaa aagttaagat aatgggggct aagacaatag 6720
gagtaaataa ttctttctct aagatttaat agtttatctt ccaaattaaa aaaaacagag 6780
ggagagggaa aaaaattatc catttaaaaa tcaaagcaag tctaaaagga aattcctctt 6840
aggtgtttag ctttctaact acatttctgg ctataatgca taaaattact ataatatcat 6900
tacacacaaa tctgtttttt taacagaaaa ttgtcttaga attcatatat tctagaagca 6960
gttacttaag aattaagatt ttctggatga gagaacaaaa gagtcatgaa gatctaaaga 7020
aaaatgatgg tggctcatac ctgtaatccc agcactttgc gaagccaagg cgggcggatc 7080
atgaggtcag gatatcgaga ccatcctggc caacatggtg aaaccctgtc tctactaaaa 7140
ataaaaaaat tagctgggca tggtggtgtg tgcctgtaat cccagctact taagaggctg 7200
aggcagaaga atcgcttgaa ccagggagtc ggaggttgca gtgagctgag atggtgccac 7260
tgcactccag cctggcgaca gagcgagatt ccatctcaaa aaaaataaaa aataaaaaaa 7320
gaaagaaaaa tgaaaagact gacagttgag aaagtactga cagttacaat tatcaagcga 7380
aagacttcat acataacaaa aaaacatgga aaacttttaa actgatcaaa caataaatga 7440

atttattcat tacataaggt tttatttttt ttttttttag tcctttatct tttttttttg 7500
agacggagtc tcgctctgtt gcccaggctg gagcgcaatg gcacaatctt ggctcactgc 7560
aacctctgcc tcctgggttc aagtgattct ccaaataacc aaaaaaagtt atttattgct 7620
tggtgtggtg gctcacgcct gtaatcacag cactttggga ggccgaggca ggcagatcac 7680
gaggtcagga gattgagacc atcctggcta acacagtgaa accccatctg tactaaaaat 7740
acaaaaaaag tagctgggca tggtggcggg agcctgtagt cctagctact cgagaggctg 7800
aggcaggaga atggtgcaaa cccgggaggt ggagcttgca gtgagccgag attgctccac 7860
tgcactccag cccaggcgac agagcgagac tccatctcaa aaaaaaaaaa gttatttatt 7920
aaaatgaatt tgctataagt tattactgac ttaatgagtc agacctccac caaggaatat 7980
gtgattgact actgtagtta attttttgca agtcattaaa catttattgt tgcctcctgt 8040
gtgccaggcc catgctggac tctgagtgac acaaaaacag gtaagatagg gacctgaccc 8100
tccaaagact ctgaaatcag aagtgttgca gtaagtcctc acattggata gggtacttta 8160
ccatagataa agcagagaca ccaaggatca gagaaaagat gaagctgtcc caaagcagag 8220
ttaggacctt gagccgaggt ctcctaacat ctagttcagc actcttcctt tttgatcatt 8280
cttttagaat atgtgacttt aaaaagagga tattcacaga tggaggcatt ttaaggcact 8340
gtgccacctt aataggaaag gtaactttta ggcaggtggc cttagctgag atctctccca 8400
aatcttaagg cagctactca ttcttttcac ttgtataaaa gagctcaaca cttatctctc 8460
caccttacca ggatatggac ttttatgtaa taatgcagga attggcacat ttctgcaaaa 8520
tcagttttta ctaaccctga cctctcccca tctagaccct cttttgtgat cctgcctttt 8580
ccagccactc catgattgtc cttctgactt cctgttcatt gtgctttgta gaaccatgcc 8640
tggttctttc ttctttctga agtgctttct tcctcctctg taaaattgac cctttacttt 8700
tctttctgac cctgtgtaat attttctttt tggagaatct ggcctgaaag gaaaaaacat 8760
gtttgactgc tttatgagac aataaaactg aaagtctcat tttctatacc taagaataat 8820
ttgttatatt atttattcag gtataaataa ttaaaaataa gtatagtgca aacttatagg 8880
caaatctgga gcagtcttca taattattac aaaagcacta caaggacctt tgtaattttc 8940
tagttaagat tattgctaga cactatcata aagtctgaga aaggtcaaat cagggaagcg 9000
ttttgtaata aatgccaata cagtattggg gggcttatta atctttatga tgactattga 9060
gttaacattc aaaagtcaac aaacactgaa ttagccctta atataaacag gaggcaatac 9120
atgagagtta ccttacatgg aaaaatatag aaccttctgc ctctttctgt agaggtatgt 9180
agaggcacag accccctaac ctaagcatgc tcttttggta agataaacag gtattcagtg 9240
atttctcaaa cataactata gaggaactta aaaatcttct tagtaaatga taggatatat 9300
gtatagaagt gtcatagttt gctcaccttt ccatcaaagc gttttattat atattgatcc 9360
agacccaagt ctgtaaaaga aaaggaaaaa acatttagct actgataata actcaagtga 9420
aatttataaa atgacagctc ttggttttgt ttccagtcat tcagttaaaa gtacaaaaca 9480
tttattcaaa ggccacagga atgaatattt tgccccactt tcagatttaa agtgtgagtg 9540

ctaagagata ccaacccttt tattttacat atcagaaaag taaggctcta agaaggtaaa 9600
aatgtgcaaa cctattcaac agtagagtcc agttagaact ctggactcct cccaaggtgg 9660
tgctcttttc attatatcaa ggtgctatat tacttttcat ttaggtaagg aatattagtt 9720
taatctgaat gcataataat tttttctatc aagttcaaag aaaataatta aacaccaaaa 9780
aaaaaaaaaa aaaactattg cagtacacct aaacagacac aaacagacca tcttaacatt 9840
accttttaaa attaggttaa atttaaaaat ttacttggga atacctagct ttggattctg 9900
tgcactgtga aaaattaagt gactatattc ggtggcattg agggcaggga tagttttgtc 9960
tttacacata gctttatccc cagcatttag aacagtgctt gatcaataaa tatctgtaaa 10020
agaatgaatg aataagaaac agtccttgcc ttgaaaagtc acaatttagt tagaaagaca 10080
tacggatata ataattaaca ttacaaggga gcatacaatt agttcaataa aagggtgctg 10140
tgggagtttt gagggtggag aaattagtac tcagttacac ttattttctt gcataattat 10200
tcagttattt tctttcagtt gtcactagtt caggccctta cctcactatg cccacagtat 10260
tacaatcatc agattatcat tgctcccaga aaaagctttt tgcatttcta atttaaacat 10320
ttggttatac attcttctta tctgaaatgt gcctcatctt ccagaagcca gccctaaaca 10380
aactgaacca ctgactgtaa aagaaacaga taatttaaga cacagaaaga aatgttagaa 10440
acaattttaa tttgcaacct gacagatatt taagagagtg taagtcataa aggctgatat 10500
ttatcagaac aggcttctga taaaagaaca gagaacaaga aagagttcta gacaattaaa 10560
actttgattg cctatctccc tctatttttt attttatttt attttatttt attttattta 10620
tttattttga aacagagtct tgctctgtca cccaagctgg agtgcagtgg catgatcttg 10680
gctcactgca acctccacct cctgggttca agtgattctc ctgcctcggc ctcccaagta 10740
gctgggatta caggtgtgtg ccacgatacc tgtctgattt ttgtattttt agtaaagatg 10800
gggttttgcc aggttggcca ggctactagg tcaggtctag aactcctaac ctcaggtgat 10860
ccacccgcct cagcctccca aagtgctggg attacaggca taagccactg catccagccc 10920
tattttttaa tgtactgtca aaagatgagg ataaggtaac ctcccaaact actgccaaag 10980
gacagcggga tagaaaatag aaaatataag atagaattat agaaaacaga gactcaatcc 11040
aagaggttta gtatcctaca ggacctttct cattcagaaa cagggagaaa ataatccaaa 11100
aaatactagc agaaactttc tcataactat agagagacag actaaaagac cttaccaagt 11160
gttctaagca tattgaacaa aaaaaactac ccacacttca gtgtatcctt gtgaaatttc 11220
agaataccaa ggataaaaaa atcctaaaga ttttcaggaa aaaatcttcc tggtccctta 11280
caaaggcagg agaatcagaa tagaatcaga tgtctcacaa gtaaaacttg gtacatcaac 11340
taaggcagat gtatgcttac taattgcatc agcactgtcc aacagaaata tagtgtgaat 11400
aacaaatgca agtcacatgt gtaatttaaa attttctaac agccatcttt aaaaaggtag 11460
aaaggaagaa attctgtcat ttgaggcaac atggatgcaa ctggctgaca ttatgttaaa 11520
tgaaataagg caggaacaga aagttaaaca ccgcatgttc tcatccctac gtggaagcca 11580

gaaaaagtcg atctcatata agtagaaagt agaacagggt gtaccacagg ctgggaagga 11640
taggggaaat gggagggact agaaaagatt tgttaaagga tacaaaatta cacctagata 11700
ggaagaacaa gttctagtgt tctatagcat tgtagagtta acaacaatat ttatacagtt 11760
tcaaatatct agaaggaagg cactgacaca aagaaatgac aaatgtttga tggtgatatg 11820
ctaattaccc ttatctaatc actatataaa ttatatgtat caaaacagca ctatgtaccc 11880
cataaatatg tacaattatt atgtcaattt aaaaataagg ttaattaatt aattaattat 11940
tttttgagat ggagtctcgc tttgtccccc aggctggagt gcagtggcat gatctcggct 12000
cactgcaacc tccacctcct gggttcaagc gattctcctg ccttagcctc ctgagtagct 12060
gggactacag gtgcgtgcca ccatgcccgg ctaatttttt tttttgtatt tttagtagag 12120
acagggtttc accgtgttag tcaggatggt cttgatctcc tgacctcatg atccgcccgc 12180
ctcagcctcc caaagtgctg ggataaaagg cgtgagccac cgtgcccaga caaaaataac 12240
gttaaaattt taaaagggca aaaagaaaca agtgaaatta attttaatac tttatttaac 12300
tgaatatatc caaatagtat cattttaagt agtaatcaag agaaaatatt aatgagatat 12360
tttacatttt ttaatactac attttcaaaa tcggggtgta tcttgtaatt ataatacatc 12420
tcaactcaga cttgccacat ttcaagtgct cagtagccaa atatggctac tggctactgt 12480
attagacagc acaggtttta ggttatcagg gcaataccca gaataactca aaatgtaact 12540
taaaatttta aaggtggtta cttctgtgac atgaactgtg aagatggaga agatggaaca 12600
catttatgtt ttatatttta tgctcttcat tatattaaaa aaatggtgag ggggaagggg 12660
catgggagga caacctggca ctcactgaga attattgtca ttttataagc aaatactgac 12720
atgaagtagg ccaagacttt cattatttca agttgaaaga aatgattatt tcaccttgtt 12780
atgttttgat ttatttacct gccaaatgaa ggagatggtt ggattagatc agttcttaaa 12840
atgtttggaa gtggagtgtg gggaatgtgg atggatgaag taattcaagc tattgtccct 12900
aactactcct ctagaaaatc taataggccc aattactggg ggcagggaga gcatggagaa 12960
tgaaaggaga aaaaatagca tgtgtagttt aaaaacaata cttaggtgac ttccatatga 13020
catttccctt tcagtttgct acccaaacct tttggtccca gcttagaaac accagactat 13080
agctatgttg ttttcactga gatgtttcct atggttttta aacttttatt caaagaactt 13140
tcagtcattt aatttaatgc tcatagtgtg atataaacac tacctttgcc tttttattga 13200
taagaaagcc aaggctcaga aggttaaatg actgtttttt tattttaact ctaataatct 13260
tctaattatg gcagttgcct cttttcttga atccacaccc aaaatactat aaatatgtgt 13320
gtaaaagtag agtatatgta aaagtagagt atgtgtaaag gtacacacat actgttaaga 13380
atagtctcag tttaagcctc agagtgaaca tttaaaagac ttgtttaaag acaatggcaa 13440
ctgactttgt caaactcatt ttgtcaaaag acacaagggc aactgaatca aatatgtcat 13500
ttcaagatct acaaattaga ataataatta acattacaag ggagcataca attagttcaa 13560
taaaagggtg ctgtgggagt tctgagggtg gagaaattag tactcagtta cacttatttt 13620
cttgcataat tattcagttc ttttctttca gttgtcacta gttcaggccc ttacctcact 13680

atgcccacag tattacaata atcagattat cagttaaaaa taacagtaca aactatcaag 13740
gagaagactg aaacatttga aaatatcaat attgacaact tctgtgcatt aaaaatcaac 13800
acaaacaaaa taaaaagaca ggccccagac taggacagga gatctgcaat gtatatcaca 13860
gattttagtg agaatatata aactactttt actaaaaagg ataatcaaat ggaaaaatga 13920
acagagacta taaacaggca attcatagaa aaggcaatgt aagtagtcaa taacatgcag 13980
agatgataag cctcacttgt cattagtgaa atgcaaaata aaaccagaaa aatatttaaa 14040
acactttaaa gtgtagagtt tttgtgtgtc tgtgatcaaa gttaagctgt tatcagcttg 14100
aaataatctg ttataattat gttttttgta agcctcacgg tagccacaaa gaaaaatcct 14160
gtaatagata ctctaaaaat aaaaggcaag gaattaaaat atactaccag agaaaataac 14220
cacaaaggaa gacaaagaag acagtaacaa aggaaaaaaa ggaagagagg ggttacaaaa 14280
caaccagaaa atgaggaaca aaatggctat aataaagtcc ttacatatca ataataacag 14340
tgactataaa tggtctaaat tctccaatta aaaaacatag aatggctgaa taaatttttt 14400
ttaaaaagac ccaactatat gatgcctata agagactcat ttcaccagta aggacacaaa 14460
cagactgaaa gtaagtaaag ggaaaaagat atttggaaat agaaaccaaa agagagcagg 14520
agtagctata cccagataaa atagagtata agtcaaaaac tgtaaaaaga gacaaagaag 14580
gtattattta gtaataaagg agtcaattca ttaagaagat atgacaattg taaatatata 14640
tgaacccaat ctcagtgcac ctaaatataa agcaaatatt aatagatcta aacagagaga 14700
gagattgcaa tacaataata ttagggggct tcaacatccc actttcagta acgggcagat 14760
catctagata gaaaatcaat aaagaaacat cagatttaaa ctgtactctg gaccaaatgg 14820
acttaagaca gatttacaga acattccatc caactgctac agaatacaca ttcttctcac 14880
ctgtacatga acacttctcc aggacagatc atatgttagc ccacaaaaca agtcttaaca 14940
aatttaagaa gactgaaact gtatcaaata tcttttctga ccacaatgat atataactag 15000
aaataaataa caggaggaac tttggaaact ttacaaagat gtggaaatca agcaatatgc 15060
tcctgaataa tgggtcaatg aagaaattaa aaagaaaatt ttaaacattt attgagacaa 15120
acaaaaatgg aaacgcaata tgtttttgct atggaatata ccaaaaacaa ttctaagagg 15180
gaagttgata acaataaaca ctacactgaa aaagaagaaa gccctcaaat aaacaaccta 15240
atattgcatc ttagggaact agaaaaacaa gaacaaactc aactaaatat tagtaggagg 15300
aaggaaataa taaagatcaa gcagatagag agtagaaaaa gcacaaagag acaataattt 15360
actttcataa gtttgttaaa atttaaatct gataatacca agtgttggca gcagtgcaga 15420
ggaagaaact catatattct tggagagaag ataaattatt ctatcaactc tggaaatcaa 15480
tttggcaacg tctaccaaat gtgaagagga atgtactcta tgggctagca agtccagttc 15540
caggtatatg ctgaaaataa cttcttggct gtgcgtggtg gctcatgcct gtaatcccag 15600
cactttggga ggccgagaca ggtgaatcac ctgaggtcag gagttcaaga ccaatctgac 15660
caacagggtg aaaccccatc tctactaaaa atacaaaatt agctaggcat ggtggcgcat 15720

gcctgtaatc ccagctactt gggaggctga ggcaggggaa tcacttgaac ccaggagatg 15780
gaggttgcag tgagccgaga ttgtgccact gcactccagc ctgggcaacc agagtgaaac 15840
tccatctcaa aaaaaaaaaa aaaaaaagaa aagagaaaga aaataactta ctgcaaatat 15900
gcaccagggc acatatagga gaatattcac tgcagtatgg tttgtaatga ttaaaaaaaa 15960
aaactggcat ggagccagat gtggtatctt acacttataa tcccagcagt ttggaagtct 16020
gaggtgggaa gatcacttga ggccaagagt ttgagagcaa cctgggaaac acagtgagac 16080
acttgtctct acaaaaaaaa aatttaatta gcttggtgtg acggtacaca cctgtagtcc 16140
tagctactca ggaagtgaaa gtgggggata acttgagccc aggagttcaa ggttacatga 16200
actatgaaca tgccactgca ttctagcctg ggcaacagag caagaccctg tctctaaaat 16260
aaaacaaaac aaaaaactgg catggtagat tagattactg atctcaaata tttacttcct 16320
tcccactgac ctctatggaa aagccattgg gctttgttgt gtaacttgtt ttagccaatg 16380
ggatattagc agatgggaca taagcatcct taaaatggac ttctgaggtt ggctttcttt 16440
cttatatctt ggccatctat aagaaaaata tatcctggat gggccagtgg tctaaaaagt 16500
tataaacaca taaagcaggc tgacaaatct gctgacaaac tttcagagct atcctagctt 16560
aattagacaa cctctagcct atctacagag ctcagctgag agcagcagag agcacttcat 16620
gggatctgca gatgaaagtg cctttgcaaa gattatgaca gaaagagaag tctagcatgg 16680
ctgactccat cttgcttcta gtctcatagg ctagctgtcc ttactcattc ccgggcaaga 16740
gcccgggaat tggaggaatt tagactatag tttaacttga aagcaagaat gctaatagtc 16800
cctccctaaa actccttgct cagcgaccaa agcaaccttt gtaaaactag taaaaggcca 16860
caagattagg attatggaag aggcctgaat tccattaaaa tgtaggctta gtttctctaa 16920
tcccttacaa ctcaggtggc atgtgcccag aggtcacaag acttgtgact tccccaactg 16980
cttctataaa tagcatcact attatagaat ctaagattga tcttttgaga tatttttcag 17040
acttttgcat tctggcaact gaatgacccc acccagaccc atgactcatg actgaacctg 17100
tcctgtggcc caaacataga ggcagcctca gtgcatgagg actgttttcc acacccctgt 17160
gatttcatcc tcaaccaatc tgcagcaccc attccctagt cccctgccca ccaaactatc 17220
cataaaaacc ctagcttctc agtccttagg gaggctgact ttatgaataa taaactcctg 17280
tccttctact tggctggcct tgcattaatt aaactctctt tactgcaata cagctgtatc 17340
agttaatcag ctctatctgt gcagtgggca acaagaactt gtcaggcaat tacagagaag 17400
catgagctaa ataaatgttt gttatgccac tgacattttc tggttggttg ttacatagca 17460
ataactgact aataaatcat atactggcat atttgtataa atgtatacaa ataatatcca 17520
caacttatag cagtgaaaat aaaagaacgg ctacctgttc tttacataaa agtaagggaa 17580
acttataaaa cttatacaaa agtaagggaa atgtagtaat catgctgcag taataaatat 17640
aatcatcccc tagggaaaga caatgaatat acatgatgtt atagtcttca acaaaaatga 17700
taaacaccaa attaataaca gtggttatct ctaataagaa tgggagtgaa atgggattgc 17760
agataactct ttttatggac actgaaattt taatttcatg tcactttcaa gtttcacaaa 17820

gctgcttctt gtgatttttt ttcacccatt taaaaaatgt aaaaaccatt ctctgctcat 17880
gggccataca gaaataggca gttggctgta gatggccaat ccctgctctg tactaagcag 17940
cactacagag gattaaaaac aaaaggagtg agcaggatat tatcttgact ttttcacagg 18000
actcatgaca ggggtgcctt gtttgcttag cctgcccctg tctacctgtt gcgggaggga 18060
gtgtgcaagc aaacaagtgc aggaaccaga gcaagcaagt gcaggaacca gccaactgct 18120
tttgggtgcc agcaggagca aacgccgtac gggccacgca gcagcatctg gatgggggtg 18180
cctgcaattc ctgaagcccc agagggtggt tacagtgctc ctttagctct gccatccaca 18240
gatggcttaa gtgttaacag ctcagcgggc cctctgcctc attatgtggg acagctgccc 18300
tccgccagca agggcaaagg gccagtgtga cagccttttt tcggtatctg catttgttgg 18360
ctcccaagct cctgtctggt gtccaggaaa attgaggtcg cacgaacaaa ttgaaagatg 18420
gtgaatgcag aggattttac tgaacaatga aagtggctct cagcaggaag gcgagctgga 18480
aaagggatgg gatgggcagg taatcttcct ctcaagtcca gctgtctctg gctggactct 18540
tcaccaaaaa gtcaagccat caagctctcg cttgaagtca agtcacttct ctccaatgtt 18600
cagctgcttc tctgactgag tctggggtct ttataggcac aggacggggg tgggccatgg 18660
gtagtttagg aaaggcaaca tttgagcagg ccactggaat agaagttctc actttgggct 18720
gcaggtttca ggctttttgg cttgaaggtg gggttttgtc agggacctgc cctgtctgcc 18780
tagaattttt ctgcctcttg ttgctatcag tagaacacga ggacctttac tgaaggaact 18840
tatcttgttt ggaaaaaaac attagtgcaa aaatgttttt aaaatacatt ccatgtatat 18900
aaccatgtga aaaattatta accaaattaa aagtgctcta aggggccagg catggctcac 18960
acctataata ggctataggg cctataggct cacacctata atcctggcac tttgggaggc 19020
ccagaaagag gatcacgtga gcccaggagt tccagatcag cctgggcaac atagcgagac 19080
cccatctcaa tcaatcaaat caatccatca atcaataaag tactgtatgt aagattatta 19140
ttaaggatac ttagggacga acgtggtggc tcacgcctgt aatcccagtc ctttgggagg 19200
ctgaggtggg cagatcacga ggtcaggaga tcgagaccat cctggctaac acggtgaaac 19260
cccatctcta ccaaaaatac aaaaaattgg ctgggcatgg tggcaggcgc ctgtagtccc 19320
agctggaggc tgaggcaaga gaatggcatg aacccgggag gcagagcttg cagtgagctg 19380
agatcgcacc agtgcactcc agcctgggtg acatagcgag accctgtctc aaaaaaaaaa 19440
aaggatactt agggtaaggg aggagaccac tcttcatatt gtcttatgct gaccacccct 19500
catattgtct tatgcctaat ttctgcctcc aaagaaagaa gaagtaaaaa ctaaaaggca 19560
gaaatgaaat ccacaggcag acagctcggc gctgtgccct gggcctggta gttaaagatc 19620
aacccctgac ctaaacggtt atgttatcta tagattccag gcattgtatg gaaaagcact 19680
gtgaaaatcc ctgtcctgtt ctgttctgtt ctgttctgat taccggtgca tgcagccccc 19740
agtcacgtac ccgctgcttg ctcaattgat catgaccctc tcacacagac ccccttagag 19800
ttgtaagccc ttaaaaggga caggaattgc tcactcaggg agctcggttt ttggagacgt 19860

gagtcttgcc gaagctcccg gccgaataaa gctcttcctt ctttaacccg gtgtctgagg 19920

ggttttgtct gcggcctgtc ctgctacaag gggtcatcct agagataata tggagtccag 19980

gagaagtaaa tcacaatatg atctgtgttt ccctctttta cacccttttc atctccaatg 20040

aagatgaaat cacatcaaga taataaggtc agccagttgg atatggtgga aagaaaatag 20100

agatcctctt tgcaataggc tatgctttgc tctaaagaaa gctcatgttt ttctatatag 20160

aactttaaaa attctcccca tgccactgtt tcctcaa 20197

<210> 37
<211> 10273
<212> DNA
<213> Homo sapiens

<220>
<221> misc_feature
<222> (1)..(10273)
<223> chromosome 11 nucleotides 19435635 to 19445907

<400> 37
gccttattga ccatggtaag gagtttaggt tgaattttaa ttaggatgag aagccatttg 60

agagagttaa gcaggaatga caccctctga tttgcatttt ttaaaaggtc actagggctg 120

ccacatgaag tgaagggtga attgtaggga gtgggagaga ggagaacagg aaggccagtc 180

atccctgcag gagatgccag cctggggtgt caccagcaaa gtggagaaaa ggagacagat 240

tttgtaacaa gttttggggc accaccgtcc tgggcagagg ggagaacaga gatgcacgtg 300

acaaagcccc caccctgaag ggtcttgcag atgagttgca aactcatgtt ttgcccccaa 360

gaaataatta caaagaattg aagagaatga ggcacagaag tgggttgctg ggacatgcac 420

ggaatagggc acaaggagtt agagaagaaa gatggcaggg ggtcctccct tccaagccca 480

gttgccactc cttgcctttc ttatggcctc aaaaccccca tctggtcccc ctcctatgat 540

ggtttggcag caggcaagtt ccctatatga caggcatcct gctttcacca gtctgcaagc 600

ccagccagac gactgcatct gggaaatggc agtctcactg gcggaggagg tcctgaggag 660

ttctttgggc ctggtgccca tagctgccat cctcctgaag catgtggatg agaggtcagc 720

ccagtgacag ccacactgta gcagggagta ggcagtgctt gtgctgggtg accagccagc 780

tgtggctgct cccagagccc cttcccacag atgtctggtc aaatgtagag gaagcattag 840

gtgggggatc ctttacttgc acacggaatg attcaccaaa gccttcctgt gcctaagcca 900

cacattcatg tgaaatatga taaagtctag ccatgcatat gccttgggaa attgcccagg 960

accattccat ggctctctgt ctcctacaaa tgaattgtga taatagctaa gatttagatt 1020

gtacctacta tgtgtcaggc acttgtctaa gtgcttcatc tttgttaact tatttagtcc 1080

tcatgatgac caccttttat agaaaaggaa accgaggcac agagaggtga agactccaag 1140

tcccagagct agtgagaggt agagtcagga tttgaaccca aaaagcctta ctcctgagcc 1200

catgctttta accagttctc tacacatgtg aagaataatg ctcagactcc tgtgctagca 1260

tggggtgccc cttggcccct cataccacct ttctagtcat acctcctacc tctctgaggc 1320

agtccttcat ggctgttgag gtcaaatgct gagatggatg agtgtggatt caaatcccaa 1380
tctgccacca actacctgtg agactgtgca ggccactgtt gcagagacag catgagccac 1440
agcagggagt cttgagtgtg cgtgagcaca ggtggtgatg tggtgcttgg ggctggggag 1500
aataaaagga gatgagcgtg ggaaggcggg ctggggtcct tgttgctcag gcctagaaaa 1560
tgctaaaggc agggaagagc tacatcaatt gtaacttctg gttataagga aatttccagt 1620
tgtaagtcat agaaagccca atctatacag gcttatgcat aaagaaatgc attagcttgt 1680
gtcattgaat tgaccggggg tagctgtctt tgcaggccag gctaggtcca ggggcaacac 1740
actatgcttt taagaccttg tctttttgca tctttcctct ctgtgagcta tgttcttaga 1800
tactggtggc aaagtggctg ccaacagcct tatggtcaaa atcctcagtc tcccagaaca 1860
gagaaagttt ctgccttagc cagagtcctg ggattagctc tgagtggatt gaacagactt 1920
cactcaggtg atttccttat acctaaccca atcactgtgg cctgggggag gtgatcatct 1980
gatcagccag gcctggtcat gaactccctc taaggtagaa tgagtggagg acagtcatct 2040
ctattaccag gagaagagct aaagggtggc agcaagtagg acagatgttt ccatagagcc 2100
atctaagatg ttcaaggaat ggagtgggat aatcaaccag tattctagag agaatgctat 2160
gatggttgtg tggaggtgag tttgagacag aggatatagg gggtgtctac ttgtgctaga 2220
aaagatgcat tgggtataaa tactggaaaa gccttttgga atcagattga ggagccaggg 2280
taaggaattt aggcttttct ctaggtcttg ggagccacag aatgtttttg agcaagaaaa 2340
tgctatagcc tgagttgtgt cttaggatga ttaatactgc aggagcagtt agggtggatt 2400
gcaagtgggg agaagatgcc cttccaagac ttatcagcat ggtgtagtgg ataaagcaag 2460
aagtttagac ttggagagat taggattaaa atccttgttc tgctactttt tgtgtatctc 2520
tgggcaagat acttaacctt tctttgcctc agttttctca tctggaaaat gggataataa 2580
cctgaaagga atagtgtgat ttagaaaatg tctttagagc actcagcatg gtgtttcaca 2640
catagtcaat acacaacaaa tactagtttc tttccttttt cctttctctt cttgtgatcc 2700
agggacctga ttccactgaa gagtttgagg aagtagctgc tgacttagaa agaatgtggg 2760
gttgggcttt gaagtcagag atctgggtca aatccagctt tcttatttac cagttctgtg 2820
attttggaca acttaattaa cctccctgag cctcttttca catttgaaaa ttttccttta 2880
taatcccact ttacctaaca aataaagtac tcagcatgtt gcccacctgg cacatggtgg 2940
gtgaccccca aatagtagtt attagatttc atcatttaaa aaattccttt ggtagcttct 3000
tcccttgcgc ttcccaggtg agcaaactat cgcaaggaca aaaaaccaaa caccgcatgt 3060
tctcactcat aggtgggaat tgaacaatga gaacacttgg acacagggtg gggaacatca 3120
cacactgggg cctgtcgtgg ggtgggggga gggggagggg atagcattag gagatatacc 3180
taatgtaaat gggtgcagca caccaacatg gcacatgtat acatatgtaa caaacctgca 3240
tgttgcgcac atgtatctta gaacttaaag tataataaaa aaaaattccc ttgggttgga 3300
gggaatccaa gatgaacttc ctagataagt aaagatctca tctggcagcc ctggggtaga 3360
tgttttcctt tgcctgctag gtggggtcct gtacactcat ccccttctac atgcaataga 3420

cagagttgag acatagagca tgcttctggt gggattcata cttccaggtc cttagaatta 3480
aactcaggtc tgattttcca ggaggggact attagcactg cagcagcttc cgtctacaga 3540
gttcaggtgc tgcagttgta atttgtaacc agcactttcc catttggctc ctttgtggcc 3600
atctggtccc cttagtggag atctgttttg ttggcaacta cagtgctgag taaatgccac 3660
gggaaagttg aaatttactc accagagctc agtgcaccat ggagattccc ttctcagcct 3720
gcacagaatg atcatctcag accccgcctc ccctctccct ggtccattgc caacactcct 3780
ccacagggat ttttatagtg ctacgattat tttattgcca agaatcagag taattataac 3840
tttcggaggg atttcctagg aaaaaatgaa cttgggttta tgcataagca catgtgaaag 3900
aagaggtttg ggacttgtgc gttttatgtg gttttaggca ttaacgtgag tttcatttca 3960
cttaaaatac ttaataagtt tctacccact gtgtaatttg ttaataagga tgatgatgat 4020
gatagctgct gttaattgag aacctggtat gcaacagcca gtaggccagg tattttacaa 4080
gcatctcttc tggtcagtcc aacttggaag ggaccgttta gatcatgttt cttttcaaat 4140
catagcttga aatccattag tgagtcatga aatcaactta atgggtcaaa actagcattt 4200
taaaaatgaa ataggacaaa attacatgga acaaaatgga gtagcgagta tcagagagta 4260
ttttgagaat aaagaagaat gttttatgaa ccttatatct cggttatgaa cagatgtgtg 4320
tgtgtggaaa ctagactgtg ctggagtgca gtggcgcgat ctcgcctcac tgcaccctcc 4380
gcctcccgga ttccagcaat tctcctgcct cagcctcctg agtagctgag attacaggca 4440
cccgccatca cacctggcta attttttgtg tgtttttagt agagacgcag tttcaccatg 4500
ttggccaggc tggtctcgag ctcctgacct cacgtcatcc acccgccatg acctcctata 4560
gtgctgggat catagatgtg agccaccgca cccagccgaa aaataaattt cttaatatga 4620
gttgtgctga aaggtttgaa agccactggc ttaactgagt ggttctcaaa cttcatcatg 4680
tatcataatc acctgaaggg cctattaaaa cacagaatac tagactctat ccccagagtt 4740
tctgattcaa taggtctggg tagggcctaa aattttgcat gtctaacaag cttccaggag 4800
gtacaactac tcctaattcc ataccacatt ggaaaaccac tggccgaatt attcctatct 4860
tataggtggg gaagctgaga ttcagagaga gaatgaaagt cacccagggt tcacagctgg 4920
tgtaattaag agctaggatt cagacacaac gtgccaccaa aagagtaaac tatgctatgc 4980
ccactttggt tttagagata agctatcttc aatgagtgaa tcagcaataa ttcctctctc 5040
tatgtttcat gtatgctttt gtatttcatc tccaagtcat atataaactg cctgagatag 5100
aagaagcact ggagcttaca aggccaaagc tcataatatt tggatgagga cctaacaccc 5160
agaatggggt agtgatgacc aggtttgagt agcacaggtt tgaggaagag catccttagc 5220
ctccatcagt ctttgagaga ctcccaacct cactctgtct actccttctt ttgttctcct 5280
ctctatagac acctgagtct tagttggagt cttgatacgt gcgtgctagg gttcagaaag 5340
atcctgaagc caagagaaag cctcactgcc cagaaatttt ccctatatca gctctgtttc 5400
caggtgggcc tctacagagt ccaaaatttg gcactttctg ggctgaggta ttcagtatag 5460

tattcagagg atacttcttt tcttcgatag taactgagct gaaagagcaa tagctatggg 5520
cttagacaga gctgggtcaa atcctgtgtc cattactatg tgattctgag ccatttacca 5580
aattattttc caggtctgga tttgttgtct tttatataag actccaaata tctataactt 5640
catagaaata ttttgaggat taataatgca aataaagcag catggtgtta gcttggtatt 5700
taatgggtcc taaataagta atagagatca tgttcttcat ccactcacaa atatttatca 5760
agcacctact atgtttcagg cattgtgccg tgtctaagaa aaatacaaag ttgccttcga 5820
gcagctcaca atctattgct aaactggcct acccgtctcc actctaatcc catgcctgtg 5880
tcagacatgg ttaatcaatt gtggtatcat ttcacactga gctcaatgtg caactttagc 5940
ctccttctcc atacagccat tcagacagca ctgccaggca gatggagtga gcacagactc 6000
ctaacttctt tgcttacctt agtttggtga gagaaaccca gaaatggaca attactttgg 6060
tgagtggcca atgccacgat aagggaaagc agaggtcccc aggagctgtg tgagggctgc 6120
taaattttct ggagagggtg tggtgtctaa ccagaaaaag ctgagtctgg aaggacaaat 6180
agtagttaat tatgttcaga ggcgagctgg tgatgaggga gaagggcaat ccaggcaggg 6240
aatacaaggt tttggaggca agagggaaca gggagcactt gggggaactg gaaggacaga 6300
gtgtgggggc aggggctggg gagagctgac acagtggcag tagggaaggg ctgggctggg 6360
ggtccttcct ggccatgctg aagtcagggg accctcacca tgccccaggg agcaggcagg 6420
tgagtgtctg ccatgctgca cccggaaaga aggttgaact gatacagatt aacctggaga 6480
aaagaagcca ataggcaaga aatgtttctc ctctgtgtga aaatcctcga attcagtgaa 6540
aatatttaag ggataatgga ggcatttctc tatttcatac ggtagtaatc caggtccctc 6600
aggatcaagc gcagaacctg attagtgctg ggccagagca gcccagagca cccgtttcca 6660
tcacgagctg actgcaggct acactctagt gggactgttt gtttaaaaaa atgccatccc 6720
tgctttaaaa acctgtcttt ctccctgagc cagagctctg ttgctattgt gaggggctgg 6780
ggcccggaaa aatagctaag gccacactct tgggggtcat tggctttgaa ttttagctgc 6840
agaagtttct cgttaataag ctttagagtt tcaggaatat gaaagaagtg agaggggata 6900
caggatagat cctgctgatg agagcagaat gaagcaaaca ggcaatatcc ctctaagaga 6960
gggcctggca ttcagacttg tcctactcct gggcaacctt gggcaagtcc cttctcctct 7020
ttaggcctca gttttctcat ctgcagaatg gagaagtggg aatcacacta tccaaatttc 7080
ctttcatcta taatattcct tgtttagaca gtggttgccc aggagatgtg gttcataggg 7140
atttcaatca gcaaatttgt caggctctgt ctgttctcat ctgtcagagg gatgtgcctt 7200
gttaacatgc cttgttcaaa tttgctgctg tgattggaga caaactcgtg caaaaaggtt 7260
aggccttcct ggcaggccca cacttgaaaa tggactgcat ccctacagtt tgcatttagg 7320
acttttgtgt tagactcaga atgcactttt cagggagcat ttaatttgaa gataactttc 7380
atggccaatg cacagaaacc ctgtttagtc tttaatggaa ttaaaataat attagcttta 7440
tagaaccaag cttataatat tactattttt acgggctagc ctggagttgg gctcttttgt 7500
gagctagcct ggaatagcac tgttttgaag gggctcctat gcagtagttc tgtttctatg 7560

gattggtctg agatagtgtt gattattgac aagctaactt aaaatggagc tattttacag 7620
gggagcctgg gaaaccatct gtgaagggtc actttccact ttccttaggt aagagggcac 7680
tgggagtgtc attgctcctg aggttgtgtc ggcgtggatg ggaagggttc agggtgctcc 7740
tgatccgcca aagaaaagac agccccctgc tagaggagag gactacccgg ccatggtggc 7800
catggagatg ttatcatttc cattccaaga ttttcccata tctacaggta gagtcgaatc 7860
acagatctta actttttctt ttataaaata gcttctttgt attaaataac cattatcatc 7920
ccaacagatt ttaattattt taattgttat taaataaatc aaatttaatt agttcagaga 7980
aaacttctgt aatttccaat tcatgatgaa agcctaagta aaaatatctg acctttttgt 8040
gtgttggaaa gttatagaaa caggaactcg agagaaagtc ccctgattag ggcttttgag 8100
aaagtgacca gaccaaattt atagctgaac tgtaaggatg aaagatgata tagcataagg 8160
catagtcatt tgaggatcca ctatgggcca cagtttattc ttattatccc acttgatcct 8220
caaaactatc ccgtaatgta ggcattttta atatcctttt tacagttggg gaaactgagg 8280
tttagctaac cagtagcgga ggcacagttc aaattcagct gtctgattcc tttgctcttt 8340
ctaatatttt aggattagaa atgatctatg tcctaaggca tgagctcaga agggaatcag 8400
tagagaggaa ttaaccagtt aaagtggtgg atcagagagc accaactgtt tcccacaaag 8460
tgtgacactt tctagagatg taggggtcta gaaatataca tgaatgtgtt tcattaaaat 8520
gcggttcaca gcacagagtt catttagttg aatttcttgt ttccacgaga agagtgatga 8580
cgatgataat gttggtgctg tgtgtgtttg atattttatt taaattttta tagcgatgtg 8640
ccagatggga agacaggcag catttataaa agccttgtct ctgtgttggg tgacaggcca 8700
agggaatggg ttggatattt catttgtctg tattggtggg atttcccctc ctgtgacctc 8760
gatgcggaaa atccatttgc acctgtccac aatcccttat tttctctacc aaatgcccca 8820
atcgtgcctg tccctgagac gtggctggca gctacactct tgggagagat catttctagt 8880
ttgggggtga agcctgcagt ttctctttgc ttttgtttct gaaagtctgt gaacaaggta 8940
ggaggtgtgc aggtgccaaa gccagctgct gctcagagca gatgagcctt tcttggaaat 9000
tgtcccagac gatgaattgc ttgcatgatg cagagagatg ctctagaaaa ttgtccattc 9060
actgagaccg ctcttcgact cttgaatgca ctgacacttg cttattctac agctctacat 9120
agctaaggaa ggagccacac acgagggctc tgaagaagcc acgggatgaa cccagaggag 9180
tctgtcagtt gggacaggtc atggacacat gcaaggagcg gcaagggtgg tgtgcaggga 9240
caggtcactg ggcagatgct ccctgtcccc tgcttcagtt atgcaagtgt tggcactaat 9300
tgttacagag atcttagaaa acacagaaag ttgtaaagaa gaaaataaat tcactacgag 9360
tcttgctaca aaaatgggat ttttaaatgt atttctctaa ttctgagaac tctctctctc 9420
tttatcagcc atgattcact ctttctatct tgttctctct tctctctctc tctacacaca 9480
cacacacaca cacacacaca cacacacaga gagagagaga gagagagaga gagatagata 9540
gagagagaga gatggatatc tatctgtaat ccaaattggg atcacattac atgtacatgt 9600

ataattctct agctcaattt ttcctcttaa cagtagagca tccactttcc cctatgatgt 9660

tacattttct tcagaattaa catttttaat atgtcagata tgatatttaa atttttagaa 9720

aagccataac ttgtctagtc aataaggcat atttcttctg tgagtatacc gtaatttaac 9780

aaatctattg actactgtca gatatttagg ctgtttttag tttcttcaaa ataatattat 9840

tttaatgtac atctttgaac acaaatcttg gtggtaattt ctgattattt ccatagagaa 9900

gatttttaga agtaagttaa aaaccatgaa cttaagacaa gaaaatcact cgtggtgttc 9960

gcaactgact tgctttctgg gtacctttcc attggaggtg aaataacttg ttttatcaca 10020

tcctggatgg acagatgttt ttccatgaga ggccgaacat tgaatttaga gtaagttaaa 10080

ggtaactgat gcccagtgtt gccaaaaaca tccgggaagt gttttgatcc ttgtgaggat 10140

ctgtggacct ctcagatctg gggcgaatca gaatggcatc ttgctgccag aatcagcgtc 10200

ctgtctttgg agcaggttgt ctaagggcag gtgagaaaat actgggtgag agatgaactc 10260

cctgtcctta gtt 10273

<210> 38
<211> 2279
<212> DNA
<213> Homo sapiens

<220>
<221> misc_feature
<222> (1)..(2279)
<223> chromosome 16 nucleotides 5007651 to 5009929

<400> 38
cagaagccgc agcaggattg gggagggcca ttcccccaat ggacaagaga agctggacca 60

cagaaccagc aggtatccat ggtggcatct gctacggccg gctctcctcc catgtggagg 120

tctcggggac ccacgtggaa gcattatcaa ggtcattctt cttgccttcg ggaggactgg 180

aattaaaaat aatccaagga agggattgcc aagcttttgg cttttgaatt tcccctgagc 240

cacaaaggct ggaagtgcct tggcttgggc ttcttgcatc attagttcat taatccattg 300

tgaccaggac agaagcaacc cacaagcagc attactgttg gtgtttgggc acctgggcct 360

tgaatttagg ccaggactca aaccccagcc ttcctttcac tagctgtgta accttgacca 420

ggtgacttaa cctctctgtg cctcagtttc ctcatctgta aagtgaggat agcagtgcct 480

acctcaatag gttgtagtgg gacgaaatga gggagtagct gtaaagcttt cagcacagct 540

cccagaacac agaaagcgct ccattaatgt cagtggtcat tctgattact taatgcttcg 600

catccaaaga ctcgttttct tcctgaggtc gcctatccct gtttttcgtt attgcaccca 660

gagaaaagta ggtgtcaaga attattgccc tctgagttgg gtgtgcagtc tttctctttt 720

agaaactgct tgtgtgggcg gggcgtggtg gctcatgcct ataatcccaa cactttggga 780

ggctgaggcc ggctgatcgc ttgagtgcag gagttcaaga ccagcctggg caacatagtg 840

agaccctgtc tctacaaaaa attaaaaaat gagccaggcg tggtggtgcg tgcctatagt 900

ctcagctgct cgggaggctg aggcagaagg attgcttgga ggtcaaggct ggagtgagcc 960

atgatctcac ccctgtactt catcctgggt gacaaagcaa gaccctgtct caaaaaaaga 1020

agaaagaagc tgcttgtgtt ttctgggtaa gtccacccat ctgagggagt gtattctgag 1080

tggtatttcc cttggcagtt agaaaatgtt ttttcccaga ggttgaagta tggagagttt 1140

gagactcttt ttttctaccc tgcactctct ggaattcccc ctccctgctg taaaatttat 1200

tgtccaatga gaatagtata attgttttca acaaagtctc tttgagccag tgaaacacga 1260

tgggtttttt tgtttgcctg atcacttgct ccaaccagca gcattaaatg ttttccattt 1320

ggataaactt gcatcgtcag cttcctaaaa ccctggaggt ttcccgtggg ctcgttcaag 1380

aactttccag ctgttgttta aaagacttta gagctagctt gttcagcctg tggcctgcag 1440

gctgcacgcg gccccggact gctttgaatg tgctccaaca cgagttcgta aactttctta 1500

aaatactgag gttttttgtg tgtgtgtttt ctttgttttt tcagctcatc agctatcatt 1560

agtgtatttt atgtgtggcc caagacaatt cttcttgttc caatgtggtc cagggaagcc 1620

aaaagattgg acacccttgc tttagagcat ttgcaaccgg agtgatatca cccccaaggg 1680

gctgcatatc tgtttaatgc aggtataatt gtgtatcata tatattgtat acatatgtaa 1740

tggattggat agcatgtccc caaattcatg tccacctaga accttagaat gtgacctcat 1800

ttggaaataa ggtttttgca ggtgtaatta gttaaggttc tcaagattgg ttattgggcc 1860

gtgtgaggga ggatcacaat ttttatatat aaagcagaaa taggccaggc gcagtggctc 1920

acgcctgtaa tcccagcact ttgggaggcc gaggcgggtg gatcacaagg tcaggagatt 1980

gagaccatct ggctaacatg gtgaaacccc gtctctacta aaaaatacaa aaaattagcc 2040

gggcatggtg gcgggcgcct gtagtcctag ctactcggga ggctgaggca ggagaatgcc 2100

gtgaacccgg gaggcggagc ttgcagtgag ccaagattat gtcactgcac tccagcctgg 2160

gtgacagagt gagactccgt ctcaaaacaa caacaacagc aacaacaaca acaggaatgt 2220

tctggaggct ggaagtccgg aatcaccatg gaggtagggc cctgctcctt ctgaaggct 2279

<210> 39
<211> 18015
<212> DNA
<213> Homo sapiens

<220>
<221> misc_feature
<222> (1)..(18015)
<223> chromosome 16 nucleotides 5009929 to 5027943

<400> 39
tgtaggggag gacccttcct tgcctcttcc agtgtctagt ggcacctcgt ggcttgtggt 60

tgcatcactc cagtctctgc ctccatcatc atatggcctt cctctctctc tgtgtctcct 120

cttcttgtag ggatgccagt cattggattt aggacccatc ctaagtccag tataacgtct 180

tgagatcctt aactaattcc acctgcaaag atcttacttc caagcgaggt cacattttaa 240

ggttccggat ggacatgaat tttggggcat gctactcaac ttattatgta tgcatacaat 300

agctaaccag atacacagtt atatctgcat taaacagata tgaaaatccc tggtgcccat 360

ctctcctggg ctgccctgcc ctccccctgg ggttttacca aagtgccaga cctggtgtcc 420
tggagagctg catcccaaaa cagccacagt cctgacgtca gaaggttgtg atgaaccctg 480
tccagggtgc cacctgtcct tcccgtgagc tctcttgggg cacggagagt ggagctggct 540
gccacacaga tcctgggact ctgaaaagcc ttcgtcgttt tggagctggt ggctggggtt 600
gtcatttgtg agagcagagc aggtgtggtg ttttgtgtga tggcacgagg gccatctgtc 660
cacccagctt ctgctcccca gccctgccca gcacagctcg tcaggggaga ggaggcggct 720
tgcacttgcg tgtgttcatg ggcggtgact cttccgtgga tttctctgga actgtgggtg 780
gcgggtttca aaggaacagc cagctgactc gtgggtgcgg cgggtatcga gaaagaacag 840
ccctgtgggg gaagatggag ctccggaggg tgggcgggag ctgggatcag gcctgagctg 900
tggggccctg ctgatgcctg tctcagccac agcgctttcc ttggaggcct cctggggcct 960
ggacccagag cgagcaaaac atgcccaggg ctccctgtgg cccagaggct gcctctggga 1020
tcagttcttg ggcacagacc ccctgggacc tctgcggagc caggttggac gcaagcagct 1080
gcagtgggag ctggggtagg gcctgggagg aagggagccc agggcctcca gggatgtcag 1140
aggctgtggc taccaacttc tccctctcac ttccctgcct cccttagtgc ccagccccca 1200
acaaggcatt ttggaggaat gcggatcccc aaaagtcaca tttttttttg agacagggtc 1260
tcactctgtc acccaggctg gagtgcggtg gcatgatcat ggttcactgt agcctcaacc 1320
tgccgggctc aagcgaccct cccacctcag ccccaagtag ctggaaccac aggtgtgcgc 1380
caccatgcct aattcttgta tttttttgta gcggcggggc catgttgccc aagctggcaa 1440
aaagcacaat ttgaaacctc taagccagga tttttttttt tttccggaca cagactgctt 1500
ggagaatccc atgagaactc aggacacaca cacgaatttc atttgttttg gggccccaaa 1560
gagtccttga aagcccaccc attgacgcca ggatgggaac ctttctgggg tctttccgaa 1620
tactaaagtt ctgtgcagag caccccagaa gctgagacgt gtcaccatgg cgatggccca 1680
gcatgtggaa ctctcagggt tcccagttga gctgcagaga cttcagtgtg cagtccaaga 1740
ccaccgtgcc tgtgcctggg ggtcttgagt cactactgtg ggtgggggag gggaccctta 1800
tgctgctggg atcggagcac atgaatcccc agccggcttg cctgcagttt gatgacttta 1860
gctcttgtat atttgtgtcc ccaccagcac cataggttgg tactggaatc tcagacactt 1920
ctgcctcctg ctccatgtcc tatctaaggt ctgccgtcca aaatctaaat gcctgagtcc 1980
caaaatgtgc ctctcctgta acaatacaag tgtcacacgt ttgggttgcc ggagacccca 2040
aaacccaata caaattgcct caagcaaaaa atgggaattt atgggttcat gtaattcgga 2100
agtccaagaa tgatctggct ggcttcaggt atagctggat ccaggcattc aaatcatgtc 2160
cccccacccc caccccaaca ccctcagtct agtcgttttc cacactggct cacccgctcc 2220
atctcttggt tctgatcagg tcttctccac actggagaag agttggtttc cgttcctcct 2280
ccttgtggcc caccttggcc caccttgtgg ccctccttgt gtctaacctt ggaagttaga 2340
cagtcccagc agagagagtt tcttcttcca gctgcagcag aagttccacg atgggctctg 2400
acgggctgac ccggggcagg tgcctaactc tggaccaatc tctcaggccc acctgcaact 2460

tggggtgaga agacagtgcc acccaaacca cctggatgga gaatggggga gaggtactac 2520
cctgatagca acacgctaga ggctcacagc cagggctcct gggttttggg gtctccggca 2580
acccaaaacg tgtgacactt gtactgttac aggagaggca cattttgggg ctcaggcatt 2640
tagattttgg acggcagacc ttagatagga cgtggagcag gaggcagaag tggccacctc 2700
cccgggcact ctcattacca tggaaggctt caggtcacaa gatgttgcaa ctttcccaca 2760
gctccctgtg gttgccaggg cacattcggt ctctgggtag ggcagactgg cactgccagg 2820
gagtcaaccc ccaggagcgg ccctcaacca atgatgaatg ggtgttggct gataaatacc 2880
ccagcttcct cgcccttgtg tggtgcaacc aagacctgtt ctaccaactg tcccagaggc 2940
ccccagcagg attgagcccc tgttgcctac agtatagact tgctcactcc tgcactctgc 3000
acaggctgcc ttccctccct gtcctctacc agtgcttcct ggggccgcct ccccaataaa 3060
gtacttgctc tcacctcctt gtcttagttt ctgcatctgg ggaatcctag cctaagacag 3120
ggctgttgct agaagagaga ctggatgtcg ggcaggcaaa gaaaatcaat aacacaatga 3180
caaattaaaa agaaattaca ggctgggtgc ggcagctcat acctgtaatc ccagcacttt 3240
gggaggccca ggcgggtgga tcactttagg ccaagggttc gagaccagcc tggccaacat 3300
agtgaaaccc cgcctctact aaaaatacaa aaattagcca ggcatggtgg tgcatgcctg 3360
aaatcccagc tacttgggag gttgaggcat gagaaacgct tgaacccggg aggcagaggt 3420
tgcagtgagc cgaggttgca ccattgcact ccagcctggg gaacaaaatg agactgtctc 3480
aaaaaaaaaa aaaaaaaaaa ttccaggggc cacaggactc acaccgcttg gcatgtttga 3540
gtcacctgac ctgggacccc tctaactttg ctgtgacagg ccctcttcag gtactgctgc 3600
ctctgggcct ggattccagt gcacaggact gtcacttttc caacttagcc gccagcacaa 3660
cagagatgtc ccctcagagg tcatggttct caggccaagg gtcatttcag gatggtcatc 3720
atggccatca tcgtggccgt ggcgtcagac tgagagtgat ccctgtagat tccaggatct 3780
gcagccaggc ttgggaactg ctcttaggaa tacacctgaa tgactctaag gcgcctgact 3840
ttgcagctcc tttttaagag gcagagcccg tttcccttct ccctgaatct gagctggcct 3900
ggtgacttgt gcaggcgccc accagggctg ccagatcttt aaagagaaac cagcaagctg 3960
gaatttttgt caatctctgg actttttgag atggagtctt gctctgttgc ccaggctgga 4020
gtgcagcggt gcgatctcag ttcactgcaa cctccacctt ccgggttcaa gcgattctcc 4080
tgcctcagcc tcctgagtaa ctggattaca ggctcccacc accatgcctg ttttgtattt 4140
ttagtagaga cggggttttg ccatgttggc caggctagtc tcgaactcct gacctcaggt 4200
gatccgccca cctcagcctc ccaaagtgct gggattacag gcgtgagcca ctgcaccccc 4260
gcccttattt attttaaaat ctcttgactt ttaaacatgg gtaactaatt taaattctat 4320
aaaaatactg tgaggaaaaa ctaagccctg gtcattcaaa ttatggtcag gggccaggcg 4380
cagtggctcg aacctgtaat ctcagcactt tgggaggcca tggcaggagg ttgacttgag 4440
gccaggagct tgagaccagc ctgggcaact cagtgacacc ccttctctgc aaaaaaaatt 4500

ttaaaaatta gctgggcatg gtggcatgtg cctatagtcc cagctacttg agagactgag 4560
gtgggagaat cacttgagcc caggaggtca aaattgcagt gagccgagat cgtgccattg 4620
cactccagcc agggcaacag agcgagaccc tgtttctaag aaagacaaga agtgtggtct 4680
ggggacctga agcatcagca tcgtcagaga gcttgttgga aacgcagaat tgctggcctc 4740
accccaggcc caaatctgca ttttaacaag atctcctcgt gacctgtggc atattaaagt 4800
ttgataagca ctggattaaa caagcaggga acaggacttg gctccaggat gccgttgtga 4860
cttctgtttc aggataagcc acacctgcta agccctagca gtgacctaag tccatgattc 4920
agaatccaca caccacctgg tggctccttt tattatatca atttattcaa gtgattaaaa 4980
aacttcttgg cagcaggatg acaaaacagg tctgtaaaca tgatctcatc cccccatccc 5040
ttcttccaag gagcttccta tgacaggaaa tcgaattcta gttggcaaat ccagtgatga 5100
ggtctgtaga aaaggggtcc cgtgtcacag ccaggaaggc agactcgtcc ctttaaccca 5160
ctccagccag gggtgctggc caggatgtgc tgttctgtca tgacgtggtc actgagtctg 5220
gttcgttggc agccccttcc ccaggaggag ggagactctt tggcagtgcg gcagccctcc 5280
cgggggcacg ggcttctcgg cagcagacac aggcaggcca gaagcaggca gctgagcctc 5340
tccagcgagc atggtattgc tagggttgca ggtgccctgg caggtggcca ggtgaggggc 5400
tgaggcacaa gtgctatcag gaacttggct gccccacagg ggctgaggac acccagatgg 5460
tccacgccag tggccttgaa atttcccctg cagaagccag accgtgggga aacaagcttt 5520
cgcgacgtgc caccccgggc agcttgaggc ttcagtcctt gaaggggttg tgggcgcccc 5580
ctggctgctc cttctctgcg gccagaggct ccccgttcat ctcctgcagc gggtggtatg 5640
catagtcccc atgcaggcgc cggttcctct ctgctctgga caggagcaag gacaggtttg 5700
ctgcagtgct gatcagcagg aggaaggcca gcgccagggt gagggctagc caggcggtcc 5760
tgcagacagg agagaagccc caagtggggg actgctgggt gggctcaggg cttgggtagc 5820
actggagggg cttccctcta cccggcatag atagcactaa atcaggtgcc tccagggacc 5880
acacagggaa tgttatgagc aaaatggact aggtggggat ggggcaacat ttaagacatg 5940
tgcagggaac agcagaggca gtcatgacgt acaatgcagg tccgtgctgc cccatctcct 6000
gatttttttt tttttttttt gagacagagt ctcgccctgt catccaggct ggagtgcagt 6060
ggcatgatct cagctcactg caatctccgc ctcctggctt caaacgattc ccctgccttg 6120
gactcccgag tagctgggac tacaggctcg aatcaccacg cctggctaat tttcgtattt 6180
tcagtagaga ccaggtttca ccatgttggc caggctggtc ttgaactcct gacctcaagt 6240
gatccacctg cctcggcctc ccaaagtgct aggattacag gcgtgagcca ccacgcctgg 6300
cctgatattt taagagaagc cattaaaaag gtttttaagg ctgggcgtgg tgctcatgcc 6360
tgcaatccca gcactttgtg aggcttaggc gggcgtatca cttgaggtca ggagttcaag 6420
accagcctgg ccaacatggc gaaatcctgt ctctactaaa aatatgaaaa ttagccgggc 6480
acctgtaatc ccagctactc aggagactga ggcaggagaa ttgcctgaac ccaggaggcg 6540
gaggttgtgg tgagctgaga tcgcaccact gcactccagc ctaggcaaca gagtgagacc 6600

ccgtctcaaa aaaaaaagtt taaaaaaatg tgaatgttac tgatttttag atgctgctga 6660
tatattttaa ctttttaaaa attatgtaag ccaaataaaa cacagctgga tgctgggcat 6720
gggggctggg aggccaaggt gggaggatca cttgagccca ggactgtgca cctcgtctgt 6780
gcaaaatagt gagatcctat ctctaaaaaa cagtaagaaa taaaattagc cgggcatggg 6840
ggcacgtgcc tgtcgtccta gctacttggg aggctgaggc gggaggatcg cagtgagctg 6900
actgtactac agcacttctg cctgggtgac agaatgagga aacctcctgc ccccaccctg 6960
ggatctcatc tagcccagtt tgcattctga gctccagtgg gggatgctga caacagaggc 7020
tgggcactcg aaggccctgg gctctgctcc agggtcccca aagcagacag tggggagagc 7080
tggtcccgct tcctctaagg cagggagctg gcaggggaca agggcagaga tggacctcac 7140
aggggaaggg tgcgggagag aagggggatt cctcccgccc tggccccagc tcccacagaa 7200
gcacctacct ggtgaaaaag gagagttctc ccgccctcag ggtggcttca ggtggctgga 7260
gacactgctt tactgtaaca taccagagac aggctgaagg ggccggagca ggagcgtctg 7320
cccatacccc tccccttgga gggataggta cctctggaga cgctgcagtt gccagtcttg 7380
gggtcacagg gacaatggtg ctcacactta caaggcctct ggcagcccgg cccatgccag 7440
ccaaggggac actctatgga aaggagatgg gaggagggag gagggaggag aaaggttggg 7500
gcctccagtg tcagagcctt tctcgacagg acaggatacc tgcccctgcc catgtgtaca 7560
gagccgggct aaaggtgaag caccccctgc cctccctgga ctagcccctg aacgtgacct 7620
tatttggaag cagaatctct acagaggcac tgcagttagg atctcaagat aagaccgtgc 7680
tggatttgag gtgggccctg gagccagagc ccggggaccc ttgttttagc cgagcccttt 7740
gtggaacggg ccgcagcagc cacaggggag tcacacagtg atgtgcaggt gaggccgggg 7800
cagaagacaa gaggcaagca tttcccagac cagaaggtgg agacagaagc agcagaggag 7860
cagtgggggc tgccgggggc caccgtctcc ccatccgcta gtggcgtggc agctcctacc 7920
ttcactgcag ttggacccgg tccatccggc atcacagcgg cagccggctg ccgagacaag 7980
accggggagg ccaggtgagg gcctagggca aggcagggct gggcagagcc ccctccccag 8040
aacccccact cactctccgt gcacagtccg tgctggctgc agttagaggg gccacagtcc 8100
agctcatcac agccgggacc ccgccagaag tgcccggtgc attggcagtg cccgtccacg 8160
caggtcccgt ggccgtggca gtcaggcggc tggcagcggg gttcgtgcac acacaccacg 8220
gtggacactt ggcggggaca gcgccacatg ttgtcctggc tgtagaggga tgtgatgtgt 8280
gaggagagga cacccccaga cggcccctca actccctcca ggctgccccc aaccacccct 8340
ctctccattc tcacctgtct acctacctac ctacaagatg gcgtctatct atttttgaga 8400
tgaggtattg cgatccttcc acccgagcct cctgagcagc tgtaaccgta aggtgtgcac 8460
catcacgcct gcctaatttt tttttatttt ttcataaaga cagggtctat gttgcccaca 8520
ctggtcttaa actcctgggc ccaagcgaac ctcccacctc ggcctcccaa agcactggga 8580
ttccaggcgt gagcccctgt gcccagccct ctccagtctt ttataggttc ctccgctccc 8640

tacaatccct cccagccctg ggcctttcac atgtggtgcc ctctgcccgg aaggctctcc 8700
cacccccacc cagctaccac ctccttcaga tctcagttct tattctgtca tcctcaggga 8760
ggtctttgct gaccaactga ccctgctcct aggcctgggt agtactggca tcctggggga 8820
gggactgcag caatttctgc tctcttgaac ccccggggcc tggcacatag caggcactca 8880
atattggctg aatgagacag gctggggcag acctggactt cagccctcac gaaggcggct 8940
ctcacgtgct taccagtgat ctgacgggta actggccaag gtcccgttga gcacaaaggt 9000
ggcagagcca cccccatcca ggttgatggc gttgaccacg tcctgtttca gcaggaactc 9060
cgccatttcc cacaggttga tgctgcggca caaagcggcg ctgctcaggc tcagcgccca 9120
ccatcatttc tcatttaact ccttttcctt ccacagtgca gagcatcacg cccacagtgc 9180
aggcacattt ctaagtggcc cccgccccca agcatgtctc atcctagccc ccggaagctg 9240
tgaacacgtg agatcacacc catgcctgtg ctgttacatg gcataggaat ccttaaggga 9300
ggggaaacgg cccggaggat ctgggtgcag ccactggaat cacaggagtg ttcaacacag 9360
agagccttct ctggctggtg acagaatagg aagtgagatt tgaaatacga gggggatgag 9420
agggtggccg gacaactggg ggagaggaca cacatgggaa ggaacctggg cagcttgaag 9480
gagctgaggc cccagatggc ggccagcagg gaaaccaaga cctcagtcac aactgaggag 9540
gcgacgggga ttcttccaac aacctaaaca agaggattct ttccagataa gagaagagcc 9600
ctgcctaacc agcatcttgg ttttgtccat gggacaccct gaacatagaa cattccaagt 9660
tgaccagcct ggccaacatg atgaaaccct gtgtctacta aaaatacaaa aattagccag 9720
gtgcggtgac tcacgcctgt taattccagc actttggaag gccgaggtag atggatcacc 9780
tgaggtcagg agttcgagac cagcctggcc aacatgatga aaccccatct ctactaaaaa 9840
tacaaaaatt agctgggtgt ggtggcgggt gcccgtaatc ccagttacct gggaggctaa 9900
ggtgggagga tcacctgaac ctgagaggta gaggttgcag tgagctgaga ttgcaccact 9960
gcactctggc ctgggcgacg gagtaagacc ctgtctcaaa gaaacaaaca aacaaaagaa 10020
cattccctgc tgagtgttgc taaatttgtg ctggttatgc aaccagagca aaccaaaaca 10080
ctctctttac aggggctaga aatgaggctc agggaggccg agggacttgt ctacggcata 10140
ggtgggtaga caagtcatcg atggctaagc tgggattgga accaagatct tctccctgct 10200
cactttgtga ccatttcctt gtatcctatc tagggttcag gaaaggtgag gcctaaagct 10260
gaagatgatg agttcttgga atcggcaaag atctcagaac cctcatcctg tgggaaggtg 10320
agggggccct gggagggggg acagataggt gtcaacatcc cagaaagcag gtagagtcag 10380
agggtggctg tcattgggtc aacgttcctc ctagcaggac tgagccccgg ccggggggcc 10440
tcagctccca ggactcaccc acgctgctcc gtttggccgt ctgcatgaaa gagcaccagc 10500
tgccctttcc ggtcgtggcc aatggccgtc ctggctgata tcacattcac aaatttgcta 10560
aaggaacctg aaggaaaagc agcctggctg atcaccgccc cttgggaggc ctcctgcttc 10620
ttgcctaact ccacttccca ctggtctgag ctacctggta cctccctggg aagcacagca 10680
gcctccttgc tcgtctccct gcctcccctc tgcacatccc agtctcccca gggcagccgg 10740

ggggtctctt cttacaggta aaccaagcta tgccagcctc caggtgaaga ccctctaatg 10800
gtttcccctg ttcttagaac aaaatccaaa gagccagcat gccctcccca gcctcttcct 10860
cctcctgcct cagctcctgg gtgtcccctc accaccctgc agccactttg cacttcctgt 10920
tccctctggc tgggaactgt gcttttctct agcttgctcc tttgtcagat cacctcctta 10980
gtgaggcctt ccttgaccgc ccactctttt tttttttttg agacaaagag tctcgctgtg 11040
ttgcccgggc tggagtgcag tggcatgatc tcagctcact gcaacctcca cctcccaggt 11100
tcaagcaatt ctcatgcttc agcctcccaa gtagctggga ctacaggcac acaccaccat 11160
gcctggctaa tttttgtatt tttttttttt gtagagatag gtctcactat gttgcccagg 11220
caggtcttga actcctgggc tcaaccgatc ctcccgcctc ggcctcccaa agtgctggga 11280
ttataggtgt tagccaccac acctagctta ctgcccactc taaaatggtc atggtatcct 11340
gtccccatcc cagtattctg catcccagtg gttctcaacc ttgagtgtcg gtcagagtca 11400
ccatggaggc ccgttaaaac atagactctt gggccttgcc tctgattctg caggtctgag 11460
ggtgtggggc tttgaaacaa gctcctgggc cacgccgatg ttgcaggtcc atgaagattt 11520
ttttctcttc acttttctct tcctgtaccc acagctccgg tccccatctc tcccctttcc 11580
cctaacttta tttttgtttc tgagcactta tcaattcttt tcagcatatt ctataactat 11640
tttttgtcct atatattcaa tataagccta tcttcctccc catgaatccc cagtacccag 11700
aatagtgctg ggcacaaagt agctgctcaa tacttgttga aagacttaag aacagctccg 11760
cccagcccac tggtcctggt tctcgagagg gttgttgcca acagcctccc catccaacct 11820
gtctcctgtg tctcgtcaca ctctgtggct tggctctcgt tgatgtagat gcttccatta 11880
cgaatcagcc acacgacccc actcagcagc tgcacaaatg ggttctcagt gtccagcacc 11940
tcctcctcag acaggtacct ggatccgggg aaggtgggaa gctcactcac cagcaggggc 12000
aactgcagat agtcaggctc tttctcccta gccatcccaa cctggctgct agattccccc 12060
tcatgcccca ggaacctcct ggggcctgag tgtagctggg acaacttaac agccctagga 12120
atctccctgt tgtcagagct gaagctggac agggttctga caatacaatc cccacaagcc 12180
tttgcaaaca gtctgtttca tttaacagaa atgaccatcc cccaacagag ctcaaatcca 12240
tctatggccc tcaatcccca ggccataccc agcccaacta ccacttgatc cctgttacgg 12300
cccttcattg gcttccttgt ttctccactg ctctctccca cctggtccca ctcaggatca 12360
gaacaagctt tatcatcaac aaatactttt tttttgagac tcaaataatt tttaagataa 12420
ttttaaataa ttcaaatcat caatcttgcc agggcgtggt ggctcacacc tgtcatccca 12480
gcactttggg aggctgaggt gggtggatca cttgaggtca ggagttcgag accggccagg 12540
ccaatatggt gaacccactc ccccgttccc caccccatcc cgtctctact aaaaatacaa 12600
aaatcagccg ggcgtggtgg cacacgtccg tagtcccagc tactccagag gctgaggcag 12660
gagaatcgct tgaacccggg aggcagaggt tgcagtgagc cggtatcatg ccactgccct 12720
ccatcctggg caacagagca agactctgtc tcaaaaaaaa aaaaatcatc agtcttgatg 12780

agttgatgtt tctgacaccc cactcctttt tcagtccctc atacacacaa gcccccctga 12840
gcctctgagc ctttgtgcgt ggggttcctt tggactgaaa cgctcttcct ccagttctga 12900
ccttggctca cttagtgatt catcttcaag ttcccactca aaggtcacct cggagaggcg 12960
tgttccagat caccctccct aaaatcaaca ctatttacaa cgggttcacc ccgttggttg 13020
ctttgctgca cacacagcaa tcagaatgta tgttagctat ttacccggct tttgttgatt 13080
tttctagtaa tgggggaggg ctgttaaggc aaagactgtg ttgttcacgg ctatctcacc 13140
ggggcgcggc acattgcctg atacaagcaa gtgctcaatg atactggatg atgaataaac 13200
tctgcaagga agcgattcct atccccattc tgcagaggag gaaacagggg ctcagcttgg 13260
ttaagtgact tgaacacgga ggcacggcta caacccaaat ctcaggccct ctgccctcga 13320
cagcacgggg ctccctgcct cctcacccgg tgaccagggt cccgtcgcgg cggatcccga 13380
actgcgcgtt ctgcagcccc ccggagctgc tcacccgccg ctcgtcgctc accacgttcc 13440
ccaggcactc gcccgagttc atgcggaaga agccgccgtt ctgggcgaca cggcagtcgg 13500
ccgcccgcgc cgtctcctcc acggtggcgc gtcgtctcgc cgcgcagccg ccgggtccac 13560
cgggctccag caccgagaag gtgcgcaggg gctcaacggc ccgcgtcagg tggccggcca 13620
ccgcgcggtc cctgaagtgc gacacgaagg tgcgcacggc cagaccgccg gcgccgggag 13680
tcgcgggagg cggaggccaa ctctcgtgct cgcggttgcc ggcgcgcacc cgtgtgcagt 13740
cccgggggag gcgcgcgcgc gcgcgtggat agggcagtag caagtcgtcg tcgcgggagg 13800
ccctgcgggg acgggcggcc gtgagctcag ggggctgtcc tcgtgagctc ggaggacagg 13860
ggggaccccc cgaaccaggc tggcccaggg agctgcactc accccgagtc gaggccgccg 13920
gacgcttccc agaggaagcc gaatagtgca agccggagga gaagccagcg acccgtggag 13980
gtcgccatat tggaccgggg cctcgggtca tgtgggctcg cctcacgtga ctcaagtccc 14040
agggtggccg ctttcagctc ggactgaatt acctgcgctc ttttcggctg gcgggctggc 14100
gggcggtgct ggtgatgtta actttttatc tccaggtaat ctcaaaccta caggaagttg 14160
caaggagagc ataaagaact ctcgtagaac tgttattcat gtttagcagt ggtttaggct 14220
tgcctgattt actcaatcat ttgtactttg tttttttttt ttcctgaacc aatggcaacc 14280
aaattgcaga tgtcatgatc ctttacccca tacctcgcca tgaatctcct aagaacaagg 14340
gcattttctt aaccacaggg caaatatcat taggacaact cccattgaca tttctgtgat 14400
gtcatccaca gctaaccttc aattgtccct ctaatgtctt tttttttctt ttcctttttg 14460
tccgcccctg accctgtaac ccaggagttg cttttagttg tcatgtctct ttgatctctt 14520
catctggaag gctcctcagc cttttgtctt ttactacctt gacatttttg aagacagccc 14580
agctacatga tttgtgtgac tcaatgcaaa atgaaaacgt ggggacactt actaaaaaac 14640
ccagaaaact attaagaatt tcaagatggt cagagcaaat ggttgcagca agccagggac 14700
cttctgagtg agcccatgaa gctggccctg agtatagaag ccagtttatt ttgtagcatg 14760
tacttcaagc ttcctttgag aggattcttc tcttcaggcc tcctgtccca gtgcagtgtc 14820
cagggttggg tggtgtcttg gtgccagtgc agtgcgtggg ccattccagg agccacggca 14880

agtctaattg gcttgatact gttgcggtct tgccagtgca ccttaatata gcagtttctc 14940
cttgtctgaa ctagtcccgg ggttctttgt cctacctcca agaaaactaa ggagcgtgga 15000
cacaagggtg gggttggagc gaacgtttaa taagggaaag aagaaagctc tccaaagtgg 15060
agacgggagt ccgagtggat tgtcgggtta cagctgaatg caaaaagctt tttttttttt 15120
tttttttaag acagagtttt gctcttgtca cccaggctgg agtgcaatgg tgcgatcttg 15180
gctcactgca acctcaacaa cctctgcctc ctgggttcaa atgattctcc tgcctcagcc 15240
tcccgagtag ctgggattac aggcacacgt cactgcgccc tgctaatttt tgtattttgg 15300
gtagagacga aattttacca tgttggccag gctggtttca aactcctgac ctcaggtgat 15360
ccgcctcctt ccgcctccca aagtgctggg attacaggcg tgagccactg tgcctggccg 15420
caaaaaactt ttataggaca ctgctctcct cctggtaact gagtaacttc ttaccagtaa 15480
aactgcctgt gcagctcccc ttatgttatg cagctgtggg tatgtctcta ggcaagtgca 15540
aatcgctgct tctcttgttt gtgtaactgt gggtttgttt tagataaggc cccctcctcc 15600
ctgtgccggt ttccatggag cccactgtgt atatgcctga aaaggagagg aaactttttc 15660
ccgggagcct gctaattatg caaagaacaa agggcttctg cgctggaccc tgtctgctta 15720
tctccgtgca ggtccagcct gagttttttc ccaggttgct ttatttttgc cagtttctat 15780
gacttttcag gcaggccact tctgaggtca gaattttccc caacggattt ttcttttcct 15840
tctccctcac taccacaggt atggggggac acagataggg gccaggccag ccccttttca 15900
tccacagtca gcacttctgg tgcttgatct caggcccctc tttgagccac tgctcctttg 15960
agccagtcca cggtgagggt tagtgaactc agttagagag tgtcccccca aactcatgtc 16020
cttaccagaa cctcagaatg tgaacttgtt tggaaattgg gtctttgcag atgtaattag 16080
ttaagatgag gtcattctgg agtagggtgg gccctgaatc caatctgact ggtgttcttg 16140
taaaagacac aaatacacac agaggaaaga agctcatatg aacacaggca gagattggac 16200
tgatgcagtc ataagtcaaa gaatgcaaag aaggctagca gccaccaaga gctaggagag 16260
aggcgtggga gagactcttg agctttgaca gagaacatgg ccatgctgat gccttgcttt 16320
tggaaactaa tacaattcag ggacctcaaa ggggtcccgg gaacagctct gacgctccta 16380
gttcacttta accatgtaca aaatgactgc tcctcctaga ggcttaaatg accaatatat 16440
tgatgtgaag ggagtatatc tttctgagct ttgcattggg ctgctaaaaa taaccagcag 16500
aggagcccag gcctgacgcc tactccctgc ctccggaagc gctctttttt tctgcctcag 16560
ttaggagttg caggaacagg cagtctttgg caagttctga ctttggtttt ctccctactt 16620
tgaggtttgc tactcctttg ccaagttttt ctaagcacat aacgggtgcc acagaaattc 16680
tgcatttgaa cagagcagcc catatccaca gttctagcca aagctgctaa gaagccaagg 16740
gttttctttt ttgctcttca gcctggaata cttttgtccc ctcttagcat cctcatttga 16800
gttctaccat catttgtgtg ttcaacaggc tgagcacctc gtatgtgctc cgttgcagct 16860
gatccctgcc ttcatggagc ttagcttttc aaggggaaac agacatgaaa cagttatgtg 16920

atgaatgcaa gaagcaaagg gcaggactgc gagagaagga tgttgcaggt gtggcaggga 16980

agcctctcca agccccttta gactgtgatt gaaggaaaga aaagatgcgg ctgtgtgcag 17040

atctgggcca gagcctgaag tgggaaacag cttgggaggt gcagcaggaa gagccacctg 17100

gtgggagagg agggccgtgg gggaggggtt ggaggtggtg agcagggttg aagtgggagg 17160

gccaaggcaa ggccagatcg tacatgcagg gatccaggga aatgagtatg gatttcagcc 17220

aaagtgaaat agtaagccat tggaaggttt ttttttaaac tttattaaga taaaatttac 17280

atactgtaaa attctcctaa gtgtacaatg caatgatttg tagtaaatgc acagcgatat 17340

tccagcatca ccattatcca gttttaggat tttttttatc acccaaaaaa gttttgttat 17400

gtaattctat tattatttag agacaggatc ccattctgtt gcccagactg gagtacagtg 17460

gtgcaatcat cgctcactgc agcctcgact tcttgggctc aagcagtcct cccacctcag 17520

cctcctgagt agctgggact acaggtgtgt actaccacac caggctagtt tatttattta 17580

tttttagatg gagtctcact ctgttgccta agctcgagtg tagattgaaa tggcctgatc 17640

ttggctcact gcaacctcag cctcccaggt tcaagcgatt ctcctgcctc agcctcctga 17700

gtagctggga ttaccggggt gtgccaacac gcccggctaa tttttgtatt tttaatagac 17760

acagggtttc accatattgg ccaggctggt cttgaactcc tgacctcaag tgatccaccc 17820

ccgttggcct cccaaagtgt taggattata ggcgtgagcc actgtgcccg gcccgaaagt 17880

catttttgta aaactcttag gaaatggaaa cgtcaaagga tcaatttctt ctgtccgtgc 17940

tgtgtatagt atctgttgtg taaagggctg ggagggcatt aacagcatcc cagaaactgg 18000

cgttgaggtt ctgag 18015

<210> 40
<211> 2818
<212> DNA
<213> Homo sapiens


<220>
<221> misc_feature
<222> (1)..(2818)
<223> chromosome 16 nucleotides 77369103 to 77371920

<400> 40
ataagtacct attatgtgcc agatactagg ataggcactt tgcatagaaa ctcacatttc 60

gactgggcct ggtggctcac gcctgtaatc ccagcacttt gggaggccgg ggcaggcaga 120

tcatgaggtc aagagatcaa gagcatcccg gccagcatgg tgaaaccctg tctgtacaaa 180

aaatataaaa atgagctggg tgtggtggca catacctgta gttccaactg ctcagaatgc 240

taaggtagga gaatcgcttg aacccaggag gtggaagttg tagtgagcca agatcgcatc 300

actgcactcc agcctgggtg acagagtgag actccatctg aaaaaaaaaa aaaaaaaaaa 360

gacatttaca ttttatcttt actgtagcta tggctgccta tcccaaggat gggaagtctg 420

aagttcaaac tggtgatgat ccccacccca agtcacacag ttagctttat agttaaatca 480

gggtgtgcct gatttcctag ccataccagg cagcctattt cttgaataac ctcccatccc 540

ctgtgtctcc tttgtggagc tcttggcaat actctggtga ttggtaacca ctgatacgcc 600
gccagcagtc cacgcttggg tggaaggcag gtttcacgta actgatttac cacttggtaa 660
aggaacgagg tgacagcaag accacaaact aataaatatg ccggtattga cataatgtcg 720
gacctcagta tgattgatgc atgtgttgtt gactgggatg tcacatgttc aggctgtaat 780
cctggtctca gtatgactgt gaggacgggg ccaagtcatt tcacaatttg ggtgagtgtt 840
ccaagcttct cagaagagcg acgcaataga aattcaaggt atgaaattca aggtgtggtc 900
tcagagtcat tttatcttac agcttctctg ctgtgttttc tcagtatgtg aaatgggaac 960
atcacttacc atctactcac ctcctgtttg agttcaaggg aaggtttgca gattggcctg 1020
aaggaagact tctcttgaaa aactcaaacc aaagagcaca ccgccgtccc caaccctgag 1080
ccatttctgt ccttttcatg gaaagggaac tgtgttcgcc agctcgtaaa gccccggcgt 1140
ctcctcctag aagaaacacc cacattgacc cagcagtgtt cattttccca taaggtggtg 1200
ctggataggg ataggatgtc gccccttgtt caccccttgg caataatctg tttgattcac 1260
tccaaatgct gcttctgatc tccatctatt tcaggggatg accagtctga ataaaaccaa 1320
tgagcttaat ctacttgtgg aatgttatag ccggaaggga ttcatagaga tcactctatc 1380
gcctaaaatt ctctcttcta catatctcag tgttggaaaa cataaatgtg actccaactt 1440
gtcttttttg ttgtttgttt ttgagacagg gtctttgttt cccaggctgg agtgcagtgg 1500
catgatcttg gctcagtaca acatctgcct ccctggctca agggatcctc ccatctcagc 1560
ctcctgagta gctgggacta caggtgcatg ccaccacacc tggctaactt ttatttattt 1620
ttagtagaga ggcagttttg ctgtgttgcc cagactggtc ttgaactctt gagctcaaga 1680
aatccttcca cctcagcttg acattacagg taagagccac cgtgtccggc ccccctttct 1740
tcttttttaa agtggggtat taaggctcag aaaagagtgt ttctgccact gcagaggatt 1800
gctgacatta ttagttgctc actctgtcca aggcattgtg ctcagctctt tgtacagatt 1860
gtttaaccct cacaagtcta tgaatagagt atattaccca cttcatttta agtaggaaat 1920
tgaagctcag ggaatcgtag cagagggccc cagttcacac tgctgatcag tgatgcgact 1980
gggatcacca cagcccatct ggttcagaag cttgttctcc taaccaggat gtgagagaca 2040
ttcttgtcct aggcagcacg ttggtggcag aggtgggatc tagactgagg ctctcattct 2100
gtaccctctc tggccccaag gaaggaaagg ggaaggagaa gggcctttgg gactgggtgg 2160
atgtaacact ttctgccacc tctactcttc ctgctataga ttaaaacaga agctctgaat 2220
tgcagccaca aatcaataat gaaaagctac cagaccaagc cctttcttgt ttagtgttgt 2280
gtcttctgtg tcaacatctc tggaacatag tcaatggcag tacccagtag aagccatatg 2340
gatacttttt ctcaagtgtt gaaataaatt aatggtgtgg ccatggttct cgtacttcag 2400
gtccctgtga gcccctgggg tcctacacaa cttggggtaa tgctatggtc acctgcatcc 2460
cctcctctcc agccctcacc tggttttctc ttctccttcc aagaaaaaac acccatacca 2520
ttcttaacat ctctggaagc tgggggtcag gagagggaga tccataaagt tcttgtcttc 2580
cccgactcaa gaagcttaag ggtacattca ctcactcatg aattgactgg tttattcatt 2640

cactggttga ttaattcatt cattcactgg ttaattaatt aattcatgtc atcctttgga 2700

tgtcttgcag agctgagtgc tgaaataaaa tgataaacaa gtcaaaaaca aaaaggcctc 2760

tgacttaaca ggacttccgt gatgcggggg aagaagacaa taggcaagta agcaagta 2818

<210> 41
<211> 22611
<212> DNA
<213> Homo sapiens

<220>
<221> misc_feature
<222> (1)..(22611)
<223> chromosome 19 nucleotides 41997678 to 42020288

<400> 41
gactactttc caaccttgct ctctcccacc tatagtttgt attttaattt ttcttaagag 60

acaggggttt tgctatgttg cccaggttgg acttgaactc ctatgctcga gggatcctcc 120

taccatgtcc tcccaagtag ctgggattac aggcgtgcac caccacaccc tgctcccacc 180

tattatttca tcattttgat taggtcttgg cttatccttc cactgttttt gaaagtataa 240

acatagctgg gtgcggtggt tcacgcctgt aatcccagca ctttgggagg ctgaggcggg 300

cagatcacga ggtcaagaga tcgagatcat cctggccaac atggggaaac cccatctcta 360

ctaaaaatac aaaaactagc tgggcatggt ggcgcatgcc tgtagtccca gcctctcggg 420

aggctgaggc aggagaatct cttgaacccg ggaggcggag gttgcagtga gccaagattg 480

tgcaagcgca ctccagcctg gcaacagagt gaaactccat ctcagtgtac ttgtcacctt 540

aaaaaagatc tagtcaacat gaatgaaaat ggcttattac aatacagtta cataacaaaa 600

aattattttt cacacaaatt gtgaaattcc atagatgaca ataatttagg agggaactac 660

ctgttaaagg tgtgacttac aaaatgtgct aaatatgtgt acatatagtc accatttctt 720

aactcatgta acactaaaag ataccaagaa cttcacttag aagaaattga taaagtaact 780

tttcatcatt cagaattacc catccaggct ggtcacggtg gctcacacct gtaatcctgg 840

cactttggga ggctgaggtg ggcggatcac ctgaggtcgg gagctcgaga ccagcctggc 900

caacatggtg aaaccccatc tctattgaaa atacaaaaat tagccaggca tggtggaggg 960

tgcctgtaat cccaggtact cgggaggctg atgcaggaga atcatttgaa cctggcaggc 1020

agagattgta atgagccaag atcaccccac tgcactccag cctgggcaac tagagtgaga 1080

ctccacctca aaaataaaaa ttatccatcc tgctcagagc ttaaacatta taagcaaata 1140

tctactatat tattctttga ttaaatattg ggttcatcta tgtaagtttt tttcatgtat 1200

cttcatgagt gaaatttttc tggaatttga tgttcaagag ctatcccatc tacttttatt 1260

ataaaaatgt tagtctttta gcatgccctg gcaaattttc cataggattt tctattcttc 1320

agggtgtagc ctgtacagtt ttctagtctt acatgtttgt tttgttttgt tttagacagg 1380

gtcttgctcc atcaccgact gcagtgcagt ggggtgatca cagcttactg cagcctcaaa 1440

ctcctgggcc caagcagtcc tctcacctca gcctcctgag tagctgggac cagaggcatg 1500

tgccaccaca cctggctaat ttattttttg cagagatggg ggtctcccta tgttgtccag 1560
gcaagtctgg aactcctggt tcaagtgata ctcctgtctc aacctcccag agtgtttgga 1620
ttacaggcat gagccactgt gctcaacctc atcttacatg atactttttt ctattttttg 1680
aactttttgg gttgtttttg aaacttaatc atatttaata atgttttcct agaaaaaaac 1740
cgtacacata taaaaatcat gtctaacaga gttcacatac ttaatactaa aaaatatctc 1800
caaacatata gttatattcc atttctcctt ataatatagt agaggcactg acaaagtcta 1860
atgaataata gttttcttca aaggactaga ttttgatgaa aaacctgtat ggttttttat 1920
tccatgattt cagcttttca ttaagccttt ccattcattc atttggggta tttgttctta 1980
ttctaccttg ctgtgtaatc attatttaac tcaataaata tttgaggccc tacaacatta 2040
taggcactgt cctaagtgct gtggatacag tgctgtggat acagtgatga agagaaacaa 2100
actgccctca gtcttcttgg ttttatacac agagcaattt ctattgataa ctgttttagt 2160
tatttcacag gatttaaaat ataggtctca ttatcattct aaatactctg taatttcact 2220
ttttatttgc tctttaatct aagaatggtt tatatatagt agtaaatttc aaagaggtaa 2280
gaatccatat ggctatccat ttgctagttt ctgagttata actgtggtaa agaattatat 2340
aacacttggc tttagaaatt tgttagagag aagttatttc tgaatgtctt ttaaaaaact 2400
gctgaacact caacaacccc acttttacta aagtcataag gctttctttt ataggttttt 2460
ttgatgtagc atgtgaaatt aatattcaaa gctttcttca ttgtatttta ctgatttttt 2520
ttcagaatta acattctgat gttcagtaag acataaatag agatgacata tttgtctata 2580
atcattatag tcttaaaagt cctcctctca taaaatggct taataccaat acaaatgaca 2640
gtcactaagt catgtaaatc tcaaatcatt caacccttat aatctaatga atacgattaa 2700
acagacatag atttaagata ctttccaaaa aaaaaaaata gaggcaggat gtattgaaac 2760
aagacatgcc aaataaattg tatgacatag ttcaaggctt ttctagatta attacatgat 2820
ttttgtggaa atacaacaga attttgctaa tgatgaaaga tttccaaaat gaattatata 2880
taaattcttg tgggtttttg tttttgagat ggagtcttgt tctatcaccc aggctggagt 2940
gcagtgatgt gatcttggct cactgcaacc tccacctccc aggttcaagg aattctcatg 3000
cctcggcctc ccgagtagct gggactacag gcgtgtgcta ccacgcccgg ctaatttttg 3060
tatatttagt agagacaggg tttcaccatg ttggtcaggc tggtctcgaa cccctgacct 3120
caggtgatcc acccacctct gtttcccaaa gtgctaggat aacaggtgtg agccaccatg 3180
cttggccaaa ttatatataa attctttatg caacatctct gatgagaatg ttttgagaat 3240
gattcaaaaa gaattcatgc tatctcataa aattttaccc tgatattctg caattgataa 3300
ctgatcagta atttctttcc tatattgatt gcatgatgaa ttctctgctg ctgctgctgc 3360
tgctgctgct ggatgtgtgc tctgttaaaa tgccttccaa tattacttac atttgtggtg 3420
ttcctcaaga gaaaaaaata aatgacatca attaatgagt catgaataaa gcccttccta 3480
cacttttata taatatttca caagagtgaa atgaagtgag agcttgaaga aaatgctttc 3540

tcaaaatctt taaattcata ggatttctca aaagtgtaaa tattctggtg ttgagcaaag 3600
tttgactcat gactaaaggt gttcccatag tctgtccatt cacagagatt tgcactatta 3660
tgaatttttt gttcagtaaa atatgaatgg tgactaaaga tgtggctact tttcttgcat 3720
ccataaggtt ctgcatcagt atgaattttc ttatgtcgtg taagctgtga accccagata 3780
aaagatttcc cacattcttt acatacgtaa ggttcctcac cagtatgcat tctctgatgt 3840
cgagtaagtt gtgaacccca gagaaaggct tttccacatt cttcacattc atatggcctc 3900
tttccagtat gaattttctg gtgtcgatta agttctgaac cacgaaaaaa ggtctttcca 3960
cattccttac attcatagtt tctctcacca gtatgaattt tctgatgtcg attaaactct 4020
gagccatgaa gaaaggtctt tccacattcc ttacattcat aggatttcct ctcattgtga 4080
attttctcat gttgagcaag atacgaagcc caagtaaaag tcttcccgca ttgcttacat 4140
tcataaggtt tcctgccagt atgaattcgc tgatgtcgag caaggtgtga gctccaaata 4200
taggctttcc cacatttctc acatttataa ggtttcctac caacatgaac attctgatgt 4260
tgagcaagat ttgaaccacg aataaaggct tttccacatt ccttacactc atgagatttc 4320
tcaccagtat gaattctctg atgctgagtt aggtgtgatc cacgagtaaa agctttccca 4380
cactccttac attcataagg tttttcacca gtgtgaattc tctgatgttt aataagatgt 4440
gatcgctgac taaaggcctt tctacattca ttacattcat agggtttttc accagtatga 4500
attctctgat gtcgagtaag atctgagcca caactaaagg ccttcccaca ttccttacat 4560
tcataagatt tctcaccagt atgaattcgc ttatggacac taagttgtga atgaaatcta 4620
aaggctttcc cacaatcctt acatttaaaa ggtttctcac cggtatgaat tctcttatga 4680
ccagtaagac tcgaacgtaa actaaaagac ttcccacatt ctttacattc atatgttttc 4740
ttaacagtgt gaactgtctg atattgaata acttctgaat caggaagaaa ggtattccca 4800
cattctttat ctccacagaa tttctcactt gtgtgaatta actgatgttg agtatgatct 4860
gaaccagaaa taaaggcttt ccccagttct ttaaattcat tcagtttgtc tcctgtatta 4920
agtctctggt gtagattaaa cactgtatgc tggttaaaag tgggcctttt ttcacaggtg 4980
cgtatcacct gcttgaagca ttcctcttga ttatcttgaa gtgtttgaaa ctgagtgttg 5040
ccttcccagt cacctctaaa acataaacag tcaaggctgt ggtttttaca aattctcatt 5100
atttccaatt gggctatttc tctctcaaaa atgccatttt ttggtaataa cttcttggtc 5160
tgacacctcg actgcatgtc tgaaaaataa gaaggtaaaa acatcatacg gttgtatgta 5220
caaaaagcaa tacaacttct aaaatagata tagaaaatct tgaagtaaag catatgagaa 5280
gtgaatggct tagaaaattc tcaaatatga gcaatatgaa aaaaagggca agaaataagt 5340
agaaatttaa aagaaataaa agttgagtca agtgatgaga aatatgtttt attttgatca 5400
tattcatctg aggagctcaa taaaaaatga ttatccaggt atcacctcaa taacagaata 5460
ataagtcata aaatacctac aagtttccaa gtactatgat gaatgtctta tataattcag 5520
agcttaaaaa taggaatgct gggaaatagc agaatatcag tctaatatga gggaagtaat 5580
ctgatccaga gttaccaaag tactaggatc tcaatagcag ggtatgctgc atttaggtta 5640

aaggagataa ttatgggtga tagttcagac agaaaaatag tttgaaggcc aggtacagtg 5700
attcacacct ttaatcccag cacttggaaa ggctgaggca gatggattgc ttgagcccag 5760
aagtttgaga ccagcctagg caacatggca aaaacccatc tctataaaaa attagccagg 5820
agtggtggtg catgcctgtg gtcctagcta cctggaaggc tgaggtggca gcatcacctg 5880
agcctaggag atgaggctgc agggagccat gatcacacta ccacactcca gcatgggtga 5940
cagagtgaga ccctgtcttt tttttttttt tttttttaat actttgagtt ttagggtaca 6000
tgtgcacaat gtgccagtta gttacatatg tatacatgtg ccatgctggt gtgctgcacc 6060
cattaactcg tcatttagca ttaggtgtat ctcctaatgc tatccctccc ccctcccccc 6120
actgaccctg tcttaacaac aacaaaaaaa tagtttgaga aattttttca agggagaagg 6180
agtctggcac aagctaagaa taagtactcc tctgtgactc ttttttctaa tgtatatatt 6240
taggatcgag tacaattgtt atcagttttt cttgggagac agcaagtact ctagcagtga 6300
ctcaatgaat tgaaaggcag tttagttaca aagtatgagc agtaatgtac tgcaatggaa 6360
agatgacaaa ctgcagtttc agactctagt tctgtttact aaaactactt tttaggtgat 6420
tggaaaatta ctgagcttga ctgagctctt aatttttcat attataaaaa ggaataaaac 6480
atcactttgc agaggtttgt tgcaaggatt gggaataata cgctatttct attaagtgcc 6540
tgacacagga tacgtgttga ataatcagta gctgatgatt attattagag aggaaaagct 6600
ggagaatacg gggaactaga caagtcagag tttgtctttt tatttattta tttatttatt 6660
tattttgaga tggagtctca ctctgtcgcc caggttggag tgcagtggcg caatctcagc 6720
tcactgcaac ctccgcctcc tgggttcaag cgattctcct gcctcagcct cccaagtagc 6780
tgggattaca ggcgcccacc cccatgccca gctcattttt gtatttttag tagagatggg 6840
attttgccat gttgtccagg ctggttttga actcctgacc tcaggtgatg attcacccgc 6900
ctcggccttc taaaatgctg tgattacagg catgagccac cgcgtctggc tgggcttgtc 6960
ttttatgaca ctgtgttctt ctcgttgtac ttcataagcc ctaacctttc ttctgccact 7020
tcctcctcac tgtgaccaga aactgttatc acctgttact ctgattttca actaagcagc 7080
ctattaggtc ttctcttaat gttccctgta tgcagaaatg tgtggccatg ttccaatcac 7140
agaagagaac aaaaagtttc ttaagagaca atatattctt gttaattctt ttaaacctaa 7200
aataagaaaa tccataaatg ttggttaaaa gaatgaacta ataaggagaa ctaataaatg 7260
caggcaatgt acaatcaagg agaagatagg aaaaatgaca aaaattcaaa agggatgggt 7320
aaaataaaaa aatgaggtta aaaaaatttt tttaactgaa tgaagaattt tacgaaaatt 7380
aaattttaaa aaagggagat gagaaggaac ttgatccaaa tcatagataa aataagggcc 7440
actggagaaa agaatggcac ttaccttttt gttacatgag tgaaaaatga gagcacaaaa 7500
atagagtggt atttggagtg aagagaaagt tctttgagaa aaggttttgt ctctatctag 7560
gcctcaggcc cttattaaag tgttttttgt ttgtttgttt gtttgttttg ttttgttttt 7620
gagatggagt ctgactgtac tccagcccag gctagagtgc agtcatgcag tcttggctca 7680

ctgcaacttc tgcctccaag gttcaagcaa ttctcctgcc tcagcctccc gagtagctgg 7740
gactacaggt gtgtgtcacc acactggcta gtttttgtat ttttagtaga gacgaggttt 7800
caccatgttg gccaggctgg tctcgaactc ctgacctcag gtggactgcc tgcctcggcc 7860
tcccaaagtg ctgcaattac aggcgtgagc cacggcaccc agccagtttt tgtttttgtt 7920
tttgttttcc cctgagacag agtctcactc tgtcgaccag actgcagtgc agtggcacga 7980
tctcggctca ctgcaacctc tgcctccctg gttcaacaat tctcctgcct cagcctcccg 8040
agtaggtggg actacagctg cacgccgaca tgcctggcta attttttgta tttttttgtt 8100
tttgtttttg tttttgtttt tttagtagaa atggggtttc accatgttgc ccaggctggt 8160
cttaaactcc tgagctcagg caacccacca aagtgctagg attacaggcg ggagccacca 8220
cacccggccc attttttggt cttaagggga tattccagac caatatttca gaggtgttgc 8280
tgcctcactg tgcagctgag ctgttatcca caacaatggc ctccttgtgc gtgttcagcc 8340
ctcactcacc tgggcaaggt cctcttgtct catccctcaa aatcttccag ggctctttcc 8400
ctttctccaa taaagagaac gcttctggct gataaatgca aaaacctgcc cagaagatga 8460
gaaacagcaa agaaccacat tgtaaagatt ctaaaatttg caactacttc tttggttact 8520
aaggaagaga aacaattaca ggcaggacaa aaataagtat aaactatggg aaagatggaa 8580
gtgcccactg atgataacta ttgccagatt tacaaagcgc aaaccatttt ctaaaaaaca 8640
aaggtcagaa gttaccctgg acatttaaaa gacaataaaa aaaatcacaa tacagaaagc 8700
aggaatttct aaggaaaatg ctgaatctta cccagtgaga ccatgttgct gtagttctcc 8760
aacatcacat ctctatataa atccctctgt tccaggtcca ggcactccca ctcctcctga 8820
gagaaatcta cagccacatc cctgaacatc atcaactgtt ggaaagaata attccaagta 8880
ttaatggtga aatttttaaa aataattata atccctatag atgaaagggg aaaggctgga 8940
aaggggcact gcagggagtg gggcatatat tgggcgaaaa actggagttg gctgcctgtg 9000
gtttcagggg aaatgacatg ggaataaggg tgacaaaagc caagtgtcta catgctcttt 9060
tttttttttt ttttttttga gacaagagtc tcgctctgtc gcccaggctg gagtgcggtg 9120
gtgcaatctc tgatcactgc aagctccgcc tcccgggttc acgccattct cctgcctcag 9180
cctcccaagt agctgggact acaggcgccc gctaccacgc ccggcaaatt ttttgtattt 9240
tttagtagag acggggtttc tccgtgttag tcaggatggt tttgatctcc tgaccttgtg 9300
atccacccgc ctcggcctcc caaagtgctg ggattacagg tgtgagccac gacacccagc 9360
ctacatgctc cttttttttg ttcttttacg agacagagtg ttgctctgtc acccagaccg 9420
gagtgcagtg gtgcaatctc gactcactgc aacctctgcc tcccaggttc aagtgattct 9480
tgtgccccag ccaccctaac agctggggtt acgtgtgtgc accactgcgc ctggctaatt 9540
ttttgtattt ttagtagaga cagggtttcg caatgttggc caggctggtc tcaaattcct 9600
gacctcaagt gatccaccca ccttggcctc ccaaagtctt aatgagccac tgcacccggt 9660
aaaaactcct gttgctcagt taccacatct ttatgatctt caaatcacaa ataccaaaac 9720
atcagaatgg tagcccagaa cagacatcat aggagttttt tgttgatacc actgagaggc 9780

tacaccagcc tggaagaact agccccagat ttattatgtg agaaaagttg gaggttttgt 9840
tagttgtaag cagtctttac tgggcacaaa cagacttgtg catacagagc ctttggccca 9900
ggtaactgtc ctttgcttaa gctactactt ttagttgaat tatctgttag tttaaactga 9960
gagaattctc aagagataca ctccctggct gaatattatt ttctgtggaa aatgtgtaga 10020
atctccagac tttgttaagc agctgacaat cagcaaattc agtgtcataa gtgattactg 10080
tgaaagtcat cagatgagtt ttttgtcgat accactgaga ggctatacca gcctggaata 10140
actagccctg gattgttaat gatattaacc tttcattaat ttcctggaaa atcccagatc 10200
tctttgaact caacctccca tcatatctgc taaggaaagc atcacaaagt gccacatacc 10260
taaatgaatc ccaaccaaca ccaactccca tcttgattaa atgaaacatc ccattctaca 10320
gagtaaataa aagcctttat gtagtatgtc ccctttaatt attctatccc ctcttgttaa 10380
gccacaggtt tgaaatgaaa agtctttact gttcccctta aggaaatgta tttctcttct 10440
tcagtgctct gccagattgg gacacaggag ataactgaaa gccctggtgg atagatttgg 10500
tcatactggt tggagaaaag agggatatct agggaaagca ttggactgtt tcacagtcat 10560
tacttatgac actcaatagt gattcaggta agcagtttaa ggataagcaa taaaaatatt 10620
cacatgattt gatataaatg ggttatattt ttggagagag gcaattccaa ctcacatggg 10680
ccatgactta acattccaag tccaccagtc cttctctgga ttctttcttg gtgaggcagc 10740
gtgctgggaa agcagagcta gaaggaaaga atcacaaggt caggcctttc tcagagctct 10800
caaagggaca aataggaaat gccattttct tccttggagg taagtcagaa ggaacctgaa 10860
gaaatcctgg ccctatccaa aaccagagag attcaacaaa agcaggatga agtttcagtc 10920
gaatcactta tgccaaaatc atgatctgga tcaggatact actagataag tcaattctgg 10980
cttgccctaa attcatacat acctacatta acatttatcc tcaagccgcc tatgaatgtc 11040
agttaattgt ctcatttaaa acatctggaa atgggtcact tgctcaaatt cactattagg 11100
aaatatcata ggtttgctga atgcaaagct tgttactttg aatactgtat cctaccctct 11160
ggatttcaga atttcttatt ttattgagtg aaggatgagc tgggcctttc ccttatagga 11220
gcagcccatg atctctgcct cctgtgtttt cattgacagt gagcatcagg gtggcttttt 11280
catgaaggga acccaaagat ggaaggaagg ctggattggg cgagtagtca gtgttgtgaa 11340
gcacgaactt ttattaaaaa aatatattgg ccgggcgtgg tggctctcgc ctgtaatccc 11400
agcactttgg gaggcgaggc gggcagatca cgaggtcagg agatgaagac catcttggcc 11460
gacatggtga aaccccgtct ctactaaaaa tacaaaaatt agccaggcat ggtggcgcat 11520
ccctgtaatc ccagctattc gggaggctga ggcaggagaa tcgcttgaac ccaggagttg 11580
gaggttgcag tgagccgaga ttgcaccatt gcactccagc ctggcgacac agcgagactc 11640
ggtctcaaaa aaaaaaaatt atatatagat aattttataa ttatctataa ttatatatat 11700
aataaaaatt atatatatat atgcctctct gaatctagct ttgcacttag cacaagggaa 11760
tcagataaat aagttgcaag acctactatc aaggaattca cagtcagaaa agtaagcagc 11820

aattacaata ctgtaggaaa taaaggtaat catggttatg aaaggagcca actagttcta 11880
aggagcccaa accaaaacta gtttttaaca gaaaatttcc tataggaaat ggtacttcag 11940
ctgactcttg aggggctgtt ctgtgtggga aatgcgcgag gggagaagaa aagacacaca 12000
atacctttaa gggtaaacaa cctttatccc acataaatgg caatgcagat ataataagca 12060
aatgatataa taagcaaatt gcaatgggaa gagaagggaa aatatatgta catacataga 12120
tatatatgtg tgttgtgtgt gtatgtgtgt atatatgtgt gtgtgtgtat atatatacac 12180
atacacacac acacacacac acacacatat atatatatat atatatatat atatatatat 12240
atacacttac caaactataa gcattcacca ccagactggg aagcaacagc ctgggctcca 12300
gagtcagaca ctacacccag cagactatgg aggattcacc accagaccgg gaagcaacag 12360
cctgggctcc acagtcggcc acccatccgt gcacaggcga ggagaggtct catgaagctt 12420
tggtgcagtc tgggagctta gctctttttg taacaagttg tttggcatga ggcccagtca 12480
caagggccct tcctgactgg gctcaaggaa cacaaaaagt tcaacctgtt tttgcgattg 12540
tctattgttt ttcaatgact aatgtatagg aatagattga aatagagaat tctccgaaac 12600
agcactggat gaacgcttca aggggctcac acaacctgtt ccaggacttg gtgaccattg 12660
tttgtgtcca tgttcaattg agttcaaatt taatatttaa cttttcctcc acaggggctc 12720
accagagaag acagggcaag gtgaggggga tctcctagca gaggaaacaa catgcgccaa 12780
tcaagcaaag atatttttgt gcattcaagg aacacagaga ggaaagacag tggcagatgg 12840
agacgtataa agcagggatc ctgttatgaa aagccctaaa aaatagggta tagagtttgg 12900
gccatatttc agaggtaata agaaagcatt ctgcattttt aaagtaaggt ttaaaaatgt 12960
ttctaattag agatacagca aagttggtgt gagaggataa agactgatat acataatctg 13020
aggagttcat gaaaattcca tgtaaagcct gtagtatgaa ttggtcctag gagacagaca 13080
gcttcagggg catagagaac tttaagcact tttccaagaa gagtgaaggg ctggagaata 13140
aattgtgaac taaaatatat tttgtgtttg agaacatgaa gtacttgaga gtgattatta 13200
cacggtttta agaaaaaact agttctttta aataaaactt ttttttagct gagcacagtg 13260
gctcacgcct gtaatcccag cactttggga ggccgaggca ggcggatcac ctgaggtcgg 13320
gagttcgaga tcagcctgac caacatggac aaacctcgtc ttaattaaaa atataaaatt 13380
agccgggcat ggtggcacat gcctgtaatc ccagctactc aggaggctga ggcaggagaa 13440
ctgcttgaac ctgggaggcg gaggttgcgg tgagctgaga tcgtgccatt gcactccagc 13500
ctgggcaaca agagcaaaac cccttctcaa aaaaaaaaaa aaggaaaaaa aaatttttag 13560
ggacagtgtc tcaaactgtc agtcatgctg gagtagagtg gtgcaatcac agtttactgt 13620
tgcctcaaac tgctaggctg aagcgatcct tccacctcag tctgctgaat agggtgggac 13680
tacaggaatg caccactgtg cctggctaat ttattttatt tttgtcgaga cggggtccgc 13740
tatgttgccc aggctgttct caaactcctg gcctcaagaa atactttccc accttggcct 13800
cccaaagtgc tggaattaca ggtgtaggcc accatgtcca gccaaaaact aattctaatc 13860
ctgatattcc tcctcctgga aaccatgtag acagaaacaa gaaaatcaaa gtaaatccca 13920

accatcagga aacagaaaat gaacagattc caaattattt ggagatgtga aaagaaaatg 13980
aaagaacaat gtctgtggaa gccacagtga tgctacaaag aaaaacaaat gaggaatatc 14040
cattcccatg gggagtagac catatcctga atgaaagttt ctgtgagcag gaatgatgtc 14100
aaaaggaggt ggtaaaagga ggcccgaaga tgtcaggggg attcaaggtg gcaaaacaca 14160
ttggttgtat ttttttttta aagctttatt gaaatataat tcacatacca tacaattaag 14220
ccatttacag tgaacaattc aatggttctt agtatattca caaatacgtg caaccacagt 14280
caatgttaga atattttcat catttcgaaa agaaactcta accctttggc tatcactcct 14340
tggcttcccc actgccacat gtgccccata cttagcctca aataatgact aatctacttt 14400
ctgactctat ggattttcct attctggact ttcatatgaa tggaataaca tatgtggact 14460
tgttactggc ttctttcacg tagcataagg ttctcaaggt ttatccaaat tgtagcatga 14520
atcagtactt cattccttgc tatgcctgaa taatatctct gcaaatgaat agaccacctt 14580
ttgtctaccc attcgtctat taatggacat ttgggttgtt tccacctttt gacaattact 14640
attatttttg agatgaagtt tcgctcttgt tgcccaggct ggagtgcaat ggcacgatct 14700
cgactcacca caacctccac ctctggggtt caagtgactc tcctgcctca gcctcccgag 14760
tagctgggat tacaggcatg taccactatg cccagctaat tttgtatttt tagtagagac 14820
ggggtttctt catgttggtc aggctagtct caaattcccg acctcaggtg atccgcccgc 14880
ctcggcctcc caaagtgctg ggattacagg cctgagctac catgcccggc tgacaattat 14940
ttttaaaatg ctgcaatgaa cattcatgta aaagtttttg tttgaatatc atctattttg 15000
tagttttcag agtataagtt ttattttcta tatagaaaat catgccatct gcaaatagag 15060
atgctcttag ctgtgttccg ttcttttctc cagtgtcttt tatttctttt acttccccat 15120
ttgccctagc ccaattgcca gtacaatgtt aaacagaagt ggggatcctt gtctggtccc 15180
tgatcttaga gggaagacat ccagtctttt accattaagt gtgatgttgg ctgtgggttt 15240
ttcctagata tcaggttgaa ttcctcttta ttcctagttt gtagaatgtg tatgttttaa 15300
aaaaaatcat aaagggtatt ggctctggtc aaatgcctta tctctatcta ttgagatgat 15360
tgtgtgattt ttgttttttt attctattga tatgatgtat cacattaact gacttttgat 15420
ggtgagccaa ccttgcattt ctgggataaa tcccactcag tcatgttata taattcattt 15480
tagatgttac tggatttggt ttgctactac ttgttgagga tttttacacc catattctta 15540
tgagatatac tttgtctcgt ttcagtatct gggaaatact ggcctcataa aatgagctgg 15600
ggacttggct gtattcctta attatagtag aagacactgg gtgaactcag agctgataat 15660
aggagtcctc ctgaaccagt tgagcttctg aatggtaaag aaggcctggc tattcaaagg 15720
gaaccaaatt ttaaaaaaat tgtcttctac tggggtaaca gaggaagaaa cccttgagca 15780
aaaaaacctt aaaaactacc cttgccccat tcactttttt cacatgaaaa taccagtgac 15840
agctattcca ggaaaatcta ttacatcctc tccaaaatat aaattagata taacatgcgt 15900
aagatagtta gataaaagtt ttaggctggt gcagtggctc acgcctgtaa tcccagcact 15960

ttgggaggcc aaagcgggcg gatcacgagg tcaggagatc gagaccatcc tggctaacat 16020
ggtgaaaccc tgtctctact aaaaatacaa aaaattagcc aggtgtggtg gcatgtgcct 16080
gtagtgccag ctacttggga ggctgaggca ggagaatcac ttgaacctgg gaggcagagg 16140
ttccagtgag ccaagatcac gccactgcac ttcagcctag caacagagca agactccatc 16200
tcaaaacaac aacaacaaca acaacaaaaa acacacaaag ttttaaaagt gtgtcaaaat 16260
cccccatgta caaaaaaact cactaaaaaa atcagagaag atgaaaatca tcacccaaca 16320
tttcagtctg aattaaaaaa aaagtatgat gcaattgcag ctctaaaagc agaccacaag 16380
caaaaatata cgatctcagg agagagaaga aagtaataga tagaccatag atgggagtta 16440
gtggaattca agactgaaag agaaaatagg ctaagtcaca tcagaaatga agaataaact 16500
agctagtgca gcggcatgtg cctgtggtcc cagctacttg ggagactgag tctagaagat 16560
tgcttgagtc caggagttca aggttgaggc cagcctgggc aacacagtga gacccccatc 16620
tctaggcgca taagatctga caaatgccac agaaagtata gtatggaaca cagaatacag 16680
aaattaatga acagaacaaa atttaacaaa gaaacaaaaa tcatttgaag aaatgaaaat 16740
gaaaagagag atccaatatg caaaacgcag agcacctaaa aatgaaatcc aaattaagag 16800
gatatatcta aaacgatggt tgtagctgtg tatctaacac tacagtttta atactcaaga 16860
tgaatttcct gaaatagagt ataaataaac gtgatgaaaa gtgaaaccat attacaggtg 16920
aaaatgaacc aaaatagtag caaaacatga cctaataaaa ttagactaaa actcatgggg 16980
aatcaagata aaaatatcaa atataagaaa aaggaaattc aattgtcatc agacttctgc 17040
agtgctaaat tcagtacccc aaacagtggg gtaaatcttc caatatactt gtgaaaaaaa 17100
caaatgaggg agtcaaaaca tttttatatc cagccaagct gtcattcgag tataaaggat 17160
ataaggagtt ttatgatgca acttaggaaa tactgttgac atgcgctgtt tcagaagaag 17220
ctactagaag acatactcca gccacaaaag aacaatagtg aaagtttcag caaaagaata 17280
gtagtgatgc tgaacatatt taaatgcaaa tctaaataaa atagtaaaag cggaattatg 17340
gtagccagca tcctggatgg ccccaaaggt acccagtatg tggtattcac aacactgtgt 17400
agctgcctcc tatactgaat aggattaact tgtatagcaa taaggtactg cggaaatgat 17460
agagtatgag atagatataa aagacactgt ggcttccagc ttatgcgttc ttgctgtcct 17520
tttgctctgg agaaaaccag ttgacgtatc acagggacac ttaagaaacc ccatggagag 17580
gtctacatgg tgagaaactg aagtctattg ccaatagcca tgtgagagcc acgttggaag 17640
gtgatcctct agtccctgtt atgccttcaa atgatagcag ccctggccaa gagtgtgact 17700
gcaacctcaa gagaccccaa gccagaactt cccagctaag ccactcctga actcctggtc 17760
cacagaaaat ggtgttaaga taataaatat ttatttctgt tttagggtaa tatgttttac 17820
agcaatagat aactaatata gaaatgaagg ttagagaata gaaaatagac tagctcattg 17880
attgcctcat ttgtgataac tgagagataa agggtgtcat ttcaagctga aaatcaaggg 17940
gaagaaattt agaaatgttt aggactacag ccaaggcagg aggactgcct tgaggccagg 18000
agtttgaggc cagattaagc aaaagaatga gaccttctct aaaaaaaaaa aaaaaatgaa 18060

tacaaatgta agtcaaacag ggcaaaatgt caattaaatt tcaacataca agcaaaaagt 18120
aggaaattta aaatgacagc acttagcagg aaaaattcaa attcctataa ataaatctaa 18180
caatatatgt aacatacaat atcttttgtt tgttggtttg tttttgagac agagtctcgc 18240
tctgtcaccc aggctggagt gcagtggcac aatctcggct cactgcaact tccgcctccc 18300
gggttcaagt aattctcctg tctcagactc ccgagtagct gggactacag gcaccagcca 18360
tcatgcccag ctaatttttg tatttttagt agagacagag tttcaccata ttggtcaggc 18420
tggtctcgaa ctcctgacct caggtgatcc acctgcctca gcctcccaaa gtgctgggaa 18480
tacaggcatg agccaccgca cctggcctgt acaatgtctt tatattaatt actacaaata 18540
gttaaagaag acctaaacaa atgaaataat ttatcacatt catgggatga aaggctgata 18600
attttcaagg tgtcagtttc cccaatatag tggtatagga agcttaaaag taaaaggata 18660
gcaaaagata taccatgcaa atcataatca aaataatgct taggtggcta taatatcaag 18720
acaccataga tttcagagca aataaataac atggaagaaa ggatggtctt gaatacatgg 18780
tgccaggtca actgaattta tatgaggaaa acaaatcatg atttcccctc acgacacaaa 18840
aatcaattcc aggtttatat acctacatgt gacagataaa tatagatgaa aatacagaat 18900
atcttcatac cttggggata agcaaagatt tgaataagat acagagtaca aaccataaaa 18960
gaaaaatgat aatgatcaga ctacatttta aaaagcaatt tttgttcatc agaaaagagt 19020
gaatgggctg gtcatggtgg cttatgcctg taattccagc actttggaag gctgaggcgg 19080
gtggatcatc tgaggtcagg agttcgagac cagcctgacc aacatggaga aaccttgtct 19140
cttactaaaa atacaaaatt agccagacat ggtggcgcat gcctgtaatc ccagctactc 19200
aggaggctga ggcatgataa tcacttgaac cggggaggca gagcttgcgg tgagccgaga 19260
tcgcaccatt gcattccagc ctgggcaaca agagcgaaac tccatctcaa aaaaaaaaaa 19320
aaaaaaaaaa aagacagtga gtaaaaatgc aaaccttacg ctaggagacg atatatacaa 19380
tacacaaagc tgacaaggga ctcattcaga ttatacaaag aactcttaag gattaacaat 19440
gaaagacaag tttcccaaca gctaggctaa gaacttgaac tggcacttcc caaaggaaga 19500
tatccaaatg gacaatgcac attaaaacaa aggtattcaa gttccttagt catcagttag 19560
gaaaaccaca atgcaatacc actacatatc caccatattg gcaaacattt tacagtctga 19620
caatgccatg tgttaacaag aagatggatc aattggaact tgcataaact gttgatggga 19680
acttttaact ggttcagcca ttttggaaaa ctgacagtat ctgcttgagg agattcacat 19740
actcttactc accaattcct ctccttggaa taacccaata gaaatgcata catgtaaaca 19800
caaaatgaca agtgcaagaa tgctctcttc ctgattattc atgagaataa cgtattgtct 19860
gtatatatga taaacttcaa taaaaagact aggaagaaat acagcatatt acaaggcaaa 19920
tacaaacaaa aaaataagca gaggccatga tcttgagcat gctaaagcct gcaattcata 19980
ataaacatat aacaatcatt aatatctgta tatcaaagca acaacaactt tcataaagca 20040
aaagttacag gttatataaa ataaaatttt ggctgtgcac ggtggctcac acctgtaatc 20100

ccagcacttt gggaggccaa ggtgggcaga tcacaaggtc aggagttcaa gaccagcctg 20160
gccaatgtgg tgaaaccacg tgtctactaa aaatacaaaa aaaattagcc gggcatgttg 20220
gcgcatgcct gtagtcccag ctactcagga gcctgaggca ggagaattgc ttgaaccaga 20280
agttgcggta agccgagatc ccaccactgc actccagcct gggcgacaga gggagacccc 20340
gtctcaaaaa aaataaaata aaataaaata aaatttcatt aatacacctc tctcaatcca 20400
agacaaatca agtgtaaggc aaaaagtaag tatagactga ccagatgtta gatcacaaat 20460
aaaagttcac taagttccaa agtttaaaaa taacacaata tagcaaaact gtaaattata 20520
ttattggtga agtggggacc tatgtacttt actggaagtc ttcaacataa tcaacagagg 20580
ttgaggtccc cttatgtcac agggatcaag actcctccac aacactgagg ccaagacact 20640
acaccactgt attagttttc tactgccatg aggtagtggc ttaaaataat acaaatctat 20700
tatcatatag ttcagtagaa gtctgagttg ggtcttacta ggctaaaatc aaggtgttgg 20760
tagggctgtg ttcctttctg gaagctccag gggagaattc gttttcttat gttttccagc 20820
ttctagaggc caccaacatt ccttggctta aggcccactt cctctatcta gtaagcaatg 20880
tggcatctct ctgaccattc ttctgcggtc acatctccct ctgactttaa actcagccag 20940
gaaaggttca ctgactttaa ggacccatgt cattatatta gtccaacaag ataatcgccc 21000
ccatttcaag gttcttaact ttatcccctt tgcaaagtcc tttttgccat ataaagtagc 21060
atcgtcctag tttccaggga ttaggaagta aacattggtt gcagggccat cattctgcct 21120
accttgaccc cccttctatc ttaagcttcc ttctttctga gagatactca gtgaaggaaa 21180
taatgtatac agtggtccat ttccaagaca aaatgccttg aattggctta ggtaagcaaa 21240
ctacagaaga aacaggatat actaggcccc tgcttggata gatgatgcct gcttgtcagc 21300
ctcccgcttt ctccctttcc cccacttagt tgccttcacc caaaccaaag aagtttagtc 21360
taagatgaaa gtttgctagc ctgcaaaata gctcattttg tctgctttta tcagcctgcc 21420
cagctgctta ggtcataagt caactattga agagaccttg agctaactag gattgcagtg 21480
cattgtgggc cgcaaccaaa tgcagcaaga caagcctaaa aaaaaaacac acacacacac 21540
cggctgggcg cggtggctca cgcctgtaat cccagcagtt tggaaggccg aggcgggcgg 21600
atcacaaagt caagagatcg agaccatcct ggccgacacg gtgaaacccc atctctacta 21660
aaaatagaaa agttagctgg gcgtggtggc acgcacctgt agtcccagct actcgggagg 21720
ctgaggcagg agaatccact tgaacctggg aggcggaggt tgcagtgagc cgtgattgca 21780
ccactgcact ccagcctggt gacagaacaa gactctgtct cacaaaaatc aaaaaacaaa 21840
acacctgaag cccctaccca acaatcaata ggcaacatcc gggaagattg tgaccccata 21900
gttatcagcc tatgaggaac caggggagga acctgtgcac taggggataa attgcttgtt 21960
gaaactgttg ggtgtgccta tcagacactg atcttgcaag accatcatta aaagtctcac 22020
ttttgctgtt ctccgggtct ctgagtccat tctttgggac gggtaagttt gtttctcaca 22080
ctcaagaaat catctttcac tatcccacgc tatctctcca aaactgactt gaatccaacc 22140
agaataaatg tcaatccaac cttgtattta tatttatcta ctataagaag tcccagaatt 22200

tctagtcaac tgtcttttcg gacattgtat accatcaagt ctcatttgtc cccttttctc 22260

attcttccca actttcaaca cttttcctgt atgccttcat aatggaattc ataattcatt 22320

aacaaccaac tcctctttta tcttcaacca cttccttgaa cacgccctaa cattctgagt 22380

ctaacagaaa ctaggctgta tctggaggac tctgcagccc tctcaacatt tccccagcta 22440

cagcctctta ccagtgggtc tgaaaatgga acagatggcc ggcctccttg ttgttcacta 22500

ccttcagacc tttcttcctc cctccacaaa ctctagtttt gaatttcctg ccatgagatt 22560

gtatataact caccacatct cacttttgtt gacctctacc aaacacaggg t 22611

<210> 42
<211> 10777
<212> DNA
<213> Homo sapiens

<220>
<221> misc_feature
<222> (1)..(10777)
<223> chromosome 20 nucleotides 17424830 to 17435606

<400> 42
cctgggcgtc cagctgcaag gctgttccct ttccaaccat gttgactctg gaacaccatt 60

tccacaaagc cctctgagct atctggaaat aaggatttca aggccctata tgcatgagca 120

tccatattac caccttctct tttctcccca aatgctgcgc ttgttcagaa ctgggagatt 180

tcgatgtgag gtgaggtctt acagaccaaa tgttgaaggc agatgccaca tttaccagcc 240

aagctgtttc tgcctggttt gagggagggg agaatgagag atgactgagc agccactctg 300

cactggacac catgttacac gcgttggcac aaaccctttg attcaatgtg gggaggcggt 360

gggatgtgat ccagtgttca cttactgccg agcggaggga gcaagaagtg aatattgagt 420

tgtacttttt gcccaattaa atgccttgtg ttgcaaaagt tattgctgtc aaaccataaa 480

acaaatataa aaatacaaaa taaatgcctc acattaggga tggcatgccc aaggtagcct 540

agccccctag tcgatttttc tccttataaa cttctcagct cagtcactta gcaagccaga 600

gagggcagag ggtgggaagg agtcctcctt cacataaaag catttaattt ccagatggat 660

ctgaagcaag catgaatgtt aaagtcagga agagagccgc ttggtttttt tcaagacaga 720

aaaaaacaga agtccagtgt tggggagcgt tacctttccc accggatcca gtgctgagag 780

agactccatg gctctgggtg aacaggggaa tgggagtttc aatgaaggca gaggtcagcc 840

tggcagaaag aaccagaaag aacaagccaa acagaggtca ggggcaagag cgactctcgt 900

taatgttgag caaaaatgaa aaaaaagctt tctctagacc aaattgctca cgaattagat 960

atcattttaa acagacagcc atacacacat ttacatactg gatgaggaca taaaggaagt 1020

gatcatttct ttgagctcaa gagtcataaa ctgtgaaaca tacctataaa aaattgtaaa 1080

gtggggattg cttataacct aaaaatgcca cttctccatg agacagtgcc ttttttattt 1140

ttttgccttt tgtttttttg cctttttgat ttttttagtt agagtagtca acccaggctg 1200

aagtatttag ggcaaacaga gtatgatatc tccaacttaa gatatatgta tatatacgtg 1260

tatgtatata tatatacaca cacacacgta tatatacgtg tgtatatata tatacacgta 1320

tatatacgta tatatataca cacacgtatg tatatatgtg tgtatatatg tatatgtgtg 1380

tgtatatgta tatatacgta tatatatacg tgtatatata tacgtatata tacatataca 1440

cacacatata tacatatata catatatcta ctcatatata cgtatataca tatatacata 1500

tacatgtaca cacacacatc atatataaat agagaaaaaa tgataaagca attgagataa 1560

aatataaaca actggtgaat ctggagtatt caggagttcc ttgttctatt cttgcaacat 1620

ttctatcaat ttaaaattag accaaaatca gaacttatag ataaataaaa gaggtatcca 1680

gtcacctttc tgtgaagccc tgtcccaaaa caaaaatgtt tacaaagtca ccgaaatgcc 1740

agctccagac ttattcttgc agatgtgtgc atagtgttca ctgtgacata aatataatgt 1800

ggaatcatgc cagatacacc gaccagcact ggagaaagaa aaataaaatc ctgcttctcc 1860

tgccaagcat tcgagtgctc acagccaaga gagagcaccc agcgcgaagg gaagagctgc 1920

tcctggtggg gacctcagtt gtggggggct tctcttttca ttcagtcgaa acccaggaat 1980

ttagcaagac tgaagtgaag aatcacaaat taattcccaa cccgttctct ggaaatcacc 2040

gtagctatga ataccagcat agctggagag aaagagagaa tttcccagct ttcttaaagt 2100

gagtagggat cgactcacag ctccattatg gagaagacca gacagccaca tagtcacagc 2160

cacagactga ctcagcaagg tgctaagaat tgtgaggatg gcagcaggag catcaccaac 2220

acctctgagt gccctcaagt gccaggctcc gtgccacgta gctcacctgc agcacctcat 2280

taaatactcc tgggcatccc acctgctggg acacttatgt ccccagaggc ctatgagcaa 2340

aacacaggct tctcagtgag gccttttctg accaccctac ttaaaactgc aagcattccc 2400

ccatcacccc tattgccacc accctcctcc ctgcgggaac ttcctcctca gcactggtcg 2460

taccttcatc ctctgtctcc cctactagtt atccaacacc catggggtca ggaattgttc 2520

tgtttccttc cctgacacat gcccactgcc tataactgtg cctggcacac aataggcact 2580

caataaagag ttgtggaagg aataagtggc ctggatgggg agggcacacg ctcacctgtt 2640

gccttcaatc tcgatgatac gatgataccg gtccagctca ttcttcaggg tttgctgggc 2700

gaccgccagc tgccggcagt cgtaagaaga cttctccagg acccgctctg tctcctcaat 2760

ctccttgcgc agtgtctcaa tctgctcgtt ataaagctga atctcatcgt cataacactc 2820

atgggcactt ttaatagctt gttccagagt tgttgtctgt gggcaaggac acgctgtaag 2880

aaaatccatc ccccttcttc tctaagagac agacctcacc aggaaaagga acctggactc 2940

ggtgaatgtt ttatgttgtt tgtgtgtttc tctgtccatt cgagattagc aatgaatcac 3000

tttggaatcc ttaagctaaa aagcaatgtg aaaactgagc tcctgtgtcc ttacacaatt 3060

tttttttttt ttttttttga tagaggatct cactttgtca ccaggctgga atgcaggagt 3120

gcaatctcgg ctcactgcat cctcgacctc ccaggctaaa ggcttccacc tcagcctcct 3180

gagtagctgt gactacaggc aagcaccaac atgctgggct tttttttttt tttttttttt 3240

ttggtagtga tagggtccca cttcgttgcc caggctggtt tcaaactcct ggcttcaagt 3300

gatcctccca cctcagcctc ccaaagtgct ggaattacag gtgcgagtca ccgcacccag 3360
cccttcttgc actaatatca gcaccctctg caattagtcc ataaggaggc ccaccccaaa 3420
ggcagtctga agacaaacct agaaaataaa acgaaaccta ggccaggtgg ctcacacttg 3480
taatcccaga actttgggag gccgaggcta gcagatcact tgaggtcagg agtttgagac 3540
cagcctggcc aacatagtga aaccccatct ctactaaaaa tacaaaaatt agctgggagt 3600
ggtggcacac gccataatcc cagatacttg ggaggctgag gcaggagaat cacttgaacc 3660
tgggaggcag aggctgcagt gagccaagac cacaccattg cactccagcc tgggcaacag 3720
agcaagactc tgtctaaaag aaaaagaaaa aaaacttgct atcggtggaa taatatgtat 3780
aattcaaata aagtacattc ttcagacttt aatgaacaag cataaatctg atagcaaaaa 3840
aataagtaaa aagaagcaca tactaaatag ataagatgct acctttattg aaatactgag 3900
tgggagaaaa gtttccttgt tctcattcag ctttgatttt ctcccgagtt tggatcggca 3960
gagttacatt tatgttttgg tagctaaggt gtatgtgtgt gcccttgtgt acacataaaa 4020
taatgtcaga gaaatacagt acattaatca gagcaagtaa aaattaaggg aggaaaacag 4080
aaagagatag ttttatattc agtgtaagag gttgtgatga tacacaaaga cccacaaatg 4140
ctcagtcatg ggccacataa cagtactgca gtcaacaatg gactgcatat aggatggtgg 4200
tcccataaga gtacaatgga gctggacaat tcctgtcgcc ttgtgacaac cgcagccatc 4260
ctagggccgt ggagccatac gttcactcac atgtctgtga ggacgccggc gtaaacagac 4320
tgtgctgcca gttgcataaa aaaaagtcta gcacatgcaa ttatgtacaa cacataatac 4380
ttgatgatga taataaacaa ctatgttact ggtttatgta tttacgatac tacacttttt 4440
ctcattattt tggcgtgtag tcccactttt aacgagatgt ttctggtaag acagtgacgc 4500
cgcattaccc cagccgcagc ctcctacagc tcacgtttcc cacagctctt gattgcaata 4560
ttttctcttg tgcttgatca agtgttatta aaaaagagtc aagaagctta aaaaaaattt 4620
aaactttata aagtagaggc cgagatatca tgattctttt ccaatctcat tcttgagtag 4680
gaaagaattt gacacgcatg gtcctcaaaa acaaaacaaa acaaacaaaa cctcacttct 4740
acctagcgca gcccttctgg aagccagggt gcactggaac cacctgaggg tgttaagagt 4800
ctgactcagc agggatgggg ggccggagac tccgccttgc tcaccagttc ccacgtgatg 4860
ttgatgctgc cggtccatgg gccacacttc gagtcacaag ggcaagacaa cacatctata 4920
ttcaacttca tgtatgtcca ctgcaggcat ttcctacatc cagacctaaa tgtacctact 4980
ggacaccctg gaagatgatc attgccatca aaacccaatt cttcacttat acactttatg 5040
cccttggatc atgaaattcc ttaagtgaaa atgtagcttt caaaggtgtt cggcaaatgg 5100
ccgggtgtgg tggctcacac ctgtaatccc agcacttaag gaggccaagg cgggcagatc 5160
acttgaggtc aggagttcaa gaccagcctg gccaacatgg tgaaacacca tctctactaa 5220
aaatacaaaa attagctggg cgtggtggct ggtgcctgta atcccagcta ctctggaggc 5280
tgaggcacct gaacccggga ggtggagttt gcagtgagcc gagatcatgc cactgcaccc 5340
cagactgggt gacagagcac cccagactgg gtgacagagc accccagact gggtgacaga 5400

gcgagactct gtcaaaaaaa aaaaaaaaag caggtgttca gccatagatc tgttgcatta 5460
gaagggagga ggaagggaag aaaaggtacg tgtgctaagc actttgggga gatgtctgtc 5520
cagtccacgt tgtccctgtg tctgatgtca gccccaccca tccaaaggca cggtcccagc 5580
aacaagatga ccccactctc ctgtgcagag ttataggacc aggggtgggc ctgacctagg 5640
ctggcctgtc acatgccttc tctcctggga atctggaaag gggtctcaga aatcgcagag 5700
ttggtgggag gttctagaca acagggacac atgaacgggt ctgaggctgc cgctctggag 5760
tggcgagctt tcatgtgcat ggcaaagttg agaaaggtaa ttatttggag aggaaaagaa 5820
atgaagccga aaggcaaaga caaacagaaa ttagggagcc atggcaagag aaggaagaga 5880
tggaaaagga gacggagagc ggctgcctca gctcccagtt cctggttcct gattcctgat 5940
gcacacctgc tctcagattt catgccctgc cttttatacc ctgctccttt ggaaaaaaaa 6000
aaaaatagta cctttccggt ggagaaaact ggcagacagc accttaacca agtgatcaga 6060
gttattgtca cctgtatgag gacacagcca cataacgaaa tactcaggac acagcgtcac 6120
tccatggggg tcttgccaag aatgcagaac ctccatctaa tcctgagaaa acatcaggga 6180
aacccaaaag gagagacatg ccacaaagcc actggtctgg gttcttccaa agcatcacgg 6240
tcatggaaga cagagaaaga ctggggacca tcccagtatg ggggagacaa tcaagacatg 6300
acacctaaat gtgagtagga tctcgaccag gaaaaaacac gcgtggagca actggtgaga 6360
ttttagtgca gtctattaat gcgacaggat cactgttaat ttccaggttt ttatcatcgg 6420
cccgtggttt tgtggaagtt gggtgagggg tatatattgg aagctactgt gctgtttttg 6480
caacttttca aaaatctaaa attacttctg aataaaacaa gagtcccctt cctgtttcag 6540
ctagtgtgat ccagtttctg tttctcccaa gaaatctgga ttcaaacagt cgcctgtaat 6600
agcaaataca agtcagacac actaacgcct ggtgcagcca catctcaccc acaccagagt 6660
gttcagacac tccaggaaac actttcaagg aatatgggga ttgaaaagac ccctttgagc 6720
cccaaatctc aaagaagtgt tggaattaca tggtgaggga agtgtgtggg gctgtagaga 6780
acatagaggg gccatgtgtt gagggatggg gcgggagggc tcatgagaca gactattctc 6840
acttttaatt tttgagacag gcagggtctc actctattgc ccaggctgga gtgcagtgct 6900
gccatcagga ctccctgaag cctccacatc ctgggctcaa gtggtcccca cacctttgtc 6960
tcctcagtag ctgagaccac aggtgaatgc caccatgccc agccaattaa aaaaaaattt 7020
ttttttgtag agacaaggtc taattatgtt gcctaggctg gtcttgaact tctgggctga 7080
agtgatcctc ccaccttggc ctcccaaagt actaggatta caggcgtgag ccatagcacc 7140
tataatctct cacttttcta tgtttgaaat cttccataat aaagagtaaa agaaaaaatt 7200
acatatgaag agaatgtacc ccagatcact gacccaagag ctctggggtg gccgttcctt 7260
ttgggtgtgg gttccccagc ccagagcggg ggtagtggct catgctgata agtcctaagc 7320
tgggtcaact catcaacctc acaacaacct tatcagggaa gggctgtcat tatttccact 7380
ttacagatga ggaaactggg gcacactggc aaagtggctt gcccataaat accacagcag 7440

gattcgaatc tacctgaaca aggtctgaaa cacctcccag aaccaggcca cccttgccct 7500
gcacctcagc ctcctccctg ctgcaggcaa caatatgcaa gcacacacgt gcgtgcacac 7560
acacacactc tgtctccagg gcccctgcaa actccccatt cccacacagc aacaggccaa 7620
caccagcctg ttctctctga agacatggga aagtcagtgt gagccatgca gagacctctc 7680
cttgcaggtt ttcacttagc aaagttttaa actgtaaata gaaccacaaa gcatcacaga 7740
atgggaaatg agagcccagg aaagtcacct gtgatctttc taccctacaa gtggagtgca 7800
atctttttat gtctcaggaa gtggtgattg ttgtttttgt ggtagtggta gggggtggag 7860
ggatggggtg ttttcttggg tgtgcatttg tgtttgtttg aagaaggctg catgttttaa 7920
aggctggaga tcctctgctg gaacactgcg ggaggagctc ctgtcctggg cgcccccaca 7980
gcccccatcc cccacatgac catgaggccc agagccctcc aactccgtac tggtcgcaga 8040
tgctctacgg gattctgctg agtccagtga agtgatgact tggtttataa aagtggactg 8100
atgtctatac cgatgtggtt tttacagctc cataatttaa acctttcatc agaaggaaga 8160
gtgttcagga aaaccagtaa gagctcctgt ctcaggagct caacaggaat ggagatgcct 8220
gtgacgagag ccactgtggc acggcgtcca ctgccaccca gcaccagcct ggccgaggag 8280
aatgagacac ggaccctcct ctagaaggcc gcagtccagc aggggaggca cgcccagggc 8340
atctcagagg aagaaagggg cttccaggag gaggcaggac cggtcctggg aaccgaggaa 8400
ggaaagggac ttccagaaag gagagaagga atgagcaaag gggaggtccc agaaactgca 8460
ggaggggtca ggagacgaag agaagagagg ttggcccagg gttcctccaa cggtggggcc 8520
atgtggcaac ctgaacttta tccggagcca ggtgactgct gtttctgcct ggaccgttag 8580
attgcctggt ttatatctgt ctccccactg ggctgtgagc cgctgagggc acgacacagg 8640
caggagcctg acgcgagccc catctggagg ggtgagctct cacctcagtc cttaggaccc 8700
cagacacctt taggtgtaag gttttttttt tgtttttgtt tttgtttttg ttttgtctta 8760
aaaaaaaaaa aaaggaaact cattacatac tgtcttacac aaactaaacc actagatggt 8820
gctaagtgac tttaatgaga aatgagacat gggcggttcc cactttgcaa ttttaaaaag 8880
tgactttctt tacttccctg tcacagaaac tctcagtcca tgcaatttag ggaactagaa 8940
ttacctgtcc tttcctctcc acctgtccga agtgcttcct ccggggctct tacgggtata 9000
tgtgcactta tttgaaggag aggcttccag cttccagctc caagcccagg cactattctc 9060
ccatcgtccg cccagcgtct cccagactga gcccgcagag ccacgtggag ggcttaaaca 9120
aggcgcaccg gcccgcccca gagctcctgg gtcagtggtc tggggtgcct gtgaatctgc 9180
atttctgaca agttcccagg tggtgccccc gctgctggga cggggacctc accctgacga 9240
cccctgctgt ggagagttcg ggaagcttcg gacgccctaa ccataggcgg aacccgctgc 9300
cacggccctg gtgctagcag acgctctctc tcggcccgcc ctccaccgag ttccggccac 9360
agagcgggcc ctgcccggcg gtgtgcggcc acatcttcct tcagctctag cttagctgct 9420
acattgggaa gccactaaaa cctttaaaac cagcaaaaac ccagccacaa acatttgaaa 9480
tgaggcccaa gcctgggaca cagggggacc aggtggaaca ttcgcccact ccctgggacc 9540

cgcccttacc ctgtggttct cagtgtggct cctggaccag ccgcagcacc atcacaggga 9600

cacctagaca tgcagcatcc cgggccacac cccagaccca ctgagtcagg agctcccggg 9660

cgcacccagc cacgtctggg ttttcatcag agctaaggag gcagaatttt ggcccccgtg 9720

accttttccc cttggtgcta cacccttgaa tatgttacat ggcaaaaagg gactttgcag 9780

atataattaa agttcttaat cagttaccct tttttaagga aaggagttta ttcaagatta 9840

tcccctaaaa acagaagtca gagaaacgtg taagtccaag tcattgagat gagacgcagg 9900

aagagggttc agagttcgct gccggctccg aacgtggagg ggcctgaagc caaggagcct 9960

gggcagccct gacagccact cctggccagc agccggcaaa gaaacagggt cctcactcct 10020

gccaagaact tgaatgagac tggaagcaaa ttctccccta acagcctctg gacaagggcc 10080

caggctggcc aacatctttc tttctacctt ttgagaccca gagcagagaa accgatagga 10140

cccacccaga cttcccacct acacaactgc gatgacacgg gtgctggtgg tgactcatca 10200

aggcagcaac agaaaatgga cacagccgtg tttcatcagc tacatgctat agcttgttac 10260

tgagtgagta taagcacatc tgtcatttta tttaaaatga ttcagtaata ttttgataac 10320

tgttttaata taattagttt cttttttttt tttttggaa acggggtctc attctgttgc 10380

ccaggctgga gtgcagtggc tcagtctcag ctcactgcag cctcaacctc tccaggctca 10440

ggcagtcctc ccatgtcagc ttcctaagta gctggaacca caggcacgca ccaccacgcc 10500

cagctaattt ttgtattttt tgtagaggtg gggtttcgtc atgtggccca ggctggtctc 10560

aaattcctgg actcgagcag tccactcacc ttgccctccc aaagtgctgg cattacaggc 10620

gtgagccacc acacccagcc tggtttcctt taaattatct gtattttatt ttatgccttt 10680

accatcattt ttctgagaag cggtctccat cagaatgcca cgggtgggag tggaggaggc 10740

agcacagaag agatgtttga gaccctgctt tagagac 10777

<210> 43
<211> 30
<212> DNA
<213> Artificial Sequence

<220>
<223> fusion between a first sequence situated from nt 33725146 to nt
 33725160 on human chromosome 1 and a second sequence situated
 from nt 33736836 to nt 33736850 on human chromosome 1

<400> 43
gctaagtttg gcaaacagcc ttcctggcca 30

<210> 44
<211> 30
<212> DNA
<213> Artificial Sequence

<220>
<223> fusion between a first sequence situated from nt 33741237 to nt
 33741251 on human chromosome 1 and a second sequence situated
 from nt 33773974 to nt 33773988 on human chromosome 1

<400> 44

acagggctta ccttcgccct tcgttcagcc                                        30

<210>  45
<211>  30
<212>  DNA
<213>  Artificial Sequence

<220>
<223>  fusion between a first sequence situated from nt 224909363 to
       224909377 on human chromosome 1 and a second sequence situated
       from nt 224922607 to nt 224922621 on human chromosome 1

<400>  45
gaagaggaga gaagatatta atagttgcaa                                        30

<210>  46
<211>  30
<212>  DNA
<213>  Artificial Sequence

<220>
<223>  fusion between a first sequence situated from nt 228390021 to nt
       228390035 on human chromosome 1 and a second sequence situated
       from nt 228392012 to nt 228392026 on human chromosome 1

<400>  46
ccggtgtcca ggaagttgtg gtaaaagatg                                        30

<210>  47
<211>  30
<212>  DNA
<213>  Artificial Sequence

<220>
<223>  fusion between a first sequence situated from nt 68637748 to nt
       68637762 on human chromosome 3 and a second sequence situated
       from nt 68639883 to nt 68639897 on human chromosome 3

<400>  47
actttaagtt ctgggagaag tccaagttaa                                        30

<210>  48
<211>  30
<212>  DNA
<213>  Artificial Sequence

<220>
<223>  fusion between a first sequence situated from nt 6138383 to nt
       6138397 on human chromosome 4 and a second sequence situated from
       nt 6161247 to nt 6161261 on human chromosome 4

<400>  48
cagtaaactc cagacgccca agccacctgc                                        30

<210>  49
<211>  30
<212>  DNA
<213>  Artificial Sequence

<220>
<223>  fusion between a first sequence situated from nt 24575643 to nt
       24575657 on human chromosome 4 and a second sequence situated
       from nt 24595596 to nt 24595610 on human chromosome 4

<400>  49
ttgcttcaag tctgggattt ttgtttcagt                                        30


<210>  50
<211>  30
<212>  DNA
<213>  Artificial Sequence

<220>
<223>  fusion between a first sequence situated from nt 143690849 to nt
       143690863 on human chromosome 4 and a second sequence situated
       from nt 143707785 to nt 143707799 on human chromosome 4

<400>  50
ctttcttcct cattctaaat tggcaaaagt                                        30


<210>  51
<211>  30
<212>  DNA
<213>  Artificial Sequence

<220>
<223>  fusion between a first sequence situated from nt 166750582 to nt
       166750596 on human chromosome 5 and a second sequence situated
       from nt 166784680 to nt 166754694 on human chromosome 5

<400>  51
gcatactctc ttcctctttg cagtttgaaa                                        30


<210>  52
<211>  30
<212>  DNA
<213>  Artificial Sequence

<220>
<223>  fusion between a first sequence situated from nt 170305131 to nt
       170305145 on human chromosome 5 and a second sequence situated
       from nt 170321630 to nt 170321644 on human chromosome 5

<400>  52
agtagcctag tgtaagtgga gctgaaaaag                                        30


<210>  53
<211>  30
<212>  DNA
<213>  Artificial Sequence

<220>
<223>  fusion between a first sequence situated from nt 37873456 to nt
       37873470 on human chromosome 7 and a second sequence situated
       from nt 37891872 to nt 37891886 on human chromosome 7

<400>  53
ggaataaaat gagagcatag ggggaactct                                        30


<210>  54
<211>  30
<212>  DNA
<213>  Artificial Sequence

<220>
<223>  fusion between a first sequence situated from nt 73954169 to nt
       73954183 on human chromosome 10 and a second sequence situated
       from nt 73974381 to nt 73974395 on human chromosome 10

<400>  54
caagtaagac tatttataat tggccagcta                                        30


<210>  55
<211>  30
<212>  DNA
<213>  Artificial Sequence

<220>
<223>  fusion between a first sequence situated from nt 19435620 to nt
       19435634 on human chromosome 11 and a second sequence situated
       from nt 19445908 to nt 19445922 on human chromosome 11

<400>  55
gccagattgt ggaggctgac aagagctgca                                        30


<210>  56
<211>  30
<212>  DNA
<213>  Artificial Sequence

<220>
<223>  fusion between a first sequence situated from nt 5007636 to nt
       5007650 on human chromosome 16 and a second sequence situated
       from nt 5009930 to nt 5009944 on human chromosome 16

<400>  56
gattgacatt ccctggtagg ggaggaccct                                        30


<210>  57
<211>  30
<212>  DNA
<213>  Artificial Sequence

<220>
<223>  fusion between a first sequence situated from nt 5009914 to nt
       5009928 on human chromosome 16 and a second sequence situated
       from nt 5027944 to nt 5027958 on human chromosome 16

<400>  57
gctccttctg aaggcgggca ggagattctg                                        30


<210>  58
<211>  30
<212>  DNA
<213>  Artificial Sequence

<220>
<223>  fusion between a first sequence situated from nt 77369088 to nt
       77369102 on human chromosome 16 and a second sequence situated
       from nt 77371921 to nt 77371935 on human chromosome 16

<400>  58
aaaggtaatc attttaatat acaagataaa                                        30


<210>  59
<211>  30
<212>  DNA
<213>  Artificial Sequence

<220>
<223>  fusion between a first sequence situated from nt 41997663 to nt
       41997677 on human chromosome 19 and a second sequence situated

from nt 42020289 to nt 42020303 on human chromosome 19

<400> 59
ggtaaaatca aaggtatttt ccccctatat 30

<210> 60
<211> 30
<212> DNA
<213> Artificial Sequence

<220>
<223> fusion between a first sequence situated from nt 17424815 to nt
17424829 on human chromosome 20 and a second sequence situated
from nt 17435607 to nt 17435621 on human chromosome 20

<400> 60
gtctgcagac ctggcgaggg cagcagggaa 30

<210> 61
<211> 64615
<212> DNA
<213> Homo sapiens

<220>
<221> misc_feature
<222> (1)..(64615)
<223> chromosome 5 nucleotides 112893833 to 112958447

<400> 61
   aaaaatctgt ccttcccccg acctccccta cccaatttaa gaggctattt ttgaccaaat 60
   actgtctata gtaccagtgt ctatttctaa aatttttctt atgtatcttt aatctcatta 120
   tctcttcctg cactaggatt ctgctttttg ttgtttacag tgtgagcata atattcttct 180
   tcattatatt ttaccttttt tagtggttgc cctagagttt gcagtataca ttcacaacta 240
   aaccaagtcc actttcaaat aacactatac caccttacag gtagtgcgag taccttaaat 300
   aacaaaataa ccctaattcc tccctcctgt cccttgtatc attgctgtca ttcatttcac 360
   ttatatgtaa ccatacataa acatacatgt acacacacat aagatatata cataatcagc 420
   tacattgttg ctattactat tttgaaccaa ttgttatgtg ttagatcaat taagactaca 480
   aaaaataaaa gttaaaaaaa tttttttttt ttacctccat ttattccttc tttagtgttc 540
   tttctttcat tatgaagatt caagtttctg gcctatgtta tttttctttc tctgtaaaga 600
   acttaaaaaa aacatttctt gcaaggctgt agtctactga caacaaattc cctcaatttg 660
   tattcatctg agaaagtctt catttcttct tcatttttga aggatagttt tgcaaggtac 720
   agaattctag gatggtgttt ttcttcccgc cccccaactc cttctctccg ctcttttctt 780
   gcatagtgtc tgaggaaaac ttggatgtaa ttcttacctt ggctcctttg taggtaaggg 840
   ttttcccccc actctggctt cttttaggat ttttcagaag atctctttga ttttctgtag 900
   tttgaaaatg atacacttat cttctctttt aaaatatttg ttggtggtac ttacgcattt 960
   atgcttctgt atgctgggtt ctgacccgca gatcctgacc cagtgatgga tgagagacat 1020
   acactgacac agatattttg cctgtcagtt aggctgaggg gctgggccac tcacagacac 1080
   tgaggaaggt gctgtaaaga gttgtagctg cggtcccatc agccagcgaa gctcacattt 1140
   attcagtata gattaaatga caaaggtctt gagtagacac cactagaggg taattgacat 1200
   tgcctacctc ctgagtagag agcaattatg tggtcctaag accaattaaa ggttggtctt 1260
   aggaccacat gagtaaacaa gctgtttaga taaactcctc tgtgtctacg agctcagctt 1320
   ttaagagaat tcagctgcct tcagccaaac actttatgca acccccaggc attccaagag 1380
   ggtttttgtt tatttcctat aattttacaa tttctctcat catcctgacc gagcccccac 1440
   atctggatga actagtcaag tttaaaaaaa aaaatcctag gggccagcac atgtatgaaa 1500
   agatgttttc ttttactcac agtttaaaaa atgaaaataa aaacagttat attttttaac 1560
   tataagattg gcagaaatta aaaaattgat ggtaccaagt atttgtatgg gtgttggaag 1620
   gcagctcttc tgctttgttg gtaggagtat aaattgtttt ggggagcaat ttgataattt 1680
   ttttactcta aattaaaaat ttatttactc catgactcag cattttcact actagtgatt 1740
   tcactgctac tacatatatc tttctaaaac tctggagata ggtgtttact gcagtgttgt 1800
   ttataatatc aagcaacaac atcaataaca aacctagaaa actggaaatt acttaaatgt 1860
   ttaatcattt gttaaattat agcatatcca tgagatggaa tatgcagctg ttaaaatggg 1920
   ctaggtagat ctggtatgtt atgtgaaaac tgcaaattac ggacattaag tacagtcatc 1980
   actttgcatg gataaaaatc atgcttatat acacaaatat tctgggttgt gtaaaacttt 2040
   ttttttcttg aaagatttca taagtaacag tagttacttt tttggagaag atataagggc 2100
   attggtggca gggagtaggt aagattttga ttttaaatcg ttccaacctg tttgaatttt 2160
   atttataaaa aggtatctac aatcctggat tatagactat ttttgttttt taacgctttt 2220
   cattatttaa aaaatcccag tggaatttta atttttattt tattgacttt atggaataat 2280
   ttggggagaa gttaacattt ttacagcatt gattatcccc atccatgaat atggcatatt 2340
   ttctttatat attcaggtct tcttttatgt ttttccagta gcattttata atcttcatgt 2400
   aaaccttaaa tattcctgga tatatttgat tttagatatt ttagctttag tttctattgt 2460
   gagcaagatc ttttcaaatt acatttttta gctgattact agttacatga atgttgttga 2520
   ttttatgatt atatttggac ttgttataga acttcaattc tagtggtttt tcagttgatt 2580
   cacttagatt ttttgtatta tgtgatcatg tcatcttaca gtttttaaaa cttttgtttc 2640
   agattcctca gttcctttta gatgattgct ttaaaaatgg tatcccctgc cgtatatttt 2700
   gtactcaacc aagacgattg gcagctatcg ctgtggctga aagagttgcc gcagagagac 2760
   gggaaaggat tggtcaaaca attggttatc agatccgatt agaaagcagg taaaaacagt 2820
   tctcatgtat cttctctttt atcatgctta aaaaagacta tgtatatttc actaaaaata 2880
   gaggatgtgt tcgttgaaaa gttaggtgat cattttgctc caagtaagtc tacatcaaga 2940
   tattaaagtc atgtctaaat ctagagtagt attgaatgaa gagtttgaaa aagactttga 3000
   accatagata aaaattccat ttcaggcggg acgcggtggc tcatgcctgt agtcccagta 3060
   ctttgggagg ccgaggcggg tggatcacct gcggtcagga gttcgagacc agcctgacca 3120
   acatggagaa accctgtctc tactaaaaaa aaaaaataca aaattatccg ggcttggtgg 3180
   tgcatgcctg taatcccagc tactcgggag gctgaggcag gagaatcgtt tgaacccgga 3240
   aggcggaggt tgtggtgagc tgagatcgca ccatggcact ccagcctggg caacaagagc 3300
   gaaaccgtct caaaaaaaaa aaaaaattcc gtttcctagt ttcatgatca tagttatttt 3360
   acctctttga gcctcagtgt tcttatctat aaaatgagtc aaagaaaagc atctagagta 3420
   caggtttgat gtgaagttta tagtgaacac tcaataactg ttagtgaatt tattatcagt 3480
   agtagtataa attgagcatc ttaaatccaa aaatctgaaa tccaagatgc taaaaaatct 3540
   aaaacttttt gagtgccaac atgacactca aaggaaatgt tcatcagagg attctggatt 3600
   tacgattttc agatttggga tgctcaactg gaggtattct aaaatctgga aaaatctgaa 3660
   acctaaaacc ctttctgtct cgagcatttt ggataaggaa tactcagtat atgtagtatt 3720
   agtaatgcct tcttgacaag tagtactttt tttattctca gtggtgatta aaaaatctga 3780
   ttccatttgt aacatacttt agctgcaagc aaaaagtatt taaggttttg ttttgttttt 3840
   agagcagctt aggttcttta acaaaataca tcatttaata taaaaggcta ttgtcaggca 3900
   gtctcacata actgcaaaac ctcatagctt tactaataaa ctgtacatac cggtacacta 3960
   ttagtaatat ttattaaaat gtgtacgagg ccagcattta atgtgattat attttagtta 4020
   catgaaaaac ttgcaattgt gcactttgtt ttgctttttt tttaaaaggg tttctccaaa 4080
   gacacttctg acattttgta ctaatggggt attgcttcgt acattgatgg caggagatag 4140
   tacgttgtcg actgtgacac atgttatcgt ggtaagaata ttgctgaatt tgtcatatgt 4200
   aactcagatt gcttaaaatt taaatacata tgccgtttca ggatagtctc aaaaaattta 4260
   aatacataat tacatttttg gaatagggta gaaaagagaa ccaacttatt cttttgaaca 4320
   ggctgtaatg atcaatttat tttttgtcta taattattag aaatattttg tattacaaaa 4380
   ataataaagc cattaggctt tattgaggga aaataaaata agcaggtagt tttaaaaaat 4440
   gtgcttttca tatatgaaaa tgatgcatgc acttagagtt ctcaaaaaaa agggagattt 4500
   cttttatctc tatacttggt tgtttgatcc cttcattaat atcatatatt tcccaatgat 4560
   aggggtatac taataaactc catgattaag ctgctaaatt aactaactgc tgatgaacta 4620
   agttgatgag tttacccctt tgttaaggaa ttctaagctt ttacctagtt tgtgtcaaaa 4680
   aaaattagat taagttttat aaacttggaa taagcagctt ttagaaactt acccaaacca 4740
   aaggatgcac ttcataagct ctgatgctaa aagaaacgta tttacataga ttttttatgt 4800
   attaaaagca cattaaattg gactttactt ataatttttt gtagttttat attttgtggg 4860
   aaaaaagggc aatatagaca gctctctgta tctgtgggtt ccatgactat ggattcaaac 4920
   aactgcaggt tgaaaacatt taaagaaata ttgtgtgtct gttgaacatg tacagacttt 4980
   tcttgttgtt attccctaaa gaatacagtg taactattta cctagcatta cactgtacta 5040
   ggtaatatag gtagtctaga gatgatttaa agtatatgga aggatttgca taggatatgt 5100
   gcaaatacta tgccattttt ttttttaatc agggacttga gcatccatga attttggcat 5160
   gggggaaggc cctggaacca ttcacccaca gatgccaggg gacaacttta ctttgtcatc 5220
   tgcatttcat tgttaatgtc taattatttc tgaaattcct gtaatacaaa attttcatga 5280
   atattgattg gccttgtcca catttaattt gaattagtgt aaaacccagt ggtgccattg 5340
   tttttaaaga cttagtcatc aataatgttt taactgttcc atggttttat ttctgtttac 5400
   tataggatga agtgcatgaa agggatcgat ttagtgattt tttacttaca aagttaagag 5460
   atttgttgca aaagcaccca actttgaaac taattctttc tagtgctgcc ttggatgtaa 5520
   atctctttat aagatatttt ggaagttgtc cagtgatata tagtaagtta aattgtcaga 5580
   tttcttctat aatttttatg aaagaacact tttggacatt atttatccta acagaaactg 5640
   gggaatgttc tgaagtagag gccatctatt accgttccac ctgagatagg cttaattgtc 5700
   acttctttct gttttcaact tctccttttc tatcaacagc tctttggtat tgtgtagtga 5760
   gcctaattct cttaccttaa aaggaaatat gctttatttt cactttgtgt tccttcttaa 5820
   attgtcctat aactatttcc ctttattggt agatatcttt gaaaagtagt ctatactccc 5880
   atgttttact tcacattcac ttctgaaaat gctaaaacaa cttttgccct tgtcctttat 5940
   taaaaacgga ttttgccacc agtctttaaa aaaatgataa attcctcttc aaaaaataca 6000
   aaagttaggc cgggcgtggg ggctcacgcc tgtaatccca gcactttggg aggccaaggc 6060
   aggtggatca cgagttcagg agatggagac catcctggct aacacagtga aaccctgtct 6120
   ctactaaaaa tacaaaaaat tagccaggcg tggtggcacg cacttgcagt cccagctact 6180
   cgggaggcta aggcaggaga atggcgtgaa cccaggaggc ggagcttgca gtgagctgag 6240
   attgtgccac tgcactccag cctgggtgac agagcaagac tccgtctcaa aaaaaataaa 6300
   taaataaaaa tacaaaagtt ctgtttactt cagtgaaata atacaaaaat atatatagaa 6360
   aaagttcaca attgccccta tccttgttcc atgtcctact tttctgattt atggatgtta 6420
   acagtgttat atatattcgt ttttacttaa tgtaagaaca caaatgtatg caaatatata 6480
   cgtatttata acatttacaa aaattggctt tatggattta tgatttatgg atgttaacag 6540
   tgttttatat atatttgttc ttcatcttca caaaaatgta tgcaaatata tacacattta 6600
   taacgtttac aaaaattggt tttataaaca tgagcacatg gctaatacat tatttgcaat 6660
   ttgctttatc atgaacagtt ttccagttta gtaaccctgg atctagttct ttattttaag 6720
   cacctgcata ataaagtatt tcataatatt gatgtactat atttatttta ccatttcttt 6780
   atttttggat attcaagttg gttctagatc ccccgcctcg cccctgccaa caatgttata 6840
   atgaacatcc ttttatgtat atcctgatgt actggatttc tagggataga tttctgaaag 6900
   tgaatttgct aaataagagt atatctattt ttaattttaa cagcttttgc cattttactt 6960
   ttcaaaaggc tgaaatgatt tacatttctg aagtgtataa ggctatttat ttgaattttt 7020
   accggtgtcg tttgacatta acctaattgc caatctgata ttcaaatttt aggcaaaaat 7080
   cttacttaac ttttctgtgg tatgttttat ggttggctct ctctcttttt tttttttttt 7140
   tttgtgaaat gctcagatgc tgtagtttgt ttgataacat tgctgcttgc taaacctcat 7200
   tcttcattat atcccatgct ttctcagtct cccctgtggg cttctcttct tttacatgat 7260
   gcttaaatag tggtattctc tagaattatt cccttgatcc gtttcctttt ttttttttct 7320
   gttcgttccg tcttccttcc ttcctttccc actattccca tcctcttgtt tcctgcccta 7380
   tgctatactc tactaggtat tggagatgca tagttttcct ggaagctctc attcatgccc 7440
   tgggcttcta tttttctttt tatactgatg acttctgaat ctttattttc agttgttctc 7500
   cagagctttg gatttagata tttctagtat gccccaaact gaatacttca tctcttttgc 7560
   aaaacccttt tctcttcttg tgttacctgt cttagttaat catactacta ttgacctaat 7620
   cacctgaatc acaaacctat aagttcttaa ctccttgtaa taatcttggg tgatcaggga 7680
   catcagatct gtacatatgc taatcgaaag ctcatttttc attttacatg cctaaaacca 7740
   aaccccattt cccaccagca aacctgttct tttcaggttt ctttgttacc tcagagaatg 7800
   acattatcat tcagatttgc aagccagaca tcaaaggact atatttgaca ctttattttt 7860
   tttccatatc caatataatg ctgagttcta cggattttac ctcccgccta gctctagaat 7920
   ttgtctgctt cttttttggt tttcactatt attaatctca ttcaggttaa cctgaactat 7980
   tgcagtagct gctacttggc ttgacgtagc attctgctgt ctttaaatcc attctccata 8040
   ttgcagtcag agtggtcttc tttgaagtag aattctgatg atgtcaagca ctttactgag 8100
   cttaaatctt tgaatgactt ttacctcagg ataaagacat aaacccttag taagacttat 8160
   cttttcctct tacctcatct tgaacgatat ccctctccat tcccacactc ccaccatttt 8220
   tcaagtaagt tagcctttta gttccttgaa acacctttag gtccttgaaa ctcctttcta 8280
   gcacagggac tttgcacatg ctgtttttca tcatcattat acacttttta ttcttttcat 8340
   atggctttgt tattttgttt atcttcttgt ttctcatttt cttagtgagg atactttctt 8400
   gatgttgtca aatcccccgt tacatactgt catggtgtct tgtacttgta tctcaggact 8460
   cttcacaact ctatatttac cttcatattc tggatttttg gattgatttc tgtctcttcc 8520
   actagcttgc ctccagggaa caggaaatag ctctgtttcc tcttacaatt tctacttata 8580
   atacaaatga tattgatgcc agacacatga taagtaggta gtaaacacta gaggaaaaaa 8640
   cccaagcaca caaacagttg tctaatagtt gtttgtaagt caaagttata gttaaaggac 8700
   aatattataa gagtcacttg aactatgaaa ttgcttttgg atattatact gagattcatt 8760
   aacttgggat tttcatgttt ttatgtgaat tttctccaag atgcaagttg agtatttaat 8820
   ttttttttat tatttagtac agggaagacc atttgaagta aaagaaatgt ttctggaaga 8880
   tattttaaga acaactggat atacaaacaa agaaatgtta aaatataaaa aggaaaaaca 8940
   gcaaggtaaa ttttttaata aaagaaatat aaaagaaatt actattgata atgactttgg 9000
   agtactccat ccttacattg tggaggtaca aggacacttg aaagtgtttt tctcttgtat 9060
   ataattgtat aaccttatat tggaatattg tcccaaatat tcctttgctt tctggtttgt 9120
   gtccagaagt tataatctgt ggagagaaac tgaaagtggg tgccagagtt gaggaggtgt 9180
   ctatatccaa acattcagcc accttttaag gagatagaag aatatatgct ggctgcccac 9240
   agcatatctc atagggaagg actgggaagt gaatgggagg gagtgaaaaa agtagtatgt 9300
   gattaataac aaactttgat aaaaatgttg agttttccaa atcttgatga tgttacagga 9360
   tttcattgat actttgaatt tagcattatt ttcctgttaa tacgtctctt gtaatgacat 9420
   atttcagctt ttgaattagg gttcagattt ctttttctta attttaactt cattattcat 9480
   catagaagac ttggcttcaa gctttgatac cttgtttatt ctgtatgccc ttggacaaaa 9540
   atctgaactc ctttggattt tccccttgaa aaatgtaatc agcaatgctg atttcattgg 9600
   gaagcactga attactgata tgtaagttgt aaaactccta gatcttgaaa cttaaaaaaa 9660
   aaaattaaaa aaaaattttt ttagagacag gttcttgttc tattactcag gctggagtgc 9720
   attggtgcaa tcatggctca ctgcagcctc gacctcctca tctcaaggga tcctcccacc 9780
   tcagcctctt gagatgtaga ccactgtgcc caactaattt ttaaaatatt ttgtagatac 9840
   gcagtctagt tacatcgccc aggctggtct tgaactcctg gcctcaagca atcctcttgc 9900
   ctcagtgtcc taaagtggaa ctacaggcat gagccaccat gtccagctga aacatttgaa 9960
   gatgaaaatt atatataatt gtatctcaga aaactagcgt ggtggtagaa cttcaacaaa 10020
   tggttttcta caaagtgctg tatgaatatt ggtcattgat ttaaacaata gttataacat 10080
   ggataaaaat actcttaatt ctggacttac ttagaagtaa acttattagt aggacttgga 10140
   acaataggtg aatgcatgaa tgtattagcc cattttcaca ctgctgtaaa gatactacct 10200
   gagacttgga aatttataaa gaaaagaggt ttaattcact cacagttctg catggctggg 10260
   gaggcctcag gaaacttagt catgatggaa ggcaaagggg aagcaaggac cttctttaca 10320
   tggcagcaag agagagaagt gcaagcaagg gaaataccag atgcttataa aaccatcaga 10380
   tctcgtgaga actcactatc atgagaacag catgggggaa actgccccca tgatccaatc 10440
   acctcccacc aggtctttcc ctcaacacct gcgaattata attcaagatg agatgtgggt 10500
   ggagacataa agcctaacca tatcaatgaa gttttcaagc agtttcatgt gtctttttgt 10560
   ttcagcataa cacattttta ttgaatattc ttaatatatt ttttaaaagt aactacagaa 10620
   taagaccaac ttttaagaga taccagattc ccataattta tttaaacaaa ggaatacatt 10680
   tcaaaaagga aatgatttgt ctacatattc tttctttgat aattaattta gaagagaaac 10740
   aacaaaccac acttacagaa tggtactcag ctcaagaaaa tagtttcaag cctgaatctc 10800
   agaggcagag aactgttcta aatgtgactg atgagtatga cttactggat gatggtggtg 10860
   atgctgtctt cagtcagctg gtatgacctc cctggtttcc cactcatatt ttgtgtgaaa 10920
   attgtgtagg ttaaagaaca ttttatgagc ttataatttt tttttttttt tttgagatgg 10980
   agtctggctc tgtggcccag gctggagtgg agtggcgtga tctcggctca ctgcaagctc 11040
   cgcgttccgg gttcacgcca ttctcctgcc tcagcctccc aagtagctgg gactataggc 11100
   gcccgccacc atgtccagct aaatttttgt atttttagta gagacgggat ttcactgtgt 11160
   tagccagggt ggtcttgatc tcctgatctc gtgatccacc cgcctcggcc tcccaaagtg 11220
   ctgggattac aggagtgagc cactgcaccc agccaagaga ttgtaatttt ttactgcata 11280
   attgaatggt aattctcttg gtttatttta gaccacaacc aatttttcag agcaactttc 11340
   tactctttaa aaaaataatt tacccttttt ttgagcatgt gatagtatgt aatgcagata 11400
   tgtttgcagt tttctactag tgtttaaagt tgtaatgtat ccaaatgaac tgtacatgag 11460
   tttttaaaaa tacttttaaa gaatatttaa aaattggtaa taaaaattgt tatttaacat 11520
   ctttatagat atgcacgcta aatatactat atatataatg atatatatcc tatatgttta 11580
   taggattata acacatgcta tataaaaaaa catatagtat agacttattt gggtcatgga 11640
   acagcatttt gaccatgtat atttgtggtt cttaagactg gctgtatatt ttgtcacctg 11700
   aggagctttt aaagccctat ggaatctagc accctaccaa caagagattc tgatgcactt 11760
   gctctggggt gggacctggc atcagtatta aaaagctcca ggtgatactt cccttgatgt 11820
   gcagttggga gagagaacta ctgatctgta tgtgcattta acaacatcat attggaagca 11880
   caattaacat cgtgactaga tcatagtcct ttgttgacaa actcattctc agataaaagt 11940
   tgagtataag gactagatac tgtattgtga gtgataatgt ttgtgatgaa aggagaaatt 12000
   ttttatgtca aagcatttgc tatattatca taggattagt agtcctgatg gccattgtgg 12060
   ccatcttatg ttctttgcaa gcaaatgtta ggtaatttat gataatttat gatttttact 12120
   gttttttgta gactgaaaaa gatgtgaatt gccttgaacc atggttaata aaggaaatgg 12180
   atgcttgcct ttctgatata tggctacata aagatattga tgcctttgct caggtctttc 12240
   atctcatttt aactgaaaat gttagtggta agtttatttt aattttaaaa aattaccctt 12300
   acagttattt atggtggaag tatagtttaa ttcaaaacgt atgtactacc aataattgca 12360
   caaccaaaaa ttttattttt taaaagtatt tattttattt caaatgtaca aatcaggatc 12420
   ttcttgctag cacagcatag tagaaataaa tattgtgtta aatcagattc ttaagctaat 12480
   gtctccattg cagtttgaag agctgtgtga ttacatgaca attatgtgca aaaatgctgt 12540
   tatgtgtatg tgttgtgtat atactcttgt ctttcccttc cttggaaagg atttagatgt 12600
   caagtattta ttatttcacc tgaagccctc agcattataa ttgtaattat tattgattac 12660
   tttcttcttg aaatatttta ctcagttaac ttccagtatt tcatacttgg cttattttct 12720
   tcatatttcc ctggctctaa cttcttggtt ttcttggcaa gatctttttc tttttttctg 12780
   acctgtaaat atgaaatctc ctaggtctca atccttaaga ccttaattct atttatactt 12840
   aatccttaag tggatctcaa atagtcattg ctttaaatat ctattttaaa tgacccctaa 12900
   ctttacatct ccagacctca tctaccccca gatttaaata taccttatca cttgttatct 12960
   ccagttggat gtctgttagg tgaataaaat tggatactcc tcaaacaact tctgatcccc 13020
   tccctacgta ccttcctctc aacctgcact tttcttgtgt ttcccttttc agtaaatggt 13080
   cactatcatt cacctagttg ctaaggcagc aaagcataca gaaagtctgc tgtcatattg 13140
   cacatataat tcattagcaa atcctattta ctttgcattc taaaataaat cctgaatatg 13200
   accacttttc acttccccta ctgtacattt cactccccct actgtacatt tcactcccct 13260
   agtccaagct agcctctctt ctggactatt gcagtagccc gttaaccatt tctgttttta 13320
   tgttactgca gtctgttctc cgcagaaaaa taagactgat cctttcataa aggaaattgg 13380
   atcattccac ttgcttgttc tcaactctcg caggcttcca gttacactgt ggataaaaat 13440
   caaagtcctt tccatgatta ggcctctggc ttcctccttg atcttatttt ttgccacttg 13500
   cctccttgtt cactgtgcct agacacactt atctccttga tattcttcat gtatgccaac 13560
   acattccctt tgggactttg ttttgttgtt tcatatgcct agaatactat ctccactggt 13620
   aatcacatgg cccaatgcct acttatttag gtctctgctt acacattgcc ttctcttttc 13680
   tgaccacctt actaaagttg taccattttc tcacgatatt tccttactct cctttattat 13740
   tctttattgt actttcatta cctgtcatgt atttttattt tttcattact tatttcatct 13800
   actagaatga aaactacatg agggcagaaa ctttgtcagt tttacttatc tctgtgtcac 13860
   cagagattca tcgtttatta agtgctgaaa aatctttgtt gaataatgat attattatta 13920
   ttgttatttt tgagacaaag tctctctctg tcatcaggct ggagtgcagt ggtgtgatct 13980
   tgactcactg caacctctgc ctcccgggtt caagtgattc ttctggctca gcctcctgag 14040
   tagctgggac tacaggtgtg cgtcaccatg cccagctaat ttttttgtat ttttagtaga 14100
   gacggagttt caccatgttg gccaggatgg tctcaatctc ttgaccttgt gatccgcctg 14160
   cctcagcctc ccaaagtgct agaattacag gcatgagtca ccacacccag cccaaataat 14220
   gatattactt ttaagcatct ttaccacatg ataatctgtt ggaacatgtt tagtgacagg 14280
   aagttcacta cctttctttt gaatttgaat tgttttagta gttaactttt tagtatgttg 14340
   atgtgaaatc taactacata gcatctccct ttttctgctt ctggagtgaa catatgtatt 14400
   tatgcctgac agacttctag gtatatgaag atgattattc tttttctttt aaattttttg 14460
   tttttttttt gggctgactg tacctattac ttcaactgtt ctttaatata acatgatatc 14520
   tggtcccctc cttccctggt ctgccttctc tggatatgtt ctattttatc ccttttgaaa 14580
   agcatagtat ccagttctga gtgttaatct cttagtaacc atcttagcct aaaaccttct 14640
   actctttact ggtattaatt cagccaaccc cattgtcata ttgattcata ttgtttttga 14700
   aatttttagt tcttttttta aacttacgat tttattgaat taaattattt aatcatcatg 14760
   tacttgtttt gaattttttt gagcttgaga tattctgttt attcattata aattttatct 14820
   taaagttaga tttggcccgt tgtttctgcc taacaatttt ttaaaaaatt ctaattgtta 14880
   tctattgcat taataagctt tctctgtttg tcaatttgag gtgtctgggt caattaccaa 14940
   gcatgtggta tgctgcaagg gactaggtca atatttgggt gtggttgttc aagcagttat 15000
   aatcctacac atcttctagt ctaacttctt aaaaaataag tgagggtttt ttgacttttt 15060
   ttttctcttt gtaaatgtgt gttggctcct attgatttat tttttaaagc tcattattct 15120
   tttttgtttt gttttgtttt gttttgtttt gttttgtttt gttttgaaac agggtctcac 15180
   tttgtcaccc aggctggagt gcagtggtgc tattgtggct cactgcagcc ttgacttctg 15240
   ggctcaggtg attctcccac ctcagcctcc agagctttgg atttagatac ttctaatatg 15300
   ccccagactg aatacttcat ctcttctgca aaacctgttt ctcttctttt gttgaagagc 15360
   tggccaggct ggctttgaac ttctgggctc aagcgacctg cccacctttg cctcccaaag 15420
   tgctgggatt acagacatga gccactgtgc tgggccctca ttattgattt tttgaaaaat 15480
   aattgatctc catttttttt tttttttttt tttttttttt ttgagacgga gtctcgctct 15540
   gttgcccagg ctggagtgca gtggcgggat ctcggctcac tgcaagctcc gcctcccggg 15600
   ttcacgccat tctcctgcct cagcctccca agtagctggg actacaggcg cccgccacta 15660
   cgcccggcta attttttgta tttttagtag agacggggtt tcaccgtttt agccgggatg 15720
   gtctcgatct cctgacctcg tgatccgccc gcctcggcct cccaaagtgc tgggattaca 15780
   ggcgtgagcc accgcgcccg gcctgatctc catttttaaa agtaactttt tggatttgaa 15840
   agtgtagata tttgttcatc tgcagttttc ttgcgttctt tctttttcca ttattttaaa 15900
   aaatatttac tctgaagagt ttagcaatct tacttgaaag ttaaggaatt taaattctga 15960
   aaacagtgac tttaaaatat ttatgatgca ttttaagtat ttaagtatta tccttagctt 16020
   tcttcctgat atttctttag tgaatactag actgttaatt tgattatggt catcctgtgt 16080
   agactcaggt ctctcctctg ctcttatgta cagtttgtac atttcccaga gtccagtatt 16140
   ctaatgtttt aagccatgtg aggaaaacta aaatgttaca gtagctcaat cattcacttc 16200
   tcatcttttt gtttcatttc taaaatctca accctcaatt tgcaccagaa ctgaaaatat 16260
   gtcatatgat ttaggaaact ctgggcttca tagatttctt tttagtagca tcattttccc 16320
   catgatcatg tagaagcagc taataatcag ttaagacttg acattctctg tcatacttac 16380
   tgtatctatt gctgcattac agatgattct aacacttaga agcctgaaac atttattatc 16440
   tccatagttt ctgagagtca agaatctggg agtggtttag ctgagtggtc tggctccaag 16500
   tctctcatga ggttgcggtt aaactgttcg tagggactac attcatctga aggtttttgg 16560
   ggctggagag tatgctttca agaagcctca cttacatagc ttttggcagg agtcctcagt 16620
   ttgttgctgg ctgttggcag aatagccatg agttcttgtc aactgtcctt ctcaataggg 16680
   ctgccttatt ctacctgtga ggtgcagctg gcttctctga gagtgattca agagagaaag 16740
   aaaaagagca ggaggaagtt gtagttactt tttatgacct agtttccaga cttgcacacc 16800
   acctctttca cgttattctg ttcattagaa tgagtcactt agtccagttt atattcaggg 16860
   agaagggaat taggctccac ctcttgaagg gaggagtgtt aaaaatgttt taaatgtttc 16920
   acatttattc atacccttct gctcaaaata cacacagttt gatgctgttc tccaaggcca 16980
   tgaattgaat attcagttga agaagtaatt tgcatacaat gagtacagat cagaataaat 17040
   tgatctttta aaatgcctga ccatcccaga cttattgaat ctgaatctct gagtaaggta 17100
   tggctatctg tgcttttaaa aatacaaagc agaaacaaca acaacaaacc cacctcaaaa 17160
   aacaaacccc aaagcaaaat aaaaatacat gccattatat atttgtccaa atccgtaaaa 17220
   tgcacaaaac caagagtgaa tcctaatgta aactatggac tttggttgat gtgtcagtgc 17280
   aagttcatca gttgtaaaaa atgttccact ctggtggggg atgttgataa tgggggaggc 17340
   tatacatgtg tgaggcaagg gacatatggg atatactttt tattttcctc tcagttttgc 17400
   tgtgaatcta aaacttgttc taaaaaaata gtcttaaaaa gaaagcctga ggcaattttc 17460
   ttgtctctgc ctcactttaa aagaacactg atgtatattg ataatggata ccatgtttca 17520
   gagtgtttat taaatagaag ataattgggt atacatactg tatatgaaag attaaaattt 17580
   tccttttgta gttatcttaa attttactgt tgagggtttt ttaaatgaaa tctttctgaa 17640
   attaccaatt ttgtaattta agcaaattaa tgctaagcaa ttatattgaa ttgcttgaga 17700
   aatatattct aggaagaaac ttcaatagaa ctttaattat ttttctaatg taagggtttt 17760
   atgggaaata aactttctgt cagtgttaat cccattaagt gtcttatcaa atagccttga 17820
   atgggggatt aaaagcaaag taaaataaca accaaacatt gtctcaaact ttgcatttga 17880
   agcttttaag tattcattac tagttactga tgaaaattaa cttttaaatt atttaattat 17940
   gtcttgtcat ttggtgaaca aggtaatatc tagatttagg aagattattt aaggttatgc 18000
   aagttaatag ctttcctttt ctgttgaaat aatatagatg agcttaaaac agtttaacat 18060
   cgatctttcc ttctcctacc ttccaaccaa cagtctttat tcaatttaca ctagacatat 18120
   cataacagat tgctgtaagt gatctaagtt aagggttatg tagtgctgtc attatgtagt 18180
   taaggagcaa ctctcaggct aattatcctg aaactcagtt ctgctatttc agatttttta 18240
   aagaatgaaa atggtttgaa gtactttgga aatttaagtt ttatttatct tttccttttt 18300
   ttagttgatt acagacatag tgaaaccagt gcaacagctc tgatggttgc tgcaggacgt 18360
   ggctttgcaa gtcaagtaga acagttaatc agtatgggag ccaatgtcca tagtaaagca 18420
   tcaaatggct ggtaatttta aactacgttg attcttcata tatctttgta tagctacaca 18480
   tatctttttt tgttttctta tgtttgtctt tatattaaaa actattgtgt aataattgct 18540
   ggtgaatttc tgttttaatg ggttaataat tttgaatgac ttgtttttta actataattt 18600
   atcttatttt ttatttaaaa atttttttga aaagacaggg tcttgatgtt gcccaggctc 18660
   atcatgaact agttttacaa ttttgatgac atcttatata tcctttgaat tttggcagga 18720
   tggcattgga ttgggctaaa cactttgggc agactgaaat tgtggatctt ctagaatctt 18780
   acaggtaaaa ctttgtacta ttttaaatta attctaccgt ctcttaaaaa agagcttcac 18840
   catataaatt atgggctatt caaatgtaga ccatttatag tcttttatcc agagattttt 18900
   ttttgtgatt ctaaggaaat tttctgtctg atctaccatg gtataatatt tgtcttattt 18960
   taagaaatca gtagagggat ttccaattaa acatggtagg tacaacataa gtatctctgc 19020
   attttctcaa accttttaca tttctcaaaa cctcactaaa atgacaatga agcaatataa 19080
   agaaatttaa aaggtgtaaa ccaaagtcaa agagaatgga agaggacagg gcaactgata 19140
   gaagatgtta attttgggat gatagatagc tgattgattg acttagcaga actaagaaaa 19200
   ctcacagtgc caatgagaaa cactcatttt agctttgaaa ctccttagag gcttgttcag 19260
   tggcctttga gtgaagcagt aaaaaaagag ttagttatca aagagaaaac ttagaataca 19320
   aaactataat aaaagatatt gcattcatgc aataaaaagt gtgctatcaa aaaggaacag 19380
   aacacaaatc tttgttcaga ttttgtcttc atgaggccta cccaaatttg ccgtatctga 19440
   tattacaatc taccccccac gcaatgtcca tcctctttaa cctgttctac gtattctttt 19500
   tttctccaga agccctacca ccctctgata cactatgtaa tatacttgtt cactgttcta 19560
   ttgatttatt gtctatctct tctccctacc agaatgtagt aagcttattg tctgcttgtt 19620
   caagaacagc aactagatgt agtagatgtg cagtgagtgt ttgttgaatg aatgaataaa 19680
   taaataattc ttgtaaatta aaaatatgat agcagaaaaa aagaagagtt acaagataaa 19740
   gtttagaaaa ttgctttcag agcaggaaaa ttgtagaaaa aacataagaa aatttgcttc 19800
   ctatgtactc ctttattagg aaactgttgg agaatgtgct tcacaaaaaa gaagtaaaaa 19860
   gaaaaacaaa aattggggga gaaaaaaaaa acgggtaaaa ccgggcaggt acagcacagg 19920
   ggataggtga aaggaatgct caggatgctg gtacagagga ttgttttgat gacacctgtg 19980
   tctagtaggc ctgatcgcat aggaacagaa gatgtggggc tctagaggga ggtctctgtg 20040
   ggggaaaaat ttacctggca ttgtcaatca tattaaaaag ctttatgaat ctgttagata 20100
   atttgggaag aattactatc agatcacaga aaactaagca aataaaaatt agacagttgt 20160
   tactctagga aaaacaaaaa gttacacaag aaaggaggta taatcatagt atattattag 20220
   gctaagcagt aaatgtttac cagtcatagt aaagtaaagg ctgaatgaag atttaacaaa 20280
   aattgtagga actggtgtgg gtggaaagtg tagggaagtg gaagtggtaa cctcattttt 20340
   cattagttgg aggttcgcag ataatgacta cgttgttaaa acaataaata gcagaaaatg 20400
   aatgttgctt agaaatatga cagcaaatac taaaaaggaa tggctaaaac aatagaatag 20460
   tcctaagcag tgtgactagg agtttttgtt ataacttcta taaaaccatt taccaccact 20520
   atgtatatgt attgctttga tttagcaaaa aaagaaaaat cacaagggaa tcctgtaaaa 20580
   ccaatcgaat taattgtctt ttagatagaa attttagata aatttacatt agatatttaa 20640
   ataaattatg aagctttcag attagatatt taaagaaaat tttgaatata tgttttataa 20700
   tacattgaaa gcttctgttt tcaaccttta gagactcaaa aaagaaaaca atggagcctg 20760
   aagttaaaat atattaaaat atctgttatt caagagcata taaaatgttt tatttgacct 20820
   gggtttttgt acttaatttt atagatgtaa aattggaaat gtagtctcag acatttgaaa 20880
   ttagttgtga cattgagtct caaaattctt ggtaggttta aatgctattt ctgatatggt 20940
   aggtttagtt gtcatttcaa tagtagcacc cagaacttgc agtcctttta ttgttaacac 21000
   tttgaatgtt ccagacattc ttatggtgtt aagtaaagct cactagatac tgcaggttac 21060
   caggtaaagc gtttatctgg ataatatttt aattatcctg gctaactggg ctttcttttt 21120
   atttgataga tcatattatc aattataaat ttcaggtatt taaaattgaa tatattatta 21180
   tgtttactgg attattgata atttatatcc aaaagttata gctttaaaca gaatataatg 21240
   tcttcttcat ttatgtgtta aaaattaaaa aacaataaac ttacagtgtt ttaaagtaga 21300
   tcttaaaatt tataagagct gagaacacat ggacacaggg aggggaacat cacacaccag 21360
   ggcctcttga ggggttccgg gataggggag ggatagcatt aggagaaata cctaatgtaa 21420
   atgaagggtt gatgggtgca gcaaactacc atggcacgtg tatacctatg taacaaacct 21480
   gcatgttgtg cacatgtatc ccagaactta aagtataata aaacatttac aagagccaat 21540
   tattaagttg tagaagttta atatttttcc cattgaaaat agtcttacgt tatttttctg 21600
   ctaaattctt taagagagat tggatttatt tgaacttcct agaaattctg ttgataattg 21660
   ctagtgatag cagtagttta catttttaca tataagatga agaagcaagg catttatttc 21720
   agtaatttat gttgattctt gcttttggaa aatagcataa tcaacttgct tttagtatag 21780
   aattttctaa ttaacagtca tgttaagttg gtacctactg agttttttaa aaatgtgaac 21840
   tatacatcag aatattgttt atagtagtaa acaattagat acaacctatt aagaaaatat 21900
   aaatgatagc tgaaagtttg tggtttttaa cagttttatt gagatataat taatatacca 21960
   tataattcac ccatttaggt gaccaattca atggtttttc atatgttcac agacttatgc 22020
   aatcatcacc acaatcaatt ttagaacagt ttcatcacca caaaaagaaa cttgtaccca 22080
   ttaggtgtct ctccctattc cgccccaact ctcctaggcc tcctccagct ttagaccaac 22140
   cactaatcta cttccagttt ctatagattt gtctattttg gacattttat ataaatgaaa 22200
   tcatgtgata tgtggttttt gtgattggct tttttcagtt agcataaaat atccatgttg 22260
   tagcactatt tttatgacta atacatattc tatggatata ccaaggtttg tttgtatatt 22320
   tgtcaattga tgaacactta ggttgtttcc actttctgac tatgaataat actggtatga 22380
   atatttgtgt acagattttt atgtggatat atttttttca tttctcttat atatacacct 22440
   aggagtggaa ttgctggggc atatggtaac tctatgttta accttttgaa gaactgccac 22500
   attgttttcc aaagcagctt taccatttta catttccacc agcaatgcat gagggtttaa 22560
   atttctccac accctctcca acacttgtta ttgtctgtct ttttgattgt agtcatccta 22620
   gttggtgtga agtggtattt cattgtggtt ttgatttgca tttccctgat agctaatgat 22680
   gttgaacatc ttttcatgtg cttataggcc atttgtatat ttactttgga gaaatgcctt 22740
   cagattgtgc ttttctaaaa gcattcattg ccaaatttta ctttgaataa tgtgcttgaa 22800
   atcaaaacat gaatcatatt atagctagga cattaagtgt cacttacctt accttcctta 22860
   gggaatattg gaaatttgaa ggacataagg attacttttg ttaatggaaa ttgactttgt 22920
   cttttttttt tttttttttt tcagtgctac actggaattt ggaaatctag atgaaagttc 22980
   tctggttcaa acaaatggaa gtgacctcag tgctgaagac agagagctcc tgaaagctta 23040
   tcatcatagt ttcgatgatg aaaaagtaga cttggatttg atcatgcatc ttctatacaa 23100
   tatctgccat agttgtgatg ctggtaaata cgtgttgtct aaaagaactg gagcaaaata 23160
   tatactttaa gaattaatat tttaagtgta caaattttta tataattgca ctgatagtag 23220
   taagtgaata atctcctgtc atcttatgat agtttaccag tacttgaaaa catgtgatac 23280
   agatttttat aattctgttg attgcctctg attcacaatg agatttaata ttaaaaagtc 23340
   attcttctcc aaggtgcagt actaattttt ctgcctggat atgacgaaat tgttggactg 23400
   agagatcgca tcctgtttga tgacaagcgg tttgctgaca gtacacatag gtaagggcta 23460
   aagccttata tctgttgctt aaaataacgg acaggtctta tagtatgttt tctctttcaa 23520
   ttgtctgcag ataccaagtc tttatgcttc attcaaatat gcaaacatcc gatcaaaaga 23580
   aagtattaaa aaacccacct gcaggtgttc gaaaaatagt aagcttcata aaatcttctt 23640
   tttaacactt tcattagtta ttttttctgt ttttttatta actttaaagt aatatcttgt 23700
   tgcagattct ttccaccaat attgctgaaa ccagcatcac agtcaatgat gttgtctttg 23760
   ttattgattc tggtaaggtg aaagaggtat gtatgggtaa gttgtagttt tacttaaatg 23820
   aagaagatta agttctactt caacaaatac ttttatttat tatttagttg tttttctttt 23880
   aattttacct ctactcaaga cagcttggac cagcgcatgc ttttataagc agaatttacg 23940
   aaaaatgtct ggttctagat aaatttaaca tactaaatat tgattttact aaatattctg 24000
   ccttaaataa ttcagtatat agaatcaatt tttgttattt taacaaagta tacaaaaatg 24060
   tagctctgct caccaaatac taggaagaaa gtaaagggtt tggttagtta tagtgaaaat 24120
   gttgtcacac agtatgggaa tttgattaca tattttttat gttttccttc tacatatgca 24180
   catggattat gtctttgttc actgttaaat ctttagtgcc tcacctatag caggtgttca 24240
   aaagatactc agttcaagag aaccatgctg aaagaaagca aagaaataga taaaccatgg 24300
   gataaccagc agctaaattg gacttaacac ttgcttttgt aaaaaaaaag tttcattgga 24360
   actcagctag tctactttta ccttttctct atggttgctg ccttcatact gcaaggcaga 24420
   ttggagtatt tgtaacagag accctctcaa agcctaaaat atttactatt tgaaagtcct 24480
   tttcaaaaac agccaacctt tgttataaaa aaaaaaaagc catttatttc cttttagtac 24540
   tggaatgccc agcagttgtc catgttccta acttttggta gataacattc gctgaaaaaa 24600
   tgaaagtggt aaagataatt gatttttcaa gggaagacat attactcatg tgtggaaagc 24660
   aaattatatc tatttagtgg ataaaaatga tcgtttttaa tgtgaaaaaa gtaattgcct 24720
   tatgttttca atgtaatttt aaggaatatt ttatagaaaa taaaatttta ttataaaaat 24780
   tggccatatg ctttataatt gaataaaggt gggataaatt ttatttttat ataatagtac 24840
   attaatgagt attatcacta aaaatcttaa tggtgagcca atgaaaattc agattttatt 24900
   gccctaaaat tacctaaagt ggataaagtg ggttttgaat attttagaaa gttcctttag 24960
   gtgttactta tttattaagt aactgaaatt taaaacatgg gtagtgtcaa aagttattta 25020
   acatacaaaa ttgacaaatg taatgtcata ttaaaaacca aataagctgt tttctaaaaa 25080
   ttttataaac tattatgcaa acatttttat ttgctctatg cagttatttt atgaaaggga 25140
   aagtataagt accgagtgac tgaaatacat gttctagtta ggcttgtatt tctttaaaat 25200
   atttctctac aataaatttt cactgtaggt tatttgaata agtatgtgga taacaatggt 25260
   tcttttttac tttcttcatt gtaacgttcg ggaaaggcta actttgttag tagctcagca 25320
   ggaagtaaag tggaagtggt taaaaatatt tctctttggg ataagattat tgattgttat 25380
   tcttactcca caaggttagg tccctgaaaa gtatttcaga aaatgtcttt ccttgatctt 25440
   cttattctgc acagtgatgc aaatctgata ggaaagtgac ctcaagggaa tagatccgtt 25500
   ctctccaatc ttgatgtctt atgagaactg gctgccatta tccagctgtg gcagagagca 25560
   acaaatagct cagcagagca cagaatggag aagttttacg ctcttctgag tttagttttt 25620
   aaatcctttt ttcttagtat gtttgtaacc agaaaatcag ggtttattaa aaatcagtac 25680
   tgctgtttta attatttaat caagagagga ctttgaatta aacttttggc atgttaaaat 25740
   atgttgataa cattcttgct attacctttt aaataccttt tatattctaa cataaagatg 25800
   gtttaaatat tacagaaatc ctttgatgct ctgaattttg ttacaatgtt aaaaatggta 25860
   tggatttcca aagctagtgc catacagcgg aaaggcaggt aatgtatatt taatgtatat 25920
   tcattcaatt gcctgtaaaa tggaaacgtt tttatatgca ttacacattt atcatatatt 25980
   ccatgttcaa taaaatagta gttttcatgt taaaattttt atattatttt tgcaaagcaa 26040
   gccattttga tacttcctta aaagttgaat gtaggttatg ttaaaattaa aaacttttgc 26100
   ctagttaaat gtacattcag atgtaagaag cttaaaaaac aaaaccaaaa attctaaaca 26160
   tatgcaatgt aggacaaaga gctaatttcc ttattatata aagaagctct ttgaaatcaa 26220
   ctaaaggcca ataatccagt taaaatggat aaagggcaca aataggcaat ttaccaaaat 26280
   aaaaaatatg catgtcctac taatacatta acctcacata tactacctaa tatgtatcta 26340
   aataaatgca aattaatgag gtattatttt ttgaatctca gatgggcatt gatgaaaatg 26400
   tttaacagta tgtacaacat agaggaaaaa catgggccaa tagtctttca tattttactg 26460
   atgggagtat agattggtgt gacttcttca gagtgcaatt tgaaactatt tatccaaatc 26520
   tactctttga ccatagattc cacttgtcat aatttatcct gagtatgctt gcacacgtgt 26580
   gcaaagatgt atgtttaaga ctgttctttg aagcattgtt tataataatg taaagtctgg 26640
   gaacagtgtg atgtccatca atactgaagt gattaaataa attatagcca cacagtggct 26700
   ttctgggtag ctgttaaaag gaaaaatgta gatctgtgtg ttcttatatg gaatgagttt 26760
   tcaggatgca ttgttaattg aaaggataca gaacatgcat ctagtagtct tttgtggtaa 26820
   gaaaaatata tgtgtgtttg tgtatgtatt ttgacacaaa tgcatcgaga agtctgggag 26880
   aaacattaaa aaacaatgaa tgattgttaa ctctgagaag gaggatttgt ggggttcttg 26940
   gagtaaggag acaattttat tttttttgta atttggttat tttactaagt gtttgggtta 27000
   ctttttaatt ttcaaaagat aacattaaaa agtatcattt tcagtaaata tttatcttgc 27060
   attttattat aattagcagg aaaacatgaa atgctgactt tcaaagaatg cctcatatac 27120
   tgaagactca tgtgcattat ttctttactt tgtaacttca tagaatgccc atgagggctt 27180
   tgttgttgtt atgtcagttg ttttcttatg accaatagtg gcttgataca ttaagctgga 27240
   tgtccctgct attaatagta tattattgtg ggtaaggctg ggtgtagtgg cttatgcttg 27300
   taatcccagc actttgggag cctgaggtgg aaggatcact tgagtctaag agtctgagac 27360
   tagcctgggc atcatagtga gaccttgtct ctacaaaaaa ttaaaaaatc agctaggtgt 27420
   ggtggcacag gcctgtgggc ctagctactt gggaggctaa ggtgggagga tcatctgatc 27480
   ctgggaggtc gaggctgcag ctgccgtgag ctgtgatcat accactctac tccagccgga 27540
   gcgacagagc cagacactgt ctcaaaaaaa ataaaataac aaatagtata ttattggaga 27600
   tgaaggccag aactgcaaaa attttcattg ccatttttca acaaatgttt atcaaaagtc 27660
   ttccctgtgc acatgttacg gatagtgata aataaaccat agcctgatag gtaaagctgg 27720
   aggtagggca aatgttgcag tccatgtggg agtccagtaa attaagcaat tttaatacag 27780
   tgttatttgt gctacaactg gatgtttcca gagttgttaa gaatgaatct gagacaattg 27840
   tgaataaagg gggaggcatt tctaaaaggc agagggaaag tataaccagg tatacttatt 27900
   tgcctaaagg caagagtgaa tagggaggat gtttggatag aatttagttt agagaatagt 27960
   gaaaaatgag ctgaaagttg gggacaagtt tttaaggacc attttgagtt ttctctcttt 28020
   tttaaaaaaa taaacagtgg gatgacatga gtatagtatt ctttcagctc tctgaagaat 28080
   ggattggcag gaacaaaaat gatggtagga ggacccagta ggaggcttct tcattaatac 28140
   tttcctataa taagggagag atgaaagtag tctggattgt tttagtggtg gtggaaataa 28200
   aaataaatag ataattaatt caagagatac taaagaaagt aatgttggtg attgattgtg 28260
   tatgtagggg gtgaggggta aggaagagag tggagttaag gatgtcatcc aggtttctgt 28320
   cttaaaaaat cggacagtag tgccatagtc agatagggaa aattggaaga gtgttttcac 28380
   tggagagaat aattaatttt tggttatttt gagtttgagt tgcctgtaga aaatcgaagt 28440
   ggattgggag gctgaggcgg gcagatcaca aggttaggag ttcaagacca gcctggccaa 28500
   catggtgaaa ccctgtctct aataaaaata caaaaattag ccaggtgtgg gggtgggcac 28560
   ctataatccc agctactcag gaggctgaag caggagaatc gcttgaacct gggaggcaga 28620
   ggctgcattg agccgagacc acgccattgc actccagcct gggtgacaga gcaagactcc 28680
   gtctcaaaaa aaaaaaaaaa agaaaatcta agtagagctg tctaagaagt ttctcaagta 28740
   taggcctaga attaggataa gaagatataa tacagatgga aattgaggtt atgaatataa 28800
   ttaaagttcc ttcaggggag agggtagtgt aagaagagca gatggctaag ggttcaatct 28860
   cagaggacca taaacattta aggactaggc aaatatagaa atctcttaga ggagcctgga 28920
   ggtaagtcag aggctgaagg gtgggtatag tgtagggaag tataaggggt cttcaaaaac 28980
   tttatggaaa atgcatctta tgaaaaaaat tatgcatgga gttcaaaaat tttttgcacc 29040
   aaaataaact catattaact tgttataacg tatctgaaca ggatctcatt tgaggcacta 29100
   agaaagataa gacatcagtt tgaaaaaagc ccctaccaga gcaacatgaa ttcttctaaa 29160
   attgaagtaa gagcaaatat cacatttatg gtgacagtgc cctgaagaaa tcaacagttt 29220
   acaaatggat aactcatttt aagaagggat gagatgatgt taaagataaa gcccatagca 29280
   ccagactgtc catgtcaatt tgtgaggaaa aagaaatctc atttgtgccc tagttgaaga 29340
   ggaccaacaa ttaacagcac aaaaatagcc aacactgtag atatcacagt tggttcaggt 29400
   tacccaattc tgactgaaaa attaaaaact gagcagactt tccacttgat gggtgccaaa 29460
   accattgctt ctagatcaac tgcagacaag agcagagctt tcaatggaaa ttttaaacaa 29520
   ttggaatcaa ggtcctgaag catttcttgg aagaattgta acagaagatg aaccatggct 29580
   ttaccagtat gatcctgaac acaaagaaca accaaaactg actaccaaga ggtagaagtg 29640
   gtccagtcaa agcaaaagcg gactggaggt catggcaaca gttttttggg atgcgtaagg 29700
   cattttgctt atcgagtttc tggagggcca aagaacagta acttctgctt attatgacag 29760
   tgttttgaga agagttagct aaagctttag cagaaaaacc cataggaaag cttcactaga 29820
   aagtccttct ccaccacagc aatgctcctg ctcattcctt tcatcaaaca agggcaattt 29880
   tatgaaagtt tttgtgggaa atcattaggc atccacctta tagtcctggc ttggctcctt 29940
   ctgaattatt ttattttcta atcttaaaat acctataaag gactcccatt tttcttaggg 30000
   taataatgta aaactgcatt gacatgatga cattccctgg accctcagtt ctttagggag 30060
   gactaaatgg ctggtgtcat cacttacaaa agtgtcttga acttaatggt tgagaaattt 30120
   tatttttttc tcttttaatt ccatttttcc acaaactttt tgaagtcctc ttgtatttga 30180
   tgaaggaaag agttgatcaa taatgtcaga ttttatagct cttataatgt gttatagtga 30240
   tgattgccta tctgggttgt gagctccatg aagacaagaa tatttctata tctctatccc 30300
   taagcatagc tagccctgcc attagctgat atagtgactt tgggtgaatt taggagactg 30360
   cttttcttcc aggtattttt ctgctgcttc atagaaaatt atgcaagtct ttatgatgac 30420
   aggataaatg gcattccctt ggagctgtgt aatgcataat ttgtatgact ctaatcactg 30480
   ggcctgaaca cagtgcttga tataagctca gtaaaattga ttgaatttaa attaccaata 30540
   ttcattcatg ctttgttttt tgctcacttt tatatgtatg ctatttcttt tgatttttac 30600
   cataacccta caagatgtat caggtaggtg tccttcatga atctcactgt ctgaaataat 30660
   tgagacccag actagtgaag tgctttccaa aatgattgat aagaggataa gaaacagaat 30720
   aggatttaaa ctcaggtatc ctgatcctgg atcatttttc tctttcctaa atcacattgc 30780
   gtttacagtt tattgtagcc tttattcgtt gtttgagtcc taatcttgta ctttattttt 30840
   aagggcaggg cgatgtagac ctggaatttg ttttcgtctg ttcagtagac tccgattcca 30900
   gaatatgttg gaatttcaga ctccggaact tttgagaatg ccattacagg taaaaattta 30960
   tttaaatata aaaggcattt ttttgggtga gggaagtgag gggccatttc aacttctatg 31020
   gattaggcct ttaaaaaatc aatttgtaaa acaaaattat tcaatatata ttatggaggg 31080
   tggctatgtc tataacaaat attttttcta tataagaaaa tttaattcat aaaccatcaa 31140
   aattttaaag aatcacttga atatttaaat tatggtacag ttttaaagga tatatatata 31200
   ttttttatct atctatctat ctatctatct atctatattt tttttttttt gagtcagagt 31260
   cttgctttgt tgcccaggct agagtgcagt ggcgcgatct cggctcactg caacctccac 31320
   ctcccgggtt caagcgattc tctggcctca gcctcccgag tagctgggat tacaggcgcc 31380
   caccaccgtg cccagctaat ttttatattt tcagtagaga cagggtttca ccatcgtgac 31440
   cagcctggtc tcaaactctt gacctcatga ttcacccgcc tcagcctccc aaagtgctgg 31500
   gattacaggt gtgagccacc gcacccagcc atattttttt aaatgcagca ttttatgagt 31560
   ttcaactata tagtgttcac cttttaagtt ctcagaggga ttagcaaaca aattatttaa 31620
   ggtccaaaag ataatatcca aatctcacta taagatgctg agattatcta gtagttttag 31680
   aactgagttc actgagattt tctgacctct attaattgtg ggtgtgtgtg tgtgtgtgtg 31740
   tgtgtgtgtg tgtgtgtgtg tgttttaagt tttctaactt catggaacag ccagacccta 31800
   tagcttctag ggaagattag cagaatgcgt ttatttgatt ctttagtgtg tttggctaat 31860
   gctggcttcc caacccttaa agagaggcag gataatgtgt taaacatcca tatacctaag 31920
   atgatttccc acttcccaat gcttatgtgt gattttctgt tcctcctggg agaaaattca 31980
   gaatccttaa caagagcata aattgcctgt tcctgcctgt attaattgtg ggtgtgtgtg 32040
   ggtgtgtgtg tgtgttttaa gttttctaac ttcatggaac agccagaccc tatagcttct 32100
   agggaagatt agcagaatgc atttatttga ttctttagtg tgtttggcta atgctggctt 32160
   ccaaaccctt aaagagaggc aggataatgt gttaaacatc catatatcta agatgatttc 32220
   ccacttccca atgcttatgt gtgattttct gttcctcctg ggagaaaatt cacaatcctt 32280
   aacaagagcg taaattgcct gttcctgcct gtgtctccag tcacaggaag gaaggaacag 32340
   gtgatttctt gttacttatc ctctcttaca ccattggttt tagtttctca agcacgtctt 32400
   ttttccttct gccttacagc tttcacaggt atctactctc tccttagatt gtttcccctt 32460
   attccttata tagttactcc tactcaatct ttacatctta gtttaagtgt ctttcttcag 32520
   gaaaactatc tgtaacttcc catggtctcc ctctcccacc cttttctgaa cttgcttaaa 32580
   tcatatttac ttatttagct ccctatagcc ttcatagcac tgacgataat cgtaattaag 32640
   tagttaatta tataattaat tatttaaagt ctgcttattc atattacagt gtaaactcaa 32700
   tgagaatgga cacttccttc tgtccccttc tttttctcca atttgcacag ggccttgcac 32760
   ttagaaagca cttaaatatt tgctgagtag ataaaatatt ctagaatgta gtaagtataa 32820
   aggggggaaa agattttttt ttttttggac agtgactatg aatgttatgt atacgaacag 32880
   tgtgaggaga tgttgccaat gggagcaatg gcaatcttgg tataagttga ttgaagaggg 32940
   tatttattaa ggttctggca tctagggcaa agtatgagct catcttttac agaaagacat 33000
   tgttccttct actgttcatt ctaatcagag actctactag tctgtgtttg ggaactgtag 33060
   ttcccatgat ttctttaaat gtgtaggtta gtattctttt taattttaaa aaattattta 33120
   ctgttttggt ttataggaac tttgcttaca taccaagctg ttagccccag ttaattgtcc 33180
   cattgctgat tttcttatga aagctcctga acctccacca gctttaattg taagaaatgc 33240
   tgtacaaatg cttaaggttg gtgtcttcat agttttattt tacatgacat ttttacttga 33300
   aattttaaac taaaaggaaa tatgtcttaa ctaattttgt ataattattt tttatttttg 33360
   tcctcctgca tgtgtccaga taaactaatt ttgtactttt aaacaattat cttaaatgat 33420
   catgtacttt aaataatatt gtcatgtatg aaacagtaga tatagttctt ttatatagta 33480
   taatagaaac gaaatatatt tttatgagat gtagtagcaa aagaaaatct atattcttat 33540
   ttctcattta ggggttttta attatgtgtt ttgattaaac agtttgctca catcaaagtc 33600
   tgttctgtct ccttttactt agacaataga tgcaatggat acatgggaag atctgactga 33660
   acttgggtat catttggctg acttgccagt agaaccacat cttggtaaaa tggtcttgtg 33720
   tgctgttgtt ttaaagtgtc tggaccccat ccttacaatt gcttgcacac tagcttatcg 33780
   agatcctttt gtactaccta ctcaggcctc tcaaaaacgt gcagctatgc tttgtaggaa 33840
   acgttttact gcaggagctt tcagtgacca tatggcactt ctcagagcat tccaggtact 33900
   attactgtca ttttatttct gaagacgttg ctgggtaaac acgtttctgg ggcaaatgta 33960
   ggaattttaa aaataattta taaattttaa aattgcatta attttgtttc attatgaagt 34020
   cacattaata gtcctgaaaa atatcttctt gctgttgtta tgttaaaatt aaaacagttt 34080
   tgctcttttt tcccattttc tccttatagg aatatatgtg tgtatttgta tatatattta 34140
   tttgtgttta tgcttctatt accaggtttc tttaaatcat gtcttgggca aggaatgttc 34200
   taaaatttca gttaagaaat actgtatact atttaaagta catagaataa agcttataag 34260
   aaaattatat tgatgtaata tctatcgatt tttcttagag ttcagccttt ttcttgtttg 34320
   ctcaagacac tatgctgaga acttaatttt gtaaagcaaa atacaaaaat cacttaaaaa 34380
   ccttttaaga taatggtaat ggcaagttga ggaaagaggc aaatggaaaa gaaaagatgg 34440
   ggaaaatagg atcctgggga agacaaatta aatgtaattt agaatttcag atatgtgggg 34500
   aagaatatgt tatttcttga aaacataggc attgtattta ttctcaaatc ttcttgaatg 34560
   tcgactgtgt ctatacattt ttttgttgtt gttttttgag acagtcttgc tctgtcaccc 34620
   aggctggagt gcagtggcac agtcttggct cattgcaacc tcagcctccc aggttcaagc 34680
   agttctcctg cctcagcctc ccgagtagct ggggttacag gcacccacca ccatgcccag 34740
   ctaatttttg tgtttttagt agagacaggg tttcaccatg tgggccaggc tggtctcgaa 34800
   cttctgacct caagtgatct gcctgctttg gcctcccaaa gtgctgggat taccatgtgt 34860
   gagccaccat gcccagccat attttttaaa aatctaactt tgtttatgca gaaagtactc 34920
   tttttcagac acagaagatt ttttcaccac atataatttt caaaaataat tacaagctct 34980
   tttgtgagta ttgatcaatg gataactcat tgttattaat tgggtatatt aaataccata 35040
   tattatagta gatagttgag tgagaagtag agggagattt agagtagaat ttctatctgt 35100
   tctttgcttt tcccccagct ccttcaaaag cagagaggat aaattattgc ttcagtcttt 35160
   tagttatata tataccaaag caagagaacc tattcacaga ggagagcttt ttatactgca 35220
   tatagatgat ttatagaaat cctactttcc attcttaggt agagtccagg gttgacatag 35280
   agattatttt ttggaggaca agagtggcac gattcttaga tataagtgtt aagaagtctt 35340
   agttgaaaac ctatactttt gtagagagta aaattagcag atgcatctga gaaggcaaat 35400
   atttttacat tgttagtcat ttaggctact tgacacttag ttctctttgc gattttataa 35460
   actcaatggc ttttttagca tttgagtata aattaatgat aaatattatt tgatcataaa 35520
   gtcttagatt ttatagaaaa ttagaataag gcaaattcac gtcgaggaat tcgtgtagtg 35580
   atttgtagtt acttgttgcc tcactaagaa gcgattagta aatgtgtctt gttatgcaat 35640
   tattctcttt ttataggcct ggcaaaaagc acgaagtgat gggtgggagc gagccttttg 35700
   tgaaaagaat tttctttcac aggctactat ggaaataatc ataggcatga gaacacagtt 35760
   gcttggtcaa cttagagcat caggtaaagt ttttccctca aagagcctat ttaagttact 35820
   gagagtaccc tgcccatatt tcatagtatt tcttatgtta actgatgcca tctattattt 35880
   ttatgacatt atcatgttgt gtatcttata ttcatgcatg atttgtagac ttcgatatgt 35940
   cttcaacttt tgcttgttga tgaaaaaatg ctattgcagg aggtgtattt gagaaatcta 36000
   gggacagaat ctatatgctt gtgatactgt tggcaatgaa caatttgtat tggttggcag 36060
   agatcaattt agatcttata gttattttct tttatcacca ttaatgaata cttatttagt 36120
   tctgtgaaat agaaatttta aaaaattgat atgaatgctg ctcttcatat ttttggaaca 36180
   caaaaacagc tagtaatact tgaagtcacc tttttttttt tttttttttg aagaggatgc 36240
   tagtattgct attacctttt agaattgcct ttttgagtag caaaaaaaaa ttgatgtggt 36300
   agtcagaaga atagtaccag agcctggatt catgggttag ctgaagtgcc agagtaatat 36360
   aaggaatgaa ccagaggact taagagttga cttgaacata aggaaatatg aaggactttt 36420
   gaaactcaag caaaaattac catatattta cttgacttca tctacagaat tagtatctat 36480
   cataaccaaa atgttgttaa taacagtcag aagaatggtt tgggtttcca gctatgaatt 36540
   tagtttgttt ctggcagcag attatgaaga actttattgc aagaatgtgg atctaaaaaa 36600
   atttaagccc caagtgattc ttatacttaa tctaacctga gcagatctac acttccctag 36660
   aatgttggtt ctgtatctgt tttagggtgg ggaggggtgg ttcatatttt aaacaacttt 36720
   ccagaggaga ttctattaat tatcgtgaaa tttgaatatc acaaaattta catgagttgt 36780
   acaagttttt ggaaaaaata cacaaaagta tcttttgcac aatagaaaaa ttggtgtggt 36840
   ttttttcccc tctaggtttt gttagagcac gaggtggtgg tgacattcgg gacgttaaca 36900
   caaactctga gaattgggct gtcgttaaag ctgcattggt ggcaggcatg tatcctaatt 36960
   tagtccacgt ggacagagag aatctagtgt tgacagggcc aaaggagaaa aaagtacgat 37020
   ttcatcctgc ttcagttctc agtcagcctc aatataaaaa ggtaaaagat tttgaatgct 37080
   gttgggtttt gaggtgaaac taatttaggt agttcactat caatgaaaaa tgctccaaat 37140
   acctgcctgc tttaagaaat taattagtta tatatatata tatatatata tcattaaata 37200
   ttggaacaaa acttataaat tttagagttg gaaaaggtaa catgttaagt aatgagtact 37260
   tttgtacgaa ctacatgagc gatgacttgt tttaattttt gcttacgtac taatatacat 37320
   catctatttc attgagagac tttacatttt aaaagtattt ccaataaact aggtgataaa 37380
   cacaattaac aatttttaaa atttattttc ttaatagatt cctccagcca atggtcaagc 37440
   tgcagcaatt aaggcactgc ccacagattg gcttatttat gatgaaatga ccagagccca 37500
   tagaatagct aatattagat gttgttcagc agtgacgcct gtcactatat tggtattctg 37560
   tggaccagct agattggcaa gtaatgctct tcaggaacct tcatccttta gaggtaaatg 37620
   tattaaggaa ataaaaatct atttgaaatg aatttttttt ttttaccaaa taatgaaatt 37680
   attttactaa accaaataat gaaattataa agatttatat agtagcagct ctattttgat 37740
   gtactaagac taattaggaa atactaaggt atactactgt ttttgaaatt tatttataag 37800
   gtaacttgat gtttatttgt gcatttttta ttaatcttgc tgttgggaag aaatttttct 37860
   tttccacttg aatttaaatg atagtttaac gtaaataaag ttaattgtaa aagaaataat 37920
   agccacccat aatctcattt ttctcatgct aacacttttt aatgttatat tttttctaca 37980
   tgtttacata ttttaaatta tttcacccaa actgttattt tatattttgt tattttcatt 38040
   acagagatat caaaaatatt tttagtattt tatattaatg actgcaaaat agtatatcac 38100
   attgaagtgg cataatgcat ttttttcctt caacttttat tttaagttca ggggtacatg 38160
   tgcaagatgt gcagttttgt tgcataggta aaggtgtgcc atggtggttt gctgcacaga 38220
   tcatgacatc atgtatctat caagcccagt gtccattagc tattcttcct gatgttctcc 38280
   ctaccctcta ccacacaccc ccagtaggcc ccagtgtgtg ttgttcccct gtctgtgtcc 38340
   atgtgttctc atcattcagc tcccacttat aagtgagaac atgcggtgtt tggttttcca 38400
   ttcctgcatt agtttgctga ggataatggc ttccaacttc agccatgtcc ctgcaaagga 38460
   catgatctaa ttccttttta tggctgcata gtatcccatg gtgtatatgt accatatttt 38520
   ctttatctag cctaccattg atgggcattt gggttgattc catgtcttta ctattgtgaa 38580
   taatgctgca ctgaacatac aaatgtactt atctttataa tagaatgatt tatattcctt 38640
   tgggtattta cccagaaatg gtatttctgc ctccaggtct ttgaagaatc accatgctgt 38700
   ctcccatggt ggttgaatta atttacacta gtgtaattta attacacagt agtgtaaaag 38760
   tgttcctttt tctctacaac ctcaccagca tgttgttttt tgacttttta ataatagcaa 38820
   ttctgatctc attgtggttt tgaattgcat tccagtaatg atcagtgacg ttgagctttt 38880
   tttcatatgt ttgttggcca catgtacatc ttttgaaaag tgtctgttcg tgtcctttgc 38940
   ccacttttta atgggttgtt ttttcttgta aatttgctta agttccttgt atactctgga 39000
   tattagacct ttgtcagatg gatagattgc aaaaattttc tcccattctg taggttgtct 39060
   gtttcctctg atgatagttg tttcttttgc tgtgcagaaa ctttagttta attagatccc 39120
   atttgtcaat tttgcttttt gctgcaattg cttttggcgt ttttatcatg aaatctttgc 39180
   ctgtgcctat gtcctgaatg gtattgccta gattttcttc gagggttttt atggtttaga 39240
   gttttacatt taagtcttta atccatcttg agttaatttt tgtcgaaggt gttaggaagg 39300
   ggcccagttt cagttttctg catatagcta accagttctt tcagcaccat ttgttaaata 39360
   aggagtcctt tcctcattgc ttgtttttgt caggtttgtt aaaaatcaga cggttgtaag 39420
   tgtgtggtct tatttctgag ttctctattc tgttccatca ttctatgtgt ttgttcttat 39480
   accagtacca tgctgtcttg gctactgtag ccctgtagta tagcttgaag tcgggtagcg 39540
   tgatgcctca gctttgttct ttttgcttag gattgtcttg gctatatggt ctcttttggt 39600
   tccataagaa ttttgaaatc gtttcttcta agtctgtgaa gactgtcagt ataatgggaa 39660
   tagcattgaa tctgtaaatt tctctgggca gtatggccat tttcatgata ttgattgttc 39720
   ctatccatga gcatggaacg tttttccatt tgtttatgtc ctctctgatt tctttgagca 39780
   gtgatacgta gttctccttg aagaggtcct tcacttccct tattagctgt attcctaggt 39840
   attttatttt actttatttt attttatttc atttttttga gatggagtct tgctctgtca 39900
   cccaggctgg agtgcagtgg cacaatctca gctcactgta acctccgcct cctgggttca 39960
   agcaattctc ctgcctgaga ctcctgagta gctgggatta caggcacgca ccacaattcc 40020
   tggctaattt ttgtattttt actagagaca gggtctcacc atgttgggca gggtgatctt 40080
   gaactccaga cctcaaggtg atccatctgc ctgccttggc ctcccaaagt gctgcaatta 40140
   caggcgtgag ctatcatgct tggccttttc ctatgtattt tatttttgtt gcaattgtga 40200
   atgtgaattc attcatgatt tggctctctg cttgcctgtt gttggtatat aggaatgcta 40260
   gcaatttttg cacattgatt ttatatcctg agactttgct gaagttgctt atcagcttaa 40320
   gaagcttttg gactgagata atgggttttc tagatgtaga tcatatcaac tacaaacaaa 40380
   gataatttga cttcctcttt tcctatttga atactcttta tttctttatt ttgagatgga 40440
   gtctcacttt gccgcccagg ttggagtgca gcggtgtgat ctcagctcac tgcaacctcc 40500
   gccttctggg ttcaagcaat tctgctgcct cagcctccgg agtagctggg attacagacg 40560
   tctgccacca tactccacta attttttttg tatttttagt agatttggca tttcaccatg 40620
   ttggccaggc aggtctcaaa ctcctgactt caagtgatct accacctcgg cctcccaatg 40680
   tgctgggatt ataggcatga gccactgcac ccacccgacg tgaatactct tcattttttt 40740
   ctctggcctg atttccttgg cctgaacttc caatcctatg ttgaataaga gtgatgagag 40800
   aggacatcct tgtactgtga cagttttcaa ggagaatgct tcctgctttt gcccattcag 40860
   tatgatactg gctgtgggtt tgtcataaat agctcttatt attttgagat atgttccttt 40920
   actacctagt ttattgagtt tttaacacga agacatgttg aattttattg aaggcctttt 40980
   ctgcatctat tgagataatc atttggtttt tgtctttaga tctgtttatg tgatgaatta 41040
   tgtttattga tttgcatatg ttgaaccagc cttgcatccc agggatgaag ccaacttgat 41100
   tgtggtggac aagcatttag atgtgctgct gtatttggtt tgcccatatt ttattgagaa 41160
   tttttgcact gatattcatc agggatattg gcctgaagtt ttcttttttg ttgtatctct 41220
   gtcaggcttt ggtatcagta tgatgctggt cttacagtat gagttatgga ggagtccctc 41280
   cttttcagtt gtttggaata gtttcaaaag aaagggtatc agctcttcta tgtacttctg 41340
   gtagaattca gctgtaaatc catctggttg ggcttttttg gttggtaggc tctttattac 41400
   tgcctcaatt tccaaacttg ttattggtct attcagggat tcagcttctt tctggttcag 41460
   tctcaggagt atgtatgtgt ccaggaatct acctctttct tctagatttt ctagtttatt 41520
   tgcatagagg tgtttatagt atcctttgat ggtagtttgt attcccatgg ggtcagtggt 41580
   gagatccctt gtatcatttt tattgtgtct atttgattct tctctctttt cttctttatt 41640
   aatctagcta gcagtctgtt ttattgattt ttaaaaagaa ccagcttttt gatttgctga 41700
   tttttttgga gggtttttcc tgtctctatc gccttcactt ctgctctgat attagttatt 41760
   tcatgtcttc tgctagcttg ggtttgtttt ctctttgttc tctaattttt taaattgaga 41820
   tgttaggtgg ttaacttgaa atctttctag ctttctgatg tgttcattta gtgctatgaa 41880
   tttccgtctt aacactactt tagctgtgtc ccagagattg tggtacattg tctctttgtt 41940
   ctcattactt tcaaataact tcttgatttc tgccttaatt ttgttattta cccaagagtc 42000
   attcaggagc aggttgttca gtttccatgt agttgtgtgg tattgagtga atgtcttagt 42060
   cttgagttct aatttgattg tgctgtggtc tgatagactg ttatgatttt acttctttta 42120
   catttactga ggagtgtttt acttccagtt atgtgatcaa ttttagaata aatgccatgt 42180
   ggcgatgaga agaatgtata ttctcttgtt tttgggtaga gagttctgta gatatctatc 42240
   aggtccactt gatccagagc tgagttcagg tcctgaatat ctttgttaat ttctgtcttt 42300
   gatgatctgt ctaatgttgt ctgtggggtg ttaaagtctc ccactattat tgtatgggag 42360
   tctaaatctc tttgtaggtc tctaagaact tgtttaatga atctgggtgc tcctgtatta 42420
   gcttgcatat atatttagga tcatgagttc tttttgttga attgagccct ttaccactgt 42480
   gtaatgcctt tctttgatct ttatggttta aagtctgttt tatcagaaac taggattgca 42540
   accactgcct ttttctgttt tccatttgct tggtaaattt tcctccatcc ctttcttttg 42600
   agcctatgtg tggctttgca tgtgagatgg gtcttttgaa gacagcatac tcgtgggtct 42660
   tggctcttta tccagtttgc cattctgtgc ctttcacttg ggacatttac cccatttacc 42720
   ttcaatatta cttttatggt tatgtattgt tatgtgtgaa tttgattctg tcatgatgct 42780
   agctggttat tttgcagact tgtttatgtg gttgcttcat agtgttactg gtttgtgtac 42840
   ttccgtgtgt ttttgtagtg gctggtaatg gatttttccc tttcatattt agtgcttcct 42900
   tcaggagctc ttgcaaggca ggcctggtgg tgacatattc cctcagcatt tgcttgtctg 42960
   aaaaggatct tatttctcct tcacttataa agctcagttt ggctggatat gaaattctgg 43020
   gttggaaatt cttttcttta agagtgttga atattggccc ccaatctctt ctggcttgta 43080
   gggtttcctc ttagaggtct actgttagtc tgatgggctt ccctttgtat gtaacctggc 43140
   ctttctctct ggctgccctt aacatttttt ctttccttta gaccttggag aatctgatga 43200
   tttttttgtc ttggggttga tcttctcatg gagtattata ctagggttct ctggatttcc 43260
   tgaatttgaa tgttggcctg tcttgctagg ttagggaagt tctggatgat atcctgaagt 43320
   atgttttcca gcttggttct attctctttt atctttcagc taccccagtc agttataggt 43380
   tcagcctctt tatataatcc catatttctt ggaggttttg ttcatttctt ttttcattct 43440
   tttttctcta ttcttgtctg cctgttttat gtcagaaaga cggtcttcaa gctctgagat 43500
   tctattctct gcttggtctg tgcttctgtt gatacttgtg attgcattgt gaagttctcc 43560
   tgttgtgttt ttcagctccg ttaggtcggt tatgttcttc tctaaactgg ctattagctc 43620
   ccgtattgtt ttatcatgat tcttagcttt tttgcattgg gttacaatat gcttctttag 43680
   ctcagcgaag tttgttatta cccaccttct gaagcctact tctgtcattt tagccatctc 43740
   agcctcagcc cagttctgtg cccttgctag agaggtatca caatcatttg gaggagaaga 43800
   ggcactctgg ctttttgagt tttcagcatc tttgcattat ttctcacctt tgcgggctta 43860
   tctacctttg atctttgagg atgctgacct ttgaatgagg tttttgtgga gtcttttctg 43920
   ttgatgttgt tgttgttttc tgtttgtttt tcttttagag tcaggccagt cttccatagg 43980
   gctgctgcgg tttgctgggg ctccccacca gacctcagtt gcctcagttt ttcctgtacc 44040
   tggaggtatc accagtgaag tctgcaaaac agcaaagatg gaagccagct ccttcctctg 44100
   gaagttctgt cctgggggtt attggcctgt tgctggccca catgcatctg taggaggtga 44160
   ctggaagccc ctgttctgag gtctcaccca gtcaggagga atgagatcag ggacctgtac 44220
   aaagaagcag tctggctgct ttttgataca gcaggtgtgc tgcattggtg ggaacccttc 44280
   ctcatccaga ctgcctgtat agccatcagg ctggagtggc tgagtcaacc aaaccacaaa 44340
   gatagcagcc acccctcccc ctgggaactc ggccccatct cagacagacc aacccactgc 44400
   tgttggctgg ctggcatccc aagccagtag gtcataattg gtgaggtgcc atggaagtgg 44460
   atcccacaga atgatgctgc ttggcttcct ggattcagcc cccttcctag ggatatgtac 44520
   aaacagattt cctgccttgc tagggatccc agggccagag tatgtaaaac tcctacgttt 44580
   ctgtgtgtgc ctgagtggct gctctgccaa cactccacac agatctgtgg catgggctca 44640
   caaagggatc ccctgattca caggttgcaa agatccatgg gagaaatgtg gtttcccggg 44700
   aggggtcata taatcactca ctgcttccct tggctggggg tgagggttcc ttttgctctg 44760
   tgctgctcct gtgtgggctg tcgccttcac ctacttttct tcattctcca tgggttgatt 44820
   tgtatgtatg cctagtatgt ccgaatgtga gaacctggat atttcagttg aagatgctga 44880
   attcattgtg ccttttcatt cttctttgtg agtgccgcag actgcagctg tttctaataa 44940
   gccatcttga ttgatcccct ggcataacac atttaaaatg ttccttcata gttagacatc 45000
   ttgatgattt caaattattt gctatttaat gattcagcaa atatttagta cctactatat 45060
   gtcaggtact gtgctagaca ttgaaggtat aacagtgtgt gtctgtcctt aggaatttac 45120
   acagtagtga gggtagacag acagtaaaaa ataaataatc tgcatgatac tattaaggaa 45180
   atacaaagag gatcgcctta ggggtgacct acttgaaaga aagcctagac acgtttttct 45240
   gagaagactt aaactgagac ctacaatatg agaagagcca gccacatgaa aagcagaaat 45300
   gtgctgcagg tagagaaaga gggaaagcac caagattctg tgctaggaaa ttatctagga 45360
   attgacagaa aggctgtgtg attggagtgc actggtcaat ggaagagggc aatgataagg 45420
   ttgaaaggaa agcaaggact tgtaggccat agaaatgcaa agaaaaggaa gtcattaaaa 45480
   ggttttaagt aaaggaatga catgacgtgg tgtgttccaa aaggtcattt tggctaaggg 45540
   gatgaattac aggagtaatt acaagaatag aagagtggag gcaggtagga ggcttttgca 45600
   gtggtcttct aacaagatga tggctcagat aggatagtga tcgctgatat gaagagaagt 45660
   agatataaaa ttattttgaa ggtactagta actgctagat tgaatgtggg tagtaaggga 45720
   aagggaggag tcaattctca gattttggtt atagtagctt gtcactcatt tgagattcag 45780
   aaaacgaagg agcaagtcgt gtaggggaga aattaagggt tctttttaga catgttaagt 45840
   ttgagatggc taagacactc taagtaaatt tttttttagg aagcagttgg atatgcattt 45900
   ttggagctca gggaagatgt ctgggctagc aatacaaatt tggaagtcat cagcttgtaa 45960
   atgatattta tagacatgag aatggatttc ttaaagagaa tatataaatt tgagtagtgg 46020
   aagagtgatt cagtgttgga ggcaaatctg tttcaagaaa ggagtggtta gctctattga 46080
   atgtgctttg agagactgag ggcattaagt ttgtatgaag agagtagaaa tagtatgatt 46140
   gataatttat gagattatgg ggcattgcta cattgacatc ttcatcagtg tgtccttttg 46200
   ctttaattca tttaatattt tcttcgtgat aaaactcttt gaagtgggaa ttacttgata 46260
   aaaatgtata gatattattc atggttcttt ctccatactc aaccatatta gcaccgttaa 46320
   gagtgaattt catggcaact atgccatcat ttggtgttgg ttttaaagat aaaatattta 46380
   aagattcctt catttgctct ttttatttct gtcaaaaaga aataactctc catgcatttc 46440
   tttatattgc cctttagttg agttgttttt ttcatcttct gcccatttat ttgggtactg 46500
   atattttact caaaaatttg aatgatagcg ttataattta aagatgttat taatcttttt 46560
   tctgtcatgt ttaagtcaat ctattattca ttttggtgtt agtattttga gttctgagtt 46620
   ttgattgctg tgtatttaaa ctgaataatc ttttcctata ctattcactt tcaaagctga 46680
   gaaaatttca tccctccatt gatgtgataa gtattgtttt tgttttctat aatttgattt 46740
   ttaatatttt ttataaccat gttgagttga ttctggccta aaacaaagat ctaagttttt 46800
   ccaagtgctt tcccctcatc tgtacaattt attaaacagt tcttatatat cctcctttag 46860
   aaagcttgca tgattgcatg gtaaatttgg tatctttaac ctttttgtat actctattcc 46920
   tttacatttc tgcctttata ttgcaccttt ttatttagta tttttattta ttaataaatt 46980
   ctaatatctg gtagtagaag ctctagctgt aactctttac tgcctttctg ttattgagct 47040
   attgtttgat aatcttacct atttttaaat aatgaatatt cattagtcat cttcttgtaa 47100
   tccttttgca aacaacagtt cttttgtgtt tgtttgtttg tttgtttgtt tgagacagag 47160
   tcttggtctg tccctcaggc tgaagtacag tggtgcaatt acagcctact gcaatctctg 47220
   tctcccaggc tcaagtgatc ctcccacctc agtctctcga gtagctggga ctaaaggcac 47280
   acaccaccat gcctggctaa tgttcatatt ttttgtagag atggggtttt gccatgttgc 47340
   ccaggctggt ctcaaacttc tgtgctctta caatctgctc acctcggtct cccaaagtgt 47400
   tgggattaca ggcgtgagcc accgcacctg gcctcttttg tatattaatt taatgtttcc 47460
   atccaggaaa tttagtatct tttaattagc cataccttat tttaaattta tcttaaaaaa 47520
   tatatatttt tggtaacttt tataaagttt ggggatattt cctaaggtga ttcttaagat 47580
   tcttttgttg ctgtttttag ttgtcatggt gaatggattc tctttgtgta attttcttgc 47640
   tgaactttag aaacattatt gcagacatta tatattttat gtaccttgtt actttattaa 47700
   actcattaat cctttggatt ttgtatttat tgaattatat taccttcagg tggtaatact 47760
   tccatttatt accaaaatgt attcctttta tttctttctg aggatattag aaaactgaat 47820
   taaggtccat tttgtcttac tgaattttaa aggttcaaat tctaaagtta tactattgat 47880
   tataatgtta attttaataa aagtgtccct tttaaataac ggaatagtgt ttgtgtgtgt 47940
   gtatctgtgt gtgtgtgtgt gtttttgttt ttaagagaca gggtttcact ctgttattcc 48000
   ggatgtagtg cagtggtgca gtagtcatag ctcactgtaa ccctgaactc caaggctcaa 48060
   gtgatcctcc caccttagcc tcctgagtag ctgggactat aggcatgagt caccacacct 48120
   ggcatagaag gttttgtttt gttttgtttt gttttgtttt tttgtgtatg tgttaaaaat 48180
   ttgttgaatt tactaaatag catttgagca tttgttcata taatcagaaa ttatttagcc 48240
   tattgatgtg gtatatagat tttcttatgg aactgctatt ttgttctgag aataaacctt 48300
   ccttggacta agtgtgattt attacattgc tggatatttt tctaacattt tatttaaaac 48360
   ttatatttat cttcattggg gaggctgacc tgtagggttt gtgtggtgtg tgcttgtaca 48420
   tattccggta tgtaggtttt aggtgttaat caggttttgg gttaggttta gatttgtctc 48480
   attacaggaa tggtcattat ttgattccat gcaaaattga gagacttggt attctggatt 48540
   tctttgttga tgttctttaa ggatatctct gatttcttcc ttaaatatat ttgtgttctt 48600
   ctagttcctc tactttcagt tttagtaatt ttcgtgtaga aaataattaa tttctttgaa 48660
   gatttcaaat ttattattta tacatactgt atgttttagc ctatatcaat ttcatatttt 48720
   ataatgcatt ctgtattttt ataatctcat tttcacctaa ttttgtagat agatttttgc 48780
   tttcttcttc ctttattaaa tttacagagg gttatctatt aatattttca aagaactggg 48840
   ttatagtttt aattaccatt ttgtctttct aattcatctt tttataattt tctattttct 48900
   tctacttttc tttgggtttt atgttatttt tcttagatat atttagctgt atactttttc 48960
   tttttaaaac tgacctgtga ctttataaga gtattgcttt gggcaccttt catagttttt 49020
   gatatgtagt atagtcattc cttccttgaa ttatgttatt tgtttctttt tcatccagtt 49080
   attaaagatt gtgcttcaaa ttttatattc ttgtcttttt tattttgttg cattgtgatg 49140
   agacattata gtcttcaaat ggtccctttt ttttcctttt aaataaatac tgagatttaa 49200
   catgtgatct tttgagtttg aaggacttcc cacacaaatt gttgtatgtc aggagggttt 49260
   gtgtatatga agcgtattta ttcttcttag ttttttgcca taacgtaaga aggtaaaagt 49320
   gatttttttt tcattatgta cttggacagt ggatggcatt cccaatgaca gtagtgatag 49380
   tgaaatggag gacaaaacta cagctaattt ggcagccttg aaacttgatg agtggctcca 49440
   tttcacactg gagccagagg taagttgctt tcaagtagtg taataaattt ctttcattca 49500
   gttagtgtga attgatatat aatatgataa aatttttttc tttactattg agcccttagt 49560
   atcagagaat ataacttgtt tttcagtttt cttggggacc tcattcattc tcttctgtat 49620
   tggggtttat tccaacagag gacttggaaa acctagttgt ggctcattat ttaagcaagg 49680
   taattgtctt agtttgttgt tgctgctata gtaatttata aagaacagaa atttatttct 49740
   cacagttctg aaggatctta agtctgagat caaggtgctg acgtcggttg agggctgctg 49800
   gaccctgcct cctatagagt ggaggaatac tgtgtcctca tatggcaaaa ggcagtaggg 49860
   caagagggca tgaactccct ccatcaagct cttttataag gatacctaat cccattcatg 49920
   aagccagagc cctcatgact caatcacccc tcaaaggcca acacctccca ttactattgc 49980
   attggaggtt aagtttcaac atgaagtttt ggaggggaca aaaacttcaa actgtagcag 50040
   taatccttga gtatttctgg atatataaga aagtattgag tattgggtac acaagaactc 50100
   cctttatttt gtgtaactgt gttttctgag agaaagatat gattccatac tattttctct 50160
   ttgcatatac ttctgaattg ggggtttgag ggaggatgaa tgtttctttt caaattacaa 50220
   attttcatga caaagcctcc ccttgattct atctctactg tccaagatca ttctctatac 50280
   atttttggga ggcgtcaagc tgtcaatagt taacactggc taggtttact attttagcat 50340
   ccattcattc atcacttcaa cctactacac tctcacttcc attcccacca attcatcact 50400
   tcactgaaac tgtacttgcc aaggtcaact gtgtccttca tgttgtcaaa gtcagtctgc 50460
   tgtctgggat aaatgtgacc tctattattt atcactattg tccatatcct cctttttgta 50520
   ctcctgccct tccccagttt tcttccttac tcctgcccca aattctttgt cactagttac 50580
   tgtaacatta atctttctta attctttctc tgtagtcagt ccttcttagt tgtttcacag 50640
   accctgctgt gtgtgtttgt gccttaaatt ttaagttctc tagcactttg tgtcttactc 50700
   ttatcttctt actccctcta tacattccct ggggacttta tctctatgtg tatattttgc 50760
   aatcatttag gaactggtga ctttcaagtt tagagctctg tcttagacct ttctgtgaat 50820
   ccagctgcct tcaggacatg atcatttgga tgttctacag gcactaaaat aacactatga 50880
   gctacatata tttttattta ccctgctgct acttttccag tattaatcac actctgccac 50940
   aactattgtt tactgtcttt cttattagac tatttaattt gattttgtgt cttagtgcct 51000
   aaaatattat aaataaattt tatttcattc atgcattaaa aataatgaaa ttagtagtaa 51060
   attatatgaa atgtgaaata agtttgttgg aaacagttgc aaactaatgg aaactaatca 51120
   acacataggt gttttgcata tgtgatattc atttgcttgt atctatttga actattacag 51180
   gcagctagtt tattgctgca gctcagacag aagtggcata gcttattttt acgccgaatg 51240
   agagctccat ctaaaccttg gtctcaagtt gatgaagcta ccataagagc aattatagct 51300
   gttttaagca ctgaagaaca gtctgcaggt ttacaacaac catctgggat tggccaaagg 51360
   ccaaggccta tgtcttcaga agagcttcct ttggcctcat cttggaggtc aaataatagt 51420
   aggaaaagtt cagcagatac tgaattttct gatgagtgta ctactgcaga aaggtaaatt 51480
   tatgacattt taccaaaatg ggtcactttt tattcaggaa aatgaattat ttatcttaga 51540
   atcatgtgct tttataatat ttaaaaatta cttttttata atctaagatc catgaaaaga 51600
   tagcaataaa gtggaaagag ttttctactt gttcaactta attcttctaa gtattaaggt 51660
   aatatgaatt ttaaactatt gatatctgtg agttatttaa taaggcaact ctttgtcagg 51720
   aaaaggtcaa aatatctaat ttctagaatt gggtctctca aaaatatata gacattttaa 51780
   aattatgtat tttacttgat atatgtgaag taacatttta cctagatacc ctgggccata 51840
   ctggctatgg ccatttaaaa gaacaacctc cttataagcc ttatctagat atcaatactg 51900
   ctgattagca ttcctataga aaagtacttc actcagtgac acattgaatg ccagttcttg 51960
   gtgtgaggga tctagaagtg tacaaaacag gtaagatctc ctccagagga tatacatttg 52020
   attgagggga ggcatataat aagtaaacaa atttactgtc atatgacaag atttctgtta 52080
   taatttagcc aaattcttct tttcttcttt tgcaattaaa tgtggtggtg tttttgttta 52140
   ttttatgaat attaatgaga taccgcatac tgaattattt atgtaaaaaa tatattttag 52200
   taatatgata gtggttacaa taaatcattg gccatgattc agaagtgttt ctggttataa 52260
   aaacatttgt tagaatcaaa tttaagttat tttataatca ggttttcacc ttgttaaaat 52320
   tcataacaaa aaacattctt ggttatgagg ctagttttgg caatactggt agaaaagtct 52380
   ctgtagtaag aatttgattt tatagttaaa ctgaaaatag aaacaatgga aacataaaag 52440
   atacgtaatg cttactgttt ttcaaggatc aggtggggtt tttagtacct tttttgatca 52500
   gatttttgca tatttgaatg aaatataaag gcttaatatc atttaagcct ttaaagagga 52560
   ataaaacatt ggtttccatg ttcttcagat attacatttt agtaataatt taaccagttt 52620
   ccaaggaaag cttgtaaaat gtgtgctgaa cctttctttc tggtctagac cctgctgaga 52680
   aaaaggggaa gactaagaag taattttttt acttagatac accgtacttt ttgaaaatat 52740
   tgttctagaa ttttcttgag cattagttat ttaggatgta ggaatatatt tccataggag 52800
   ttattctgtg gtagagttat tcctaaacca gcctatgaaa agttctgtta gattcaagaa 52860
   acgtgacata ctaatatata atgcatgagt gtttcttgta tgctgattca aaggaatcaa 52920
   taatttgaga caactgagga aatgataatt ttattgggat tggtaaccac tttggttatg 52980
   taaggacaat gcactcattt taacagatgc atatttaaat atggaaggat aaaatgacat 53040
   gatgtgtggg atttactttt aaatatgtaa gccaaaacag aaaggaagca ggaaagaaaa 53100
   aacaaaataa tgttagataa atcaactagg tgaaatgttc aaggaactag gtcagtggta 53160
   ctgtgagatg tcctacattc tgaatttgtc tctgtttttt tcctggcact atttatttct 53220
   ataccctatg tatcttttat tttttattta tttttatatt ttatttttga gacagtctca 53280
   ctgtgtcgcc caggctggag tgcagtggca cgatctcagc tcactgcaac ctctgcctcc 53340
   tgggttcaag caatttcctg cctcagtctc ctgagtagct ggaattacag gtgcacgcca 53400
   ccacgcccgg ctaatttttg tatttttagt agagatgggg tttcaccatg ttagtcaggc 53460
   tggtctcgaa cccctgacct cgtaatctgc ccgcctcggc ttcccaaagt gctgggatta 53520
   caggcgtgag ccactgcacc cggactccat accctatgta tcttatataa gctggtggtt 53580
   agctctcagg gtttgatttg atccagtgtt ttaacaagaa tattttatag gtggtgttgt 53640
   gaactttata ttgcaataca tcaggaagta cttaatgtct gtcccagaaa gacccatact 53700
   ttaagaaaga ttatgaaatt cctgactgcg atgtttgtgg catttattca tagaatttta 53760
   gaatataaaa atcttggagt ttagatataa tccttttatt ttatttaaca gataaggaaa 53820
   ttaaagttcc aataagttac gaggcttgct caggatctca tacctagtta gttgtaaatt 53880
   tagaactgcc atagacatct atatttcctg accttagtat ttttaaataa tattccataa 53940
   atcataagat cattgggact ttggattgaa atggcatctg ctttgtatta taaacttata 54000
   cataactgat ctttgtatga cgaacttata tataactgat ctttgcttcc tccttcttgc 54060
   tcaagagtac tgatgaaatc tccatctcca gcattacacc cacctcagaa gtacaaagat 54120
   agaggaattt tacatcctaa acgaggtact gaggaccgat cagatcagtc ttctctgaaa 54180
   tctacagaca gcagtagtta cccaagtcct tgtgctagtc cttctcctcc atcctcagga 54240
   aaggtaagag tatctgaaag aaaatgtagt aaggaacaga tttgtagcac atgtaatcct 54300
   aggtaaataa aattgtaaaa caattagagt acttttctaa ttgtggcctc ttctagatgt 54360
   aagttgaagc tagcaaactt gatacagttt gagtaaattt taaagttttt cctgcatgcc 54420
   aaatatttct agttattacc atcttggggt attttagaat gctttaaaac tatagatcat 54480
   tcactactgg catgtaggta ggctgtttat tatttctttc ctatttcttt cgaatccttt 54540
   tttttttttt ttttttttga ggtggggtcc cactctttta cccaggctgg agtgctgtgg 54600
   tacaatcata tctactgcag ccttgaactc ctgggctcaa gtgatcttca cacttcagct 54660
   tttgagtagc tgggaccaca ggtgcatgcc accatgccca gctgattaaa gaaaaattct 54720
   ttttttatag atggggatct ccctttgttg cctcggttgg cctcaaactc ctgggctcaa 54780
   gtgatcttcc catctcagcc tccgagtggc agggattaca ggtgtgagcc accatgccca 54840
   gccctttaga aacttttttg ctaaaataaa taacaaaaag attaatttaa ctttggttta 54900
   taatttcaga atcgaaaaca gtggtttctt tctattgtta attggtttct acagtggtta 54960
   tattaaaaaa aaaacaagat gggatagggt taggaaggta ggagatgttg ataagaaatt 55020
   gaaaactatc tttaaaaaaa cttgtaataa gaataatttt aaatgttcat gatgtgatga 55080
   ctttactgtt ttagtagcta agtatttgat taggggttat ttagctgcat attgtatttt 55140
   ggcgaatgtg cctgttgaaa tactctttgt taaataagag gaaaataaat gtttttgtag 55200
   atttgcattc ttaggttttg aagttttttg gtttatttat acacctttat taaggtgtaa 55260
   cttacaataa aattcaccag tttaaactgt ataatttaat gaattttggc aaatgtatac 55320
   aatcatgtaa ctataccaca atcatctaca catttccatc acccctactc cctggtaacc 55380
   cactgttctg ctctttgtcc cttgagtttt gccttttcta gaatttcctg taaatggaat 55440
   catatatatg taacgttttg tgtctggctt atttcactta atagaatcct tttgaaattt 55500
   ctattaagtg aaataagcca gacacaaaat gctacataca taagattcag tgtatcaaca 55560
   atctatccat attgttgagt atattattag tttatctaat tactgagtag tattccattt 55620
   ctagatagac tatggttggt ttttccattc atcagttgat ggacatttgg gttgtttgca 55680
   ctttttggct attatgaata aagttgctaa cattgtacta actggtacta acattcaagt 55740
   aaaatctttg tgtctaaata tgttttctat tatttggcaa atccctagga ttagtattgt 55800
   ttgagttgtt tgataaatgt atatttagct ttacaagaaa ctgtccaact gttttccaaa 55860
   atgacagcac tattttgcat ttcctttaac aatcttaaga gttccatttg ctccatattc 55920
   ttgacaatat ttggcatttt agcttttgta attttagcca ttccagttgg gtatgtggta 55980
   ataggccatt atagttttaa tttgtatttt cccagttact aatgatgttg agcatctttt 56040
   catgtgctta ttagccattg tgtatcatct ttgtgaaatg tctgttcaaa tcttagttca 56100
   ctttttgttg ttggtttttg ttgatttgtc atctttcact gagttgtaag agttttctgt 56160
   atattctggg tacaaggctt aattattata tgttttgcat attttttcta acagtatgtg 56220
   gcttgtcttt ccatttcctt aacagtgcct tttgaagagc aaatgttttt aattttgatg 56280
   gagtccattt catcagcttt ttctattatg attagtattt ttttgtgtac taagaaattt 56340
   tgtgtaccaa acccaagatc ataattttct cctatgtttt tagaagtttt atagttggca 56400
   atcttacatt ttagatatat gatttatttt aagttaattt tcgtttatag tatgggtgag 56460
   tcaagaatta ctcgttggct tttgcttata caattgttcc agtaccgttt gttaaaacca 56520
   tttgtttttt tccattgagt tacactgaca cctttgtcaa aaatcaactg gccataaata 56580
   tgtggtctac ttattggact cttcagtttc attaatatct ctctctctct ctctctctat 56640
   atatatatat aatatataaa tatatattat gtattcatat ataatatata aatatatatt 56700
   atgtattcat atataatata taaatatata ttatgtattc atatataata tataaatata 56760
   tattatgtat tcatatataa tatataaata tatattatgt attcatatat aatatataaa 56820
   tatatattat gtattcatat ataatatata aatatatatt atgtattcat atataatata 56880
   taaatatata ttatgtattc atatataata tataaatata tattatgtat tcatatataa 56940
   tatataaata tatattatgt attcatatat aatatataaa tatatattat gtattcatat 57000
   ataatatata aatatatatt atgtattcat atataatata taaatatata ttatgtattc 57060
   atatataata tataaatata tattatgtat tcatatataa tatataaata tatattatgt 57120
   attcatatat aatatataaa tatatattat gtattcatat ataatatata aatatatatt 57180
   atgtattcat atataatata taaatatata ttatgtattc atatataata tataaatata 57240
   tattatgtat tcatataata tatataatgt atttatatgt aatatatatt atgtatttat 57300
   atattaatat attgtacata cataatatat agtatattta tatattaata taattaatat 57360
   ttatgacata ttattatata tatatttgcc agtggtacac tgacattatt ttagtagctt 57420
   tatagtaagt cttgaaatca gttaatcctt caatcaattg tccttcaact ttgtttatct 57480
   tcttcaatat tattttggct gttccaggtc ctttgtactt tcatataaac tttaaagtca 57540
   gcttatcagt ttctgcagaa aaattttttg gtattttgag tggaattgca ttgaatctat 57600
   agatcaattt aaagaaaatc aacaattaac acttcttagt cttctaatcc ataagcatag 57660
   tatatctgtt cattatttgg atagtcttta atttctctca acaatgtttt ataaatttca 57720
   gagtacaagt attacaattt tttgttaaat ttctccctaa gtatttaatg tttttgatac 57780
   tgttctttag aattttaatt gcaggtattc attgttagta tatacaaata catttgatat 57840
   ttgtatatct gccctgaatc ctatgacctt atttcattta ttaattctat tagcttttta 57900
   tgccttctga taataaagat aattttactc ttttcctttt caatctgatt gctgtttatt 57960
   tcttttttac cttatctctc tggccagagc ctccagcaaa atattgttca gtgttgagag 58020
   ccacatatct ttcccttgat cttaggggga aagcattttt tgtctgttag tatggtgaat 58080
   ttcattgctt gattttctta tgtttatacc ccacttggtc atggtgttat ttttatatat 58140
   tgctggatat aattttgtta aatgagggat ctttgttcat gaggggtatt attctgtagt 58200
   tcttttttct tatgtatctt tgtatggttt tgatgtcaga gtaatgccgg cctcataaga 58260
   ttagttggga agtgtttgtc ccacttcagt attttagaag agatgtgtag aattggtatt 58320
   atttcttcct tgaatatttg atagaattca cttatgaagc catttattta tttatttatt 58380
   tatttattta tattattttt ttttgagaca gagtctcact ctgccaccca ggctggagtg 58440
   cagtggcgcg atctcggctc actacaagct cagcctcctg ggttcacgcc attttccttc 58500
   ctcagcctcc tgagtagctg ggattacagg catgtgccac catgcctggc taagtttttg 58560
   tatttttcag tagagacagg gtttcaccat gttggccagg ctggtctcga actcctgatc 58620
   tcaagtgatc tgctcacctc tgcctcccaa agtgctggga ttgcacgcgt gagccaccat 58680
   gcctggccaa cagtgttctt ttattttaca ctttactcct ttatgttata ttgttttcat 58740
   acattttgtt tttatataaa tgctataatg tattgttttt actttacata gtcagttacc 58800
   tttatgaaga tttcaaagtc agaaaaatat atttttacat ttattattac taatcttgtg 58860
   ctcattatac ttttatgtta atacgaatgt ccattttgta ttatttactt ctgcctgaag 58920
   aacctccttt ctgggactct ggatacacag atgtaagacc atctcatatg gttccacagg 58980
   tcaccgatgt tctcttcatt tttttcagtc atttttctgt cttcatttca gatagttcta 59040
   ctgttttgtc tgtacgttca ctgatgtttt tatttagtgt ctaatctgct gttctttcct 59100
   tctaatgtgt ttttaaaggc agtattttca tctctagaag ttcaagtctt ttaaaaatta 59160
   aaaaaaaaaa aaatatatat atatatatat atatatgtat atatatcttc tggcccggcg 59220
   tggtttatgt ctgtaatccc agtggtttag gaggccaggt tgggatgata gcttgaggct 59280
   aggagtttga gaacaacctg ggcaacatag gaagacccta tcatctctac aaaagaataa 59340
   aaaaattagt tgggcatggt gatgggccac ctgtattcct agctactggg gaggctgagg 59400
   caggaggatc acatgaaccc aggaatttga ttttatagtg agctgtgatt gtgccactgc 59460
   attccagcct gtatgacaga gtgagaccct gtccccctaa aaaaagttaa taatatcttc 59520
   catttctcct ctcattatgt tcaagttttc ctctaccttc ttggacataa tgaatgtatt 59580
   tataacagat gctttaacat tcttgtctcc taattacatc aactctgtca tctctgagac 59640
   tgtttctgtt tattactttt ggcatttttt tcttttactt ttctttctgt tttgtcttct 59700
   tgttatccag tgctttgtct gtggagaaaa tgcaagtaca aattttatat tccatatgca 59760
   catgagaaac gggttcagat ttgtaaagag ctagtaaaaa tctacttgta aatgtgtctg 59820
   attgctcatt gcaatcagag tctcaagatg acaacagtat ttaacttctc ttcagcctta 59880
   attatgtaaa taacaacctt tgtatattct ttgtgtttct tataattttg ttgggcagct 59940
   aggtttttgt atattccttt caatagcgtt agactttgtt gtgatgcaca gtttagttgc 60000
   aatcagttga atccttttga agtttgcttt taagtaatgt taggaatggg ccagagcagt 60060
   ctttattctg ttattaattt agttccaata ctaaggtatg gcccttctgt gggccagtac 60120
   catatatatt acaggttcta tctgttctgg ctgaagataa cacgaaatat tcccagccat 60180
   gagtgagctc taggaattgt ttggtctact tgttttcctt tggatctttc cctagccttg 60240
   ggttatttat cttttttctc actcatgttc aggtcagcac ttagccaaaa acttgtggga 60300
   acccctctgt gtttcctttg gagctctctt tatgtgcagc tcctttctgt ctaagatagt 60360
   acattgccat gcacagtagc cacttcggtc tctgaactcc agtgtctttc tgcacaactt 60420
   aatgagacca ctggactctg tttttctttg tcctctgaaa actgcctcca ggcagtaagc 60480
   tggagaagta tttgttttgt tttctgattt tgaaagttta ttaaaacaat ttctgaaagt 60540
   atatttgagg tataaaccaa agttggtact ccaggagcct ttgttgataa gggattttga 60600
   atgctctgaa aatgagtact tgtttttggc taactttatt ttggttgagt cttgtgtagg 60660
   tttcagacat gatagagcta tttgcatttg ttgaatatat gataaaatat tttggcttat 60720
   atgtttatga agatgttatt taatggagcc tcttggtttt gaaaggtcag gttacctttc 60780
   aagaacttat gttttctttt atagggctca aaatctcctt cgccaagacc aaacatgcct 60840
   gttcgatact tcataatgaa gagtagcaat ttgagaaacc ttgaaatttc tcaacagaag 60900
   ggtatctggt ctacaactcc tagtaatgaa cggaagctaa atcgagcctt ttgggaaagc 60960
   agcatagttt acttggtatt ttctgttcaa ggatctggac atttccaggt gagccaccct 61020
   gaatcagtaa aatggggttg ttctggctat aggttaaatc atagagattt ttagaaaaac 61080
   aactggcttt tcattgcatc agaatgcaag aatgcctttt gcttgactga gaataaggga 61140
   tatattgcat ctctttagag gatgatagaa ctggaatcct ctttgttttt gccttgtctt 61200
   accagagtct ttccaggtaa actatgtttc cttctgaaga caactttaga tttactaatt 61260
   ctcaagtgga ttcaaagtaa tgagaagctt gaccttttgg taaaatattt atttaaatag 61320
   gccaaggaag ttagtattga caatcagtaa actaagtatg tttcttaacc catcacccat 61380
   cttgacttaa tgtatacctg aatttataaa ataatacatt tattatttga tgtttattat 61440
   aatttttgga aatgaaagct tagaggttag cttgtatcca tttatcatgg aataaaaata 61500
   tggactttgc tattttattt agaaatcatt attgatattt gaaattgtta ggaggctaga 61560
   actgtttttt tctaaaattt gctaagagag cttttaatga aaaatttagt agcttatttt 61620
   ttgtttttgt taggtatctg ttatttcaat attatatctt aatcttaaaa ttttaaaaac 61680
   tcagccttct cagataaatt aaatttagac atgctaatct cctcctcacc tctattcctt 61740
   cctcccattt tacctacagg gattttctag gatgtcttct gagattggaa gggaaaagag 61800
   tcaggactgg ggctctgctg gactaggagg agtatttaag gtggagtgga tacgaaaaga 61860
   aagccttccc tttcaatttg cacaccattt actcaatcca tggaatgaca acaagaaagt 61920
   gcagataagc agggatgggc aggtatacaa tggcattttt ttttttattt acttttgttt 61980
   ctgtttgttt tctgttatcc agtgctttaa ttgaggagaa aatgtaagag tacaaatttt 62040
   atattccata tgcacatggg aaacaggggt tcaggtttgt aaaaagatag taaaaaccta 62100
   cttgtaaatg tgtctgattg ctccttgcag tcagagtctc aagatgacaa tagtatttaa 62160
   cttctctttg gctttaatta tgtaaagtaa ataacctttt tatattctag tggcttgttt 62220
   atttctattg tgttttgctt tgtcttcatg atgctattta catgcatttt aagctagtta 62280
   agatgatgga aattaatgta tattgaccaa atactatgct agctgcttta gaagatataa 62340
   tttatttctt atgattaaat tattagtcca attttatata ggagaaaata gactcaaaga 62400
   ttagttttgc ctaaggtcac acagctaata atttatctga accagtatga ctcgtttcaa 62460
   atcaccatgt aaaaagtgtt tgggttcttc ttaagtgctt atcataggtt atcattccac 62520
   aggagtacta tgaagtggga ttcttttcaa ttgtgtgaga ataatttcat ttttattcta 62580
   taataatgat tttgaagaaa tatgttagct agaaatctta gaagaaaaaa tattttaata 62640
   attttcaaga taagaagata tagttgctta agagagttcc ttggaaaaaa acccaacatc 62700
   gaatatgcta ggtttcacag taaacctttg gaagttccag ctgagtgctt ttgctgaatc 62760
   tctatgaaaa acaaaatcca ttatttttag gtagaaacag ttccaggact ccatgggaat 62820
   ctgttgccat tttgtcacaa aagttgagag attttgcaga ttaactgtaa tcttaaatct 62880
   ggaagtcaac tccataaacc ttaaagcaaa gaattagaac aattcatacc agatgatttt 62940
   agcatacgct ggtaggaaaa tgaaatttaa agtgattttc ttacattttt atcagtgtag 63000
   gttttggtca ttaaaaattt tcactccagt tctttttatg tttattttga cttctgattt 63060
   atctaggaac tagaacctct ggttggtgaa cagttgctcc agttatggga acgtcttccc 63120
   ttgggagaaa aaaacacaac tgattgacac tcaggttata ccatcttgac tttgagtatt 63180
   ggcagtattt gtgatcatta ggaacctttc agattattta tctttttttt tcccctttct 63240
   tctagaactc ttagctgtgg aagaacatca tgcctattta taaccactga atgcactgac 63300
   tttcaaaaac tgaggtgggg tgtgtgttac gaatgggctt tttaacactt ttagagtgtt 63360
   gctttagaac taccatcttc atatacagga gaaaggaagc atttaaattt ttatagtgat 63420
   tatagagaat gattatatga tgtttgtaat gaataaaata gtagtttcat tatttggcac 63480
   aatagcagtt tattttaaac aaacaatttg aagttaaaca tttcattttt aaaaacactg 63540
   aattacagtt cttattgatg actttttaat gcagagtaag ttgtttaaga aaggcctgaa 63600
   tatatcagaa catctgagca ccattttaga agagtcaact cttaagatta tcattagaaa 63660
   acaaatacgt caacacctat gaaaagaagc ggggaaaaca acaacaagga caacaacaaa 63720
   aaaccagtga aattctacaa gtgtcccttt taaaaatagt gtgtgctaac taagggctat 63780
   tcattctgcc atttttaact tgagacacat ttaagaataa agttggatgt tctttttctg 63840
   tgattatgaa atgcaataaa atctgaataa agggcaaagt ttcttcctga tcagattaga 63900
   ttaagtgctt agttttattc ctaattctgc cccttttaac ctgatgtgta ccttactctt 63960
   tcctccctct cttcctccct tcctcctctc cccgctcctt tcctgcattt ctccctccct 64020
   ccttccctcg tcacatctct tatatgagca cagtaattat gacctttttg ttttggctac 64080
   aacttgattg aaacaagttc aaaatttaaa caagttcata caagttttta aaaattctca 64140
   actggtagct tgcttgctgt gcgtcttcca aactaaagcc tgcaagcgcc acaattctaa 64200
   gtgccgcctt ccattttttt tcagtatgtt ttcactggct tatgttttca gatatgattt 64260
   tcctttttcg catatatggt ttaataaatg ggggataatt ttttttttgt aaatccgtgc 64320
   ttgtgccaag tctcatattt ctttgccatc tcagaattat ctttttacca ccactgttta 64380
   taaaatttcc ttagagactt tttgaaggga aaatagagca acagggaaaa atgaaagaaa 64440
   tgtcttggtt actgagctct aaatagacag gtttgatggc acttctcatg atacatttta 64500
   gttattctta taaaagcaaa caggcaaaca tgagtgtaaa ttaaagacaa aaagaaaact 64560
   ctggttttat atttgagaac acgtgaaaaa tcatgggtca acataaaatc ttgag 64615

<210> 62
<211> 30
<212> DNA
<213> Artificial Sequence

<220>
<223> fusion between a first sequence situated from nt 112893818 to nt
      112893832 on human chromosome 5 and a second sequence situated
      from nt 112958448 to nt 112958462 on human chromosome 5

<400> 62
atataacttt tatttaacct tctactttct 30

## Description

The present invention relates to genetic markers associated with sporadic pancreatic cancer and to their use for detecting a predisposition to sporadic pancreatic cancer and/or for diagnosing sporadic pancreatic cancer.

Pancreatic cancer represents 2% of all new cases of cancer but leads to 5% of all cancer deaths (Jemal et al., 2003 CA Cancer J. Clin. 53:5-26). In the USA about 30,000 new cases are diagnosed per year (Lowenfels et al., 2004 Jpn. J. Clin. Oncol. 34:238-244). However, because of its extreme lethality, pancreatic cancer is the fourth commonest cause of cancer death after cancer of the lung, prostate or breast and colo-rectum. It is therefore a major public health concern.

Familial factors are known to be involved in susceptibility to pancreatic cancer. Indeed, whereas the lifetime risk of pancreatic cancer is of approximately 1 % in the general population, it reaches 4.7% for first-degree relatives of pancreatic cancer cases and increases with each family member who is affected. In some cases, pancreatic cancer can be inherited as part of a multi-cancer syndrome, such as that associated with BRCA2 mutations (Goggins et al., 1996 Cancer Res. 56:5360-5364), Pautz-Jeghers syndrome (Giardiello et al., 2000 Gastroenterology 119:1447-1453), and familial atypical mole and melanoma syndrome (Goldstein et al., 1995 N. Engl. J. Med. 333:970-974). In those cases, the family pedigree shows evidence of other cancers such as breast or intestinal tumours, or melanomas, in addition to pancreatic tumours. However, the vast majority of pancreatic cancers are sporadic.

Pancreatic adenocarcinoma has one of the most terrible prognosis. Life expectancy of patients is indeed of 6 months after the diagnosis and the 5-year survival is less than 4%. The only effective treatment currently available is surgical resection which can only be performed when tumours are small (inferior to 2 cm). In fact, surgical treatment is usually ineffective when clinical symptoms appear. The early symptoms of pancreatic cancer are indeed usually non-specific and often ignored by the patient and doctor. These include epigastric bloating, flatulence, general malaise, diarrhoea, vomiting, and constipation. As the disease progresses, patients display painless jaundice and weight loss. The latter together with abdominal pain are generally associated with late presentation and inoperability (S'Takhar et al., 2004 BMJ 329:668-673). In that context, the possibility of identifying patients with a predisposition to the disease and/or of earlier diagnosis would certainly be helpful.

Current methods of diagnosis include haematological and biochemical investigations and imaging studies. The latter have been greatly improved in the past decades but their use is generally restricted to identifying a resectable disease. The former include serum enzymes, tumour related antigens, ectopically produced regulatory peptides, and hormones. Tumour markers offer the best possibility of serodiagnosis but they lack the sensitivity and specificity required for screening an asymptomatic population for the purpose of early detection. Indeed, whereas an ideal marker would require a specificity of greater than 99% to avoid false-positive results, the only widely used marker for pancreatic cancer, CA19-9, displays a sensitivity and specificity of only about 80-90%. It is in fact frequently elevated in pancreatic cancer but can also be expressed in other malignancies and benign conditions such as acute and chronic pancreatitis, hepatitis, and billary obstruction. Various genetic mutations identified in some patients with pancreatic adenocarcinoma have also been ineffective markers of disease due to their lack of sensitivity or specificity.

An object of the present invention is therefore to provide alternative effective tools to early detect a predisposition to sporadic pancreatic cancer in a subject and/or to diagnose a sporadic pancreatic cancer in a subject.

The inventors have analyzed genomic DNA from patients with sporadic pancreatic cancer and identified Copy Number Variations (CNVs) anomalies.

A CNV is defined as a DNA segment of 1 kb or larger and present at variable copy number in comparison with a reference genome and may involve complex gains or losses of homologous sequences at multiple sites in the genome. These deletions, insertions, duplications and complex multi-site variants are widely present in normal individuals. New array-based technologies can now detect differences in DNA copy number at much higher resolution than cytogenetic methods (Feuk et al., 2006 Nat. Rev. Genet. 7:85-97). These techniques have been used to find *de novo* chromosome aberrations below the resolution of microscopy in children with mental retardation and dysmorphic features (Koolen et al., 2006 Nat . Genet. 38:999-1001; Sharp et al., 2006 Nat. Genet. 38:1038-1042; Shaw-Smith et al., 2006 Nat. Genet. 38:1032-1037; Shaw-Smith *et al.,* 2004 **41**:241-248; Vissers et al., 2003 Am. J. Hum. Genet. 73:1261-1270), including patients with syndromic forms of autism (Jacquemont et al., 2006 J. Med. Genet. 43:843-849).

Accordingly, studies for identifying CNVs associated with cancer diseases are increasing. For example, studies have disclosed CNVs present in patients with bladder tumours (Veltman et al., 2003 Cancer Res. 63:2872-2880). A CNV called CNV14q12 was also identified in patients with chronic myeloid leukaemia (Braude et al., 2006 BMC Genomics 6:138), and eight CNVs were detected in patients suffering of schwannomas (Diaz de Stahl et al., 2005 Hum. Genet. 118:35-44). Nevertheless none of these studies demonstrated that these CNVs were present in every patient with the disease and absent from healthy individuals. The only study demonstrating such an association is from Lucito *et ad.* (Lucito et al., 2007 Canc. Biol. & Ther. 6:1592-1599) and concerns patients suffering of familial inherited pancreatic cancer, a disease which is known to be highly linked to genomic alterations. They have identified 56 CNVs in patients with familial inherited pancreatic cancer that were not present in controls.

In the present invention, the inventors compared the CNVs identified in patients with sporadic pancreatic cancer with the CNVs present in a control group of healthy individuals. They identified gene sequences that were specifically amplified or deleted in every studied patient, but not in healthy subjects. The increase of the copy number of these genes could lead to an over-activity of these genes and the loss of one of the alleles of these genes could lead to a loss of function, which could be responsible for the development of sporadic pancreatic cancer.

### Copy Number Variations (CNVs)

In order to identify new markers that could be used to detect a predisposition to sporadic pancreatic cancer and/or to diagnose early sporadic pancreatic cancer, the inventors identified Copy Number Variations (CNVs) that were present in the genome of a group of patients suffering of sporadic pancreatic cancer, but were absent from the genome of healthy subjects. These CNVs are therefore usable as predisposition and/or diagnosis markers of sporadic pancreatic cancer.

Accordingly, the present application describes a combination of markers which comprises at least two CNVs selected from the group consisting of an amplification of a sequence situated at positions from:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 44240085 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18, or
a24) nt 68579550 to nt 68579881 on human chromosome 18; or a fragment or
mutated sequence thereof or their complementary sequences and
of a deletion of a sequence situated at positions from:
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20,
b19) nt 112893833 to nt 112958447 on human chromosome 5, or
or a fragment or mutated sequence thereof, or their complementary sequences.

As used herein, the term "marker" refers to any biological, chemical or physical mean allowing identifying the presence, and possibly quantifying the expression of a target gene in a biological sample. Such markers are well known from one skilled in the art. Advantageously, the markers according to the invention are genetic markers.

As used herein, the terms "copy number variation", "copy number variant" and "CNV" are used indifferently and refers to a DNA segment of 1 kb or larger and present at variable copy number in comparison with a reference genome. The terms "structural variant", "duplicon", "indel", "intermediate-sized structural variant (ISV)", "low copy repeat (LCR)", "multisite variant (MSV)", "paralogous sequence variant (PSV)", "segmental duplication", "interchromosomal duplication", and "intrachromosomal duplication", found in the literature, are included herein in the term "CNV".

As used herein, the term "amplification" of a sequence refers to the presence of three copies or more of said sequence in the diploid genome of a subject.

In the context of the invention, a "copy" of a sequence encompasses a sequence identical to said sequence but also allelic variations of said sequence.

In diploid organisms, two copies of each nucleic sequence are naturally present in the genome. In particular, the genome displays two alleles for each gene, one on each chromosome of a pair of homologous chromosomes (except for the genes localized on sexual chromosomes). Accordingly, an amplification of a sequence means that, in addition to the two regular copies of the sequence already present in the genome (on a pair of chromosomes), the genome contains at least one additional copy of this sequence. These at least three copies may be adjacent or not on the genome; in particular they may be present in different regions of a pair of chromosomes or on chromosomes belonging to distinct pairs of chromosomes of the genome.

As used herein, the term "deletion" of a sequence corresponds to the presence of only one copy or no copy of said sequence. In other words, a deletion of a sequence means that, at least one of the two copies of the sequence normally present on a pair of chromosomes is missing.

In the context of the present invention, the positions of the nucleotides are indicated accordingly to the NCBI human genome sequence (Build 36.1, March 2006). It is known to the one skilled in the art, that a genome sequence is variable from an individual to another. Therefore, the positions defined herein may slightly change according to the human genome sequence used. However, methods to compare genomic sequences and nucleotide positions are well known to the one skilled in the art. Accordingly, CNVs of sequences situated on different nucleotide positions on another human genome than NCBI's reference sequence, but which sequence matches with the sequence delimited by the nucleotide positions defined above are within the scope of the invention.

In the context of the invention, the sequence of a CNV may be defined by the positions of the first nucleotide and last nucleotide accordingly to the NCBI human genome sequence (Build 36.1, March 2006). It may also be defined by its complete sequence. **Table 1** gives the correspondence between the above referenced positions according to NCBI human sequence and the sequence of the marker CNVs.

**Table 1: Correspondence between the referenced positions on NCBI human sequence and sequence of marker CNVs**

| **reference** | **SEQ ID NO:** | **Position in the genome** | | |
|---|---|---|---|---|
| | | **chromosome** | **Start nucleotide** | **End nucleotide** |
| a1) | 1 | 1 | 48784697 | 48804854 |
| a2) | 2 | 2 | 132999435 | 133007869 |
| a3) | 3 | 2 | 159101071 | 159102812 |
| a4) | 4 | 3 | 54983458 | 54991470 |
| a5) | 5 | 4 | 10223944 | 10225025 |
| a6) | 6 | 5 | 150498493 | 150506817 |
| a7) | 7 | 6 | 30023851 | 30026474 |
| a8) | 8 | 9 | 129132654 | 129146243 |
| a9) | 9 | 10 | 25827676 | 25853071 |
| a10) | 10 | 10 | 49651428 | 49664331 |
| a11) | 11 | 10 | 102301095 | 102323482 |
| a12) | 12 | 11 | 10581465 | 10598770 |
| a13) | 13 | 11 | 44240085 | 44244868 |
| a14) | 14 | 11 | 112797357 | 112838193 |
| a15) | 15 | 11 | 131627190 | 131631051 |
| a16) | 16 | 12 | 26159614 | 26187203 |
| a17) | 17 | 12 | 103516177 | 103520933 |
| a18) | 18 | 12 | 116622835 | 116630658 |
| a19) | 19 | 13 | 18870123 | 18907370 |
| a20) | 20 | 16 | 7525911 | 7530624 |
| a21) | 21 | 17 | 8906153 | 8917159 |
| a22) | 22 | 17 | 9517630 | 9553522 |
| a23) | 23 | 18 | 41490240 | 41492253 |
| a24) | 24 | 18 | 68579550 | 68579881 |
| b1) | 25 | 1 | 33725161 | 33736835 |
| b2) | 26 | 1 | 33741252 | 33773973 |
| b3) | 27 | 1 | 224909378 | 224922606 |
| b4) | 28 | 1 | 228390036 | 228392011 |
| b5) | 29 | 3 | 68637763 | 68639882 |
| b6) | 30 | 4 | 6138398 | 6161246 |
| b7) | 31 | 4 | 24575658 | 24595595 |
| b8) | 32 | 4 | 143690864 | 143707784 |
| b9) | 33 | 5 | 166750597 | 166784679 |
| b10) | 34 | 5 | 170305146 | 170321629 |
| b11) | 35 | 7 | 37873471 | 37891871 |
| b12) | 36 | 10 | 73954184 | 73974380 |
| b13) | 37 | 11 | 19435635 | 19445907 |
| b14) | 38 | 16 | 5007651 | 5009929 |
| b15) | 39 | 16 | 5009929 | 5027943 |
| b16) | 40 | 16 | 77369103 | 77371920 |
| b17) | 41 | 19 | 41997678 | 42020288 |
| b18) | 42 | 20 | 17424830 | 17435606 |
| b19) | 61 | 5 | 112893833 | 112958447 |

Accordingly, preferably, said at least two CNVs are selected from the group consisting of an amplification of sequences SEQ NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, or SEQ ID NO:24; or a fragment or mutated sequence thereof or their complementary sequences, and of a deletion of sequences SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, or SEQ ID NO:61 or a fragment or mutated sequence thereof or their complementary sequences.

As used herein, a "fragment" of a sequence corresponds to a portion of said sequence. Said fragment is preferably at least 10 bp long. More preferably said fragment is at least 15 bp long, in particular at least 20 bp long. Most preferably, said fragment is at least 25 bp long, at least 30 bp long, in particular at least 33 bp long. A fragment of the above sequence may be in particular a primer or probe.

In the context of the invention, a "mutated sequence" of a reference CNV refers to a sequence including insertion(s), deletion(s) or substitution(s) of one or more nucleotide(s), wherein said mutated sequence is at least 75% identical to the reference CNV. The percentage of sequence identity is calculated by comparing the mutated sequence optimally aligned with the reference sequence, determining the number of positions at which the identical nucleic acid base (e.g., A, T, C, G, U, or I) occurs in both sequences to yield the number of matched positions, dividing the number of matched positions by the total number of positions of the CNV sequence, and multiplying the result by 100 to yield the percentage of sequence identity. Preferably, the mutated sequence is at least 80%, 85%, 90%, 95% identical to the reference CNV.

Preferably said mutated sequence of a reference sequence is an allelic variant of said reference sequence. As used herein, an "allelic variant" denotes any of two or more alternative forms of a gene occupying the same chromosome locus.

As described in the example, the inventors have demonstrated that the 42 marker CNVs defined above were specific to patients displaying a sporadic pancreatic cancer. Preferably, the combination of markers comprises at least 3, at least 5, at least 10, at least 15, at least 20, at least 25, at least 30, at least 35, at least 40, at least 41, of the CNVs defined above. However, most preferably, said combination comprises all the CNVs of sequences SEQ ID NO:1 to SEQ ID NO:42 and SEQ ID NO:61.

### Method of detecting a predisposition to sporadic pancreatic cancer

The present invention also concerns an *in vitro* method of detecting a predisposition to sporadic pancreatic cancer and/or diagnosing sporadic pancreatic cancer in a subject, which comprises detecting in a biological sample of said subject at least two CNVs as defined above, wherein the detection of at least two CNVs is indicative of a predisposition to sporadic pancreatic cancer and/or of sporadic pancreatic cancer.

In other words, the above method comprises determining, in a biological sample, the number of copy(ies) of at least two sequences selected in the group of CNVs as defined above. If three copies or more of a sequence are detected in the biological sample, this sequence is amplified. If one copy or no copy of a sequence is detected in the biological sample, this sequence is deleted. If two copies of a sequence are detected in the biological sample, this sequence is neither amplified nor deleted.

Accordingly, the detection, in a biological sample, of
a) at least two sequences situated on positions selected in the group consisting of a1) to a24) as defined above, or a fragment or mutated sequence thereof or their complementary sequences, that are amplified, or
b) at least two sequences situated on positions selected in the group consisting of b1) to b19) as defined above, or a fragment or mutated sequence thereof or their complementary sequences, that are deleted, or
c) at least one sequence situated on positions selected in the group consisting of a1) to a24) as defined above, or a fragment or mutated sequence thereof or their complementary sequences, that is amplified, and at least one sequence situated on positions selected in the group consisting of b1) to b19) as defined above, or a fragment or mutated sequence thereof or their complementary sequences, that is deleted,
indicates a predisposition to sporadic pancreatic cancer and/or sporadic pancreatic cancer.

Preferably, in the method of detecting a predisposition to sporadic pancreatic cancer and/or diagnosing pancreatic cancer as defined above, said at least two CNVs are selected from the group consisting of an amplification of sequences SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23 or SEQ ID NO:24; or a fragment or mutated sequence thereof or their complementary sequences, and of a deletion of sequences SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42 or SEQ ID NO:61., or a fragment or mutated sequence thereof or their complementary sequences.

Thus, the detection, in a biological sample, of
a) at least two sequences selected in the group consisting in SEQ ID NO: 1 to SEQ ID NO:24, or a fragment or mutated sequence thereof or their complementary sequences, that are amplified, or
b) at least two sequences selected in the group consisting in SEQ ID NO:25 to SEQ ID NO:42 and SEQ ID NO:61, or a fragment or mutated sequence thereof or their complementary sequences, that are deleted, or
c) at least one sequence selected in the group consisting in SEQ ID NO: to SEQ ID NO:24, or a fragment or mutated sequence thereof or their complementary sequences, that is amplified, and at least one sequence selected in the group consisting in SEQ ID NO:25 to SEQ ID NO:42 and SEQ ID NO:61, or a fragment or mutated sequence thereof or their complementary sequences, that is deleted,
indicates a predisposition to sporadic pancreatic cancer and/or sporadic pancreatic cancer.

More preferably, the detection of at least 3, at least 5, at least 10, at least 15, at least 20, at least 25, at least 30, at least 35, at least 40, at least 41 of the CNVs defined above indicates a predisposition to sporadic pancreatic cancer and/or sporadic pancreatic cancer.

Most preferably, the detection of all CNVs of sequences SEQ ID NO:1 to SEQ ID NO:42 and SEQ ID NO:61 indicates a predisposition to sporadic pancreatic cancer and/or sporadic pancreatic cancer.

Accordingly, most preferably, in the method of detecting a predisposition to sporadic pancreatic cancer and/or diagnosing pancreatic cancer as defined above, the CNVs of sequences SEQ ID NO:1 to SEQ ID NO:42 and SEQ ID NO:61 are detected.

In the context of the invention, the term "sporadic pancreatic cancer" refers to pancreatic cancers that are not hereditary, *i.e.* a pancreatic cancer affecting a subject with no familial pancreatic cancer history. A subject with familial pancreatic cancer history denotes a subject who has at least two direct parents displaying a pancreatic cancer.

As used herein, the term "biological sample" means a substance of biological origin. In particular the biological sample comprises nucleic acids from the subject to be diagnosed. Examples of biological samples include, but are not limited to, blood and components thereof such as plasma, platelets, subpopulations of blood cells and the like; organs such as kidney, liver, heart, lung, and the like, saliva and mouth epithelial cells.

In the context of the invention, "subject" refers to an animal, preferably a non-human or human mammal. Examples of non-human mammals include rodents and primates. Most preferably, the subject is a human.

Numerous methods allowing determining the presence of a CNV in biological samples are well known from the one skilled in the art. These methods include, without being limited, hybridization methods with DNA probes specific of said marker sequences, such as comparative genomic hybridization (CGH), matrix-CGH, array-CGH, oligonucleotide arrays, representational oligonucleotide microarray (ROMA), high-throughput technologies for SNP genotyping, for example Affymetrix SNP chips, and amplification methods such as quantitative PCR.

Preferably, the presence of said marker CNVs is determined by amplification, or by hybridization with DNA probes specific of said at least two marker CNVs. More preferably, the method of the invention is implemented by carrying out array-CGH, ROMA or quantitative PCR. Most preferably, quantitative PCR is used.

Methods of quantitative PCR are well-known in the art and include real-time PCR, competitive PCR and radioactive PCR. For instance, a quantitative PCT method to enumerate DNA copy number has been described in the US patent 6,180,349.

### Combination of nucleic acids

As described above, when the CNV is a deletion of a sequence situated on positions selected in the group consisting of b1) to b19) as defined above, the genome of the subject is characterized by the presence of sequences consisting of a fusion between the sequence situated upstream said positions and the sequence situated downstream said positions. Accordingly, the detection of one of these fusion sequences in the genome of a subject indicates the presence of one of the CNVs as defined above.

Accordingly, another object of the present invention relates to a combination of at least two isolated nucleic acids which comprise a sequence selected from the group consisting of:
c1) a fusion between a first sequence situated upstream nucleotide (nt) 33725161 on human chromosome 1 and a second sequence situated downstream nt 33736835 on human chromosome 1,
c2) a fusion between a first sequence situated upstream nt 33741252 on human chromosome 1 and a second sequence situated downstream nt 33773973 on human chromosome 1,
c3) a fusion between a first sequence situated upstream nt 224909378 on human chromosome 1 and a second sequence situated downstream nt 224922606 on human chromosome 1,
c4) a fusion between a first sequence situated upstream nt 228390036 on human chromosome 1 and a second sequence situated downstream nt 228392011 on human chromosome 1,
c5) a fusion between a first sequence situated upstream nt 68637763 on human chromosome 3 and a second sequence situated downstream nt 68639882 on human chromosome 2,
c6) a fusion between a first sequence situated upstream nt 6138398 on human chromosome 4 and a second sequence situated downstream nt 6161246 on human chromosome 4,
c7) a fusion between a first sequence situated upstream nt 24575658 on human chromosome 4 and a second sequence situated downstream nt 24595595 on human chromosome 4,
c8) a fusion between a first sequence situated upstream nt 143690864 on human chromosome 4 and a second sequence situated downstream nt 143707784 on human chromosome 4,
c9) a fusion between a first sequence situated upstream nt 166750597 on human chromosome 5 and a second sequence situated downstream nt 166784679 on human chromosome 5,
c10) a fusion between a first sequence situated upstream nt 170305146 on human chromosome 5 and a second sequence situated downstream nt 170321629 on human chromosome 5,
c11) a fusion between a first sequence situated upstream nt 37873471 on human chromosome 7 and a second sequence situated downstream nt 37891871 on human chromosome 7,
c12) a fusion between a first sequence situated upstream nt 73954184 on human chromosome 10 and a second sequence situated downstream nt 73974380 on human chromosome 10,
c13) a fusion between a first sequence situated upstream nt 19435635 on human chromosome 11 and a second sequence situated downstream nt 19445907 on human chromosome 11,
c14) a fusion between a first sequence situated upstream nt 5007651 on human chromosome 16 and a second sequence situated downstream nt 5009929 on human chromosome 16,
c15) a fusion between a first sequence situated upstream nt 5009929 on human chromosome 16 and a second sequence situated downstream nt 5027943 on human chromosome 16,
c16) a fusion between a first sequence situated upstream nt 77369103 on human chromosome 16 and a second sequence situated downstream nt 77371920 on human chromosome 16,
c17) a fusion between a first sequence situated upstream nt 41997678 on human chromosome 19 and a second sequence situated downstream nt 42020288 on human chromosome 19,
c18) a fusion between a first sequence situated upstream nt 17424830 on human chromosome 20 and a second sequence situated downstream nt 17435606 on human chromosome 20, and
c19) a fusion between a first sequence situated upstream nt 112893833 on human chromosome 5 and a second sequence situated downstream nt 112958447 on human chromosome 5,
or the complementary sequences thereof.

As used herein, the term "isolated nucleic acid" refers to both RNA and DNA, including cDNA, genomic DNA, and synthetic DNA. Nucleic acids can have any three-dimensional structure. A nucleic acid can be double-stranded or single-stranded (i.e., a sense strand or an antisense strand). Non-limiting examples of nucleic acids include genes, gene fragments, exons, introns, messenger RNA (mRNA), transfer RNA (tRNA), ribosomal RNA, siRNA, micro-RNA, ribozymes, cDNA, recombinant nucleic acids, and branched nucleic acids. A nucleic acid may contain unconventional or modified nucleotides. Isolated nucleic acids according to the invention may be purified or recombinant.

As used herein, a "fusion" between a first and a second sequence denotes a sequence wherein the 3'-end of the first sequence is linked to the 5'-end of the second sequence.

In the context of the invention, a "sequence situated upstream" a nucleotide on a chromosome denotes a sequence which is situated in a region located between the 5'-end of the chromosome and said nucleotide. On the contrary, a "sequence situated downstream" a nucleotide on a chromosome denotes a sequence which is situated in a region located between said nucleotide and the 3'-end of the chromosome.

In a preferred embodiment, when the nucleic acid as defined above comprises a fusion between a first sequence situated upstream nucleotide x on a chromosome and a second sequence situated downstream nucleotide y on the chromosome, the 3'-terminal nucleotide of said first sequence is nucleotide x-1 on the chromosome and the 5'-terminal nucleotide of said second sequence is nucleotide y+1 on the chromosome.

Accordingly, preferably, in the combination as defined above, the at least two nucleic acids comprise a sequence selected from the group consisting of:
c1') a fusion between a first sequence situated upstream nt 33725161 on human chromosome 1, the 3'-terminal nucleotide of said first sequence being nt 33725160 on human chromosome 1, and a second sequence situated downstream nt 33736835 on human chromosome 1, the 5'-terminal nucleotide of said second sequence being nt 33736836 on human chromosome 1,
c2') a fusion between a first sequence situated upstream nt 33741252 on human chromosome 1, the 3'-terminal nucleotide of said first sequence being nt 33741251 on human chromosome 1, and a second sequence situated downstream nt 33773973 on human chromosome 1, the 5'-terminal nucleotide of said second sequence being nt 33773974 on human chromosome 1,
c3') a fusion between a first sequence situated upstream nt 224909378 on human chromosome 1, the 3'-terminal nucleotide of said first sequence being nt 224909377 on human chromosome 1, and a second sequence situated downstream nt 224922606 on human chromosome 1, the 5'-terminal nucleotide of said second sequence being nt 224922607 on human chromosome 1,
c4') a fusion between a first sequence situated upstream nt 228390036 on human chromosome 1, the 3'-terminal nucleotide of said first sequence being nt 228390035 on human chromosome 1, and a second sequence situated downstream nt 228392011 on human chromosome 1, the 5'-terminal nucleotide of said second sequence being nt 228392012 on human chromosome 1,
c5') a fusion between a first sequence situated upstream nt 68637763 on human chromosome 3, the 3'-terminal nucleotide of said first sequence being nt 68637762 on human chromosome 3, and a second sequence situated downstream nt 68639882 on human chromosome 3, the 5'-terminal nucleotide of said second sequence being nt 68639883 on human chromosome 3,
c6') a fusion between a first sequence situated upstream nt 6138398 on human chromosome 4, the 3'-terminal nucleotide of said first sequence being nt 6138397 on human chromosome 4, and a second sequence situated downstream nt 6161246 on human chromosome 4, the 5'-terminal nucleotide of said second sequence being nt 6161247 on human chromosome 4,
c7') a fusion between a first sequence situated upstream nt 24575658 on human chromosome 4, the 3'-terminal nucleotide of said first sequence being nt 24575657 on human chromosome 4, and a second sequence situated downstream nt 24595595 on human chromosome 4, the 5'-terminal nucleotide of said second sequence being nt 24595596 on human chromosome 4,
c8') a fusion between a first sequence situated upstream nt 143690864 on human chromosome 4, the 3'-terminal nucleotide of said first sequence being nt 143690863 on human chromosome 4, and a second sequence situated downstream nt 143707784 on human chromosome 4, the 5'-terminal nucleotide of said second sequence being nt 143707785 on human chromosome 4,
c9') a fusion between a first sequence situated upstream nt 166750597 on human chromosome 5, the 3'-terminal nucleotide of said first sequence being nt 1667505796 on human chromosome 5, and a second sequence situated downstream nt 166784679 on human chromosome 5, the 5'-terminal nucleotide of said second sequence being nt 166784680 on human chromosome 5,
c10') a fusion between a first sequence situated upstream nt 170305146 on human chromosome 5, the 3'-terminal nucleotide of said first sequence being nt 170305145 on human chromosome 5, and a second sequence situated downstream nt 170321629 on human chromosome 5, the 5'-terminal nucleotide of said second sequence being nt 170321630 on human chromosome 5,
c11') a fusion between a first sequence situated upstream nt 37873471 on human chromosome 7, the 3'-terminal nucleotide of said first sequence being nt 37873470 on human chromosome 7, and a second sequence situated downstream nt 37891871 on human chromosome 7, the 5'-terminal nucleotide of said second sequence being nt 37891872 on human chromosome 5,
c12') a fusion between a first sequence situated upstream nt 73954184 on human chromosome 10, the 3'-terminal nucleotide of said first sequence being nt 73954183 on human chromosome 10, and a second sequence situated downstream nt 73974380 on human chromosome 10, the 3'-terminal nucleotide of said second sequence being nt 73974381 on human chromosome 10,
c13') a fusion between a first sequence situated upstream nt 19435635 on human chromosome 11, the 3'-terminal nucleotide of said first sequence being nt 19435634 on human chromosome 11, and a second sequence situated downstream nt 19445907 on human chromosome 11, the 3'-terminal nucleotide of said second sequence being nt 19445908 on human chromosome 11,
c14') a fusion between a first sequence situated upstream nt 5007651 on human chromosome 16, the 3'-terminal nucleotide of said first sequence being nt 5007650 on human chromosome 16, and a second sequence situated downstream nt 5009929 on human chromosome 16, the 5'-terminal nucleotide of said second sequence being 5009930 on human chromosome 16,
c15') a fusion between a first sequence situated upstream nt 5009929 on human chromosome 16, the 3'-terminal nucleotide of said first sequence being nt 5009928 on human chromosome 16, and a second sequence situated downstream nt 5027943 on human chromosome 16, the 5'-terminal nucleotide of said second sequence being nt 5027944 on human chromosome 16,
c16') a fusion between a first sequence situated upstream nt 77369103 on human chromosome 16, the 3'-terminal nucleotide of said first sequence being nt 77369102, and a second sequence situated downstream nt 77371920 on human chromosome 16, the 5'-terminal nucleotide of said second sequence being nt 77371921 on human chromosome 16,
c17') a fusion between a first sequence situated upstream nt 41997678 on human chromosome 19, the 3'-terminal nucleotide of said first sequence being nt 41997677 on human chromosome 19, and a second sequence situated downstream nt 42020288 on human chromosome 19, the 5'-terminal nucleotide of said second sequence being nt 42020289 on human chromosome 19,
c18') a fusion between a first sequence situated upstream nt 17424830 on human chromosome 20, the 3'-terminal nucleotide of said first sequence being nt 17424829 on human chromosome 19, and a second sequence situated downstream nt 17435606 on human chromosome 20, the 5'-terminal nucleotide of said second sequence being nt 17435607 on human chromosome and 20,
c19') a fusion between a first sequence situated upstream nt 112893833 on human chromosome 5, the 3'-terminal nucleotide of said first sequence being nt 112893832 on human chromosome 5, and a second sequence situated downstream nt 112958447 on human chromosome 5, the 5'-terminal nucleotide of said second sequence being nt 112958448 on human chromosome 5,
or the complementary sequences thereof.

Preferably, said first sequence is constituted of 20,000 nucleotides or less, of 10,000 nucleotides or less, of 5,000 nucleotides or less, of 1,000 nucleotides or less, of 900 nucleotides or less, of 800 nucleotides or less, of 700 nucleotides or les, of 600 nucleotides or less, of 500 nucleotides or less, of 400 nucleotides or less, of 300 nucleotides or less, of 200 nucleotides or less, of 150, 100, 90, 80, 70, 60, 50, 40, 30, 20, 19, 18, 17, 16, 15, 14, 13, 12, 11, or 10 nucleotides.

Preferably, said second sequence is constituted of 20,000 nucleotides or less, of 10,000 nucleotides or less, of 5,000 nucleotides or less, of 1,000 nucleotides or less, of 900 nucleotides or less, of 800 nucleotides or less, of 700 nucleotides or les, of 600 nucleotides or less, of 500 nucleotides or less, of 400 nucleotides or less, of 300 nucleotides or less, of 200 nucleotides or less, of 150, 100, 90, 80, 70, 60, 50, 40, 30, 20, 19, 18, 17, 16, 15, 14, 13, 12, 11, or 10 nucleotides.

As explained above, the sequence of these nucleic acids may be defined by the respective positions of the first nucleotide and last nucleotide of the first sequence and of the first nucleotide and last nucleotide of the second sequence according to the NCBI human genome sequence (Build 36.1, March 2006). It may also be defined by its complete sequence. **Table 2** gives the correspondence between the above referenced positions according to NCBI human sequence and the sequences of the preferred nucleic acids.

**Table 2: Correspondence between the referenced positions on NCBI human sequence and sequence of nucleic acids**

| **reference** | **SEQ ID NO:** | **chromosome** | **Position in the genome** | | | |
|---|---|---|---|---|---|---|
| | | | **First sequence** | | **Second sequence** | |
| | | | **Start nucleotide** | **End nucleotide** | **Start nucleotide** | **End nucleotide** |
| c1") | 43 | 1 | 33725146 | 33725160 | 33736836 | 33736850 |
| c2") | 44 | 1 | 33741237 | 33741251 | 33773974 | 33773988 |
| c3") | 45 | 1 | 224909363 | 224909377 | 224922607 | 224922621 |
| c4") | 46 | 1 | 228390021 | 228390035 | 228392012 | 228392026 |
| c5") | 47 | 3 | 68637748 | 68637762 | 68639883 | 68639897 |
| c6") | 48 | 4 | 6138383 | 6138397 | 6161247 | 6161261 |
| c7") | 49 | 4 | 24575643 | 24575657 | 24595596 | 24595610 |
| c8") | 50 | 4 | 143690849 | 143690863 | 143707785 | 143707799 |
| c9") | 51 | 5 | 166750582 | 166750596 | 166784680 | 166784694 |
| c10") | 52 | 5 | 170305131 | 170305145 | 170321630 | 170321644 |
| c11") | 53 | 7 | 37873456 | 37873470 | 37891872 | 37891886 |
| c12") | 54 | 10 | 73954169 | 73954183 | 73974381 | 73974395 |
| c13") | 55 | 11 | 19435620 | 19435634 | 19445908 | 19445922 |
| c14") | 56 | 16 | 5007636 | 5007650 | 5009930 | 5009944 |
| c15") | 57 | 16 | 5009914 | 5009928 | 5027944 | 5027958 |
| c16") | 58 | 16 | 77369088 | 77369102 | 77371921 | 77371935 |
| c17") | 59 | 19 | 41997663 | 41997677 | 42020289 | 42020303 |
| c18") | 60 | 20 | 17424815 | 17424829 | 17435607 | 17435621 |
| c19") | 62 | 5 | 112893818 | 112893832 | 112958448 | 112958462 |

In another preferred embodiment, in the combination as defined above, the at least two nucleic acids are selected from the group consisting of the nucleic acids of sequence SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60 and SEQ ID NO:62, or a fragment or mutated sequence thereof, or their complementary sequences.

In a most preferred embodiment the combination as defined above comprises the nucleic acids of sequences SEQ ID NO:43 to SEQ ID NO:60 and SEQ ID NO:62, or their complementary sequences.

### Kits

The present invention also relates to a kit for the detection of at least two CNVs as defined above, which comprises at least two pairs of primers and/or probes that specifically hybridize, in particular under high stringency conditions, to at least two of the sequences selected from the group:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 442400885 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18,
a24) nt 68579550 to nt 68579881 on human chromosome 18,
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20, or b19) nt 112893833 to nt 112958447 on human chromosome 5,
or a fragment, mutated or complementary sequences thereof.

In particular, said kit may comprise at least two pairs of primers and/or probes that specifically hybridize, in particular under high stringency conditions, to at least two of the sequences selected from the group SEQ ID NO:1 to SEQ ID NO:42 and SEQ ID NO:61, or a fragment, mutated or complementary sequence thereof.

The kit according to the invention may comprise either at least two primer pairs, or at least two probes, or at least one primer pair and one probe, hybridizing to two different CNVs sequences.

Preferably, the kit according to the invention may comprise pairs of primers and/or probes that specifically hybridize, in particular under high stringency conditions, to at least 3, at least 5, at least 10, at least 15, at least 20, at least 25, at least 30, at least 35, at least 40, at least 41 of the CNVs sequences defined above.

As used herein, the term "primer" refers to an oligonucleotide which is capable of annealing to a target sequence and serving as a point of initiation of DNA synthesis under conditions suitable for amplification of the primer extension product which is complementary to said target sequence. The primer is preferably single stranded for maximum efficiency in amplification. Preferably, the primer is an oligodeoxyribonucleotide. The length of the primer depends on several factors, including temperature and sequence of the primer, but must be long enough to initiate the synthesis of amplification products. Preferably the primer is from 15 to 35 nucleotides in length. A primer can further contain additional features which allow for detection, immobilization, or manipulation of the amplified product. The primer may furthermore comprise covalently-bound fluorescent dyes, which confer specific fluorescence properties to the hybrid consisting of the primer and the target- sequence or non covalently-bound fluorescent dyes which can interact with the double-stranded DNA/RNA to change the fluorescence properties. Fluorescent dyes which can be used are for example SYBR-green or ethidium bromide.

A "pair of primers" or "primer pair" as used herein refers to one forward and one reverse primer as commonly used in the art of DNA amplification such as in PCR amplification.

As used herein, a "probe" refers to an oligonucleotide capable of binding in a base-specific manner to a complementary strand of nucleic acid. A probe may be labelled with a detectable moiety. Various labelling moieties are known in the art. Said moiety may, for example, either be a radioactive compound, a detectable enzyme (e.g., horse radish peroxidase (HRP)) or any other moiety capable of generating a detectable signal such as calorimetric, fluorescent, chemiluminescent or electrochemiluminescent signal. The detectable moiety may be detected using known methods. A probe may vary in length from about 5 to 100 nucleotides, or preferably from about 10 to 50 nucleotides, or more preferably from about 20 to 40 nucleotides. Most preferably, a probe comprises 33 nucleotides.

The terms "hybridize" or "hybridization," as is known to those skilled in the art, refer to the binding of a nucleic acid molecule to a particular nucleotide sequence under suitable conditions, namely under stringent conditions.

The term "stringent condition" or "high stringency condition" as used herein corresponds to conditions that are suitable to produce binding pairs between nucleic acids having a determined level of complementarity, while being unsuitable to the formation of binding pairs between nucleic acids displaying a complementarity inferior to said determined level. Stringent conditions are the combination of both hybridization and wash conditions and are sequence dependent. These conditions may be modified according to methods known from those skilled in the art (Tijssen, 1993, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, New York). Generally, high stringency conditions are selected to be about 5°C lower than the thermal melting point (Tm), preferably at a temperature close to the Tm of perfectly base-paired duplexes (Andersen, Nucleic acid Hybridization, Springer, 1999, p. 54). Hybridization procedures are well known in the art and are described for example in Ausubel, F.M., Brent, R., Kingston, R.E., Moore, D.D.,Seidman, J.G., Smith, J. A., Struhl, K. eds. (1998) Current protocols in molecular biology. V.B. Chanda, series ed. New York: John Wiley & Sons.

High stringency conditions typically involve hybridizing at about 50°C to about 68°C in 5x SSC/5x Denhardt's solution/1.0% SDS, and washing in 0.2x SSC/0.1% SDS at about 60°C to about 68°C.

Preferably, said kit comprises pairs of primers and/or probes that specifically hybridize, under high stringency conditions, to all the sequences a1) to a24) and b1) to b18) as defined above. More preferably, said kit is for the detection of the CNVs that have sequences SEQ ID NO:1 to SEQ ID NO:42 and SEQ ID NO:61, or fragments, mutated or complementary sequences thereof.

Another aspect of the present invention concerns the use of the kit as defined above for detecting *in vitro* a predisposition to sporadic pancreatic cancer and/or diagnosing sporadic pancreatic cancer in a subject. Preferably, said subject is human.

### Arrays

In a particular embodiment, the combination of the marker CNVs defined above is assembled on a same solid support, preferably a standardized support. Its size can vary according to the apparatuses used to detect the presence or absence of said marker CNVs.

Advantageously, the combination of marker CNVs according to the invention is in form of a DNA matrix, comprising a support on which nucleic acids fragments likely to hybridize to target sequences are deposed, preferably in a standardized way. The size of such supports varies according to the preparation and detection methods used. Such small supports are also referred to DNA array.

Another aspect of the present invention thus concerns a DNA array which comprises probes that specifically hybridize to at least two of the sequences selected from the group:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 44240085 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18,
a24) nt 68579550 to nt 68579881 on human chromosome 18,
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20, or
b19) nt 112893833 to nt 112958447 on human chromosome 5,
or a fragment, mutated or complementary sequence thereof.

Alternatively, said DNA array comprises probes that specifically hybridize to at least two sequences selected in the group consisting in SEQ ID NO:1 to SEQ ID NO:42 and SEQ ID NO:61 or a fragment, mutated or complementary sequence thereof.

Preferably, the DNA array according to the invention specifically detects at least 3, at least 5, at least 10, at least 15, at least 20, at least 25, at least 30, at least 35, at least 40, at least 41 of the CNVs defined above.

As used herein, the term "DNA array" refers to a set of genes, fragment of genes, oligonucleotides deposited on a support (glass slide, nylon membrane...) with a high density. Numerous scientific publications about the preparation and the use of DNA arrays are available.

Preferably, said DNA array comprises probes that specifically hybridize to all the sequences a1) to a24) and b1) to b18) as defined above. More preferably, said DNA array comprises probes that specifically hybridize to all the sequences SEQ ID NO:1 to SEQ ID NO:42 and SEQ ID NO:61.

Another aspect of the present invention concerns the use of the DNA array defined above for detecting *in vitro* a predisposition to sporadic pancreatic cancer and/or for diagnosing of sporadic pancreatic cancer in a subject. Preferably, said subject is human.

The following example further describes the way the present inventors have identified said marker CNVs. This example is illustrative, without being limited, of the above defined methods.

### EXAMPLE: Analysis of germline gene copy number variants of patients with sporadic pancreatic adenocarcinoma reveals specific variations.

This example describes the identification by the inventors of CNVs in patients affected with sporadic pancreatic adenocarcinoma, that are absent in individuals without apparent disease.

### METHODS

### Acquisition of samples

The study was approved by the appropriate institutional review board at each participating site. Written informed consent was obtained from all participants.

The inventors prospectively collected 79 DNA samples from peripheral leucocytes from patients with a diagnosis of sporadic pancreatic adenocarcinoma. Patients with familial pancreatic cancer history were excluded. Diagnosis of adenocarcinoma was confirmed by histologic analysis. All clinical information for every enrolled patient was entered in an anonymous and coded format. All DNA samples were of sufficient quality to be genotyped. 72 patients were European (23 from Palermo, 32 from Brussels, 6 were from Leizig and 11 from Barcelona) and 7 were Japanese (as self-reported in the presence of the physician) patients. Of these patients, 45 were men and 34 were women, with a mean age of 64 years. Control DNAs from 20 individuals without cancer, matched by sex and ethnic origin, were obtained. The HapMap database (Redon et al., 2006 Nature 444:444-454) was used as a reference set.

### CNV analysis

DNA was extracted from whole blood of patients carrying a pancreatic adenocarcinoma using the QIAamp mini kit (Qiagen, Chatsworth, CA, USA), according to the manufacturer's instructions. The DNA yields and purity were determined spectrophotometrically by measuring the absorbance of aliquots at 260 and 280 nm. DNA was prepared by microarray hybridization using the GeneChips Mapping Assay Protocol (Affymetrix Inc., Santa Clara, CA, USA). Briefly, 250 ng total genomic DNA was digested with *Nsp*I or *Sty*I and ligated to adaptors that hybridize to the 4 bp overhangs. A genetic primer that recognizes the adaptor sequence was used to preferentially amplify adaptor-ligated DNA fragments in the 250-2000 bp size range by optimized PCR conditions. The amplified DNA was then fragmented by DNase, labelled, and hybridized to the Affymetrix GeneChips Human Mapping 500K Array Set. Hybridizations were detected using streptavidin-phycoerythrin conjugates followed by array scanning. Hybridization, washing, staining, and chip scanning were performed by the CRCHUL microarray Core Facility using materials and methods provided by the manufacturer (Affymetrix Inc.). The raw images were analyzed using the GeneChip operating software (GCOS Ver1.4.1) and GTYPE (Ver4.1) software (Affymetrix). Samples with a genotype call rate <93% were excluded. The CNAT (Ver4.0.1) software (http://www.affymetrix.com) was used to assess CN alterations. The genomic smoothing was set at 0.01 Mb and the defaults parameters kept for the other variables. Copy number estimates were done using data from 270 HapMap samples (available online) as a reference. Chromosome X was not analyzed to avoid gender-related complications (Andersen et al., 2007 Carcinogenesis 28:38-48). The Affymetrix Integrated Genome Browser and the Ideogram Browser (www.informati.uni-ulm.de) were used for visualization and comparison. Reproducibility of the method was assessed by analyzing six DNAs from patients with pancreatic adenocarcinoma in duplicate. More than 94% of CNVs were confirmed in both assays.

### Data analysis

Using the SAS software and *ad hoc* programs, individual tables generated by CNAT (CNATv4.0.1) were merged and single nucleotide polymorphisms (SNPs) were ranked accordingly to their CN value. Two lists containing the SNPs with CN gains (3 or 4 copies) or loss (1 or 0 copies) were generated. In order to keep all copy number variation, even single SNPs, and at the same time to avoid false positive changes due to random noise in signal intensity, the inventors only kept the SNPs with the same condition, that is gains or loss, in all 79 patients. Thereafter, selected SNP probes were merged with their respective gene annotations and physical positions accordingly to the NCBI human genome sequence (Build 36.1, March 2006) using the Netaffx web server (Affymetrix). Data was then extracted and manually analyzed on excel tables.

### RESULTS

### Copy number variants in patients with pancreatic adenocarcinoma

The main goal of this study was to identify CNVs shared by patients with sporadic pancreatic adenocarcinoma to be used as markers of the disease.

The analysis conducted with the GeneChip Human Mapping 500K Array Set revealed that the number of abnormal SNP probe hybridization signals, relative to the HapMap set, ranged in patients with sporadic pancreatic cancer from 22,284 to 56,664

(median value of 33,621). Several of them were common to all patients. When looking at how the number of common SNPs with abnormal signal evolved with the number of patients included in the study, the inventors observed a sharp decrease from 35,490 to 3,371 upon inclusion of the first 6 patients, then a much slower decrease to 230 when the number of patients was increased to 53. The inventors chose to work with the set defined by the 53 patients, although including more patients might have selected a smaller set of common SNP probes. Data from the 7 Japanese patients did not influence the results suggesting that the set of SNP probes variants common to all patients does not reflect their belonging to a particular ethnic group. The only feature the 53 patients have in common is that they present pancreatic adenocarcinoma, suggesting that the set of common CNVs reveals a predisposition to this disease.

The set included 240 probes, of which 142were found amplified (3 copies or more) and 98showed 1 copy or none. The inventors thus identified 43 distinct CNVs sequences, of which 24 displayed an amplification and 19 a deletion.

These probes corresponded to an increased number of alleles for 24 genes (**Table 3**) and to an allele loss or a deletion for 19 genes (**Table 4**).

**Table 3: Twenty-eight CNVs amplified in all 79 germinal DNA of patients with sporadic pancreatic adenocarcinoma**

| **reference** | **SEQ ID NO:** | **Position in the genome** | | | **gene** |
|---|---|---|---|---|---|
| | | **chr** | **Start nucleotide** | **End nucleotide** | |
| a1) | 1 | 1 | 48784697 | 48804854 | AGBL4 |
| a2) | 2 | 2 | 132999435 | 133007869 | GPR39 |
| a3) | 3 | 2 | 159101071 | 159102812 | PKP4 |
| a4) | 4 | 3 | 54983458 | 54991470 | CACNA2D3 |
| a5) | 5 | 4 | 10223944 | 10225025 | MIST |
| a6) | 6 | 5 | 150498493 | 150506817 | ANXA6 |
| a7) | 7 | 6 | 30023851 | 30026474 | HLA-A29.1 |
| a8) | 8 | 9 | 129132654 | 129146243 | GARNL3 |
| a9) | 9 | 10 | 25827676 | 25853071 | GPR158 |
| a10) | 10 | 10 | 49651428 | 49664331 | WDFY4 |
| a11) | 11 | 10 | 102301095 | 102323482 | HIF1AN |
| a12) | 12 | 11 | 10581465 | 10598770 | MRVI1 |
| a13) | 13 | 11 | 44240085 | 44244868 | ALX4 |
| a14) | 14 | 11 | 112797357 | 112838193 | DRD2 |
| a15) | 15 | 11 | 131627190 | 131631051 | HNT |
| a16) | 16 | 12 | 26159614 | 26187203 | BHLHB3 |
| a17) | 17 | 12 | 103516177 | 103520933 | CHST11 |
| a18) | 18 | 12 | 116622835 | 116630658 | KSR2 |
| a19) | 19 | 13 | 18870123 | 18907370 | TPTE2 |
| a20) | 20 | 16 | 7525911 | 7530624 | A2BP1 |
| a21) | 21 | 17 | 8906153 | 8917159 | NTN1 |
| a22) | 22 | 17 | 9517630 | 9553522 | USP43 |
| a23) | 23 | 18 | 41490240 | 41492253 | SLC14A2 |
| a24) | 24 | 18 | 68579550 | 68579881 | NETO1 |

**Table 4: Eighteen CNVs deleted in all 79 germinal DNA of patients with sporadic pancreatic adenocarcinoma**

| **reference** | **SEQ ID NO:** | **Position in the genome** | | | **gene** |
|---|---|---|---|---|---|
| | | **chr** | **Start nucleotide** | **End nucleotide** | |
| b1) | 25 | 1 | 33725161 | 33736835 | ZSCAN20 |
| b2) | 26 | 1 | 33741252 | 33773973 | CSMD2 |
| b3) | 27 | 1 | 224909378 | 224922606 | ITPKB |
| b4) | 28 | 1 | 228390036 | 228392011 | GALNT2 |
| b5) | 29 | 3 | 68637763 | 68639882 | FAM 19A1 |
| b6) | 30 | 4 | 6138398 | 6161246 | JAKMIP1 |
| b7) | 31 | 4 | 24575658 | 24595595 | CCDC149 |
| b8) | 32 | 4 | 143690864 | 143707784 | INPP4B |
| b9) | 33 | 5 | 166750597 | 166784679 | ODZ2 |
| b10) | 34 | 5 | 170305146 | 170321629 | RANBP17 |
| b11) | 35 | 7 | 37873471 | 37891871 | TXNDC3 |
| b12) | 36 | 10 | 73954184 | 73974380 | CBARA1 |
| b13) | 37 | 11 | 19435635 | 19445907 | NAV2 |
| b14) | 38 | 16 | 5007651 | 5009929 | SEC14L5 |
| b15) | 39 | 16 | 5009929 | 5027943 | NAGPA |
| b16) | 40 | 16 | 77369103 | 77371920 | WWOX |
| b17) | 41 | 19 | 41997678 | 42020288 | ZNF790 |
| b18) | 42 | 20 | 17424830 | 17435606 | BFSP1 |
| b19) | 61 | 5 | 112893833 | 112958447 | YTHDC2 |

### Specificity of the repertoire of Copy Number Variants in patients with sporadic pancreatic cancer

Twenty individuals apparently free of any disease were used as controls. Their DNAs were investigated for the presence of common SNP probes with abnormal hybridization signal.

Unexpectedly, finding common CNVs among these individuals was exceptional, in strong contrast with data obtained in patients developing pancreatic adenocarcinoma.

The next step was to look whether the set of CNVs common to all patients with sporadic pancreatic cancer could be observed in the DNA of the 20 individuals used as controls. To this end, the SNPs from the selected set of 240 probes were analyzed in each control patient. None of them showed a similar profile. These data suggest that the set of CNVs detected in patients with sporadic pancreatic adenocarcinoma is powerfully associated with the disease.

The inventors therefore demonstrated that a restricted set of 43 CNVs in germline DNA of all patients with sporadic pancreatic adenocarcinoma included in the present study, but not in unaffected patients. This set might be used as a diagnostic tool.

## Claims

1. An *in vitro* method of detecting a predisposition to sporadic pancreatic cancer and/or of diagnosing sporadic pancreatic cancer in a subject, which comprises detecting in a biological sample of said subject at least two copy number variations (CNVs) selected from the group consisting of an amplification of a sequence situated at positions from:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 44240085 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18,
a24) nt 68579550 to nt 68579881 on human chromosome 18;
or a fragment or mutated sequence thereof, or their complementary sequences and
of a deletion of a sequence situated at positions from:
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20,
b19) nt 112893833 to nt 112958447 on human chromosome 5,
or a fragment or mutated sequence thereof or their complementary sequences;
wherein the positions of the nucleotides are indicated according to the NCBI human genome sequence (Build 36.1, March 2006),
wherein the detection of at least two CNVs is indicative of a predisposition to sporadic pancreatic cancer and/or of sporadic pancreatic cancer.

2. The method according to claim 1, wherein said at least two CNVs are selected from the group consisting of an amplification of sequences SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23 or SEQ ID NO:24; or a fragment or mutated sequence thereof or their complementary sequences, and of a deletion of sequences SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, or SEQ ID NO:61 or a fragment or mutated sequence thereof or their complementary sequences.

3. The method according to claim 1 or 2, wherein the CNVs of sequences SEQ ID NO: to SEQ ID NO:42 and SEQ ID NO:61 are detected.

4. The method according to any one of claims 1 to 3, wherein the detection of CNVs is performed by quantitative PCR.

5. A combination of at least two nucleic acids which comprise a sequence selected from the group consisting of:
c1) a fusion between a first sequence situated upstream nucleotide (nt) 33725161 on human chromosome 1 and a second sequence situated downstream nt 33736835 on human chromosome 1,
c2) a fusion between a first sequence situated upstream nt 33741252 on human chromosome 1 and a second sequence situated downstream nt 33773973 on human chromosome 1,
c3) a fusion between a first sequence situated upstream nt 224909378 on human chromosome 1 and a second sequence situated downstream nt 224922606 on human chromosome 1,
c4) a fusion between a first sequence situated upstream nt 228390036 on human chromosome 1 and a second sequence situated downstream nt 228392011 on human chromosome 1,
c5) a fusion between a first sequence situated upstream nt 68637763 on human chromosome 3 and a second sequence situated downstream nt 68639882 on human chromosome 3,
c6) a fusion between a first sequence situated upstream nt 6138398 on human chromosome 4 and a second sequence situated downstream nt 6161246 on human chromosome 4,
c7) a fusion between a first sequence situated upstream nt 24575658 on human chromosome 4 and a second sequence situated downstream nt 24595595 on human chromosome 4,
c8) a fusion between a first sequence situated upstream nt 143690864 on human chromosome 4 and a second sequence situated downstream nt 143707784 on human chromosome 4,
c9) a fusion between a first sequence situated upstream nt 166750597 on human chromosome 5 and a second sequence situated downstream nt 166784679 on human chromosome 5,
c10) a fusion between a first sequence situated upstream nt 170305146 on human chromosome 5 and a second sequence situated downstream nt 170321629 on human chromosome 5,
c11) a fusion between a first sequence situated upstream nt 37873471 on human chromosome 7 and a second sequence situated downstream nt 37891871 on human chromosome 7,
c12) a fusion between a first sequence situated upstream nt 73954184 on human chromosome 10 and a second sequence situated downstream nt 73974380 on human chromosome 10,
c13) a fusion between a first sequence situated upstream nt 19435635 on human chromosome 11 and a second sequence situated downstream nt 19445907 on human chromosome 11,
c14) a fusion between a first sequence situated upstream nt 5007651 on human chromosome 16 and a second sequence situated downstream nt 5009929 on human chromosome 16,
c15) a fusion between a first sequence situated upstream nt 5009929 on human chromosome 16 and a second sequence situated downstream nt 5027943 on human chromosome 16,
c16) a fusion between a first sequence situated upstream nt 77369103 on human chromosome 16 and a second sequence situated downstream nt 77371920 on human chromosome 16,
c17) a fusion between a first sequence situated upstream nt 41997678 on human chromosome 19 and a second sequence situated downstream nt 42020288 on human chromosome 19,
c18) a fusion between a first sequence situated upstream nt 17424830 on human chromosome 20 and a second sequence situated downstream nt 17435606 on human chromosome 20, and
c19) a fusion between a first sequence situated upstream nt 112893833 on human chromosome 5 and a second sequence situated downstream nt 112958447 on human chromosome 5,
wherein the positions of the nucleotides are indicated according to the NCBI human genome sequence (Build 36.1, March 2006), or the complementary sequences thereof.

6. The combination according to claim 5, wherein said at least two nucleic acids comprise a sequence selected from the group consisting of:
c1') a fusion between a first sequence situated upstream nt 33725161 on human chromosome 1, the 3'-terminal nucleotide of said first sequence being nt 33725160 on human chromosome 1, and a second sequence situated downstream nt 33736835 on human chromosome 1, the 5'-terminal nucleotide of said second sequence being nt 33736836 on human chromosome 1,
c2') a fusion between a first sequence situated upstream nt 33741252 on human chromosome 1, the 3'-terminal nucleotide of said first sequence being nt 33741251 on human chromosome 1, and a second sequence situated downstream nt 33773973 on human chromosome 1, the 5'-terminal nucleotide of said second sequence being nt 33773974 on human chromosome 1,
c3') a fusion between a first sequence situated upstream nt 224909378 on human chromosome 1, the 3'-terminal nucleotide of said first sequence being nt 224909377 on human chromosome 1, and a second sequence situated downstream nt 224922606 on human chromosome 1, the 5'-terminal nucleotide of said second sequence being nt 224922607 on human chromosome 1,
c4') a fusion between a first sequence situated upstream nt 228390036 on human chromosome 1, the 3'-terminal nucleotide of said first sequence being nt 228390035 on human chromosome 1, and a second sequence situated downstream nt 228392011 on human chromosome 1, the 5'-terminal nucleotide of said second sequence being nt 228392012 on human chromosome 1,
c5') a fusion between a first sequence situated upstream nt 68637763 on human chromosome 3, the 3'-terminal nucleotide of said first sequence being nt 68637762 on human chromosome 3, and a second sequence situated downstream nt 68639882 on human chromosome 3, the 5'-terminal nucleotide of said second sequence being nt 68639883 on human chromosome 3,
c6') a fusion between a first sequence situated upstream nt 6138398 on human chromosome 4, the 3'-terminal nucleotide of said first sequence being nt 6138397 on human chromosome 4, and a second sequence situated downstream nt 6161246 on human chromosome 4, the 5'-terminal nucleotide of said second sequence being nt 6161247 on human chromosome 4,
c7') a fusion between a first sequence situated upstream nt 24575658 on human chromosome 4, the 3'-terminal nucleotide of said first sequence being nt 24575657 on human chromosome 4, and a second sequence situated downstream nt 24595595 on human chromosome 4, the 5'-terminal nucleotide of said second sequence being nt 24595596 on human chromosome 4,
c8') a fusion between a first sequence situated upstream nt 143690864 on human chromosome 4, the 3'-terminal nucleotide of said first sequence being nt 143690863 on human chromosome 4, and a second sequence situated downstream nt 143707784 on human chromosome 4, the 5'-terminal nucleotide of said second sequence being nt 143707785 on human chromosome 4,
c9') a fusion between a first sequence situated upstream nt 166750597 on human chromosome 5, the 3'-terminal nucleotide of said first sequence being nt 166750596 on human chromosome 5, and a second sequence situated downstream nt 166784679 on human chromosome 5, the 5'-terminal nucleotide of said second sequence being nt 166784680 on human chromosome 5,
c10') a fusion between a first sequence situated upstream nt 170305146 on human chromosome 5, the 3'-terminal nucleotide of said first sequence being nt 170305145 on human chromosome 5, and a second sequence situated downstream nt 170321629 on human chromosome 5, the 5'-terminal nucleotide of said second sequence being nt 170321630 on human chromosome 5,
c11') a fusion between a first sequence situated upstream nt 37873471 on human chromosome 7, the 3'-terminal nucleotide of said first sequence being nt 37873470 on human chromosome 7, and a second sequence situated downstream nt 37891871 on human chromosome 7, the 5'-terminal nucleotide of said second sequence being nt 37891872 on human chromosome 7,
c12') a fusion between a first sequence situated upstream nt 73954184 on human chromosome 10, the 3'-terminal nucleotide of said first sequence being nt 73954183 on human chromosome 10, and a second sequence situated downstream nt 73974380 on human chromosome 10, the 5'-terminal nucleotide of said second sequence being nt 73974381 on human chromosome 10,
c13') a fusion between a first sequence situated upstream nt 19435635 on human chromosome 11, the 3'-terminal nucleotide of said first sequence being nt 19435634 on human chromosome 11, and a second sequence situated downstream nt 19445907 on human chromosome 11, the 5'-terminal nucleotide of said second sequence being nt 19445908 on human chromosome 11,
c14') a fusion between a first sequence situated upstream nt 5007651 on human chromosome 16, the 3'-terminal nucleotide of said first sequence being nt 5007650 on human chromosome 16, and a second sequence situated downstream nt 5009929 on human chromosome 16, the 5'-terminal nucleotide of said second sequence being nt 5009930 on human chromosome 16,
c15') a fusion between a first sequence situated upstream nt 5009929 on human chromosome 16, the 3'-terminal nucleotide of said first sequence being nt 5009928 on human chromosome 16, and a second sequence situated downstream nt 5027943 on human chromosome 16, the 5'-terminal nucleotide of said second sequence being nt 5027944 on human chromosome 16,
c16') a fusion between a first sequence situated upstream nt 77369103 on human chromosome 16, the 3'-terminal nucleotide of said first sequence being nt 77369102 on human chromosome 16, and a second sequence situated downstream nt 77371920 on human chromosome 16, the 5'-terminal nucleotide of said second sequence being nt 77371921 on human chromosome 16,
c17') a fusion between a first sequence situated upstream nt 41997678 on human chromosome 19, the 3'-terminal nucleotide of said first sequence being nt 41997677 on human chromosome 19, and a second sequence situated downstream nt 42020288 on human chromosome 19, the 5'-terminal nucleotide of said second sequence being nt 42020289 on human chromosome 19,
c18') a fusion between a first sequence situated upstream nt 17424830 on human chromosome 20, the 3'-terminal nucleotide of said first sequence being nt 17424829 on human chromosome 20, and a second sequence situated downstream nt 17435606 on human chromosome 20, the 5'-terminal nucleotide of said second sequence being nt 17435607 on human chromosome 20, and
c19') a fusion between a first sequence situated upstream nt 112893833 on human chromosome 5, the 3'-terminal nucleotide of said first sequence being nt 112893832 on human chromosome 5, and a second sequence situated downstream nt 112958447 on human chromosome 5, the 5'-terminal nucleotide of said second sequence being nt 112958448 on human chromosome 5,
wherein the positions of the nucleotides are indicated accordingly to the NCBI human genome sequence (Build 36.1, March 2006), or the complementary sequences thereof.

7. The combination according to claim 5 or 6, wherein said at least two nucleic acids are selected from the group consisting of the nucleic acids of sequence SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60 and SEQ ID NO:62, or a fragment or mutated sequence thereof, or their complementary sequences.

8. The combination according to any one of claims 5 to 7, which comprises the nucleic acids of sequences SEQ ID NO:43 to SEQ ID NO:60 and SEQ ID NO:62, or their complementary sequences.

9. Kit for the detection of at least two CNVs as defined in claim 1 or 2, which comprises at least two pairs of primers and/or probes that specifically hybridize to at least two of the sequences selected from the group:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 44240085 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18,
a24) nt 68579550 to nt 68579881 on human chromosome 18;
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20, or
b19) nt 112893833 to nt 112958447 on human chromosome 5,
or a fragment, mutated or complementary sequence thereof;
wherein the positions of the nucleotides are indicated according to the NCBI human genome sequence (Build 36.1, March 2006).

10. Kit according to claim 9, wherein the pairs of primers and/or probes specifically hybridize to all the sequences a1) to a24) and b1) to b19).

11. Use of a kit as defined in claim 9 or 10 for detecting *in vitro* a predisposition to sporadic pancreatic cancer and/or diagnosing sporadic pancreatic cancer in a subject.

12. DNA array which comprises probes that specifically hybridize to at least two of the sequences selected from the group:
a1) nucleotide (nt) 48784697 to nt 48804854 on human chromosome 1,
a2) nt 132999435 to nt 133007869 on human chromosome 2,
a3) nt 159101071 to nt 159102812 on human chromosome 2,
a4) nt 54983458 to nt 54991470 on human chromosome 3,
a5) nt 10223944 to nt 10225025 on human chromosome 4,
a6) nt 150498493 to nt 150506817 on human chromosome 5,
a7) nt 30023851 to nt 30026474 on human chromosome 6,
a8) nt 129132654 to nt 129146243 on human chromosome 9,
a9) nt 25827676 to nt 25853071 on human chromosome 10,
a10) nt 49651428 to nt 49664331 on human chromosome 10,
a11) nt 102301095 to nt 102323482 on human chromosome 10,
a12) nt 10581465 to nt 10598770 on human chromosome 11,
a13) nt 44240085 to nt 44244868 on human chromosome 11,
a14) nt 112797357 to nt 112838193 on human chromosome 11,
a15) nt 131627190 to nt 131631051 on human chromosome 11,
a16) nt 26159614 to nt 26187203 on human chromosome 12,
a17) nt 103516177 to nt 103520933 on human chromosome 12,
a18) nt 116622835 to nt 116630658 on human chromosome 12,
a19) nt 18870123 to nt 18907370 on human chromosome 13,
a20) nt 7525911 to nt 7530624 on human chromosome 16,
a21) nt 8906153 to nt 8917159 on human chromosome 17,
a22) nt 9517630 to nt 9553522 on human chromosome 17,
a23) nt 41490240 to nt 41492253 on human chromosome 18,
a24) nt 68579550 to nt 68579881 on human chromosome 18;
b1) nt 33725161 to nt 33736835 on human chromosome 1,
b2) nt 33741252 to nt 33773973 on human chromosome 1,
b3) nt 224909378 to nt 224922606 on human chromosome 1,
b4) nt 228390036 to nt 228392011 on human chromosome 1,
b5) nt 68637763 to nt 68639882 on human chromosome 3,
b6) nt 6138398 to nt 6161246 on human chromosome 4,
b7) nt 24575658 to nt 24595595 on human chromosome 4,
b8) nt 143690864 to nt 143707784 on human chromosome 4,
b9) nt 166750597 to nt 166784679 on human chromosome 5,
b10) nt 170305146 to nt 170321629 on human chromosome 5,
b11) nt 37873471 to nt 37891871 on human chromosome 7,
b12) nt 73954184 to nt 73974380 on human chromosome 10,
b13) nt 19435635 to nt 19445907 on human chromosome 11,
b14) nt 5007651 to nt 5009929 on human chromosome 16,
b15) nt 5009929 to nt 5027943 on human chromosome 16,
b16) nt 77369103 to nt 77371920 on human chromosome 16,
b17) nt 41997678 to nt 42020288 on human chromosome 19,
b18) nt 17424830 to nt 17435606 on human chromosome 20, or
b19) nt 112893833 to nt 112958447 on human chromosome 5,
or a fragment, mutated or complementary sequences thereof;
wherein the positions of the nucleotides are indicated according to the NCBI human genome sequence (Build 36.1, March 2006).

13. The DNA array according to claim 12, which comprises probes that specifically hybridize to all the sequences a1) to a24) and b1) to b19).

14. Use of a DNA array as defined in claim 12 or 13 for detecting *in vitro* a predisposition to sporadic pancreatic cancer and/or for diagnosing sporadic pancreatic cancer in a subject.
